# EUROPEAN PATENT SPECIFICATION

(11) **EP 2 315 532 B1**
(45) Date of publication and mention of the grant of the patent: **20.08.2014**
(21) Application number: 09790646.5
(22) Date of filing: 20.07.2009
(51) Int. Cl.: A23K 1/16, A23K 1/18

(54) **COMPOSITIONS AND METHODS FOR TREATING DISORDERS ASSOCIATED WITH OVERWEIGHT ANIMALS**
ZUSAMMENSETZUNGEN UND VERFAHREN FÜR DIE BEHANDLUNG VON BESCHWERDEN, DIE MIT ÜBERGEWICHTIGEN TIEREN ASSOZIIERT SIND
COMPOSITIONS ET PROCEDES POUR TRAITER DES TROUBLES ASSOCIES A DES ANIMAUX EN SURPOIDS

(30) Priority: 18.07.2008 US 82184; 06.01.2009 US 142709
(43) Date of publication of application: 04.05.2011
(73) Proprietor: Hill's Pet Nutrition, Inc., Topeka, KS 66605 (US)
(72) Inventor: YAMKA, Ryan, Michael, Topeka, KS 66618 (US); FRANTZ, Nolan, Zebulon, Topeka, KS 66618 (US); GAO, Xiangming, Topeka, KS 66615 (US); AL MURRANI, Samer, Topeka, KS 66614 (US)
(74) Representative: Daniels, Jeffrey Nicholas
(86) International application number: PCT/US2009/051188
(87) International publication number: WO 2010/009478

(56) References cited:
- EP-A1- 2 127 534
- WO-A1-02/056702
- WO-A1-03/061706
- WO-A1-2004/021799
- WO-A1-2008/009739
- OPITZ, B., SMITH, P.M., KIENZLE, E., EARLE, K.E., AND MASKELL, I.E.: "The measurement of dietary fibre in pet food: a comparison of methods" JOURNAL OF ANIMAL PHYSIOLOGY AND ANIMAL NUTRITION., vol. 79, 1998, pages 146-152, XP002562988 DEBLACKWELL, BERLIN. ISSN: 0931-2439

## Description

### FIELD OF THE INVENTION

The invention encompasses compositions and methods for treating disorders and diseases associated with obesity in cats and dogs using the compositions as claimed. The application also encompasses genes differentially expressed in animals and particularly to genes differentially expressed in obese animals compared to lean animals. The invention also encompasses compositions and methods for modulating the amount of fat in animals with targeted nutrition that is designed to affect, among others, key genes involved in fat metabolism. The invention further identifies bioactive dietary components that individually or together can affect the expression and activity of key genes involved in fat metabolism. The invention is defined only by the claims and is limited to compositions for canine and feline animals.

### BACKGROUND OF THE INVENTION

It is generally accepted in the scientific community that the regulation of gene expression plays a key role in the development of some diseases or conditions that affect an animal's health and well being. Similarly, the differential expression of genes is one factor in the development of such diseases and conditions and the evaluation of gene expression patterns has become recognized as crucial to understanding the development and control of such diseases and conditions at the molecular level. To advance the understanding of genes and their relationship to disease, a number of methods have been developed for studying differential gene expression, e.g., DNA microarrays, Expressed Sequence Tags (EST), serial analysis of gene expression (SAGE), subtractive hybridization, subtractive cloning and differential display (DD) for mRNA, RNA-arbitrarily primed PCR (RAP-PCR), Representational Difference Analysis (RDA), two-dimensional gel electrophoresis, mass spectrometry, Reverse Phase protein array and arrays.

Gene expression in overweight animals compared to lean animals has not been thoroughly investigated. Therefore, a need exists to identify genes and proteins that are differentially expressed in overweight animals compared to lean animals. Such genes, proteins, and their fragments would be useful for formulating a prognosis that an animal is likely to become fat, developing a diagnosis that an animal is fat, screening substances to determine if they are likely to be useful for modulating the amount of adipose tissue on an animal, and using such substances to modulate the amount of adipose tissue on an animal.

Overweight animals can be defined as those animals having an excess of body adipose tissue. Generally, animals such as humans, canines, and felines weighing more than 15% of their ideal body weight are considered fat. The most common cause of an animal being fat is an over consumption of food that results in an excess intake of calories. However, there are other factors that can increase an animal's chances for being fat, e.g., lifestyle, health, eating habits, breed, spaying, and neutering. Also, the incidence of animals becoming overweight generally increases with age due to a general decrease in metabolic rate and in physical activity. Surveys estimate that 25% of dogs in the United States that visit veterinary clinics are fat to the point of being obese. Studies have shown that overweight animals are significantly more at risk for diseases such as arthritis, heart disease, respiratory disease, diabetes, bladder cancer, hypothyroidism, and pancreatitis. Typical diets for dogs and cats are disclosed in WO 2008/009739.

Modulating the amount of adipose tissue on an animal, including preventing an animal from becoming overweight or treating a overweight animal to reduce the amount of adipose tissue on the animal or treating a lean animal to increase the amount of adipose tissue in the animal, is difficult. Increasing the amount of adipose tissue on an animal usually involves increasing the amount of food consumed. The most effective and easiest way to prevent an animal from becoming overweight or to reduce the amount of fat on an animal is with dietary restriction and exercise. However, it is often difficult to ensure compliance with diet and exercise programs. Other methods involve the use of drugs such as phentennine, fenfluramine, sibutramine, orlistat, and phenylpropanolamine. Unfortunately, side effects occur with these drugs. For example, the administration of fenfluramine and phentermine for the treatment of human obesity can result in cardiac valve damage in humans. Sibutramine can increase blood pressure and orlistat may have unpleasant gastrointestinal side effects.

Given the problems with current methods for dealing with adipose tissue on an animal, the inventors have developed methods and compositions useful for treating diseases and disorders in animals and in formulating a prognosis that an animal is likely to become fat, developing a diagnosis that an animal is fat, screening substances to determine if they are likely to be useful for modulating the amount of adipose tissue on an animal, and using such substances to modulate the amount of adipose tissue on an animal.

### SUMMARY OF THE INVENTION

The invention encompasses compositions useful in treating disorders in canine and feline animals in need thereof, and is defined by the claims.

In one embodiment, the invention encompasses a canine pet food composition comprising an effective amount of one or more ingredients that interfere with the expression of one or more genes differentially expressed in overweight animals compared to lean animals, wherein said one or more ingredients that interfere with the expression of one or more genes comprise 26 wt. % to 35 wt. % of crude protein on a dry matter basis, 7.5 wt. % to 8.5 wt. % of crude fat on a dry matter basis, 20 wt. % to 30 wt. % of total dietary fiber on a dry matter basis, and 10 wt. % to 20 wt. % of crude fiber on a dry matter basis.

In another embodiment, the invention encompasses a composition for feline consumption comprising an effective amount of one or more ingredients that interfere with the expression of one or more genes differentially expressed in overweight animals compared to lean animals, wherein said one or more ingredients that interfere with the expression of one or more genes comprise: 30 wt. % to 37 wt. % of crude protein on a dry matter basis, 7.5 wt. % to 9 wt. % of crude fat on a dry matter basis, 30 wt. % to 35 wt. % of total dietary fiber on a dry matter basis, and 20 wt. % to 25 wt. % of crude fiber on a dry matter basis.

Another embodiment encompasses a composition of the invention for use in treating or preventing insulin resistance in a companion animal in need thereof, for example, a canine or feline.

Another embodiment encompasses a composition of the invention for use in treating or preventing pancreatitis in a companion animal in need thereof, for example, a canine or feline.

Another embodiment encompasses a composition of the invention for use in treating or preventing hypothyroidism in a companion animal in need thereof, for example, a canine or feline.

Another embodiment encompasses a composition of the invention for use in treating or preventing osteoarthritis in a companion animal in need thereof, for example, a canine or feline.

Another embodiment encompasses a composition of the invention for use in treating or preventing dyslipidemia in a companion animal in need thereof, for example, a canine or feline.

Another embodiment encompasses a composition of the invention for use in treating or preventing hypertension in a companion animal in need thereof, for example, a canine or feline.

Another embodiment encompasses a composition of the invention for use in treating or preventing ocular disorders in a companion animal in need thereof, for example, a canine or feline.

Another embodiment encompasses a composition of the invention for use in treating or preventing altered kidney function in a companion animal in need thereof, for example, a canine or feline.

Another embodiment encompasses a composition of the invention for use in treating or preventing respiratory in a companion animal in need thereof, for example, a canine or feline.

Another embodiment encompasses a composition of the invention for use in treating or preventing artherosclerosis in a companion animal in need thereof, for example, a canine or feline.

Another embodiment encompasses a composition of the invention for use in treating or preventing diabetes mellitus in a companion animal in need thereof, for example, a canine or feline.

Another embodiment encompasses a composition of the invention for use in treating or preventing urinary tract disease in a companion animal in need thereof, for example, a canine or feline.

Another embodiment encompasses a composition of the invention for use in treating or preventing hepatic lipidosis in a companion animal in need thereof, for example, a canine or feline.

Another embodiment encompasses a composition of the invention for use in treating or preventing hepatic dyslipidemia in a companion animal in need thereof, for example, a canine or feline.

Another embodiment encompasses a composition of the invention for use in treating or preventing hepatic lipidosis in a companion animal in need thereof, for example, a canine or feline.

Another embodiment encompasses a composition of the invention for use in treating or preventing neoplasia in a companion animal in need thereof, for example, a canine or feline.

Another embodiment encompasses a composition of the invention for use in treating or preventing oral/dental disease in a companion animal in need thereof, for example, a canine or feline.

Another embodiment encompasses a composition of the invention for use in treating or preventing dermatopathy in a companion animal in need thereof, for example, a canine or feline.

Another embodiment encompasses a composition of the invention for use in treating or preventing lameness in a companion animal in need thereof, for example, a canine or feline.

Another embodiment encompasses a composition of the invention for use in treating or preventing hyperlipidemia in a companion animal in need thereof, for example, a canine or feline.

Another embodiment encompasses a composition of the invention for use in treating or preventing glucose metabolism disorders in a companion animal in need thereof, for example, a canine or feline.

Another embodiment encompasses a composition of the invention for use in treating or preventing coronary disease in a companion animal in need thereof, for example, a canine or feline.

The description provides one or more genes or gene segments that are differentially expressed in overweight animals compared to lean animals.

Another aspect of the description provides combinations of two.or more polynucleotides or polypeptides that are differentially expressed in overweight animals compared to lean animals.

Another aspect of the description provides compositions of two or more polynucleotide or polypeptide probes suitable for detecting the expression of genes differentially expressed in overweight animals compared to lean animals and devices such as substrate arrays containing the probes.

In another aspect, the description provides methods and compositions for detecting the differential expression of one or more genes differentially expressed in overweight animals compared to lean animals in a sample.

In another aspect, the description provides methods for measuring the effect of a test substance on the expression profile of one or more genes differentially expressed in overweight animals compared to lean animals as a method for screening a test substance to determine if it is likely to be useful for modulating the amount of adipose tissue on an animal.

In another aspect, the description provides methods for formulating a prognosis that an animal is likely to become overweight or developing a diagnosis that an animal is fat.

In another aspect, the description provides methods and compositions for modulating the expression of one or more genes differentially expressed in overweight animals compared to lean animals or for modulating the amount of adipose tissue on an animal.

One or more of these aspects are achieved using novel combinations of 433 polynucleotide probes representing genes and gene segments that are differentially expressed in the adipose tissue of overweight animals compared to the adipose tissue of lean animals (Table 1). In addition, one or more of these embodiments are achieved using novel combinations of 1703 polynucleotide probes representing genes and gene segments that are differentially expressed in lymphocytes taken from overweight animals compared to lymphocytes taken from lean animals (Table 2). Furthermore, one or more of these embodiments are achieved using novel combinations of the polynucleotide probes representing the genes and gene segments that are differentially expressed in overweight and lean dogs and are considered to be key genes for fatty acid metabolism (Table 3). The polynucleotides are used to produce compositions, probes, devices based on the probes, and methods for determining the status of polynucleotides differentially expressed in overweight animals compared to lean animals useful for achieving the above-identified objects, e.g., prognosing and diagnosing conditions relating to animal adipose tissue and for screening substances to determine if they arc likely to be useful for modulating the amount of adipose tissue on an animal. Such substances, once identified, may be used to modulate the amount of adipose tissue on an animal. Various kits comprising combinations of probes, devices utilizing the probes, and substances are also provided.

It is also an aspect of the description to encompass methods to modulate the amount of adipose tissue in an animal in vivo by administration of composition of the invention that is shown to modulate the expression of genes involved in fat metabolism.

It is also an aspect of the description to modulate various canine biomarkers related to obesity by administering a composition of the invention to an animal in need thereof in an amount effective to modulate the biomarkcr.

Examples of biomarkers related to obesity that can be modulated include, but are not limited to, glucose, insulin, GLP-I , IGF-I , cholesterol, triglycerides, LDL, chylomicrons, alkaline phosphatase, type-2 cartilage synthesis, leptin, ghrelin, and combinations thereof.

Other and further objects, features, and advantages of the present invention will be readily apparent to those skilled in the art.

### BRIEF DESCRIPTION OF THE FIGURES

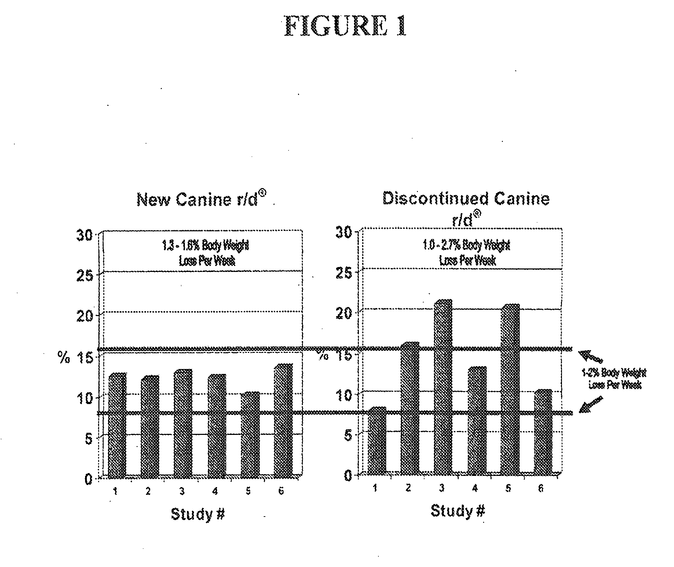

FIGURE 1 shows the result for weight loss of dogs fed the formula of the present invention as compared to a control weight loss fo[pi]nula.

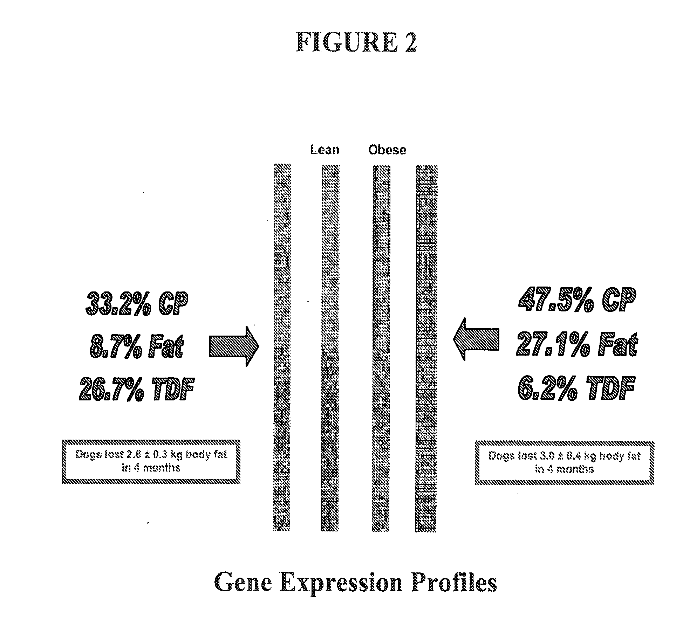

Figure 2 shows the shift in gene profile in dogs fed the formula of the present invention as compared to lean dogs, obese

### DETAILED DESCRIPTION OF THE INVENTION

### Definitions

The term "animal" means a human or other animal, including avian, bovine, canine, equine, feline, hicrine, murine, ovine, and porcine animals, that has adipose tissue. When the term is used in the context of comparing overweight to lean animals, the animals that are compared are animals of the same species and possibly of the same race or breed. In preferred embodiments, the animal is a canine or feline, most preferably a canine.

The term "antibody" means any immunoglobulin that binds to a specific antigen, including IgG, IgM, IgA, IgD, and IgE antibodies. The term includes polyclonal, monoclonal, monovalent, humanized, heteroconjugate, antibody compositions with polyepitopic specificity, chimeric, bispecific antibodies, diabodies, single-chain antibodies, and antibody fragments such as Fab, Fab', F(ab')₂, and Fv, or other antigen-binding fragments.

The term "array" means an ordered arrangement of at least two probes on a substrate. At least one of the probes is a control or standard and at least one of the probes is a diagnostic probe. The arrangement of from two or more probes (there are arrays, chips and other platforms now that go over 40,000) on a substrate assures that the size and signal intensity of each labeled complex formed between a probe and a sample polynucleotide or polypeptide is individually distinguishable.

The term "body condition score" (BCS) means a method for body composition analysis based upon an animal's body size and shape. Several methods are known to skilled artisans, e.g., methods disclosed in US. Patent No. 6,691,639 and in the reference entitled "Small Animal Clinical Nutrition", 4th Edition, in Chapter 13 (ISBN 0-945837-05-4).
The term "Body Mass Index" (BMI) means an animal's weight (in kilograms) divided by its height (in meters) squared.

The term "DEXA" means body composition analysis dual-energy X-ray absorptiometry.

The term "differential expression" or "differentially expressed" means increased or upregulated gene expression or means decreased or downregulated gene expression as detected by the absence, presence, or at least a 1.3-fold change in the amount of transcribed messenger RNA or translated protein in a sample.

The term "fat" as applied to an animal means any animal that is determined to have an excess amount of body adipose tissue or an animal that is prone to developing an excess amount of body adipose tissue using techniques and methods known to health care providers and other skilled artisans. An animal is prone to becoming overweight if the animal has an inclination or a higher likelihood of developing excess adipose tissue when compared to an average animal in the general population. Generally, without limiting the definition, an animal is considered overweight if (1) the animal has a BMI of 25 or more (a number considered to include "overweight" and "obese" in some methods of characterizing animal conditions), (2) the animal's weight is 15% or more than its "ideal" body weight as defined by health care professionals or related skilled artisans, (3) an animal's percent body fat is 27% or more as determined by DEXA, or (4) an animal has a body condition score of more than 3 as determined by skilled artisans using the method disclosed in "Small Animal Clinical Nutrition", 4th Edition, in Chapter 13 (ISBN 0-945837-05-4) or its equivalent using other BCS methods.

The term "fat-associated genes" means all or a subset of the genes identified in tables 1 and 2, particularly the genes represented by the 433 probe sequences that are differentially expressed between adipose tissue taken from overweight animals and adipose tissue taken from lean animals as well as the genes represented by the 1703 probe sequences that are differentially expressed between the lymphocytes taken from overweight animals and the lymphocytes taken from lean animals.

The term "key genes" means all or a subset of genes identified in table 3

The term "fold" when used as a measure of differential gene expression means an amount of gene expression in an animal that is a multiple or a fraction of gene expression compared to the amount of gene expression in a comparison animal, e.g., a overweight animals compared to a lean animal. For example, a gene that is expressed three times as much in the animal as in the comparison animal has a 3 fold differential gene expression and the gene is said to be up-regulated. On the other hand a gene that is expressed one-third as much in the animal as in the comparison animal also has a 3 fold differential gene expression and is said to be down-regulated.

The term "fragment" means (1) an oligonucleotide or polynucleotide sequence that is a portion of a complete sequence and that has the same or similar activity for a particular use as the complete polynucleotide sequence or (2) a peptide or polypeptide sequence that is a portion of a complete sequence and that has the same or similar activity for a particular use as the complete polypeptide sequence. Such fragments can comprise any number of nucleotides or amino acids deemed suitable for a particular use. Generally, oligonucleotide or polynucleotide fragments contain at least 10, 50, 100, or 1000 nucleotides and polypeptide fragments contain at least 4, 10, 20, or 50 consecutive amino acids from the complete sequence. The term encompasses polynucleotides and polypeptides variants of the fragments.

The term "gene" or "genes" means a complete or partial segment of DNA involved in producing a polypeptide, including regions preceding and following the coding region (leader and trailer) and intervening sequences (introns) between individual coding segments (exons). The term encompasses any DNA sequence that hybridizes to the complement of gene coding sequences.

The term "genes differentially expressed in overweight animals" means genes from which the amount of mRNA expressed or the amount of gene product translated from the mRNA is detectably different, either more or less, in tissue from overweight animals as compared to lean animals.

The term "homolog" means (1) a polynucleotide, including polynucleotides from the same or different animal species, having greater than 30%, 50%, 70%, or 90% sequence similarity to a polynucleotide identified in tables 1, 2, 3 and 5 and having the same or substantially the same properties and performing the same or substantially the same function as the complete polynucleotide, or having the capability of specifically hybridizing to a polynucleotide identified in tables 1, 2, 3 and 5 under stringent conditions or (2) a polypeptide, including polypeptides from the same or different animal species, having greater than 30%, 50%, 70%, or 90% sequence similarity to a polypeptide identified by the expression of polynucleotides identified in tables 1, 2, 3 and 5 and having the same or substantially the same properties and performing the same or substantially the same function as the complete polypeptide, or having the capability of specifically binding to a polypeptide identified by the expression of polynucleotides identified in tables 1, 2, 3 and 5. Sequence similarity of two polypeptide sequences or of two polynucleotide sequences is determined using methods known to skilled artisans, e.g., the algorithm of Karlin and Altschul (Proc. Natl. Acad. Sci. USA 87:2264-2268 (1990)). Such an algorithm is incorporated into the NBLAST and XBLAST programs of Altschul et al. (J. Mol. Biol. 215:403-410 (1990)). To obtain gapped alignments for comparison purposes, Gapped Blast can be utilized as described in Altschul et al. (Nucl. Acids Res. 25: 3389-3402 (1997)). When utilizing BLAST and Gapped BLAST programs, the default parameters of the respective programs (e.g., XBLAST and NBLAST) are used. See http://ww.ncbi.nlm.nih.gov.

The term "hybridization complex" means a complex that is formed between sample polynucleotides when the purines of one polynucleotide hydrogen bond with the pyrimidines of the complementary polynucleotide, e.g., 5'-A-G-T-C-3' base pairs with 3'-T-C-A-G-5'. The degree of complementarily and the use of nucleotide analogs affect the efficiency and stringency of hybridization reactions.

The term "in conjunction" means that a drug, food, or other substance is administered to an animal (1) together in a composition, particularly food composition, or (2) separately at the same or different frequency using the same or different administration routes at the same time or periodically. "Periodically" means that the substance is administered on a dosage schedule acceptable for a specific substance. "the same time" generally means that the substance (food or drug) is administered at the same time or within 72 hours of each other. "In conjunction" specifically includes administration schemes wherein substances such as drugs are administered for a prescribed period and compositions of the present invention are administered indefinitely.

The term "lean" as applied to an animal means any animal that is determined not to be overweight using techniques and methods known to health care providers and other skilled artisans. Generally, without limiting the definition, an animal is considered lean if (1) the animal has a BM1 of less than 25 or (2) the animal's weight is less than 15% more than its "ideal" body weight as defined by health care professionals or related skilled artisans, (3) an animal's percent body fat is less than 27% as determined by DEXA, or (4) an animal has a body condition score of 3 or less as determined by skilled artisans using the method disclosed in "Small Animal Clinical Nutrition", 4th Edition, in Chapter 13 (ISBN 0-945837-05-4) or it equivalent using other BCS methods.

The term "modulating the amount of adipose tissue on an animal" means causing the animal to lose adipose tissue, causing the animal to gain adipose tissue, or causing the animal to maintain the amount of adipose tissue on the animal if the animal is prone to gaining or losing adipose tissue. Thus, modulating the amount of adipose tissue on an animal encompasses preventing a lean animal from becoming overweight and treating a overweight animal to reduce the amount of adipose tissue on the animal, as well as treating a lean animal to add adipose tissue in appropriate circumstances, e.g., when treating a lean animal that is determined by skilled artisans to be so underweight that the addition of adipose tissue is desirable. Conventional methods may be used to assess the amount of adipose tissue on an animal, as well as to determine the animal's lean muscle mass and /or bone mineral content, information which may be of relevance in such an assessment.

The term "polynucleotide" or "oligonucleotide" means a polymer of nucleotides. The term encompasses DNA and RNA (including cDNA and mRNA) molecules, either single or double stranded and, if single stranded, its complementary sequence in either linear or circular form. The term also encompasses fragments, variants, homologs, and alleles, as appropriate for the sequences that have the same or substantially the same properties and perform the same or substantially the same function as the original sequence. The sequences may be fully complementary (no mismatches) when aligned or may have up to a 30% sequence mismatch. Preferably, for polynucleotides, the chain contains from 50 to 10,000 nucleotides, more preferably from 150 to 3,500 nucleotides. Preferably, for oligonucleotides, the chain contains from 2 to 100 nucleotides, more preferably from 6 to 30 nucleotides. The exact size of a polynucleotide or oligonucleotide will depend on various factors and on the particular application and use of the polynucleotide or oligonucleotide. The term includes nucleotide polymers that are synthesized and that are isolated and purified from natural sources. The term "polynucleotide" is inclusive of "oligonucleotide."

The term "polypeptide," "peptide," or "protein" means a polymer of amino acids. The term encompasses naturally occurring and non-naturally occurring (synthetic) polymers and polymers in which artificial chemical mimetics are substituted for one or more amino acids. The term also encompasses fragments, variants, and homologs that have the same or substantially the same properties and perform the same or substantially the same function as the original sequence. The term encompass polymers of any length, preferably polymers containing from 2 to 1000 amino acids, more preferably from 5 to 500 amino acids. The term includes amino acid polymers that are synthesized and that arc isolated and purified from natural sources.

The term "probe" means (1) an oligonucleotide or polynucleotide, either RNA or DNA, whether occurring naturally as in a purified restriction enzyme digest or produced synthetically, that is capable of annealing with or specifically hybridizing to a polynucleotide with sequences complementary to the probe or (2) a peptide or polypeptide capable of specifically binding a particular protein or protein fragment to the substantial exclusion of other proteins or protein fragments. An oligonucleotide or polynucleotide probe may be either single or double stranded. The exact length of the probe will depend upon many factors, including temperature, source, and use. For example, for diagnostic applications, depending on the complexity of the target sequence, an oligonucleotide probe typically contains 10 to 100, 15 to 50, or 15 to 25 nucleotides. In certain diagnostic applications, a polynucleotide probe contains 100-1000, 300-600, nucleotides, preferably 300 nucleotides. The probes herein are selected to be "substantially" complementary to different strands of a particular target sequence. This means that the probes must be sufficiently complementary to specifically hybridize or anneal with their respective target sequences under a set of predetermined conditions. Therefore, the probe sequence need not reflect the exact complementary sequence of the target. For example, a noncomplementary nucleotide fragment may be attached to the 5' or 3' end of the probe, with the remainder of the probe sequence being complementary to the target sequence. Alternatively, noncomplementary bases or longer sequences can be interspersed into the probe provided that the probe sequence has sufficient complementarity with the sequence of the target polynucleotide to specifically anneal to the target polynucleotide. A peptide or polypeptide probe may be any molecule to which the protein or peptide specifically binds, including DNA (for DNA binding proteins), antibodies, cell membrane receptors, peptides, cofactors, lectins, sugars, polysaccharides, cells, cell membranes, organelles and organellar membranes.

The term "sample" means any animal tissue or fluid containing, e.g., polynucleotides, polypeptides, antibodies, metabolites, and the like, including cells and other tissue containing DNA and RNA. Examples include adipose, blood, cartilage, connective, epithelial, lymphoid, muscle, nervous, sputum, and the like. A sample may be solid or liquid and may be DNA, RNA, cDNA, bodily fluids such as blood or urine, cells, cell preparations or soluble fractions or media aliquots thereof, chromosomes, organelles, and the like.

The term "single package" means that the components of a kit are physically associated in or with one or more containers and considered a unit for manufacture, distribution, sale, or use. Containers include, but are not limited to, bags, boxes, bottles, shrink wrap packages, stapled or otherwise affixed components, or combinations thereof. A single package may be containers of individual food compositions physically associated such that they are considered a unit for manufacture, distribution, sale, or use.

The term "useful variations" means (1) for a polynucleotide, the complements of the polynucleotide; the homologs of the polynucleotide and its complements; the variants of the polynucleotide, its complements, and its homologs; and the fragments of the polynucleotide, its complements, its homologs, and its variants and (2) for a polypeptide, the homologs of the polypeptide; the variants of the polypeptide and its homologs; and the fragments of the polynucleotide, its homologs, and its variants.

The term "virtual package" means that the components of a kit are associated by directions on one or more physical or virtual kit components instructing the user how to obtain the other components, e.g., in a bag containing one component and directions instructing the user to go to a website, contact a recorded message, view a visual message, or contact a caregiver or instructor to obtain instructions on how to use the kit.

The term "standard" means (1) a control sample that contains tissue from a lean animal if a overweight animal is being tested or tissue from a overweight animal if a lean animal is being tested or (2) a control sample that contains tissue from a lean or overweight test animal that has not been exposed to a test substance being examined in the corresponding lean or overweight animal to determine if the test substance causes differential gene expression, as appropriate for the context of its use.

The term "stringent conditions" means (1) hybridization in 50% (vol/vol) formamide with 0.1% bovine serum albumin, 0.1% Ficoll, 0.1% polyvinylpyrrolidone, 50 mM sodium phosphate buffer at pH 6.5 with 750 mM NaCl, 75 mM sodium citrate at 42°C, (2) hybridization in 50% formamide, 5x SSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 µg/ml), 0.1% SDS, and 10% dextran sulfate at 42°C; with washes at 42°C in 0.2x SSC and 0.1% SDS or washes with 0.015 M NaCl, 0.0015 M sodium citrate, 0.1% Na₂SO₄ at 50°C or similar procedures employing similar low ionic strength and high temperature washing agents and similar denaturing agents.

The term "substance" means an element, compound, molecule, or a mixture thereof or any other material that could potentially be useful for diagnosing, prognosing, or modulating the amount of adipose tissue on animals, including any drug, chemical entity, or biologic entity.

The term "siRNA" means a polynucleotide that forms a double stranded RNA that reduces or inhibits expression of a gene when the siRNA is expressed in the same cell as the gene. The term encompasses double stranded RNA formed by complementary strands. The siRNA complementary portions that hybridize to form the double stranded molecule typically have substantial or complete identity. Typically, siRNA contains at least 15-50 nucleotides and the double stranded siRNA contains 15-50 base pairs, preferably 20-30 nucleotides and base pairs.

The term "specifically bind" means a special and precise interaction between two molecules which is dependent upon their structure, particularly their molecular side groups. For example, the intercalation of a regulatory protein into the major groove of a DNA molecule, the hydrogen bonding along the backbone between two single stranded nucleic acids, or the binding between an epitope of a protein and an agonist, antagonist, or antibody.

The term "specifically hybridize" means an association between two single stranded polynucleotides of sufficiently complementary sequence to permit such hybridization under predetermined conditions generally used in the art (sometimes termed "substantially complementary"). For example, the term may refer to hybridization of a polynucleotide probe with a substantially complementary sequence contained within a single stranded DNA or RNA molecule according to an aspect of the invention, to the substantial exclusion of hybridization of the polynucleotide probe with single stranded polynucleotides of non-complementary sequence.

The term "variant" means (1) a polynucleotide sequence containing any substitution, variation, modification, replacement, deletion, or addition of one or more nucleotides from or to a polynucleotide sequence and that has the same or substantially the same properties and performs the same or substantially the same function as the original sequence and (2) a polypeptide sequence containing any substitution, variation, modification, replacement, deletion, or addition of one or more amino acids from or to a polypeptide sequence and that has the same or substantially the same properties and performs the same or substantially the same function as the original sequence. The term therefore includes single nucleotide polymorphisms (SNPs) and allelic variants and includes conservative and non-conservative amino acid substitutions in polypeptides. The term also encompasses chemical derivatization of a polynucleotide or polypeptide and substitution of nucleotides or amino acids with nucleotides or amino acids that do not occur naturally, as appropriate.

The invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise, e.g., reference to "a variant" includes a plurality of variants. Further, defined terms include variations of the terms used in the proper grammatical context, e.g., the term "specifically binds" includes "specific binding" and other forms of the term. Similarly, the words "comprise", "comprises", and "comprising" are to be interpreted inclusively rather than exclusively.

Unless defined otherwise, all technical and scientific terms and any acronyms used herein have the same meanings as commonly understood by one of ordinary skill in the art in the field of the invention. Although any compositions, methods, articles of manufacture, or other means or materials similar or equivalent to those described herein can be used in the practice of the present invention, the preferred compositions, methods, articles of manufacture, or other means or materials are described herein.

### Modulation of Gene Protein Expression

In one embodiment, the description encompasses one or more genes or gene segments ("genes" as defined herein) that are differentially expressed in the adipose tissue and/or lymphocytes of overweight animals compared to the adipose tissue and/or lymphocytes of lean animals. The invention is based upon the discovery of differentially expressed genes that are represented by 445 polynucleotides in the adipose tissue of overweight animals compared to the adipose tissue of lean animals and 1767 polynucleotides in the lymphocytes taken from overweight animals compared to lymphocytes taken from lean animals. The invention is further based upon the identification of 7 key genes involved in fatty acid metabolism that are diffenretially expressed between overweight and lean animals and are listed in table 3. These key genes represent members of the pyruvate dehydrogenase kinase family, the carnitine palmitoyltransferase family and solute carrier family 27 (fatty acid transporters) and genes associated with the elongation of long chain fatty acids, with the pyruvate dehedrogenase kinase family and the carnitine palmitoyltransferase family being the most important and in some instances rate limiting enzymes. The genes were identified by comparing the expression of genes in adipose tissue and lymphocytes taken from animals diagnosed as overweight with genes in adipose tissue and lymphocytes from animals diagnosed as lean using Affymetrix GeneChip® technology. The polynucleotides are shown in Table 1, 2 and 3. The tables contain information the Affymetrix Probe Identification Number (herein "APIN"), the p-value, the q-value, fold expression (fat/lean), the top BLAST annotation of the probe in question, the Accession Number of Highest BLAST Hit, the gene symbol and finally the gene description is given in the last column. A description of the putative or actual gene can be in some instances obtained from the BLAST database using methods known to skilled artisans. Generally, the putative or actual gene function is determined by (1) identifying the APIN for each gene that had 1.3 fold or greater gene expression in overweight animals compared to lean animals, (2) determining the nucleotide sequence of each such gene by inputting the APIN into the publicly available Affymetrix database that correlates AIPN numbers with sequences, and (3) inputting the nucleotide sequence into the BLAST database provided by the National Institutes of Health and determining the putative or actual gene function from the resulting sequence matches to homologous sequences in the database.

The polynucleotides and genes are identified by measuring differences in gene expression from adipose tissue and the lymphocytes from canines diagnosed as overweight with gene expression in adipose tissue and the lymphocytes from canines diagnosed as lean. Changes in gene expression can be determined by any method known to skilled artisans. Generally, changes in gene expression are determined by measuring transcription (determining the amount of mRNA produced by a gene) or measuring translation (determining the amount of protein produced by a gene). The amount of RNA or protein produced by a gene can be determined using any method known to skilled artisans for quantifying polynucleotides and proteins. Generally, RNA expression is determined using methods including but not limited to polymerase chain reaction (PCR) (including, without limitation, reverse transcription-PCR (RT-PCR) and quantitative real-time PCR (qPCR)), RNase protection, Northern blotting, and other hybridization methods. The RNA measured is typically in the form of RNA or reverse transcribed mRNA or complimentary DNA (cDNA). Protein or polypeptide expression is determined using various colormetric, flourescense and spectroscopic assays and methods including but not limited to Western Blotting, ELISA, Multiple Reaction Monitoring, Reverse Phase and Antibody Arrays. In a preferred method, changes in gene expression are determined using Affymetrix Canine-1 and Canine-2 GeneChip® available for purchase from Affymetrix, Inc. and the instructions for using such chips to determine gene expression.

Generally, differential gene expression in overweight animals compared to lean animals is determined by measuring the expression of at least one gene. Preferably, the expression of two or more differentially expressed genes is measured to provide a gene expression pattern or gene expression profile. More preferably, the expression of a plurality of differentially expressed genes is measured.

The polynucleotides, genes, proteins encoded by the polynucleotides and genes, and the complements, homologs, variants, or fragments based upon the sequences are useful in a variety of prognostic and diagnostic assays relating to the amount of adipose tissue on an animal and are useful for screening test substances to determine if the substances are useful for modulating the amount of adipose tissue on an animal. Other uses will be apparent from the description of the invention contained herein.

In another aspect, the description provides a combination comprising two or more polynucleotides that are differentially expressed in overweight animals compared to lean animals or two or more proteins produced by the expression of two or more polynucleotides that are differentially expressed in overweight animals compared to lean animals. In one aspect, the combination comprises two or more polynucleotides or proteins expressed from polynucleotides selected from Tables 1 and 2 and preferably from table 3. Preferably, the combination comprises a plurality of polynucleotides or proteins expressed from polynucleotides identified in tables I and 2 and preferably from table 3, generally 10, 20, 50, 100, 200, or more polynucleotides or proteins, as appropriate for a particular Group and use. When the combination comprises one or more fragments, the fragments can be of any size that retains the properties and function of the original polynucleotide or protein, preferably from 30%, 60%, or 90% of the original. The polynucleotides and proteins can be from any animal, preferably canines and felines, most preferable canines.

In another aspect, the description provides a composition comprising two or more oligonucleotide or polynucleotide probes suitable for detecting the expression of genes differentially expressed in overweight animals compared to lean animals. In one embodiment, the probes comprise polynucleotides selected from tables 1 and 2 and preferably from table 3. In another, the probes comprise useful variations of such polynuelcotides. The probes contain a sufficient number of nucleotides to specifically hybridize substantially exclusively with appropriate complementary polynucleotides. In certain embodiments, the probes comprise at least 10, 15, 20, 25, or 30 nucleotides. In some embodiments, the probes contain more nucleotides and comprise at least 30, 50, 70, 90 or 100 nucleotides, or more. The probes may comprise full length functional genes of the present invention. Preferably, the composition comprises a plurality of polynucleotide probes suitable for detecting genes differentially expressed in overweight animals compared to lean animals, generally 10, 50, 200, 500, 1000, or 2000, or more probes. The polynucleotide probes are made or obtained using methods known to skilled artisans, e.g., in vitro synthesis from nucleotides, isolation and purification from natural sources, or enzymatic cleavage of the genes of the present invention.

In another aspect, the description provides a device suitable for detecting the expression of a plurality of genes differentially expressed in overweight animals compared to lean animals. The device comprises a substrate having a plurality of the oligonucleotide or polynucleotide probes of the present invention affixed to the substrate at known locations. The device is essentially an immobilized version of the oligonucleotide or polynucleotide probes described herein. The device is useful for rapid and specific detection of genes and polynucleotides and their expression patterns and profiles. Typically, such probes are linked to a substrate or similar solid support and a sample containing one or more polynucleotides (e.g., a gene, a PCR product, a ligase chain reaction (LCR) product, a DNA sequence that has been synthesized using amplification techniques, or a mixture thereof) is exposed to the probes such that the sample polynucleotide(s) can hybridize to the probes. Either the probes, the sample polynucleotide(s), or both, are labeled, typically with a fluorophore or other tag such as streptavidin, and detected using methods known to skilled artisans. If the sample polynucleotide(s) is labeled, hybridization may be detected by detecting bound fluorescence. If the probes are labeled, hybridization is typically detected by label quenching. If both the probe and the sample polynucleotide(s) are labeled, hybridization is typically detected by monitoring a color shift resulting from proximity of the two bound labels. A variety of labeling strategies and labels are known to skilled artisans, particularly for fluorescent labels. Preferably, the probes are immobilized on substrates suitable for forming an array (known by several names including DNA microarray, gene chip, biochip, DNA chip, and gene array) comparable to those known in the art.

In another aspect, the description provides a composition comprising two or more peptide or polypeptide probes suitable for detecting the expression of genes differentially expressed in overweight animals compared to lean animals. In one embodiment, the probes comprise peptides or polypeptides that specifically bind to proteins produced by the expression of one or more polynucleotides comprising sequences selected from tables 1 and 2. In another, the probes comprise peptides or polypeptides that specifically bind to proteins produced by expression of one or more polynucleotides comprising sequences selected from table 3.. In another, the probes comprise peptides or polypeptides that specifically bind to proteins produced by expression of one or more useful variations of such polypeptides. The probes contain a sufficient number of amino acids to specifically bind to the appropriate polypeptides. Preferably, the probes comprise at least 4, 10, 20, 40, or 80 amino acids. In some embodiments, the probes contain more amino acids and comprise at least 100 or more amino acids. The probes may comprise full length functional proteins derived from the expression of full length functional genes identified by the present invention. Preferably, the invention provides a plurality of polypeptide probes suitable for detecting genes differentially expressed in overweight animals compared to lean animals, more preferably a collection of 10, 50, 100, 500, or 1000 or more of such probes. In one embodiment, the probes are antibodies, preferably monoclonal antibodies.

The polypeptide probes may be made according to conventional methods, e.g., using the nucleotide sequence data provided for polynucleotides of the present invention and methods known in the art. Such methods include, but are not limited to, isolating polypeptide directly from cells, isolating or synthesizing DNA or RNA encoding the polypeptides and using the DNA or RNA to produce recombinant products, synthesizing the polypeptides chemically from individual amino acids, and producing polypeptide fragments by chemical cleavage of existing polypeptides.

In another aspect, the description provides a device suitable for detecting the expression of a plurality of genes differentially expressed in overweight animals compared to lean animals. The device comprises a substrate having a plurality of the peptide or polypeptide probes of the present invention affixed to the substrate at known locations. The device is essentially an immobilized version of the peptide or polypeptide probes described herein. The device is useful for the rapid and specific detection of proteins and their expression patterns. Typically, such probes are linked to a substrate and a sample containing one or more proteins is exposed to the probes such that the sample proteins can hybridize to the probes. In certain embodiments, the probes, the sample proteins, or both, are labeled and detected, typically with a fluorophore or other agent known to skilled artisans. Generally, the same methods and instrumentation used for reading polynucleotide microarrays is applicable to protein arrays. Preferably, the probes are immobilized on a substrate suitable for forming an array.

Methods for determining the amount or concentration of protein in a sample are known to skilled artisans. Such methods include radioimmunoassays, competitive-binding assays, Western blot analysis, and ELISA assays. For methods that use antibodies, polyclonal and monoclonal antibodies are suitable. Such antibodies may be immunologically specific for a protein, protein epitope, or protein fragment.

Some aspects of the description utilize antibodies for the detection and quantification of proteins produced by expression of the polynucleotides of the present invention. Although proteins may be detected by immunoprecipitation, affinity separation, Western blot analysis, protein arrays, and the like, a preferred method utilizes ELISA technology wherein the antibody is immobilized on a solid support and a target protein or peptide is exposed to the immobilized antibody. Either the probe, or the target, or both, can be labeled using known methods.

In some aspects expression patterns or profiles of a plurality of genes differentially expressed in overweight animals compared to lean animals are observed utilizing an array of probes for detecting polynucleotides or polypeptides. In one embodiment, arrays of oligonucleotide or polynucleotide probes may be utilized, whereas another embodiment may utilize arrays of antibodies or other proteins that specifically bind to the differentially expressed gene products of the present invention. Such arrays may be commercially available or they may be custom made using methods known to skilled artisans, e.g., in-situ synthesis on a solid support or attachment of pre-synthesized probes to a solid support via microprinting techniques. In various embodiments, arrays of polynucleotides or polypeptides probes are custom made to specifically detect transcripts or proteins produced by the differentially expressed genes of the present invention.

In one aspect arrays of polynucleotide or polypeptide probes are custom made to specifically detect transcripts or proteins produced by two or more polynucleotides or genes identified in Table 2. These probes are designed to detect genes associated with lipid and glucose metabolism pathways in animals. In another embodiment, arrays of polynucleotide or polypeptide probes are custom made to specifically detect transcripts or proteins produced by two or more polynucleotides or genes identified in Table 3. These probes are designed to detect genes that are particularly relevant to overweight animals compared to lean animals.

In a further aspect, the description provides a method for detecting the differential expression of one or more genes differentially expressed in overweight animals compared to lean animals in a sample. The method comprises (a) hybridizing a combination comprising a plurality of polynucleotide probes that are differentially expressed in overweight animals compared to lean animals with polynucleotides in the sample to form one or more hybridization complexes; (b) optionally, hybridizing a combination comprising a plurality of polynucleotide probes that are differentially expressed in overweight animals compared to lean animals with polynucleotides in a standard to form one or more hybridization complexes; (c) detecting the hybridization complexes from the sample and, optionally, the standard from step (b); and (d) comparing the hybridization complexes from the sample with the hybridization complexes from a standard, wherein a difference in the amount of hybridization complexes between the standard and sample indicate differential expression of genes differentially expressed in overweight animals compared to lean animals in the sample. In various embodiments, the plurality of polynucleotide probes are selected from Tables 1 and 2 and preferably from table 3. These polynucleotides are used to prepare probes that hybridize with sample polynucleotides to form hybridization complexes that are detected and compared with those of the standard. In some embodiments, the sample polynucleotides are amplified prior to hybridization. In some embodiments, the probes are bound to a substrate, preferably in an array.

Step (b) and part of step (c) are optional and are used if a relatively contemporaneous comparison of two or more test systems is to be conducted. However, in a preferred embodiment, the standard used for comparison is based upon data previously obtained using the method.

These probes are exposed to a sample to form hybridization complexes that are detected and compared with those of a standard. The differences between the hybridization complexes from the sample and standard indicate differential expression of polynucleotides and therefore genes differentially expressed in overweight animals compared to lean animals in the sample. In a preferred embodiment, probes are made to specifically detect polynucleotides or fragments thereof produced by one or more of the genes or gene fragments identified by the present invention. Methods for detecting hybridization complexes are known to skilled artisans.

In one aspect, the method of the description further comprises exposing the animal or sample to a test substance before hybridization. Then, the comparison is indicative of whether the test substance altered the expression of genes differentially expressed in overweight animals compared to lean animals, particularly fat-associated genes, in the sample.

In another aspect, the description provides a method for detecting the differential expression of genes differentially expressed in overweight animals compared to lean animals in a sample. The method comprises (a) reacting a combination comprising a plurality of polypeptide probes with proteins in the sample under conditions that allow specific binding between the probes and the proteins to occur, wherein the proteins bound by the probes are differentially expressed in a overweight animal compared to a lean animal; (b) optionally, reacting a combination comprising a plurality of polypeptide probes with proteins in a standard under conditions that allow specific binding between the probes and the proteins to occur, wherein the proteins bound by the probes are differentially expressed in a overweight animal compared to a lean animal; (c) detecting specific binding in the sample and, optionally, the standard from step (b); and (d) comparing the specific binding in the sample with that of a standard, wherein differences between the specific binding in the standard and the sample indicate differential expression of genes differentially expressed in overweight animals compared to lean animals in the sample.

In various embodiments, the plurality of polypeptide probes are probes that specifically bind to proteins produced by expression of one or more polynucleotides selected from Tables 1 and 2 and preferably from table 3 and useful variations of such polynucleotides. These polynucleotides are used to prepare probes that specifically bind to proteins that are detected and compared with those of the standard. In some embodiments, the probes are bound to a substrate, preferably in an array. In one embodiment, the probes are antibodies.

Step (b) and part of step (c) are optional and are used if a relatively contemporaneous comparison of two or more test systems is to be conducted. However, in a preferred embodiment, the standard used for comparison is based upon data previously obtained using the method.

These probes are exposed to a sample to form specific binding that is detected and compared with those of a standard. The differences between the specific binding from the sample and standard indicate differential expression of proteins and therefore genes differentially expressed in overweight animals compared to lean animals, particularly fat-associated genes, in the sample. In a preferred embodiment, probes are made to specifically detect proteins or fragments thereof produced by one or more of the genes or gene fragments identified by the present invention.

In one aspect, the description further comprises exposing the animal or sample to a test substance before reacting the polypeptides with the proteins. Then, .the comparison is indicative of whether the test substance altered the expression of genes differentially expressed in overweight animals compared to lean animals, particularly fat-associated genes, in the sample.

In another aspect, the method of the description for detecting the expression of genes differentially expressed in overweight animals compared to lean animals in a sample is used to monitor an animal's progress when attempting to modulate the amount of adipose tissue on the animal in response to an adipose tissue modulation program. The method is performed at intervals, preferably set intervals, during the modulation program and the animal's progress monitored by comparing the results of the method at two or more points during the modulation program. A change in expression of one or more of the genes differentially expressed in overweight animals compared to lean animals, particularly fat-associated genes, or in the pattern of gene expression, or the lack of any change, resulting from the comparison indicates the effectiveness of the modulation program. For example, an adipose tissue modulation program designed to reduce the amount of adipose tissue on an animal could be monitored and shown to be effective if the amount of gene expression for genes differentially expressed in overweight animals compared to lean animals, particularly fat-associated genes, declines over time in response to the stimulus in the program. Similarly, a program to increase adipose tissue in a lean or overly lean animal should increase the expression profile for such genes. The modulation program can be any plan to modulate the amount of adipose tissue on the animal such as a diet, exercise, drug, or other similar program.

In a further aspect, the description provides a method for measuring the effect of a test substance on the expression profile of one or more genes differentially expressed in overweight animals compared to lean animals and a method for screening a test substance to determine if it is likely to be useful for modulating the amount of adipose tissue on an animal. The methods comprise (a) determining a first expression profile by measuring the transcription or translation products of two or more polynucleotides selected from Tables 1 and 2 and preferably from table 3 or useful variations thereof in a test system in the absence of the test substance; (b) determining a second expression profile by measuring the transcription or translation products of two or more polynucleotides selected from Tables 1 and 2 and preferably from table 3 or useful variations thereof in a test system in the presence of the test substance; and (c) comparing the first expression profile to the second expression profile.

A change in the second expression profile compared to the first expression profile of 1.3 fold or more indicates that the test substance effects the expression of genes differentially expressed in overweight animals compared to lean animals and that the test substance is likely to be useful for modulating the amount of adipose tissue on an animal. In a preferred embodiment, the genes differentially expressed in overweight animals compared to lean animals are key genes associated with fatty acid metabolism and the change is a 1.3 fold or more change in expression of at least two genes between the first expression profile to the second expression profile. The invention also provides the substances identified using the method.

In one embodiment, the polynucleotides are selected from Table 3 or useful variations thereof and the change is 1.3 fold or higher.

In one embodiment, the test system is an in vitro test system such as a tissue culture, cell extract, or cell line. In another, the test system is an in vivo test system, i.e., an animal such as a canine. In other embodiments, the test system is an ex vivo tissue system or an in silico system.

Test substances can be any substance that may have an effect on polynucleotides or genes differentially expressed in overweight animals compared to lean animals, particularly fat-associated genes. Test substances include, but are not limited to, amino acids; proteins, peptides, polypeptides, nucleic acids, oligonucleotides, polynucleotides, small molecules, macromolecules, vitamins, minerals, simple sugars; complex sugars; polysaccharides; carbohydrates; medium-chain triglycerides (MCTs); triacylglycerides (TAGs); n-3 (omega-3) fatty acids including DHA, EPA, ALA; n-6 (omega-6) fatty acids including LA, γ-linolenic acid (GLA) and ARA; SA, MA, conjugated linoleic acid (CLA); choline sources such as lecithin; fat-soluble vitamins including vitamin A and precursors thereof such as carotenoids (e.g., β-carotene), vitamin D sources such as vitamin D₂ (ergocalciferol) and vitamin D₃ (cholecalciferol), vitamin E sources such as tocopherols (e.g., α-tocopherol) and tocotrienols, and vitamin K sources such as vitamin K₁ (phylloquinone) and vitamin K₂ (menadione); water-soluble vitamins including B vitamins such as riboflavin, niacin (including nicotinamide and nicotinic acid), pyridoxine, pantothenic acid, folic acid, biotin and cobalamin; and vitamin C (ascorbic acid); antioxidants, including some of the vitamins listed above, especially vitamins E and C; also bioflavonoids such as catechin, quercetin and theaflavin; quinones such as ubiquinone; carotenoids such as lycopene and lycoxanthin; resveratrol; and α-lipoic acid; L-camitine; D-limonene; glucosamine; S-adenosylmethionine; and chitosan. In a preferred embodiment, test substances are nutrients that may be added to food or consumed as a supplement. Examples include, but are not limited to, fatty acids such as omega-3 fatty acids (e.g., DHA and EPA) and omega-6 fatty acids (e.g., ARA), carnitine, methionine, vitamin C, vitamin E, and vitamin D.

In a preferred embodiment, the substances useful for affecting the expression of genes differentially expressed in overweight animals compared to lean animals, particularly fat-associated genes, may be identified using methods discloses in co-pending US Provisional Patent Application No. 60/657980, filed March 2, 2005, and any subsequent US or foreign patent application that claims priority thereto.

In a further embodiment, the description encompasses a method for formulating a prognosis that an animal is likely to become overweight or developing a diagnosis that an animal is fat. The method comprises determining if one or more polynucleotides selected from tables I and 2 or useful variations thereof or one or more polypeptides that specifically bind to proteins produced by expression of one or more polynucleotides selected from tables 1, 2 or useful variations thereof are differentially expressed in the animal compared to one or more lean animals. The animal is determined to be likely to become overweight or determined to be overweight if the comparison indicates that the polynucleotides are differentially expressed in the animal compared to the lean animals by a fold of 1.3 or more.

In various embodiments, the prognosis or diagnosis is based upon the polynucleotides selected from Table 3, or useful variations of such polypeptides.

The expression profile for lean animals used in the comparison can be obtained from one or more lean animals contemporaneously with the expression profile for the animal being tested of from a database of lean animal expression profiles. Preferably, a database of expression profiles for lean animals accumulated over time is available for use as a reference.

Determining if the polynucleotides or polypeptides are differentially expressed can be accomplished by detecting the polynucleotides or polypeptides using methods known to skilled artisans, some of which are described herein.

In another aspect, the description provides a method for manipulating the genome or the expression of the genome of an animal, particularly a non-human animal. The method comprises disrupting the expression of one or more genes differentially expressed in overweight animals compared to lean animals, preferably using oligonucleotides or polynucleotides constructed using polynucleotides selected from tables And 2 and preferably from table 3 or useful variations thereof.

Methods of manipulating the genome are known to those of skilled in the art. Such methods include the production of transgenic and knockout animals and the disruption of transcription or translation. In one embodiment, one or more polynucleotides selected from tables 1 and 2 or useful variations thereof are used to prepare a construct useful to disrupt or "knock out" the corresponding endogenous gene in an animal. This method produces an animal having a null mutation for that gene locus. In other embodiments, the animals exhibit a reduction or complete elimination of the expression of one or more genes differentially expressed in overweight animals compared to lean animals, particularly fat-associated genes. The invention also provides an animal produced using the method. In various embodiments, the genome is manipulated using the one or more polynucleotides selected from Table 3, or useful variations of such sequences. The transgenic animals are preferably mammals, e.g., rodents such as mice and rats, but may be other mammal such as felines and canines.

Methods of manipulating the expression of genome are known to those of skilled in the art. Such methods include the use of antisense or siRNA molecules and using such molecules to disrupt the translation or transcription of the genome. In one embodiment, one or more polynucleotides selected from tables 1 and 2 and preferably from table 3 or useful variations thereof are used to prepare antisense and similar DNA binding molecules that are useful for disrupting transcription or to prepare short (small) interfering RNAs (siRNA) useful for functionally disrupting translation. Briefly, gene expression is inhibited by antisense molecules through binding to DNA and preventing transcription and a siRNA through RNA interference (RNAi) or post-transcriptional gene silencing (PTGS). siRNA molecules target homologous mRNA molecules for destruction by cleaving the mRNA molecule within the region spanned by the siRNA molecule. Accordingly, siRNAs capable of targeting and cleaving a mRNA transcribed from a fat-associated gene is used to decrease or eliminate expression of one or more of such genes. In other embodiments, antisense molecules capable of binding to DNA and siRNAs capable of targeting and cleaving mRNA transcribed from one or more polynucleotides or genes selected from tables 1 and 2 and preferably from table 3 or useful variations thereof.

In another aspect, the description provides a composition suitable for manipulating the genome of an animal. The composition comprises one or more substances that interfere with the expression of one or more genes differentially expressed in overweight animals compared to lean animals, particularly fat-associated genes. Preferably, substances comprise oligonucleotides or polynucleotides that bind to one or more of the genes or their transcription products and interferes with their replication, transcription, or translation, most preferably oligonucleotides or polynucleotides constructed using polynucleotides selected from tables 1 and 2 and preferably from table 3 or useful variations thereof. In various embodiments, the substances comprise antisense molecules or siRNAs.

In another embodiment, the description provides a method for modulating the expression of one or more genes differentially expressed in overweight animals compared to lean animals, particularly fat-associated genes, or modulating the amount of adipose tissue on an animal comprising administering to the animal a gene expression or tissue modulating amount of a composition comprising one or more of DHA, EPA, EPA and DHA, ALA, LA, ARA, SA and MA. In preferred embodiments the composition comprises, in milligrams per kilogram of body weight per day (mg/kg/day), DHA in amounts of from I to 30, preferably from 3 to 15; EPA in amounts of from 1 to 30, preferably from 3 to 15; EPA/DHA Combo (1.5:1 ratio) in amounts of from 4/2 to 30/45, preferably from 9/6 to 18/12; ALA in amounts of from 10 to 100, preferably from 30 to 60; LA in amounts of from 30 to 600, preferably from 60 to 300; ARA in amounts of from 5 to 50, preferably from 15 to 30; SA in amounts of from 3 to 60, preferably from 6 to 30; MA in amounts of from 3 to 60, preferably from 6 to 30; and CLA (as a control) in amounts of from 6 to 120, preferably from 12 to 60. The composition can be administered to the animal in any manner or form suitable for the composition. Preferably, the composition is administered to the animal orally in the form of a food composition or a supplement. The food composition may be of any form, e.g., a nutritionally balanced food composition known in the art such as dry foods, semi-moist foods, and wet foods for animals, particularly companion animals such as feline and canine animals. Supplements include dosage forms such as tablets, capsules, and similar forms. In a further aspect, the composition is administered in combination with one or more drugs or other substances that modulate the amount of adipose tissue on an animal. The drugs or substances include, but are not limited to, substances that suppress appetite, increase metabolism, or interfere with the absorption of specific nutrients, particularly from food. Examples include, but are not limited to, orlistat (blocks fat breakdown and absorption), anorexigenics such as dexedrine (suppresses appetite), anorectics such as fenfluramine and phentermine, and sibutramine, and phenylpropanolamine,

In another aspect, the invention provides a composition suitable for modulating the expression of one or more genes differentially expressed in overweight animals compared to lean animals, particularly fat-associated genes, or modulating the amount of adipose tissue on an animal. The composition may comprise a gene expression or tissue modulating amount of one or more of DHA, EPA, EPA and DHA, ALA, LA, ARA, SA, MA. In some embodiments, the composition comprises, in mg/kg/day, DHA in amounts sufficient to administer to an animal from 1 to 30; EPA in amounts sufficient to administer to an animal from 1 to 30; EPA/DHA Combo (1.5:1 ratio) in amounts sufficient to administer to an animal from 4/2 to 30/45; ALA in amounts sufficient to administer to an animal from 10 to 100; LA in amounts sufficient to administer to an animal from 30 to 600; ARA in amounts sufficient to administer to an animal from 5 to 50; SA in amounts sufficient to administer to an animal from 3 to 60; MA in amounts sufficient to administer to an animal from 3 to 60 and CLA (as a control) in amounts sufficient to administer to an animal from 6 to 120. Such substances are useful for modulating the amount of adipose tissue on an animal. Preferably, the substances affect the expression of a plurality of such genes. In one embodiment, the composition further comprises one or more drugs or other substances that modulate the amount of adipose tissue on an animal.

In another embodiment, the description provides methods for selecting an animal for inclusion in one or more groups or subgroups. The method comprises determining the expression profile of the animal for (a) polynucleotides selected from tables 1 and 2 and preferably from table 3 or useful variations thereof or (b) polypeptides each of which specifically binds to proteins produced by expression of one or more polynucleotides selected from tables I and 2 and preferably from table 3 or useful variations thereof and assigning the animal to a group based upon the expression profile. The groups can be any useful groups, preferably those involved in a research experiment, trial, clinical trial, or other similar category. For example, the groups can be groups involved in a research experiment or clinical trial that requires a one or more control groups and one or more treatment groups. In one embodiment, the control group comprises lean animals and the treatment group comprises overweight animals, or vice versa in another. The expression profile for a plurality of animals can be determined and the animals assigned to the control group or treatment group based upon the results of the profile, i.e., animals with a differential expression of 1.3 fold or more compared to a standard are assigned to the overweight group and animals with a differential expression of 1.3 fold or less compared to a standard are assigned to the lean group. The method is particularly useful for assigning animals to a clinical trial when testing potential drugs or other substances for their ability to reduce the amount of adipose tissue on the animal.

In another aspect, the description provides a computer system suitable for manipulating data relating to one or more genes differentially expressed in overweight animals compared to lean animals, particularly fat-associated genes. The system comprises a database containing information identifying the expression level of one or more polynucleotides selected from tables 1 and 2 and preferably from table 3 or useful variations thereof and/or polypeptides that specifically bind to proteins produced by the expression of one or more polynucleotides selected from tables 1 and 2 and preferably from table 3 or useful variations thereof in lean animals and/or overweight animals and a user interface to interact with the database, particularly to input, manipulate, and review the information for different animals or categories or animals, e.g., lean or overweight animals. In one embodiment, the database further contains information identifying the activity level of one or more polypeptides encoded by one or more polynucleotides selected from tables I and 2 and preferably from table 3 or useful variations thereof. In another, the database further comprises sequence information for one or more of the polynucleotides selected from tables 1 and 2 and preferably from table 3 or useful variations thereof. In other embodiments, the database contains additional information describing the putative description of the genes in one or more animal species. The computer system is any electronic device capable of containing and manipulating the data and interacting with a user, e.g., a typical computer or an analytical instrument designed to facilitate using the present invention and outputting the results relating to the status of an animal.

In another aspect, the description provides a method for using a computer system or the present invention to present information identifying the expression profile of one or more genes differentially expressed in overweight animals compared to lean animals, particularly fat-associated genes. The method comprises comparing the expression level of two or more polynucleotides or proteins expressed from polynucleotides selected from tables 1 and 2 and preferably from table 3 form a sample to the expression profile of the polynucleotides or proteins in the computer system.

In a further aspect, the present description provides kits suitable for determining the differential expression of one or more genes differentially expressed in overweight animals compared to lean animals, particularly fat-associated genes, in a test system. The kits comprise in separate containers in a single package or in separate containers in a virtual package, as appropriate for the use and kit component, two or more probes suitable for detecting the expression of genes differentially expressed in overweight animals compared to lean animals, the probes comprising (a) polynucleotides selected from tables 1 and 2 and preferably from table 3 or useful variations thereof or (b) polypeptides that specifically bind to proteins produced by the expression of one or more polynucleotides selected from tables 1 and 2 and preferably from table 3 or useful variations thereof and at least one of (1) instructions for how to use the probes of the present invention; (2) reagents and equipment necessary to use the probes; (3) a composition suitable for modulating the expression of one or more genes differentially expressed in overweight animals compared to lean animals; (4) a composition suitable for disrupting the expression of one or more genes differentially expressed in overweight animals compared to lean animals; (5) a food composition suitable for modulating the amount of adipose tissue on an animal; and (6) one or more drugs or other substances that that modulate the amount of adipose tissue on an animal. In one preferred embodiment, the probes are bound to a substrate, preferably in an array.

### Compositions of the Invention

The invention encompasses food compositions for canine consumption including 26 wt % to 35 wt. % of crude protein on a dry matter basis, 7.5 wt. % to 8.5 wt. % of crude fat on a dry matter basis, 20 wt. % to 30 wt. % of total dietary fiber on a dry matter basis, and 10 wt. % to 20 wt. % of crude fiber on a dry matter basis.

The invention also encompasses food compositions for feline consumption including 30 wt % to 37 wt. % of crude protein on a dry matter basis, 7.5 wt. % to 9 wt. % of crude fat on a dry matter basis, 30 wt. % to 35 wt. % of total dietary fiber on a dry matter basis, and 20 wt. % to 25 wt. % of crude fiber on a dry matter basis.

In certain embodiments, the compositions of the invention can include an omega-3 polyunsaturated fatty acid content of at least 0.02% (or 0.05 % to 10%, or 0.1% to 6%) by weight on a dry matter basis. In some embodiments, the omega-3 polyunsaturated fatty acid is DHA. In other embodiments, the omega-3 polyunsaturated fatty acid is EPA. In still other embodiments, the omega-3 polyunsaturated fatty acid comprises a mixture of DHA and EPA.

In other embodiments, the composition including the omega-3 polyunsaturated fatty acid is a food. Although both liquid and solid foods are provided, solid foods are typically advantageous. Foods include both dry foods and wet foods. Some of the non-polyunsaturated fatty acid components of the food, and useful proportions, include those listed below.

**Canine Food Compositions**

| **Component** | **Proportion of the composition (% of dry weight of composition or parts per million)** |
|---|---|
| **Protein** | 26 wt. % to 35 wt. % of crude protein on a dry matter basis or 28 wt. % to 33 wt.%, or 30 wt. % to 31 wt. %; or 26 wt. %, 26.5 wt. %, 27 wt. %, 27.5 wt. %, 28 wt. %, 28.5 wt. %, 29 wt. %, 29.5 wt. %, 30 wt. %, 30.5 wt. %, 31 wt. %, 31.5 wt. %, 32 wt. %, 32.5 wt. %, 33 wt. %, 33.5 wt. %, 34 wt. %, 34.5 wt. %, 35 wt. %, |
| **Crude Fat** | 7.5 wt. % to 8.5 wt. % of crude fat on a dry matter basis, or 7.6 wt. %, 7.7 wt. %, 7.8 wt. %, 7.9 wt. %, 8.0 wt. %, 8.1 wt. %, 8.2 wt. %, 8.3 wt. %, 8.4 wt. % |
| **Total** | 20 wt. % to 30 wt. % of total dietary fiber on a dry matter basis |
| **Dietary Fiber** | or 22 wt. % to 28 wt. %, or 24 wt. % to 26 %; or 20 wt. %, 20.5 wt. %, 21 wt. %, 21.5 wt. %, 22 wt. %, 22.5 wt. %, 23 wt. %, 23.5 wt. %, 24 wt. %, 24.5 wt. %, 25 wt. %, 25.5 wt. %, 26 wt. %, 26.5 wt. %, 27 wt. %, 27.5 wt. %, 28 wt. %, 28.5 wt. %, 29 wt. %, 29.5 wt. % |
| **Crude Fiber** | 10 wt. % to 20 wt. % of crude fiber on a dry matter basis, or 12 wt. % to 18 wt. %, or 14 wt. % to 16 %; or 10 wt. %, 10.5 wt. %, 11 wt. %, 11.5 wt. %, 12 wt. %, 12.5 wt. %, 13 wt. %, 13.5 wt. %, 14 wt. %, 14.5 wt. %, 15 wt. %, 15.5 wt. %, 16 wt. %, 16.5 wt. %, 17 wt. %, 17.5 wt. %, 18 wt. %, 18.5 wt. %, 19 wt. %, 19.5 wt. % |

**Feline Food Compositions**

| **Component** | **Proportion of the composition (% of dry weight of composition or parts per million)** |
|---|---|
| **Protein** | 30 wt. % to 37 wt. % of crude protein on a dry matter basis or 31 wt. % to 36 wt. %, or 33% to 35%; or 30 wt. %, 30.5 wt. %, 31 wt. %, 31.5 wt. %, 32 wt. %, 32.5 wt. %, 33 wt. %, 33.5 wt. %, 34 wt. %, 34.5 wt. %, 35 wt. %, 35.5 wt. %, 36 wt. %, 36.5 wt. %, 36 wt. % |
| **Crude Fat** | 7.5 wt. % to 9 wt. % of crude fat on a dry matter basis or 7.6 wt. %, 7.7 wt. %, 7.8 wt. %, 7.9 wt. %, 8.0 wt. %, 8.1 wt. %, 8.2 wt. %, 8.3 wt. %, 8.4 wt. %, 8.5 wt. %, 8.6 wt. %, 8.7 wt. %, 8.8 wt. %, 8.9 wt. %, 9.0 |
| **Total Dietary Fiber** | 30 wt. % to 35 wt. % of total dietary fiber on a dry matter basis or 31 wt. % to 34 wt. %, or 32 wt. % to 33 %; or 30 wt. %, 30.5 wt. %, 31 wt. %, 31.5 wt. %, 32 wt. %, 32.5 wt. %, 33 wt. %, 33.5 wt. %, 34 wt. %, 34.5 wt. %, 35 wt. % |
| **Crude Fiber** | 20 wt. % to 25 wt. % of crude fiber on a dry matter basis, or 21 wt. % to 24 wt. %, or 22 wt. % to 23 %; or 20 wt. %, 20.5 wt. %, 21 wt. %, 21.5 wt. %, 22 wt. %, 22.5 wt. %, 23 wt. %, 23.5 wt. %, 24 wt. %, 24.5 wt. %, 25 wt. % |

In one embodiment, the canine compositions of this invention include ingredients in an amount effective to enhance the animal's quality of life. Such compositions generally comprise:
(a) 26 wt. % to 35 wt. % of crude protein on a dry matter basis,
(b) 7.5 wt. % to 8.5 wt. % of crude fat on a dry matter basis,
(c) 20 wt. % to 30 wt. % of total dietary fiber on a dry matter basis, and
(d) 10 wt. % to 20 wt. % of crude fiber on a dry matter basis.

In another embodiment, the invention encompasses feline compositions that generally comprise:
(a) 30 wt. % to 37 wt. % of crude protein on a dry matter basis,
(b) 7.5 wt. % to 9 wt. % of crude fat on a dry matter basis,
(c) 30 wt. % to 35 wt. % of total dietary fiber on a dry matter basis, and
(d) 20 wt. % to 25 wt. % of crude fiber on a dry matter basis.

In another embodiment, the compositions generally comprise:
(a) at least one of the following:
   (i) at least 0.05% (or 0.05% to 0.30%, or 0.1% to 0.30%, or 0.1% to 0.2%) DHA, and
   (ii) at least 0.1% (or 0.1% to 0.5%, or 0.2% to 0.5%, or 0.2% to 0.3%) EPA,
(b) at least 15% (or 15% to 55%, or 30% to 55%, or 33% to 36%) protein,
(c) at least 9% (or 9% to 35%, or 18% to 35%, or 18% to 24%) fat, and
(d) at least one of the following:
   (i) at least 250 IU/kg (or 250 IU/kg to 1500 IU/kg, or 500 IU/kg to 1500 IU/kg, or 500 IU/kg to 1100 IU/kg) vitamin E ,
   (xii) at least 50 ppm (or 50 ppm to 300 ppm, or 100 ppm to 300 ppm, or 100 ppm to 200 ppm) vitamin C,
   (xiii)at least 1100 ppm (or 1100 ppm to 3500 ppm, or 2300 ppm to 3500 ppm, or 2300 ppm to 2350 ppm) taurine, and
   (xiv)at least 200 ppm (or 200 to 750 ppm, or 400 ppm to 750 ppm, or 400 to 525 ppm) carnitine, and
   (xv) at least 0.05% (or 0.05% to 0.6%, or 0.1 % to 0.6%, or 0.1% to 0.4%) cystine.

In another embodiment, the compositions generally comprise:
(a) 0.02% (or 0.05 % to 10%, or 0.1% to 6%) at least one omega-3 polyunsaturated fatty acid, and
(b) at least one of the following:
   (xvi)10% to 55% (or 18% to 30%, or 33% to 55% or 18% to 20% or 33% to 36%) protein,
   (xvii) 7% to 35% (or 18% to 35%, or 7% to 24%, or 14% to 24%, or 14% to 16% or 18% to 24%) fat,
   (xviii) at least .05 (or 0.05 ppm to 7500 ppm, or 250 to 3600, or 250 ppm to 1650 ppm, or 5 ppm to 225 ppm, or 0.05 ppm to 2.4 ppm) antioxidant, and
   (xix)at least 1000 ppm (or 1000 ppm to 5000 ppm, 3300 ppm to 5000 ppm, or 2000 ppm to 3000 ppm, or 3000 ppm to 4000 ppm) choline.

In another embodiment, the compositions generally comprise:
(a) at least one of the following:
   (i) at least 0.02% (or 0.02% to 0.3%, or 0.05% to 0.3%, or 0.05% to 0.2%) DHA, and (ii) at least 0.1% (or 0.1% to 0.5%, or 0.2% to 0.5%, or 0.2% to 0.3%) EPA,
(b) at least 9% (or 9% to 30%, or 18% to 30%, or 18% to 20%) protein,
(c) at least 7% (or 7% to 24%, or 14% to 24%, or 14% to 16%) fat,
(d) at least one of the following:
   (i) at least 250 IU/kg (or 250 IU/kg to 1500 IU/kg, or 500 IU/kg to 1500 IU/kg, or 500 IU/kg to 1000 IU/kg) vitamin E ,
   (xx) at least 50 ppm (or 50 ppm to 500 ppm, or 100 ppm to 500 ppm, or 100 ppm to 301 ppm) vitamin C,
   (xxi)at least 600 ppm (or 600 ppm to 2400 ppm, or 1260 ppm to 2400 ppm, or 1260 ppm to 1545 ppm) taurine, and
   (xxii) at least 50 ppm (or 50 ppm to 200 ppm, or 100 to 160, or 100 to 155) lipoic acid, and
   (xxiii) at least 50 ppm (or 50 ppm to 500 ppm, or 200 ppm to 500 ppm, or 200 ppm to 350 ppm) carnitine,
(e) at least 1000 ppm (or 1000 ppm to 3200 ppm, or 2000 ppm to 3200 ppm, or 2000 ppm to 2500 ppm) choline,
(f) at least 50 ppm (or 50 ppm to 150 ppm, or 100 ppm to 150 ppm, or 100 ppm to 110 ppm) manganese, and
(g) at least 0.4% (or 0.4% to 2%, or 0.9% to 2%, or 0.9% to 1.2%) lysine, and
(h) at least 0.4% to 1.5% methionine.

In another embodiment, the compositions generally comprise:
(a) at least one of the following:
   (i) at least 0.02% (or 0.02% to 0.3%, or 0.05% to 0.3%, or 0.05% to 0.2%) DHA, and
   (ii) at least 0.1% (or 0.1% to 0.5%, or 0.2% to 0.5%, or 0.2% to 0.3%) EPA,
(b) at least 9% (or 9% to 30%, or 18% to 30%, or 18% to 20%) protein,
(c) at least 7% (or 7% to 24%, or 14% to 24%, or 14% to 16%) fat,
(d) at least one of the following:
   (i) at least 250 IU/kg (or 250 IU/kg to 1500 IU/kg, or 500 IU/kg to 1500 IU/kg, or 500 IU/kg to 1000 IU/kg) vitamin E ,
   (xxiv) at least 50 ppm (or 50 ppm to 500 ppm, or 100 ppm to 500 ppm, or 100 ppm to 301 ppm) vitamin C,
   (xxv) at least 600 ppm (or 600 ppm to 2400 ppm, or 1260 ppm to 2400 ppm, or 1260 ppm to 1575 ppm) taurine, and
   (xxvi) at least 50 ppm (or 50 ppm to 200 ppm, or 100 to 160, or 100 to 155) lipoic acid, and
   (xxvii) at least 50 ppm (or 50 ppm to 500 ppm, or 200 ppm to 500 ppm, or 200 ppm to 350 ppm) carnitine,
(e) at least 1000 ppm (or 1000 ppm to 3200 ppm, or 2000 ppm to 3200 ppm, or 2000 ppm to 2500 ppm) choline,
(f) at least 50 ppm (or 50 ppm to 150 ppm, or 100 ppm to 150 ppm, or 100 ppm to 110 ppm) manganese, and
(g) at least 0.4% (or 0.4% to 2%, or 0.9% to 2%, or 0.9% to 1.2%) lysine, and
(h) at least 0.4% to 1.5% methionine.

In another embodiment, the compositions generally comprise:
(a) at least one of the following:
   (i) at least 0.05% (or 0.05% to 0.30%, or 0.1% to 0.30%, or 0.1% to 0.2%) DHA, and
   (ii) at least 0.1% (or 0.1% to 0.5%, or 0.2% to 0.5%, or 0.2% to 0.3%) EPA,
(b) at least 15% (or 15% to 55%, or 30% to 55%, or 33% to 36%) protein,
(c) at least 9% (or 9% to 35%, or 1.8% to 35%, or 18% to 24%) fat,
(d) at least one of the following:
   (i) at least 250 IU/kg (or 250 IU/kg to 1500 IU/kg, or 500 IU/kg to 1500 IU/kg, or 500 IU/kg to 1100 IU/kg) vitamin E ,
   (xxviii) at least 50 ppm (or 50 ppm to 300 ppm, or 100 ppm to 300 ppm, or 100 ppm to 200 ppm) vitamin C,
   (xxix) at least 1100 ppm (or 1100 ppm to 3500 ppm, or 2300 ppm to 3500 ppm, or 2300 ppm to 2350 ppm) taurine, and
   (xxx) at least 200 ppm (or 200 to 750 ppm, or 400 ppm to 750 ppm, or 400 to 525 ppm) carnitine, and
   (xxxi) at least 0.05% (or 0.05% to 0.6%, or 0.1% to 0.6%, or 0.1% to 0.4%) cystine,
(e) at least 1600 ppm (or 1600 ppm to 5000 ppm, or 3300 ppm to 5000 ppm, or 3300 ppm to 3400 ppm) choline,
(f) at least 50 ppm (or 50 ppm to 150 ppm, or 100 ppm to 150 ppm, or 100 ppm to 110 ppm) manganese, and
(g) at least 0.7% (or 0.7% to 3%, or 1.4% to 3%, or 1.4% to 1.7%) lysine, and
(h) at least 0.4% to 1.5% methionine.

### Methods Of Treating Or Preventing Diseases And Disorders

The invention encompasses compositions for use in treating or preventing diseases and disorders, wherein the use includes the administration of the compositions of the invention that are effective in modulating the expression and/or activity of proteins associated with obesity in animals both *in vitro* and *in vivo.* The inventors have surprisingly found that the compositions of the invention are effective in modulating proteins associated with obesity in animals. Without being limited by theory, it is believed that modulation of protein expression and/or activity associated with obesity in animals is useful in treating or preventing a disorder associated with abnormal blood glucose levels, weight gain, or fat depot levels. The invention further encompasses compositions and formulations that are useful in modulating protein activity in overweight and/or obese animals. The invention also encompasses compositions for use in modulating protein or gene activity, wherein the use includes administering to a subject, preferably to a companion mammal in need of said treatment or prevention a therapeutically or prophylactically effective amount of a composition to modulate the activity of the protein or gene associate with obesity in the subject. In an illustrative embodiment, the agent for modulating lyn kinase activity is a compound of the invention.

In one embodiment, a composition for use according to the invention is administered to a mammal, preferably a companion animal, with a cardiovascular disease, a dyslipidemia, a dyslipoproteinemia, a disorder of glucose metabolism, metabolic syndrome (*i.e.,* Syndrome X), a PPAR-associated disorder, septicemia, a thrombotic disorder, type II diabetes, obesity, pancreatitis, hypertension, a renal disease, or inflammation.

In one embodiment, "treatment" or "treating" refers to an amelioration of a disease or disorder, or at least one discernible symptom thereof, preferably associated with obesity. In another embodiment, "treatment" or "treating" refers to an amelioration of at least one measurable physical parameter, not necessarily discernible by the animal. In yet another embodiment, "treatment" or "treating" refers to inhibiting the progression of a disease or disorder, either physically, e.g., stabilization of a discernible symptom, physiologically, e.g., stabilization of a physical parameter, or both. In yet another embodiment, "treatment" or "treating" refers to delaying the onset of a disease or disorder.

In certain embodiments, the compositions of the invention are administered to a patient, preferably a companion animal, as a preventative measure against such diseases. As used herein, "prevention" or "preventing" refers to a reduction of the risk of acquiring a given disease or disorder. In a preferred mode of the embodiment, the compositions of the present invention are administered as a preventative measure to a patient, preferably a companion animal having a genetic predisposition to a cardiovascular disease, a dyslipidemia, a dyslipoproteinemia, a disorder of glucose metabolism, metabolic syndrome *(i.e.,* Syndrome X), a PPAR-associated disorder, septicemia, a thrombotic disorder, type II diabetes, obesity, pancreatitis, hypertension, a renal disease, or inflammation.

In another illustrative mode of the embodiment, the composition for use according to the invention are administered as a preventative measure to a companion animal having a predisposition to a cardiovascular disease, a dyslipidemia, a dyslipoproteinemia, a disorder of glucose metabolism, metabolic syndrome *(i.e.,* Syndrome X), a PPAR-associated disorder, septicemia, a thrombotic disorder, type II diabetes, obesity, pancreatitis, hypertension, a renal disease, or inflammation. Accordingly, the compositions of the invention may be used for the prevention of one disease or disorder and concurrently treating another (*e.g.,* prevention of obesity while treating diabetes; prevention of inflammation while treating a cardiovascular disease).

### Cardiovascular Diseases for Treatment or Prevention

The invention provides compositions for use in the treatment or prevention of a cardiovascular disease, wherein the use comprises administering to a companion animal a therapeutically effective amount of a composition of the invention and a pharmaceutically acceptable vehicle. In some embodiments, the cardiovascular disease is associated with abnormal/altered protein expression. As used herein, the term "cardiovascular diseases" refers to diseases of the heart and circulatory system. These diseases are often associated with dyslipoproteinemias and/or dyslipidemias. Cardiovascular diseases, which the compositions of the invention are useful for preventing or treating include, but are not limited to, arteriosclerosis; atherosclerosis; stroke; ischemia; endothelium dysfunctions, in particular those dysfunctions affecting blood vessel elasticity; peripheral vascular disease; coronary heart disease; myocardial infarction; cerebral infarction and restenosis.

### Dyslipidemias for Treatment or Prevention

The invention provides compositions for use in the treatment or prevention of a dyslipidemia, wherein the use comprises administering to a companion animal a therapeutically effective amount of a composition of the invention and a pharmaceutically acceptable vehicle. In some embodiments, the dyslipidemia is associated with abnormal/altered lyn kinase activity and/or expression. As used herein, the term "dyslipidemias" refers to disorders that lead to or are manifested by aberrant levels of circulating lipids. To the extent that levels of lipids in the blood are too high, the compositions of the invention are administered to a companion animal to restore normal levels. Normal levels of lipids are reported in medical treatises known to those of skill in the art. For example, recommended blood levels of LDL, HDL, free triglycerides and others parameters relating to lipid metabolism can be found at the web site of the American Heart Association and that of the National Cholesterol Education Program of the National Heart, Lung and Blood Institute. At the present time, the recommended level of HDL cholesterol in the blood is above 35 mg/dL; the recommended level of LDL cholesterol in the blood is below 130 mg/dL; the recommended LDL:HDL cholesterol ratio in the blood is below 5:1, ideally 3.5:1; and the recommended level of free triglycerides in the blood is less than 200 mg/dL.

Dyslipidemias which the compositions of the invention are useful for preventing or treating include but are not limited to hyperlipidemia and low blood levels of high density lipoprotein (HDL) cholesterol. In certain embodiments, the hyperlipidemia for prevention or treatment by the compounds of the present invention is familial hypercholesterolemia; familial combined hyperlipidemia; reduced or deficient lipoprotein lipase levels or activity, including reductions or deficiencies resulting from lipoprotein lipase mutations; hypertriglyceridemia; hypercholesterolemia; high blood levels of ketone bodies (*e.g.,* β-OH butyric acid); high blood levels of Lp(a) cholesterol; high blood levels of low density lipoprotein (LDL) cholesterol; high blood levels of very low density lipoprotein (VLDL) cholesterol and high blood levels of non-esterified fatty acids.

The present invention further provides compositions for use in altering lipid metabolism in a patient, for example, reducing LDL in the blood of a companion animal, reducing free triglycerides in the blood of a patient, increasing the ratio of HDL to LDL in the blood of a patient, and inhibiting saponified and/or non-saponified fatty acid synthesis, said methods comprising administering to the patient a composition comprising a compound of the invention in an amount effective alter lipid metabolism.

### Dyslipoproteinemias for Treatment or Prevention

The invention provides compositions for use in the treatment or prevention of a dyslipoproteinemia, wherein the use comprises administering to a companion animal a therapeutically effective amount of a composition of the invention and a pharmaceutically acceptable vehicle. As used herein, the term "dyslipoproteinemias" refers to disorders that lead to or are manifested by aberrant levels of circulating lipoproteins. To the extent that levels of lipoproteins in the blood are too high, the compositions of the invention are administered to a patient to restore normal levels. Conversely, to the extent that levels of lipoproteins in the blood are too low, the compositions of the invention are administered to a patient to restore normal levels. Normal levels of lipoproteins are reported in medical treatises known to those of skill in the art.

Dyslipoproteinemias, which the compositions of the present invention are useful for preventing or treating include, but are not limited to, high blood levels of LDL; high blood levels of apolipoprotein B (apo B); high blood levels of Lp(a); high blood levels of apo(a); high blood levels of VLDL; low blood levels of HDL; reduced or deficient lipoprotein lipase levels or activity, including reductions or deficiencies resulting from lipoprotein lipase mutations; hypoalphalipoproteinemia; lipoprotein abnormalities associated with diabetes; lipoprotein abnormalities associated with type II diabetes, obesity; lipoprotein abnormalities associated with Alzheimer's Disease; and familial combined hyperlipidemia.

### Glucose Metabolism Disorders for Treatment or Prevention

The invention provides compositions for use in the treatment or prevention of a glucose metabolism disorder, wherein the use comprises administering to a companion animal a pharmaceutically acceptable vehicle. As used herein, the term "glucose metabolism disorders" refers to disorders that lead to or are manifested by aberrant glucose storage and/or utilization. To the extent that indicia of glucose metabolism (*i.e.*, blood insulin, blood glucose) are too high, the compositions of the invention are administered to a patient to restore normal levels. Conversely, to the extent that indicia of glucose metabolism are too low, the compositions of the invention are administered to a patient to restore normal levels. Normal indicia of glucose metabolism are reported in medical treatises known to those of skill in the art. In some embodiments, the glucose metabolism disorder is associated with abnormal/altered lyn kinase activity and/or expression.

Glucose metabolism disorders which the compositions of the present invention are useful for preventing or treating include but are not limited to impaired glucose tolerance; insulin resistance; insulin resistance related breast, colon or prostate cancer; diabetes, including but not limited to non-insulin dependent diabetes mellitus (NIDDM), insulin dependent diabetes mellitus (IDDM), gestational diabetes mellitus (GDM), and maturity onset diabetes of the young (MODY); pancreatitis; hypertension; polycystic ovarian disease; and high levels of blood insulin and/or glucose.

The invention further provides compositions for use in altering glucose metabolism in a patient, for example to increase insulin sensitivity and/or oxygen consumption of a companion animal, said use comprising administering to the companion animal a composition comprising a compound of the invention in an amount effective to alter glucose metabolism.

### Treatment or Prevention of Metabolic Syndrome

As used herein, "treatment or prevention of Syndrome X or Metabolic Syndrome" encompasses treatment or prevention of a symptom associated with metabolic syndrome including, but not limited to, impaired glucose tolerance, hypertension and dyslipidemia and/or dyslipoproteinemia. In some embodiments, the metabolic syndrome is associated with abnormal/altered lyn kinase activity and/or expression

Metabolic syndrome is characterized by a group of metabolic risk factors in a person. Risk factors that are associated with metabolic syndrome that can be treated or prevented by administering a composition comprising a compound of the invention include, but are not limited to, central obesity *(i.e.,* excessive fat tissue in and around the abdomen); atherogenic dyslipidemia (blood fat disorders - mainly high triglycerides and low HDL cholesterol - that foster plaque buildups in artery walls); raised blood pressure (130/85 mmHg or higher); insulin resistance or glucose intolerance (the body can't properly use insulin or blood sugar); prothrombotic state (*e.g.*, high fibrinogen or plasminogen activator inhibitor [-1] in the blood); and a proinflammatory state (*e.g.,* elevated high-sensitivity C-reactive protein in the blood).

The underlying causes of this syndrome are overweight/obesity, physical inactivity and genetic factors, companion animal with metabolic syndrome are at increased risk of coronary heart disease, other diseases related to plaque buildups in artery walls (*e.g.*, stroke and peripheral vascular disease) and type 2 diabetes.

Metabolic syndrome is closely associated with a generalized metabolic disorder called insulin resistance, in which the body can't use insulin efficiently. This is why the metabolic syndrome is also called the insulin resistance syndrome.

Some companion animal are genetically predisposed to insulin resistance. Acquired factors, such as excess body fat and physical inactivity, can elicit insulin resistance and the metabolic syndrome in these people. Most companion animal with insulin resistance have central obesity. The biologic mechanisms at the molecular level between insulin resistance and metabolic risk factors aren't fully understood and appear to be complex.

The compositions of the invention are therefore useful in treating or preventing metabolic syndrome and disorders and risk factors associated with metabolic syndrome.

### Treatment or Prevention Type II Diabetes

As used herein, "treatment or prevention of type II diabetes" encompasses treatment or prevention of a complication associated with type II diabetes including, but not limited to, retinopathy (*i.e.,* blindness); neuropathy (*i.e*., nerve damage) which leads to foot ulcers, gangrene, and amputations; kidney damage, which leads to dialysis; and cardiovascular disease. In some embodiments, the type II diabetes is associated with abnormal/altered lyn kinase activity and/or expression.

Type II diabetes is associated with obesity and with aging. It is a lifestyle-dependent disease, and has a strong genetic component (concordance in twins is 80-90%). The problem seems not so much in insulin production, but that when the insulin reaches its target cells, it doesn't work correctly. Most Type II diabetes patients initially have high insulin levels along with high blood sugar. However, since sugar signals the pancreas to release insulin, Type II diabetics eventually become resistant to that signal and the endocrine-pancreas soon will not make enough insulin. These companion animals end up managing the disease with insulin and they need much higher doses because they are resistant to it.

When a companion animal takes in a high load of sugar, the sugar stimulates the pancreas to release insulin. The targets for insulin are muscle, fat, and liver cells. These cells have insulin receptor sites on the outside of the cell membrane. For most companion animals, when insulin has bound to the receptors, a cascade of events begins, which leads to sugar being transported from the blood into the interior of the cell. In Type II diabetics, even when insulin is present on the cell membrane, the process doesn't work. The glucose is never taken up into the cell and remains in the bloodstream.

The liver is responsible for glucose production and insulin is the regulatory agent of production. A high blood sugar content causes the pancreas to release insulin, and the insulin should signal the liver to stop making sugars. But, in diabetics, there's resistance to that signal and the liver keeps producing glucose. Hyperglycemia leads to glucose toxicity.

It is not high blood sugar that is the disease process of diabetes, but complications from the high blood sugar. A major problem faced by doctors is that some people with high blood sugar feel fine; it's hard to treat diseases that are asymptomatic since most people don't want to take a pill for something that they don't feel bad about. The compositions comprising a compound of the invention are therefore useful in treating or preventing type II diabetes or complications arising from type II diabetes and disorders and risk factors associated with metabolic syndrome. Complications of diabetes include, but are not limited to, diabetic neuropathy, diabetic retinopathy, erectile dysfunction, and kidney disease and the compounds of the invention are useful in treating or preventing these complications.

### Treatment or Prevention of Obesity

As used herein, "treatment or prevention of obesity" encompasses treatment or prevention of a complication associated with obesity. Complications of obesity include, but are not limited to, hypercholesterolemia, hypertension, dyslipidemia (for example, high total cholesterol or high levels of triglycerides), type 2 diabetes, coronary heart disease, stroke, gallbladder disease, osteoarthritis, sleep apnea and respiratory problems, and some cancers (endometrial, breast, and colon). In some embodiments, the obesity is associated with abnormal/altered lyn kinase activity and/or expression

### Other Diseases for Treatment or Prevention

The present description provides methods for the treatment or prevention of septicemia, thrombotic disorders, pancreatitis, hypertension, inflammation, and impotence, comprising administering to a patient a therapeutically effective amount of a composition comprising a compound of the invention and a pharmaceutically acceptable vehicle. In some aspects, these disorders are associated with abnormal/altered lyn kinase activity and/or expression

As used herein, "treatment or prevention of septicemia" encompasses treatment or prevention of septic shock.

As used herein, "treatment or prevention of thrombotic disorders" encompasses treatment or prevention of high blood levels of fibrinogen and promotion of fibrinolysis.

In addition to treating or preventing obesity, the compositions of the invention can be administered to an individual to promote weight reduction of the individual.

### Kits of the Invention

When the kit comprises a virtual package, the kit is limited to instructions in a virtual environment in combination with one or more physical kit components. In one aspect, the kit contains probes and/or other physical components and the instructions for using the probes and other components are available via the internet. The kit may contain additional items such as a device for mixing samples, probes, and reagents and device for using the kit, e.g., test tubes or mixing utensils.

In another aspect, the present description provides a means for communicating information or instructions for one or more of (1) using the polynucleotides of the present description for detecting the expression of genes differentially expressed in overweight animals compared to lean animals in a sample, (2) using the polynucleotides of the present description for measuring the effect of a test substance on the expression of one or more genes differentially expressed in overweight animals compared to lean animals, (3) using the polynucleotides of the present description for screening a test substance to determine if it is likely to be useful for modulating the amount of adipose tissue on an animal, (4) using the polynucleotides of the present description for formulating a prognosis that an animal is likely to become overweight or developing a diagnosis that an animal is fat, (5) using the polynucleotides of the present description for manipulating the genome of a non-human animal or the expression of the genome of an animal, (6) using the polynucleotides of the present description for modulating the expression of one or more genes differentially expressed in overweight animals compared to lean animals, particularly fat-associated genes, or modulating the amount of adipose tissue on an animal, (7) using the polynucleotides of the present description for selecting an animal for inclusion in one or more groups, (8) using the polynucleotides of the present description for using computer system to manipulate data relating to genes differentially expressed in overweight animals compared to lean animals, particularly fat-associated genes, (9) administering substances of the present description to an animal, alone or in combination with the other elements of the present description (10) using the substances of the present description for modulating the amount of adipose tissue on an animal, (11) using the computer system of the present description (12) using the kits of the present description, and (13) instructions for using the methods and compositions as defined herein with one or more drugs or other substances that that modulate the amount of adipose tissue on an animal. The means comprises a document, digital storage media, optical storage media, audio presentation, or visual display containing the information or instructions. In certain embodiments, the communication means is a displayed web site, visual display, kiosk, brochure, product label, package insert, advertisement, handout, public announcement, audiotape, videotape, DVD, CD-ROM, computer readable chip, computer readable card, computer readable disk, computer memory, or combination thereof containing such information or instructions. Useful information includes one or more of (1) methods for promoting the health and wellness of animals and (2) contact information for the animal's caregivers to use if they have a question the invention and its use. Useful instructions include techniques for using the probes, instructions for performing a gene expression assay, and administration amounts and frequency for the substances. The communication means is useful for instructing on the benefits of using the methods and compositions defined herein.

### EXAMPLES

### Materials and Methods

### Isolation of Ribonucleic Acid (RNA) from Tissue

Tissue samples that have been collected, frozen in liquid nitrogen, and thawed are homogenized and processed using a TRIzol® RNA extraction method to produce good quality RNA which is then subjected to further genomic analysis.

Materials: ice, liquid nitrogen, frozen canine or feline tissue, TRIzol® lysis reagent, chloroform minimum 99%, isopropyl alcohol, 70% ethanol (prepared with ethanol, absolute and deionized, RNase-free water), RNase Zap®, deionized water, RNA Storage Solution®, from Ambion.

Equipment: Ultra-Turrax T25 Power Homogenizer, Beckman Coulter Allegra 25R Centrifuge, Eppendorf Centrifuge, forceps, scalpel, hard cutting surface, i.e. cutting board, 1.5mL DNase and RNase free/sterile microcentrifuge tubes, 50mL DNase and RNase free/sterile disposable polypropylene tubes, P1000, P200, P20, P10 and P2 Rainin Pipetman pipettes, filter pipette tips for P1000, P200, P20, P10 and P2 pipettes, DNase and RNase free/sterile, and lint free wipes.

Preparations: Prepare 50mL polypropylene tubes with 4mL TRIzol® (one tube for each tissue selected for RNA isolation).

Tissue Homogenization: Fill a container capable of holding liquid nitrogen with 3-4 scoops of liquid nitrogen. Place a piece of frozen tissue immediately into the aforementioned container (the tissue should be the size of a pea) and place the tissue into the appropriate labeled 50mL polypropylene tube (that already contains 4mL TRIzol®). Immediately begin homogenization using the Ultra-Turrax T25 Power Homogenizer. Homogenize on the highest setting (6) for 10-15 seconds. Cool the sample on ice for another 10-15 seconds and then repeat. Continue until the tissue is fully homogenized and the solution is cloudy. Upon complete homogenization, cap the 50mL tube and return to the ice. Incubate the homogenized tissues at room temperature for 5 minutes before proceeding with the isolation procedure.

RNA Isolation: The procedures given in the Invitrogen instructions provided with the TRIzol® reagent are generally followed. Separate the homogenized sample into four 1mL aliquots in four 1.5L microcentrifuge tubes. Add 200uL of chloroform to each 1mL aliquot. Cap the tubes, vortex for 15 seconds and then shake up and down. The result should be a pink milky liquid. Incubate the tubes at room temperature for 2-3 minutes. Centrifuge the tubes for 15 minutes at 14,000 rpm and 4°C. Transfer the aqueous phase (top layer) to a sterile 1.5mL microcentrifuge tube. The typical volume of the aqueous phase which should be transferred to the new tube is 500uL. Be sure not to transfer any of the intermediate or lower phase. Precipitate the RNA from solution by adding 500uL of Isopropyl Alcohol to each microcentrifuge tube containing the aqueous layer. Shake the tubes up and down for at least 20 seconds. Incubate the samples at room temperature for 10 minutes. Centrifuge the samples for 10 minutes, 14,000 rpm at 4°C. Remove the supernatant carefully by aspirating off the liquid being sure not to lose the pellet. Add 1mL of 70% ethanol to wash the pellet. Dislodge the pellet by flicking the tube (or tapping the tube on the bench top) and shake to mix. Centrifuge for 5 minutes, 8,200 rpm at 4°C. Remove the supernatant carefully by aspirating off the liquid being sure not to lose the pellet. Using a lint free wipe carefully soak up excess ethanol to make sure the pellet is dry. Resuspend each pellet into 30uL of RNA Storage Solution. Mix gently by pipetting until the RNA goes back into solution and then store at -80°C. It may be necessary to vortex the sample for a few seconds at a low speed to facilitate the resuspension of the RNA. If this is necessary, spin down the samples, using the microcentrifuge, prior to freezing.

RNA Cleaning: The procedures given in the RNeasy® Mini Handbook are followed.

### RNA Isolation from Cells Cultured in OptiCell Chambers Using the RNeasy Mini Kit.

Cells cultured from mammalian cell lines are used to isolate good quality RNA which is then used for future downstream genomic analysis. All work related to the culturing of the cells is to be done under strict aseptic conditions.

Reagents: 10X PBS, deionized H₂O, absolute ethanol, RNA Storage Solution, β-Mercaptoethanol, RNase Zap®, Buffer RLT, and Buffer RW1 and Buffer RPE (provided in the RNeasy Mini Kit)

Equipment/Materials: RNeasy Mini Kit, QIAshredder spin columns, OptiCell knife, 20mL sterile syringe, OptiCell tips, Cell scraper, P1000 Pipetman pipette, Rainin, P200 Pipetman pipette, Rainin, 100-100uL filtered pipette tips, 1-200uL filtered pipette tips, sterile transfer pipettes, 55mL sterile solution basin, 1.5mL sterile microcentrifuge tubes, and Eppendorf Microcentrifuge.

Solutions: Buffer RLT (stock provided in RNeasy Mini Kit); -Add 100uL of β-Mercaptoethanol per 10mL of Buffer RLT prior to beginning protocol. 70% Ethanol: Make 50mL of 70% ethanol by adding 35mL absolute ethanol to 15mL deionized, RNase-free water. 1X PBS: RNase-free water. Filter the solution using a .22um filter.

Procedure: Removing Cells from the OptiCell Chamber (proceed one OptiCell at a time). Check the cells under a microscope to ensure that the cells are alive before isolating RNA. Remove and discard the cell culture medium. Using the OptiCell knife cut away the top membrane exposing the cells on the lower membrane. Wash the membrane to which the cells are attached three times with 1X PBS. Pipette 600uL of the Buffer RLT solution (containing β-Mercaptoethanol) onto the center of the membrane to which the cells are attached. Using the cell scraper, gently spread the Buffer RLT over the entire surface of the membrane, and then collect the liquid in one corner. Pipette off the entire volume of Buffer RLT and place into a QIAshredder spin column.

RNA Isolation: Centrifuge the QIAshredder spin columns at 14,000 rpm for 2 minutes. Discard the spin column but keep the collection tube and its contents. Add 600uL of 70% ethanol to the collection tube and mix well by pipetting (the total volume now = 1.2mL). Transfer 600uL of the cell lysate to an RNeasy mini column and centrifuge for 15 seconds at 14,000 rpm. Discard the flow through but keep the collection tube and the spin column. Transfer the remaining volume of cell lysate (~600uL) to the spin column and repeat the centrifugation. Discard the flow through but keep the collection tube and the spin column. Add 700uL Buffer RW1 to the spin column. Centrifuge for 15 seconds at 14,000 rpm to wash the column. Discard the flow through and the collection tube. Transfer the spin column to a new 2mL collection tube and add 500uL Buffer RPE to the column. Centrifuge for 15 seconds at 14,000 rpm. Discard the flow through, keep the collection tube/column. Add another 500uL Buffer RPE to the column. Centrifuge for 2 minutes at 14,000 rpm. Transfer the spin column to a 1.5L collection tube. Add 30uL of RNA Storage Solution directly to the silica gel membrane and centrifuge for 1 minute at 14,000 rpm to elute the RNA. Store the final RNA at -70°C.

### RNA 6000 Nano Assay

Using the Agilent 2100 Bioanalyzer and the RNA 6000 Nano Assay, analyze RNA isolated from cultured mammalian cells, lymphocytes or tissues for quality.

Reagents: RNA 6000 Nano gel matrix, RNA 6000 Nano dye concentrate, RNA 6000 Nano Marker, (all of the above reagents are contained in the RNA 6000 Nano Assay kit, Agilent), RNA 6000 ladder, RNase Zap , and RNase-free water, from Ambion.

Equipment/Other Materials: Agilent Chip Priming Station, Agilent, RNA 6000 chip, Agilent, electrode cleaners, P2, P10, P200, and P1000 Rainin Pipetman pipettes, sterile, DNase/RNase free filtered pipette tips, 1.5mL microcentrifuge tubes, sterile, vortex, IKA vortex mixer, microcentrifuge, and heating block.

Procedure: The procedure is given in the Reagent Kit Guide, RNA 6000 Nano Assay, Edition November 2003, by Agilent Technologies. The procedures are followed as given in the Guide, with the following modifications: Preparing the Gel, pg. 17- rather than separating the filtered gel into aliquots of 65uL each, keep the stock filtered gel in the original microcentrifuge tube and aliquot the 65uL as needed. Loading the RNA 6000 Nano Marker, pg. 22- add 1uL of RNase-free water (instead of RNA 6000 Nano Marker) to each sample well that will not contain sample. Not only will this conserve the amount of Marker used but also serves as a negative control to see that none of the reagents are contaminated, including the RNase-free water. Loading the Ladder and Samples, pg. 23- heat denature the samples and RNA 6000 Ladder for an additional 30 seconds (total of 2.5 minutes) at 71°C. Starting the Chip Run, pg. 26- choose the "Eukaryote Total RNA Nano" option from the assay menu.

### Affymetrix Genechip Expression Analysis

Gene expression is analyzed using Affymetrix Canine 1 and Canine 2 GeneChip® Arrays are available commercially from Affymetrix, Inc., Santa Clara, CA 95051. Total RNA is reverse transcribed into cDNA. The cDNA is used to generate cRNA which is fragmented and used as probes for GeneChip hybridization. The gene chip is washed and the hybridization signal is measured with an Affymetrix laser scanner. The hybridization data is then validated and normalized for further analysis.

Materials: Affymetrix provides most of the reagents and kit. Other reagents listed in the Affymetrix Manual but not supplied in the kit may be obtained separately (refer to GeneChip Expression Analysis Technical Manual (701021 Rev.4) for details), RNase Zap® and deionized water.

Equipment: Eppendorf microcentrifuge, 1.5mL DNase and RNase free/sterile microcentrifuge tubes, 50mL DNase and RNase free/sterile disposable polypropylene tubes, P1000, P200, P20, P10 and P2 Rainin Pipetman pipettes, Filter pipette tips for P1000, P200, P20, P10 and P2 pipettes, DNase and RNase free/sterile, and Peltier Thermal Cycler PTC-200.

Procedure: follow all procedures exactly as described in GeneChip Expression Analysis Technical Manual (Affymetrix Copyright 1999-2003). Use 5 microgram of total RNA for the first strand cDNA synthesis. Use either Peltier Thermal Cycler PTC-200 or heat block for temperature control on reactions and probe denaturing. The quality control is performed using RNA NanoDrop chips with BioAnalyer 2100. Use 100 Format (Midi Array) for the canine genechip.

### Example 1

### Determining Differential Gene Expression between Adipose Tissue Samples from Overweight and Lean Animals

Adipose tissue samples are obtained from 18 (3 lean and 15 fat) or lymphocytes obtained from 44 (12 lean and 32 fat) canine animals diagnosed as either "fat" or "lean" using conventional methods. The "fatness" or "leanness" of an animal is determined based on measurements by DEXA using conventional methods or based on a 5 point body condition scoring system. For example, an animal is considered lean if it has a body condition score of 2 or 2.5 and/or a DEXA total body fat percentage of 27% or less. An animal is considered to be overweight if it has a body condition score of 4 or higher and a total body fat percentage of 30% or higher. All solid tissue samples were snap frozen in liquid nitrogen immediately after removal from the animal. The lymphocytes were isolated using BD Vacutainer® CPT™ Cell Preparation Tube according to manufactuerers instructions and were also snap frozen until needed.
The samples were all analyzed using Affymetrix Canine-2 GeneChipe^{®} according to manufacturers recommendations in order to determine which genes are differentially expressed in overweight animals compared to lean animals.

The data was analyzed using Partek^{®} GS (Partek Inc., St. Charles, MO) for Gene Expression Data software (Partek Incorporated, 12747 Olive Blvd., Suite 205, St. Louis, Missouri 63141, U.S.A. http://www.partek.com/partekgs geneexpression ). The Robust Multichip Average (RMA) algorithm ( Rafael. A. Irizarry, Benjamin M. Bolstad, Francois Collin, Leslie M. Cope, Bridget Hobbs and Terence P. Speed (2003), Summaries of Affymetrix GeneChip probe level data Nucleic Acids Research 31(4):e15) was used for background adjustment, normalization, and probe-level summarization of the GeneChip^{®} samples. The ANOVA analysis was performed to find significant differentially expressed genes between any two groups with a minimal FDR control at 0.1 (or p-value of 0.1 or 0.05 if power was not sufficient to apply FDR) and a fold change of 1.3 in each direction. Our empirical studies have revealed that the Canine-2 GeneChips^{®} have an associated background noise level of 1.3 fold. Therefore, all analyses presented in this report employed a +/- 1.3 fold cut-off. Furthermore, the false discovery rate threshold of 0.1 (means that 10% of observations are due to chance) was chosen as the minimum level of acceptable statistical significance for studies containing a minimum of 12 animals per group. Studies with smaller numbers of animals do not have enough power to allow for the proper application of the FDR. Results are provided in the tables below.

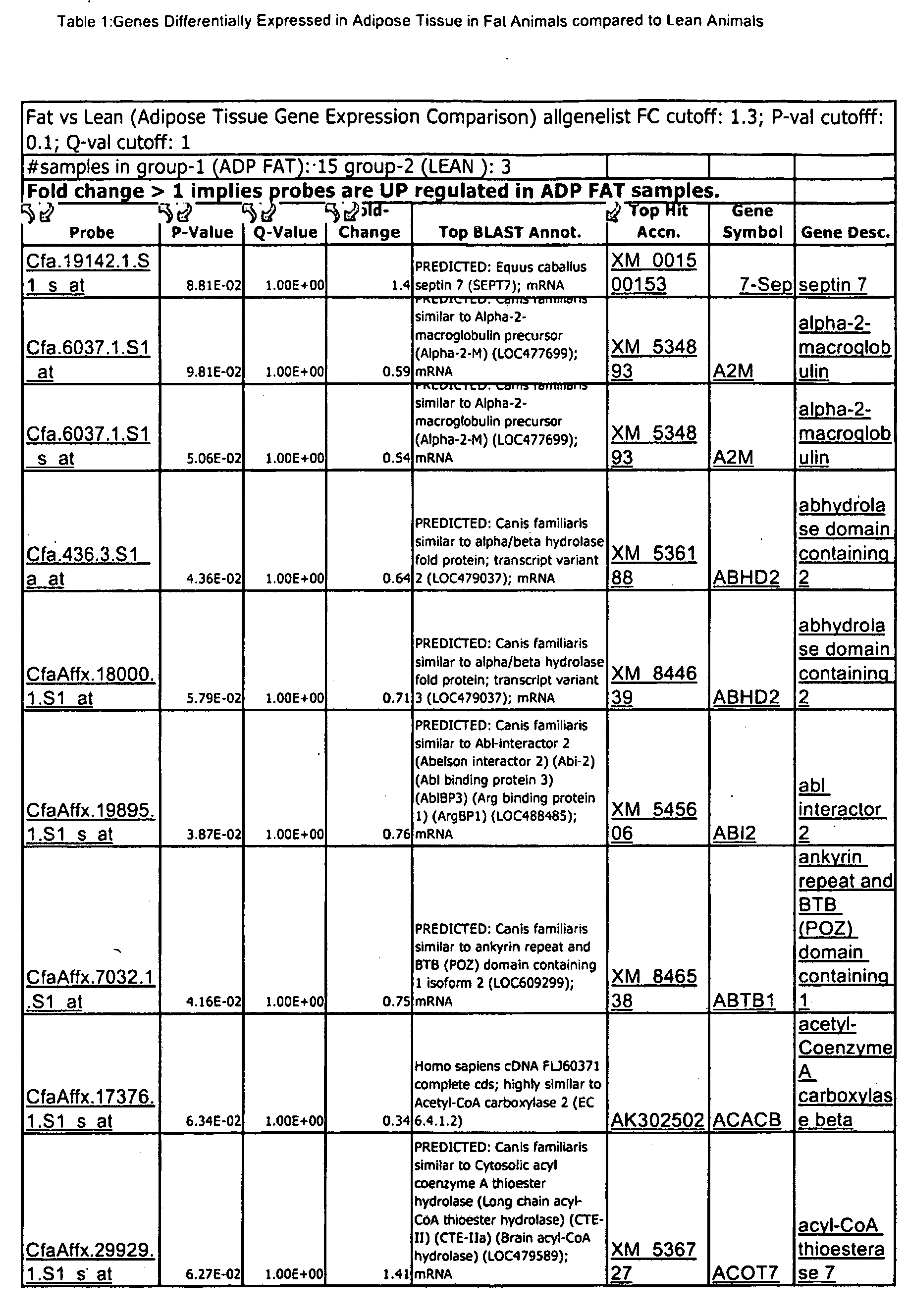

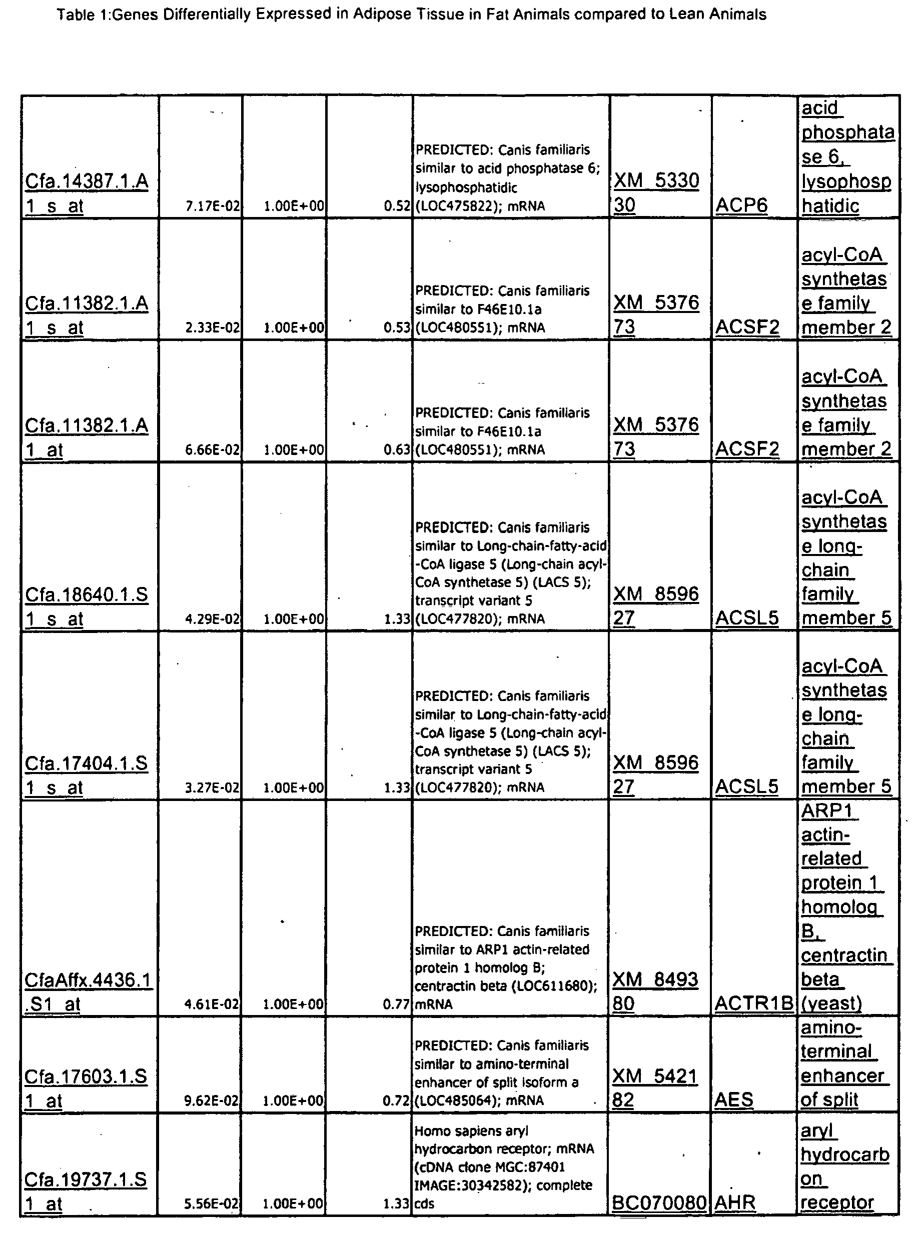

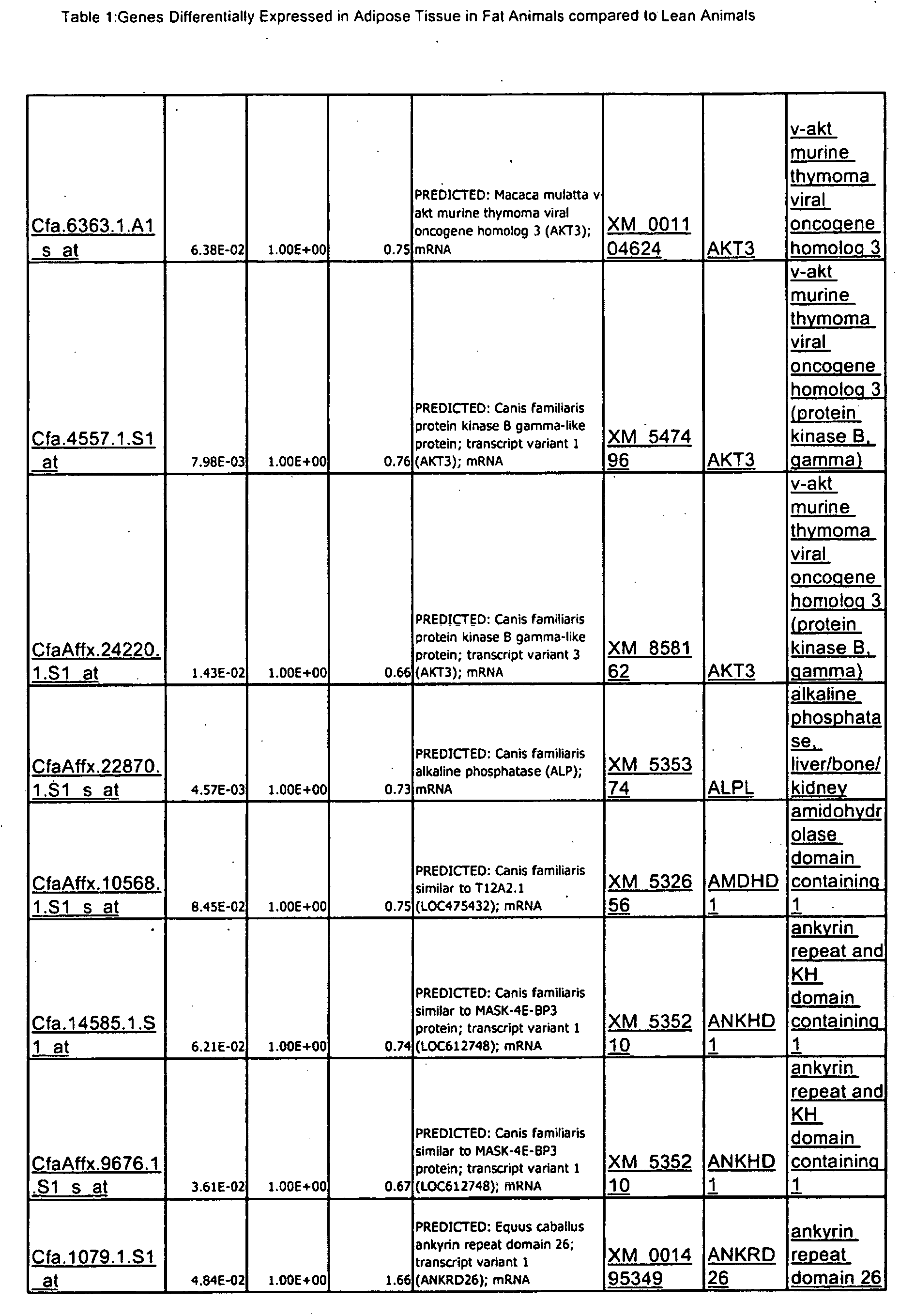

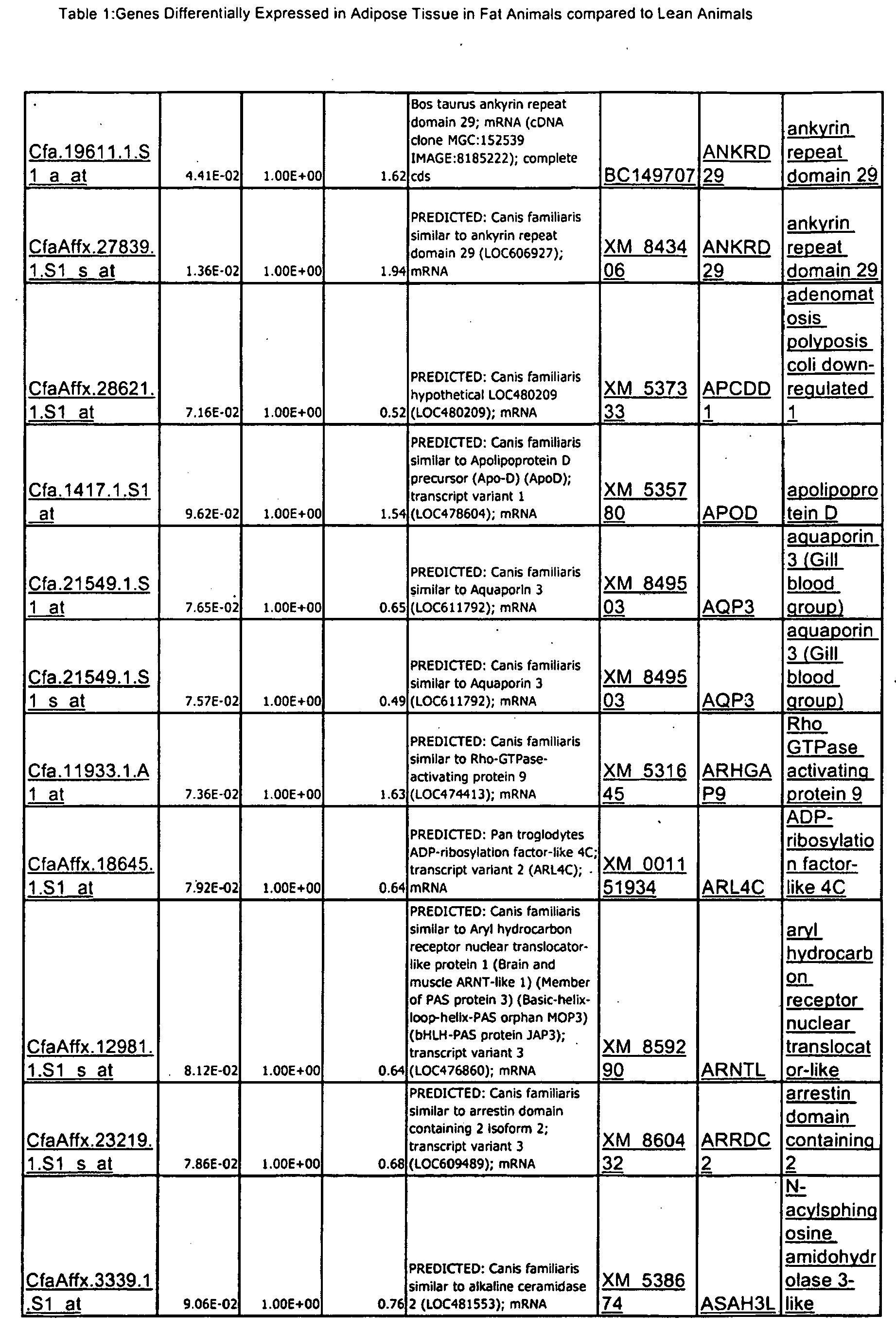

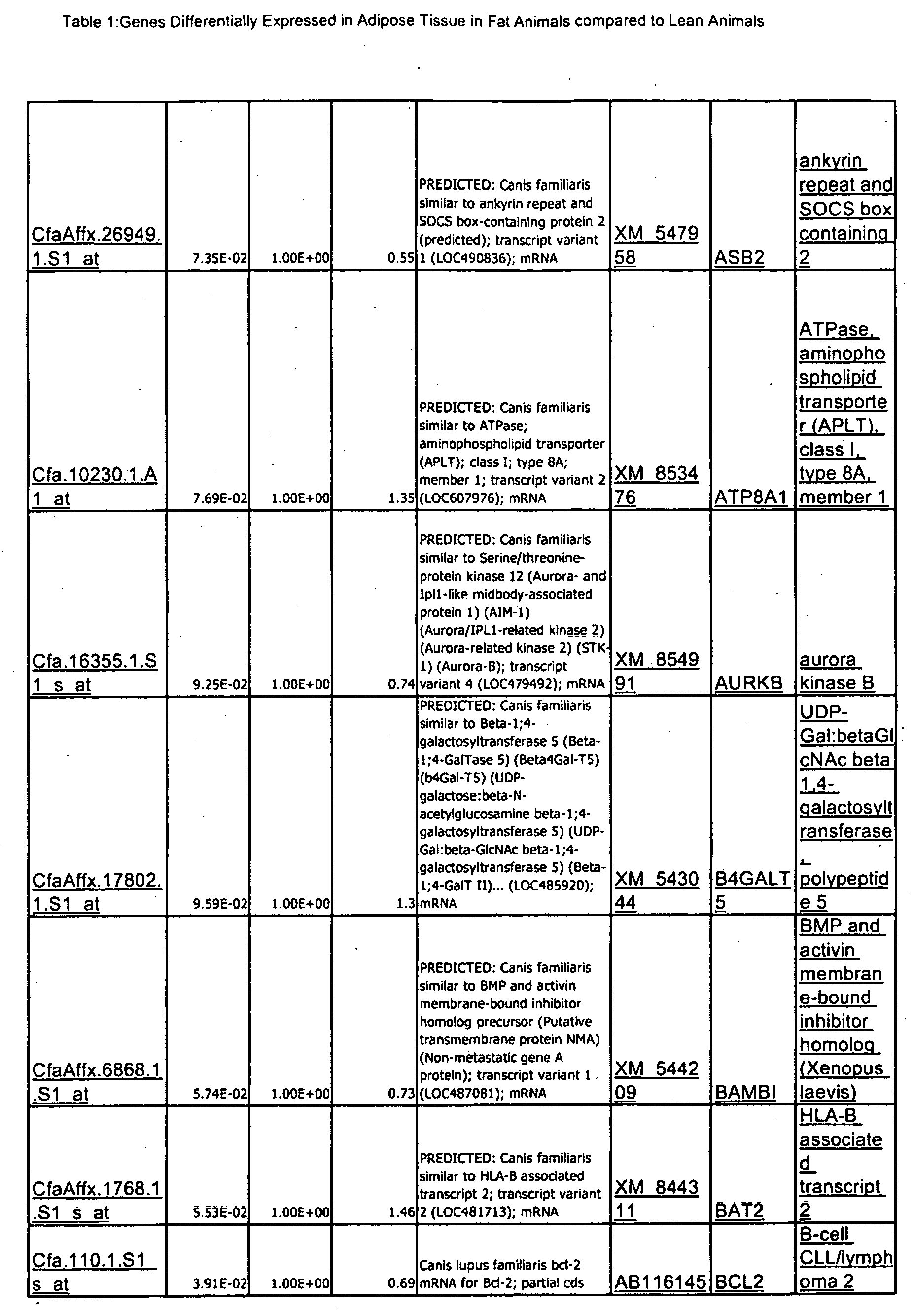

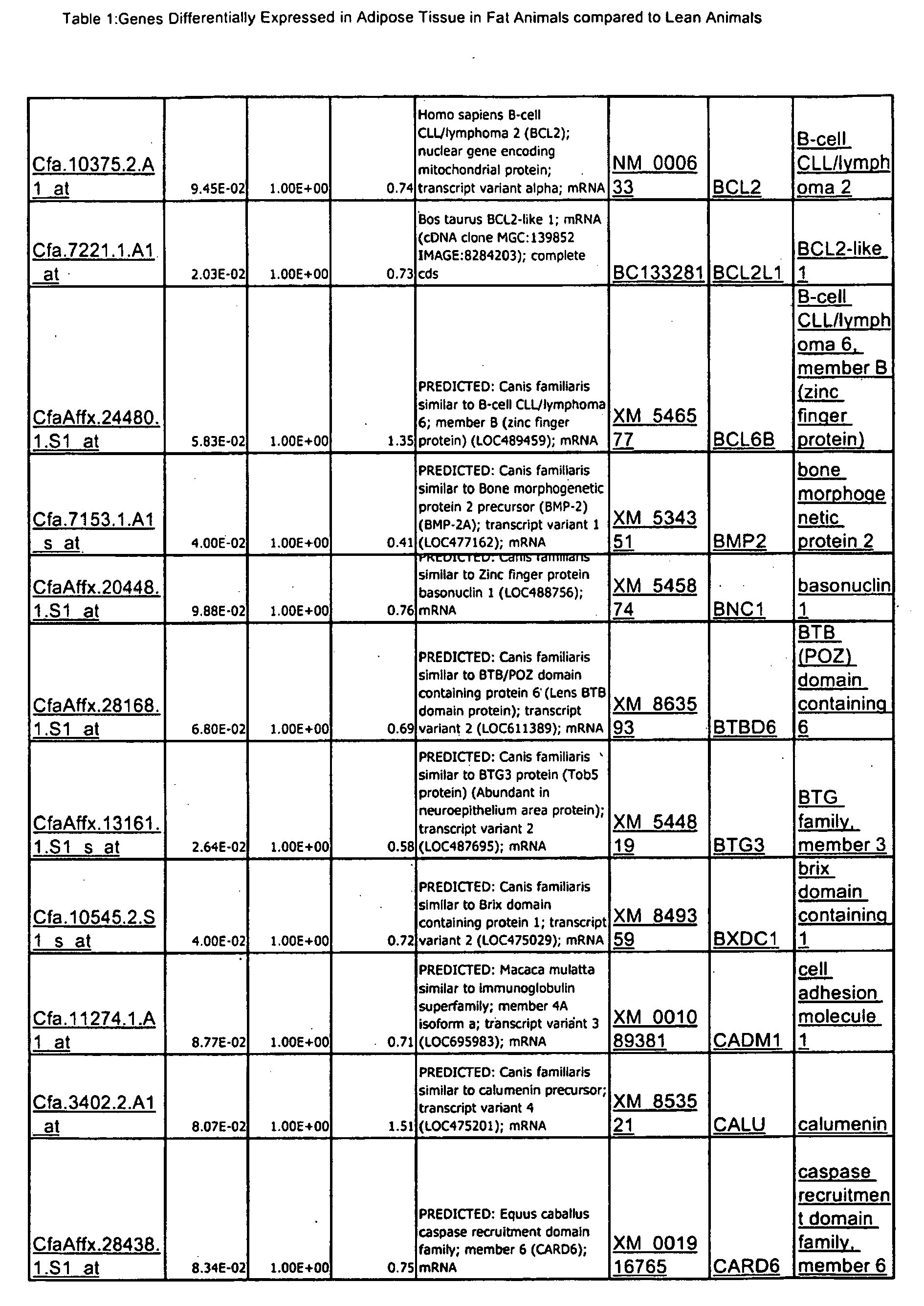

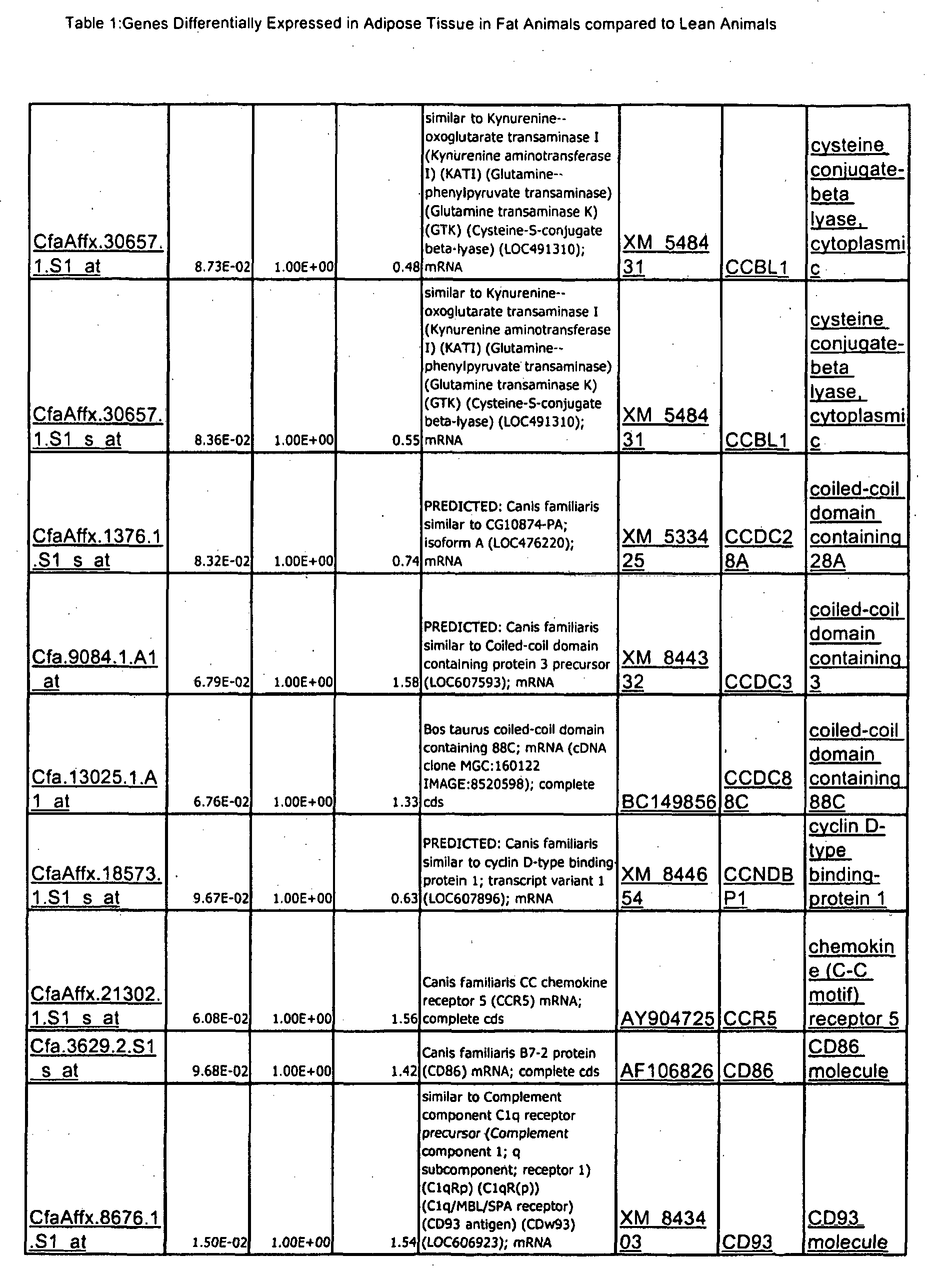

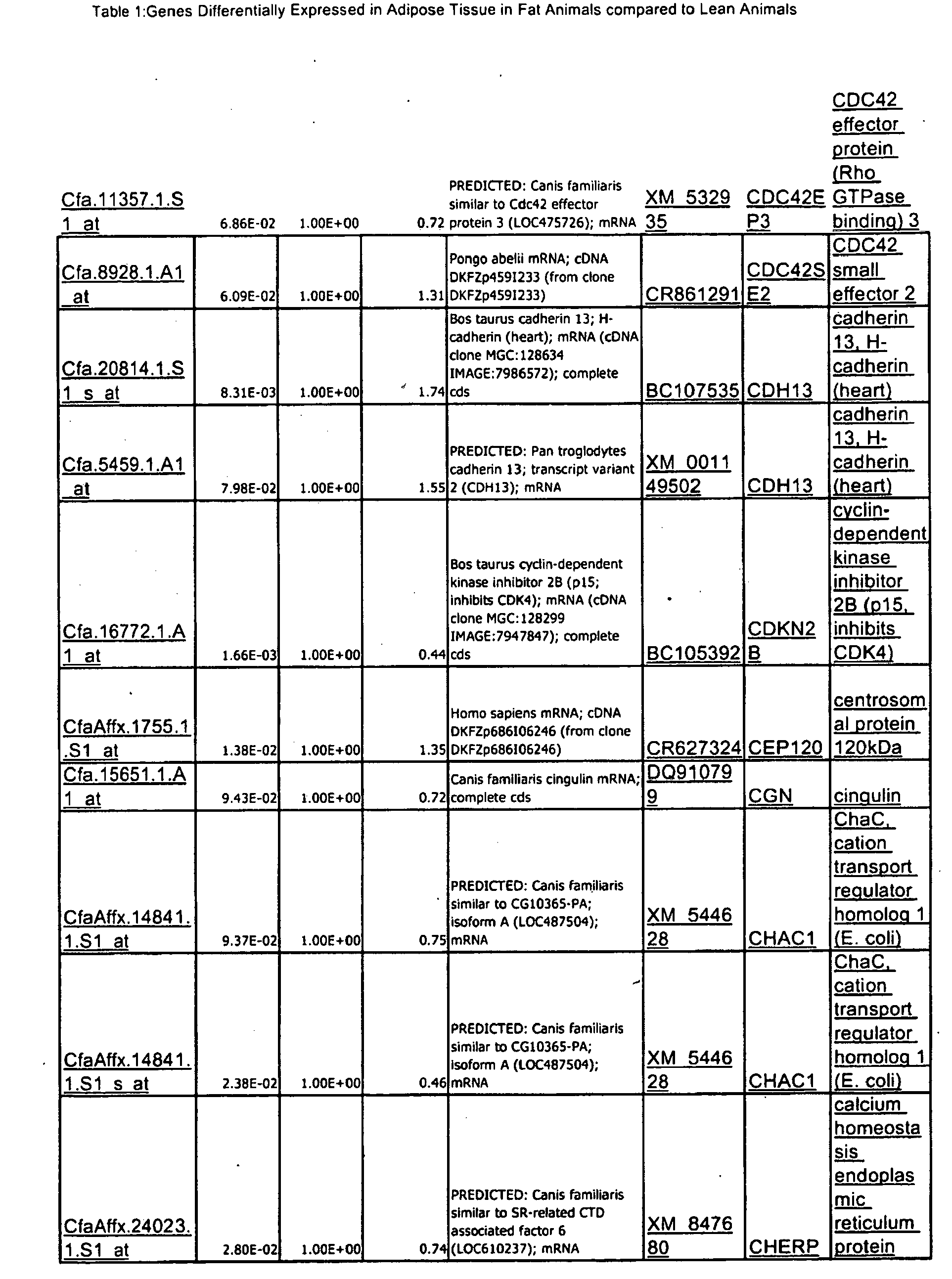

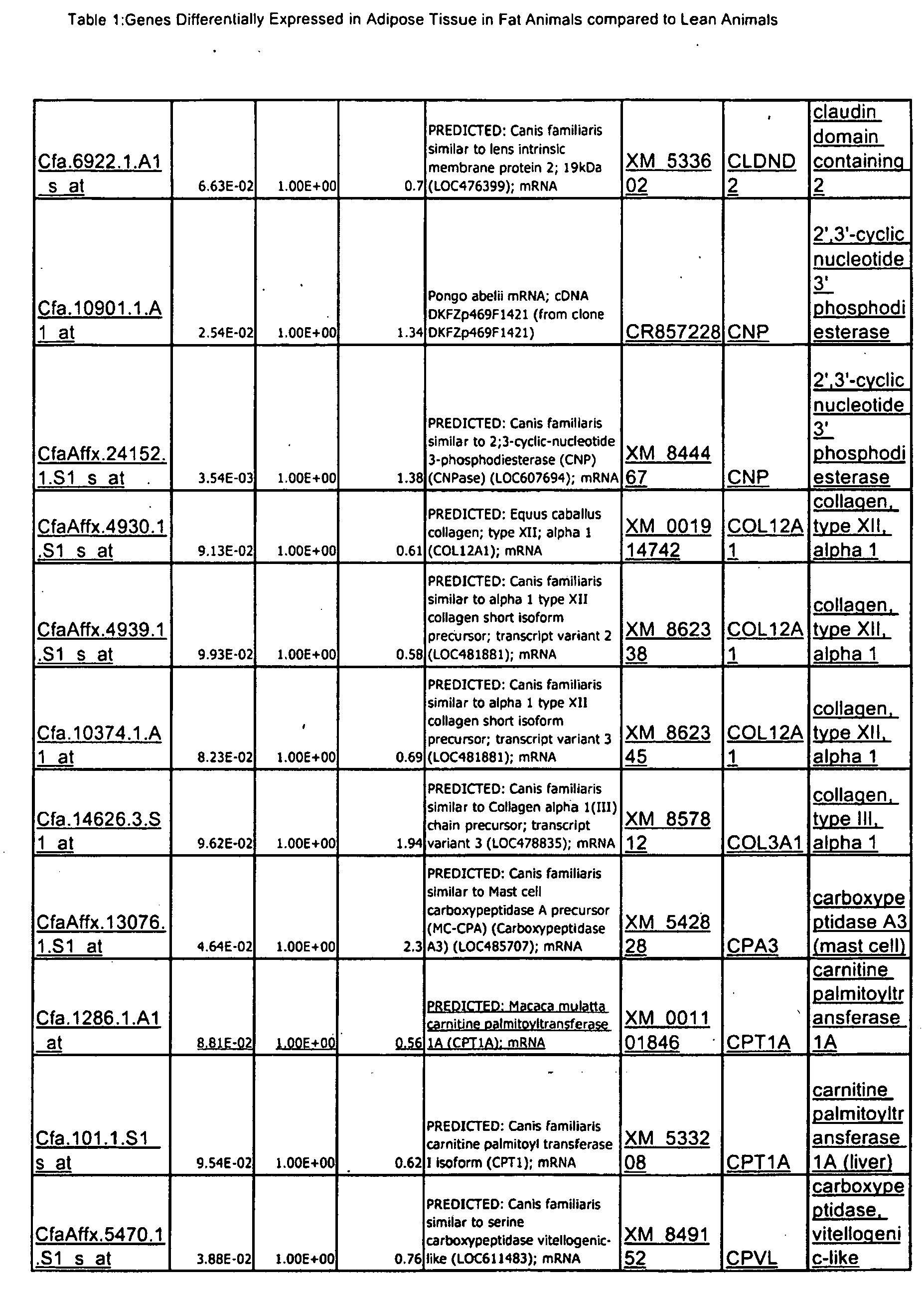

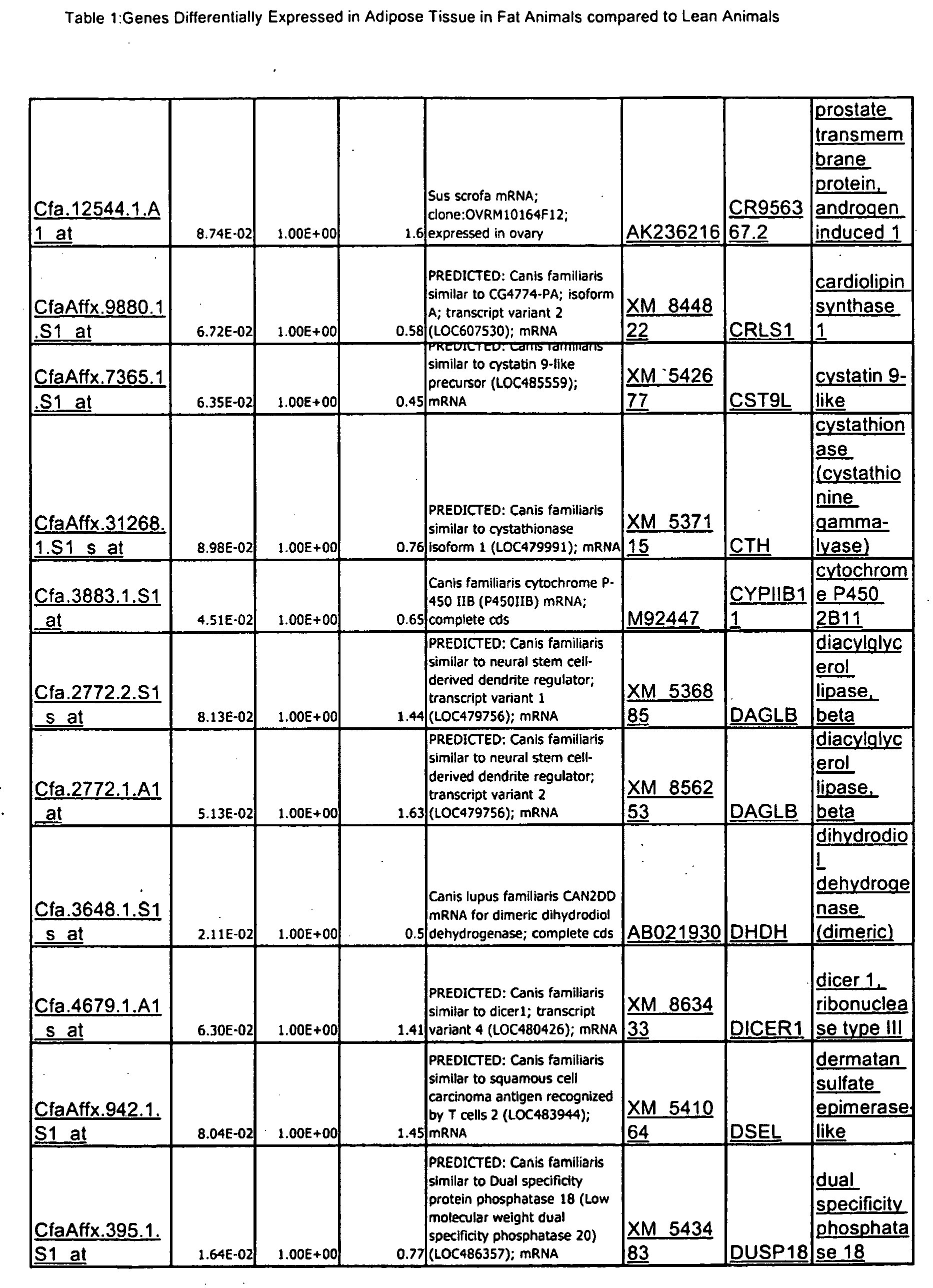

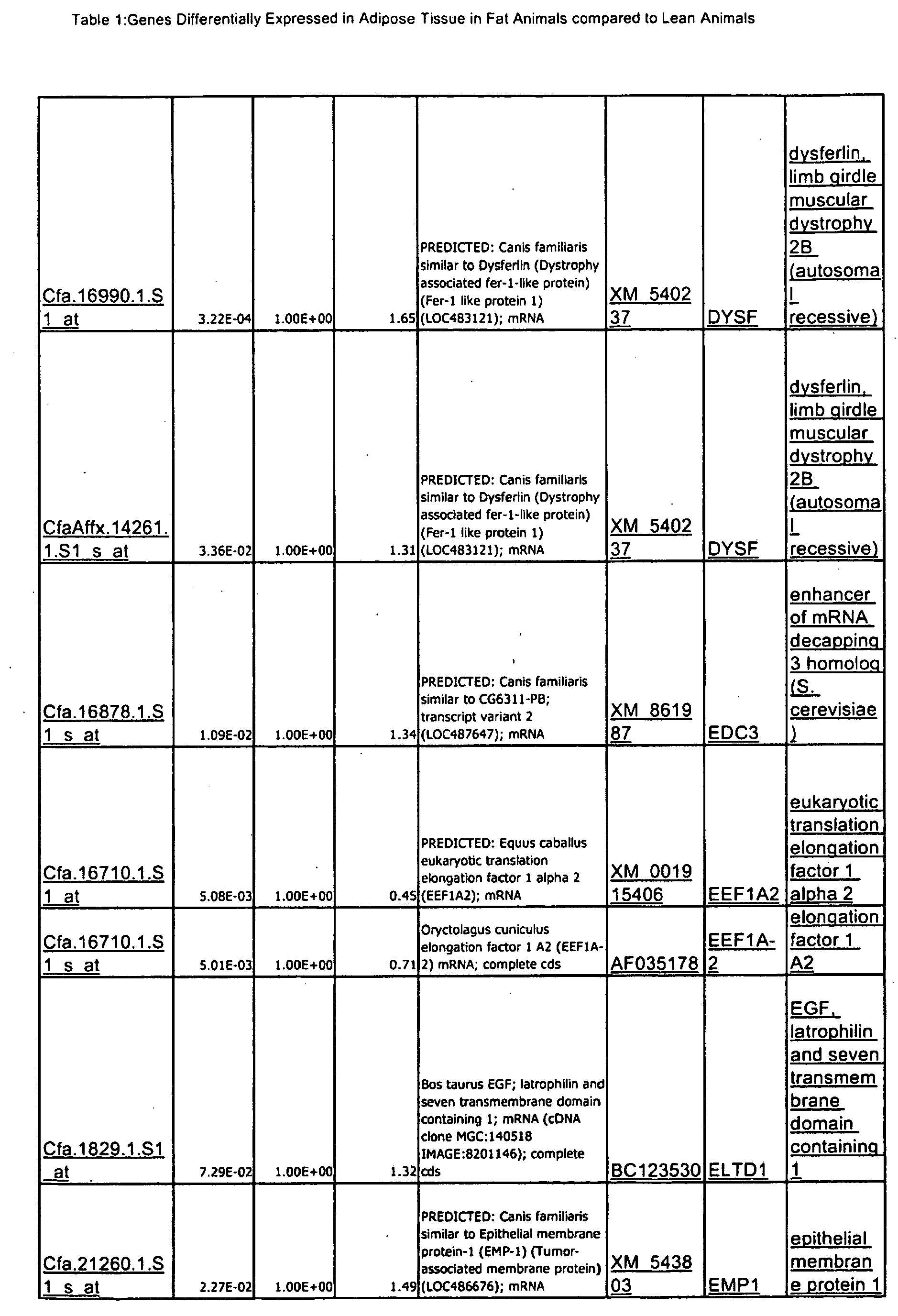

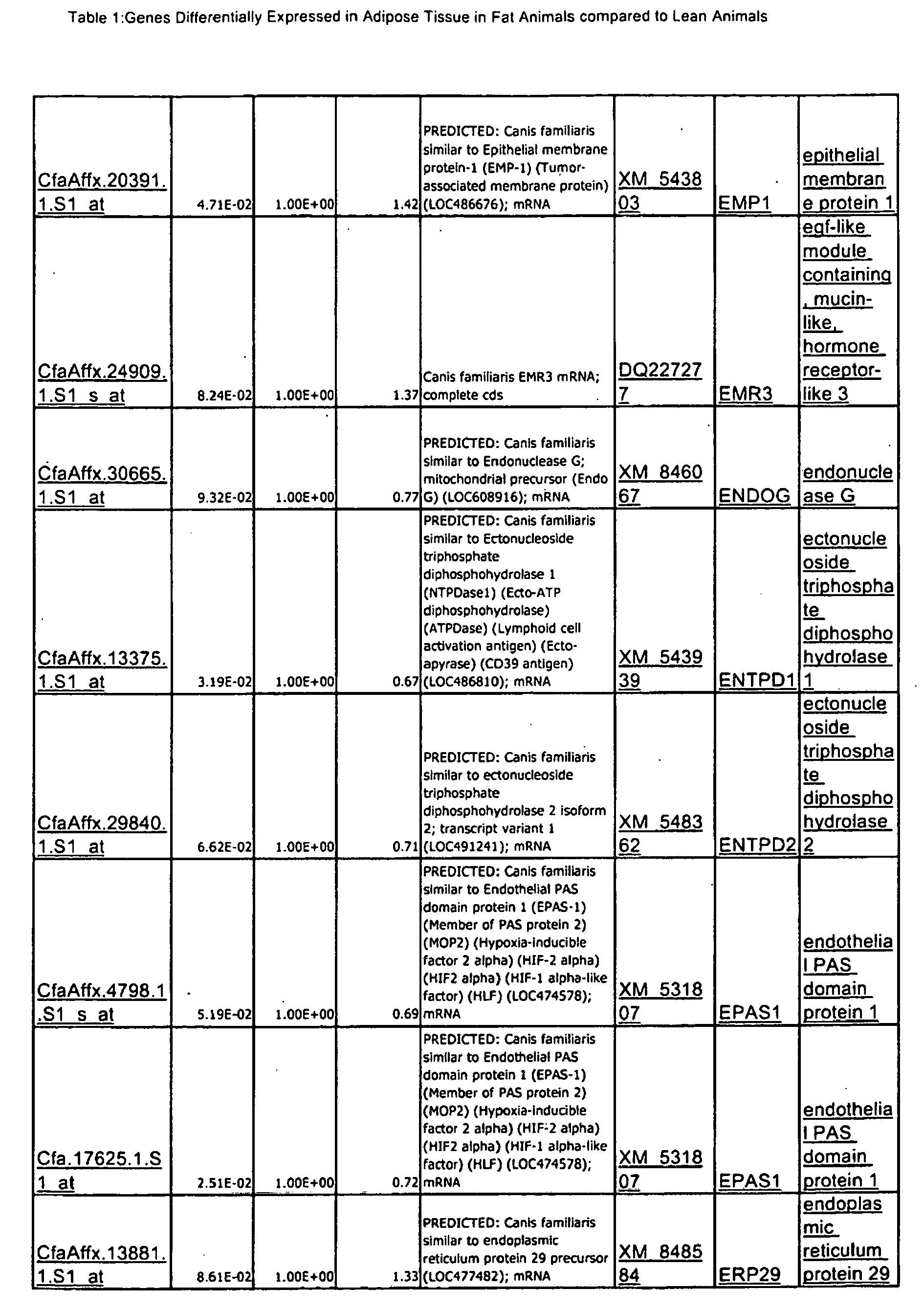

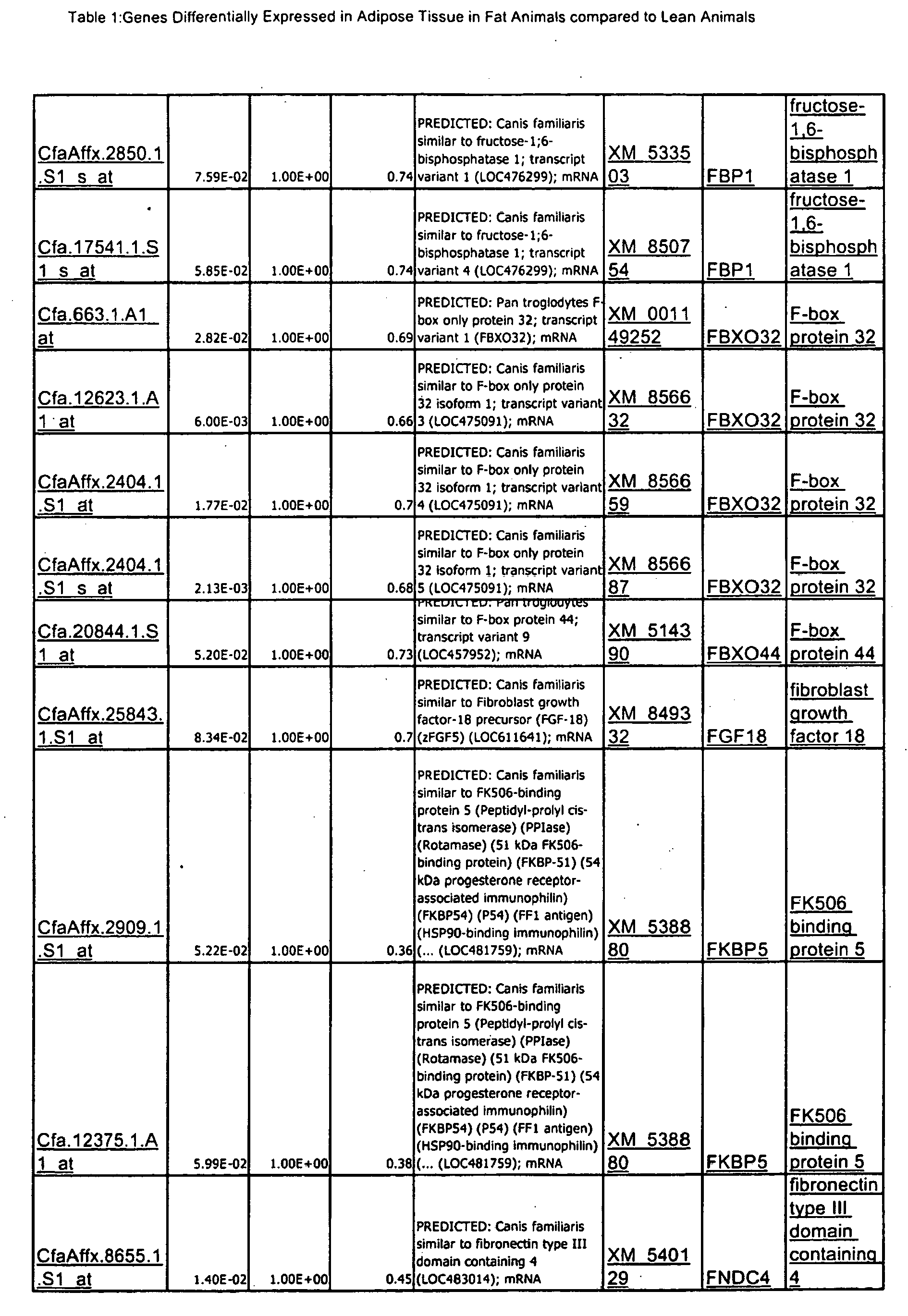

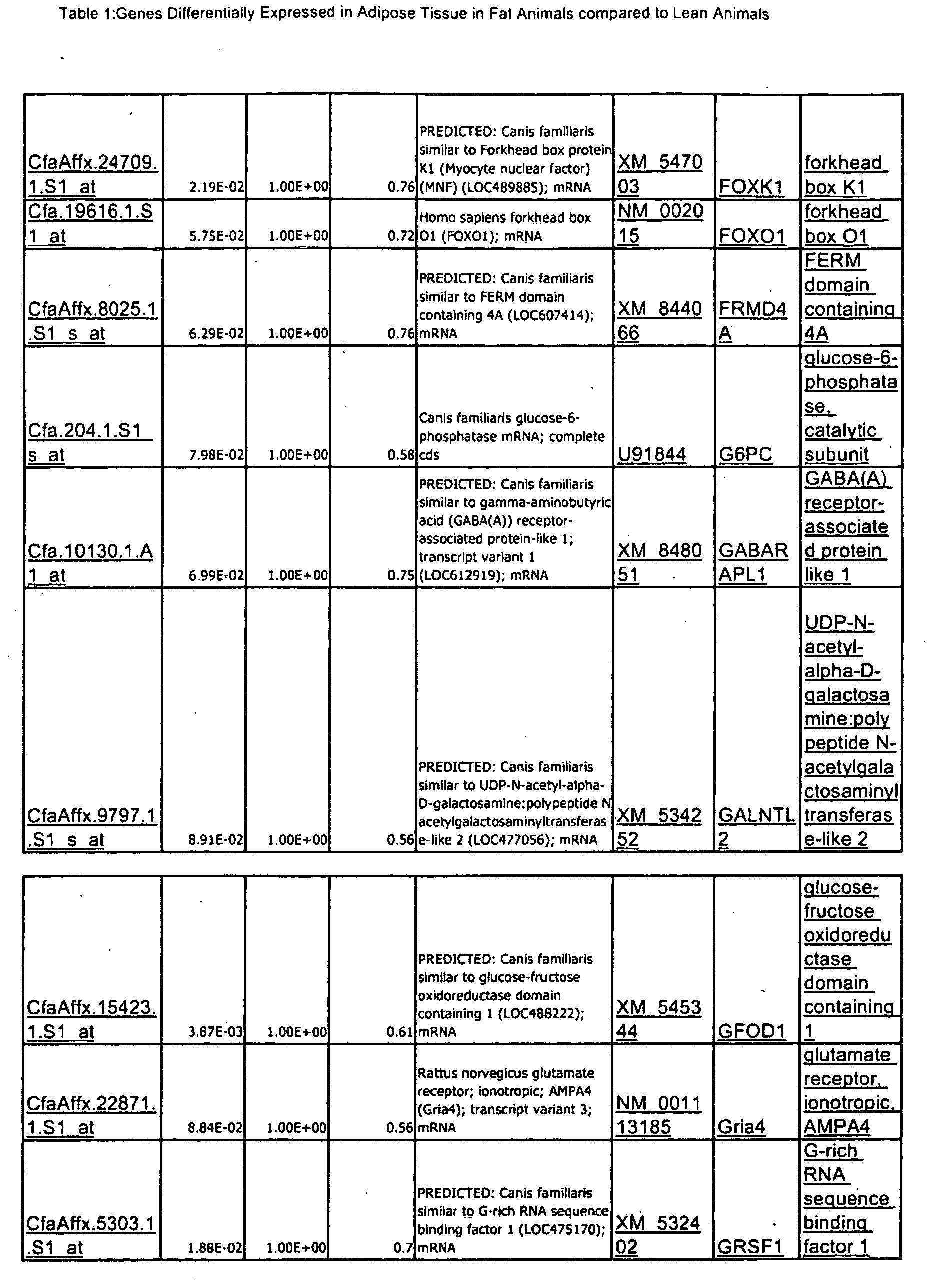

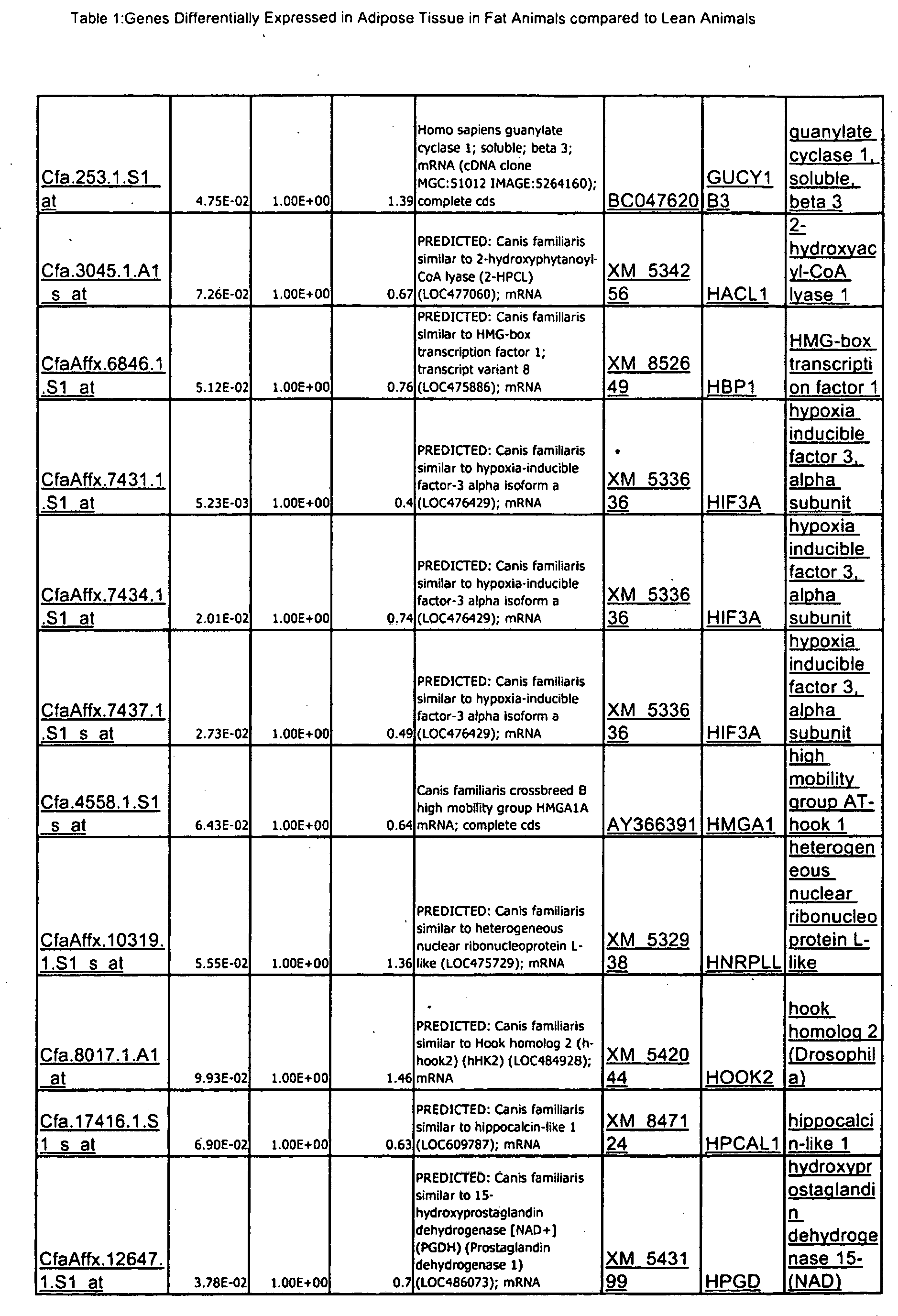

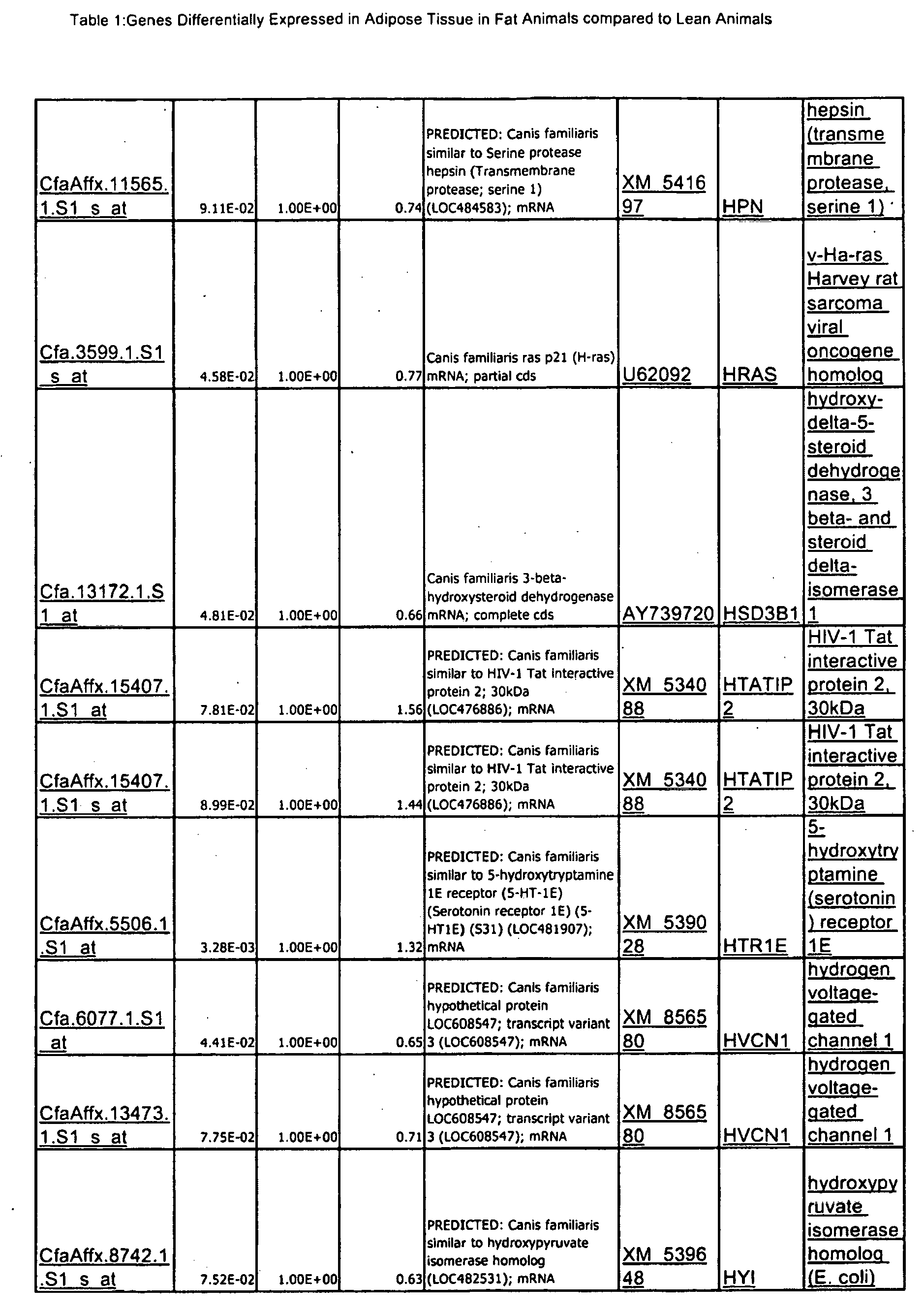

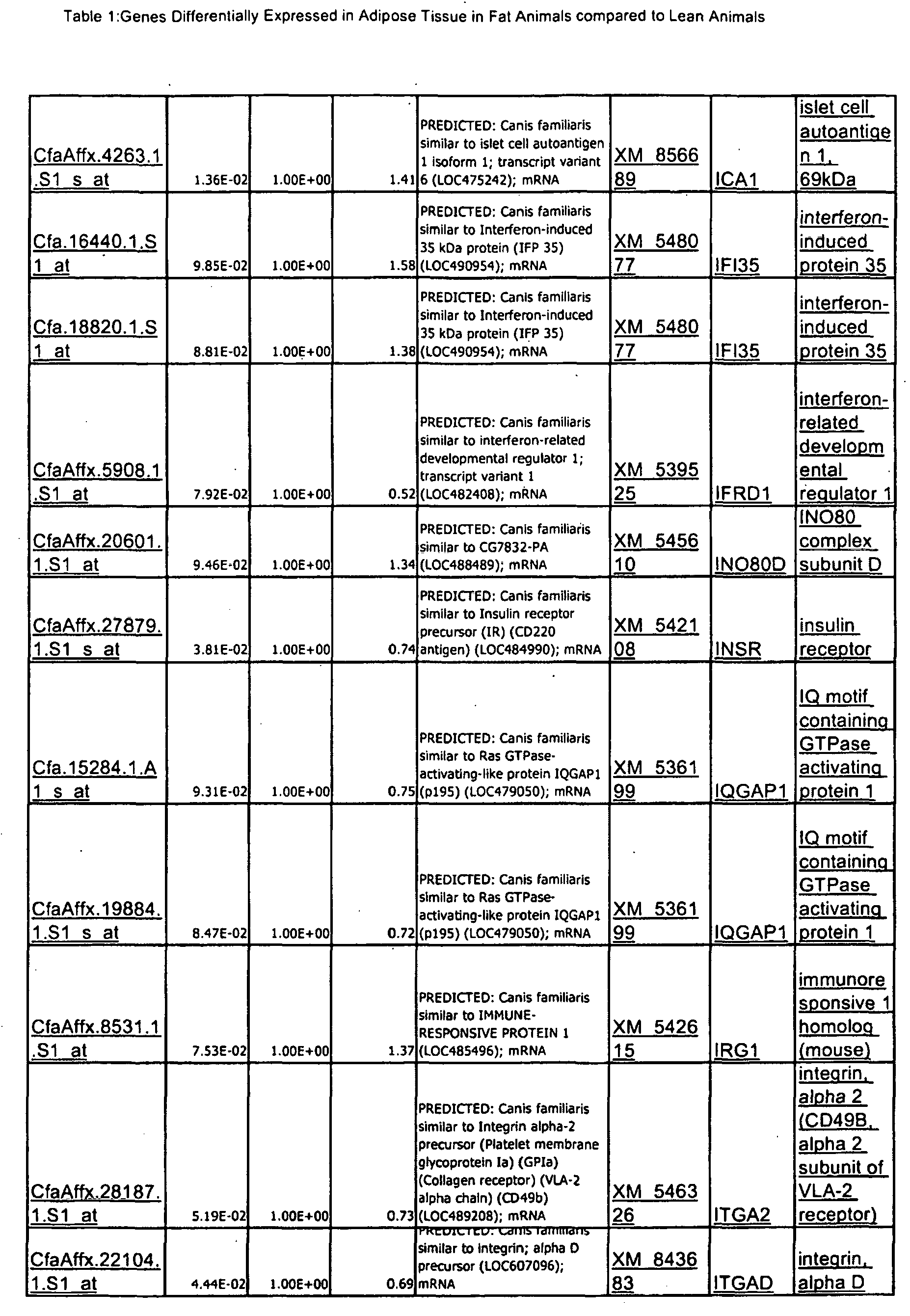

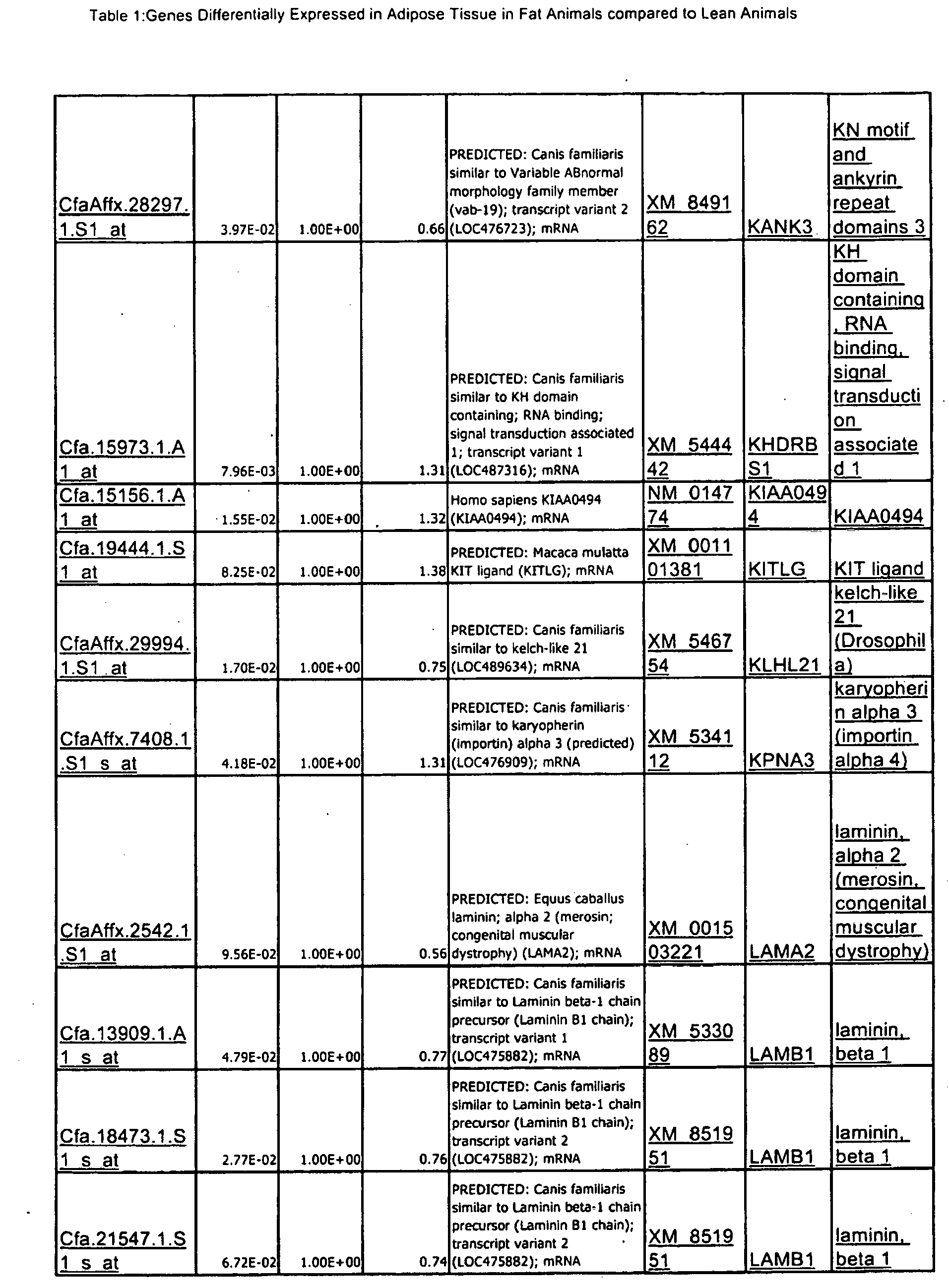

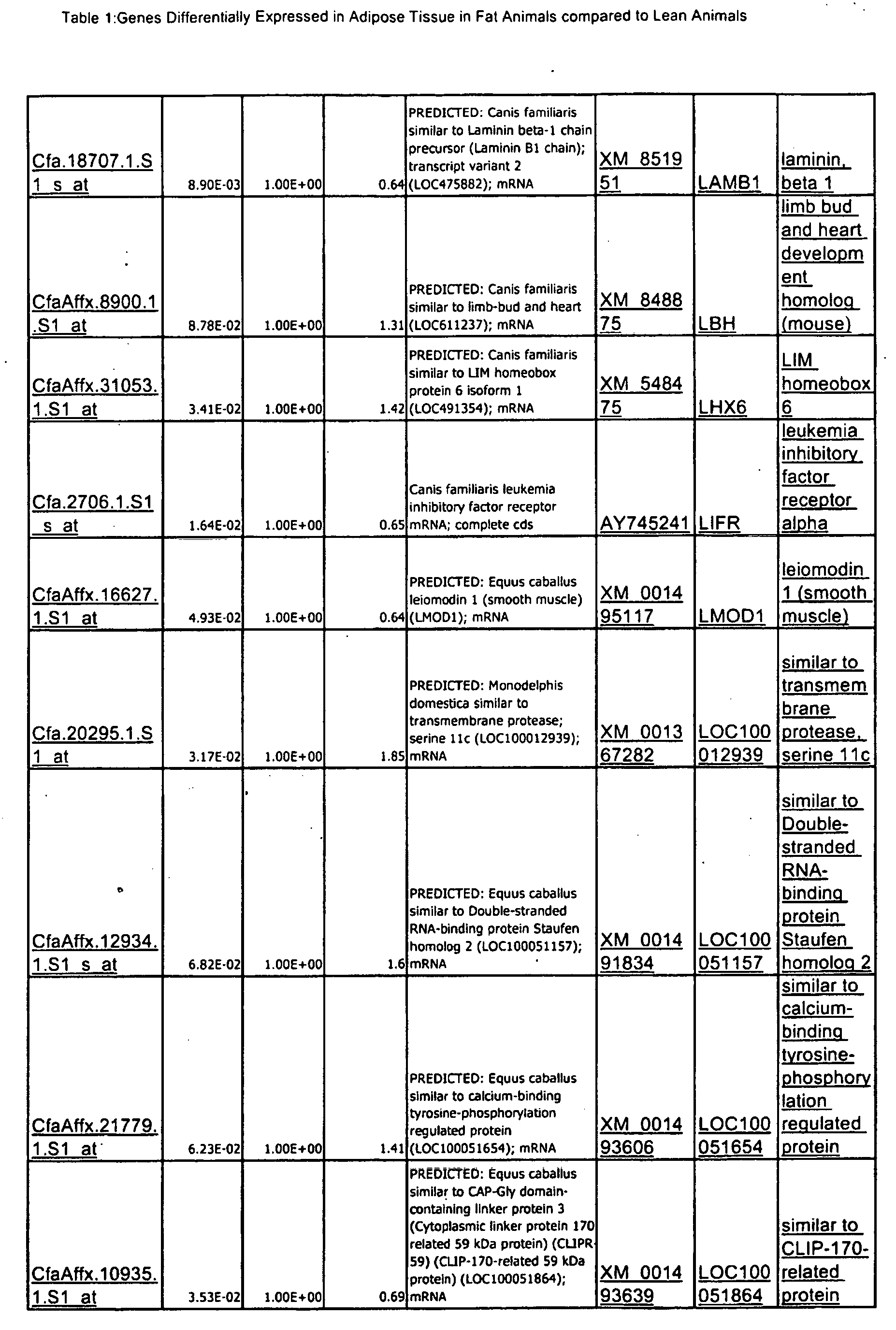

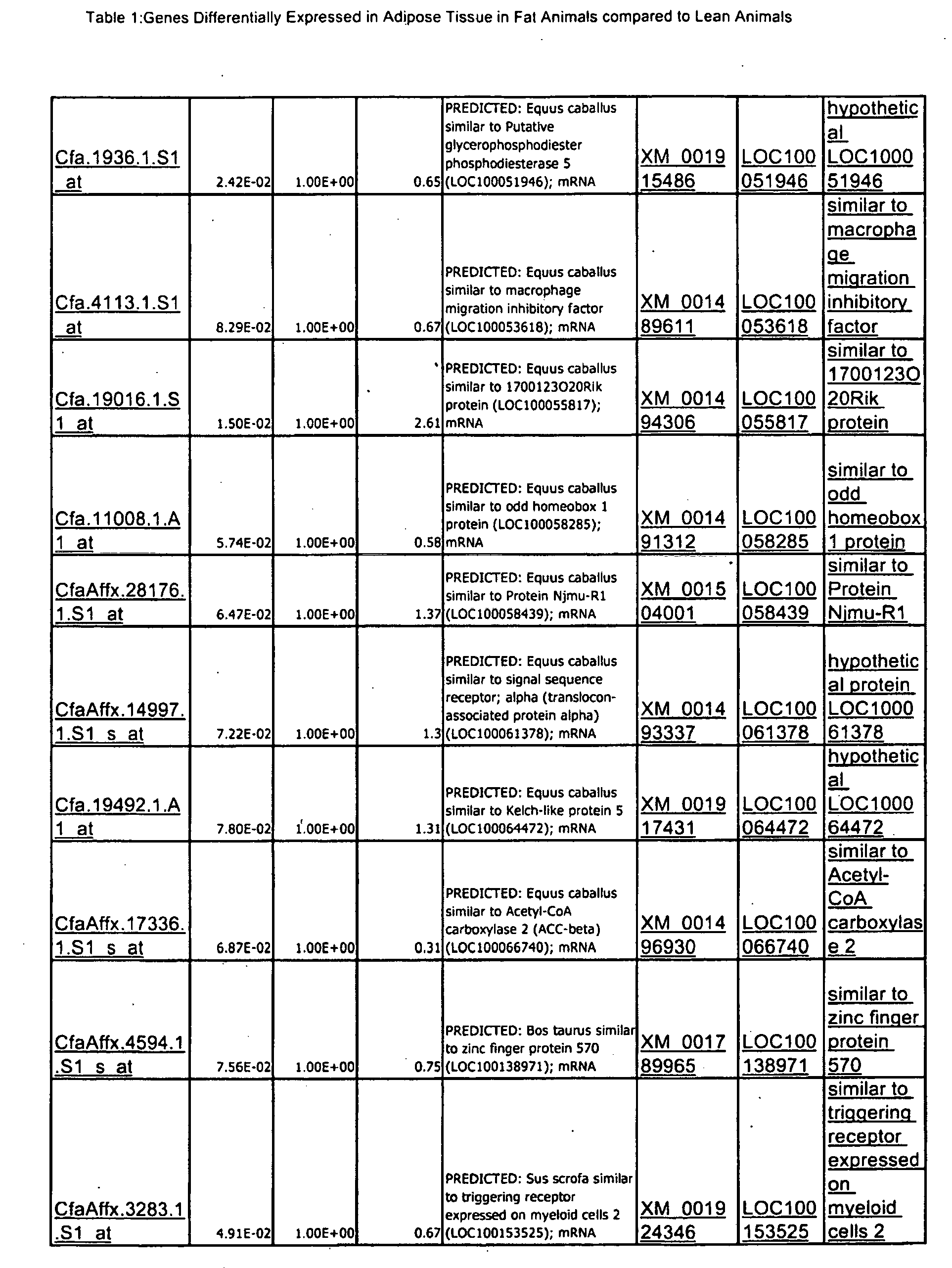

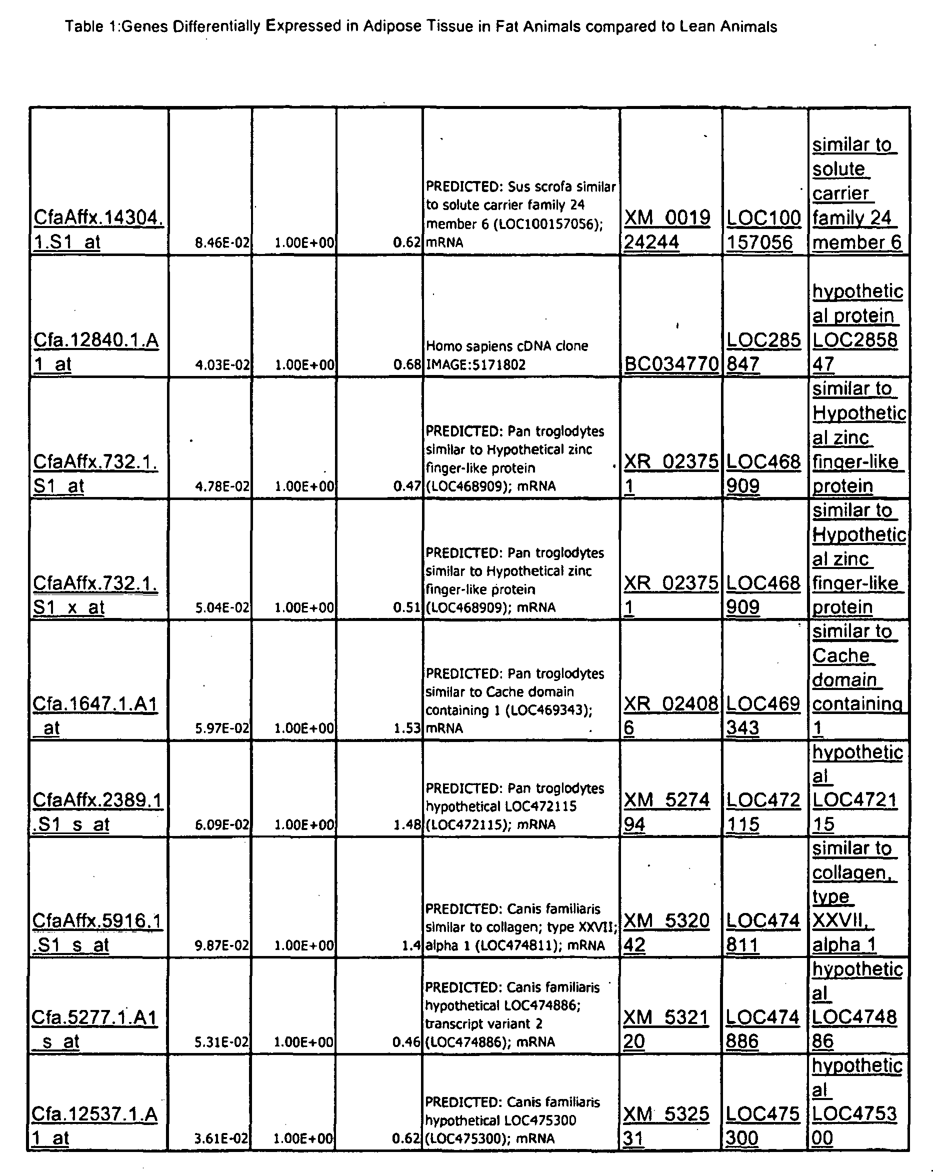

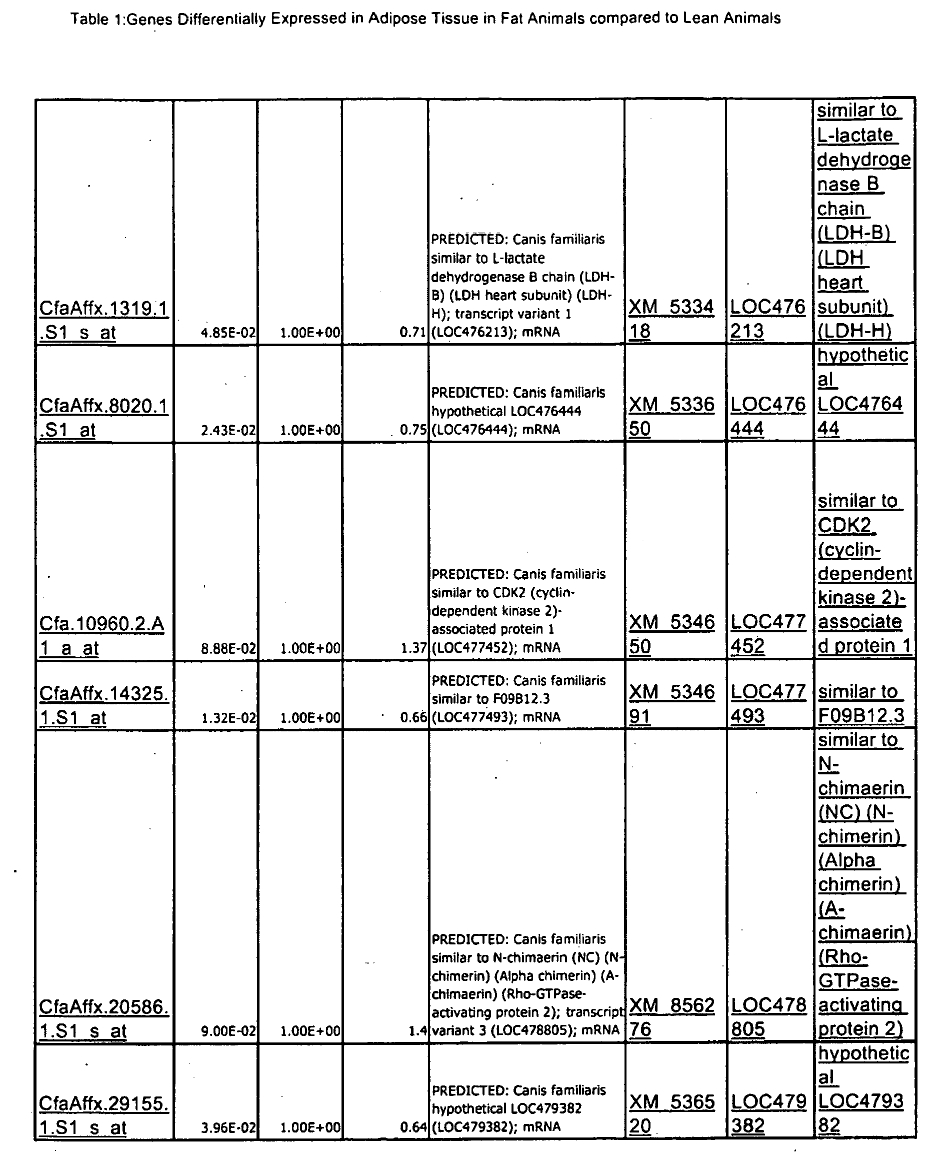

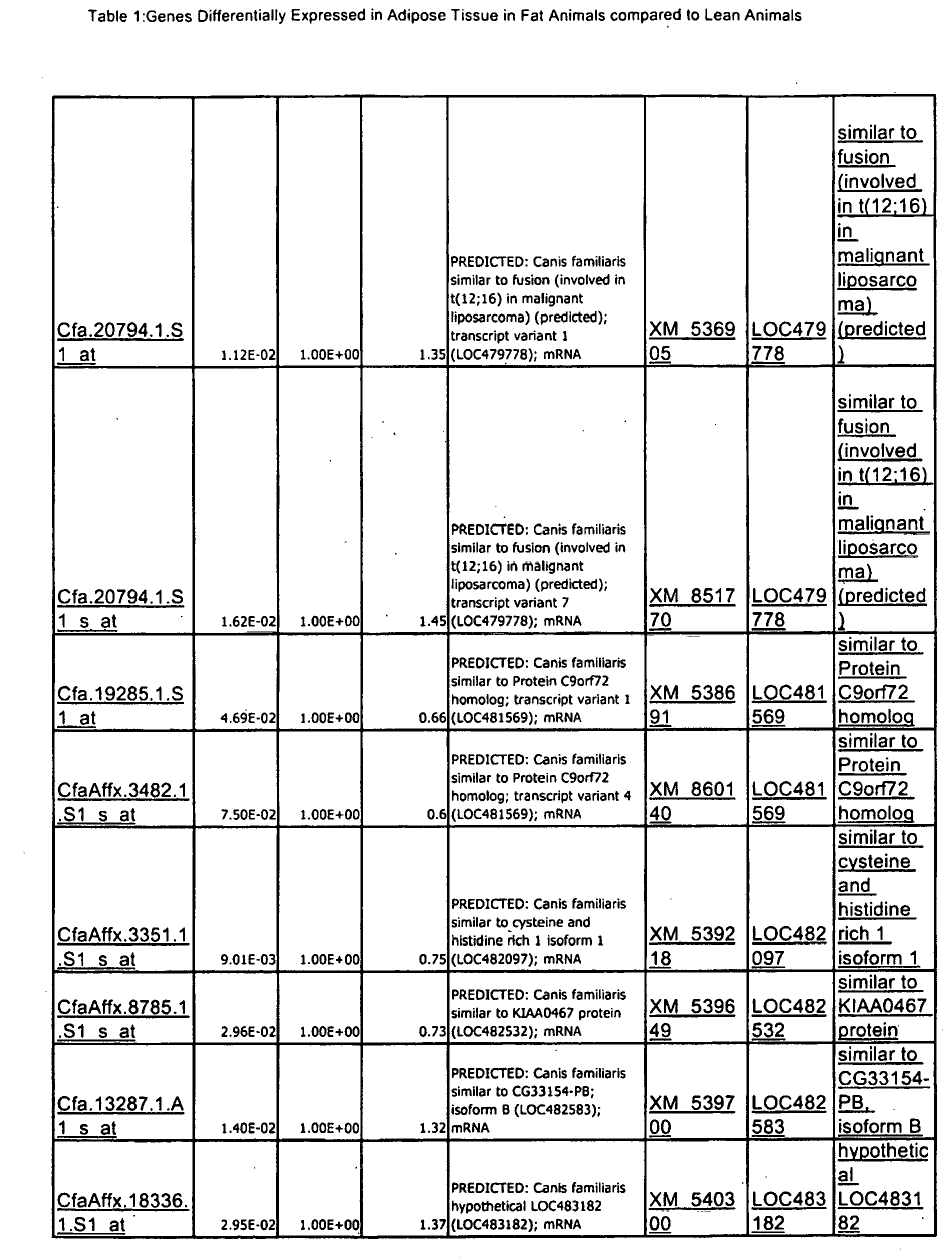

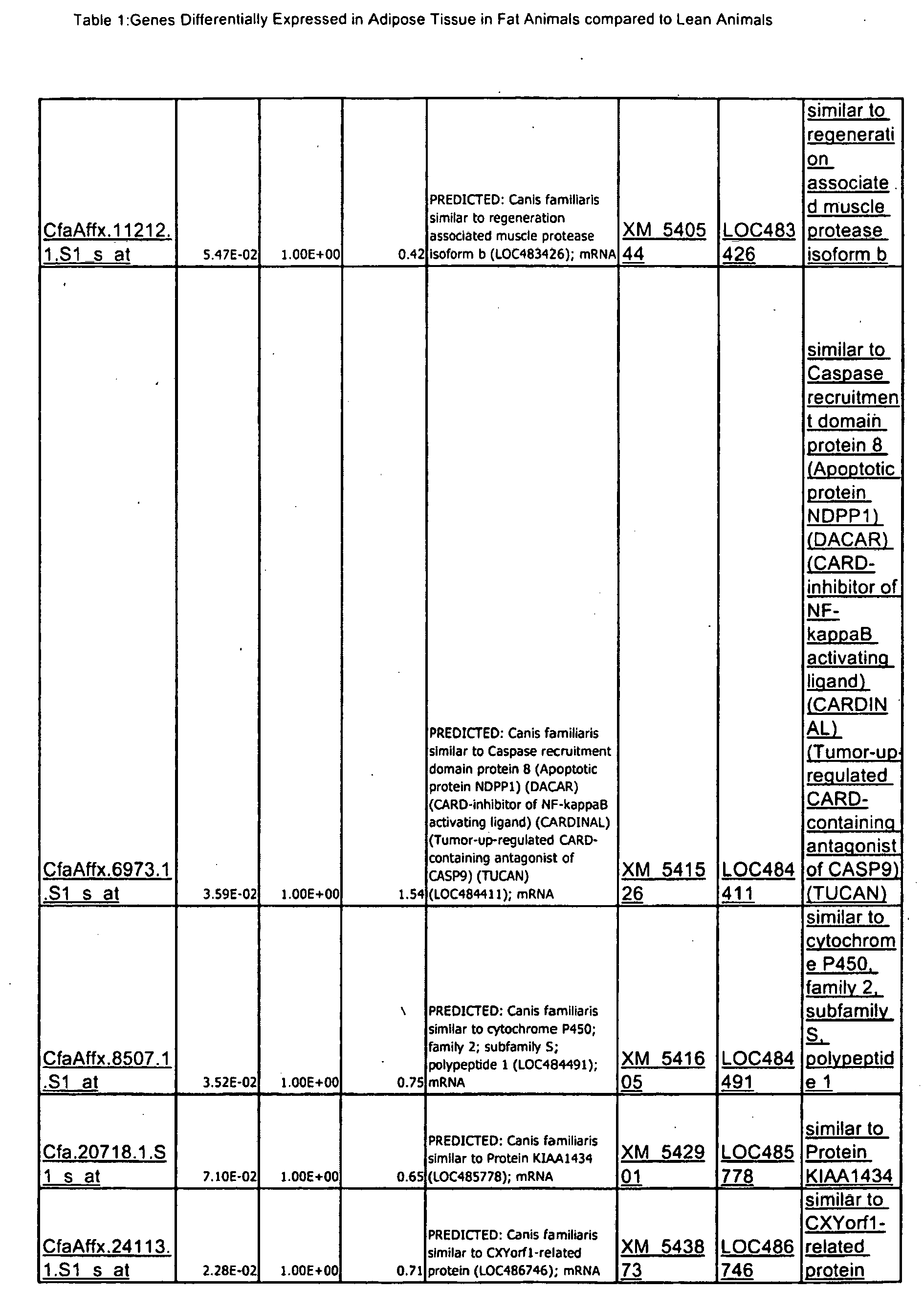

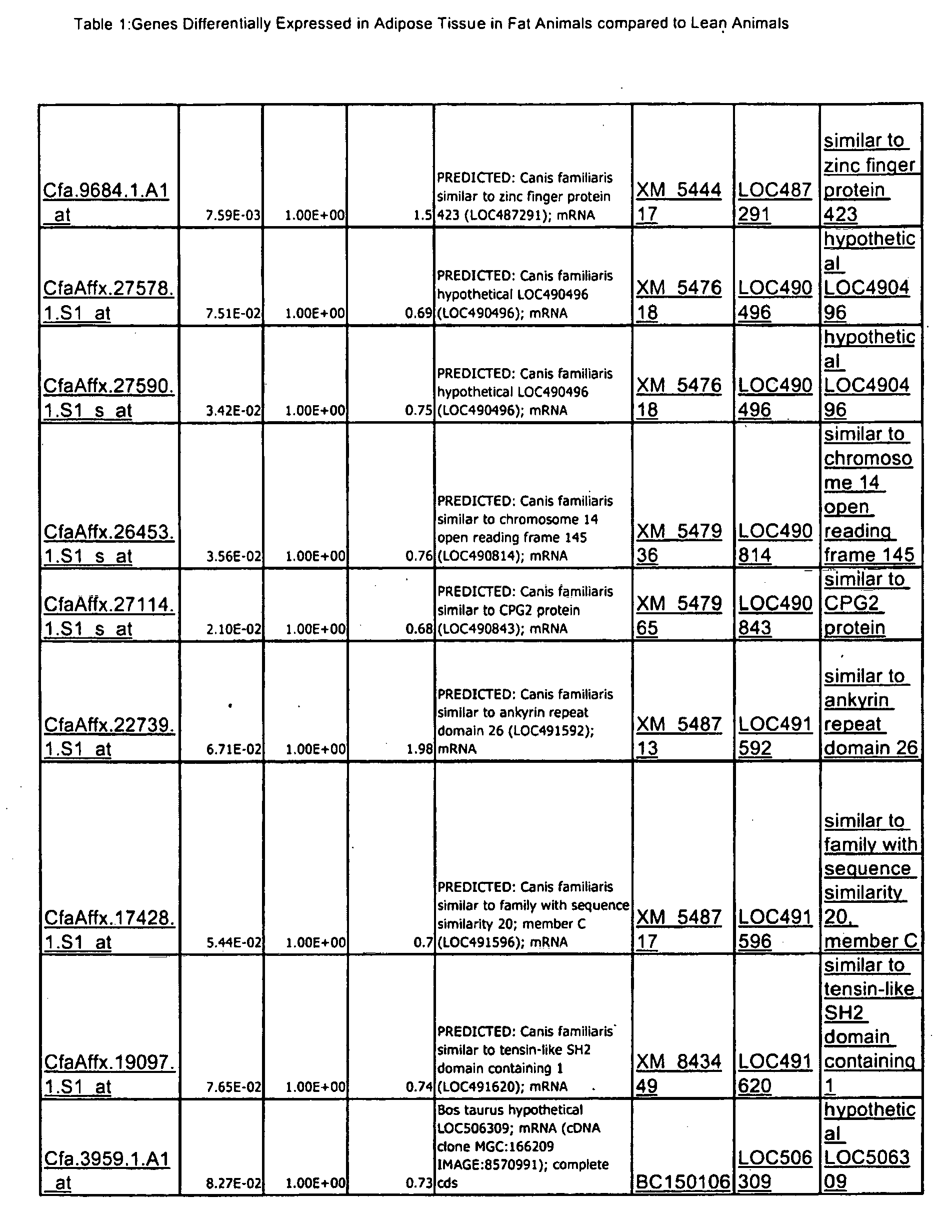

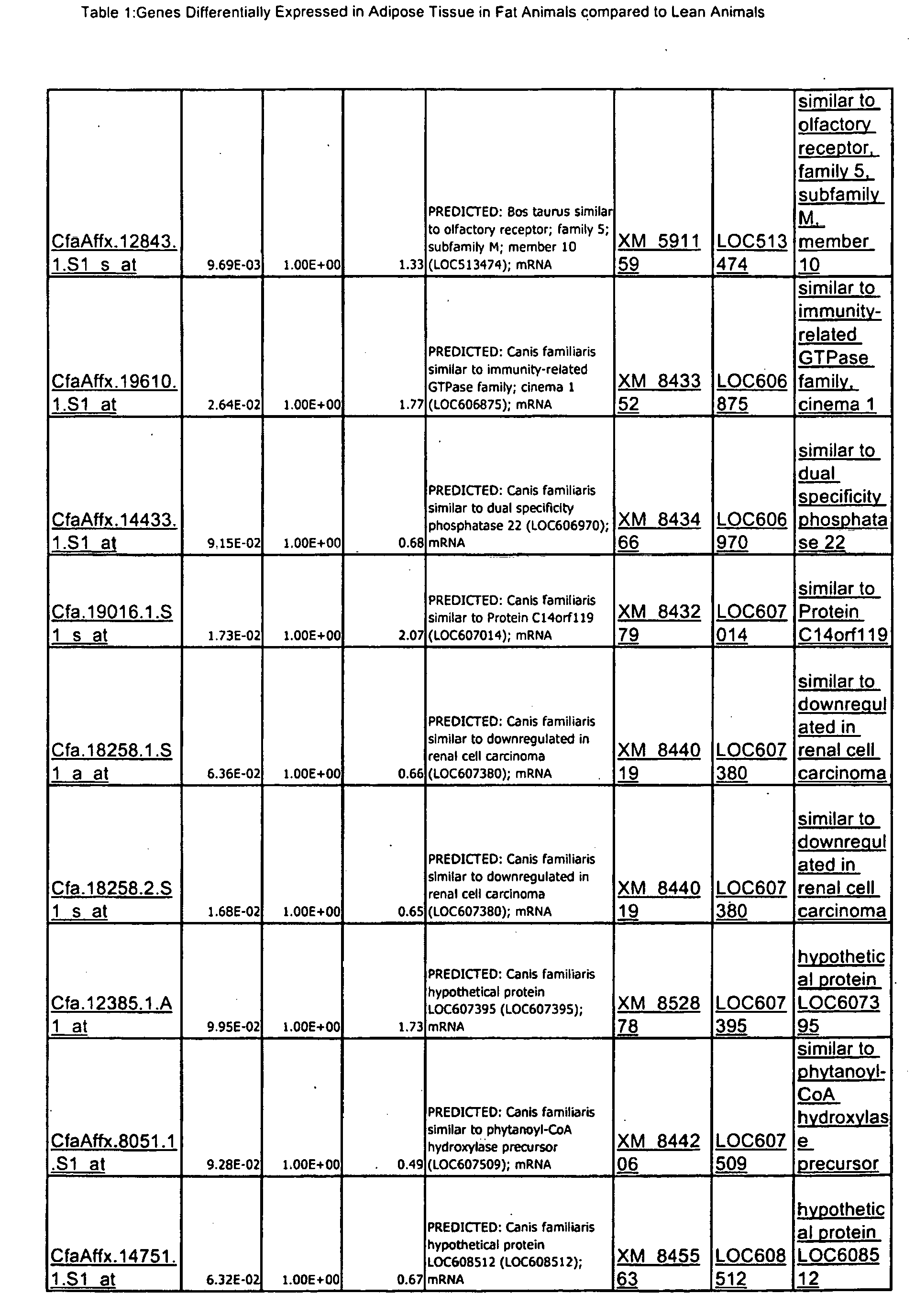

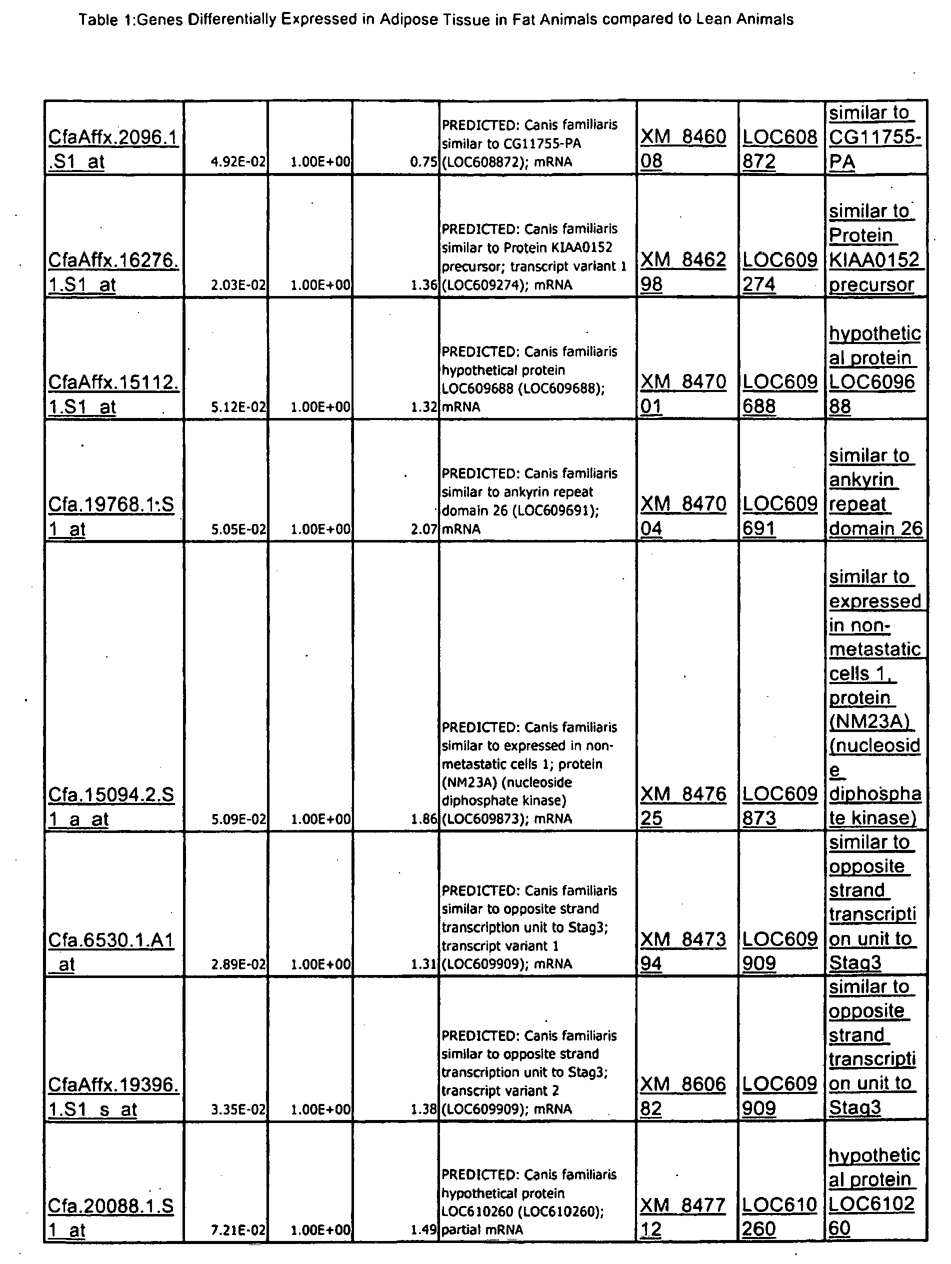

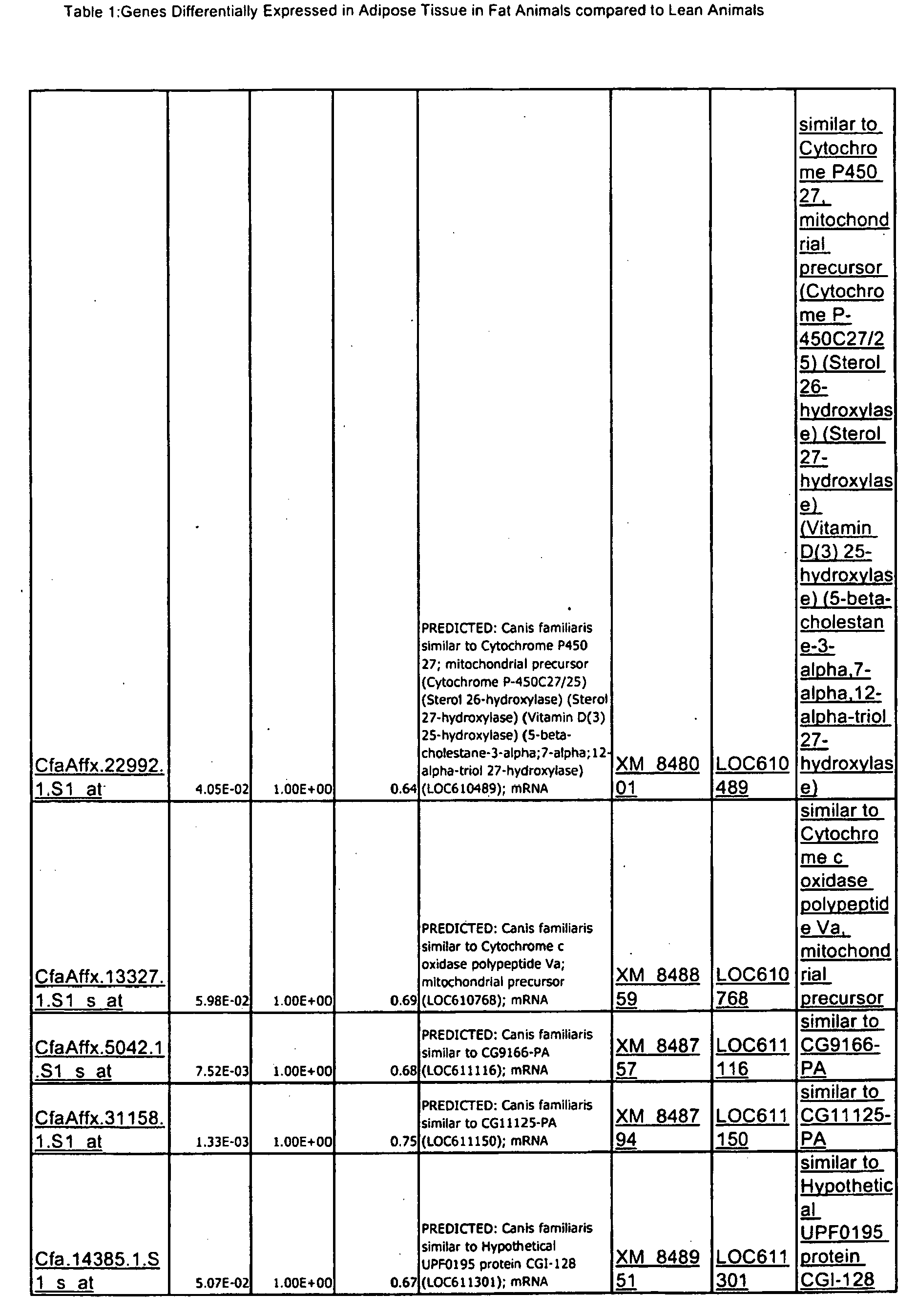

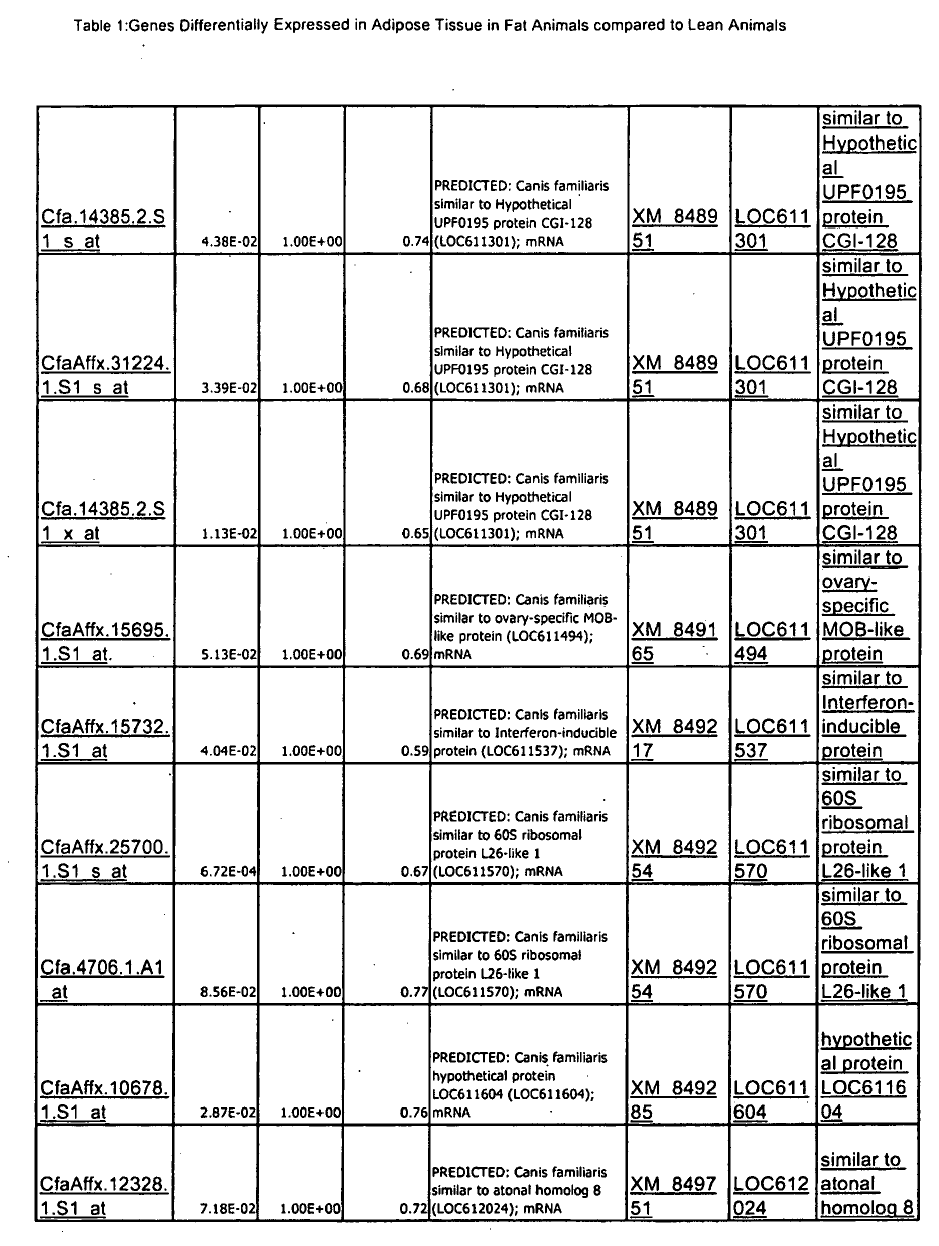

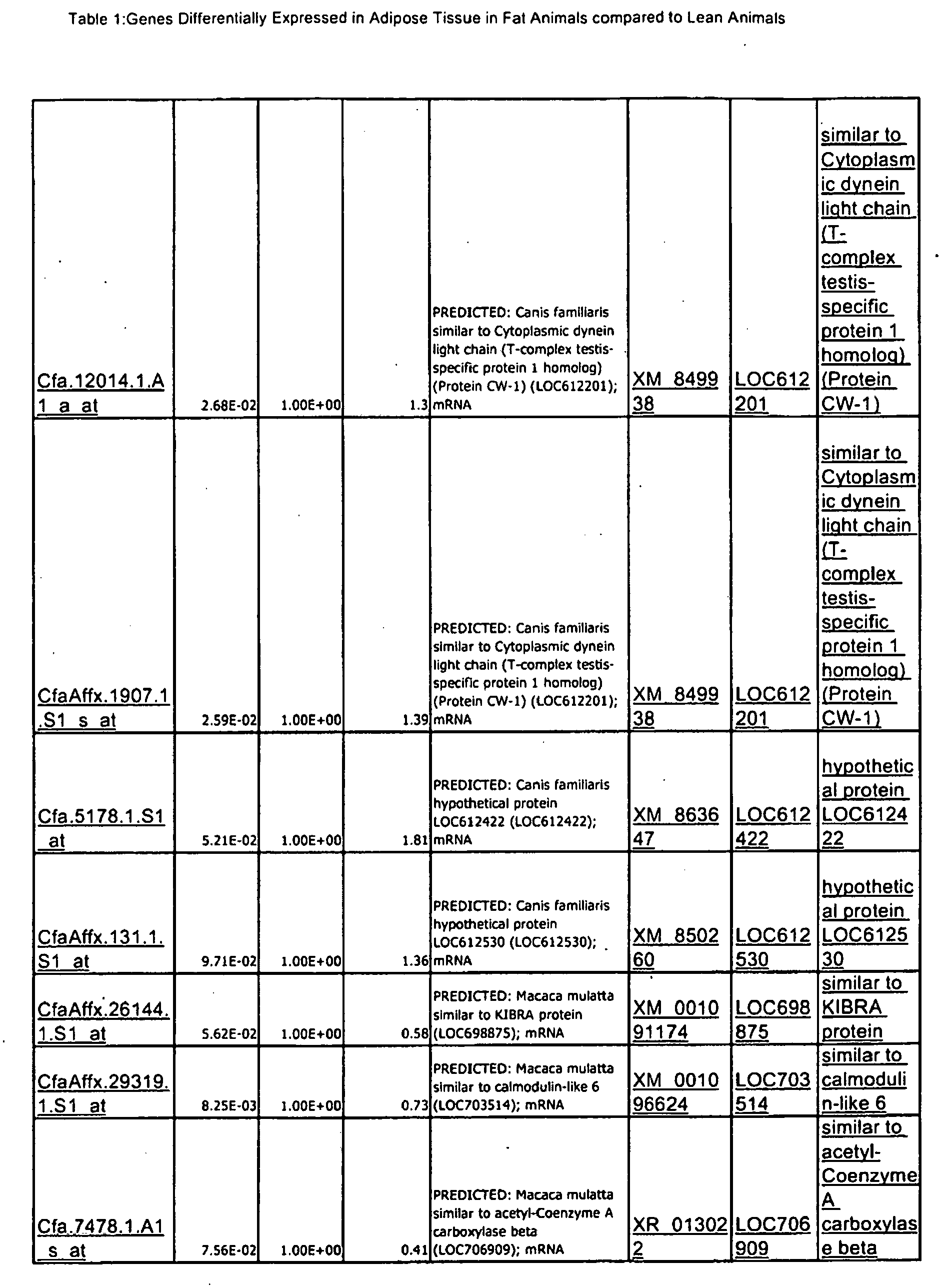

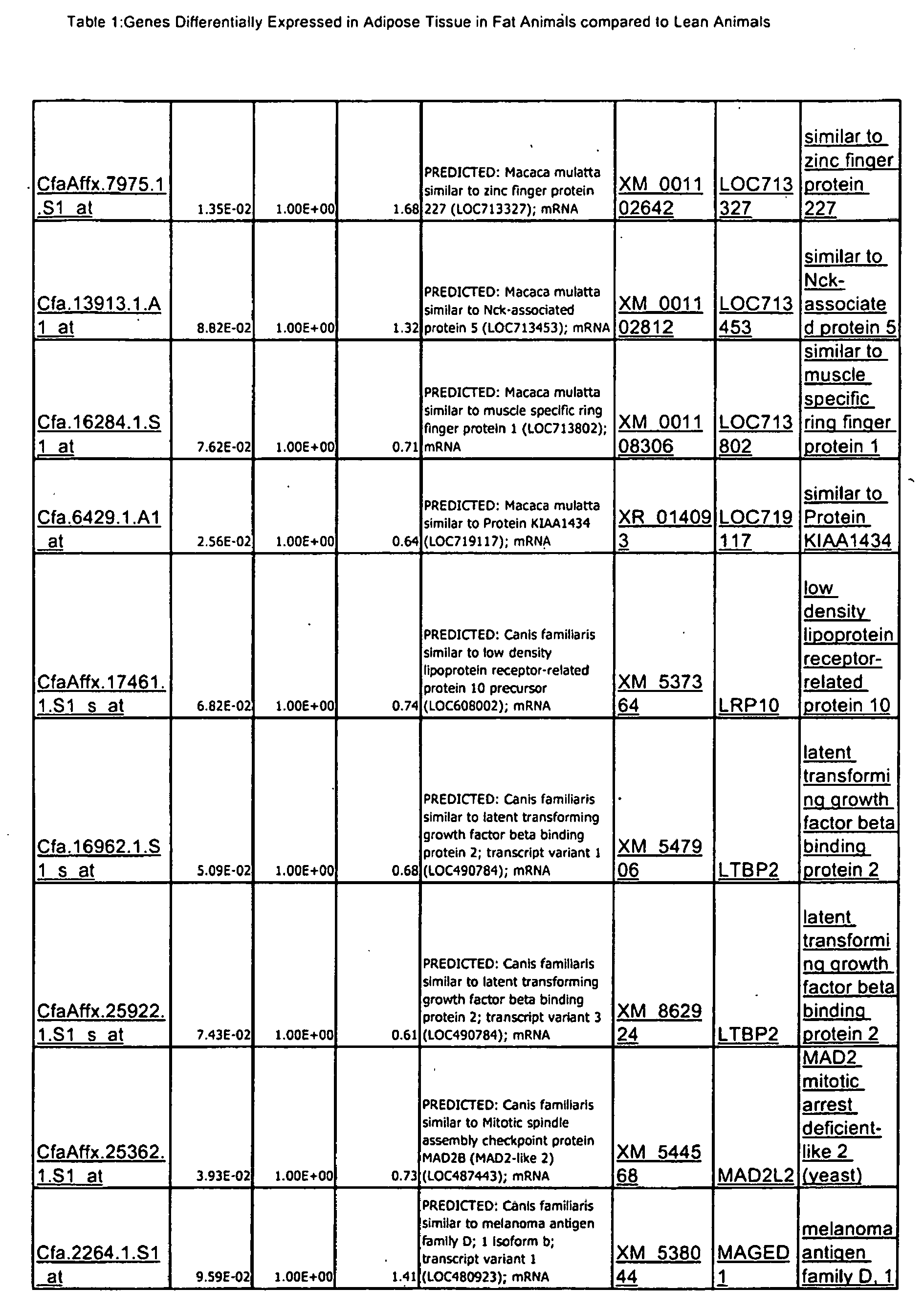

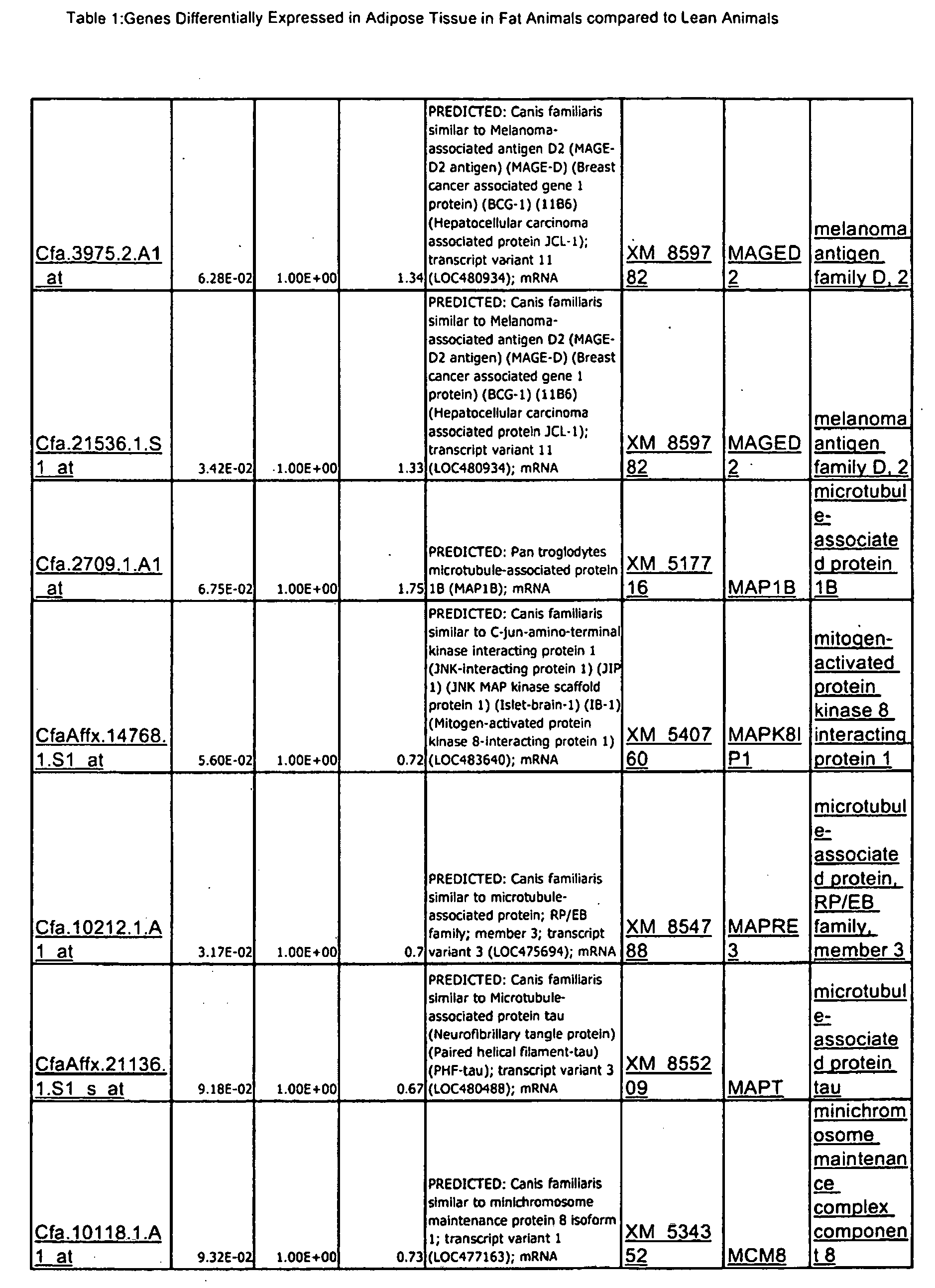

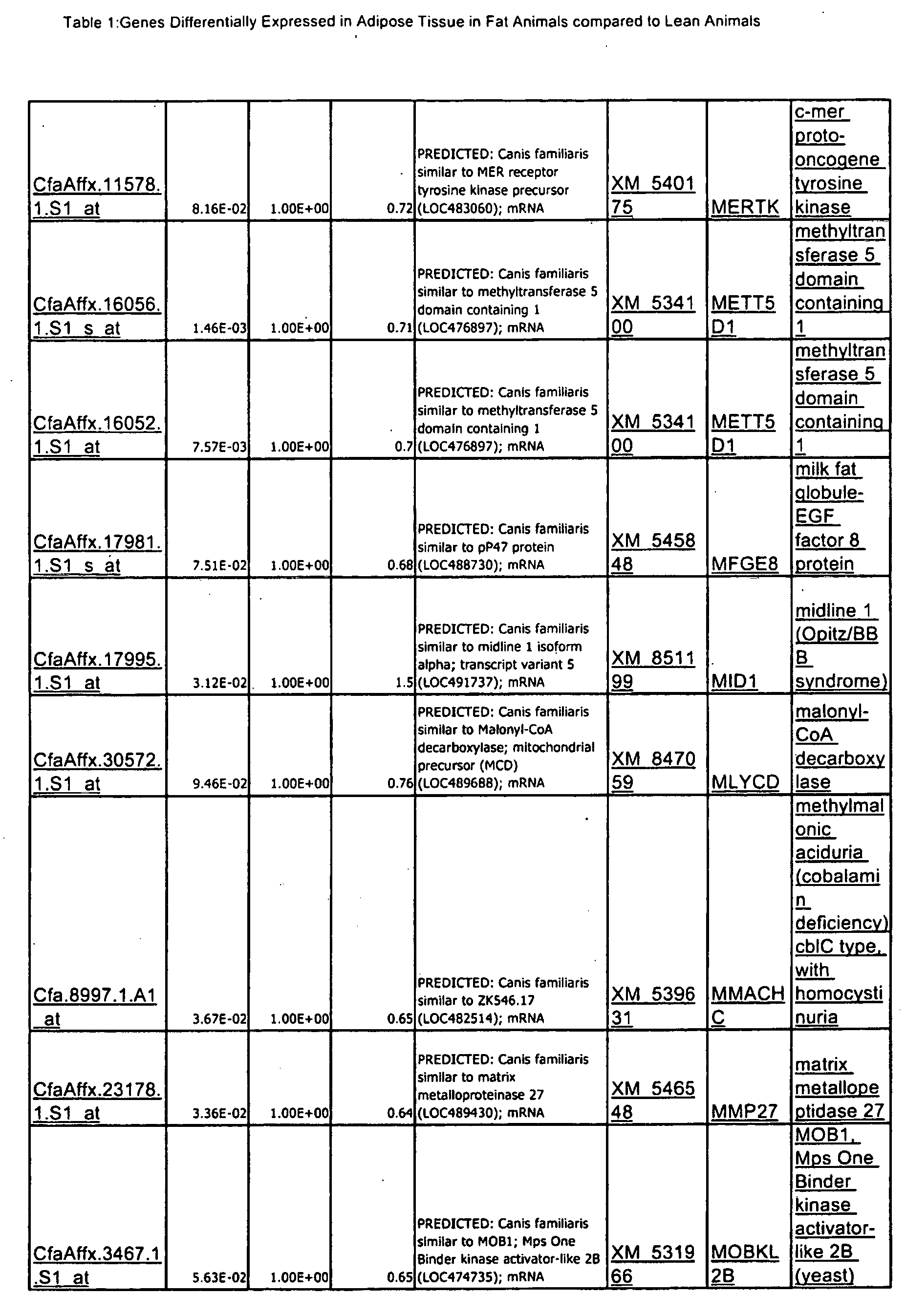

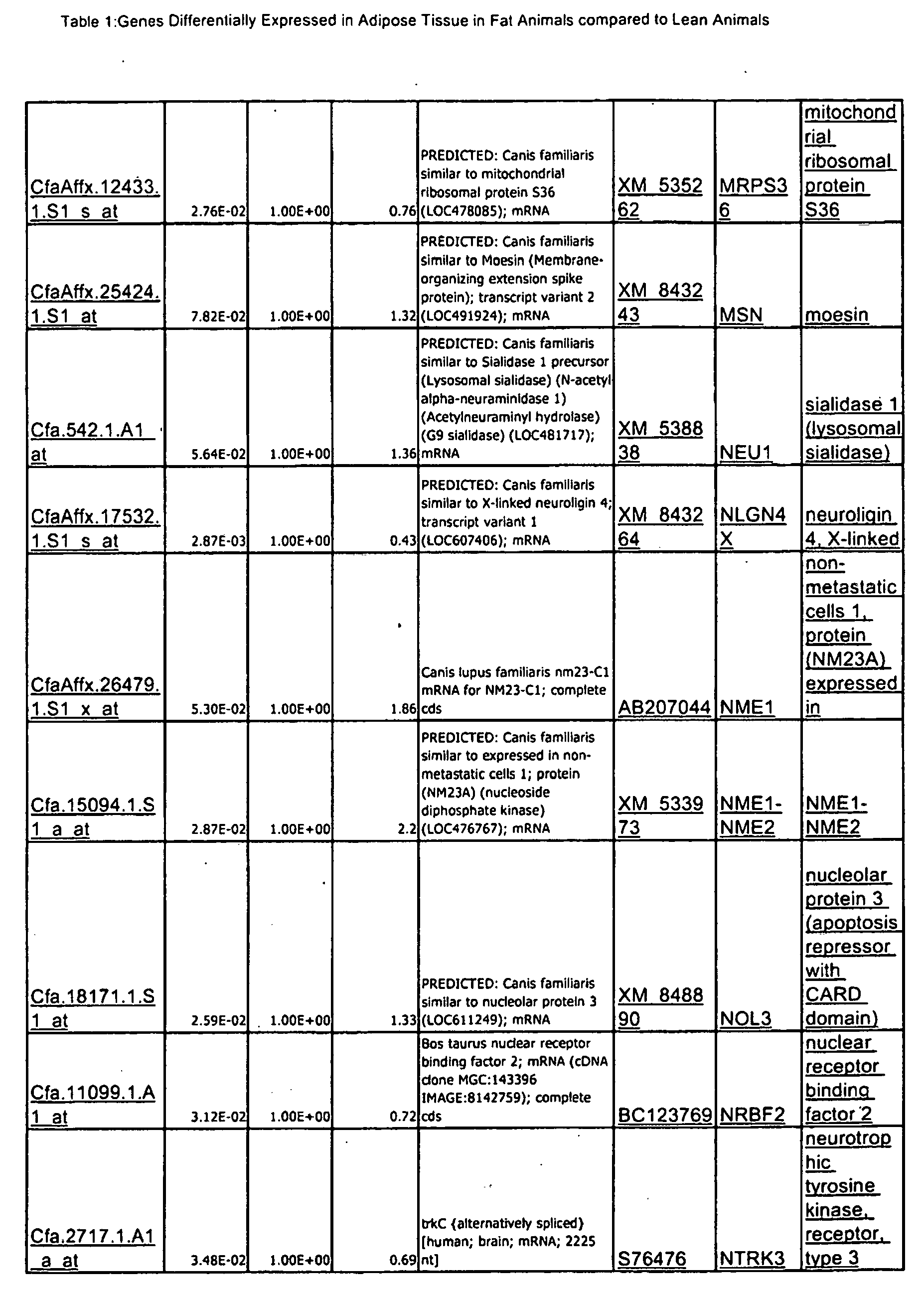

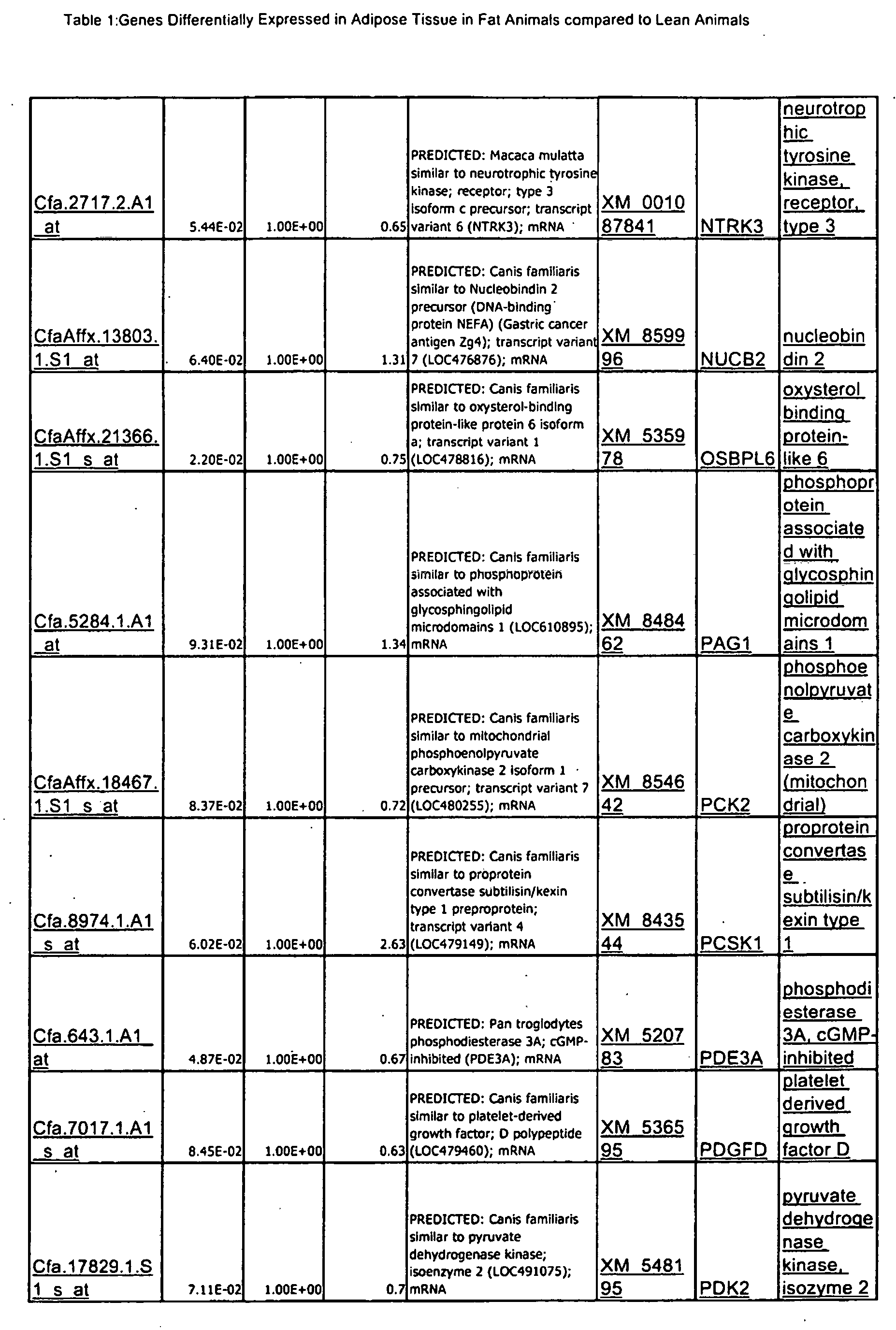

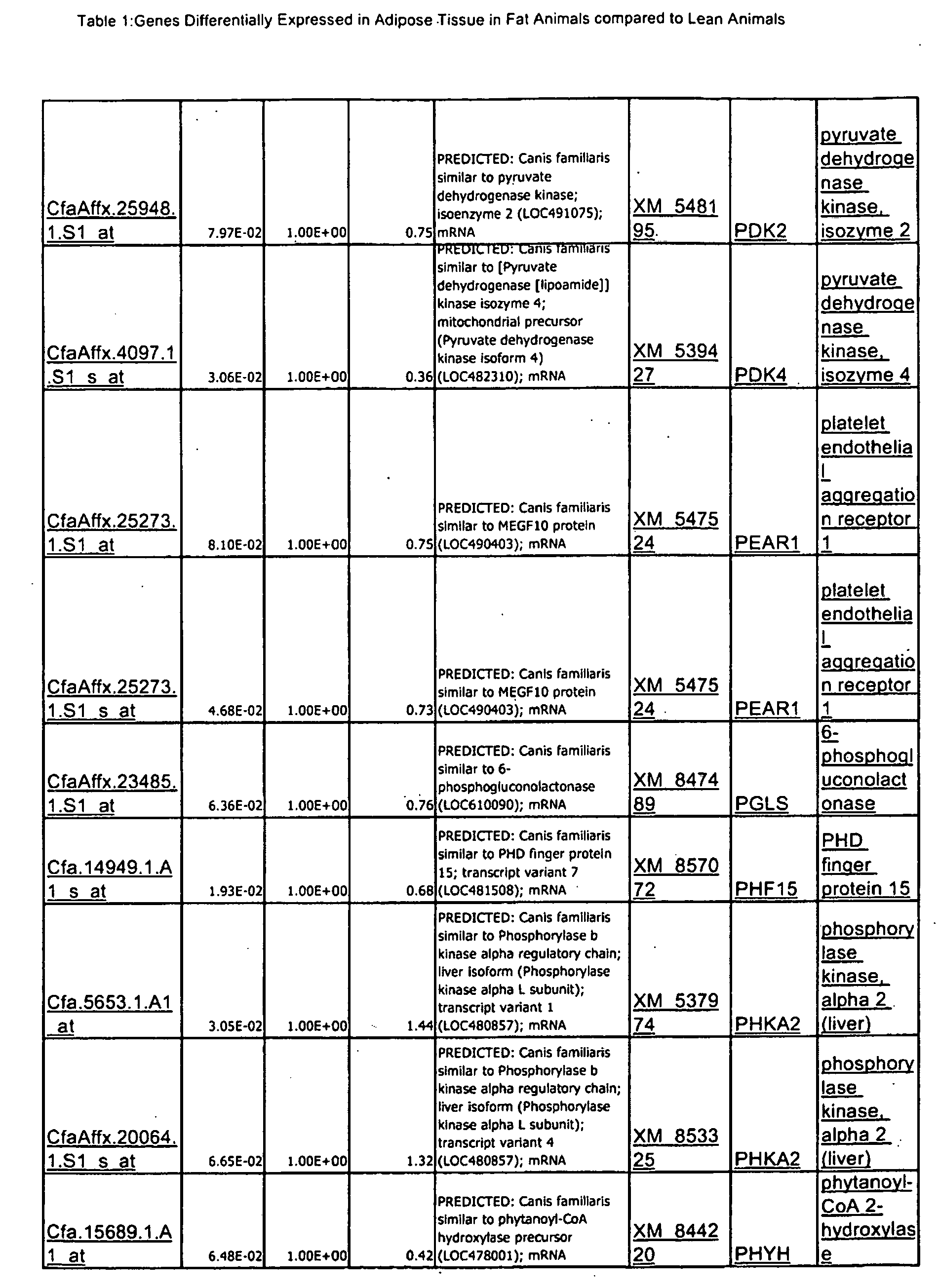

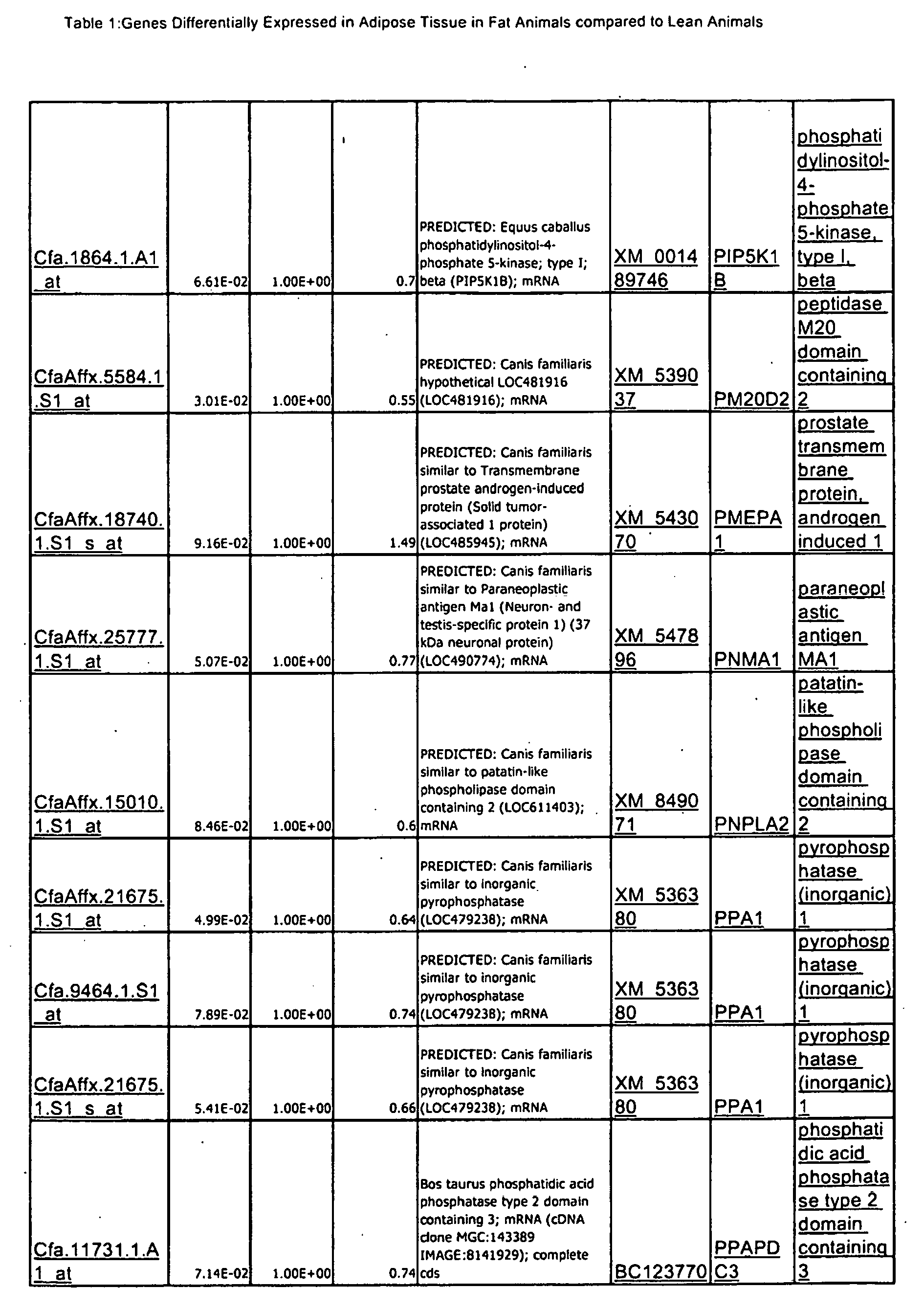

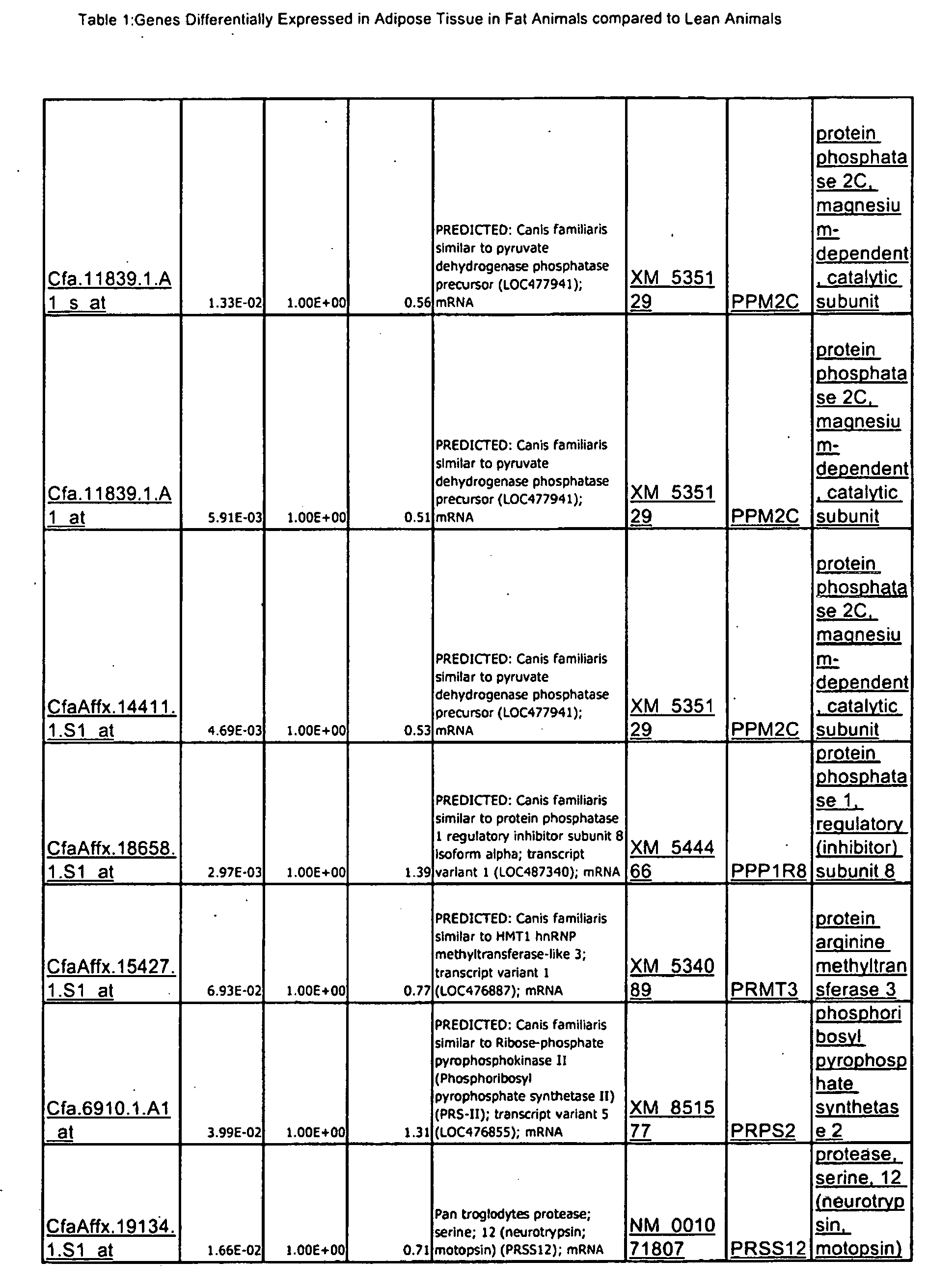

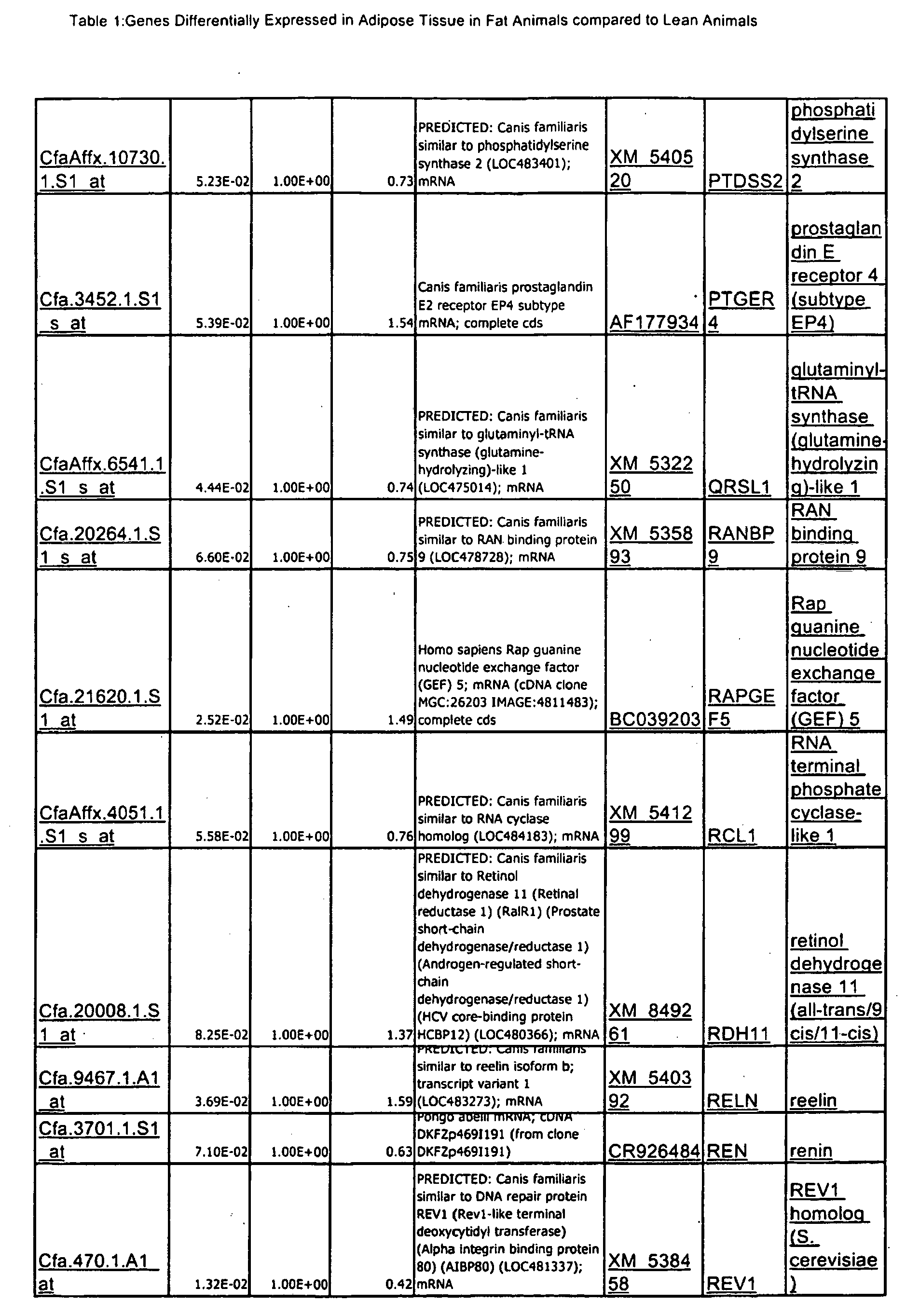

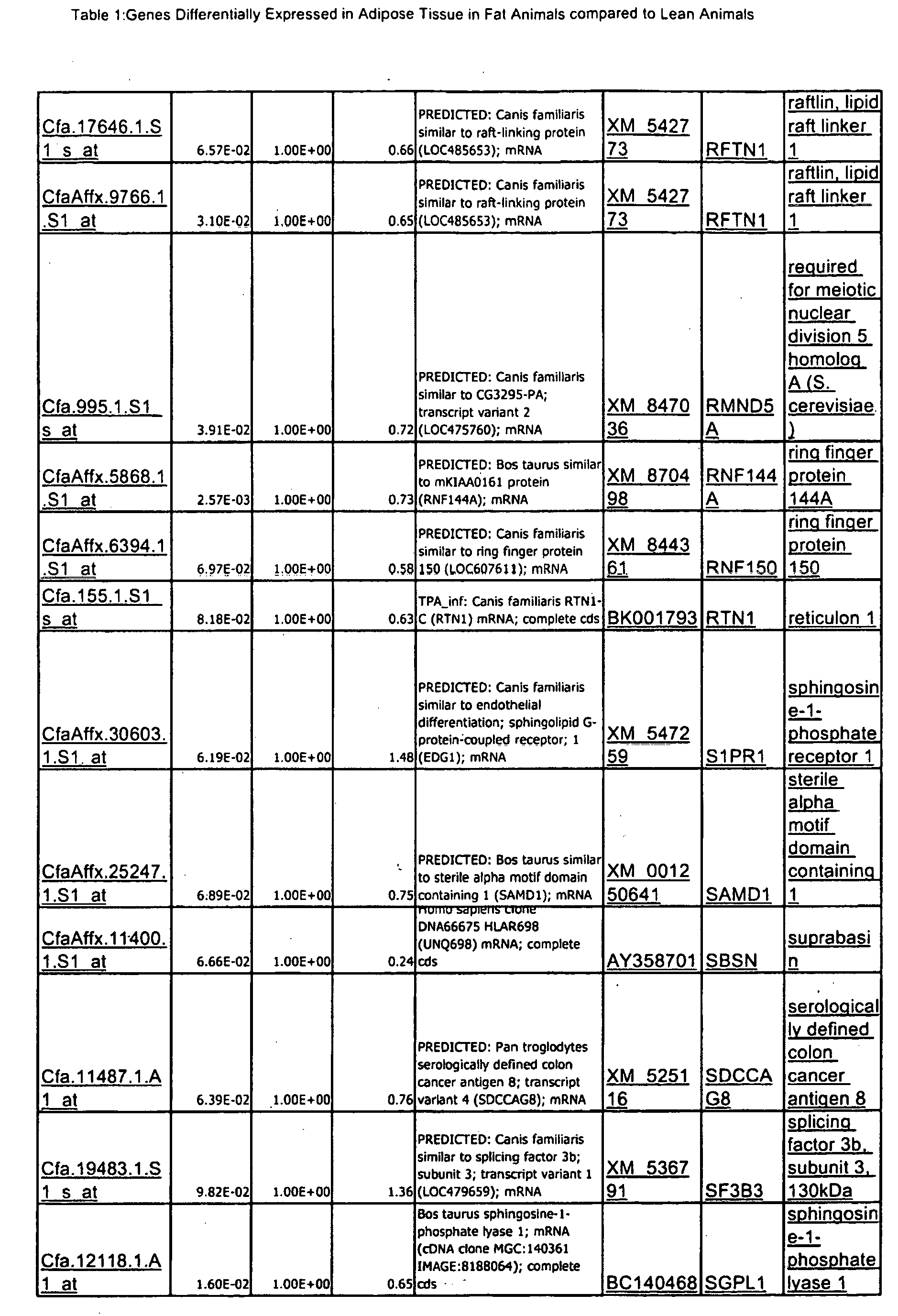

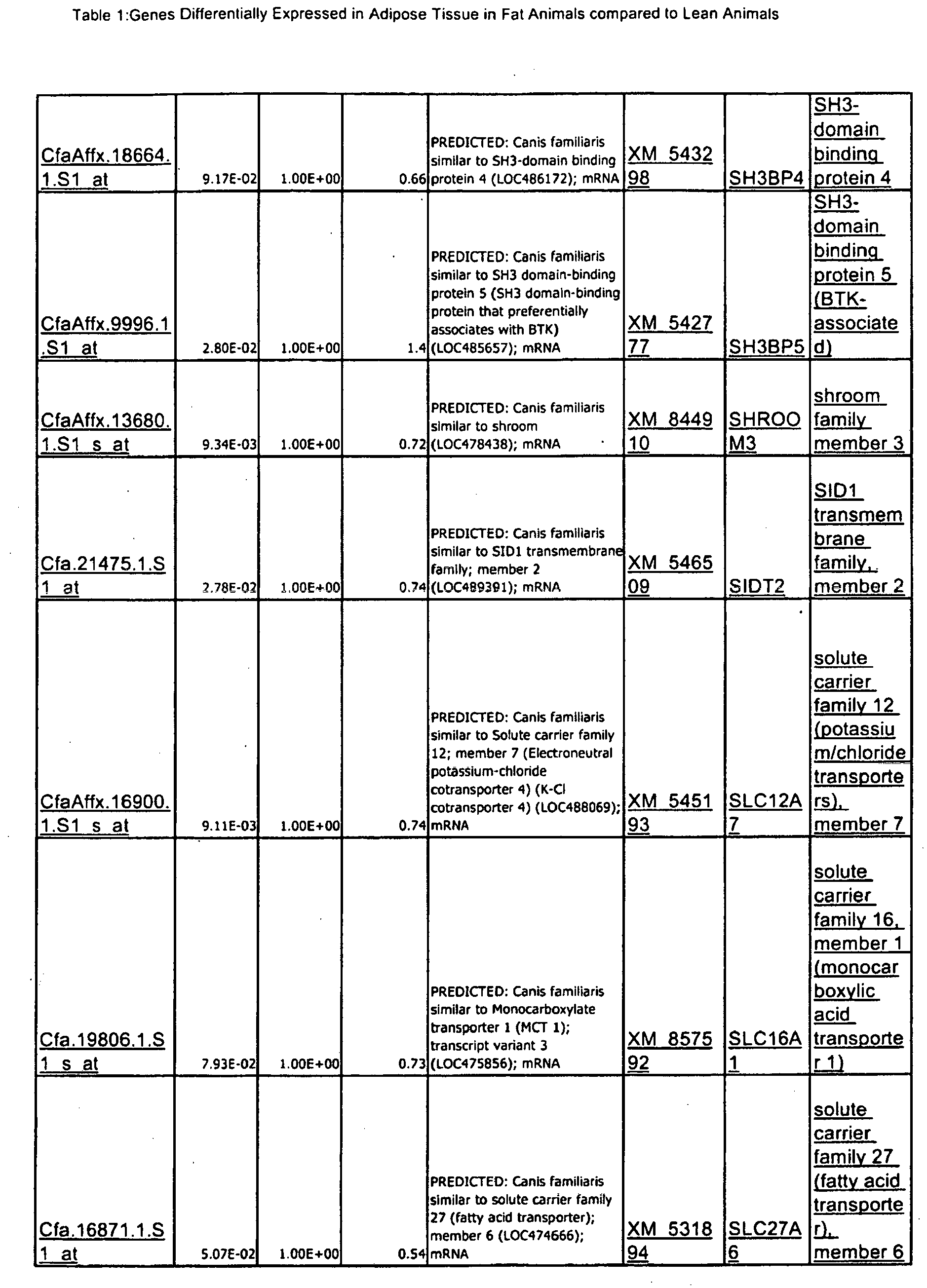

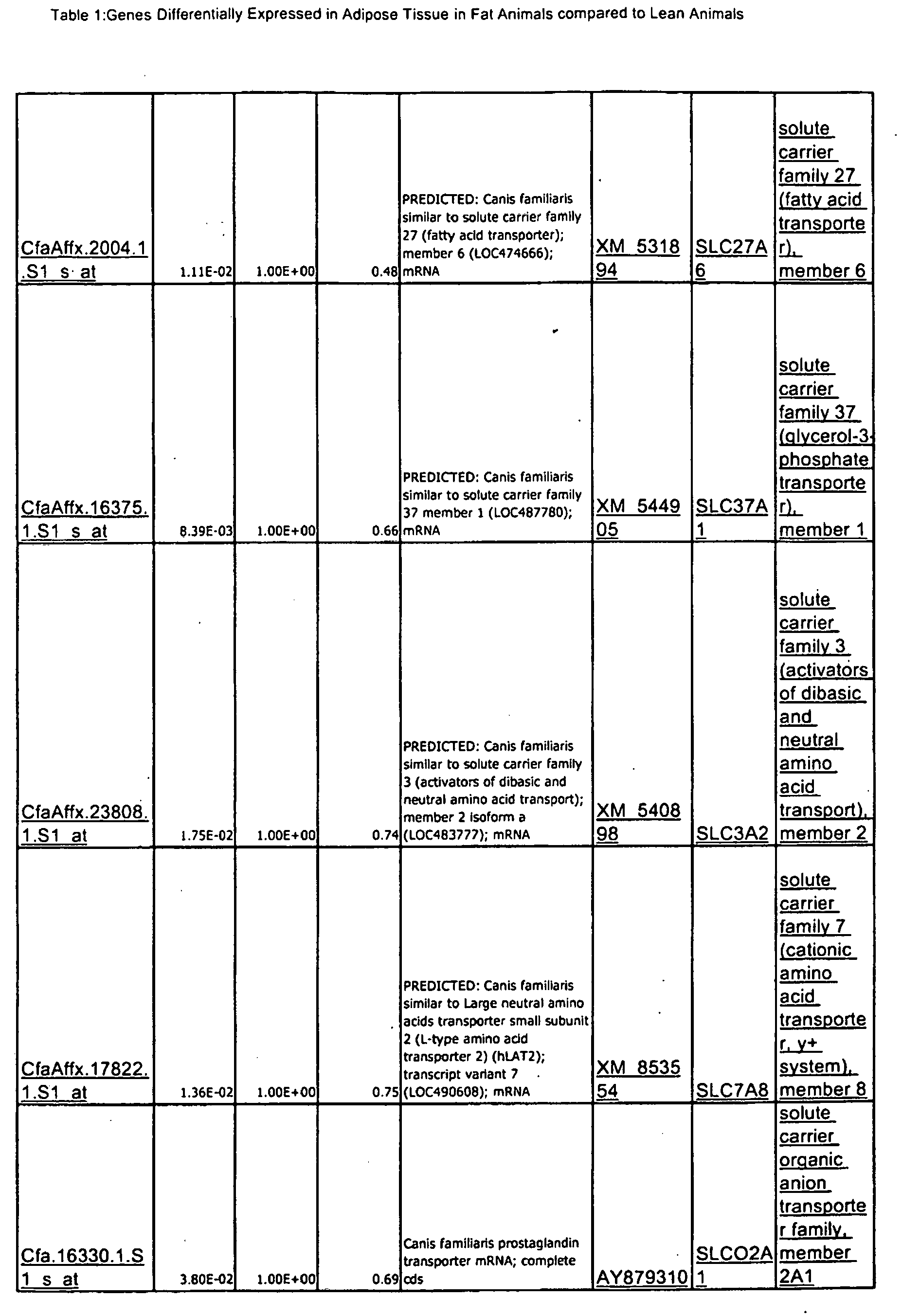

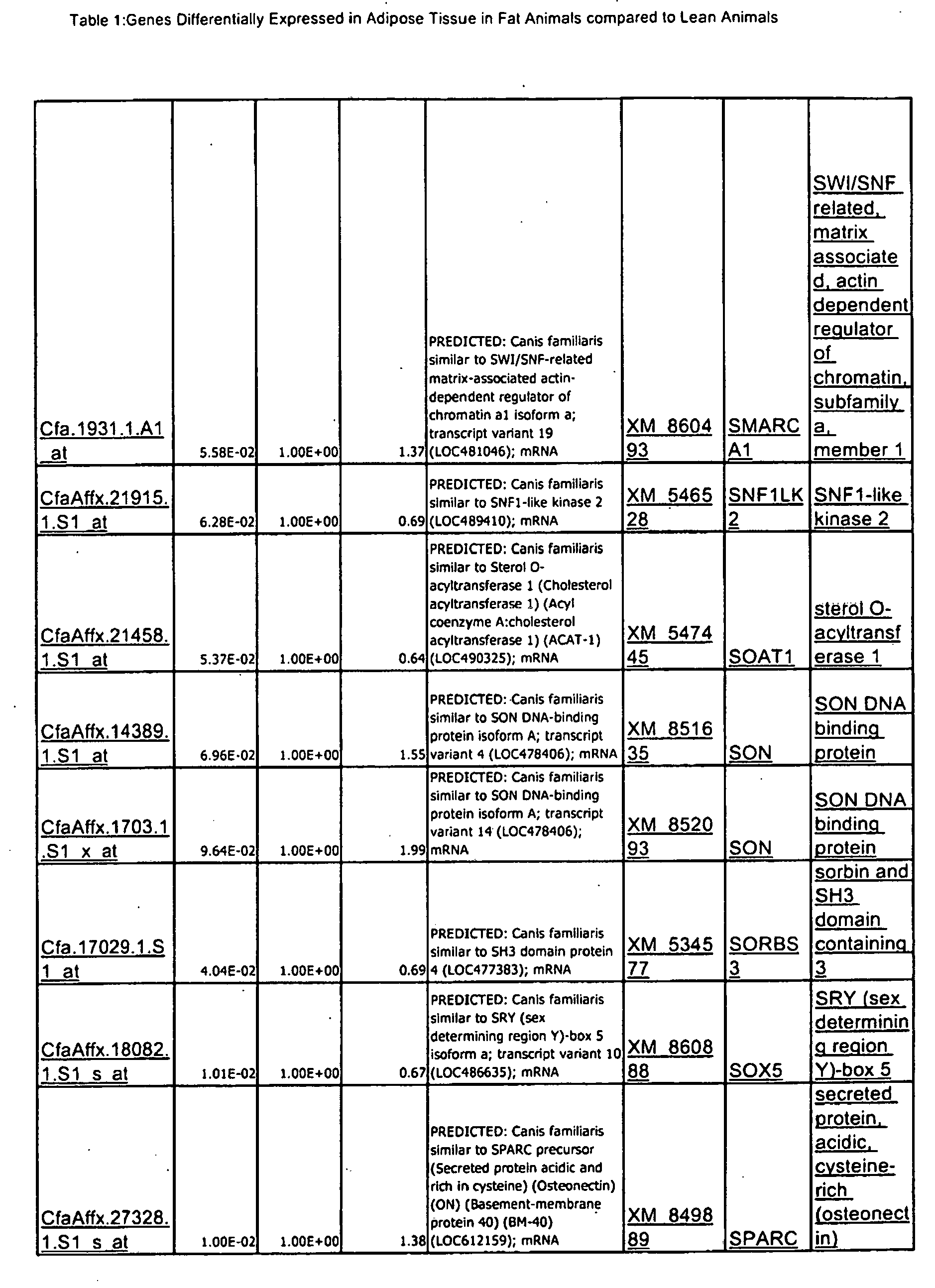

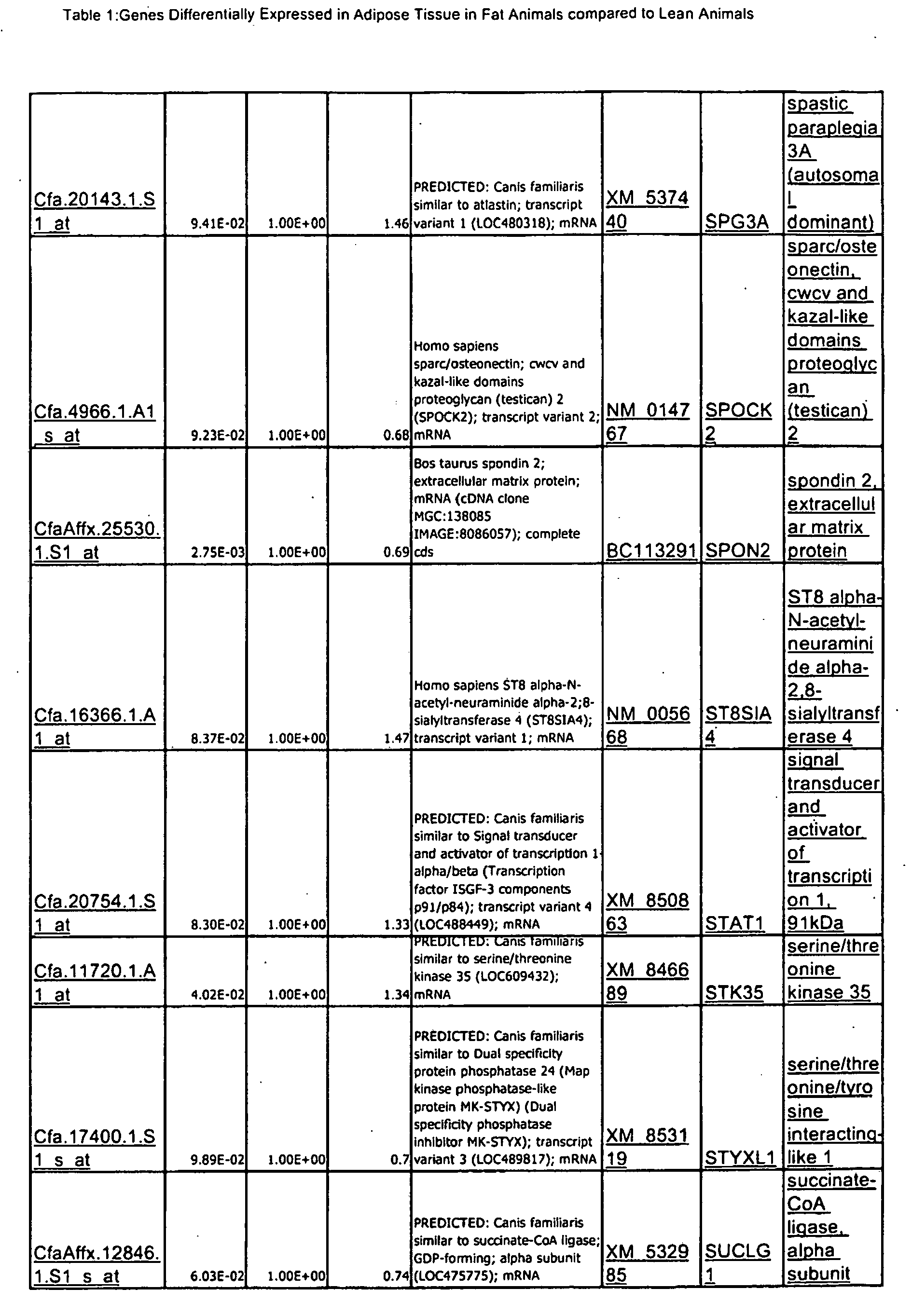

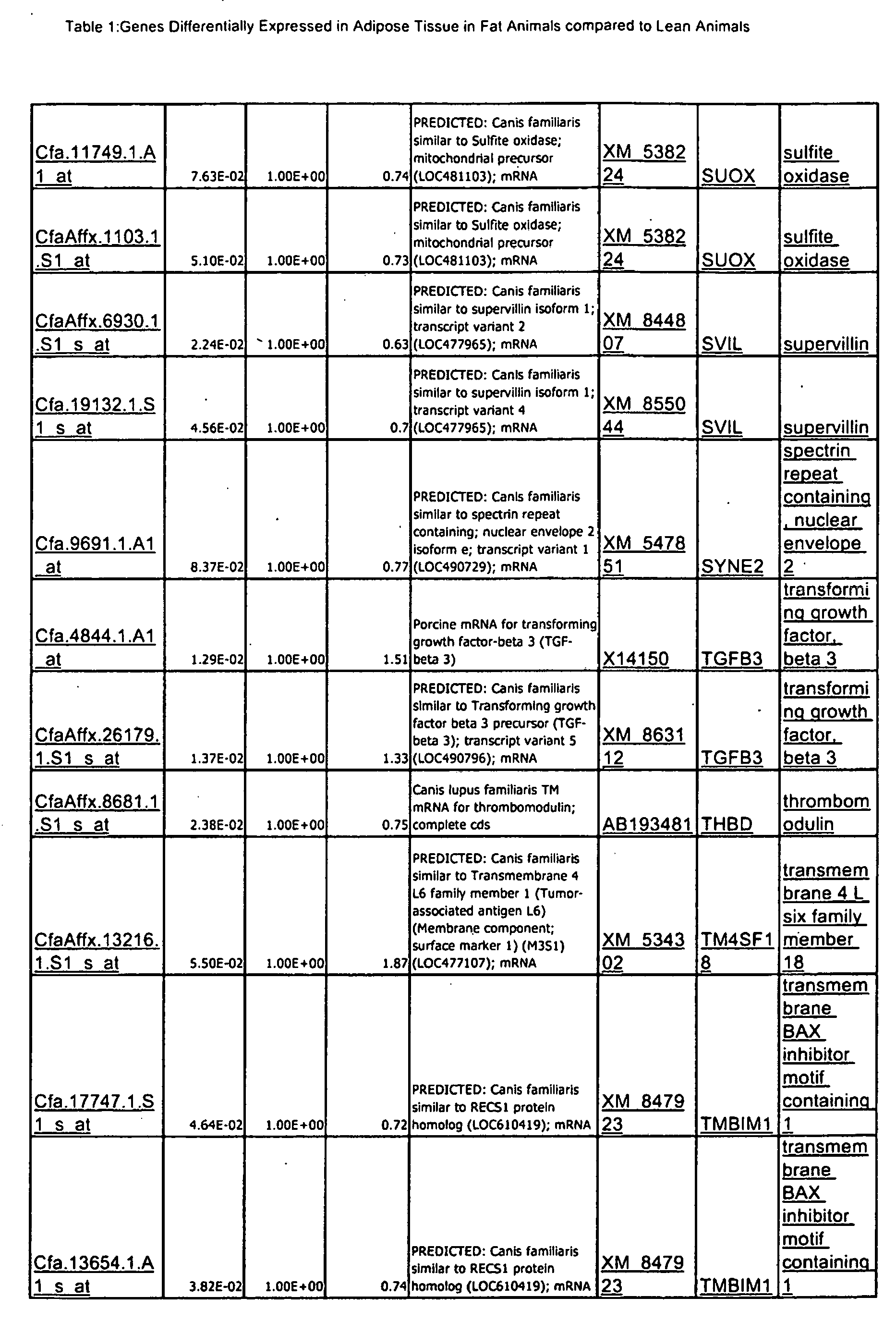

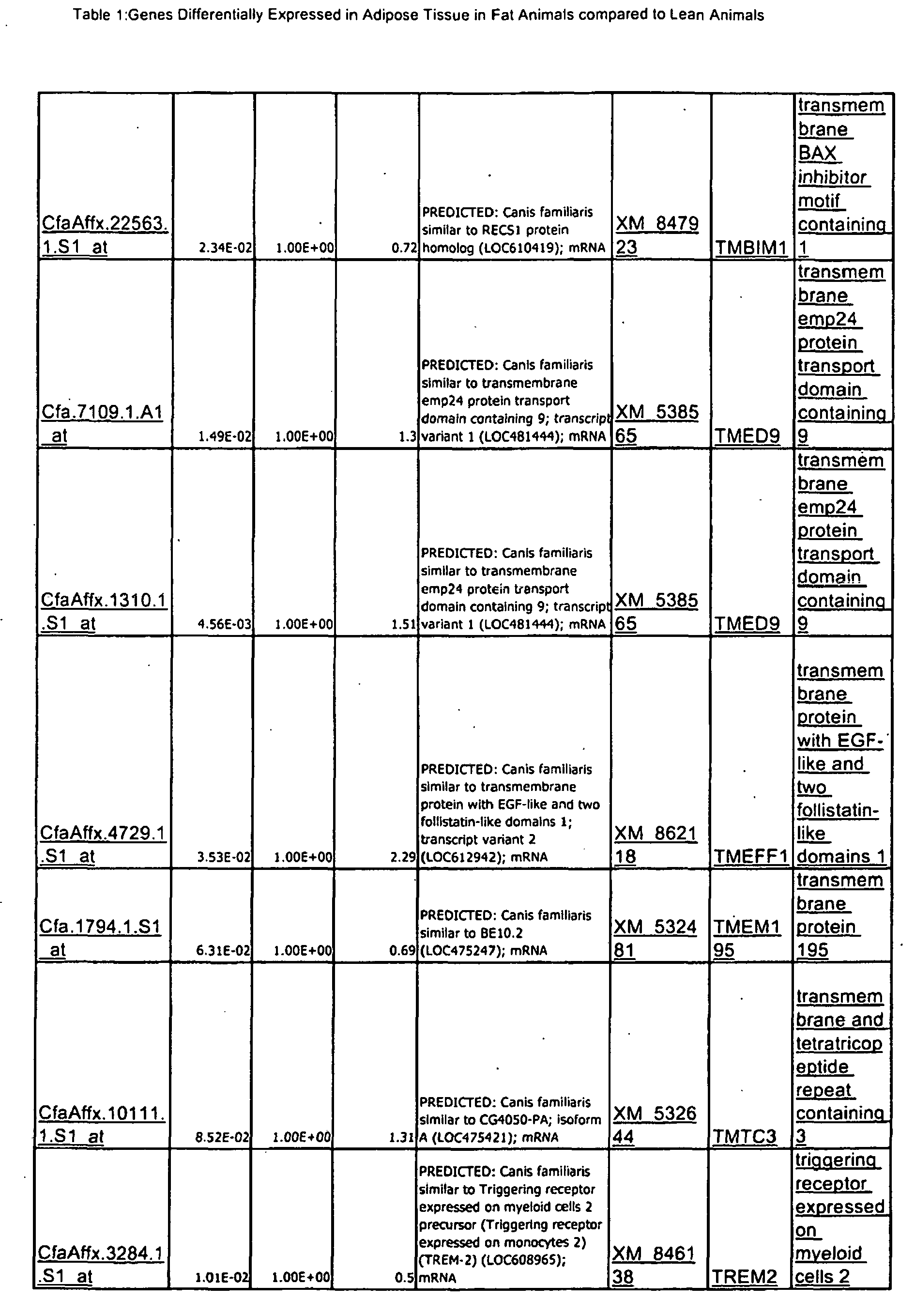

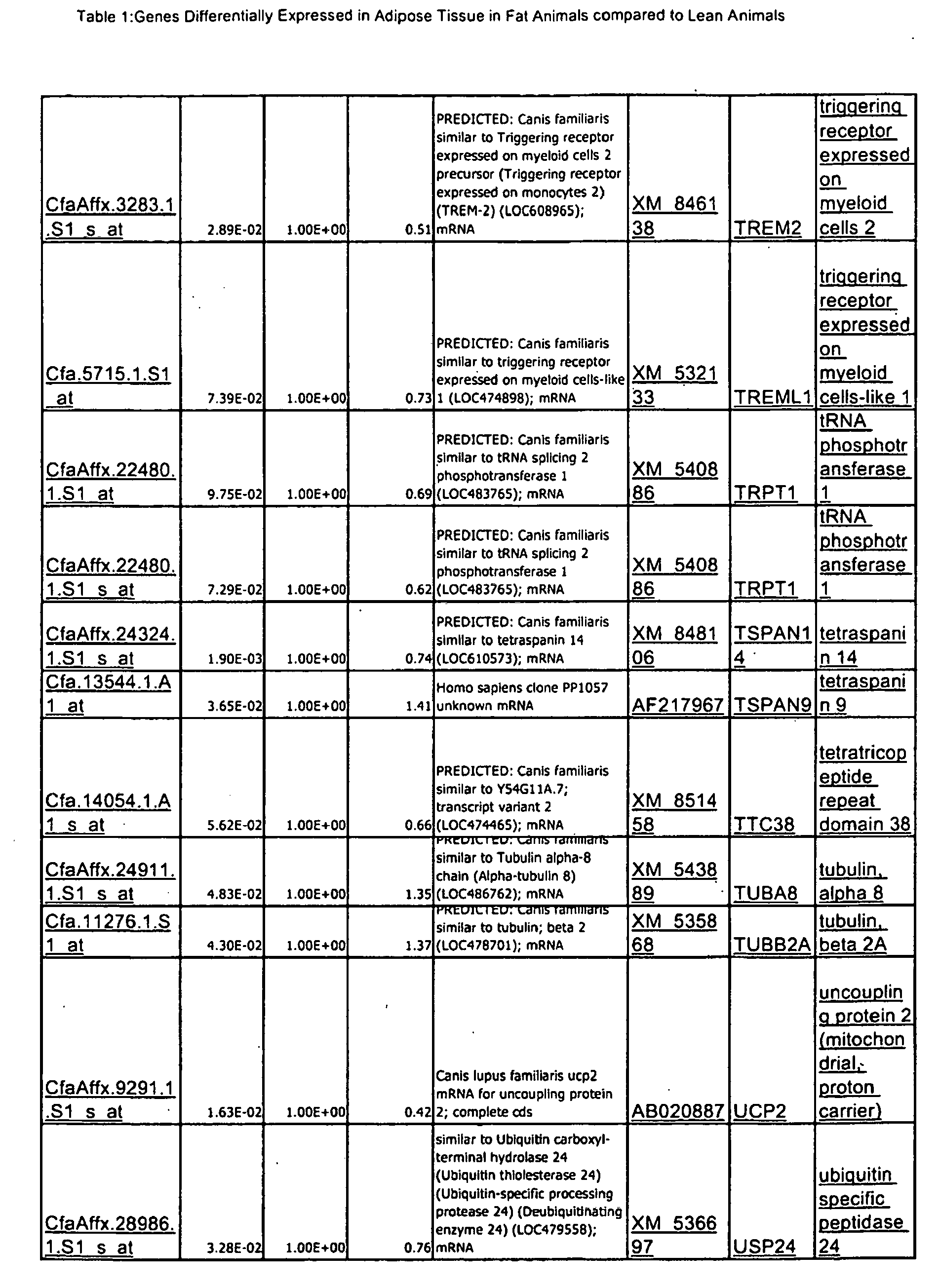

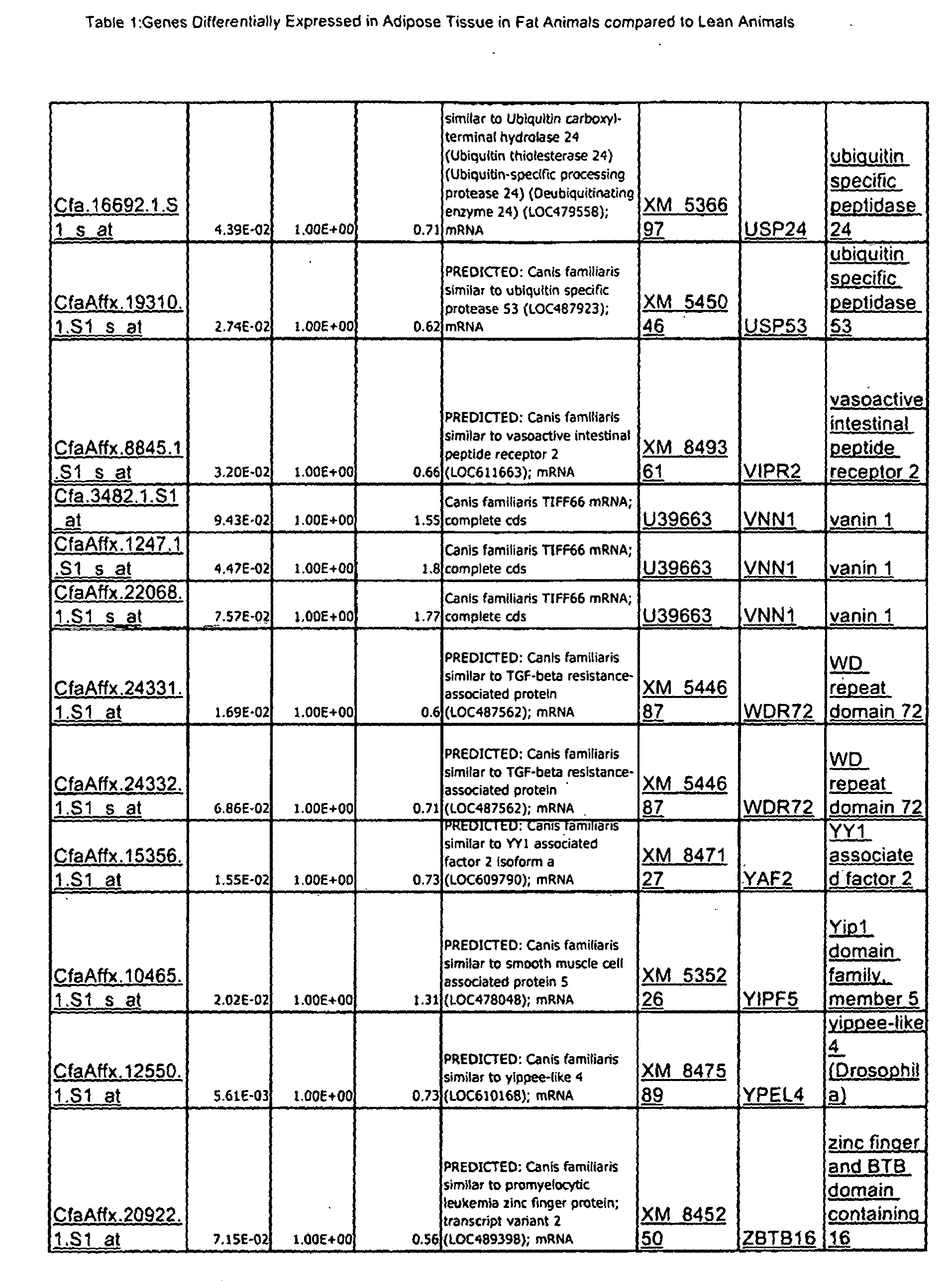

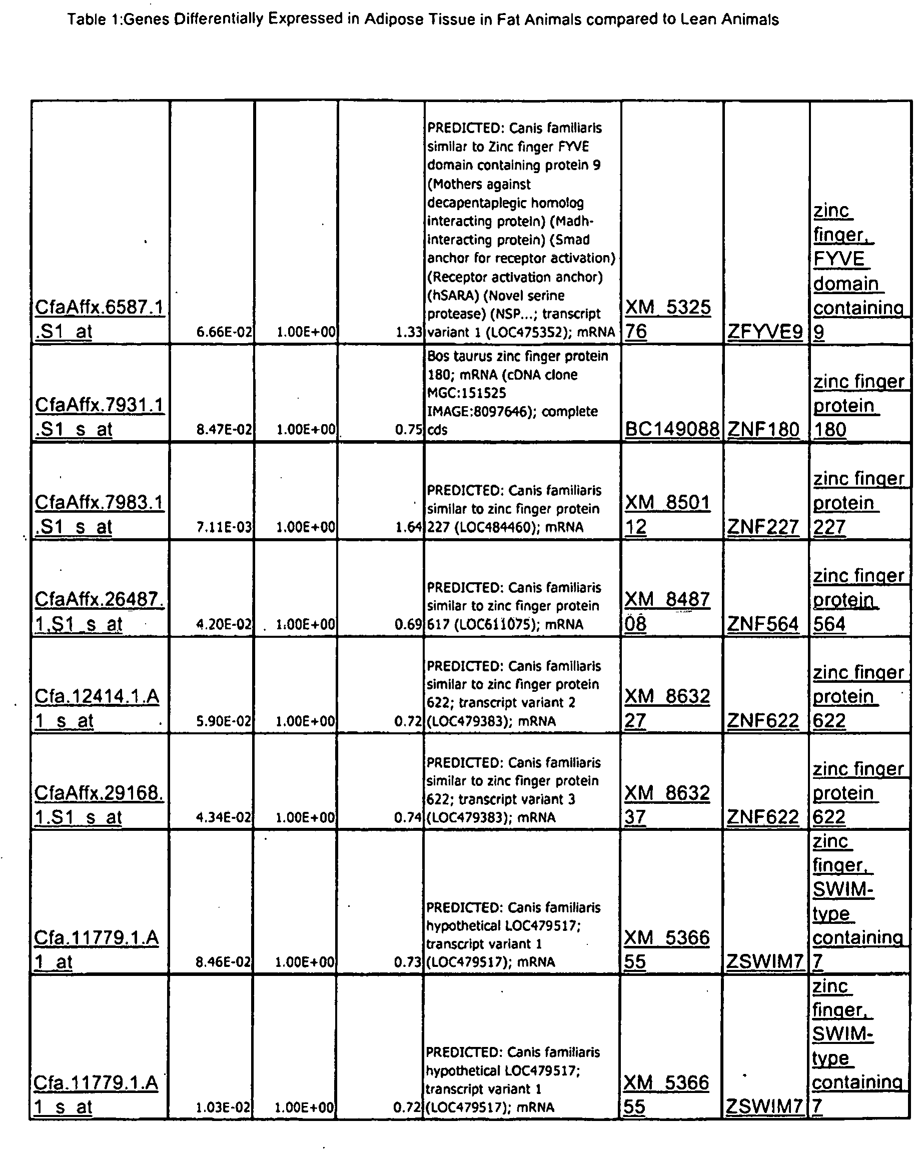

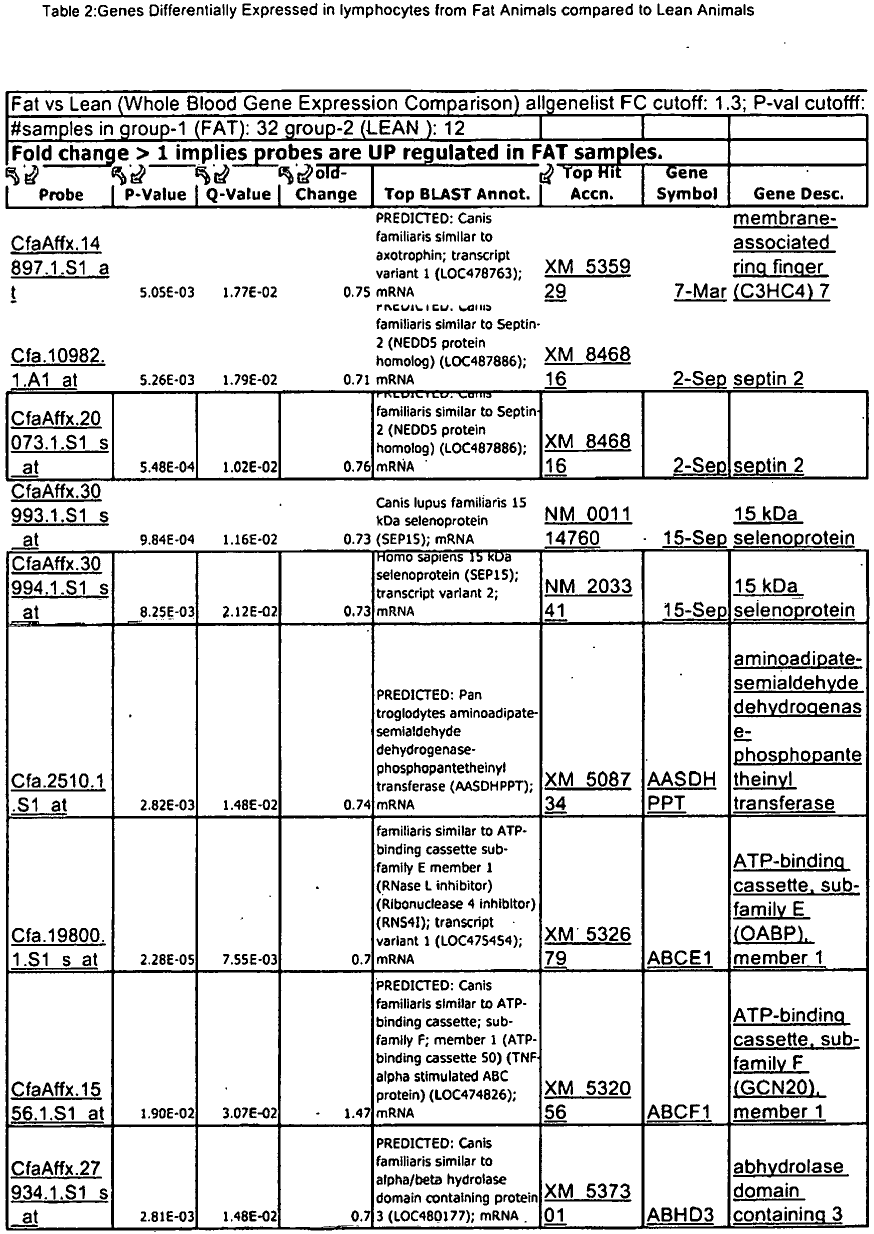

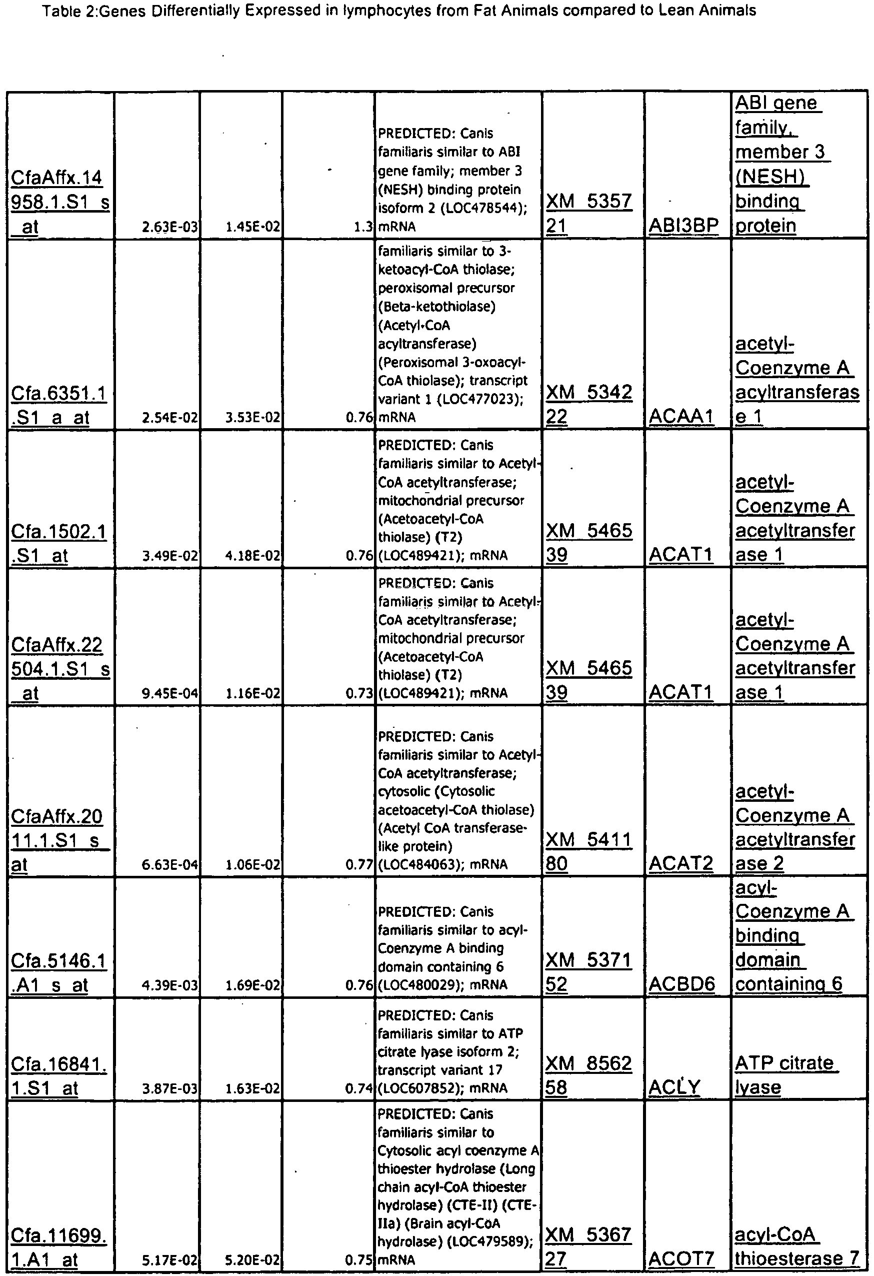

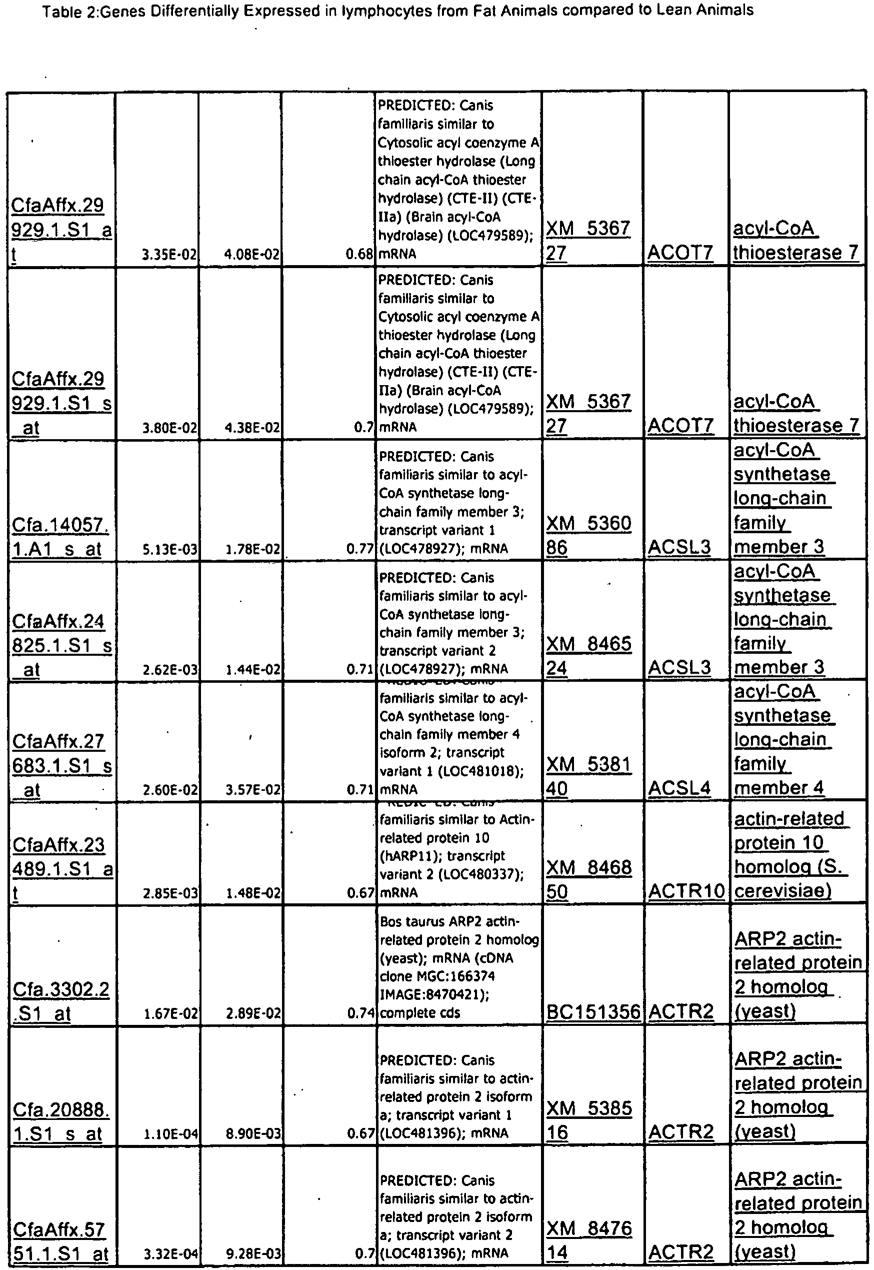

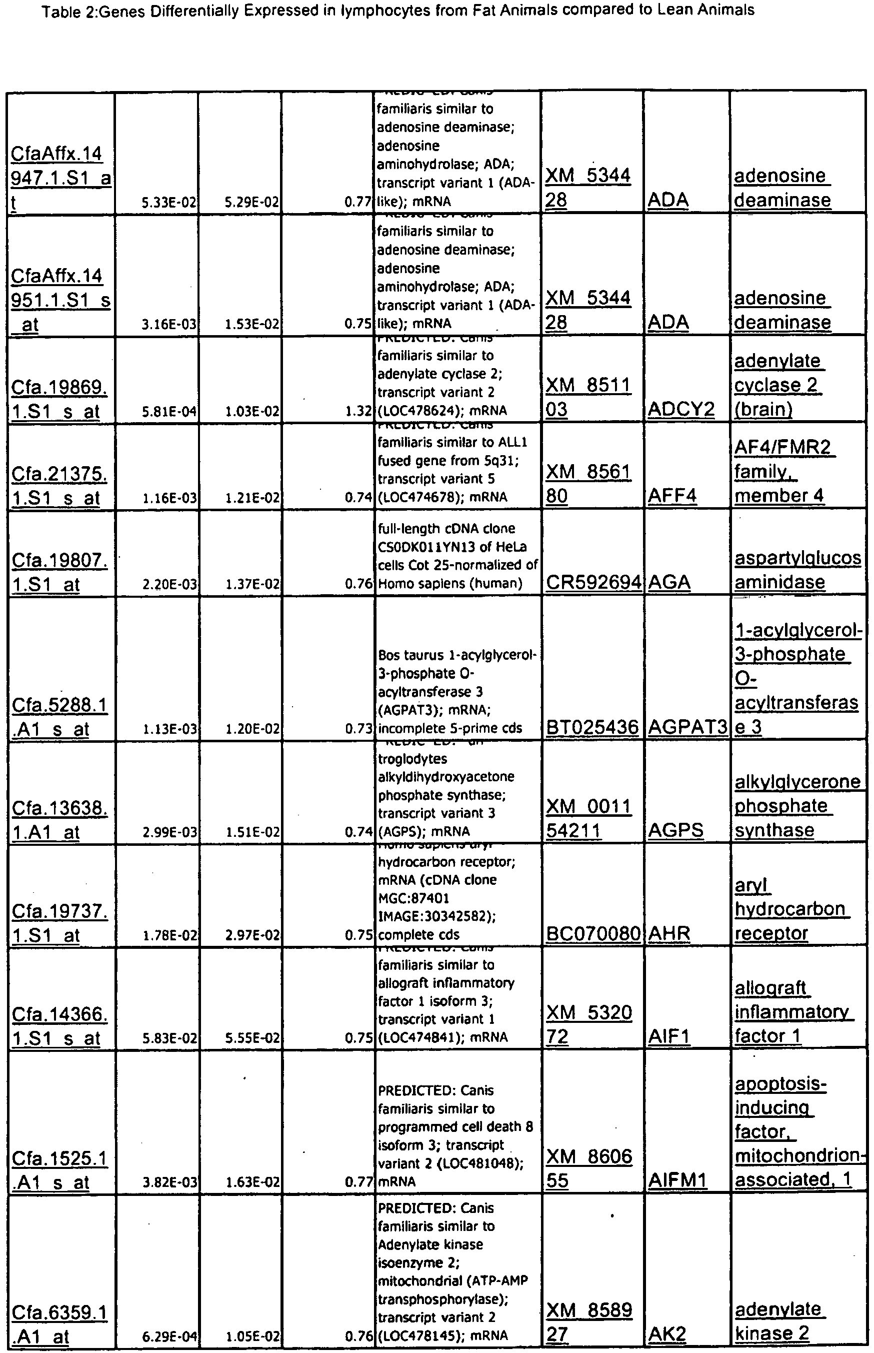

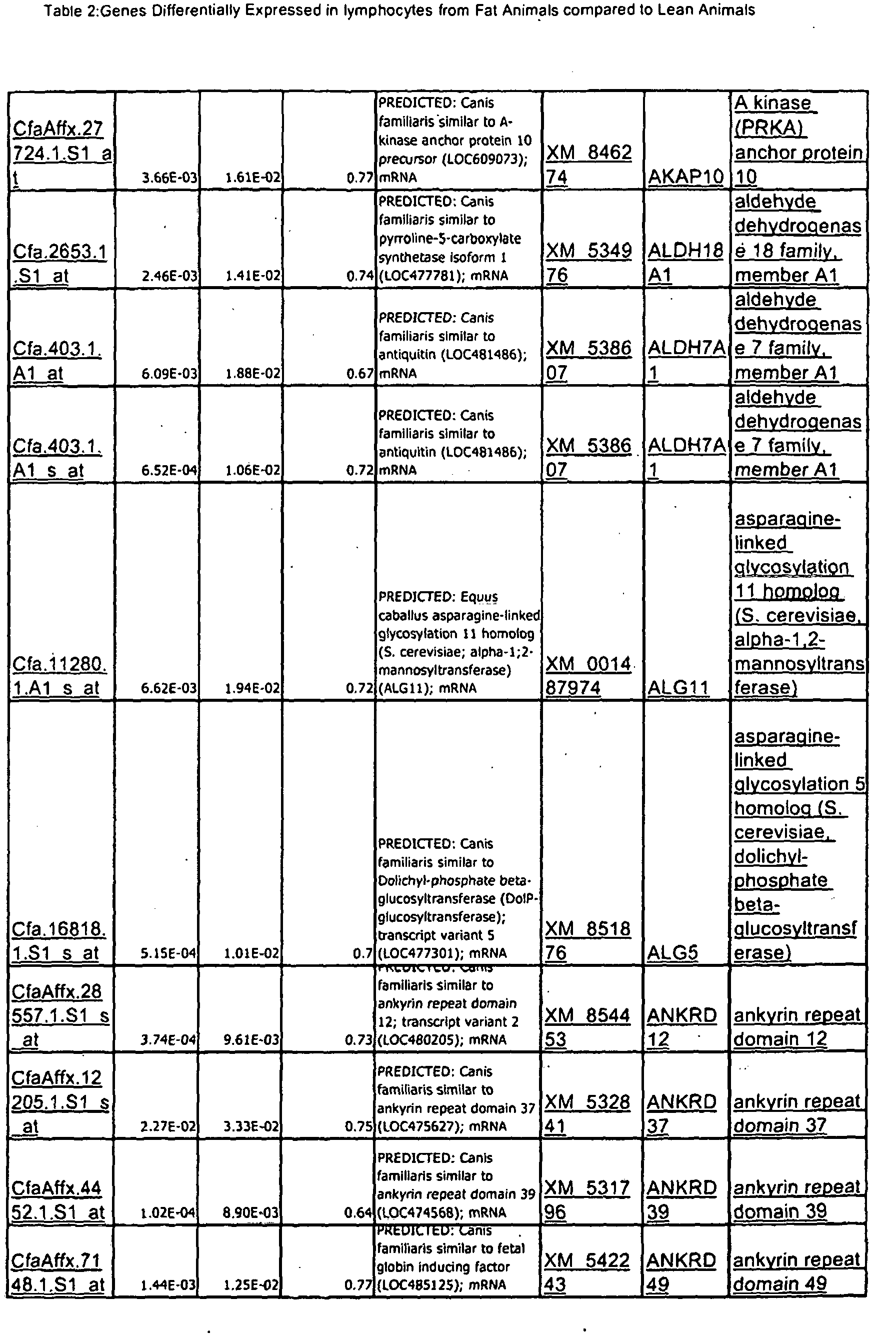

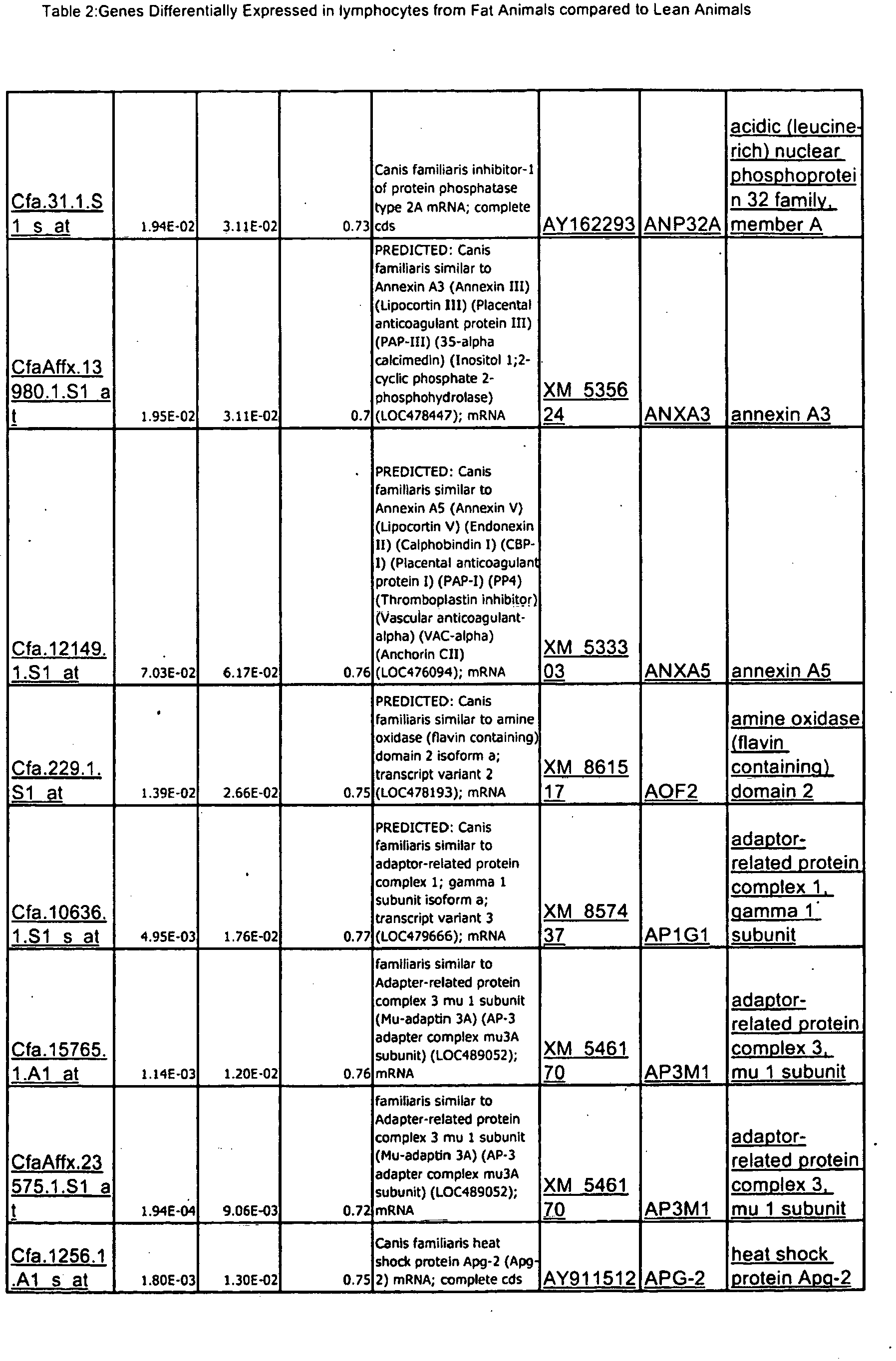

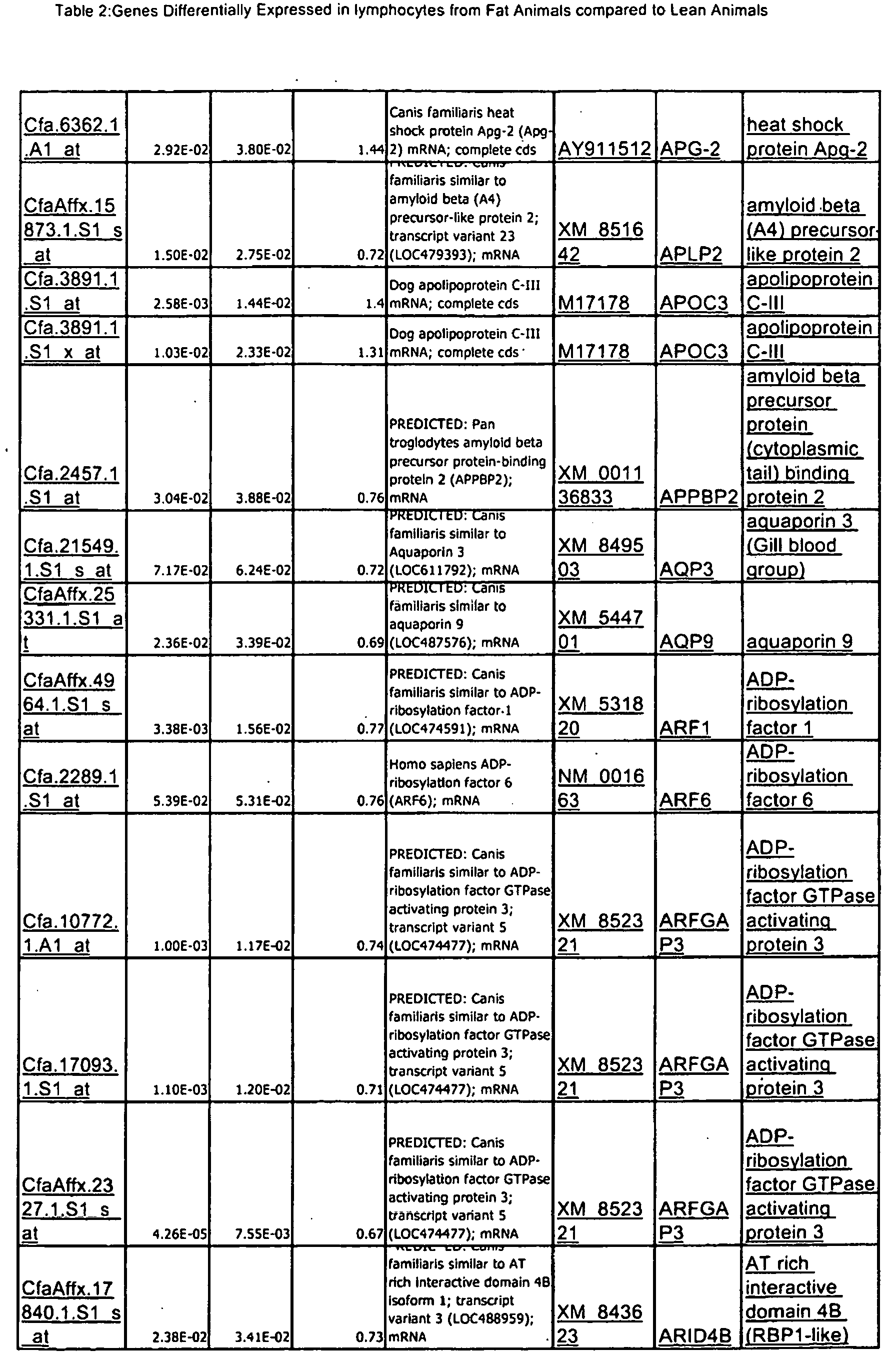

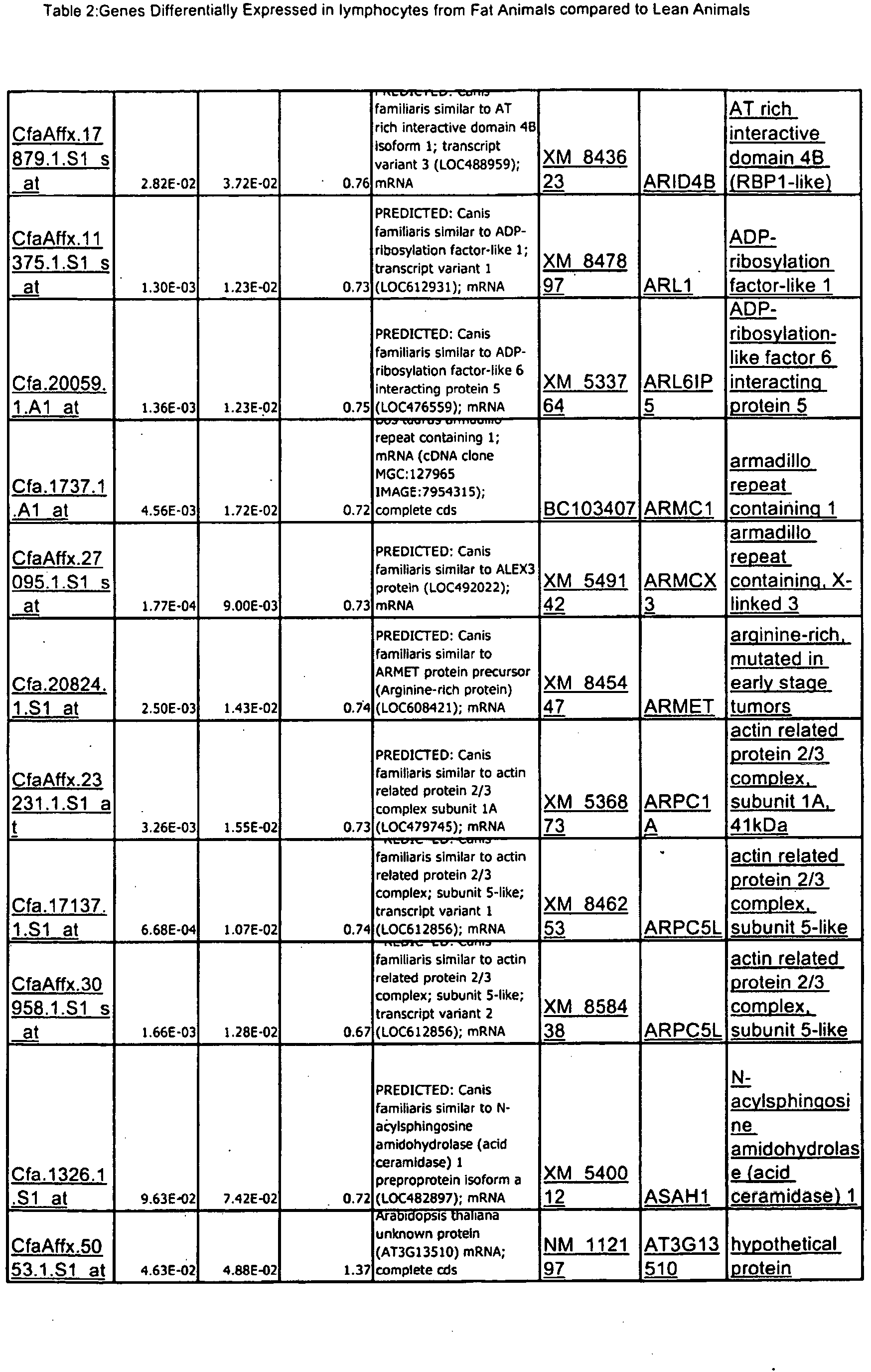

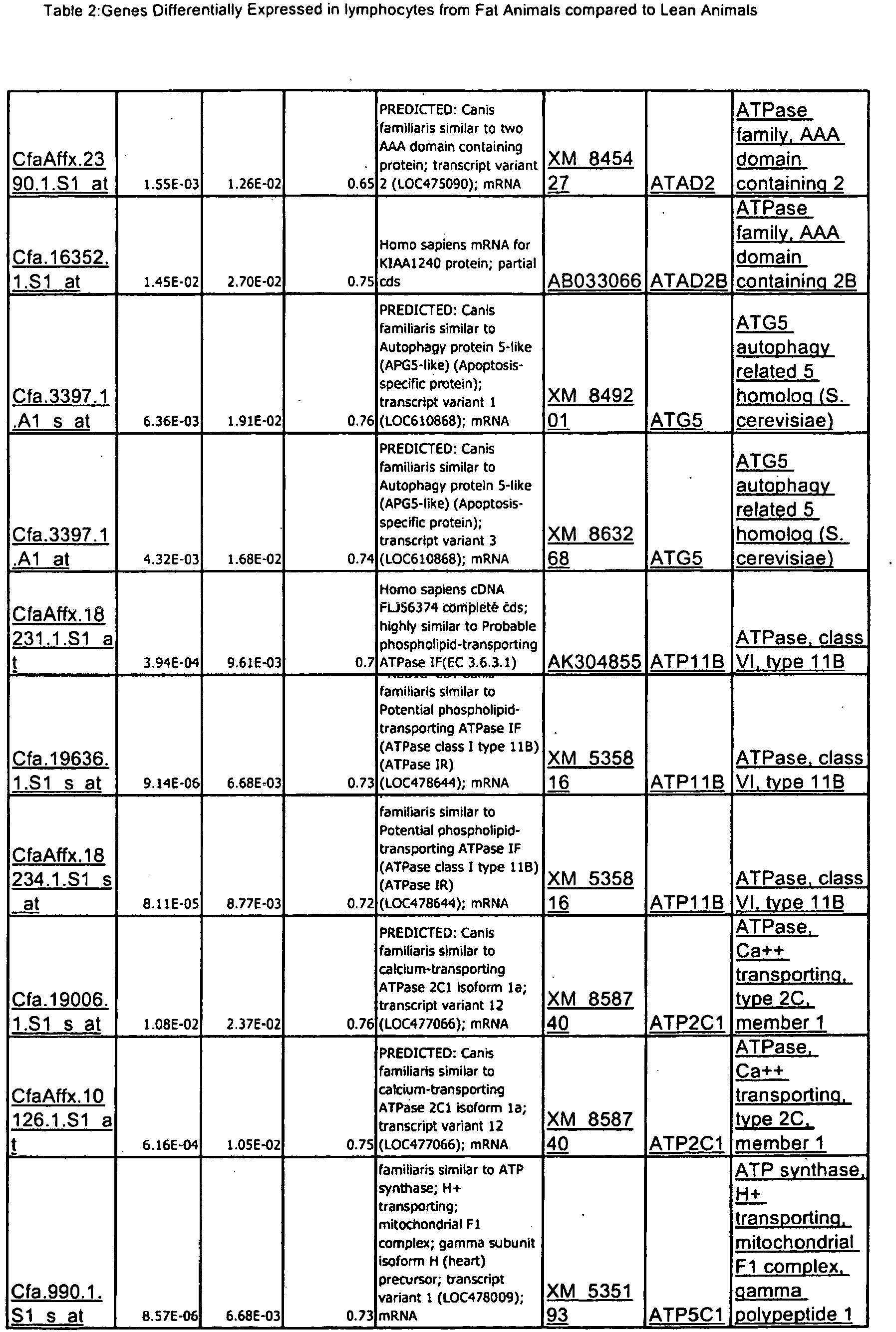

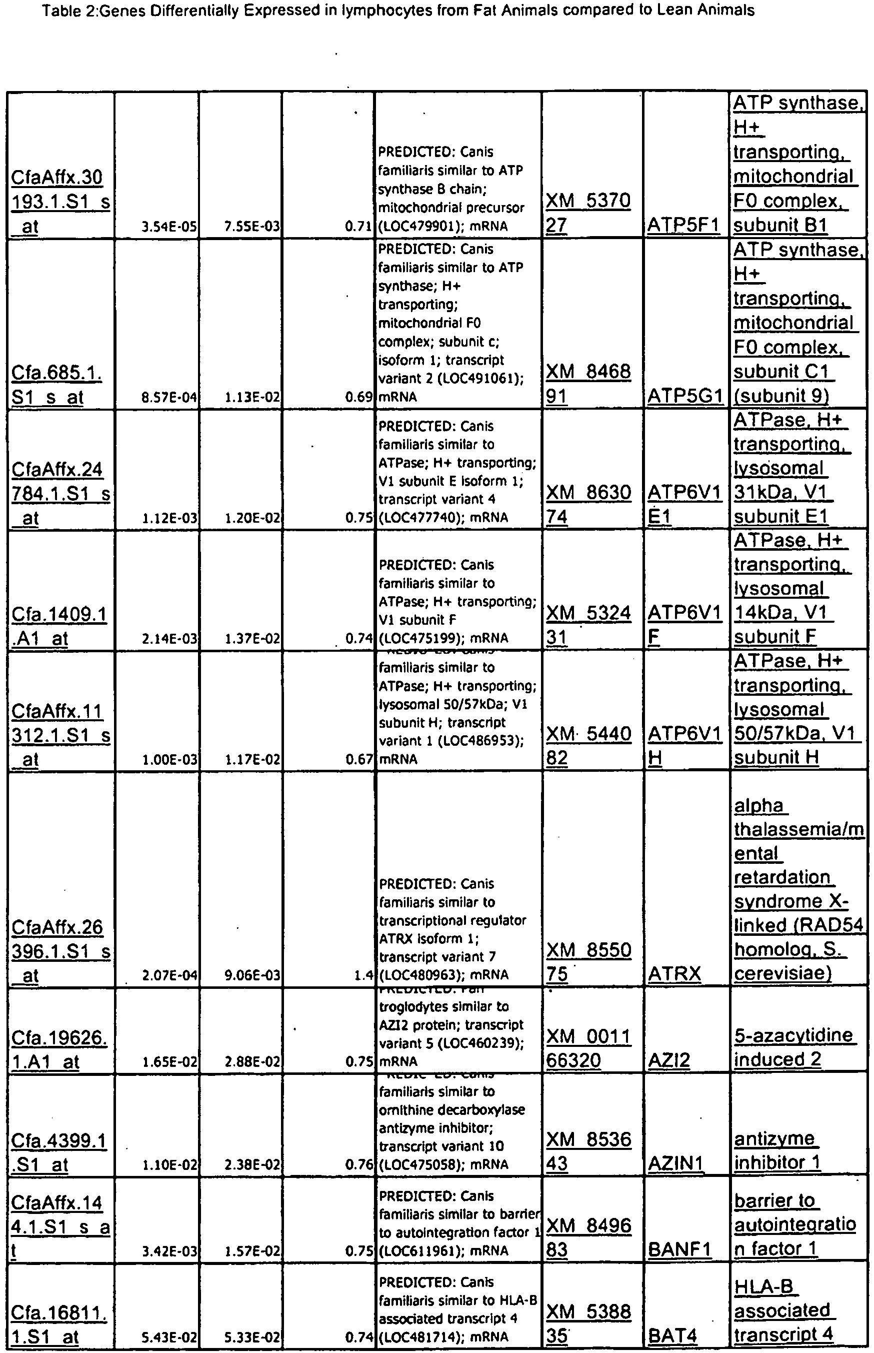

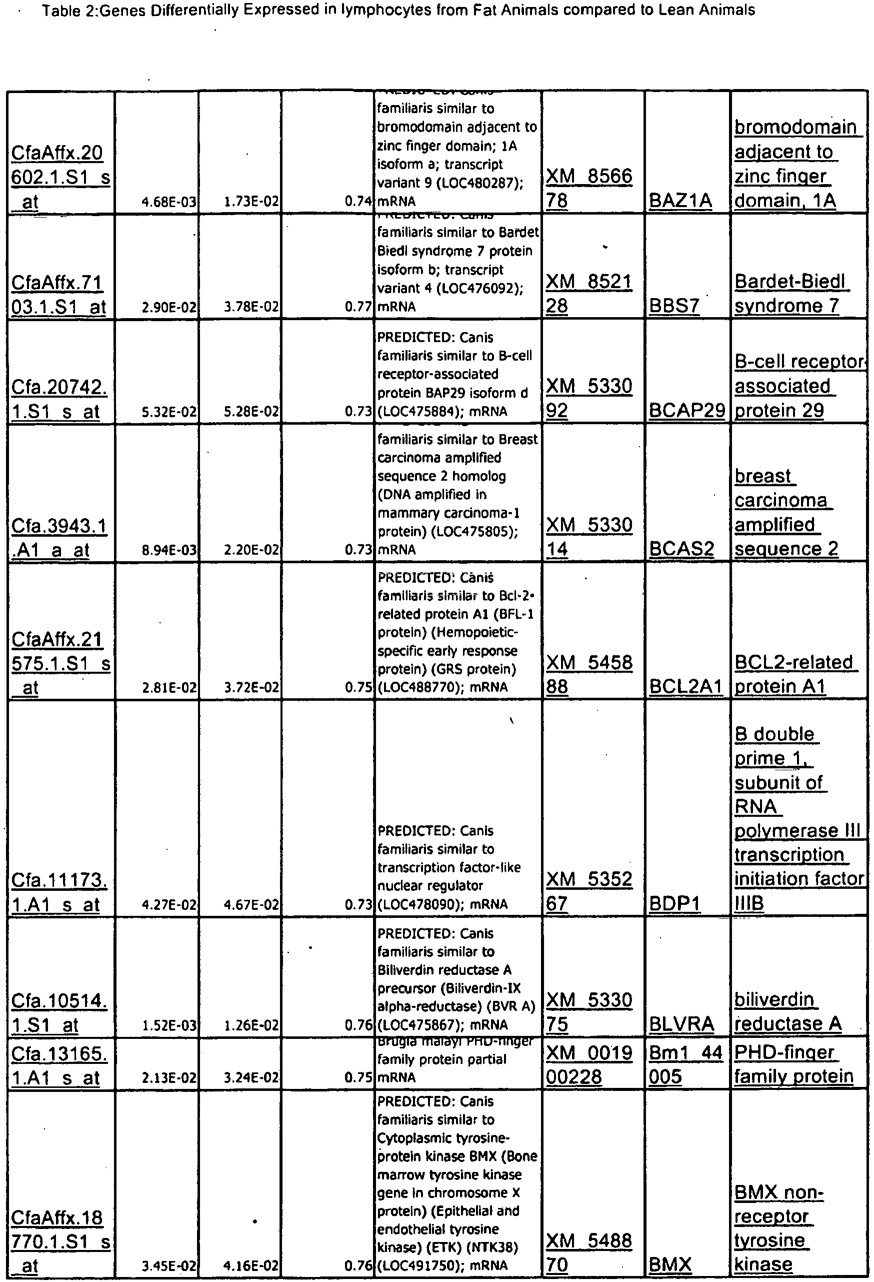

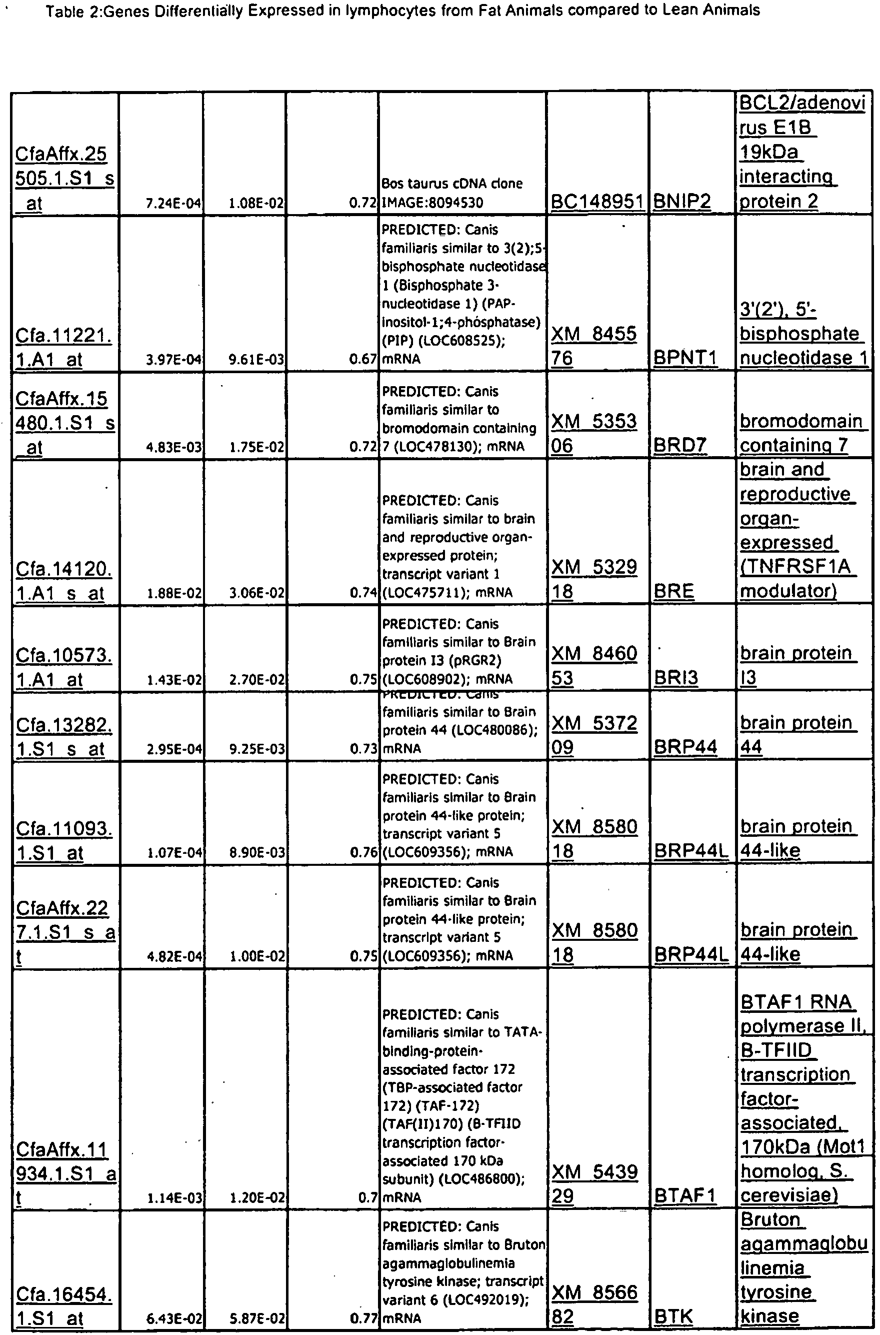

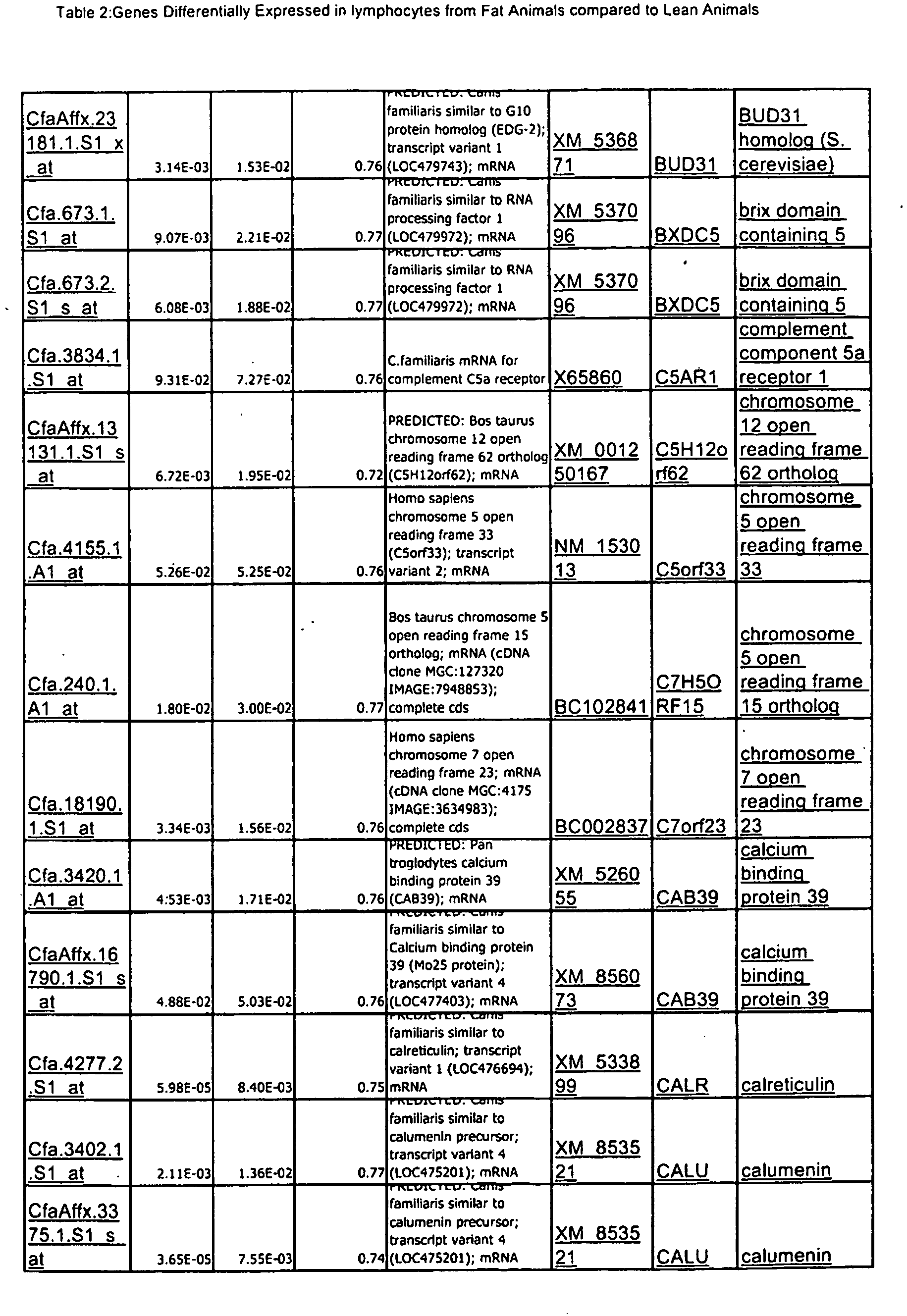

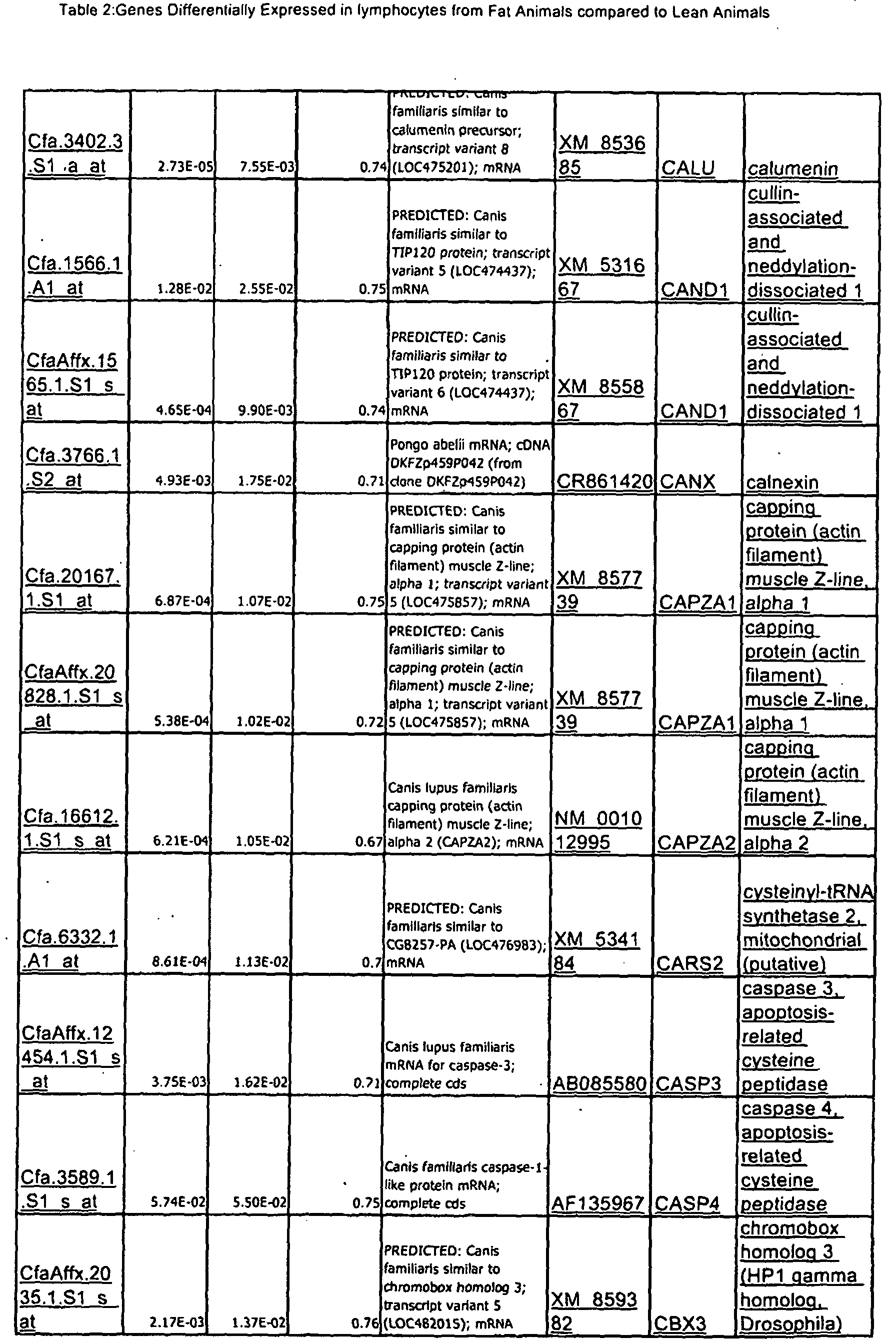

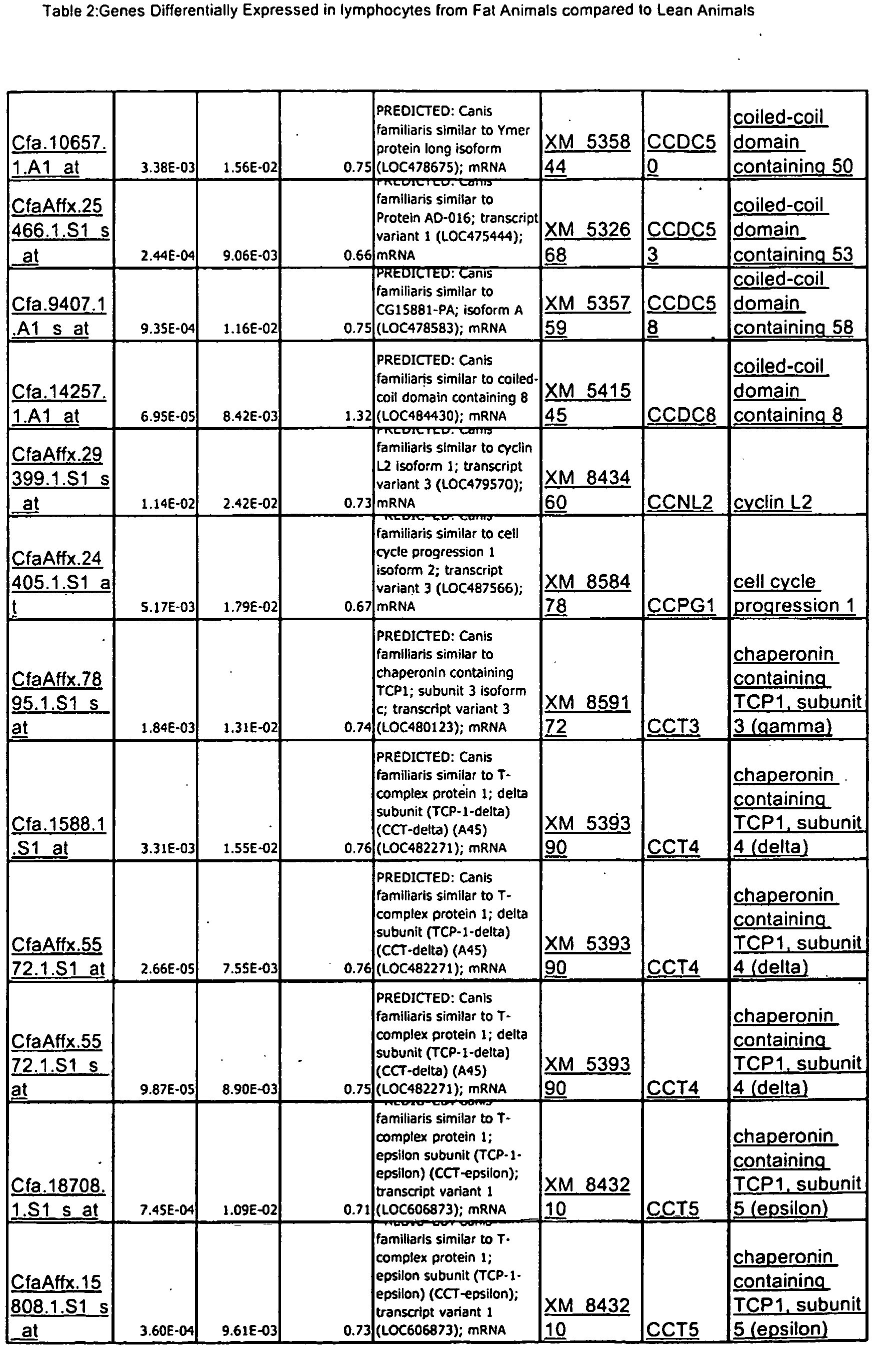

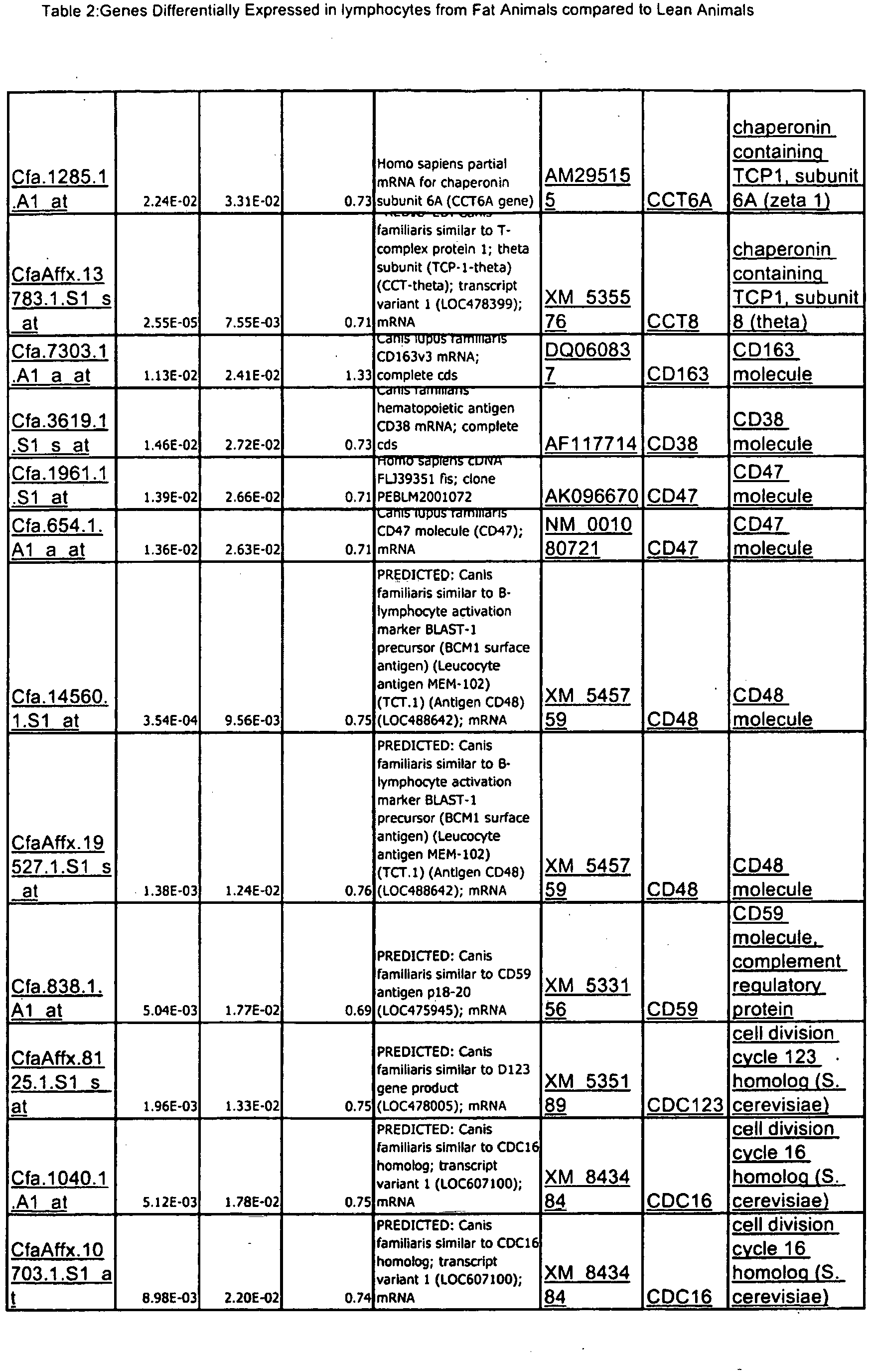

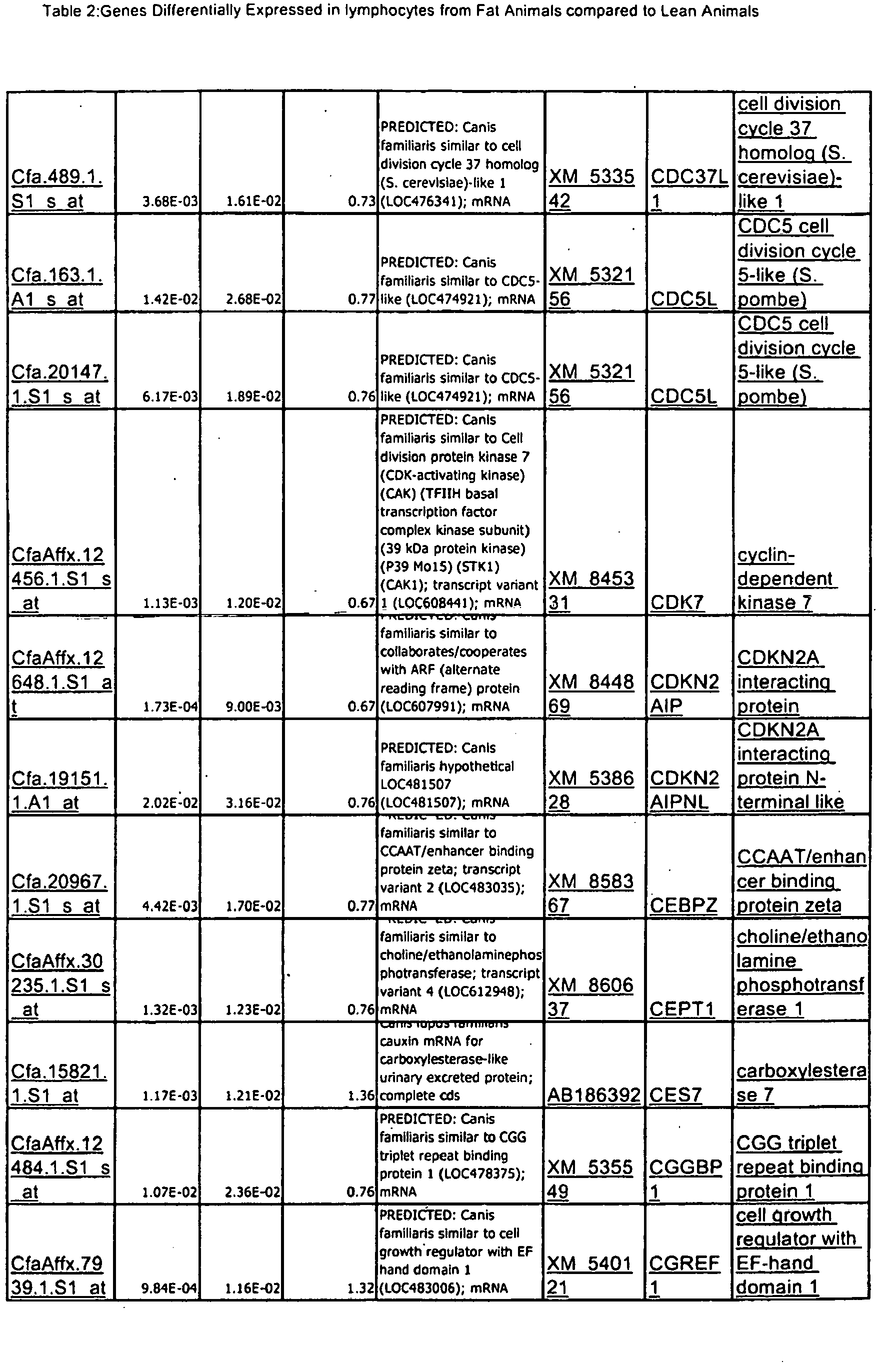

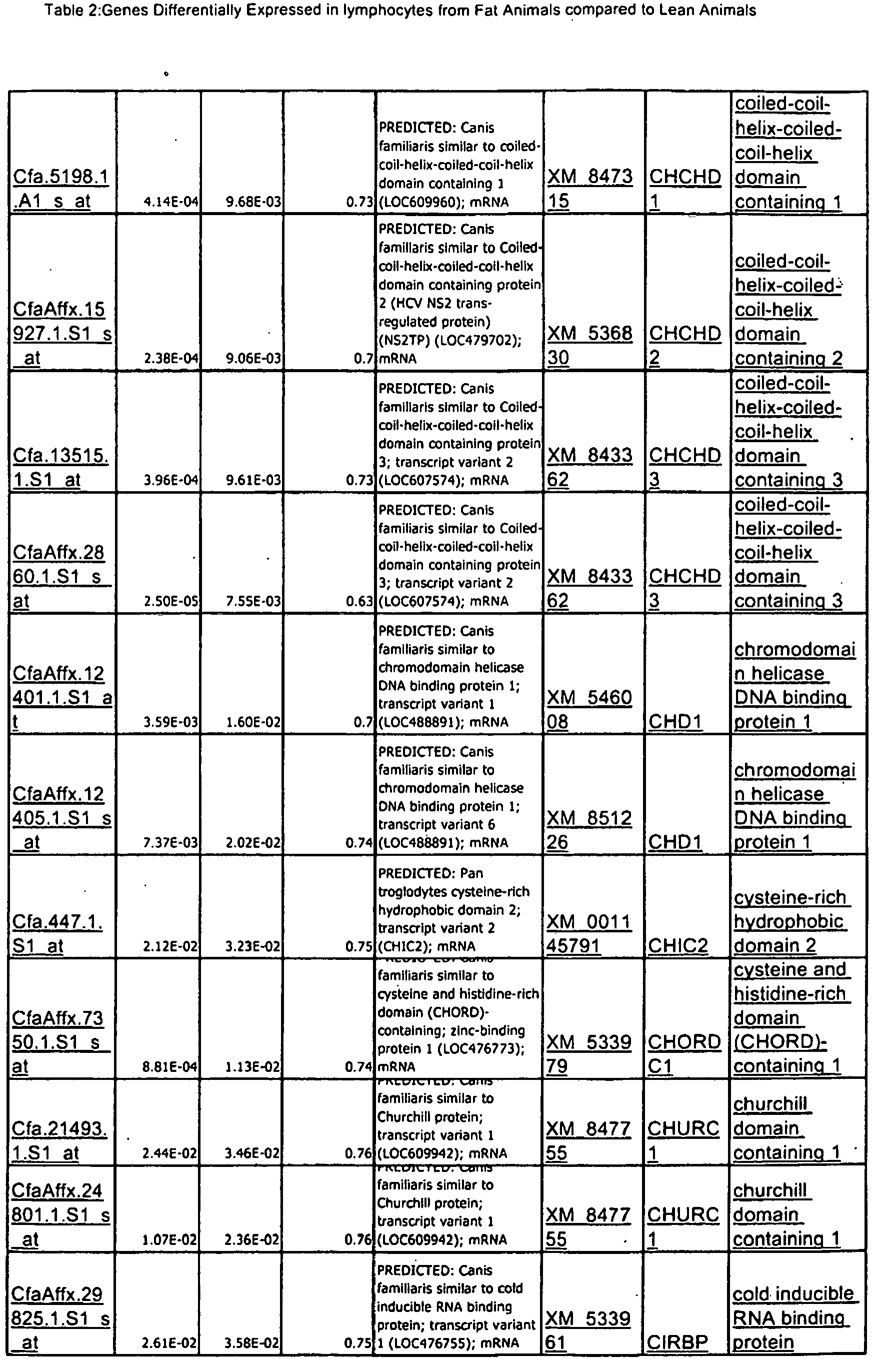

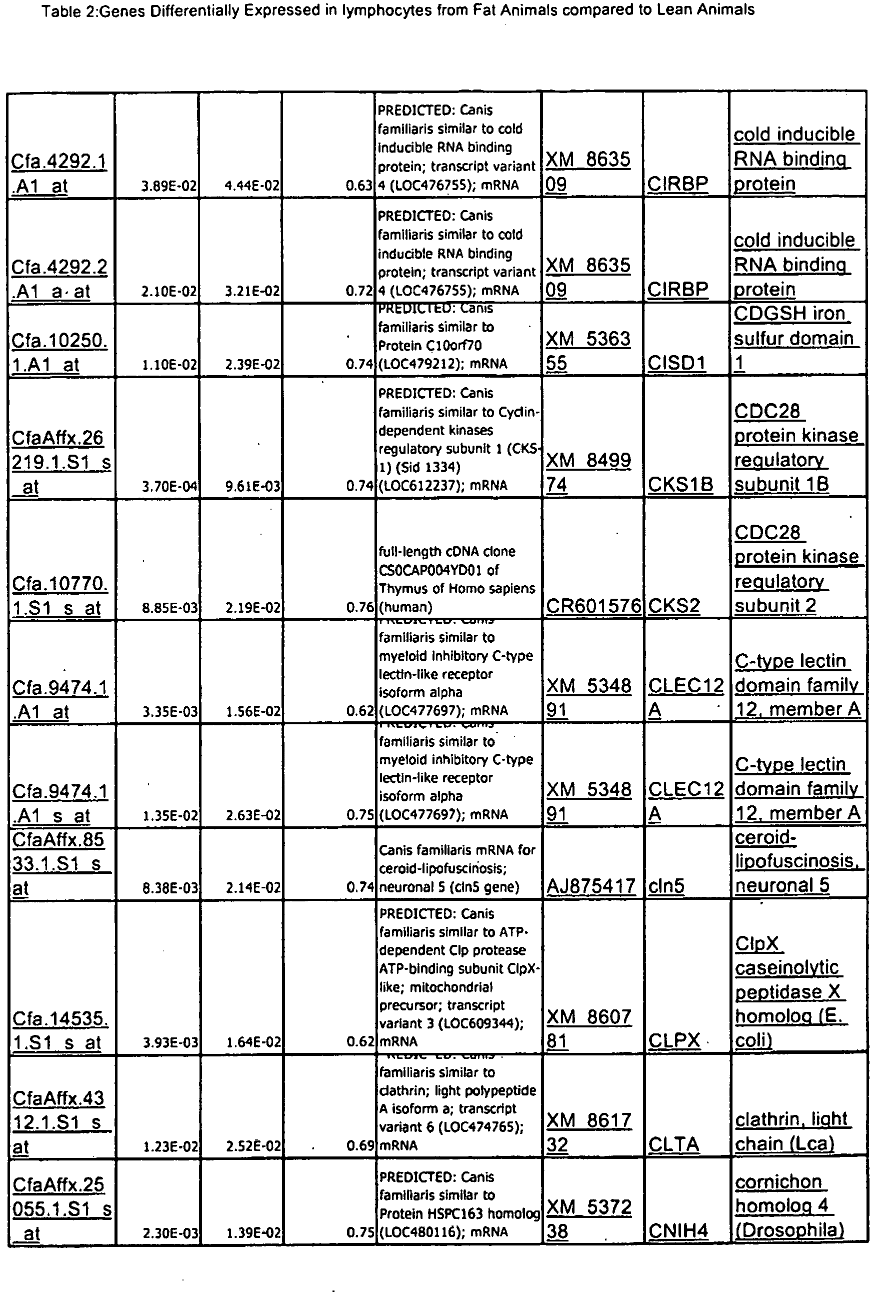

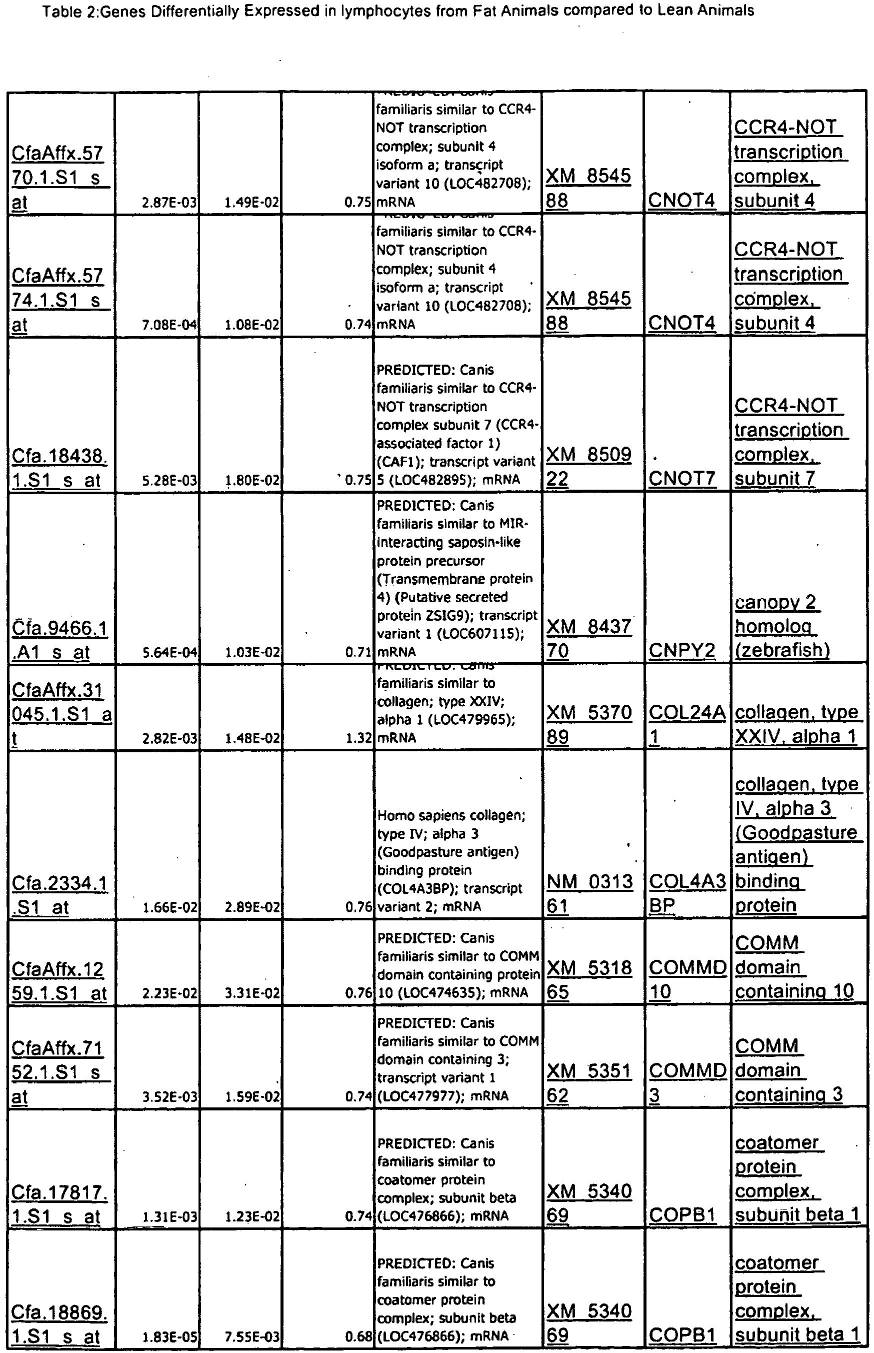

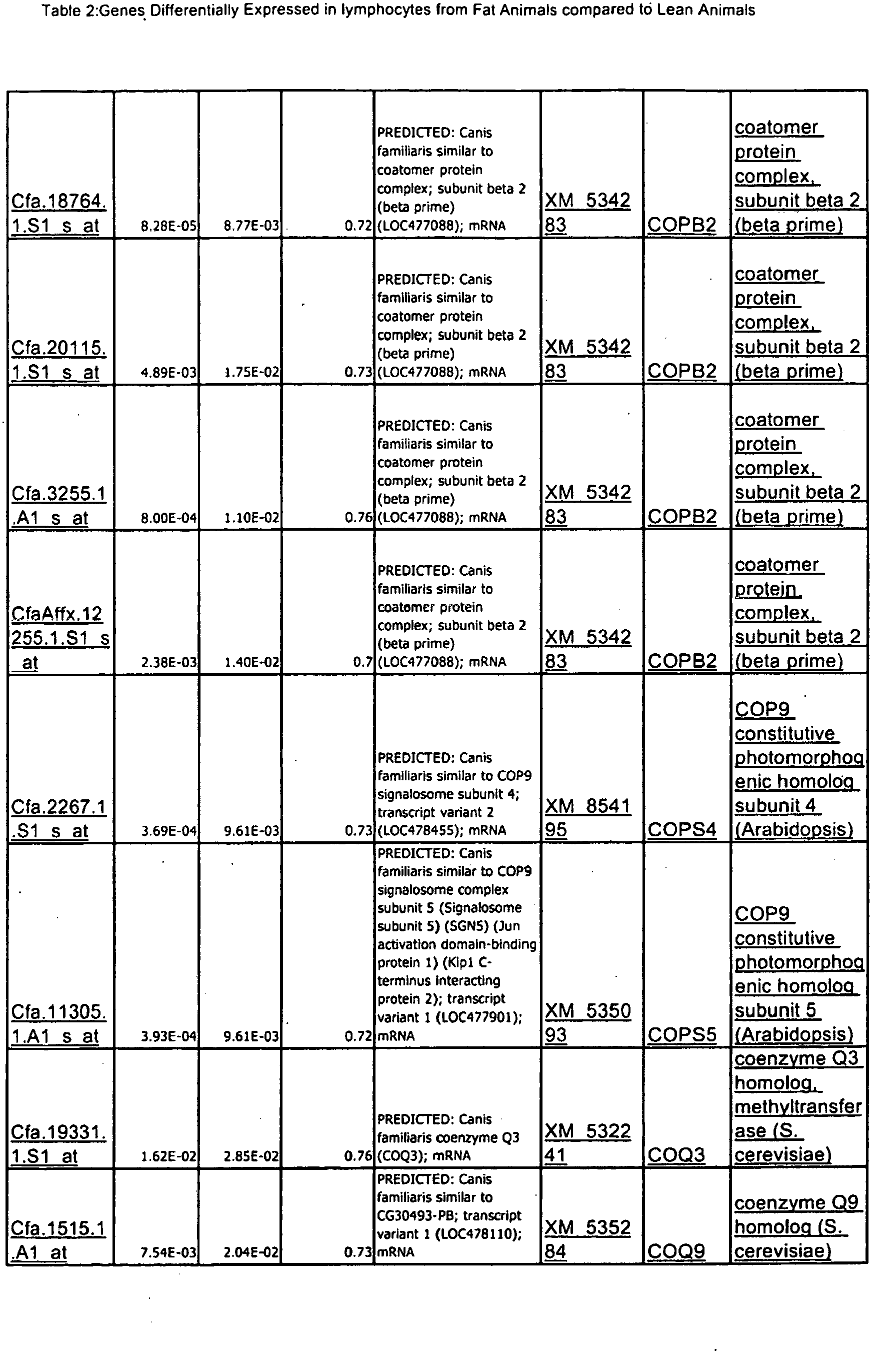

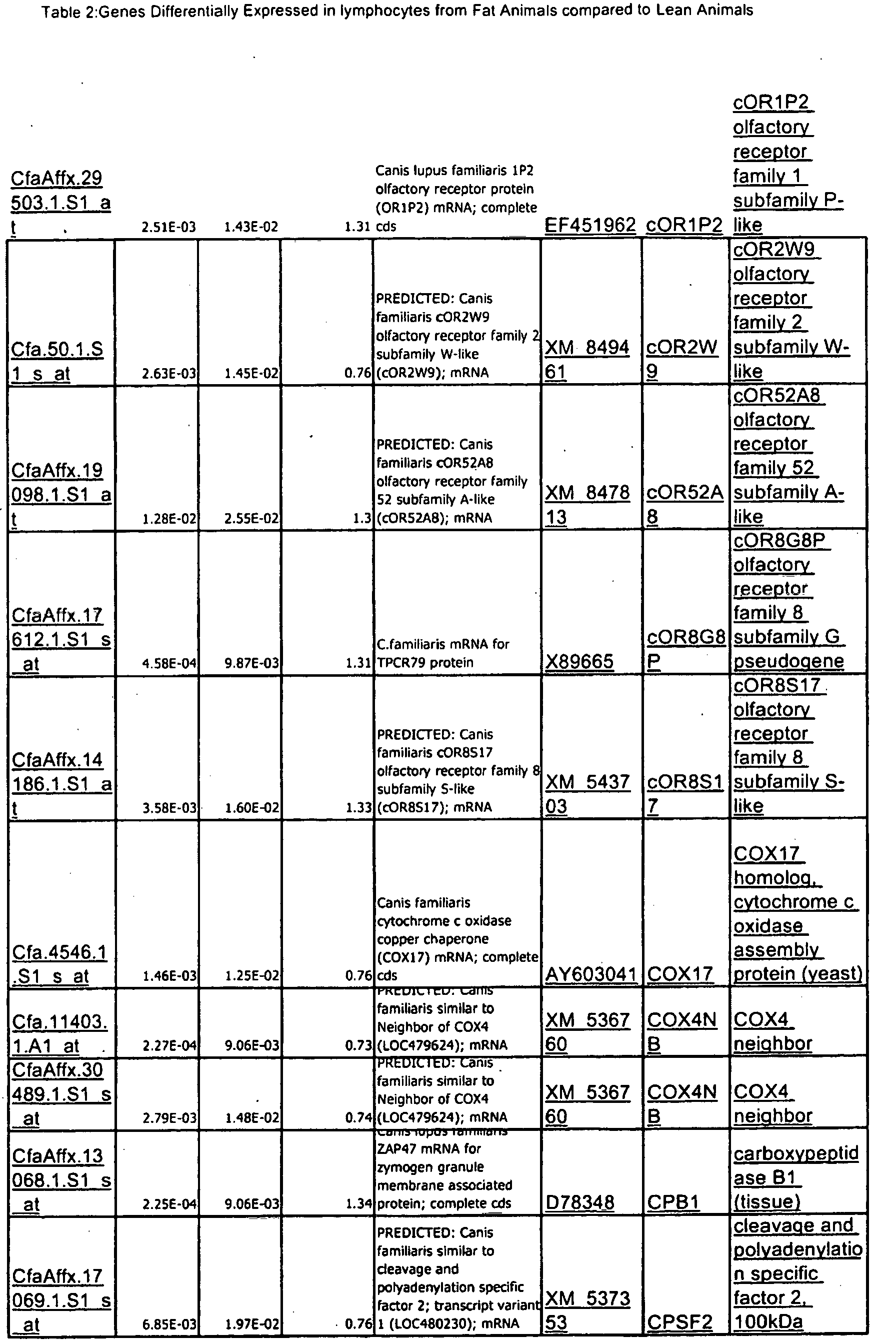

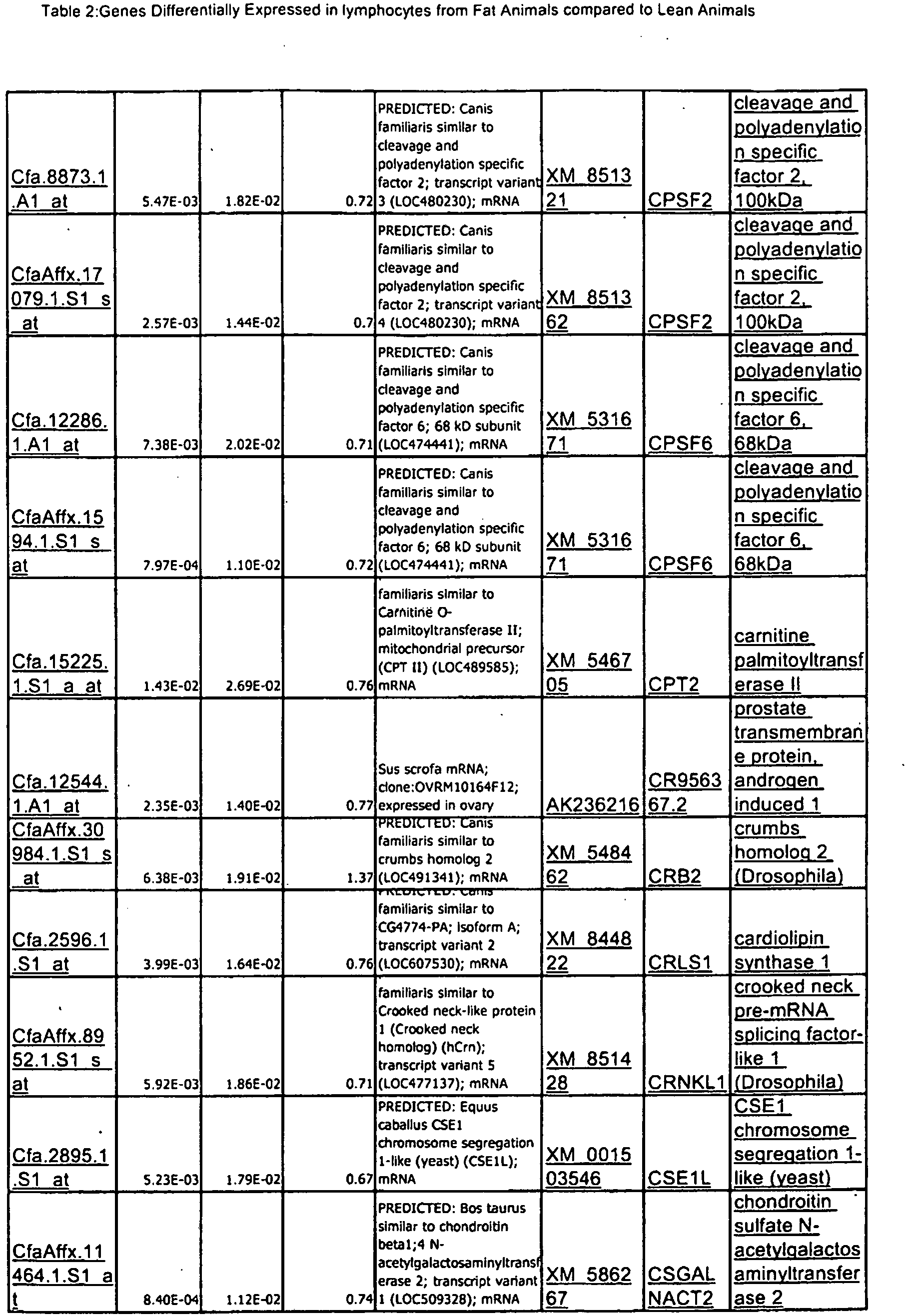

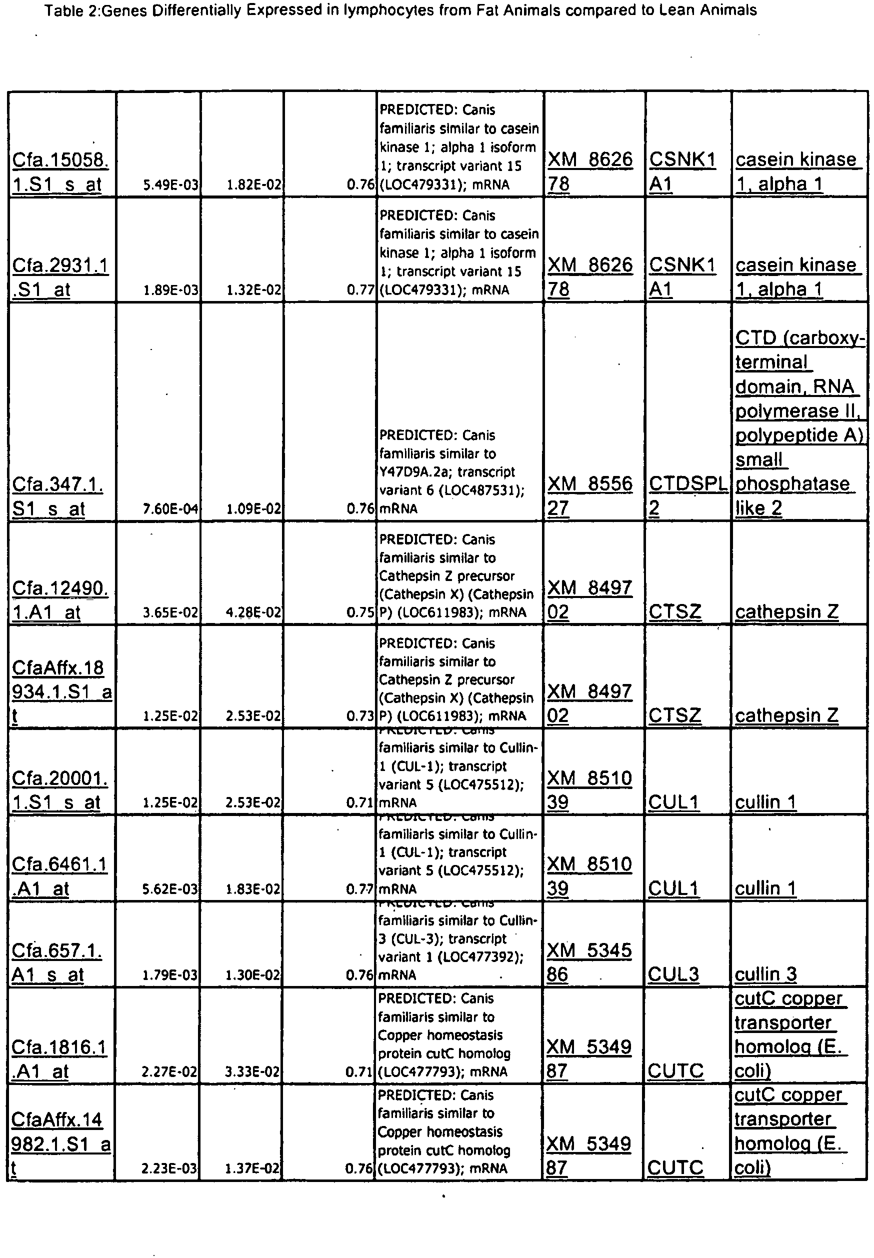

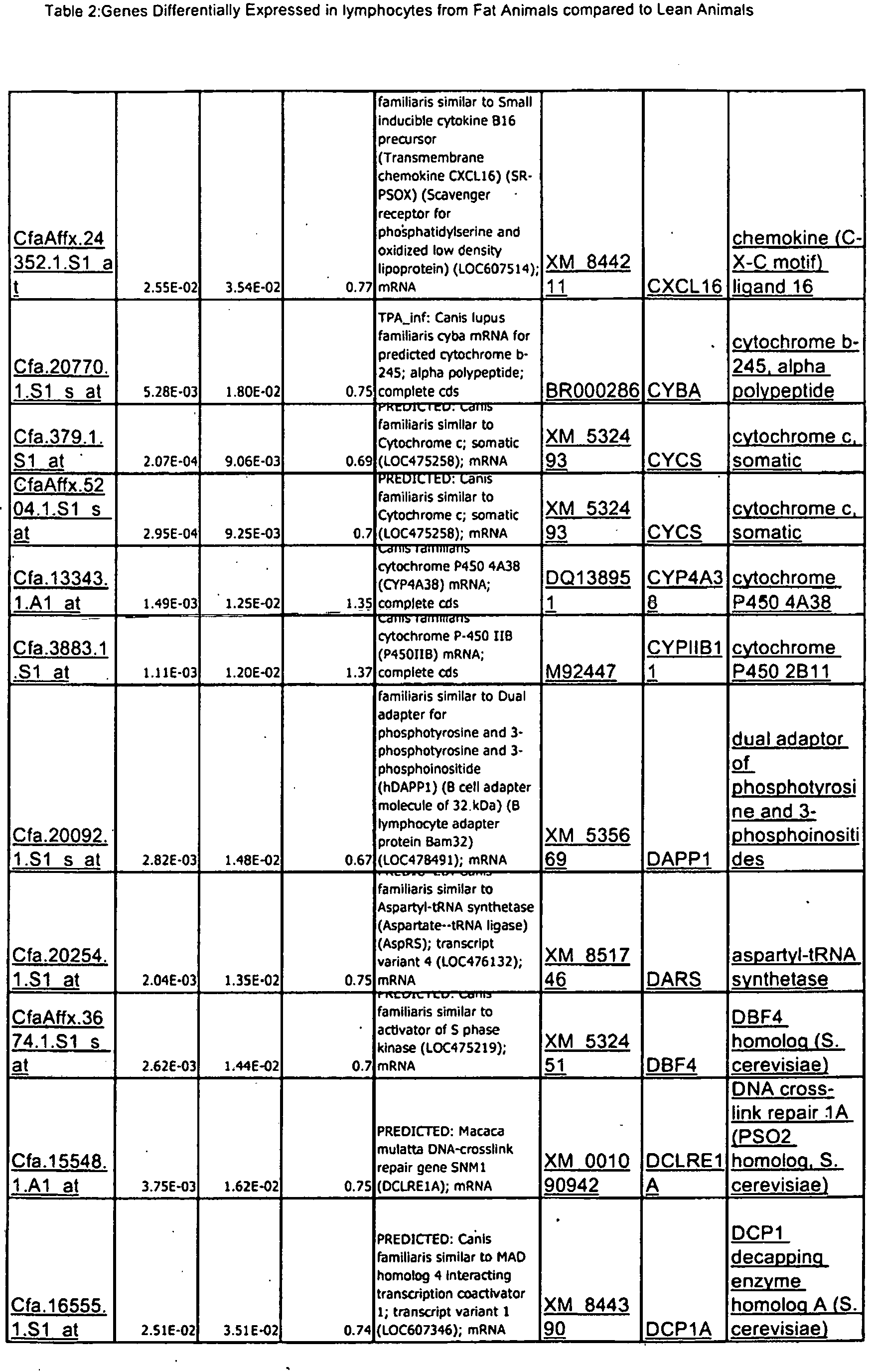

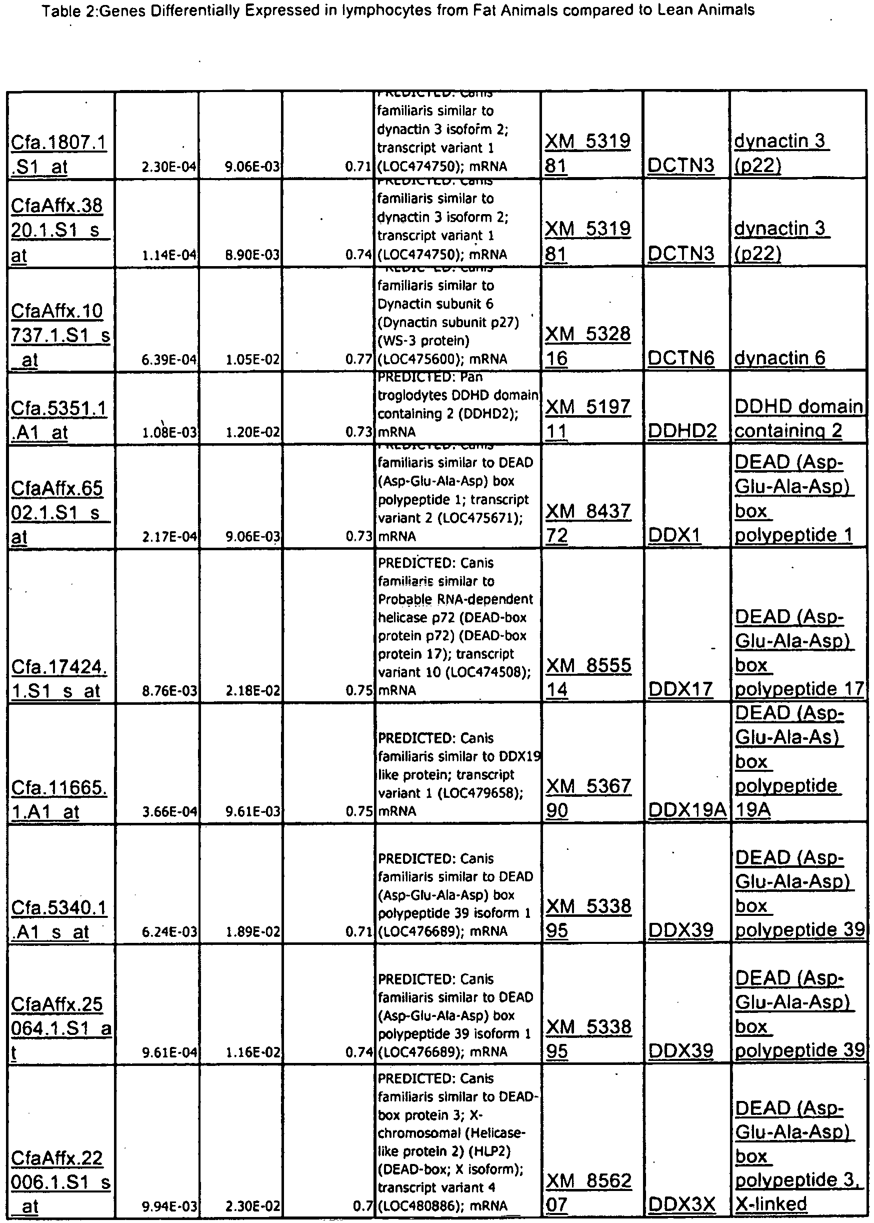

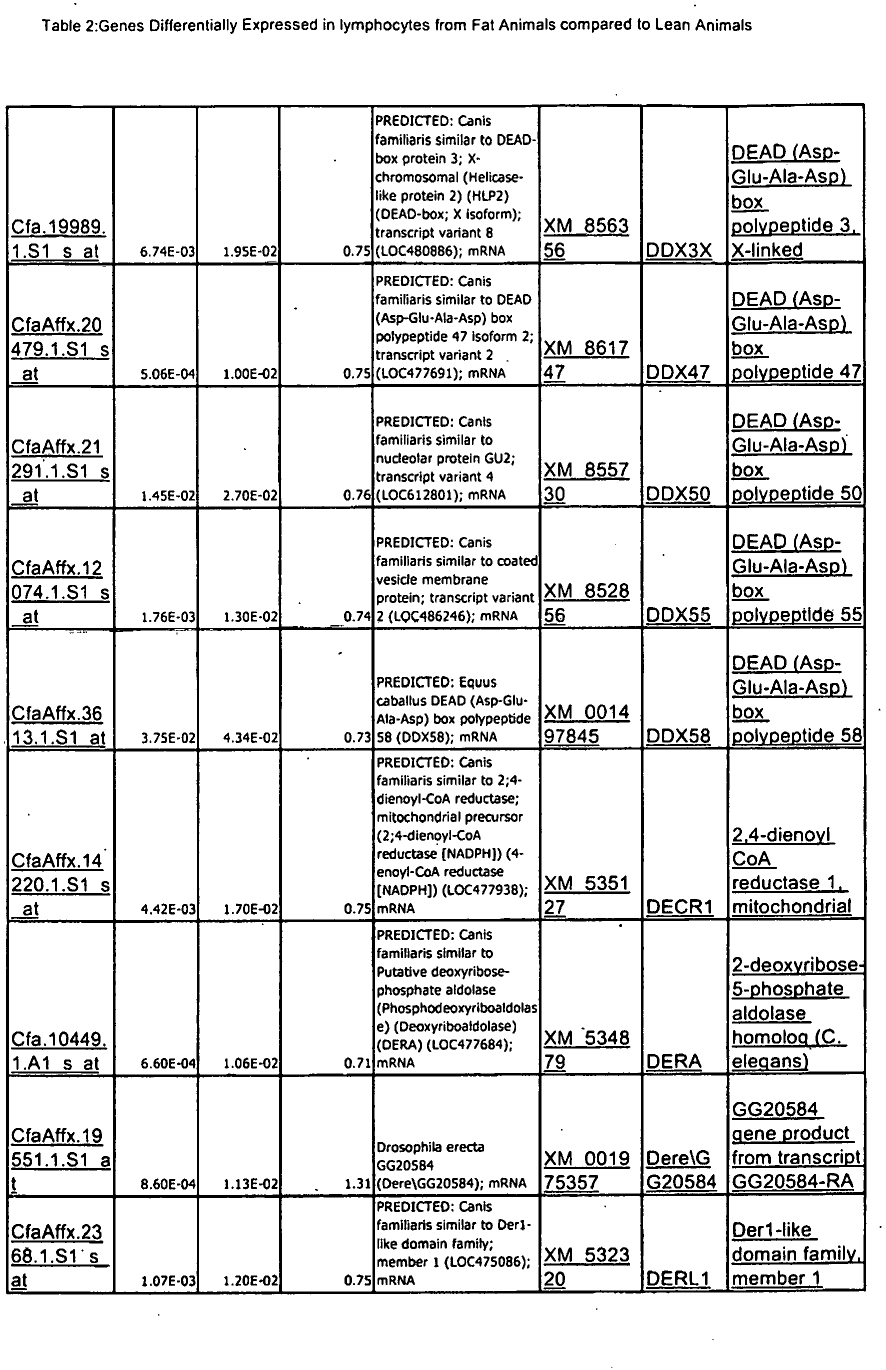

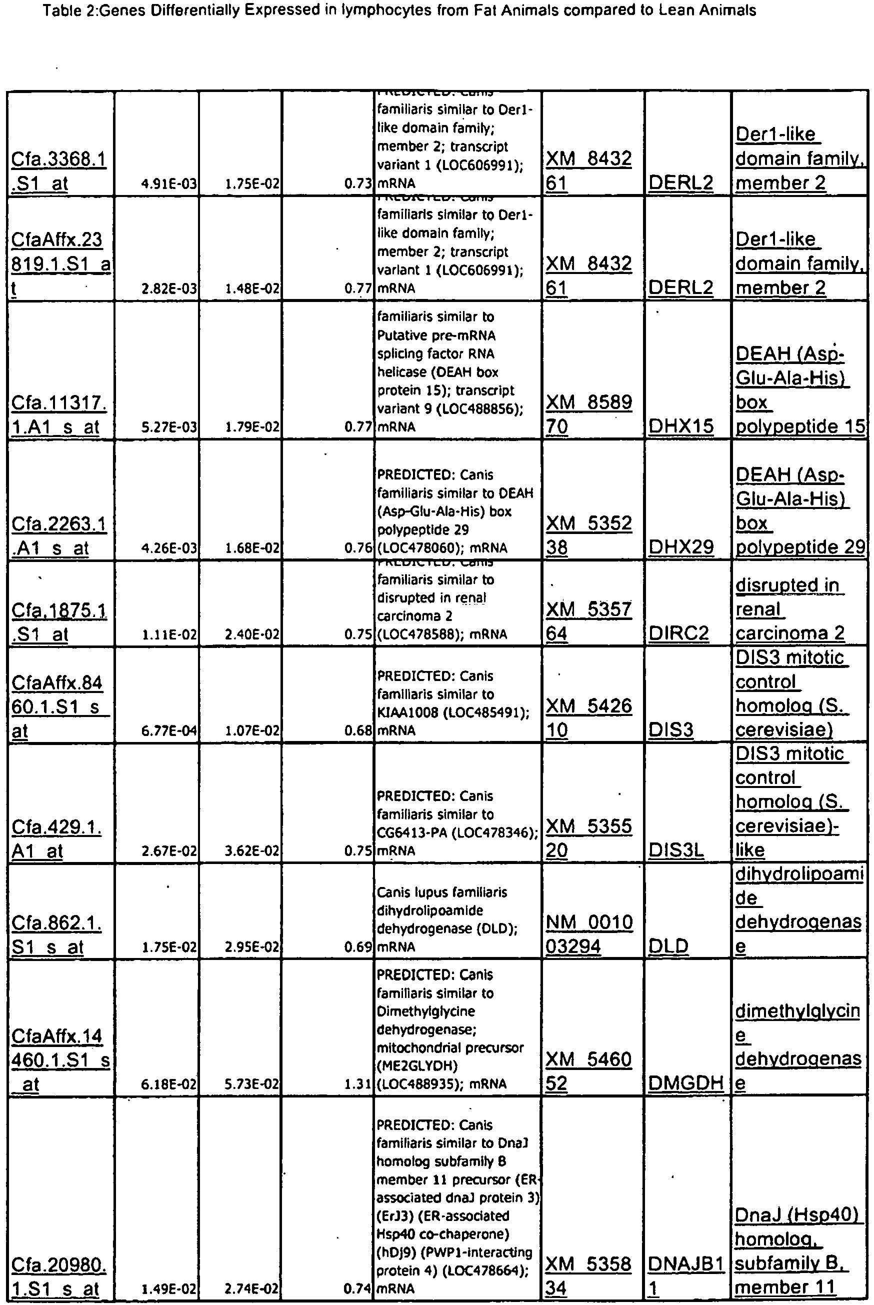

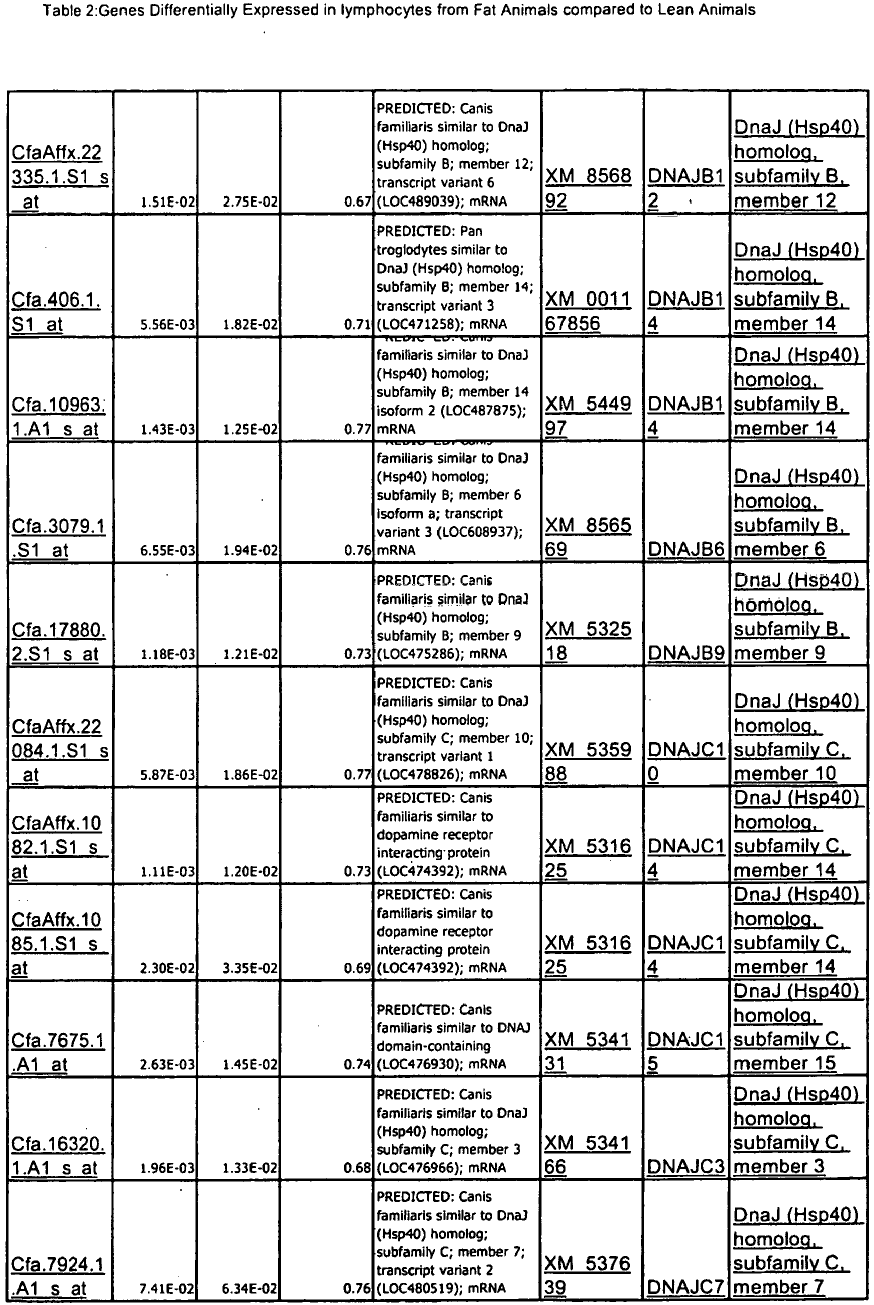

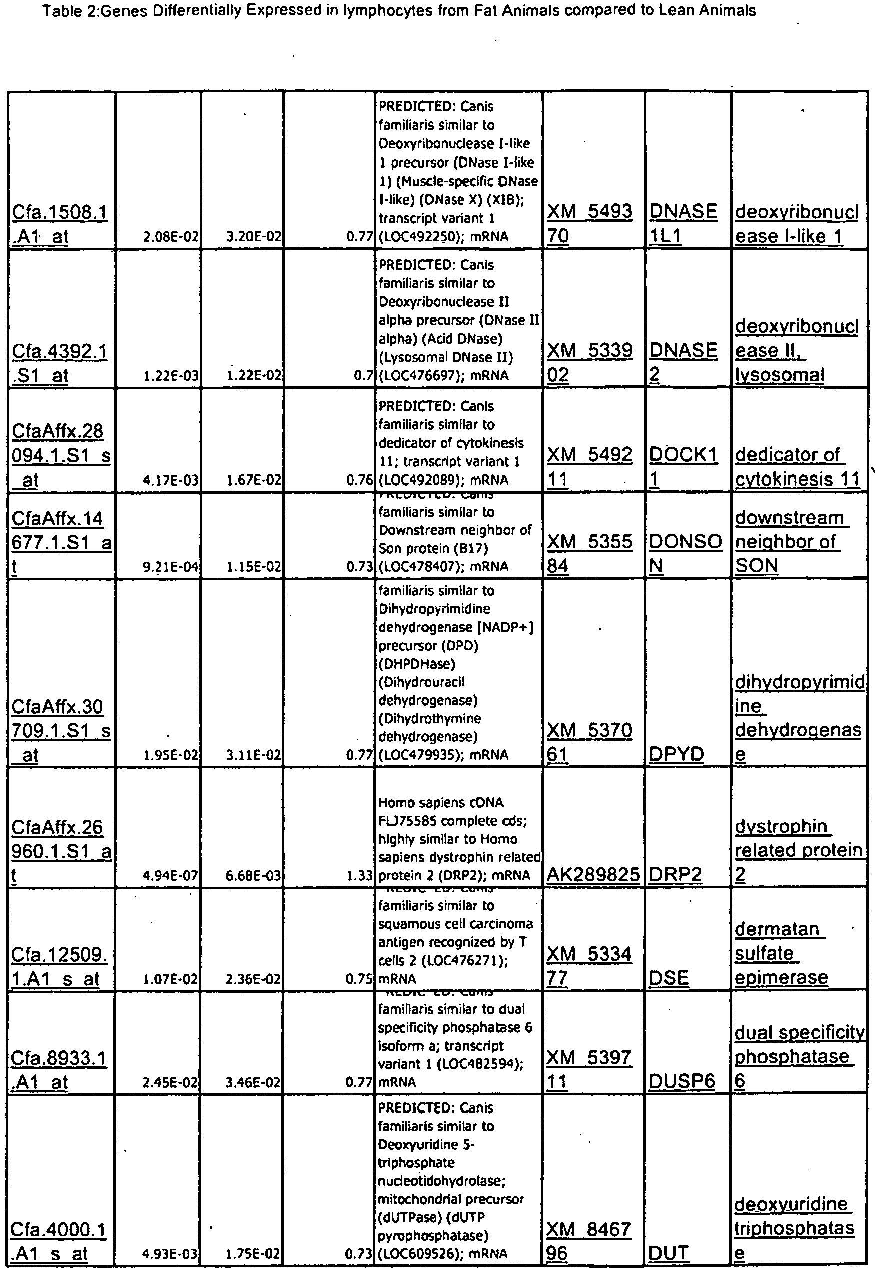

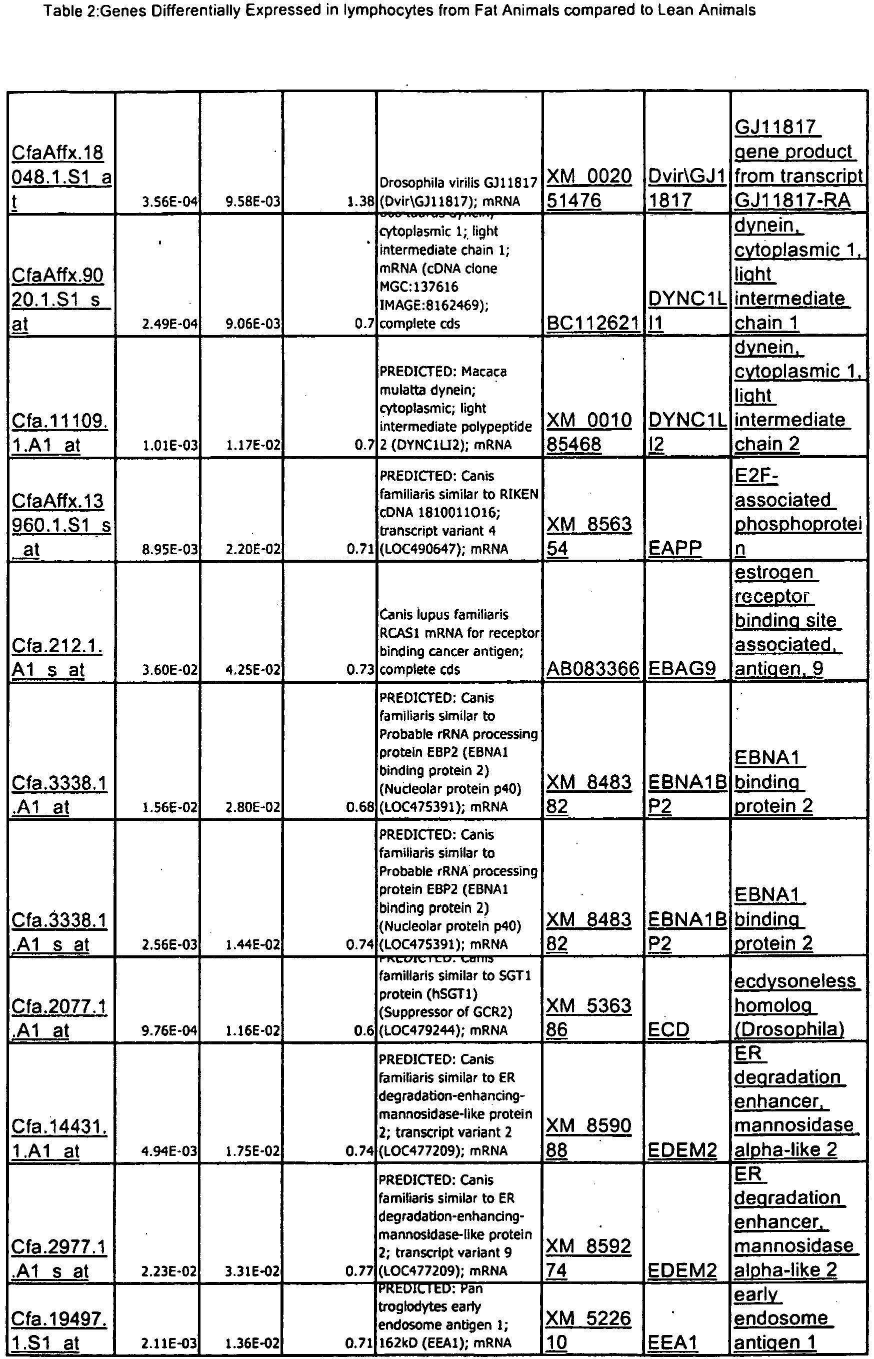

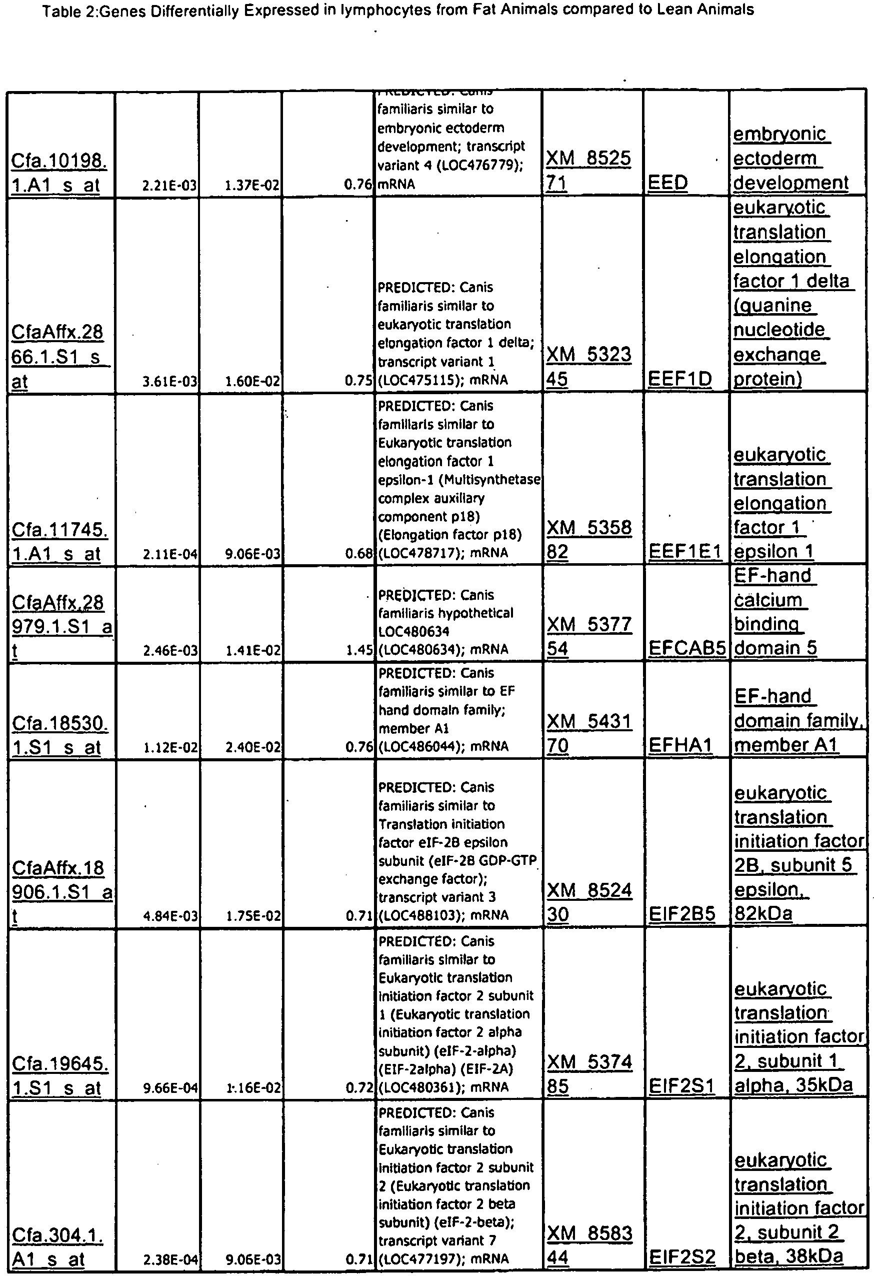

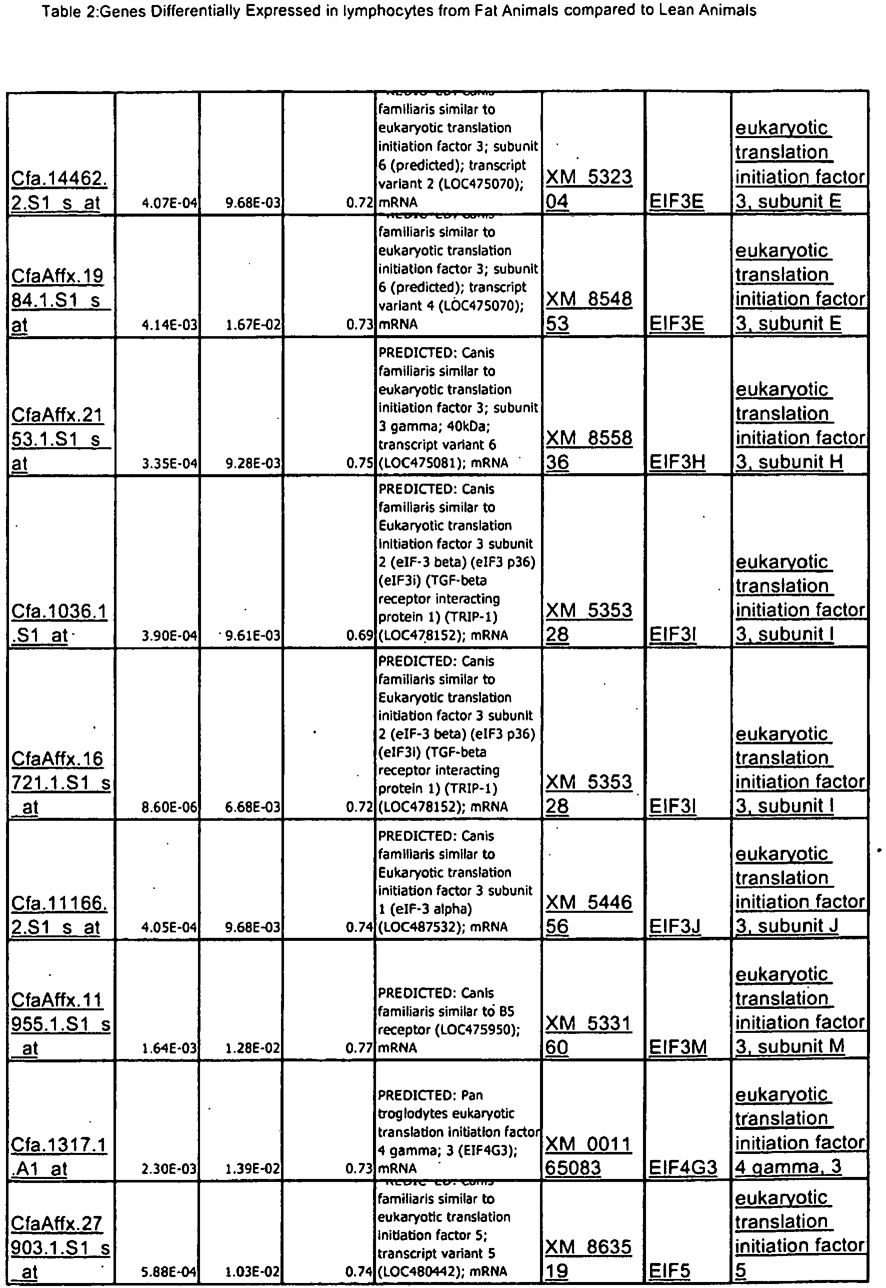

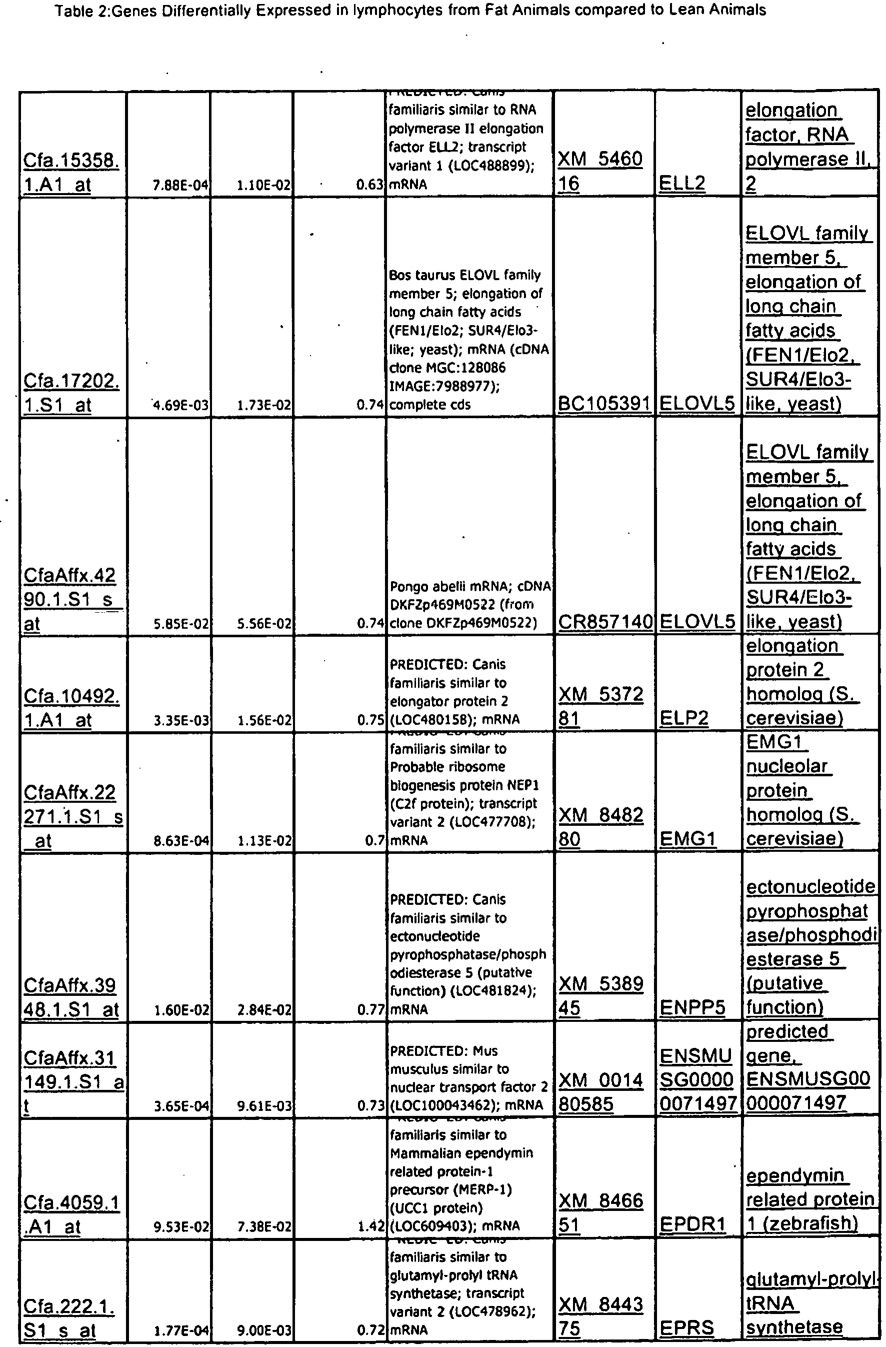

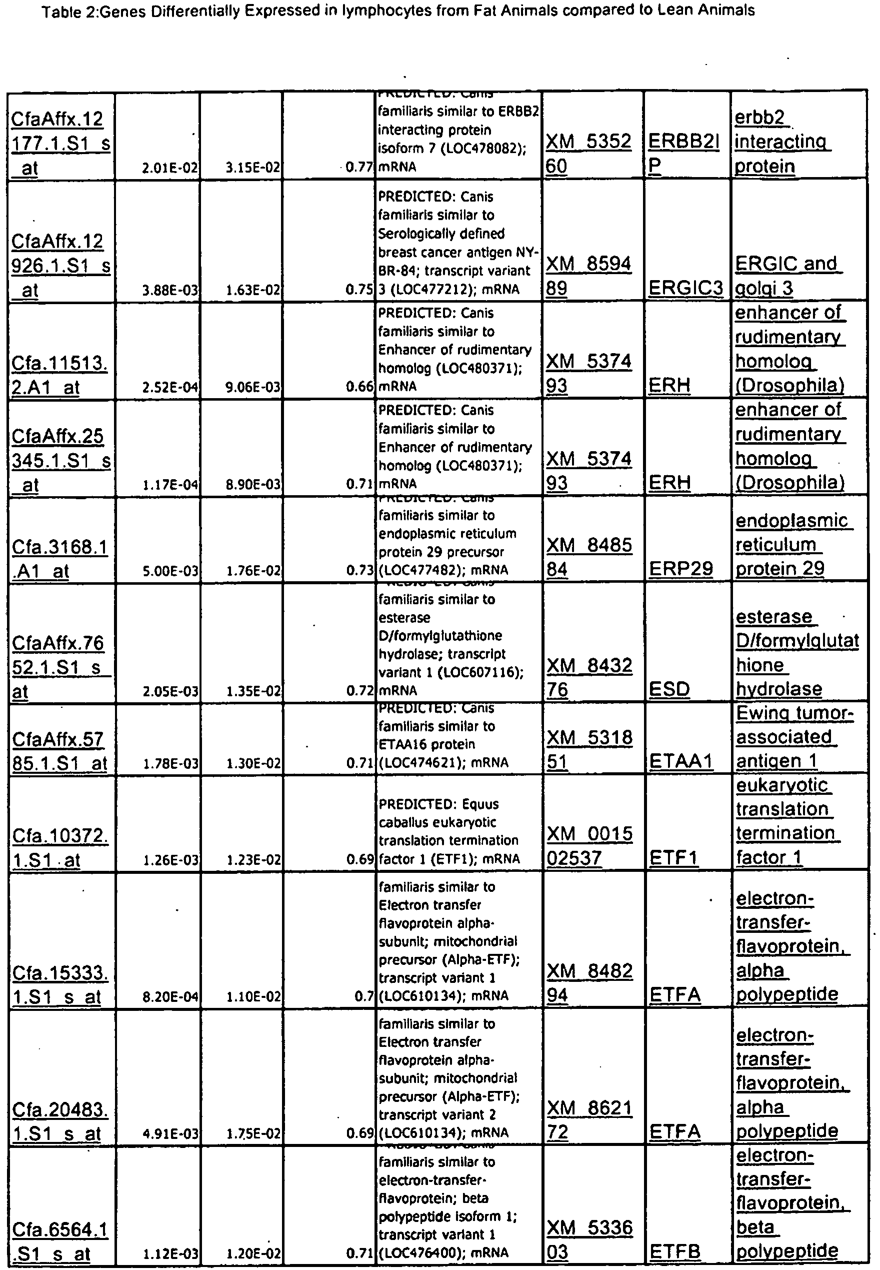

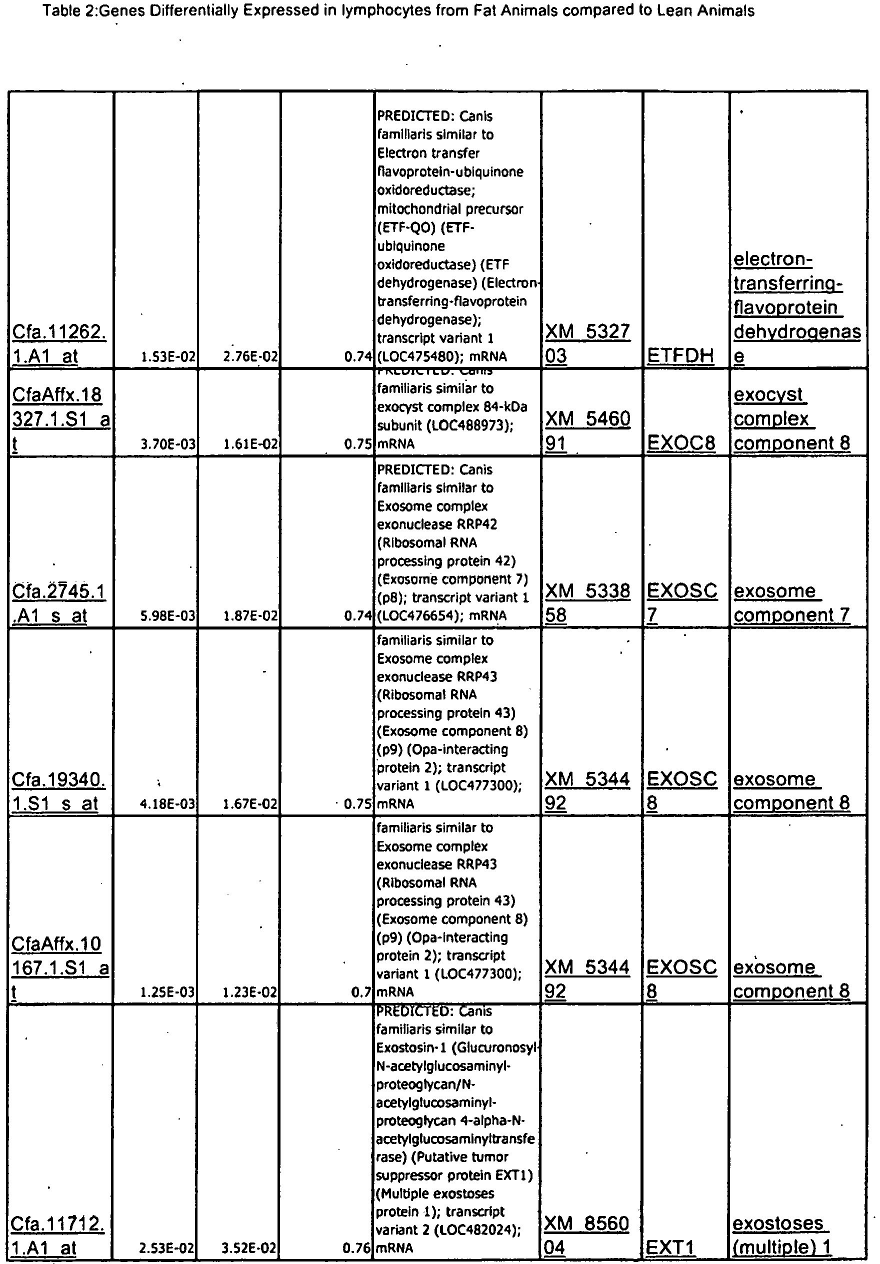

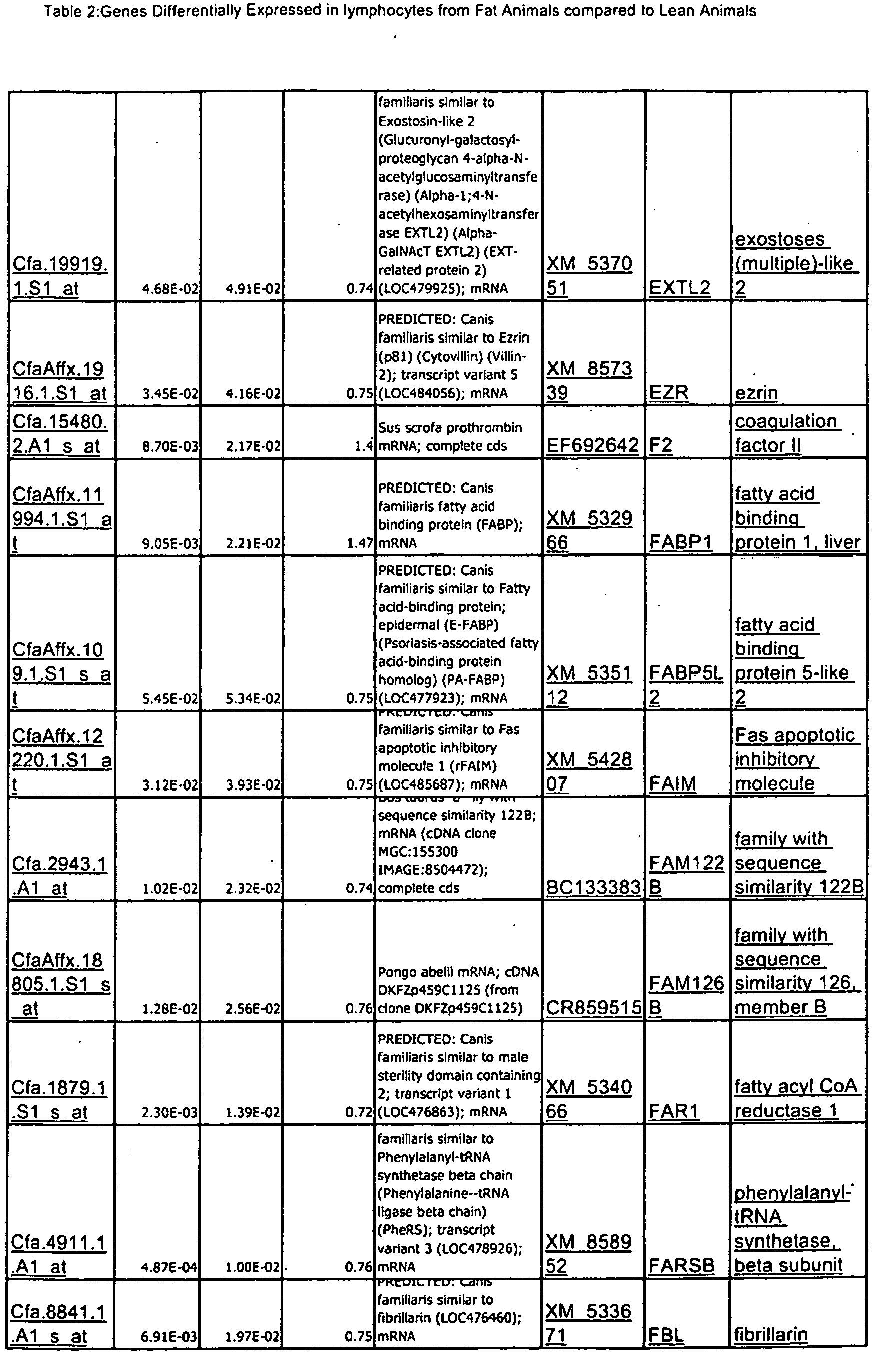

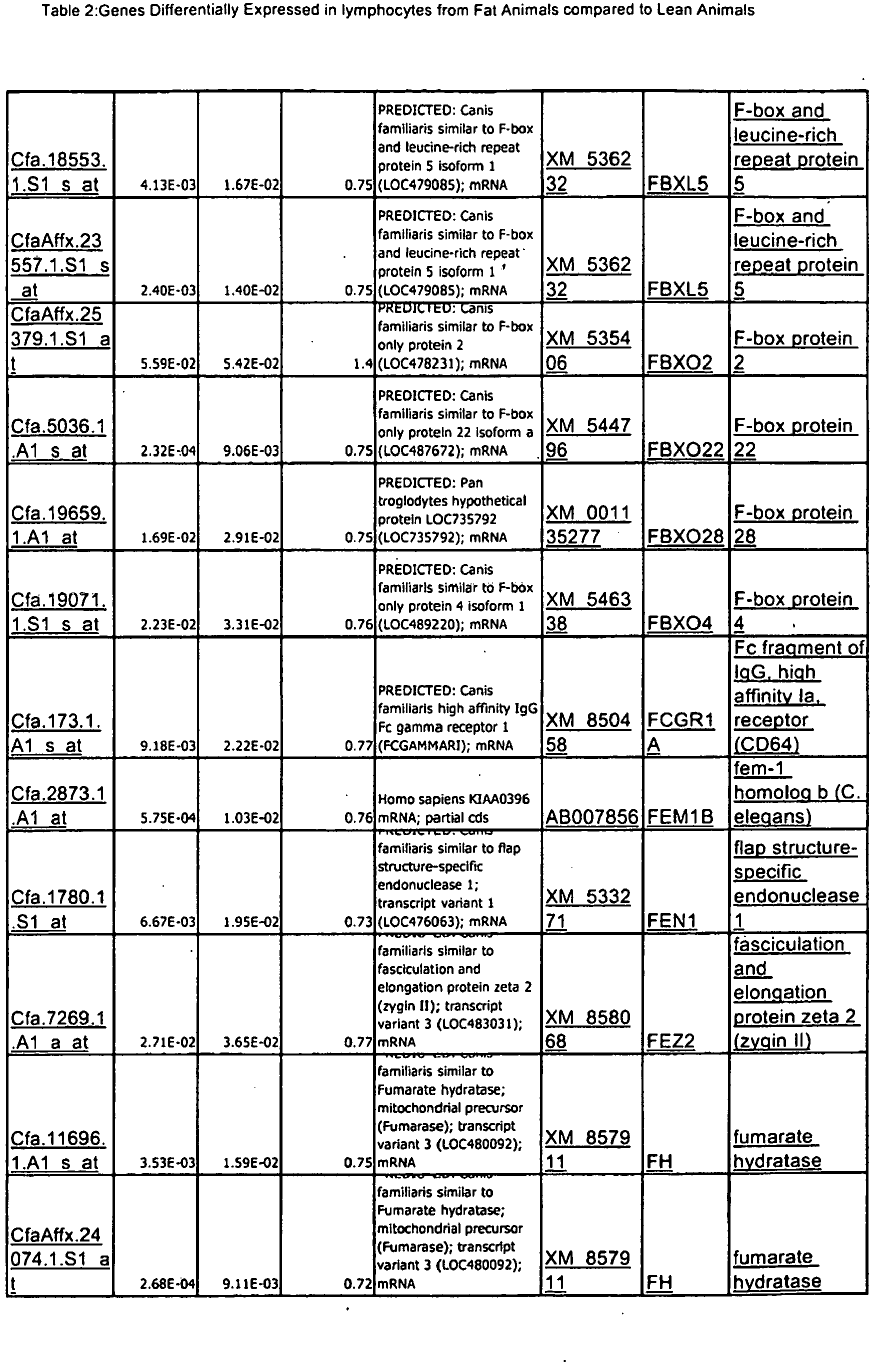

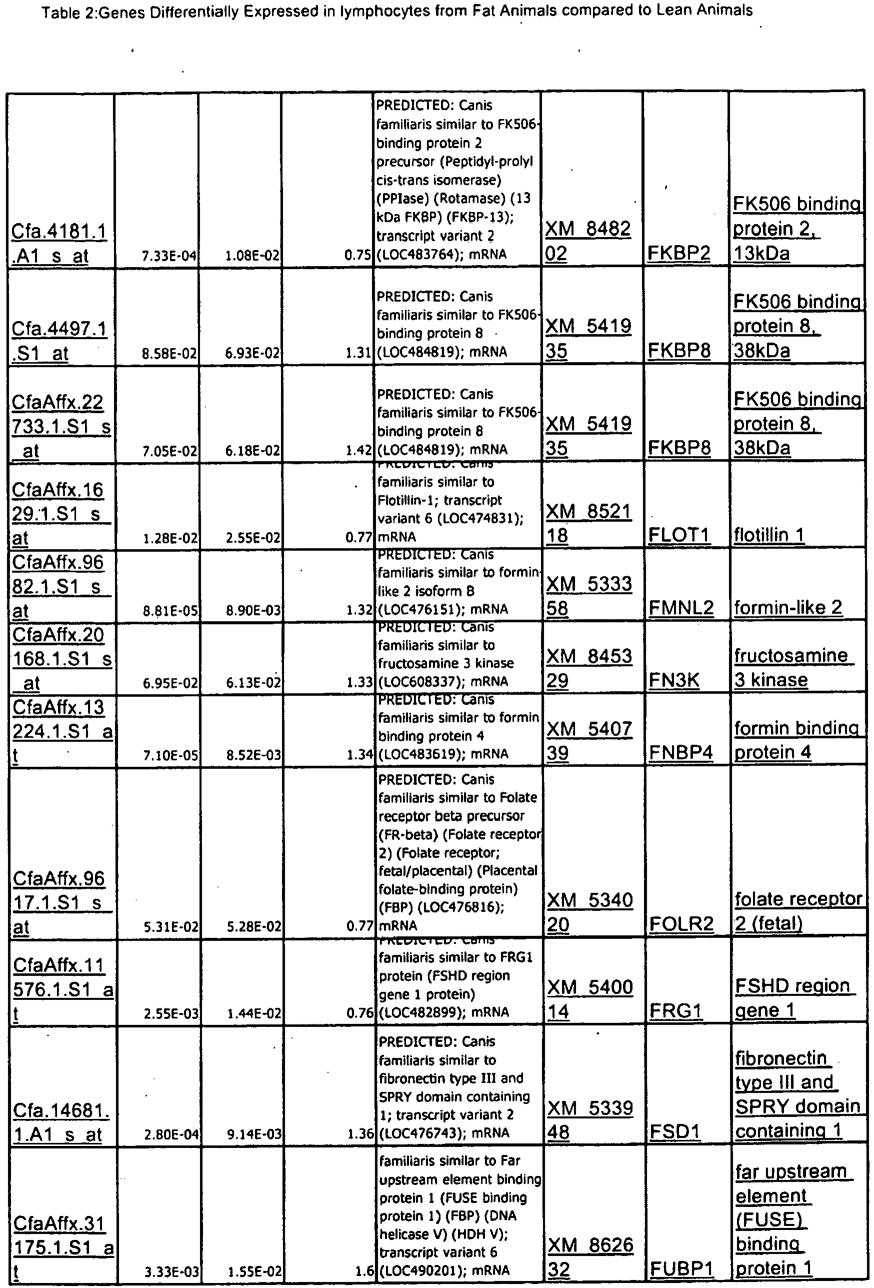

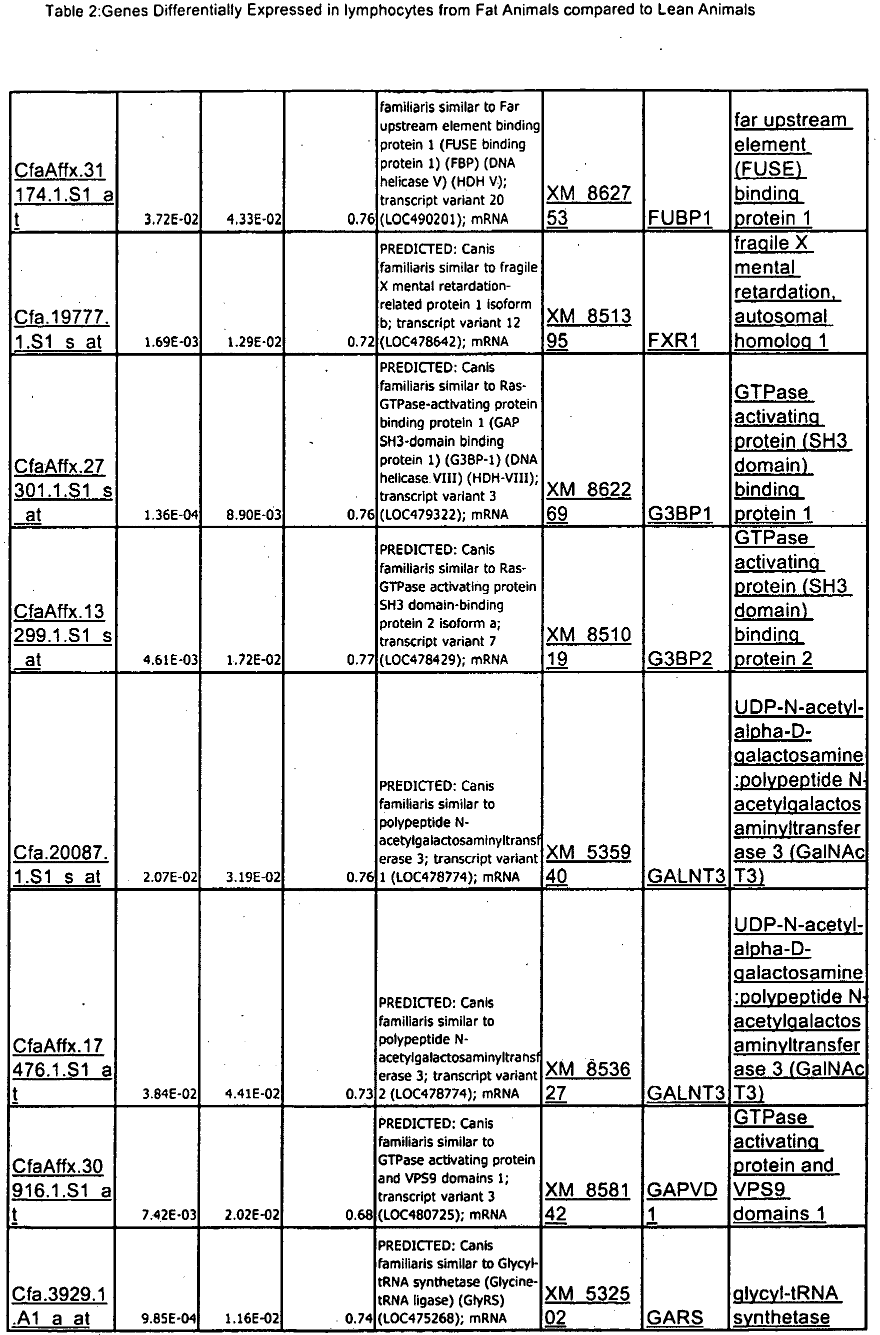

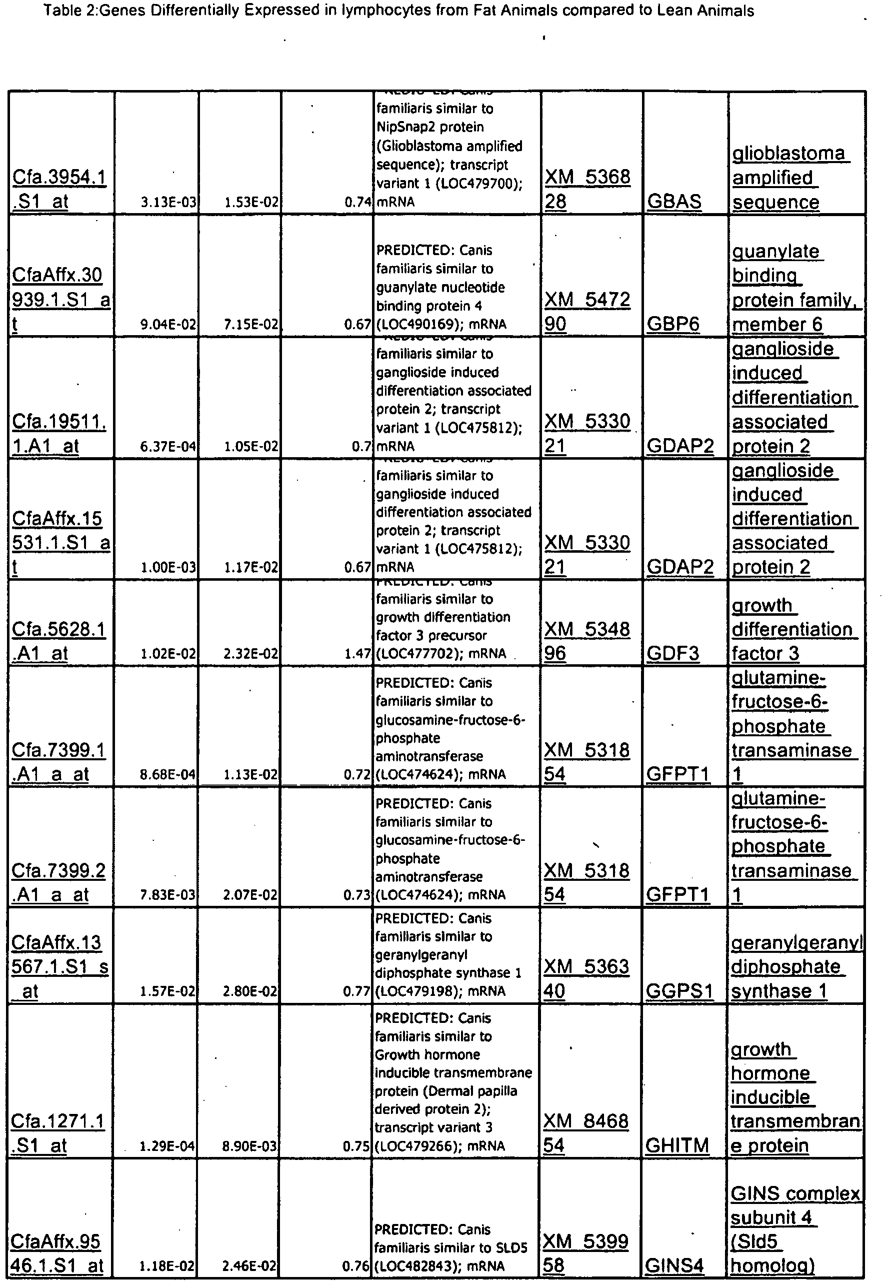

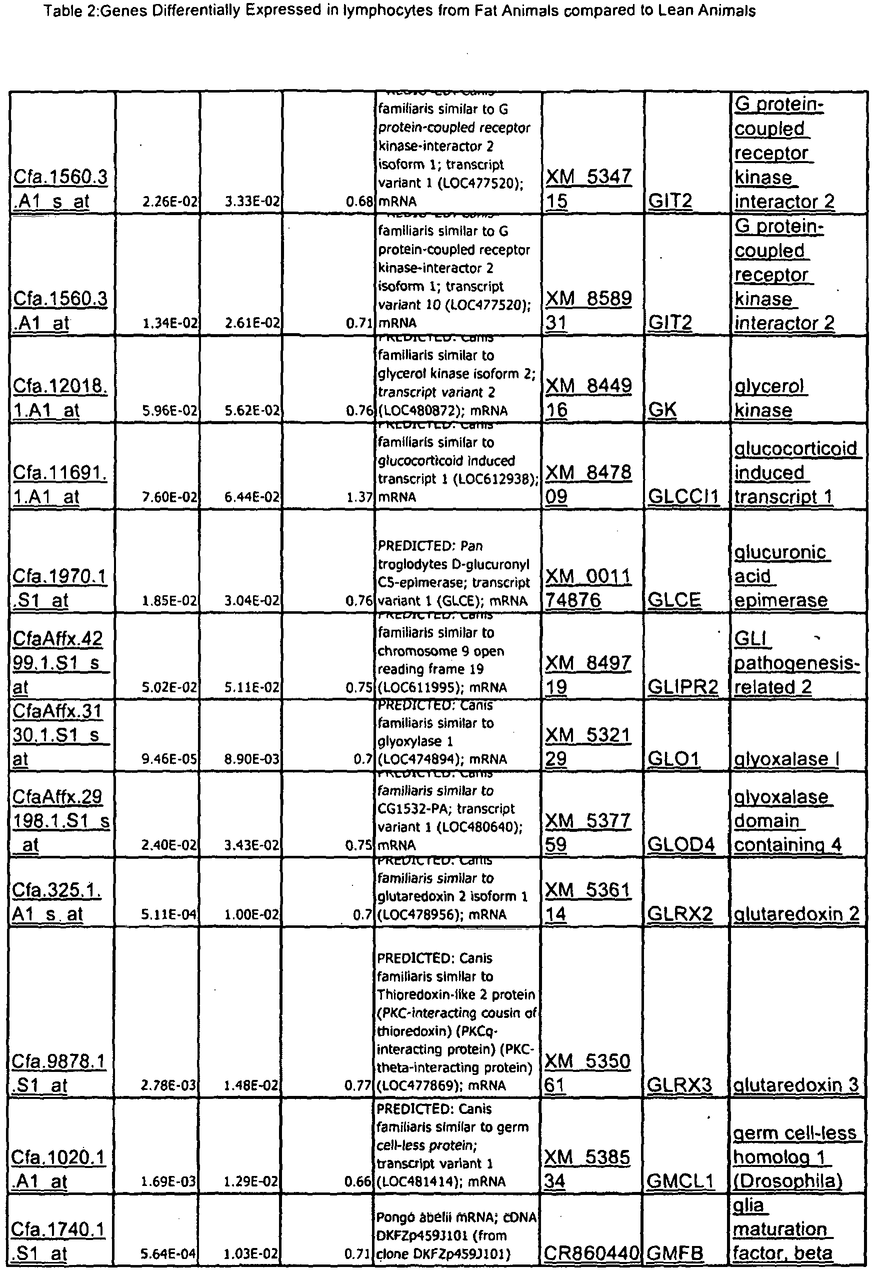

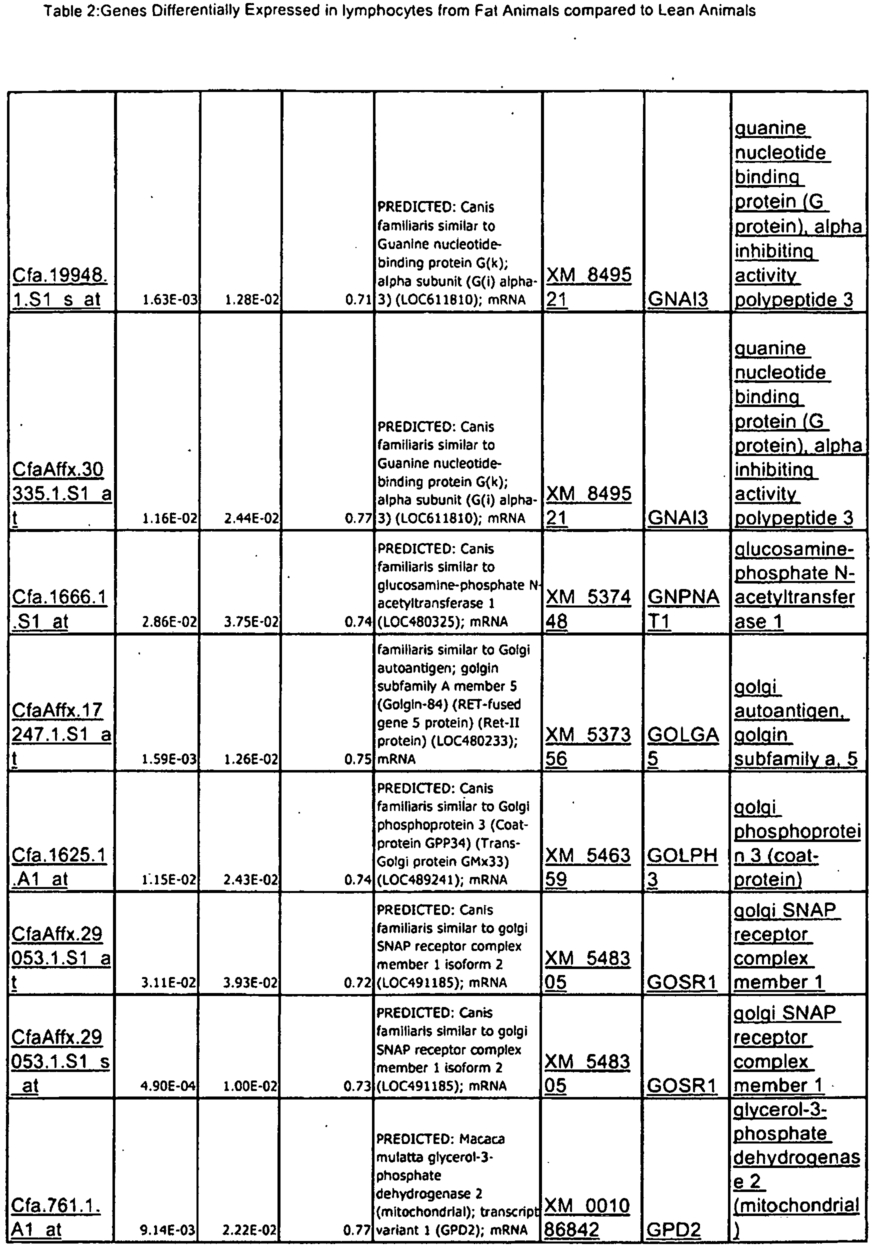

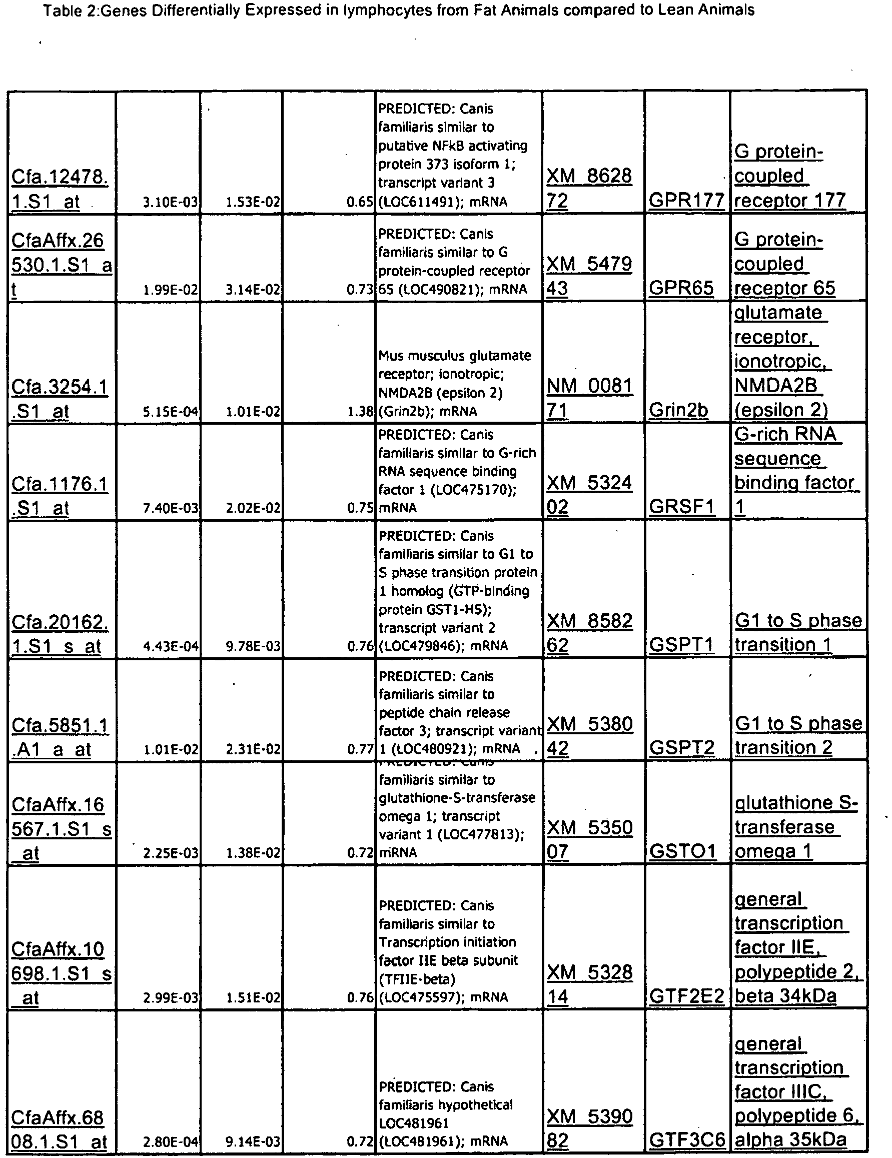

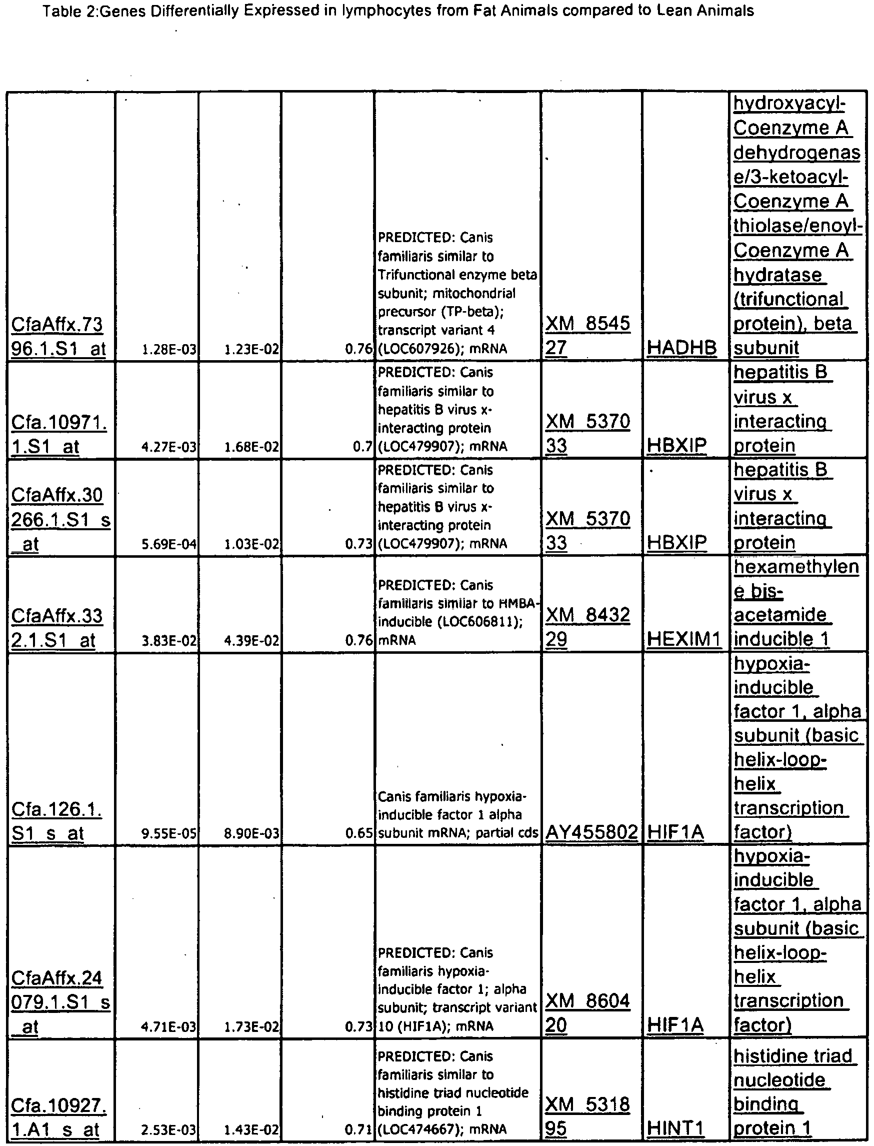

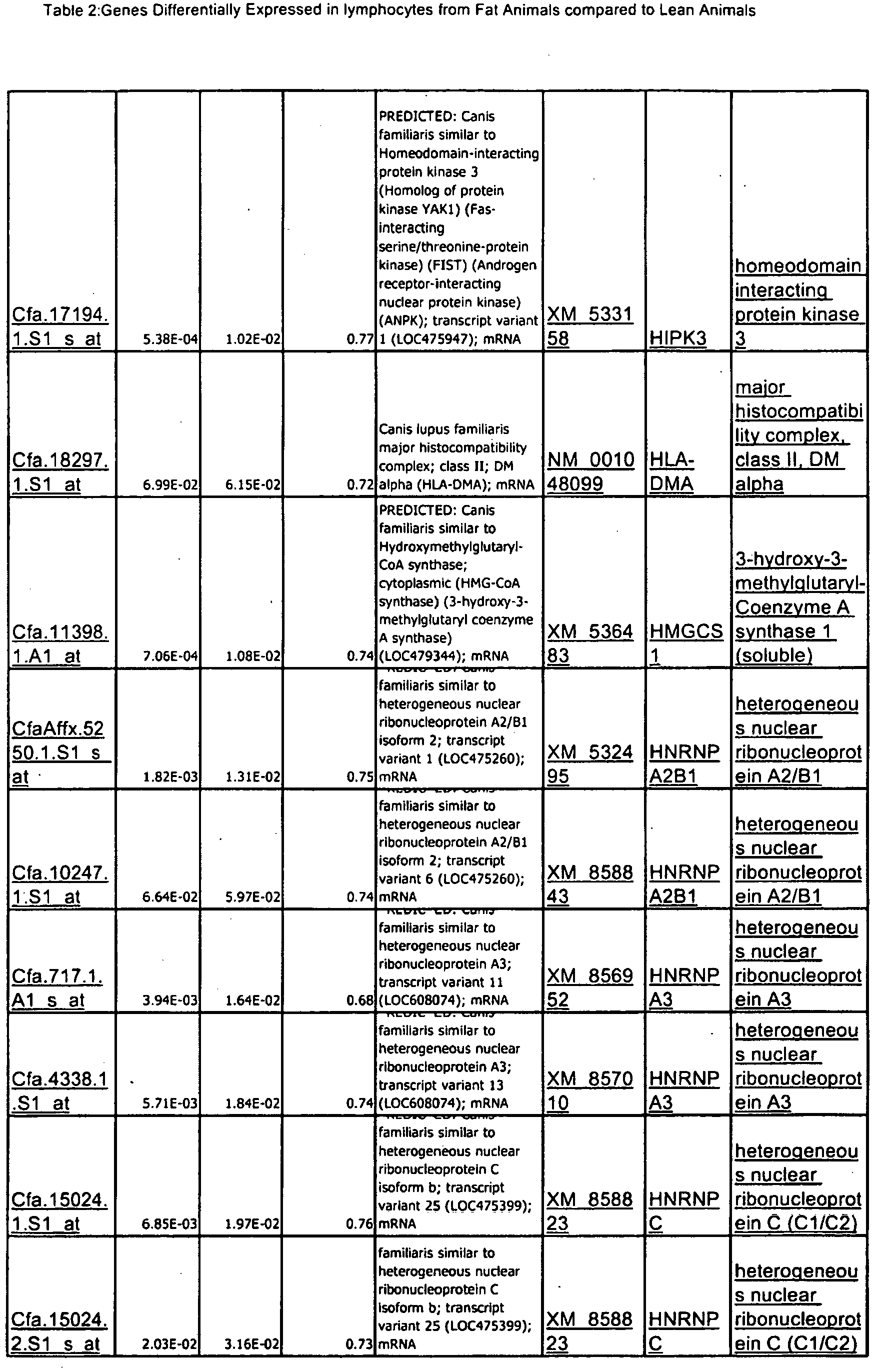

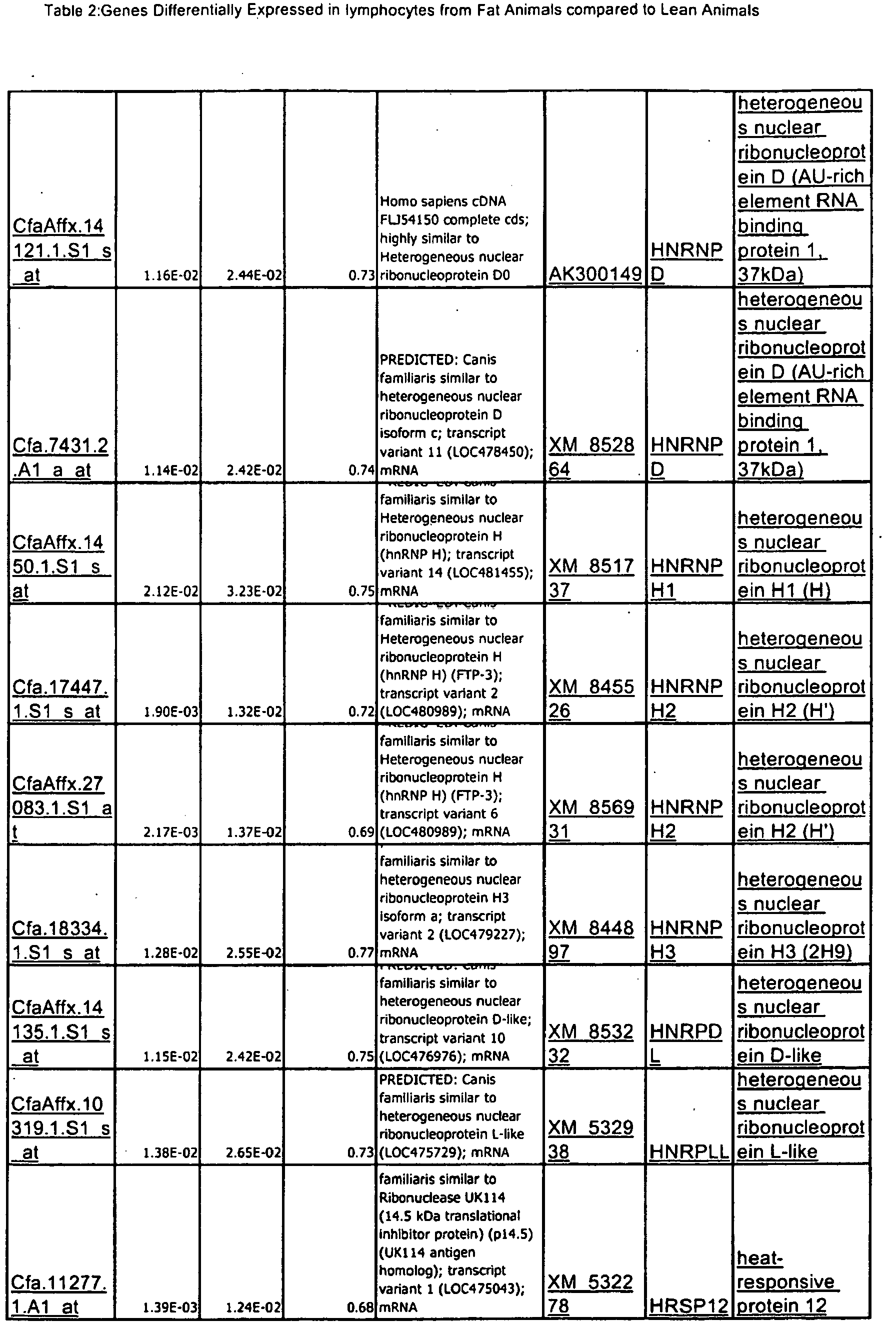

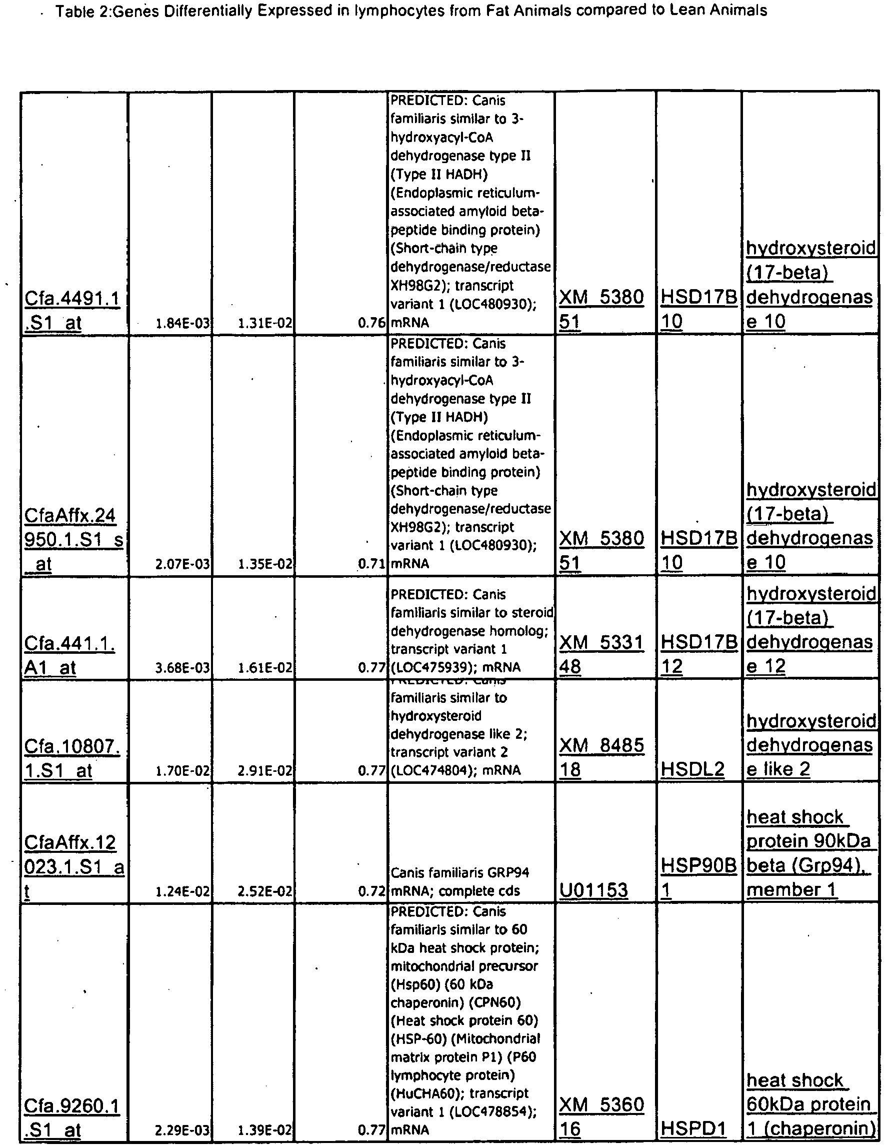

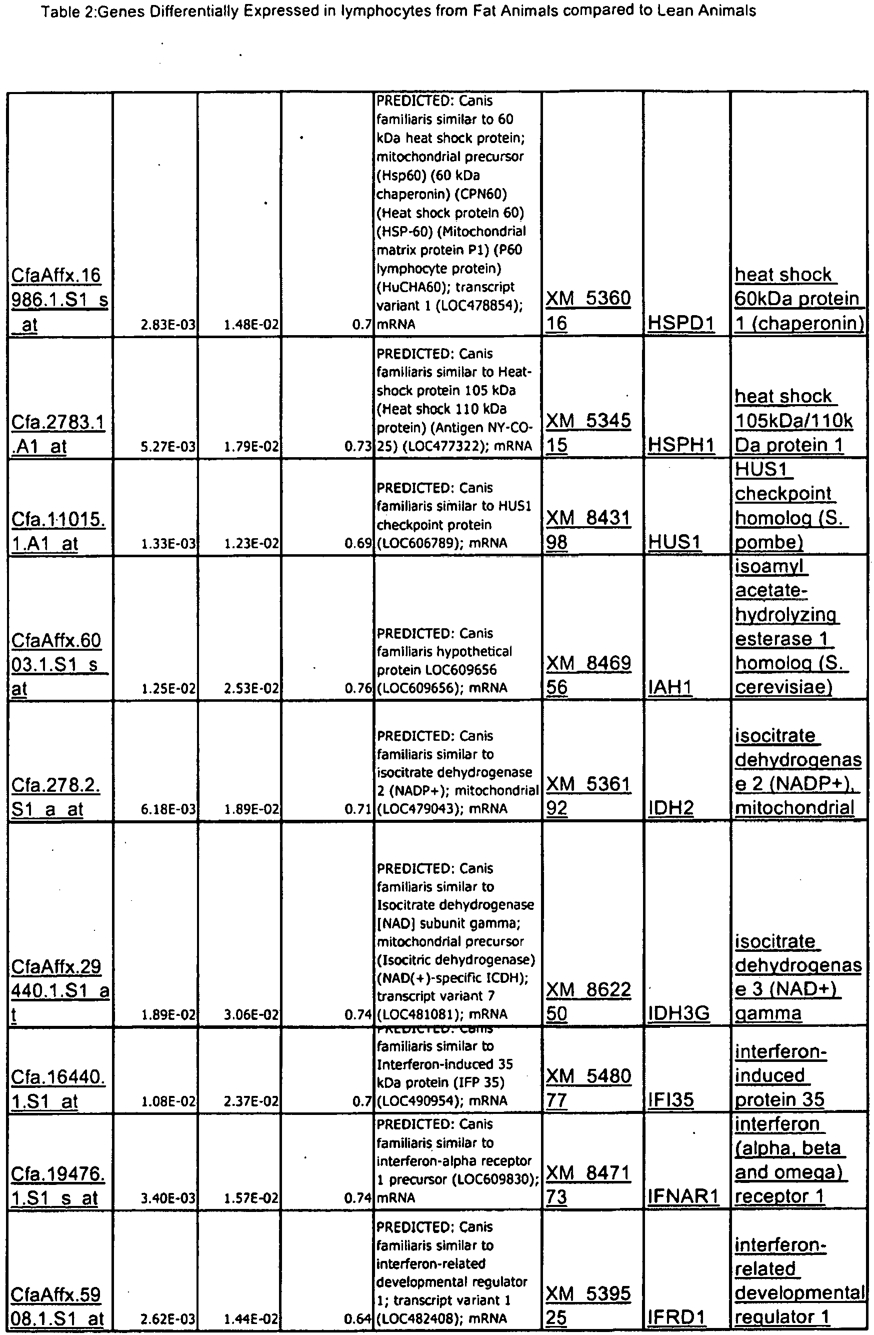

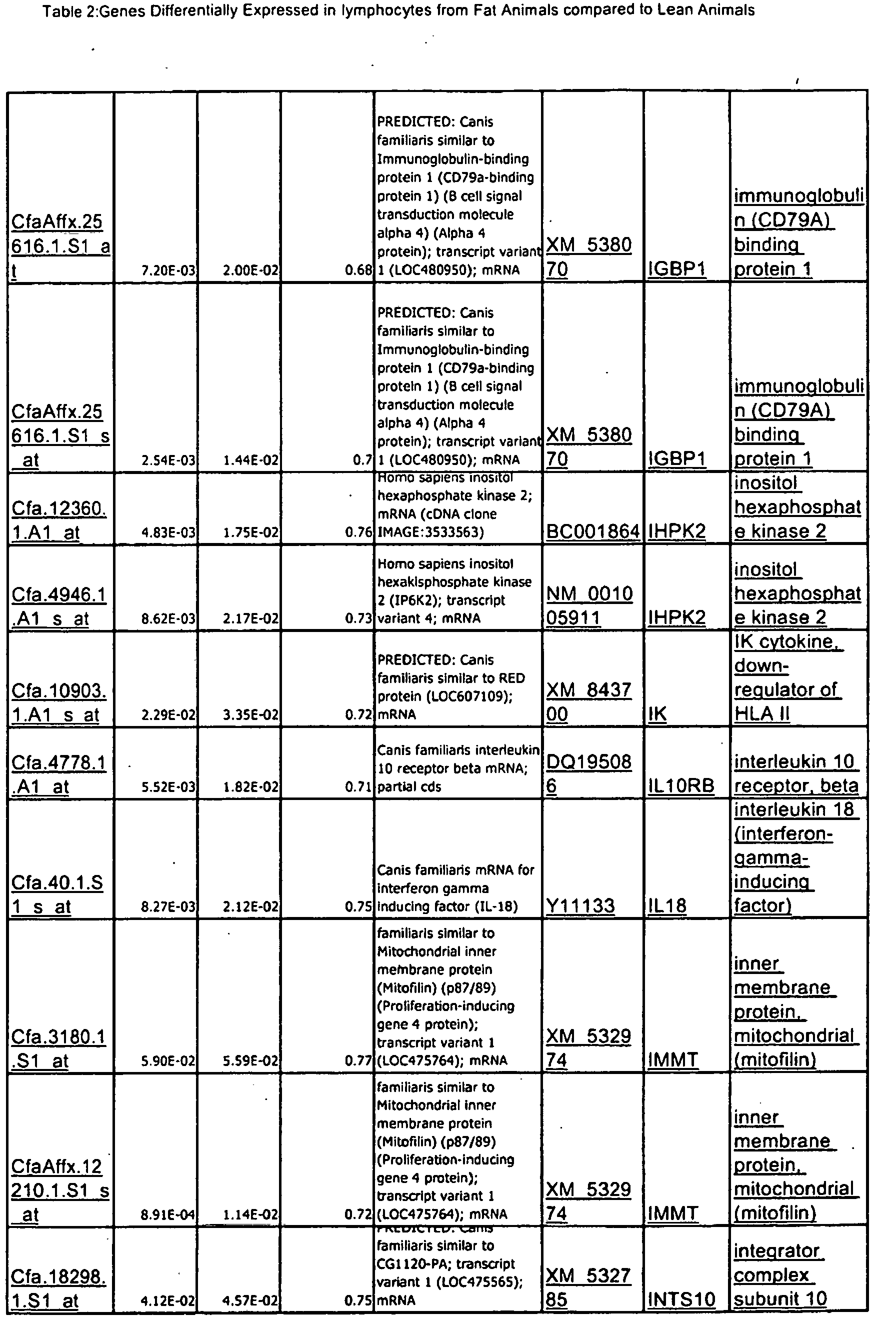

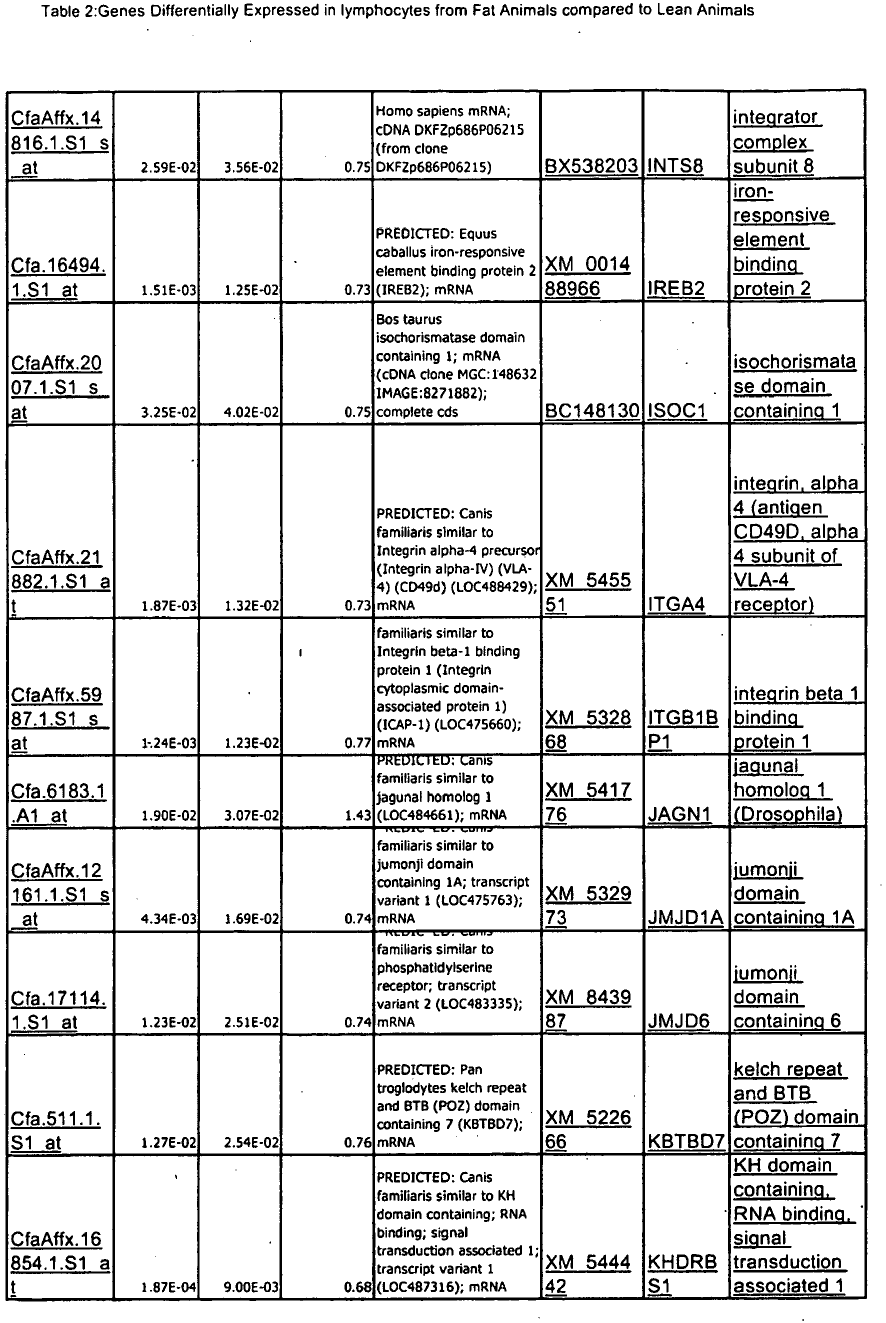

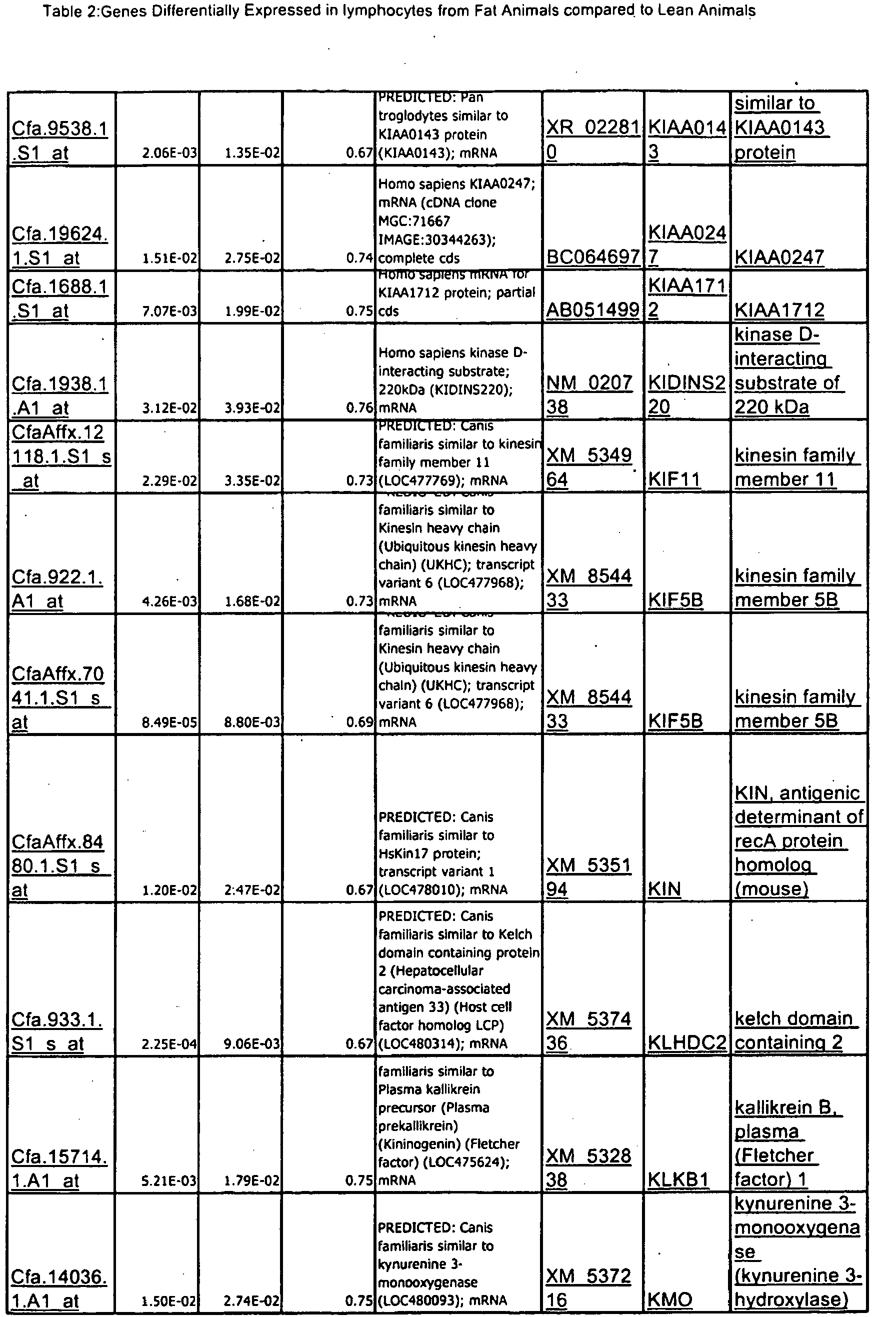

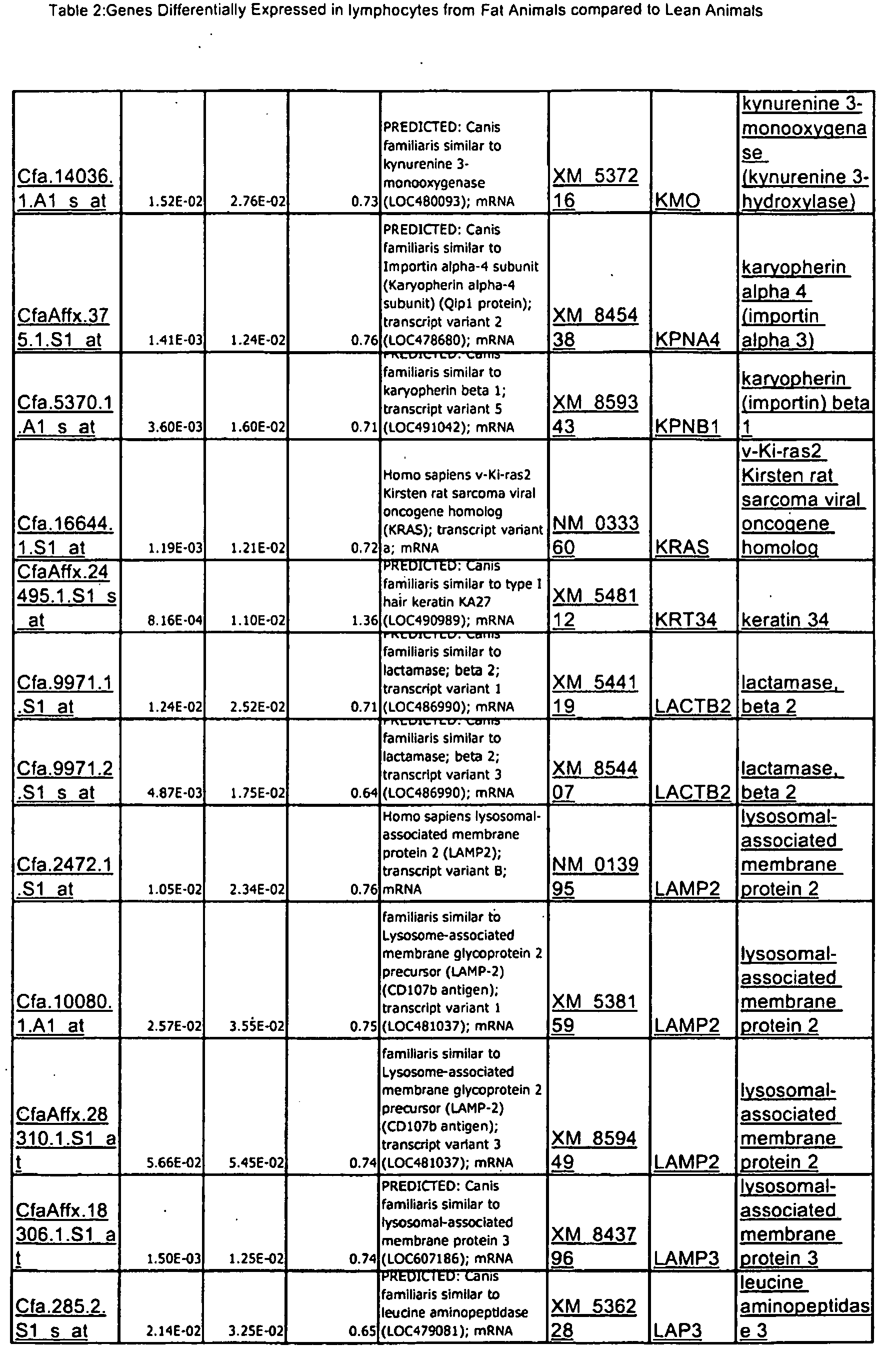

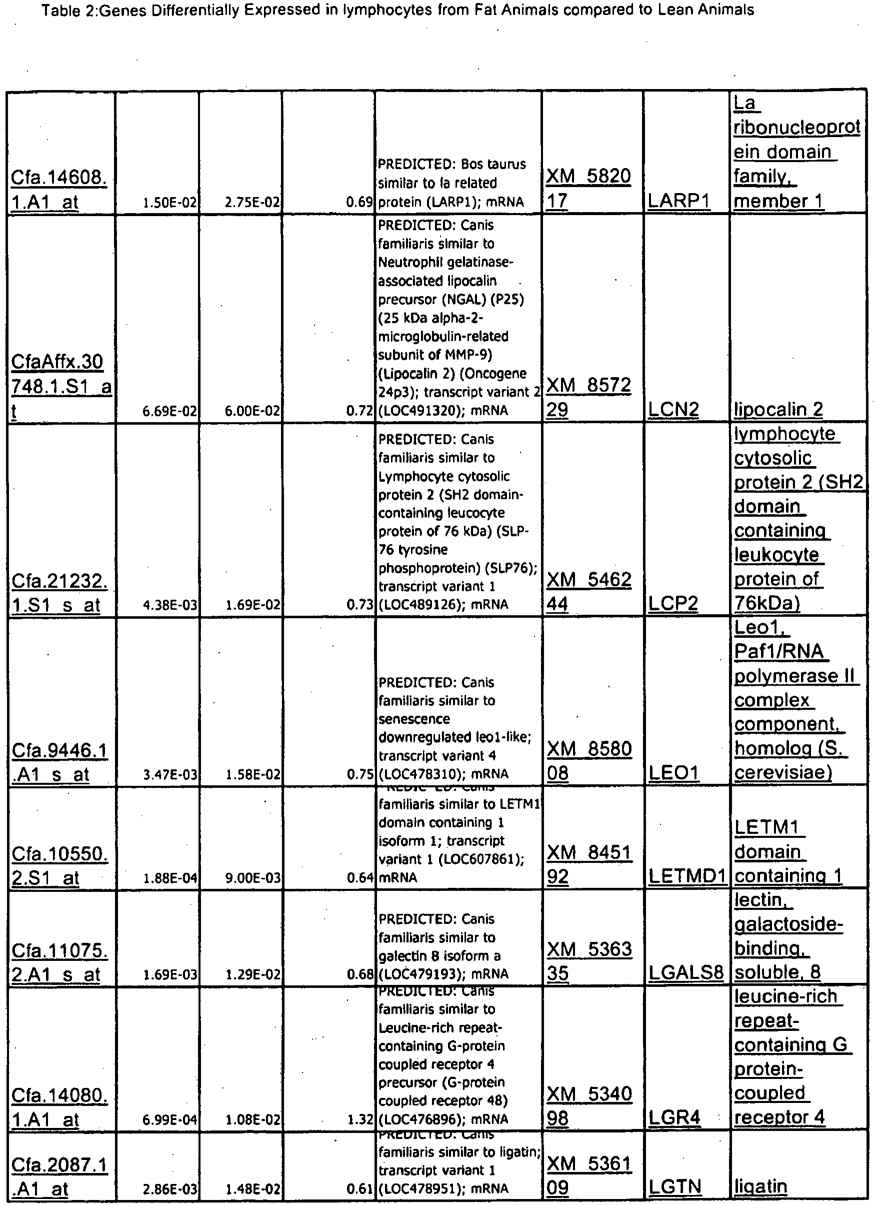

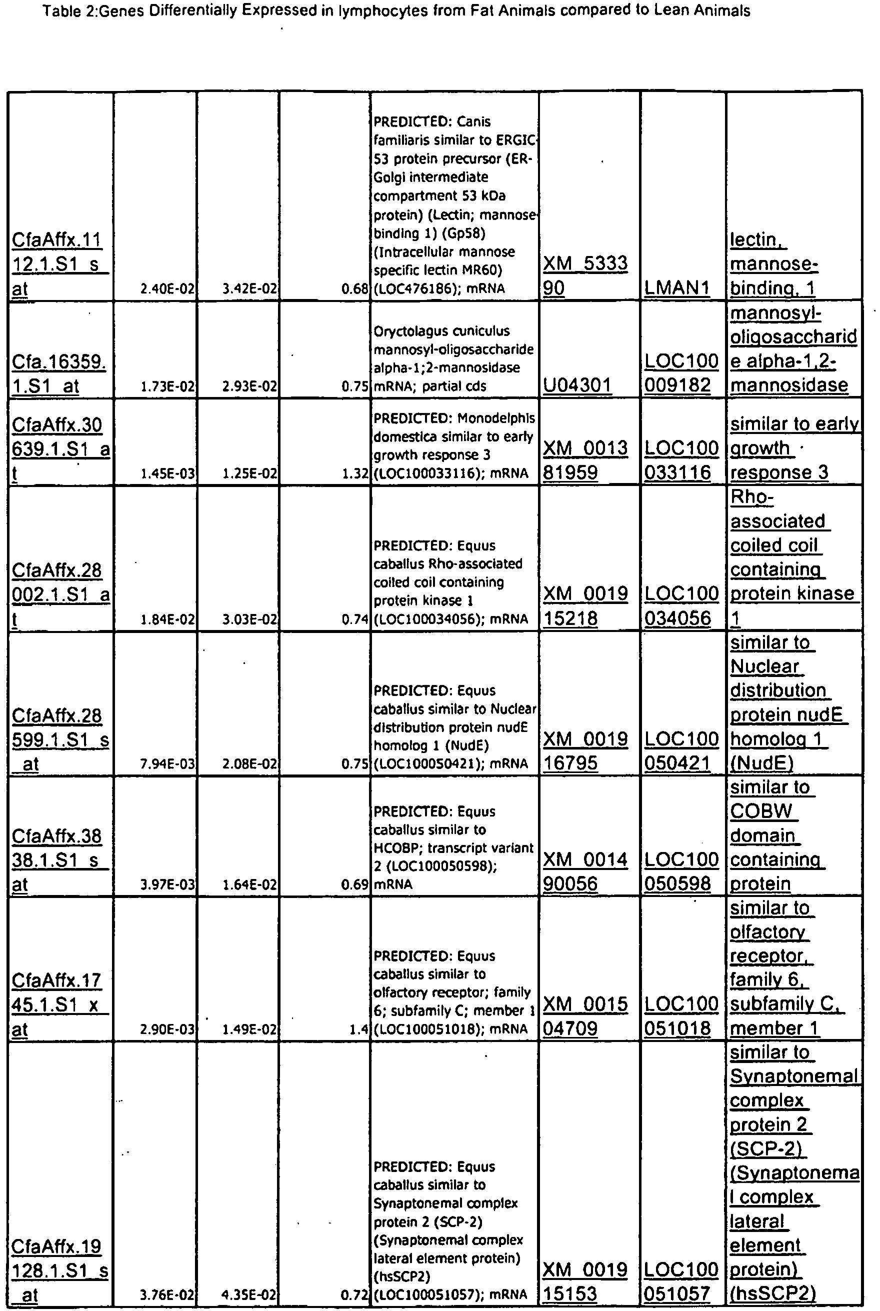

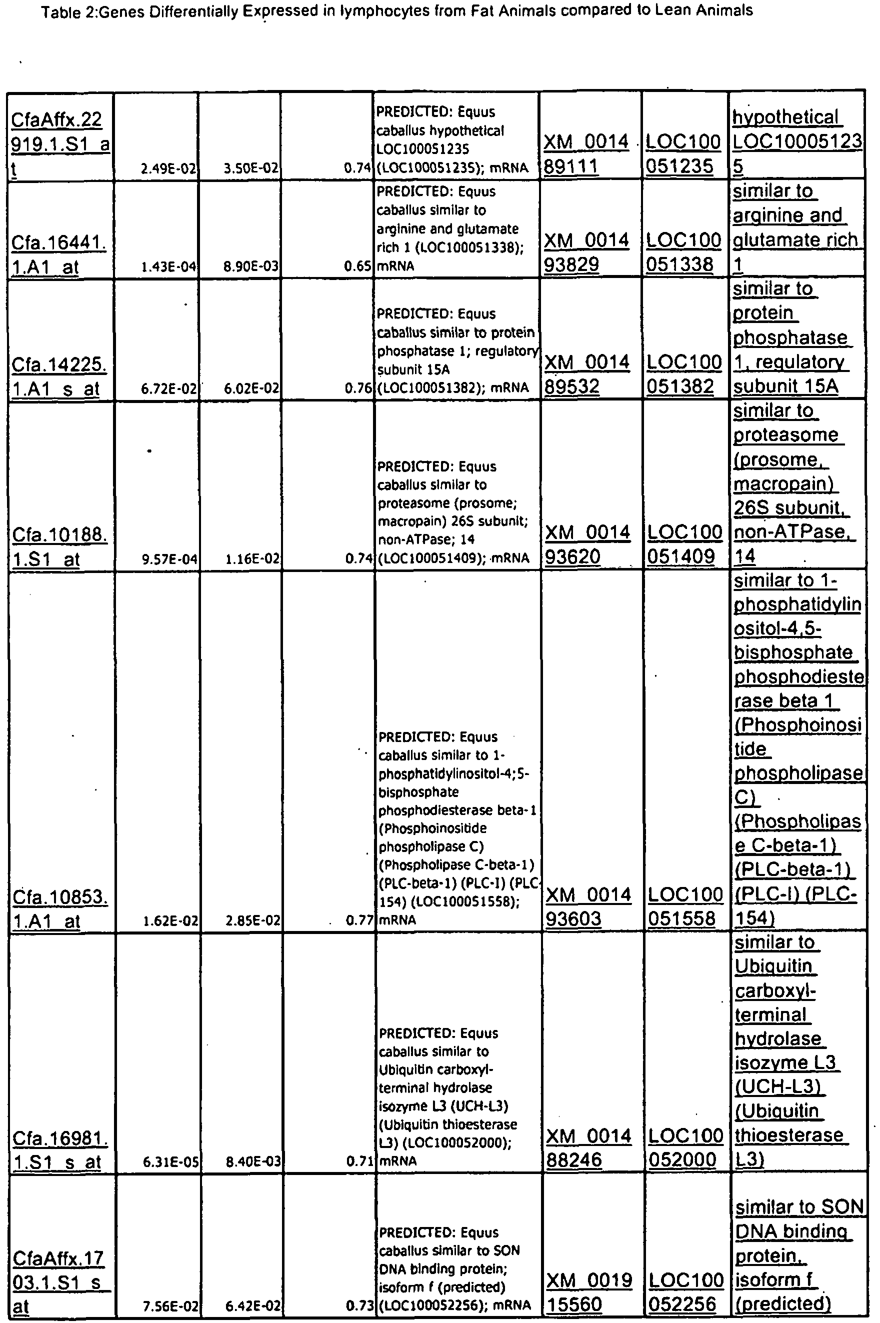

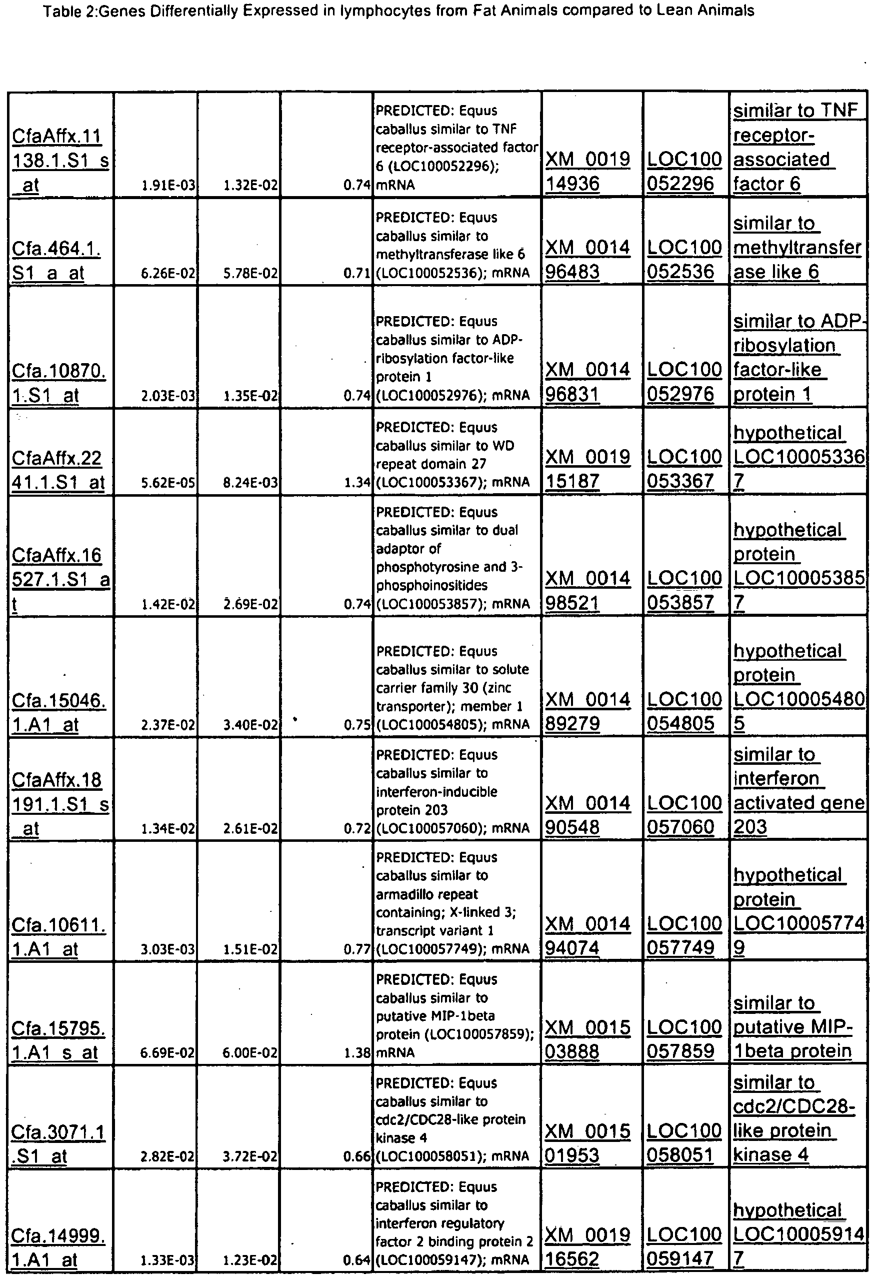

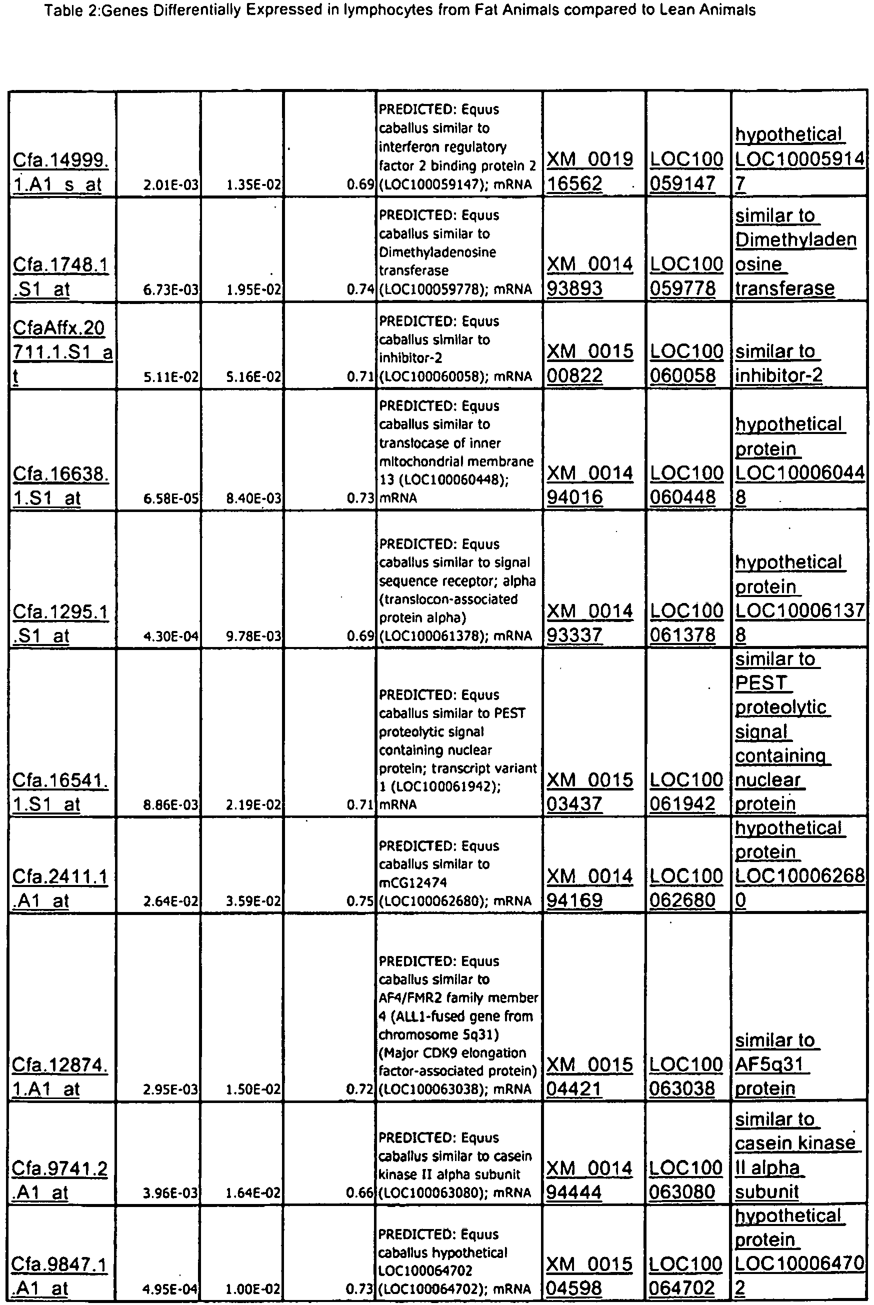

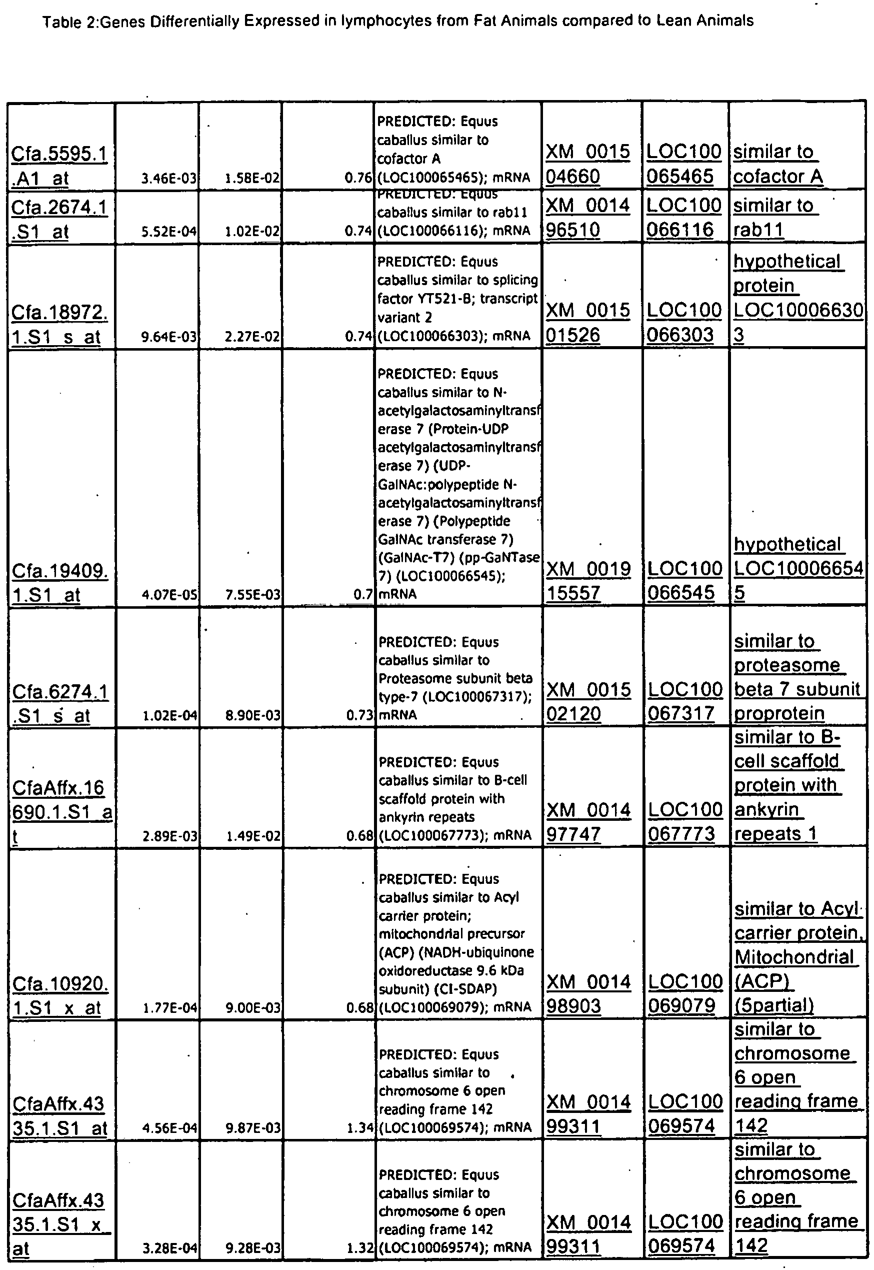

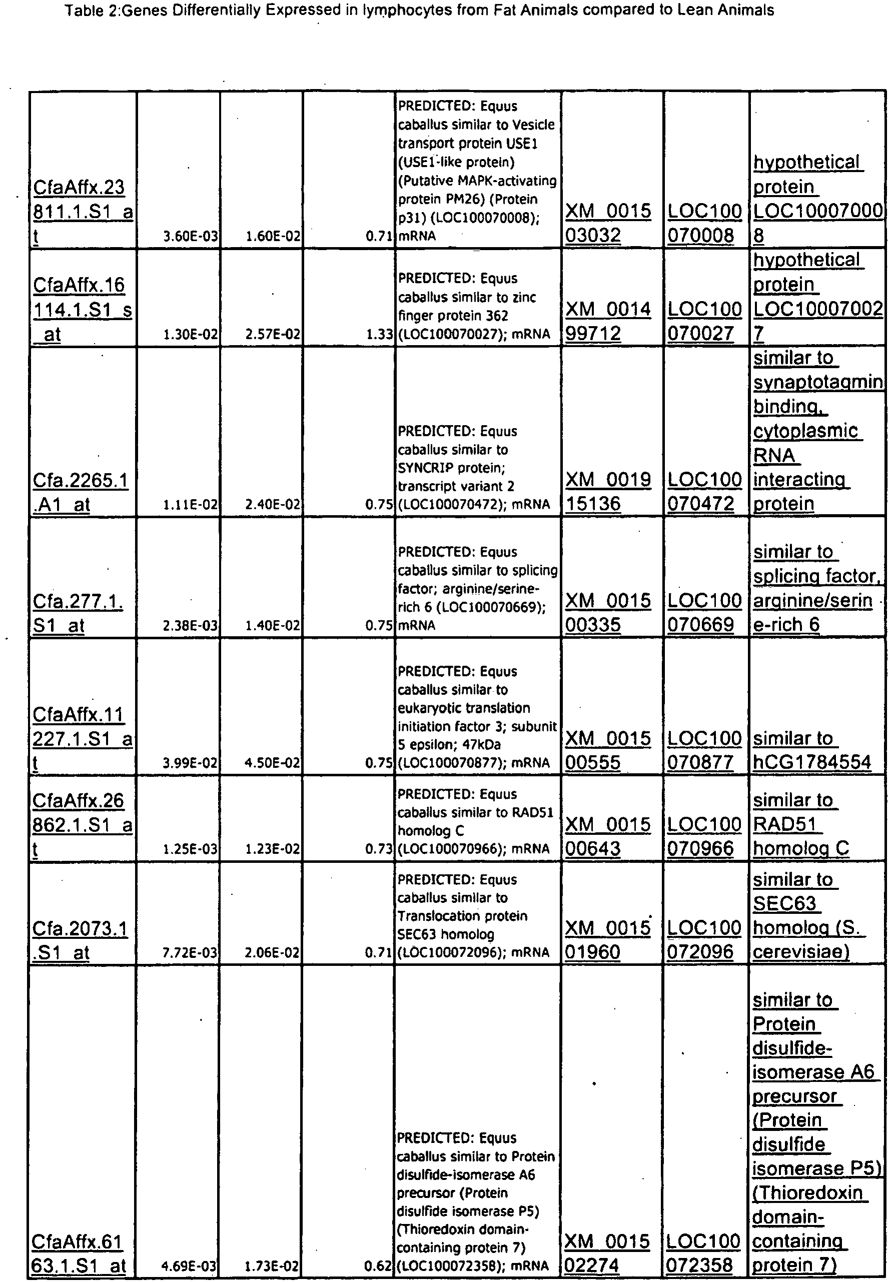

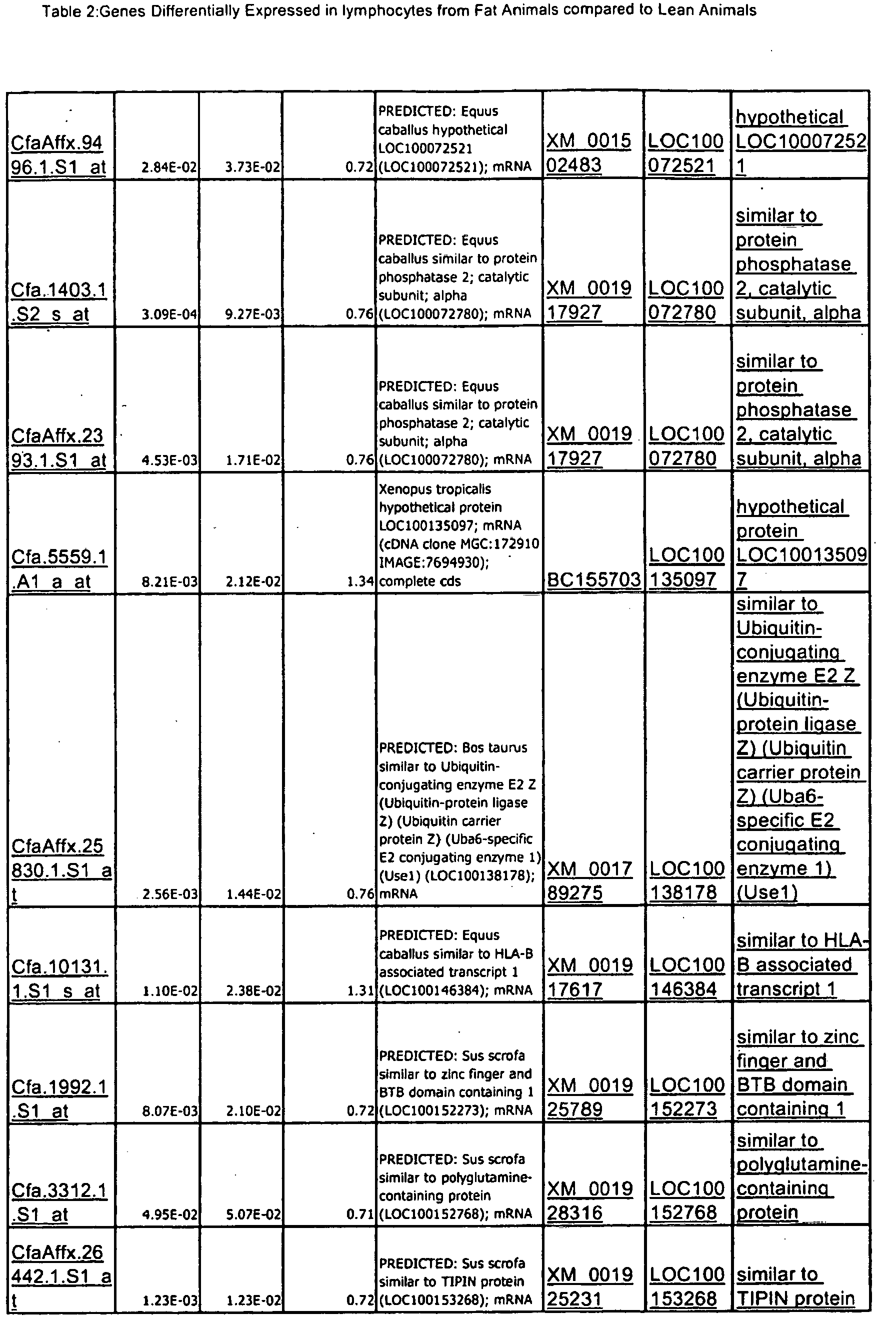

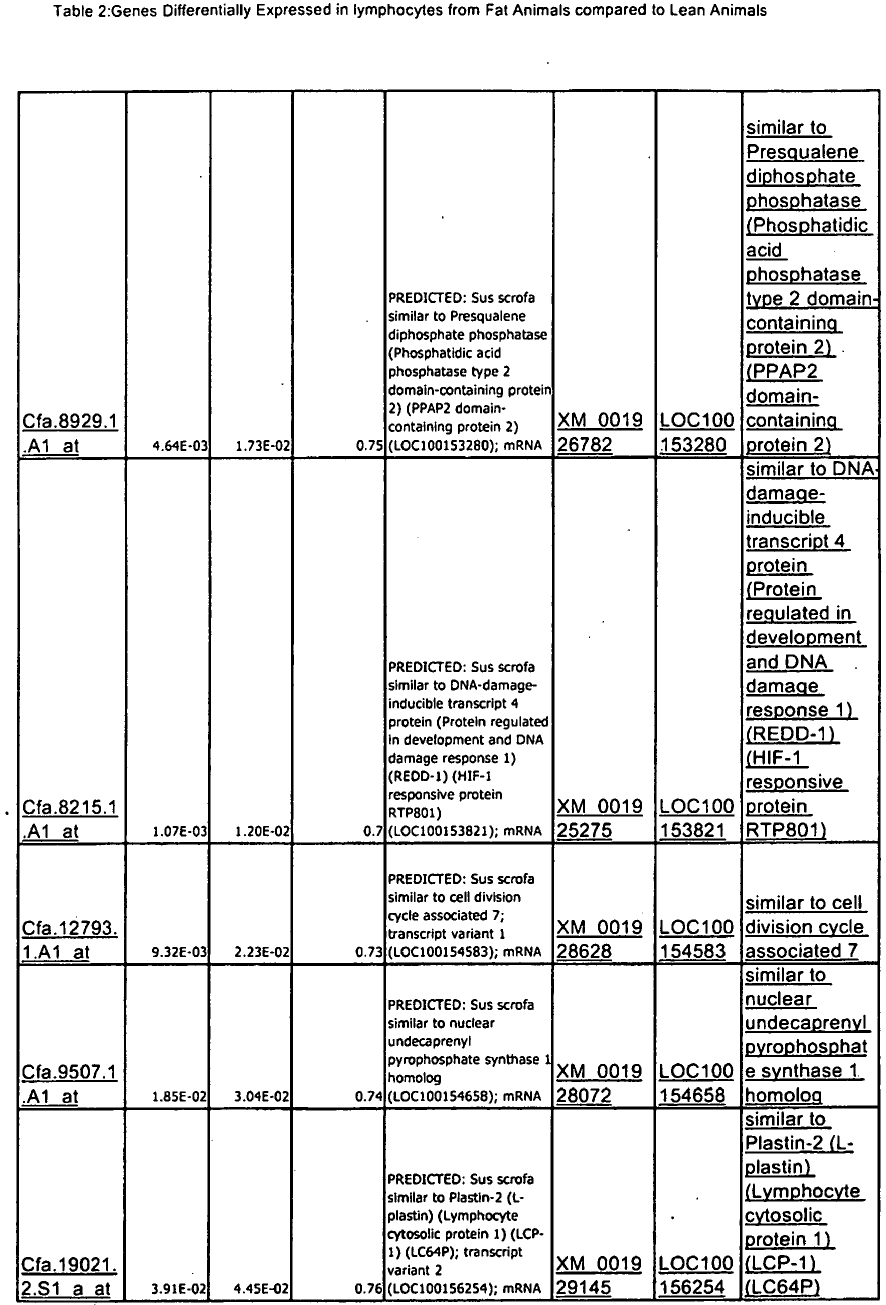

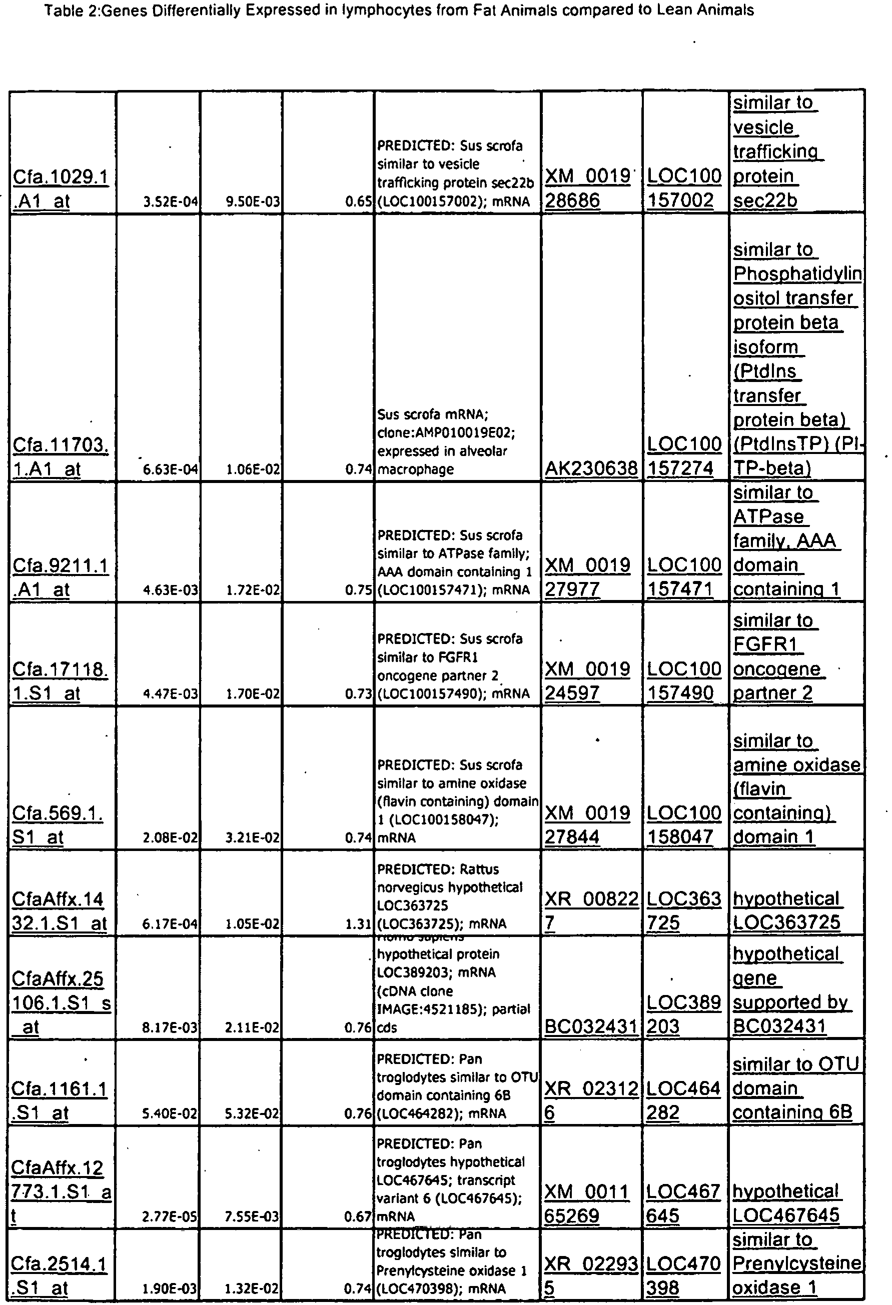

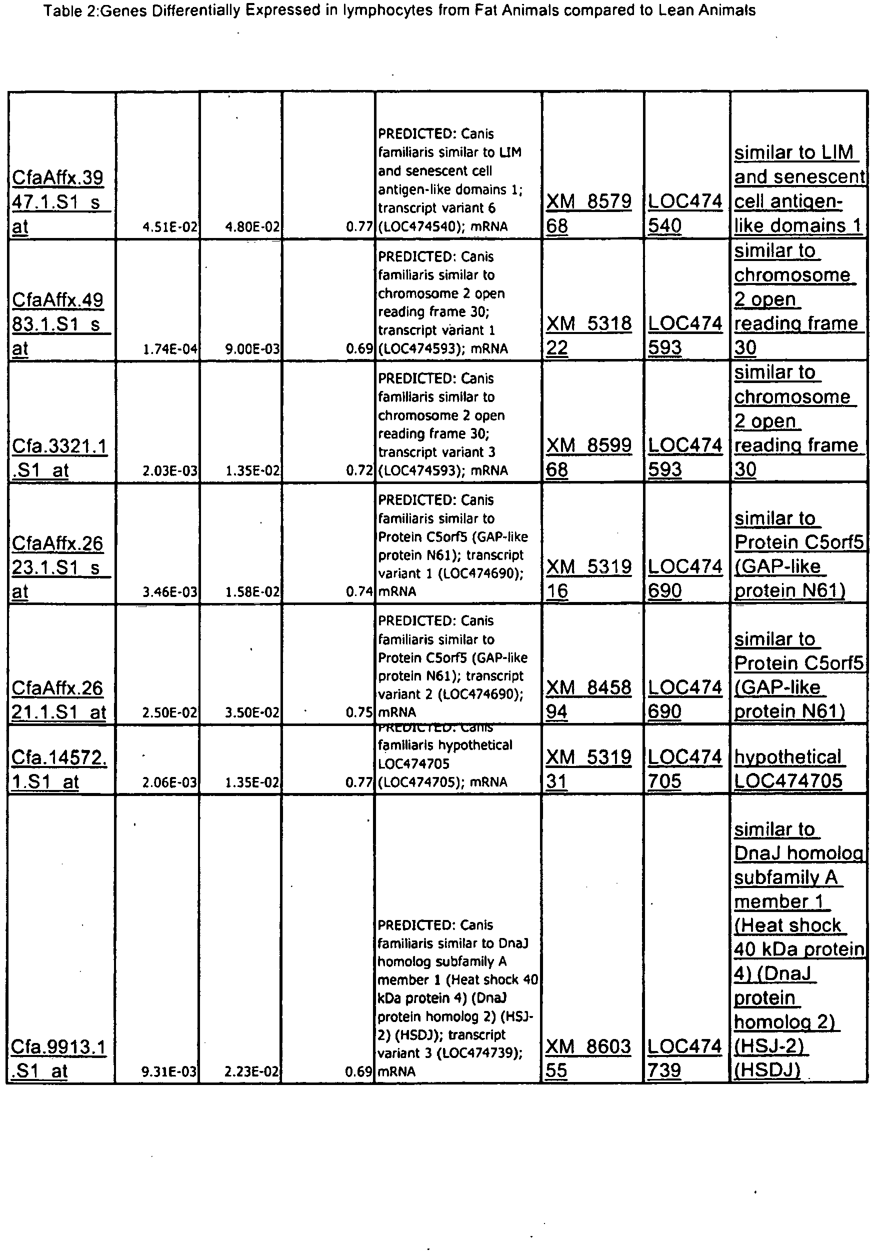

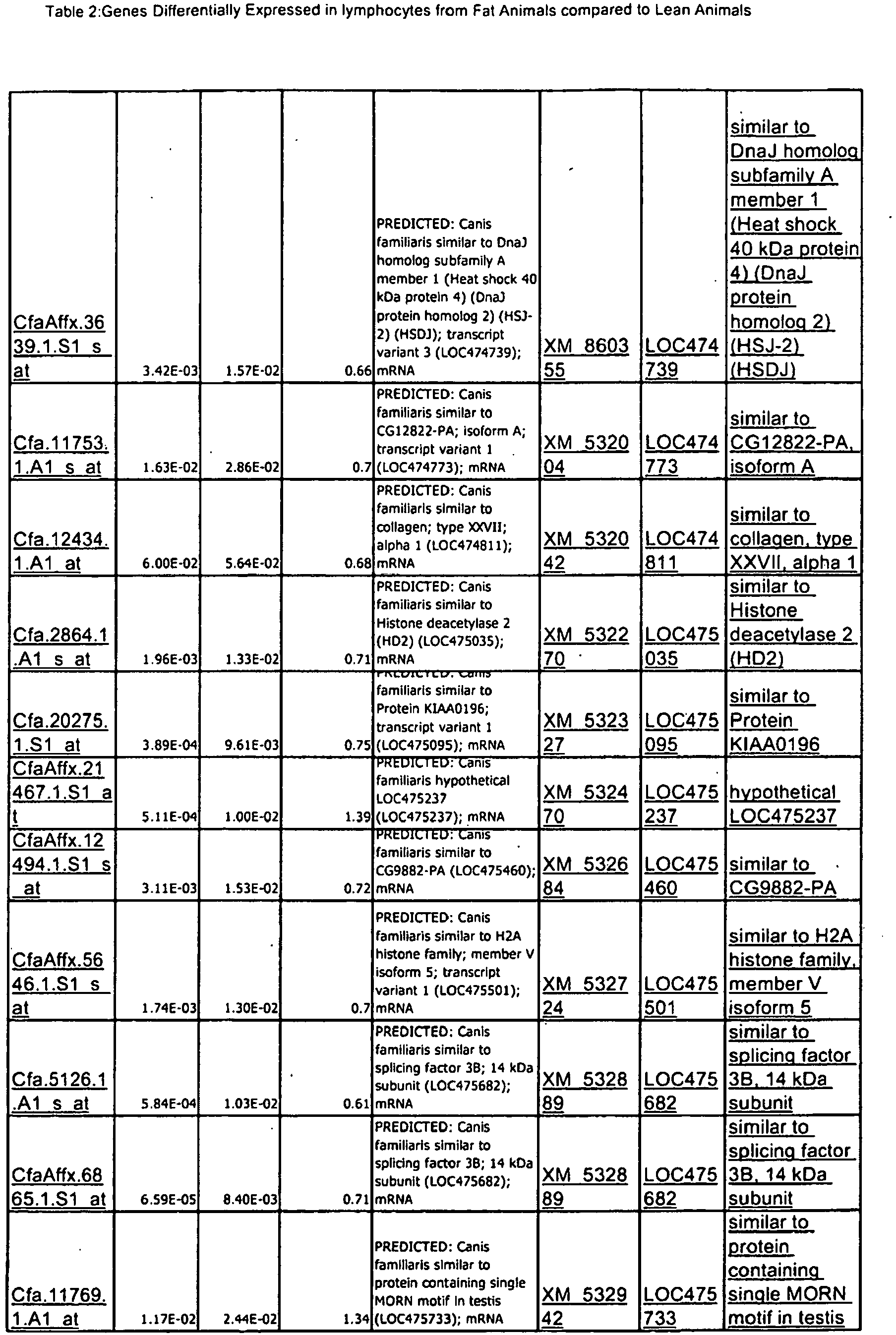

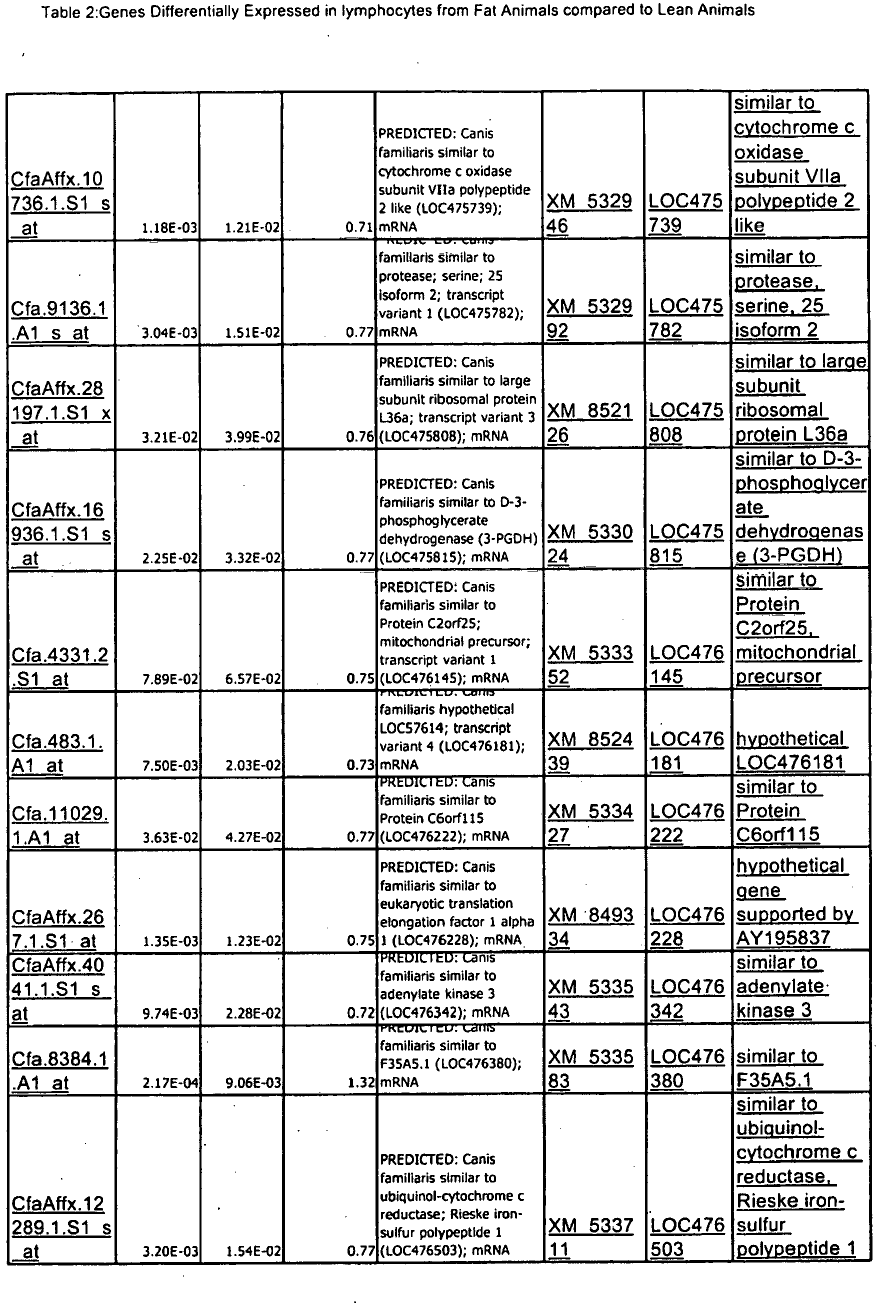

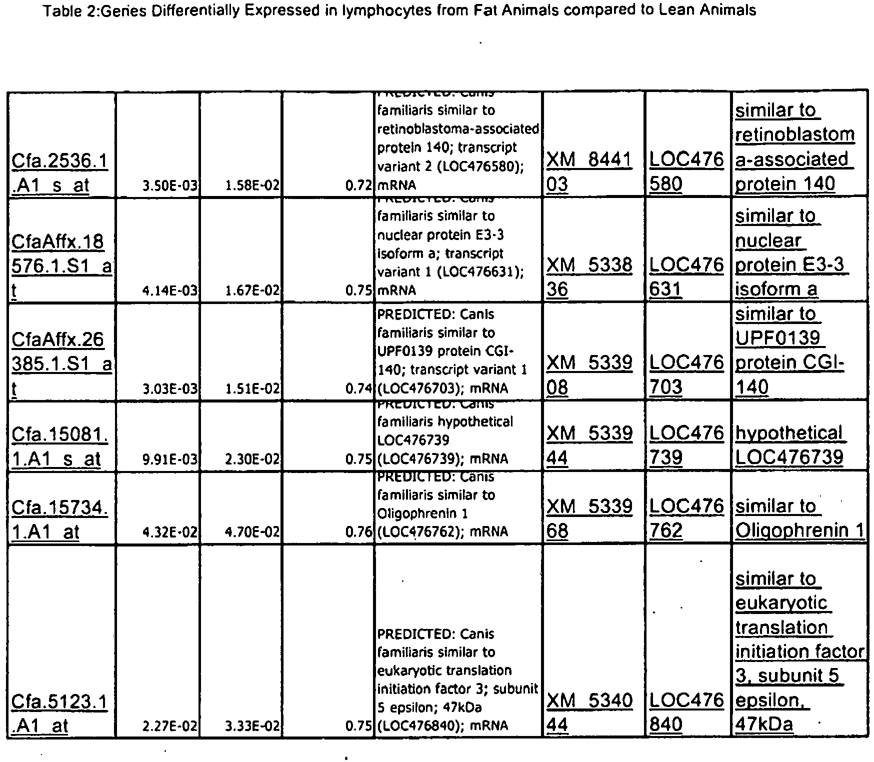

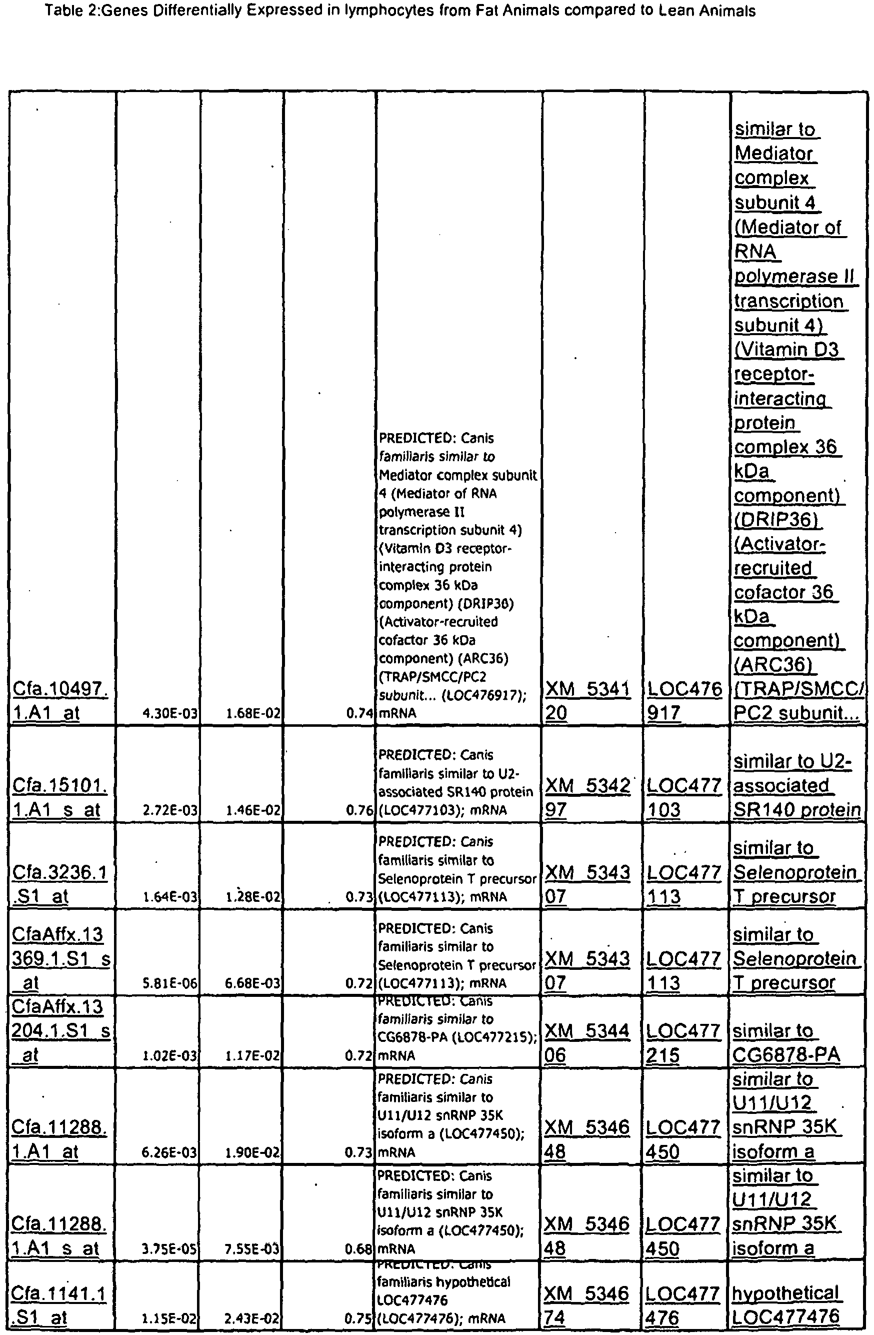

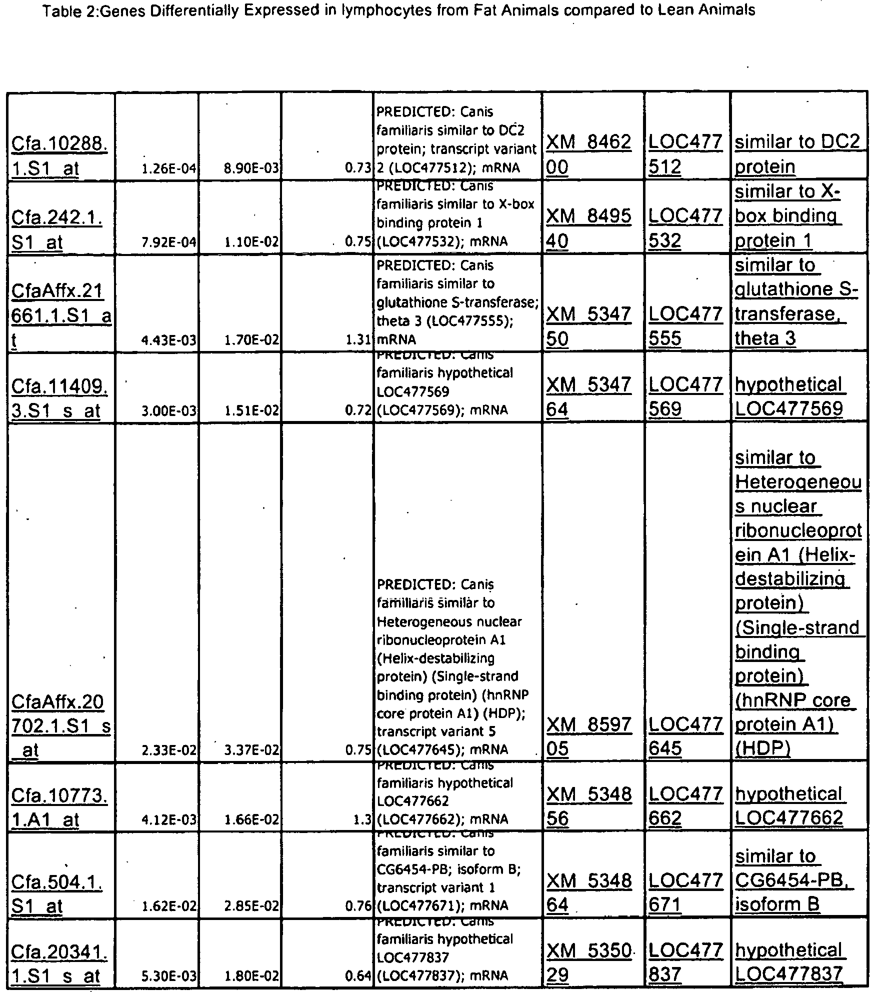

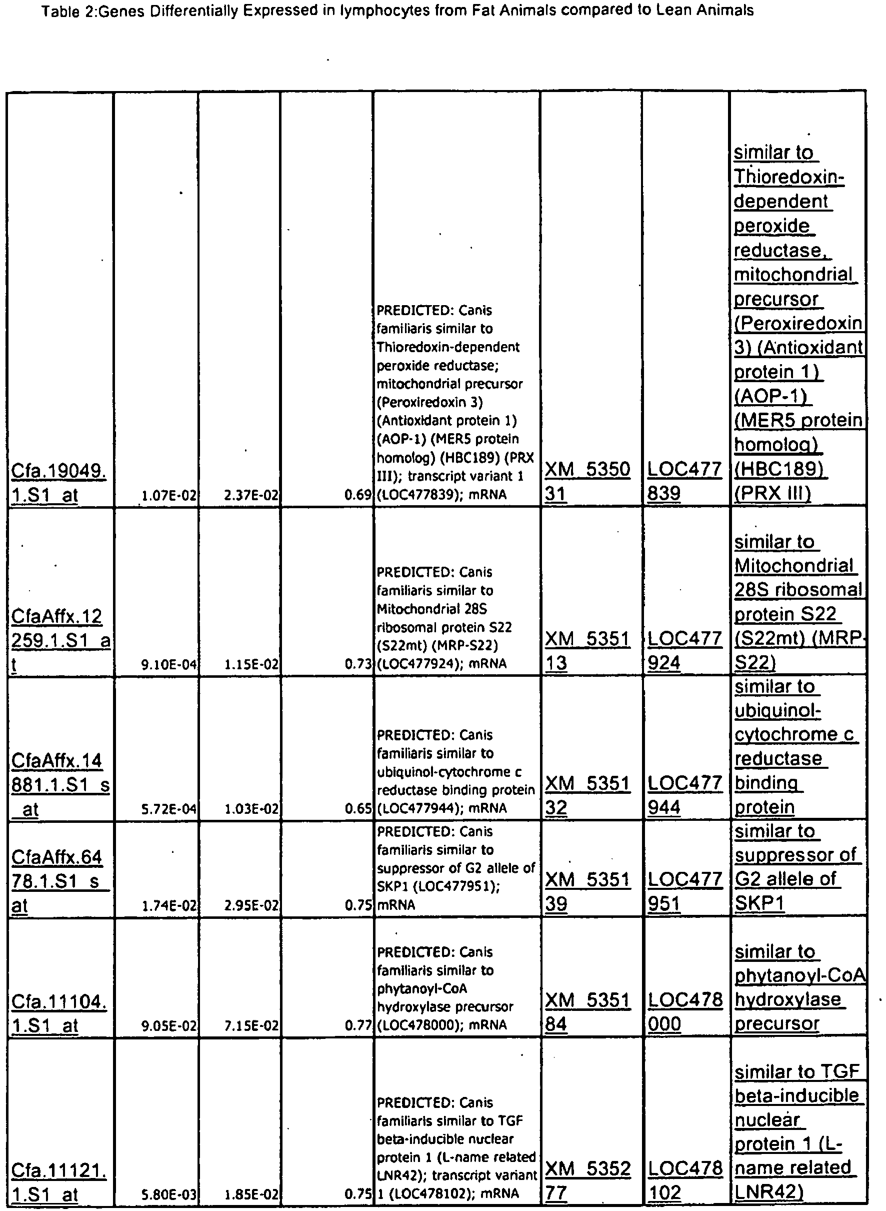

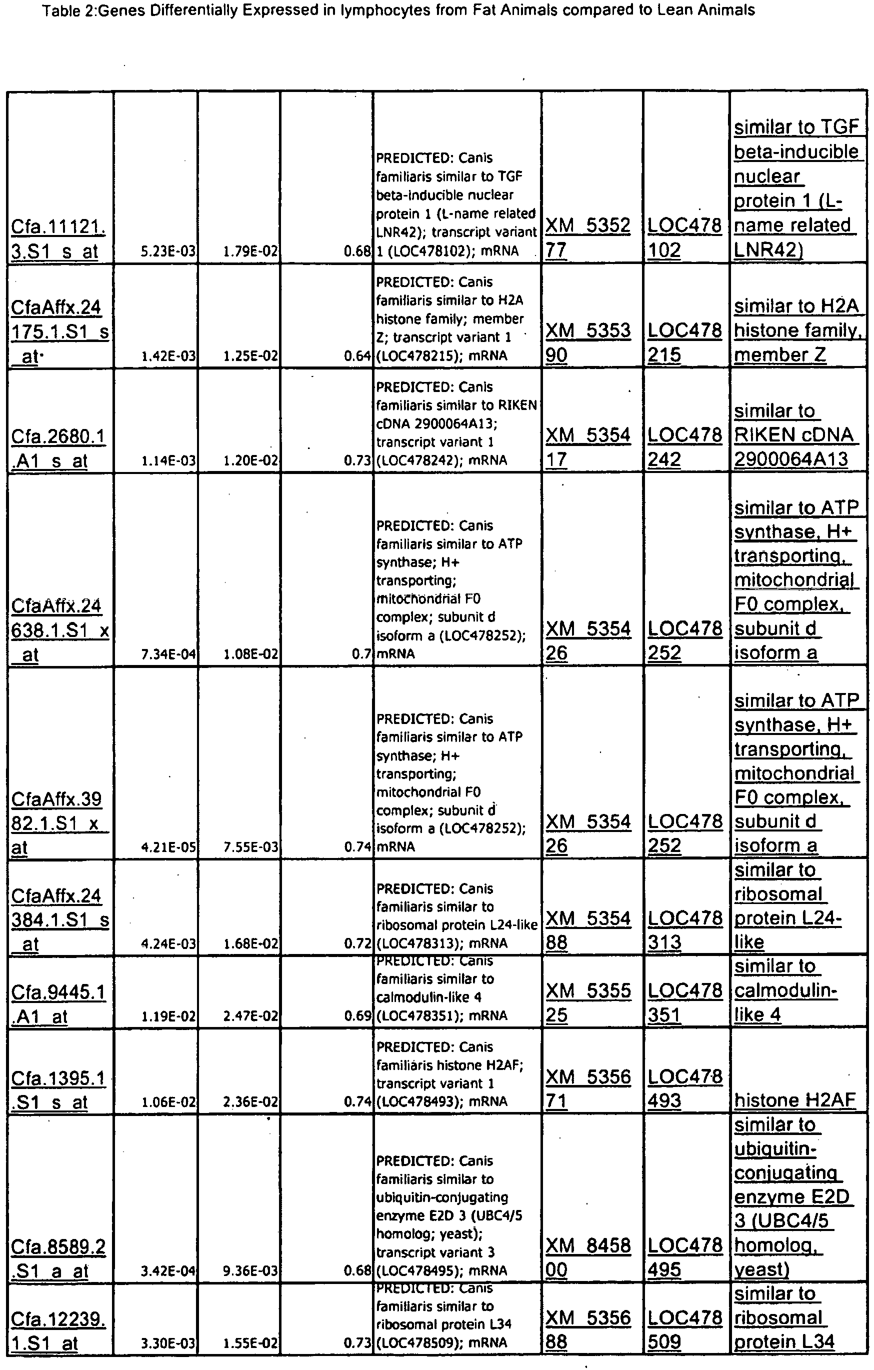

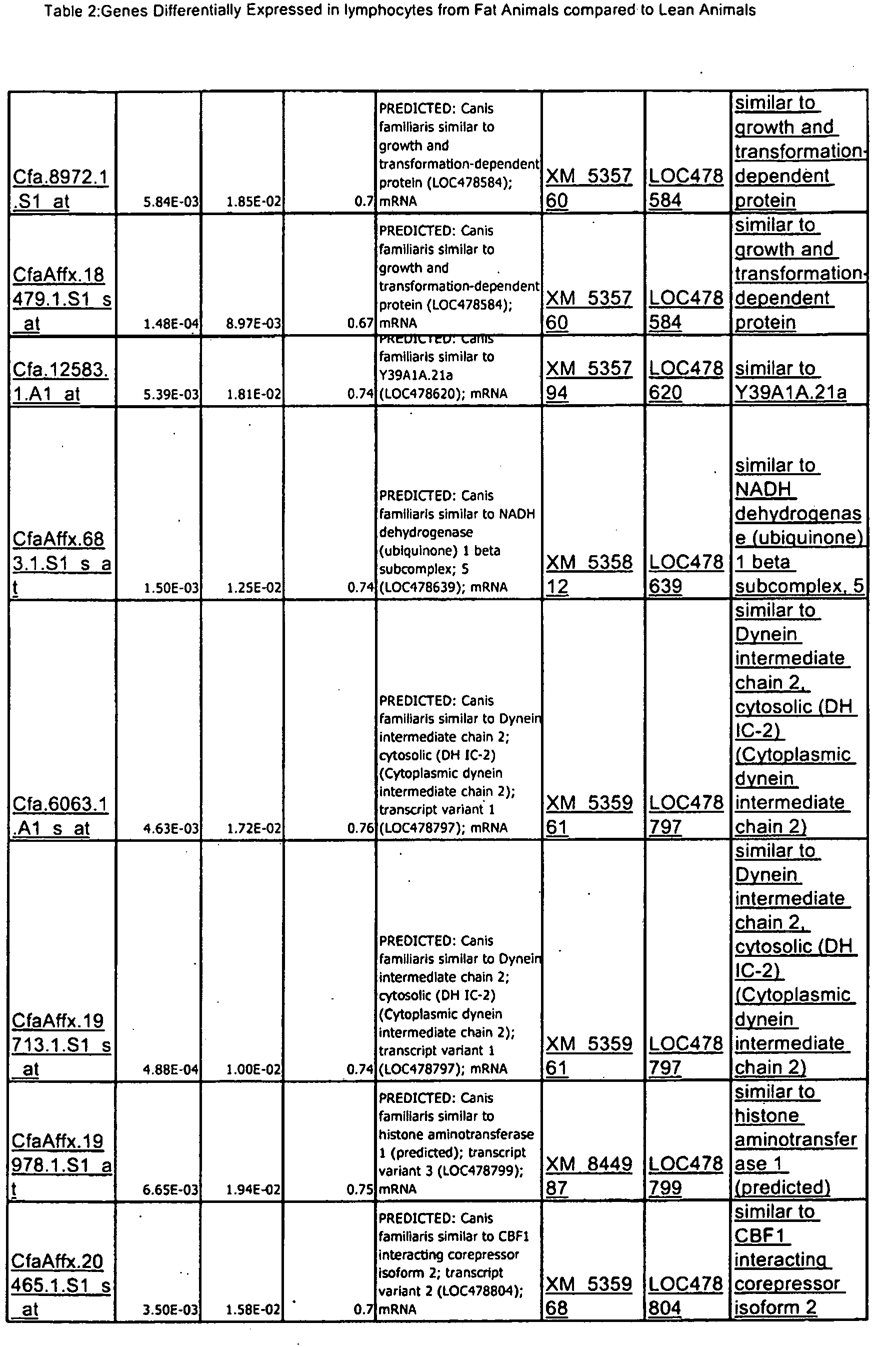

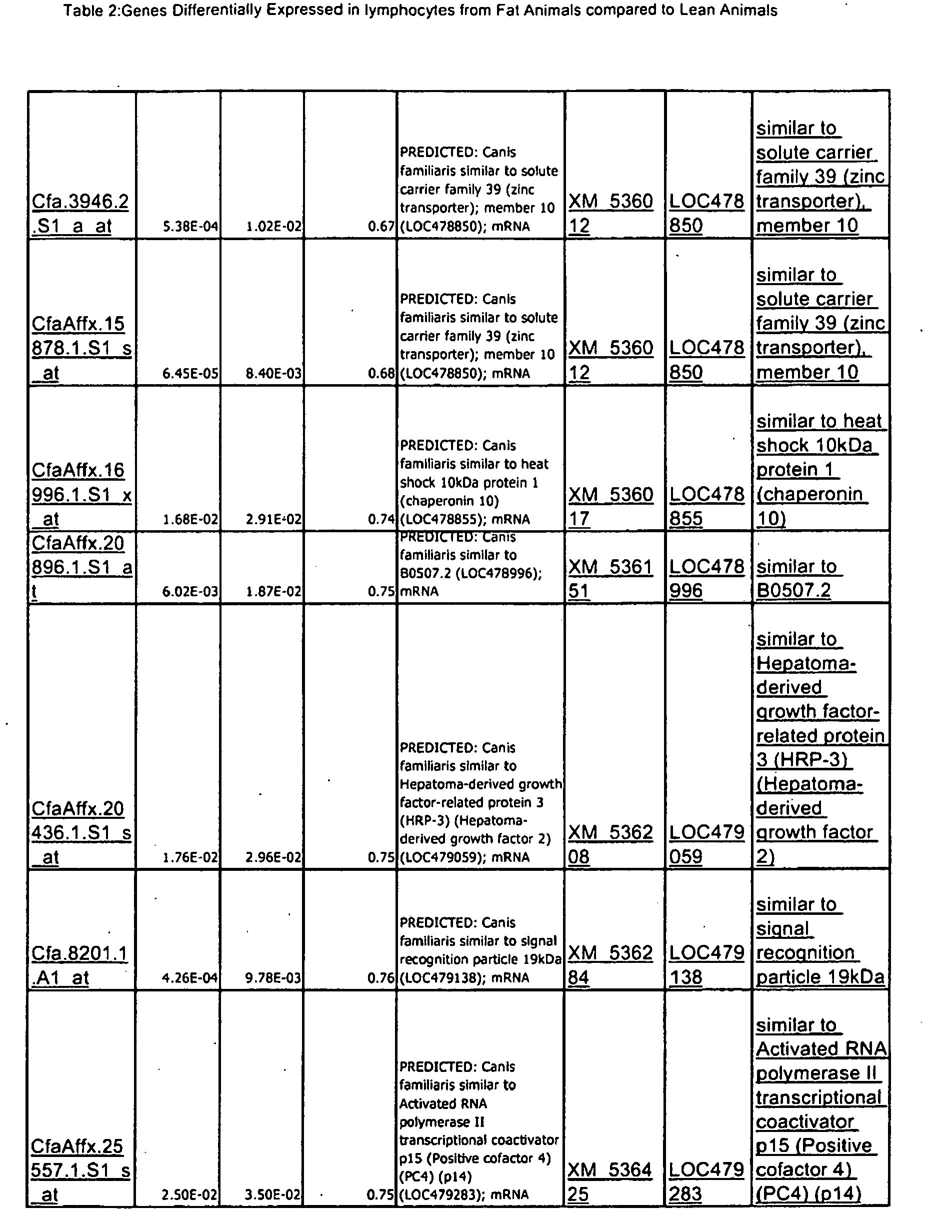

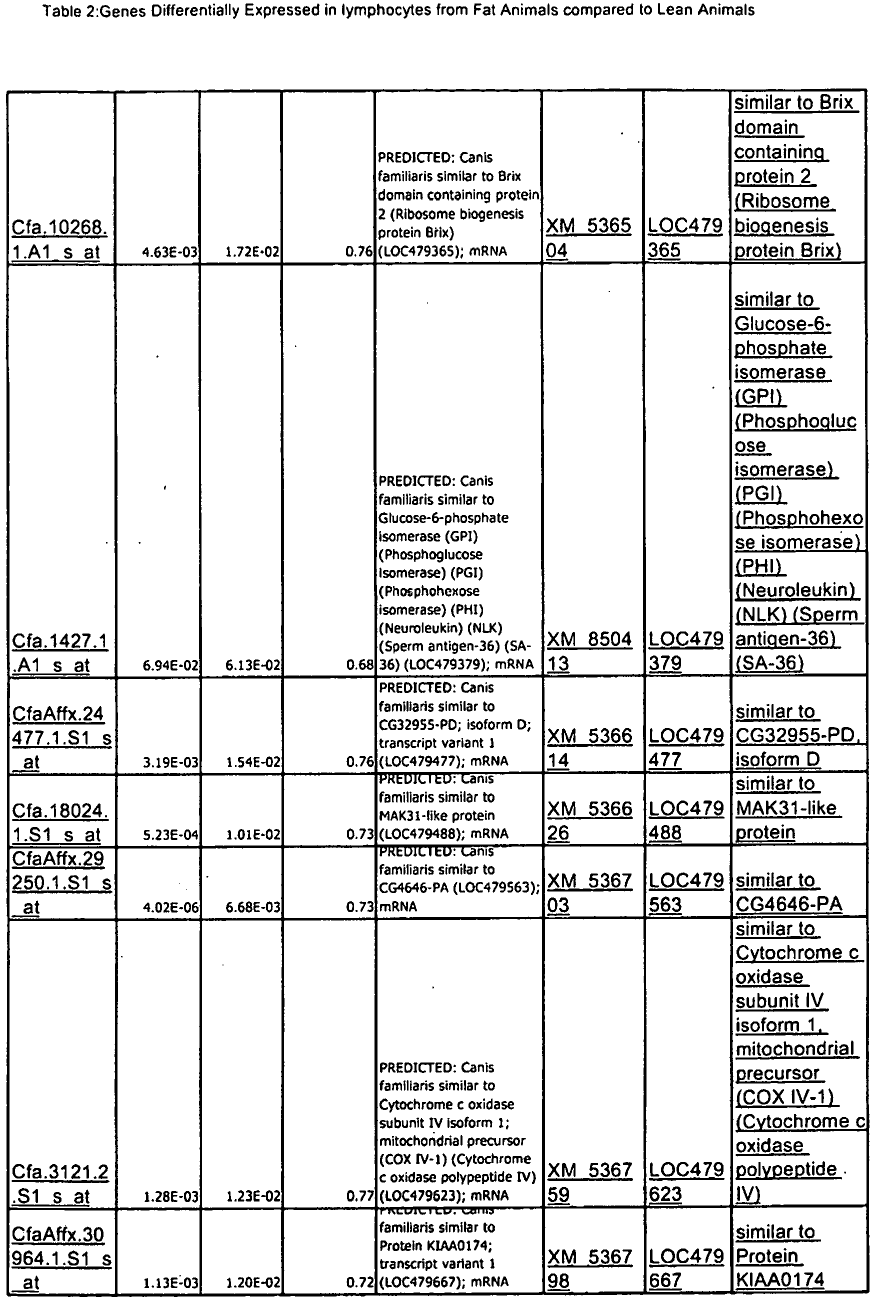

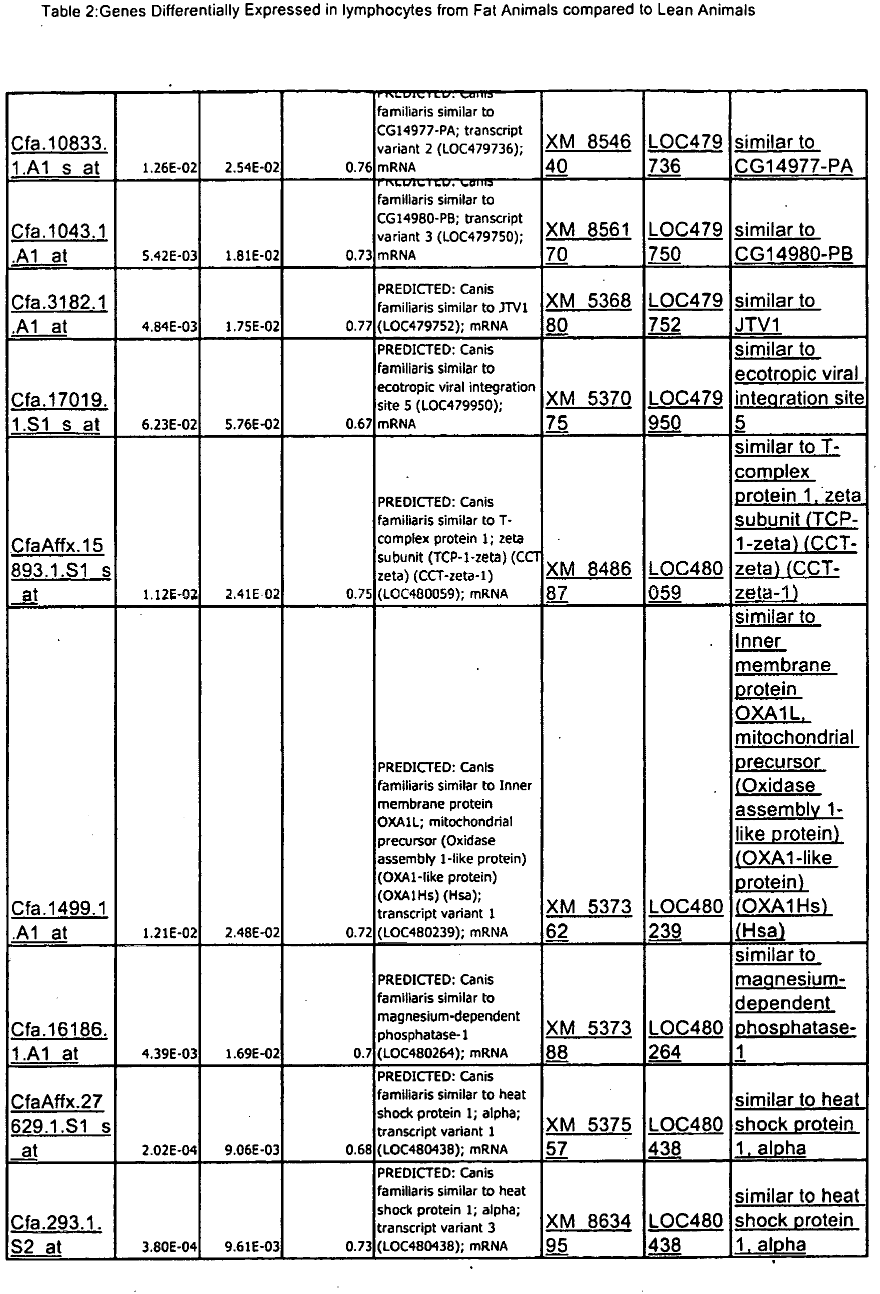

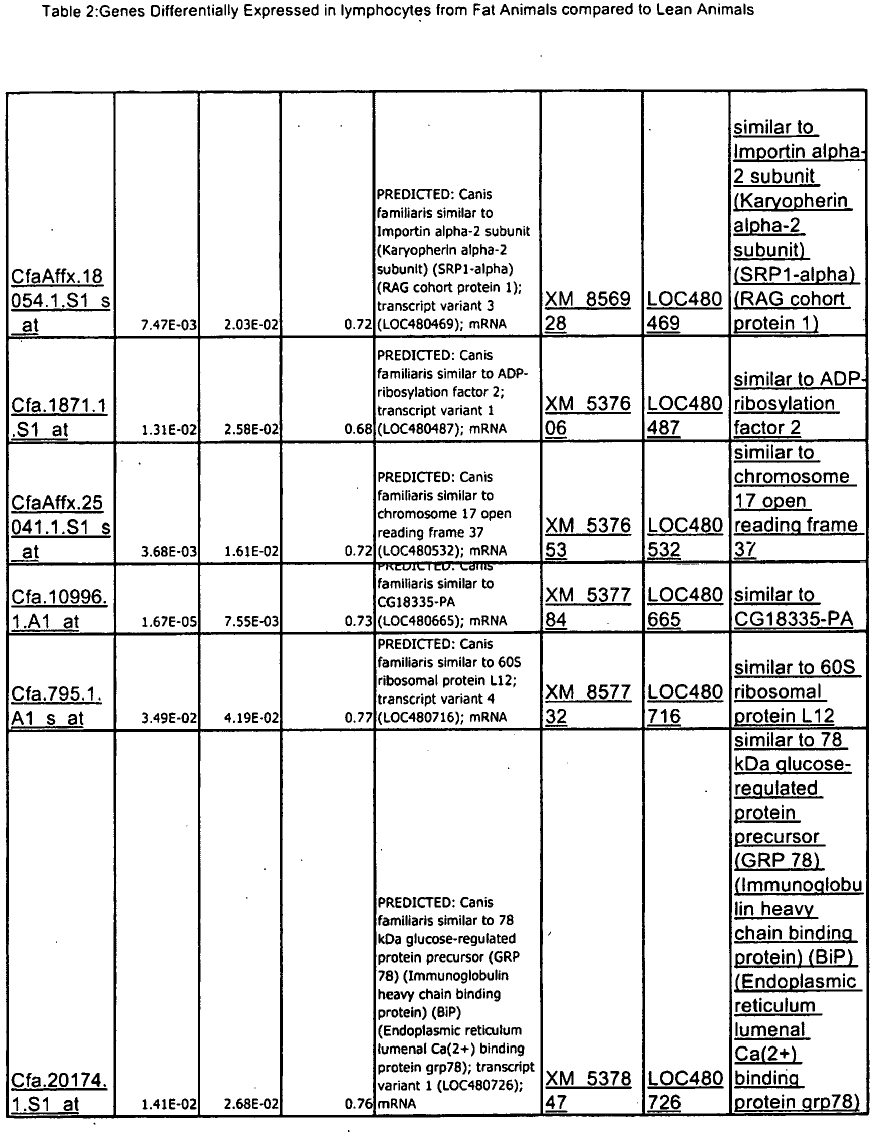

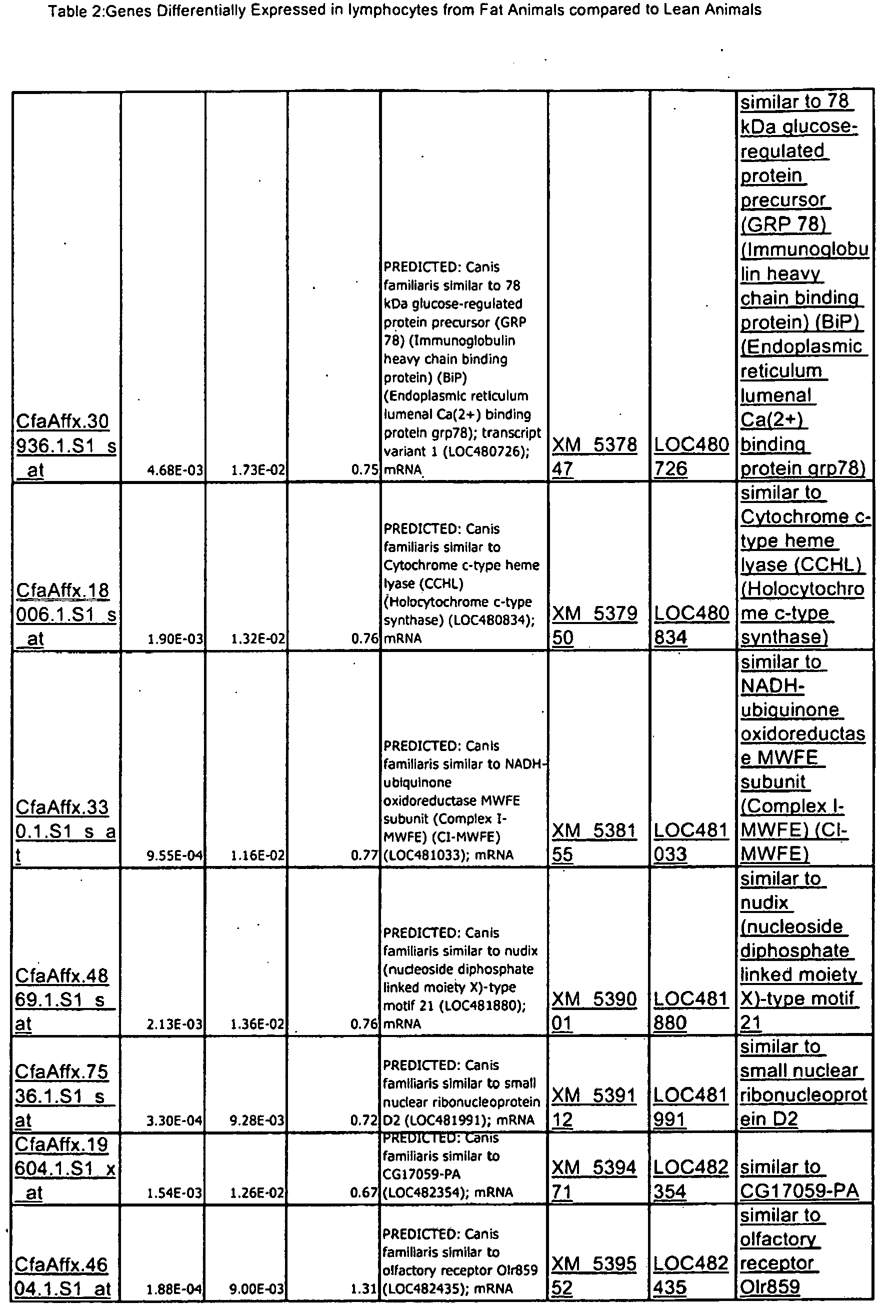

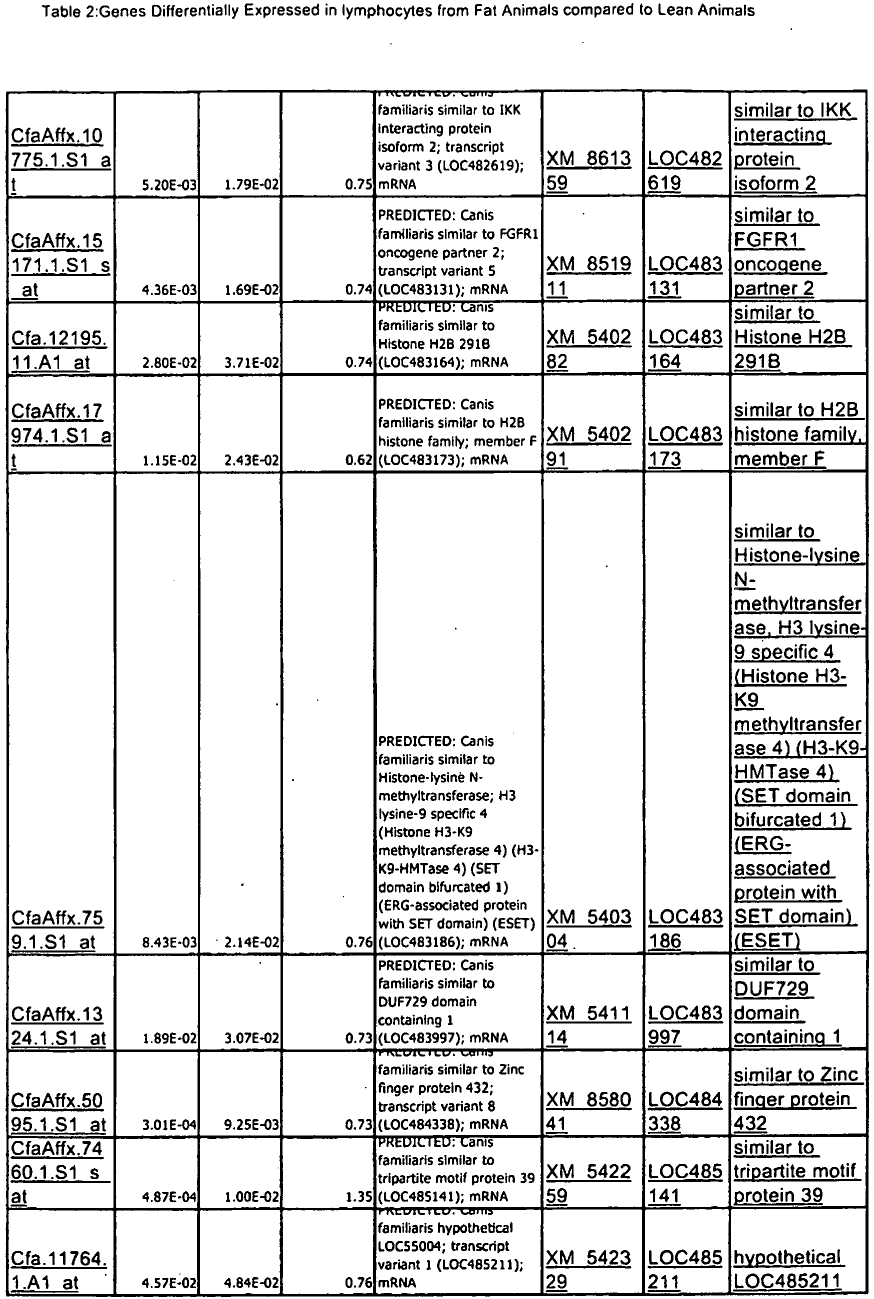

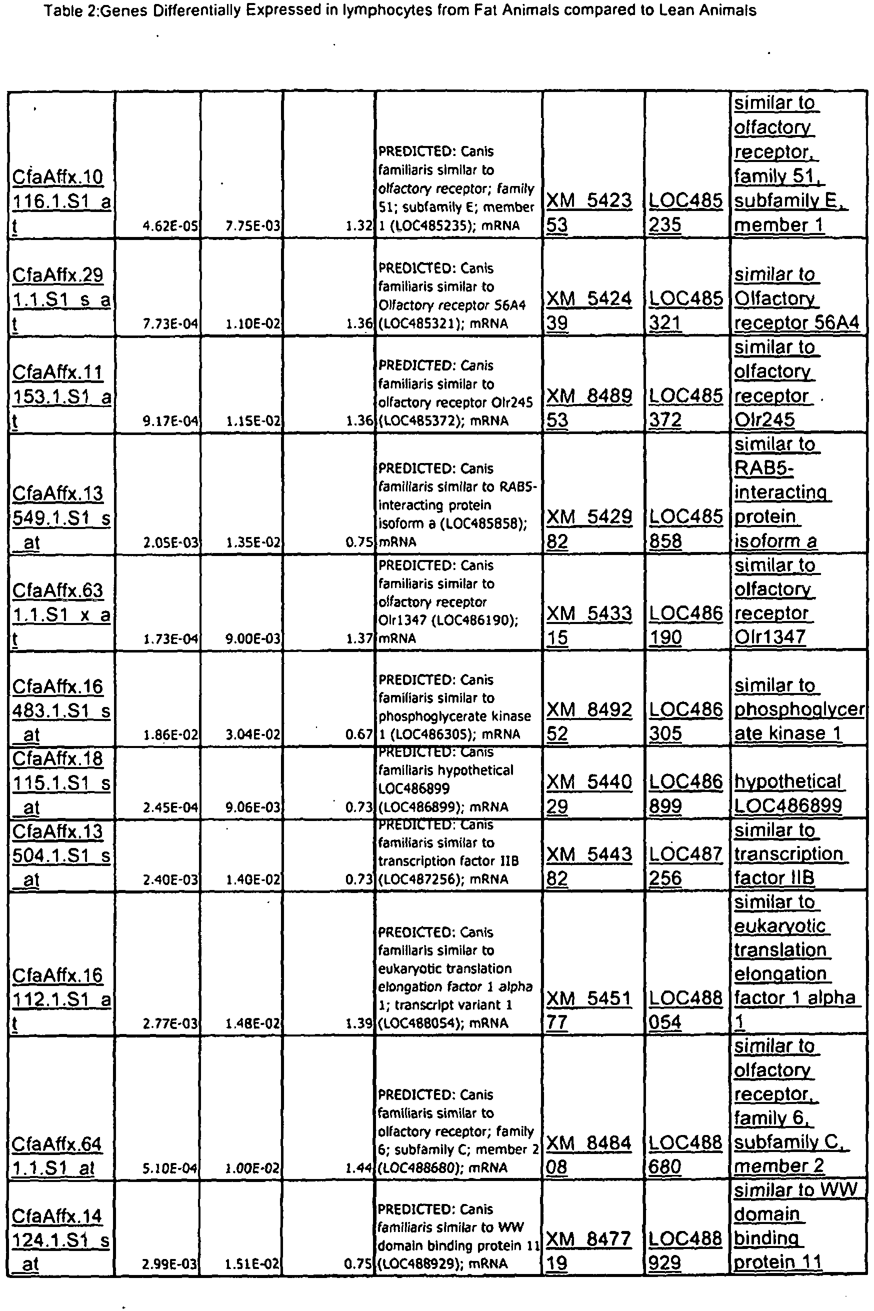

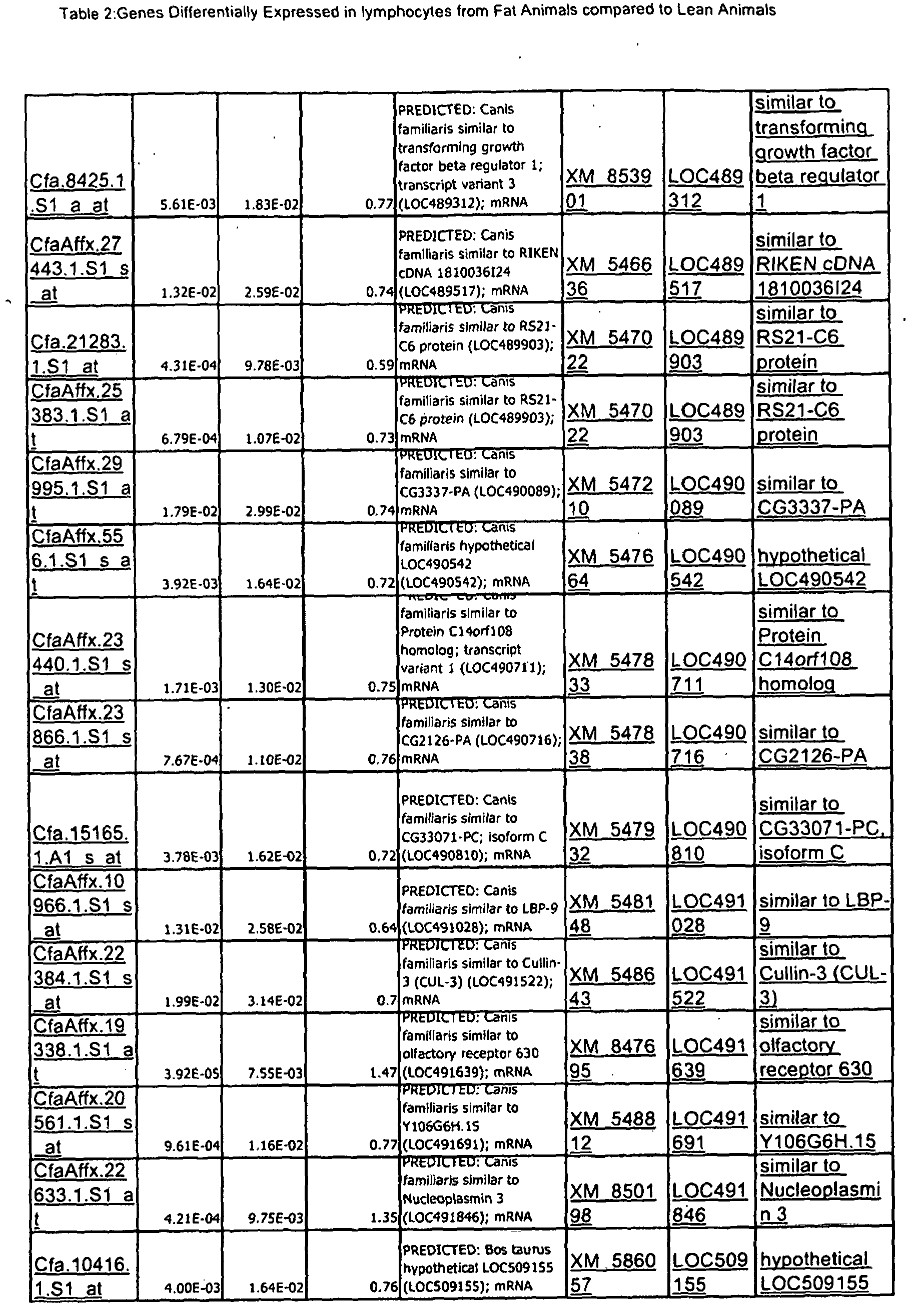

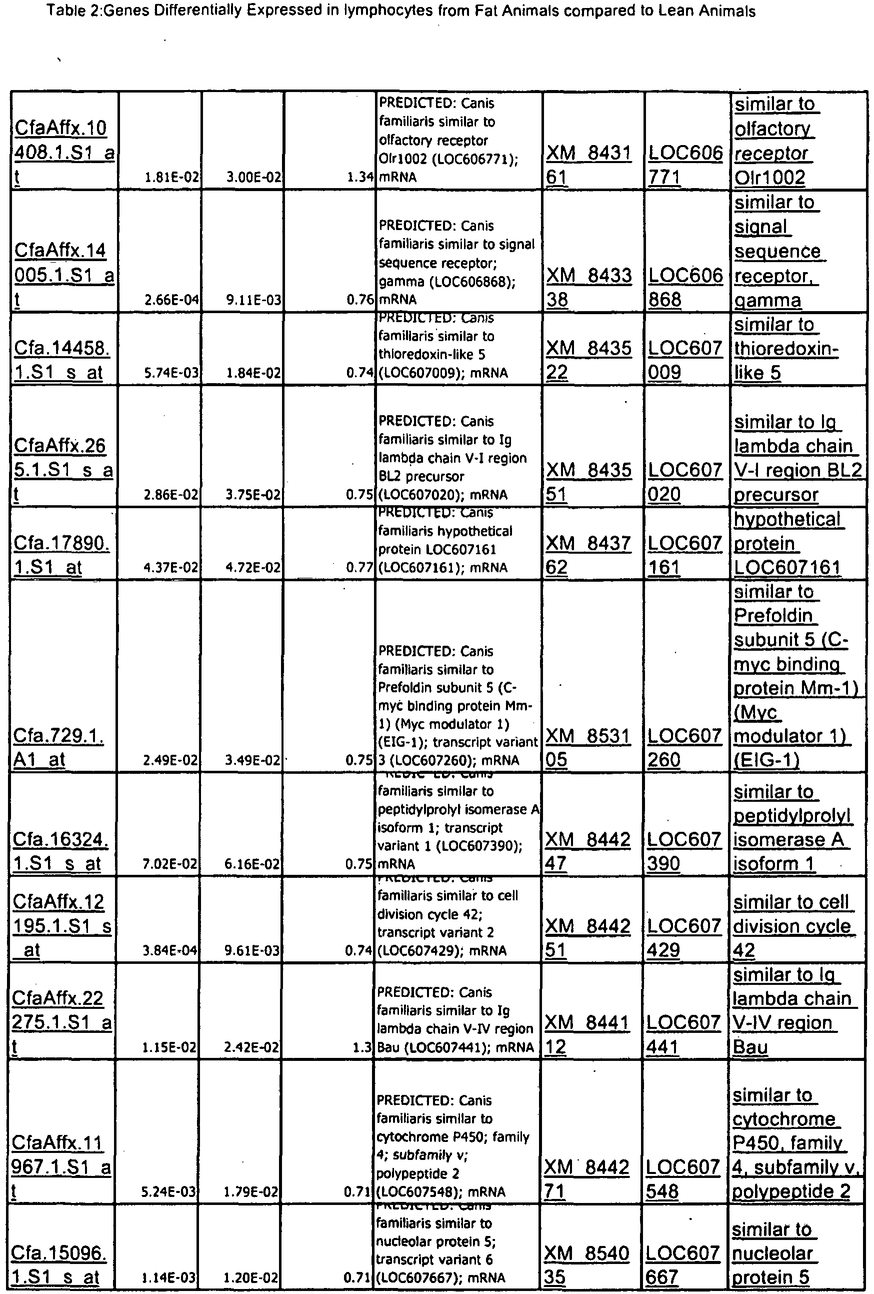

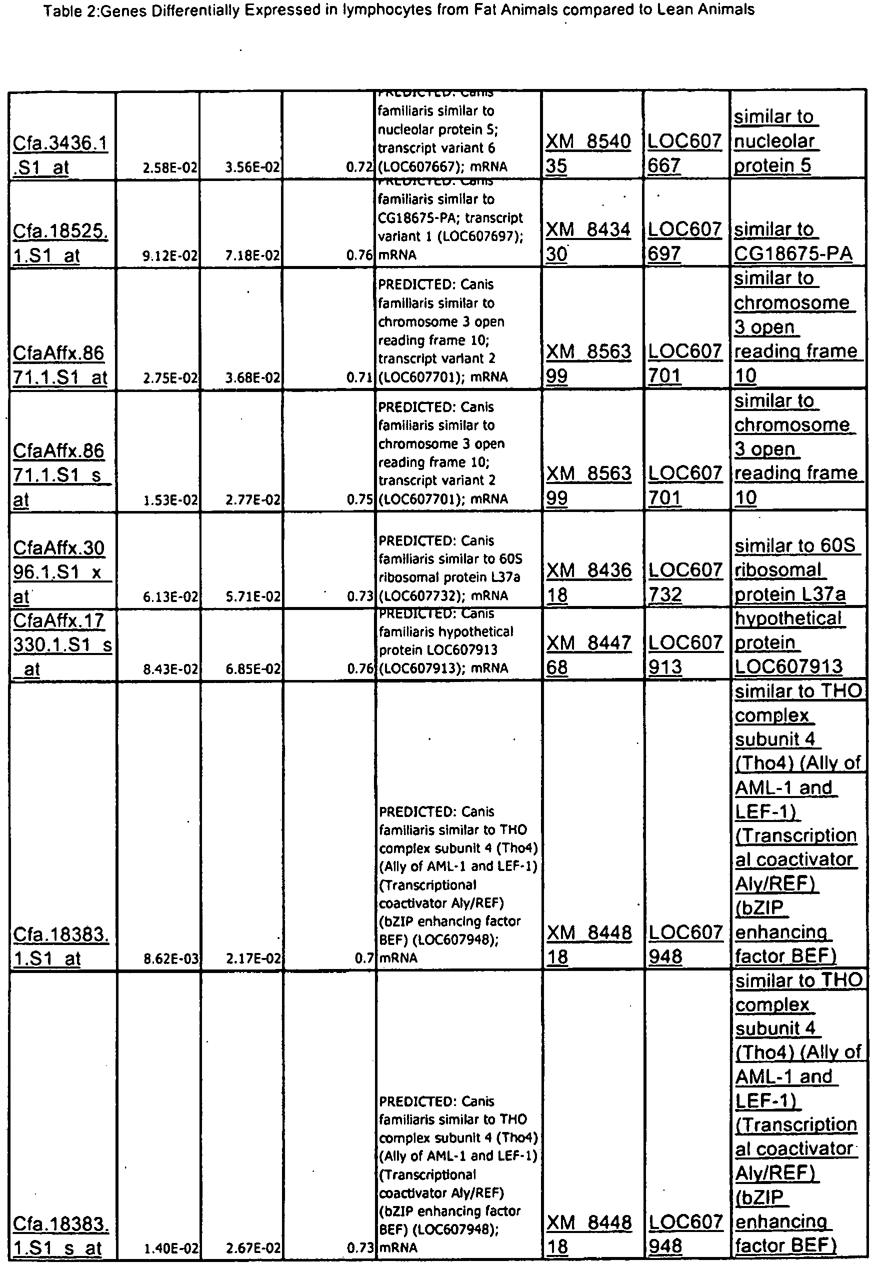

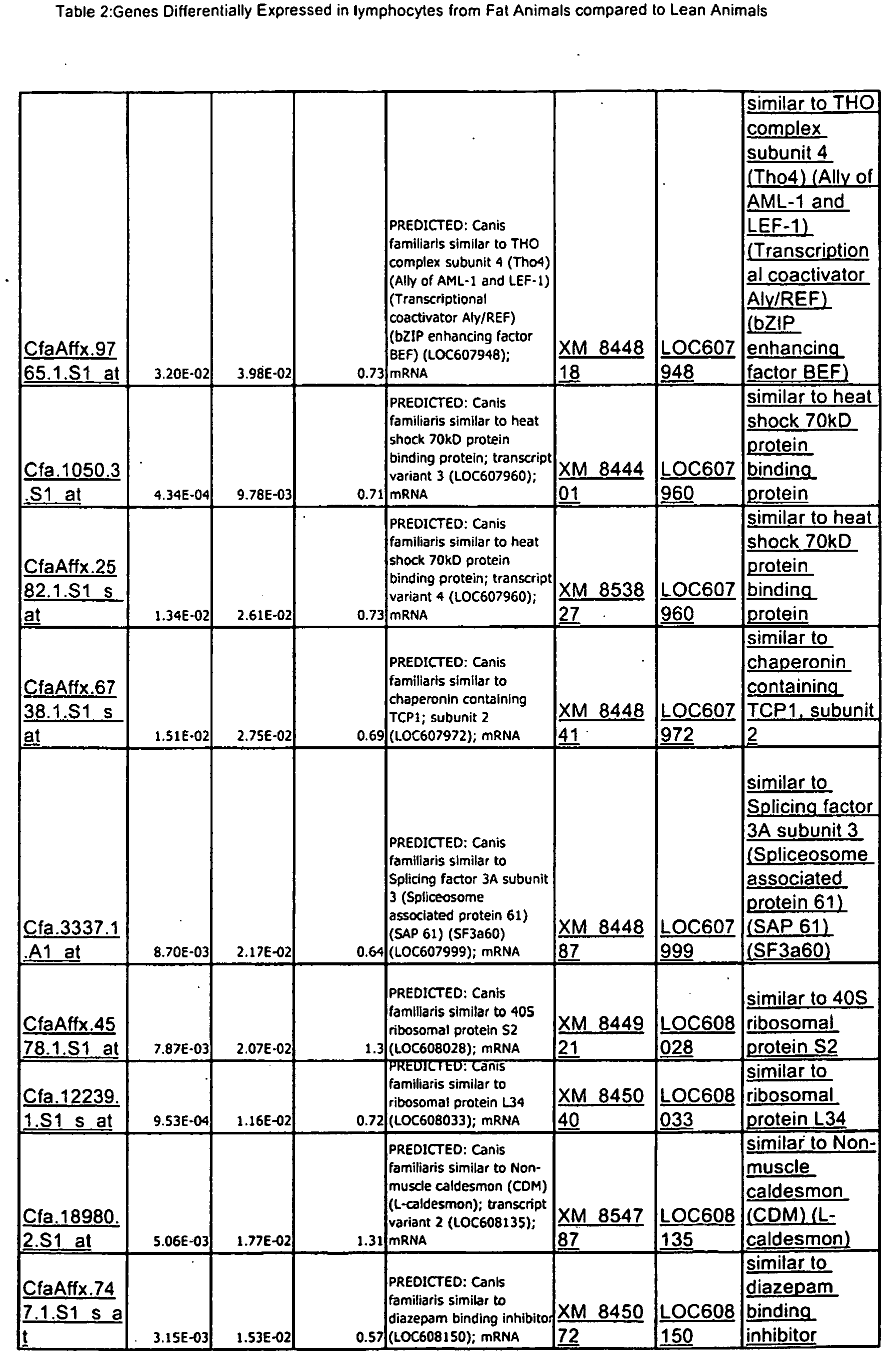

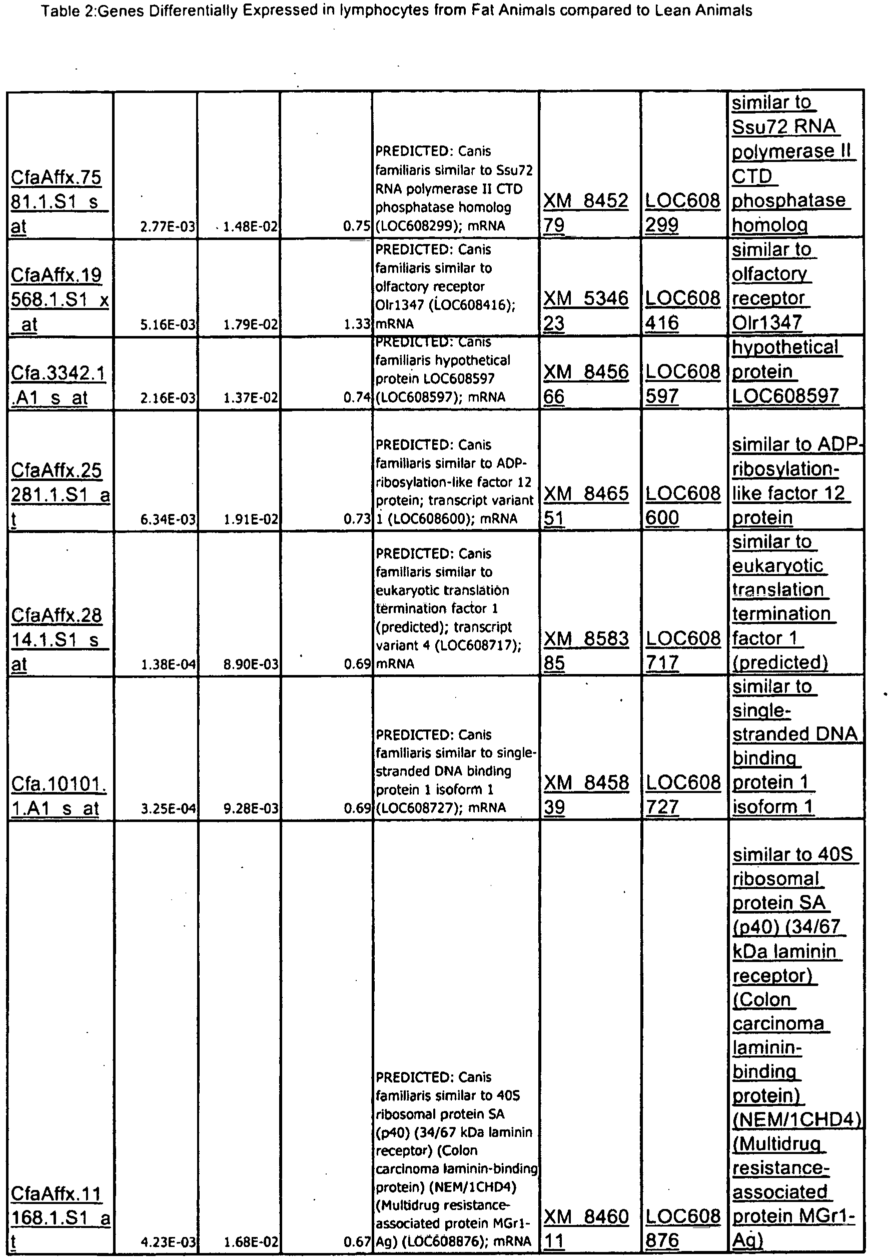

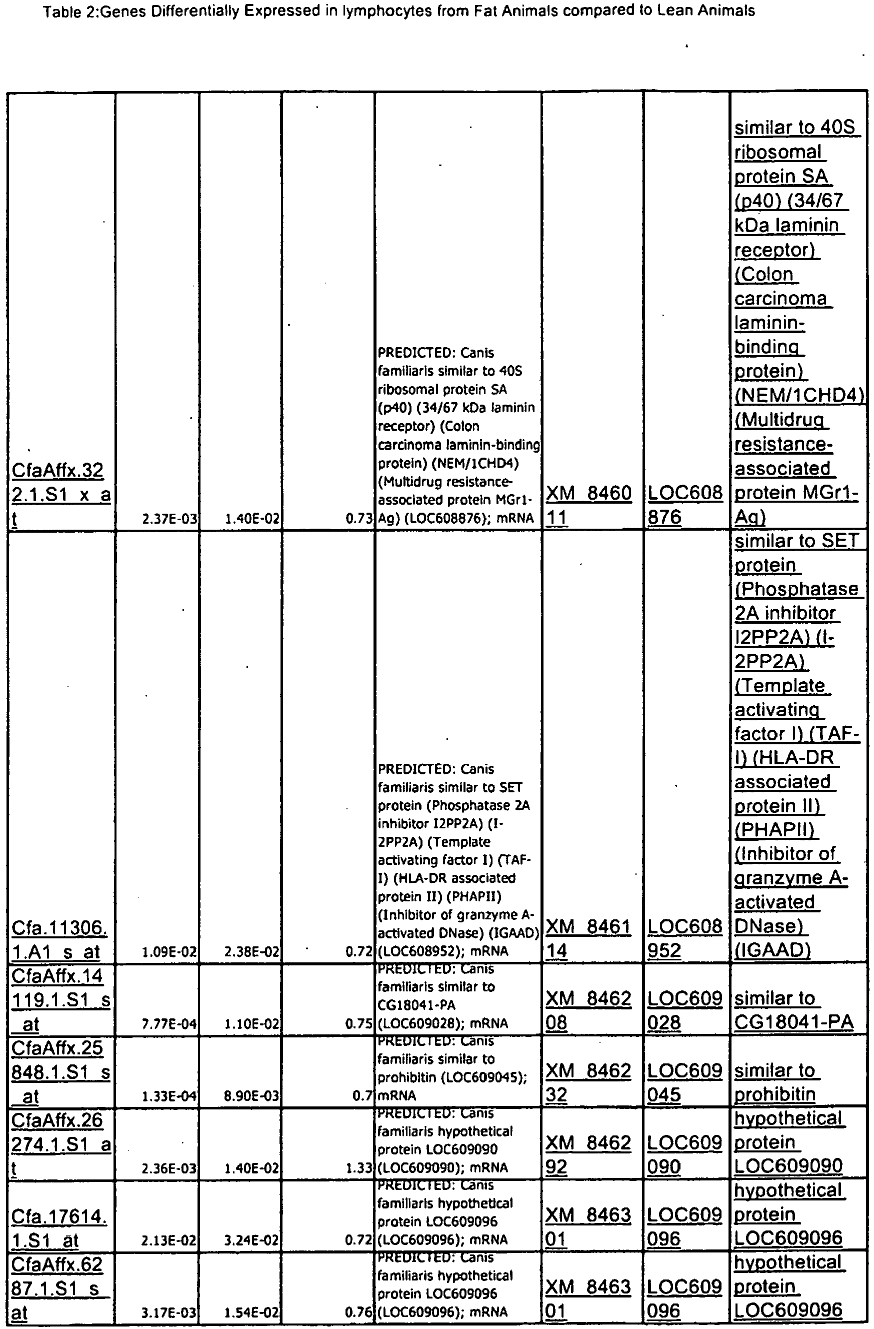

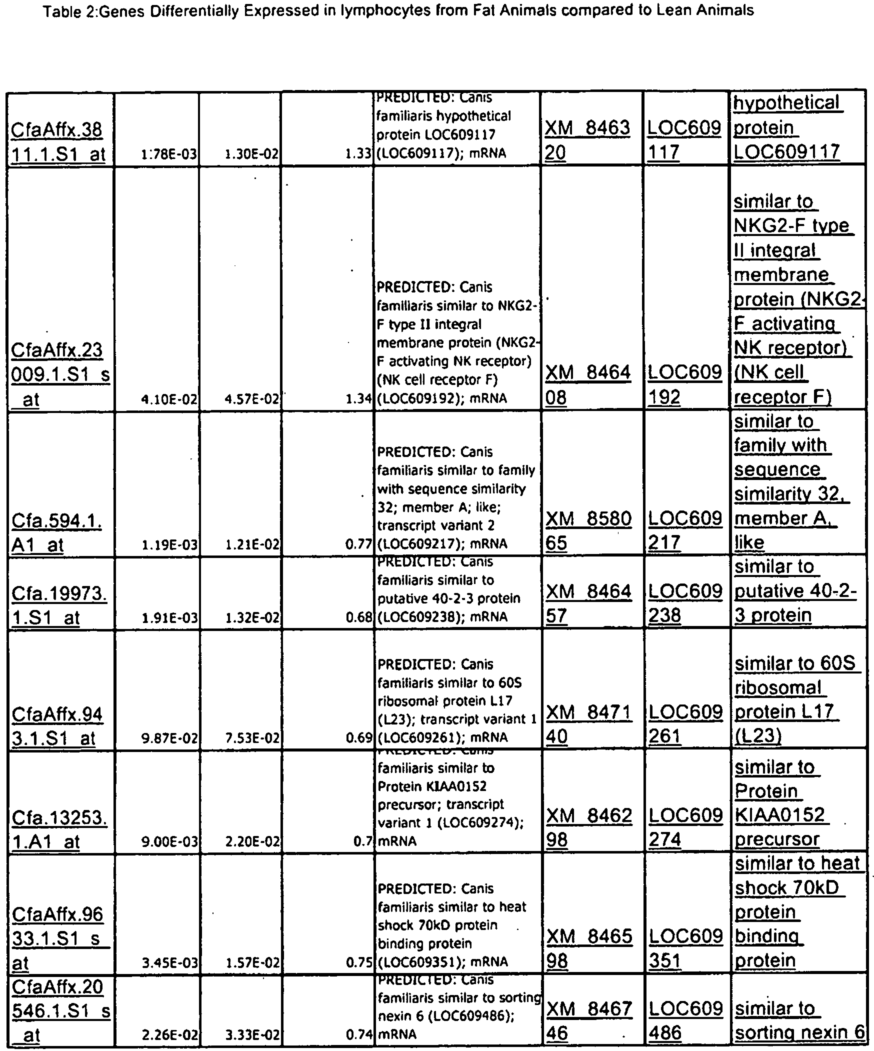

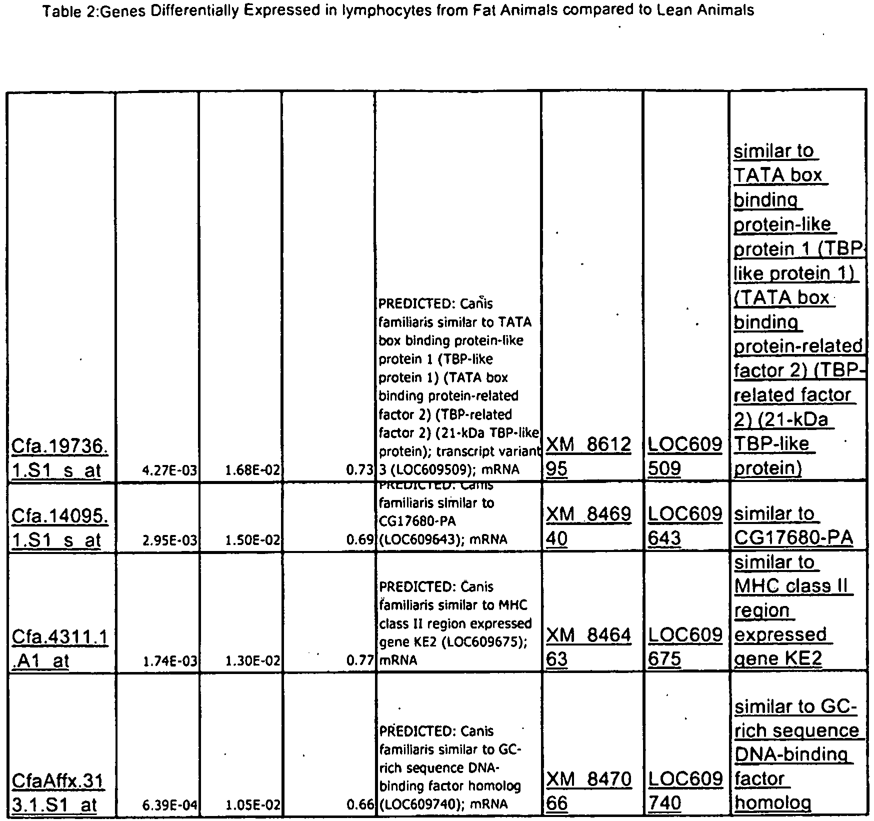

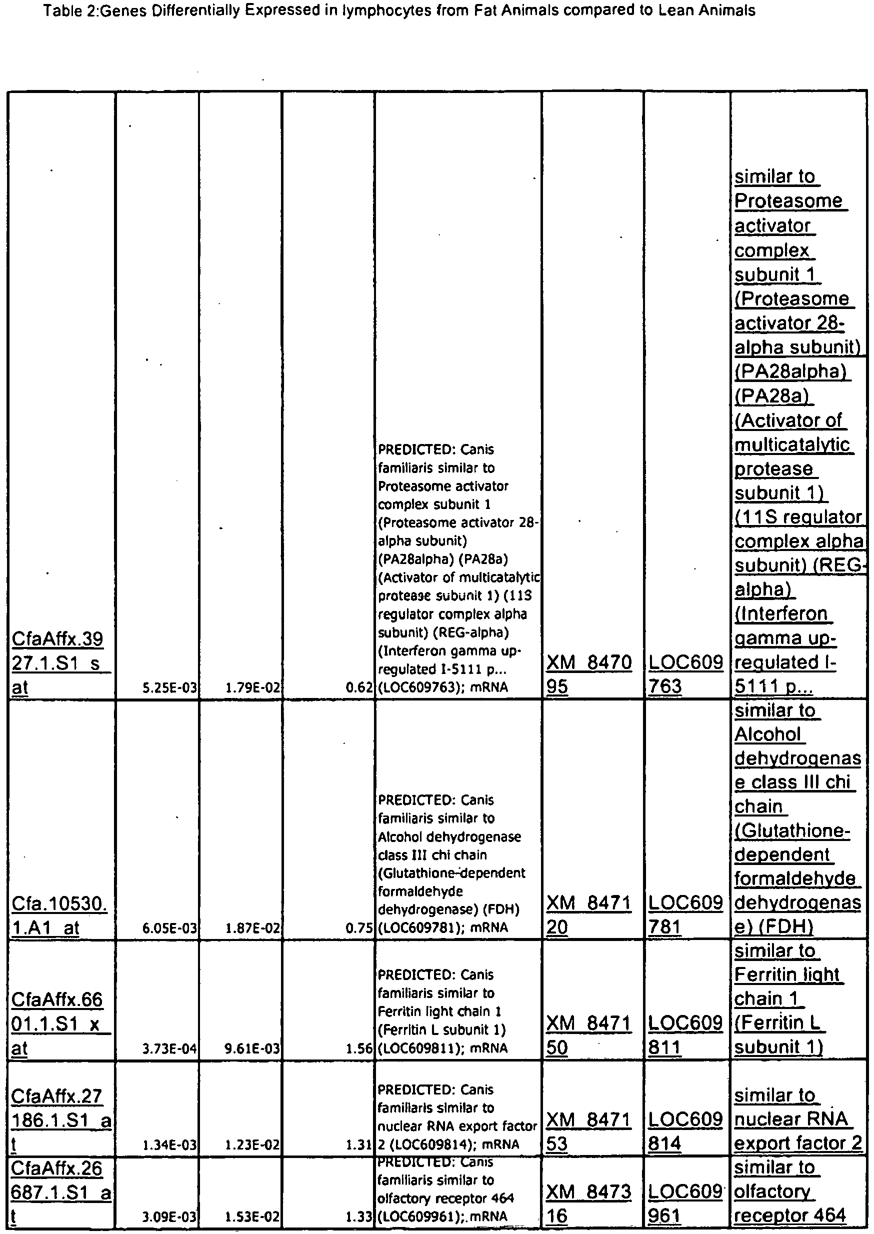

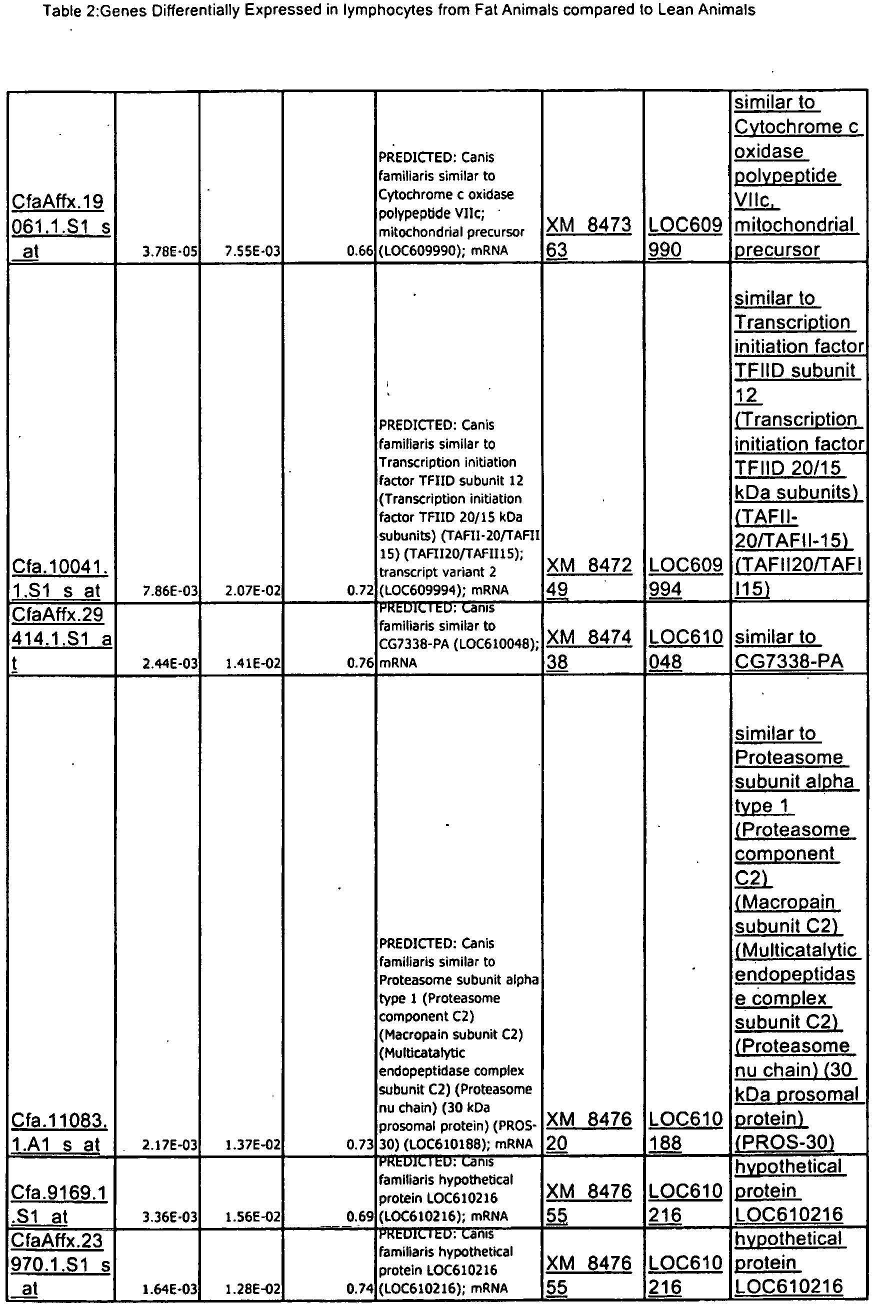

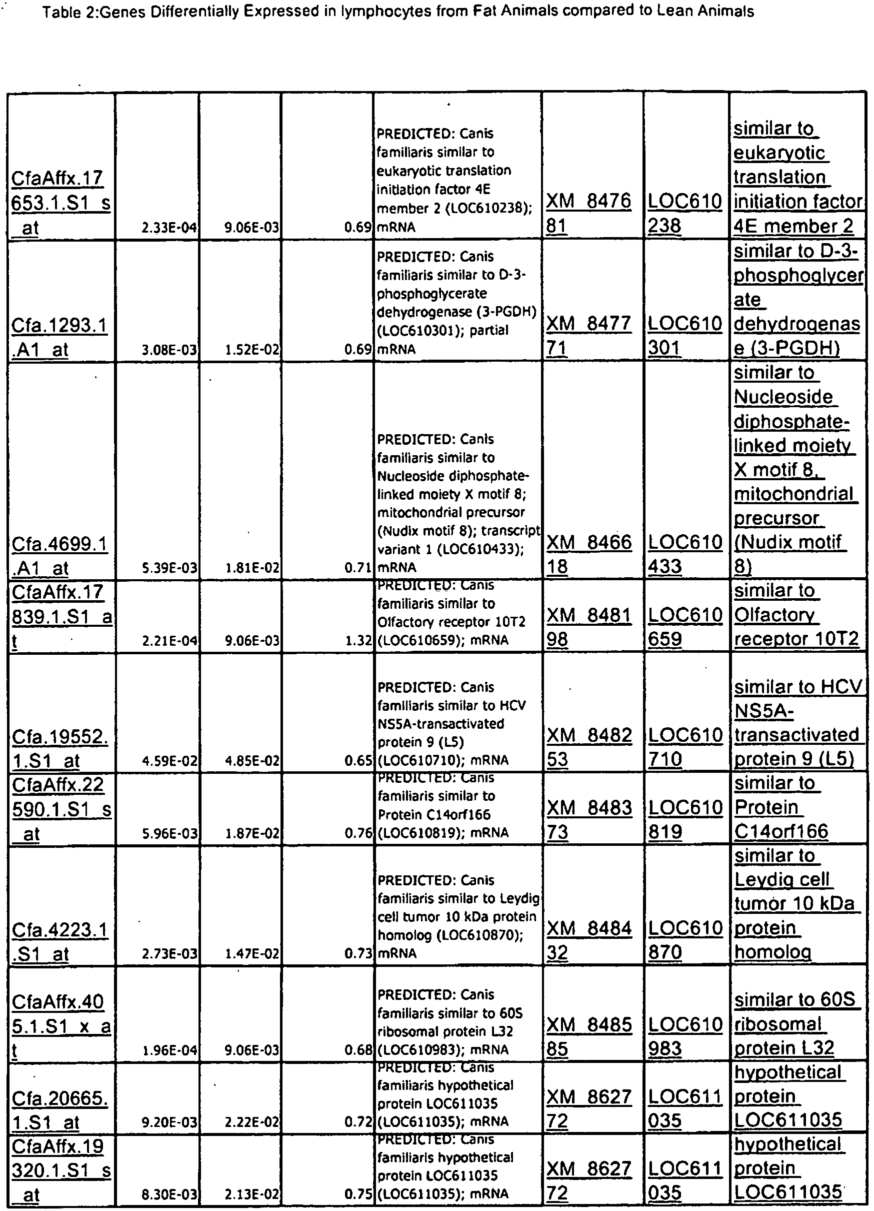

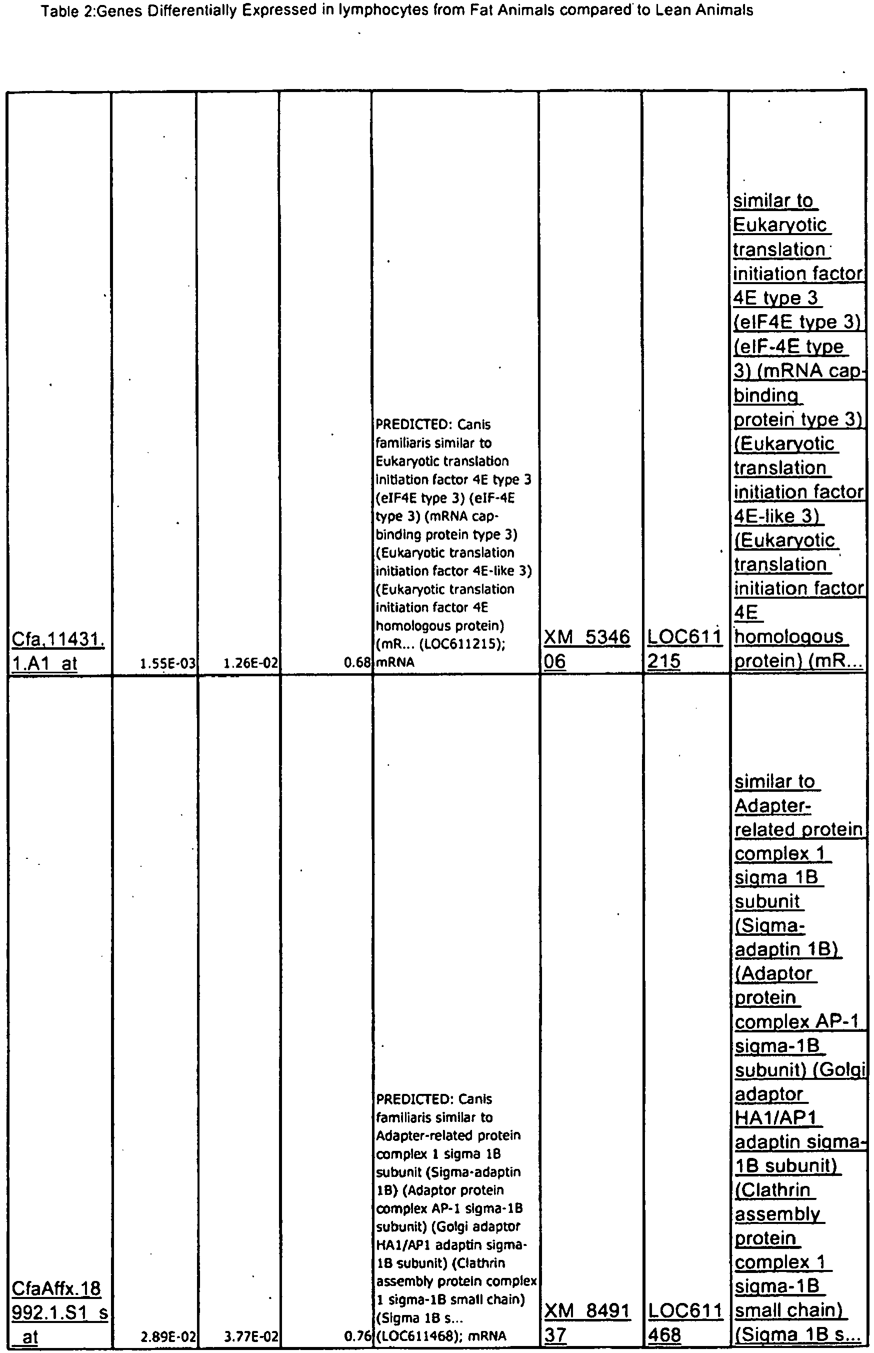

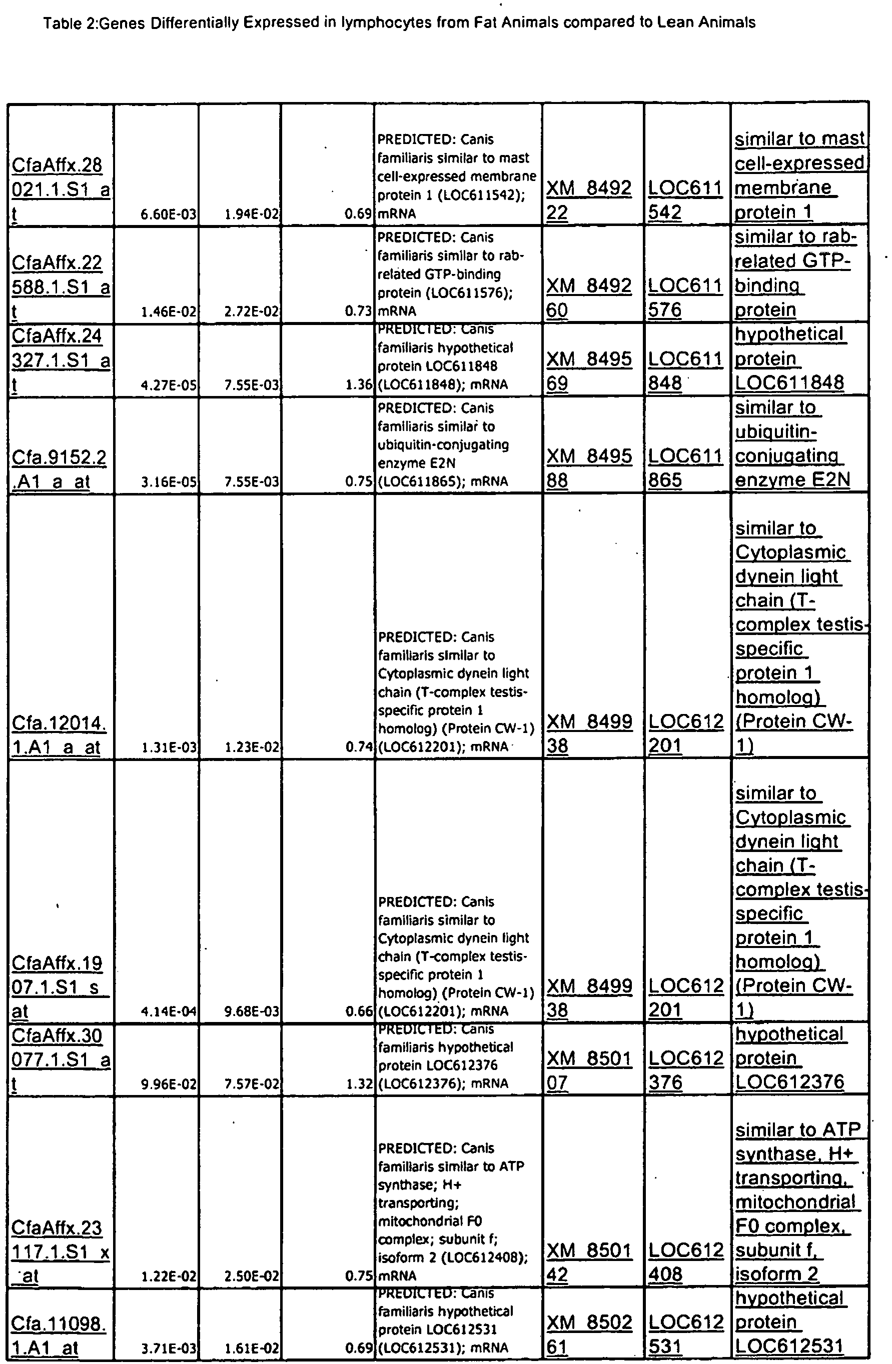

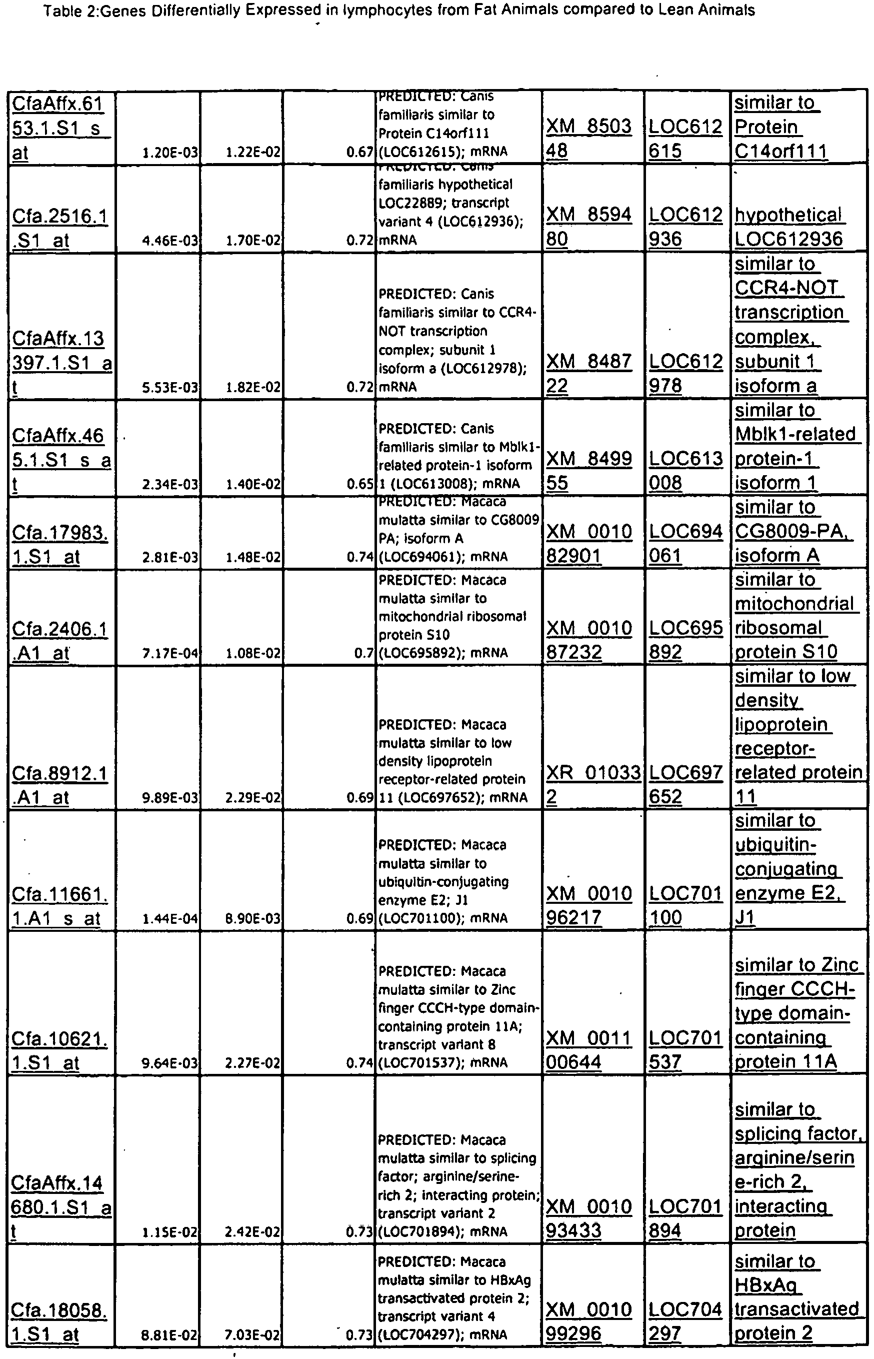

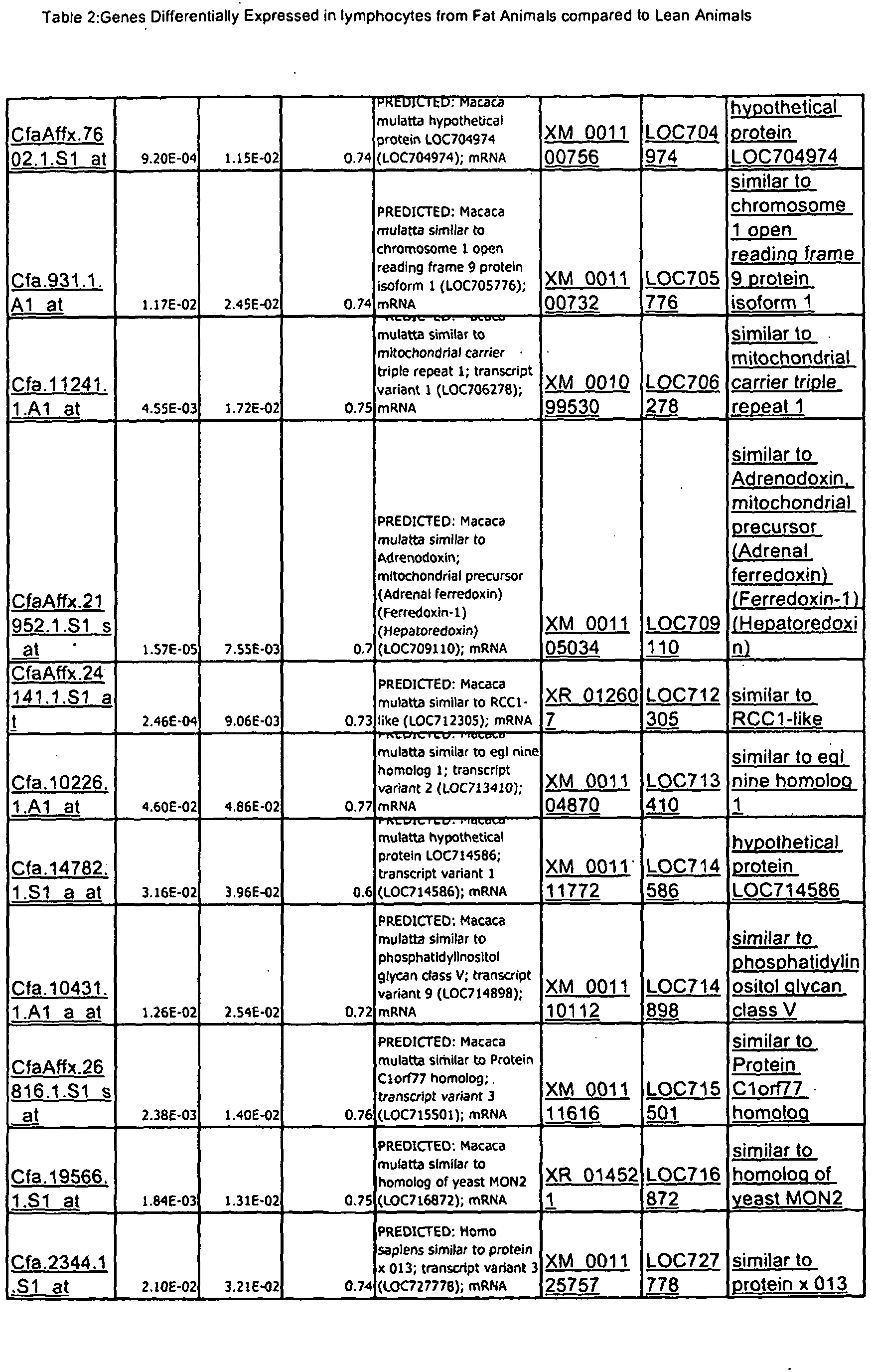

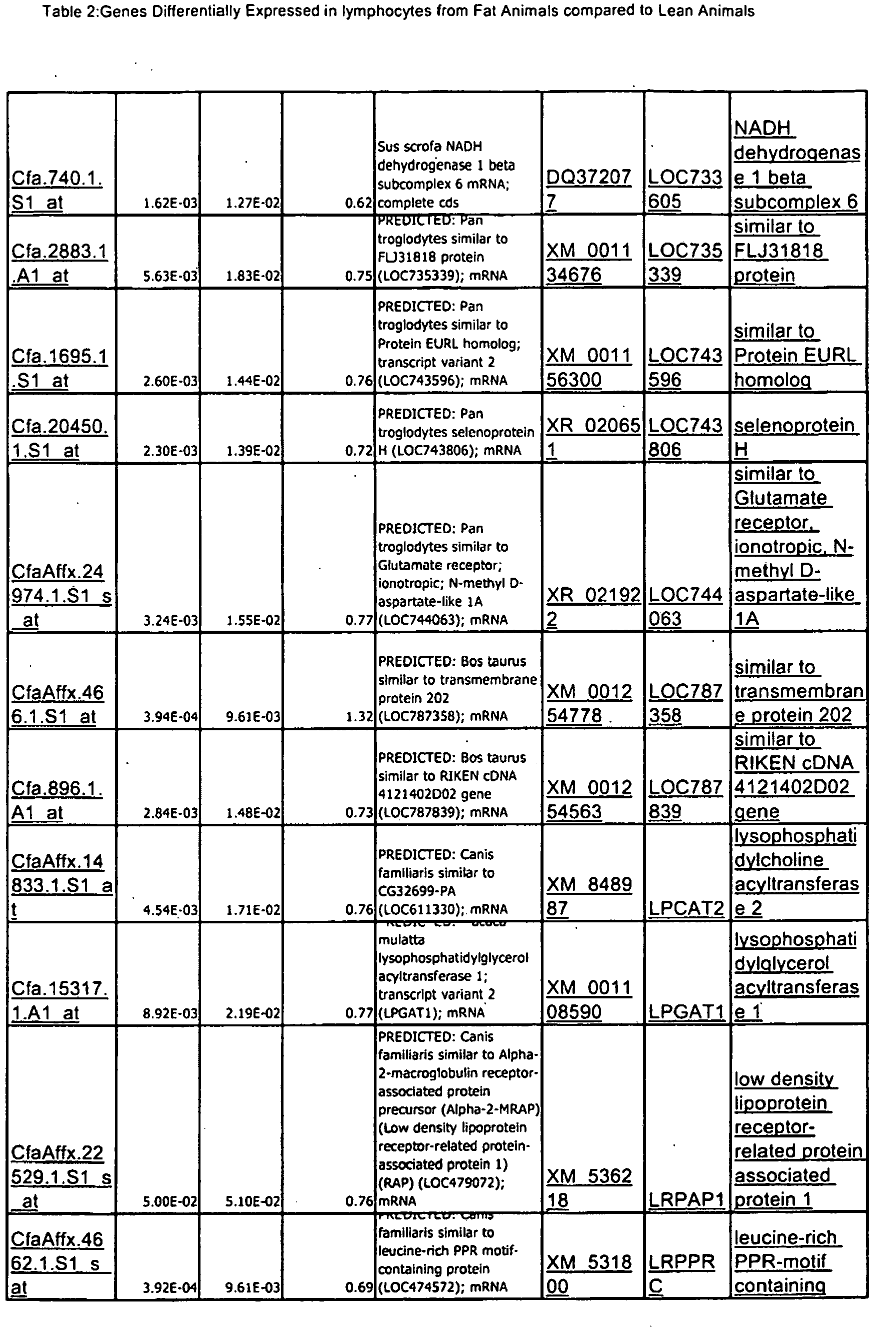

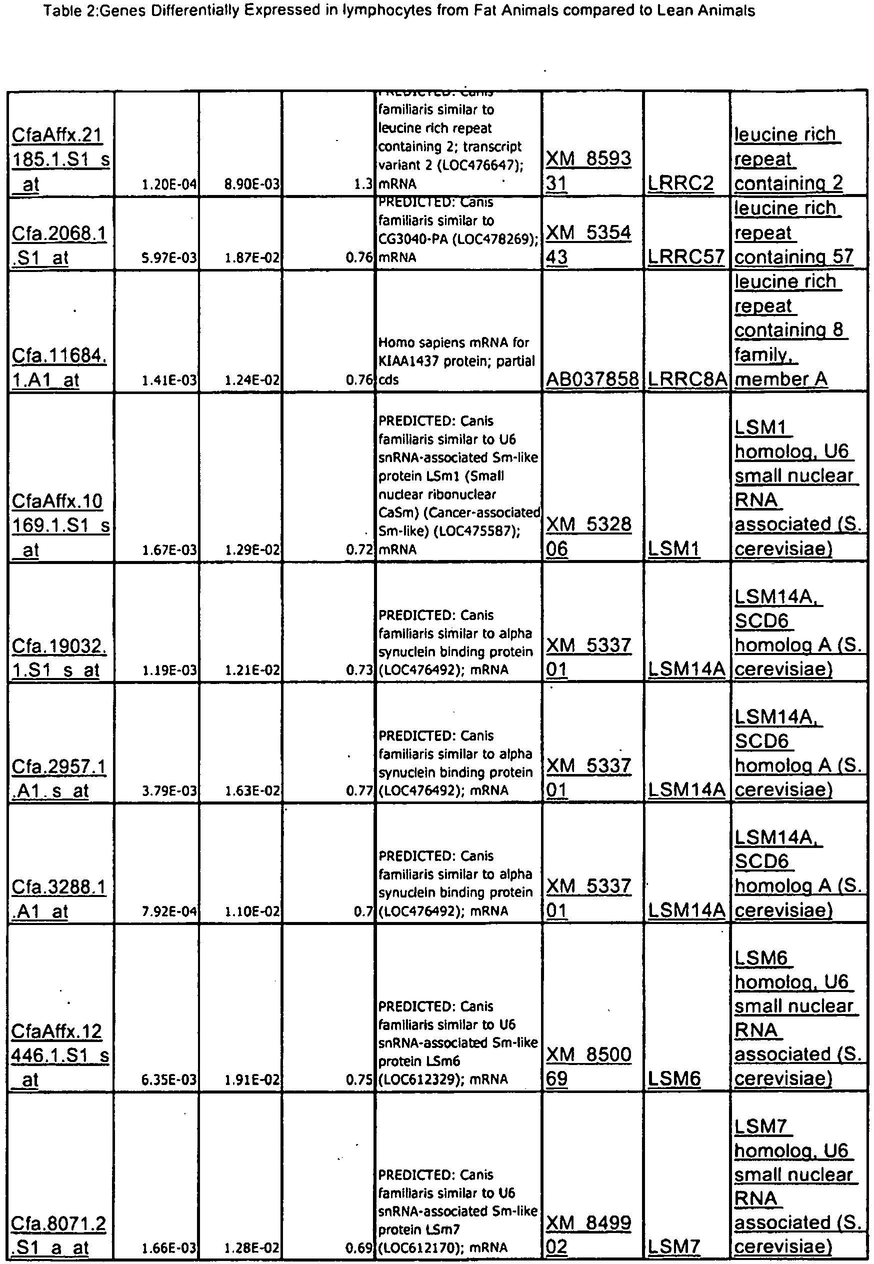

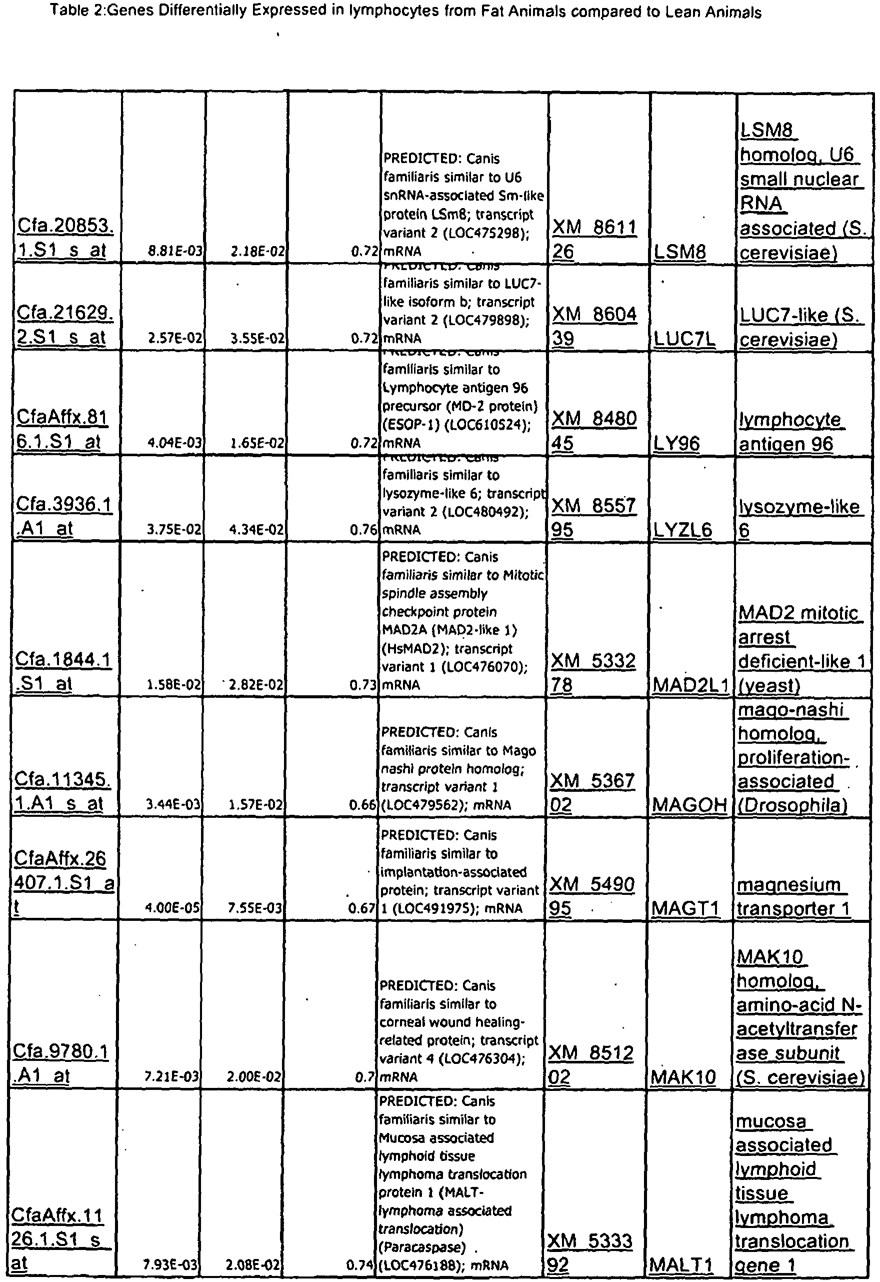

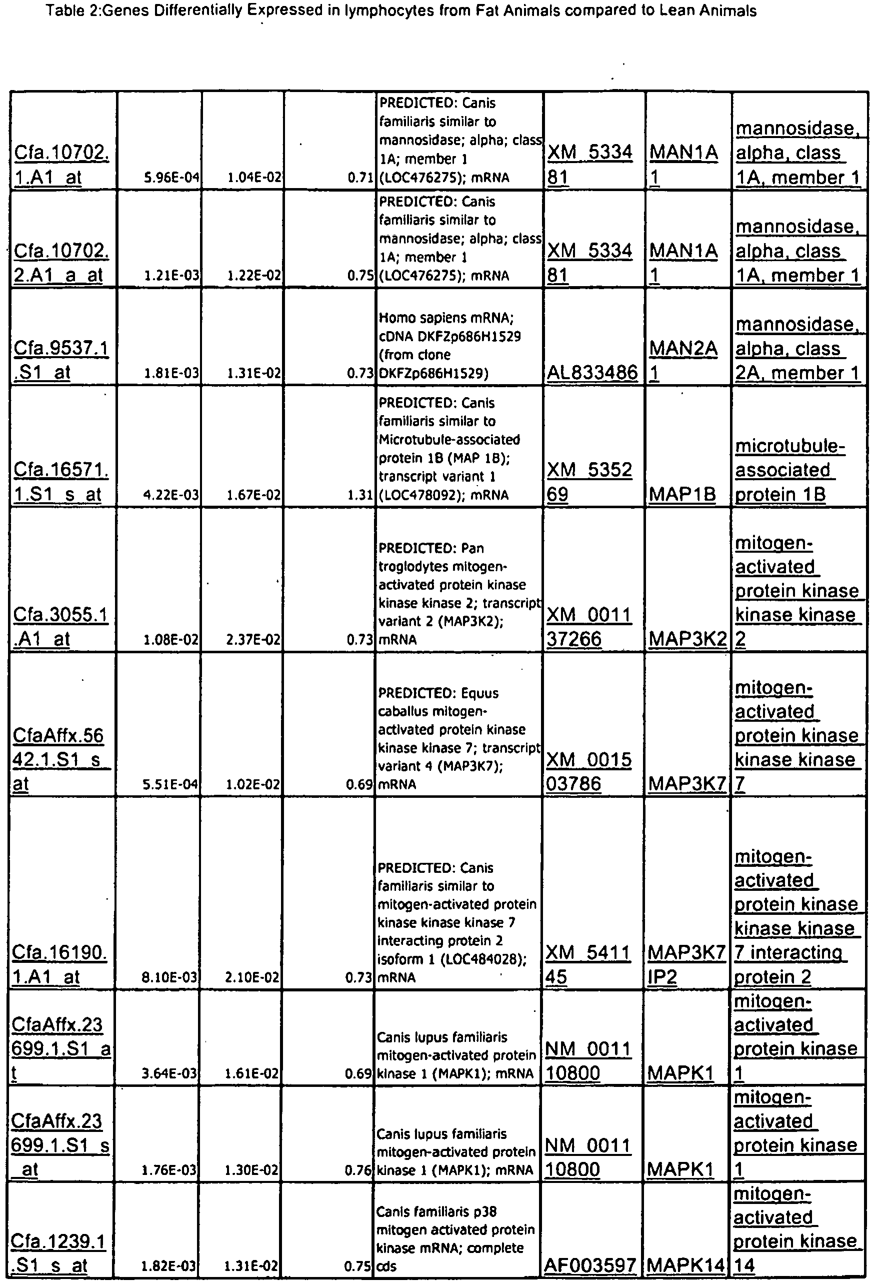

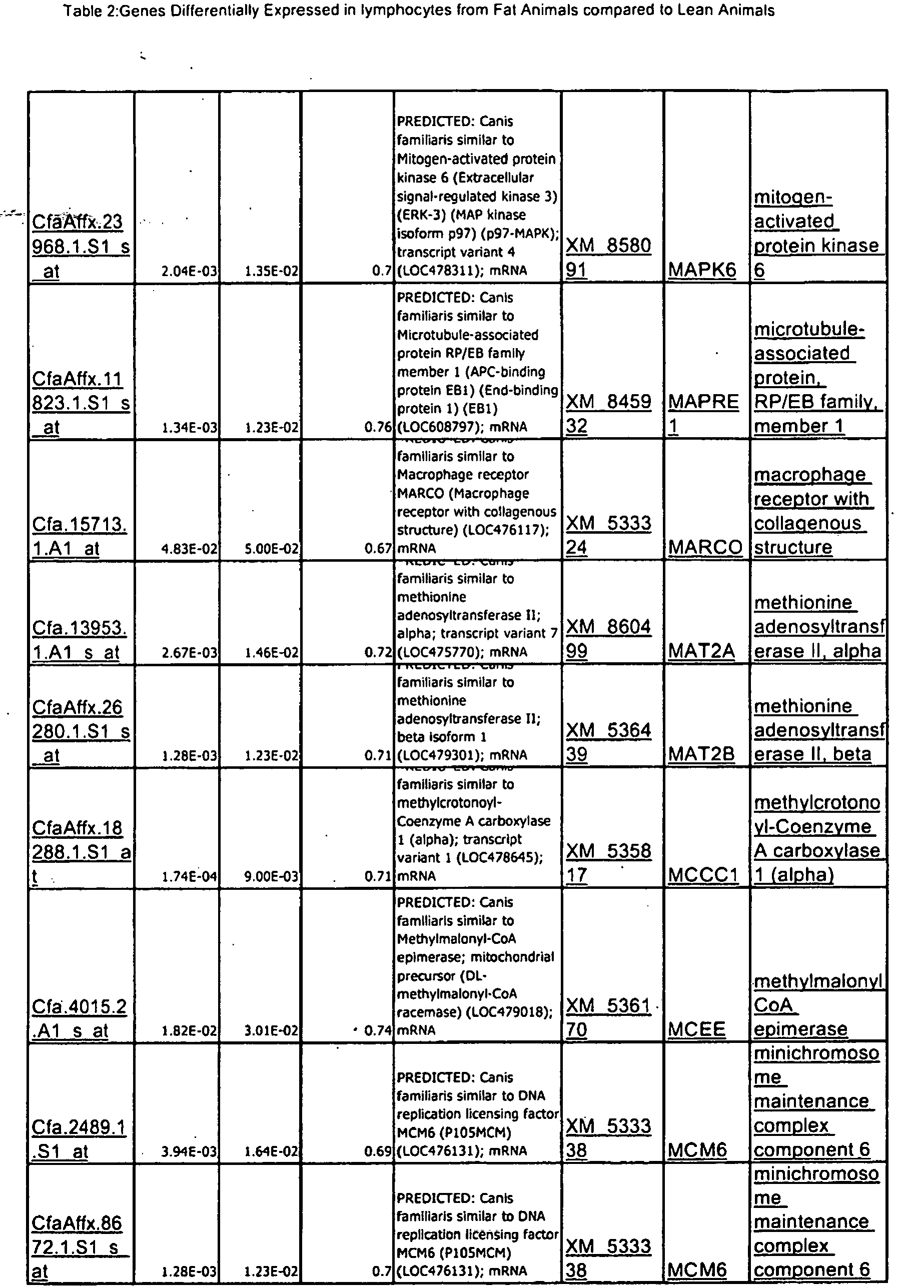

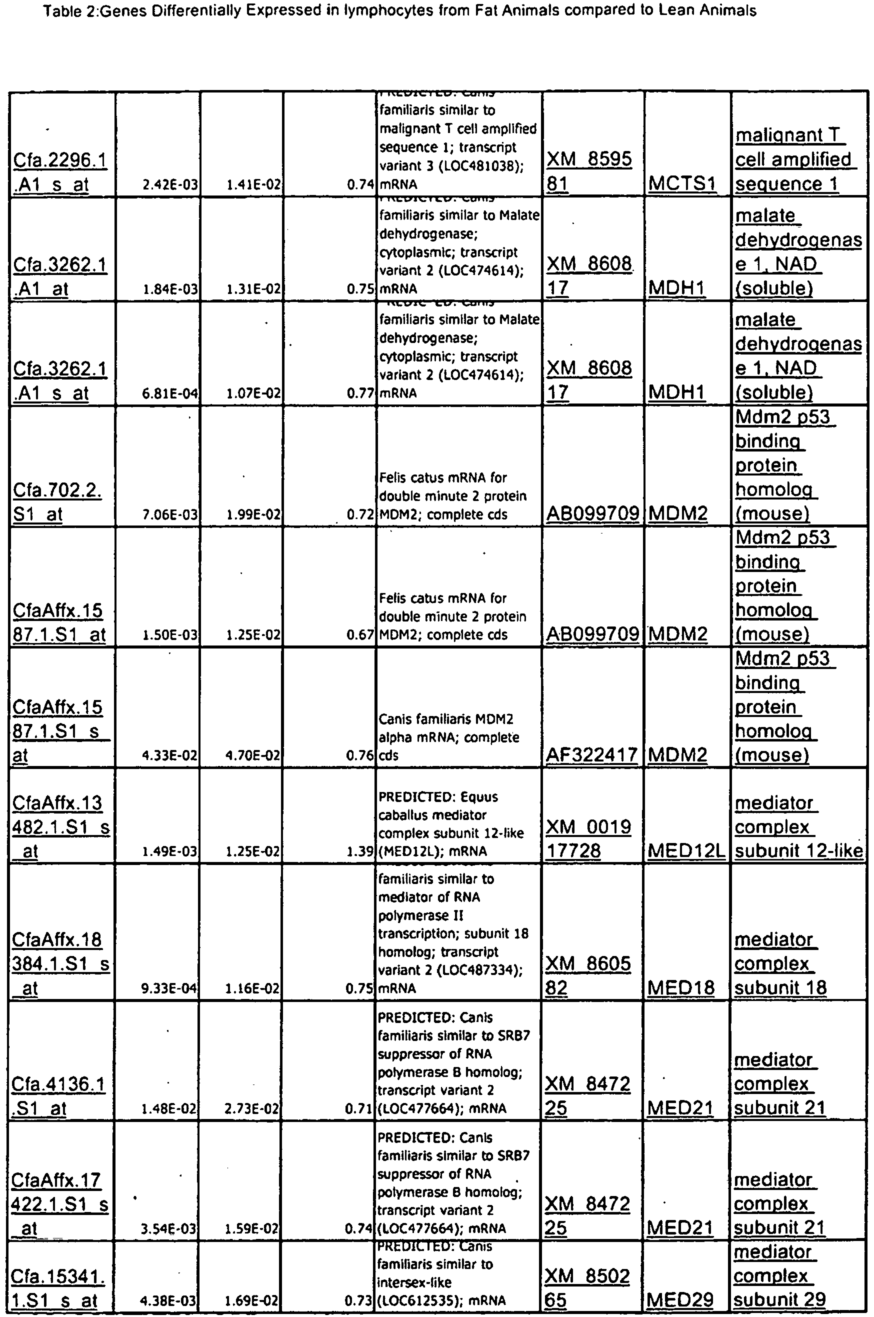

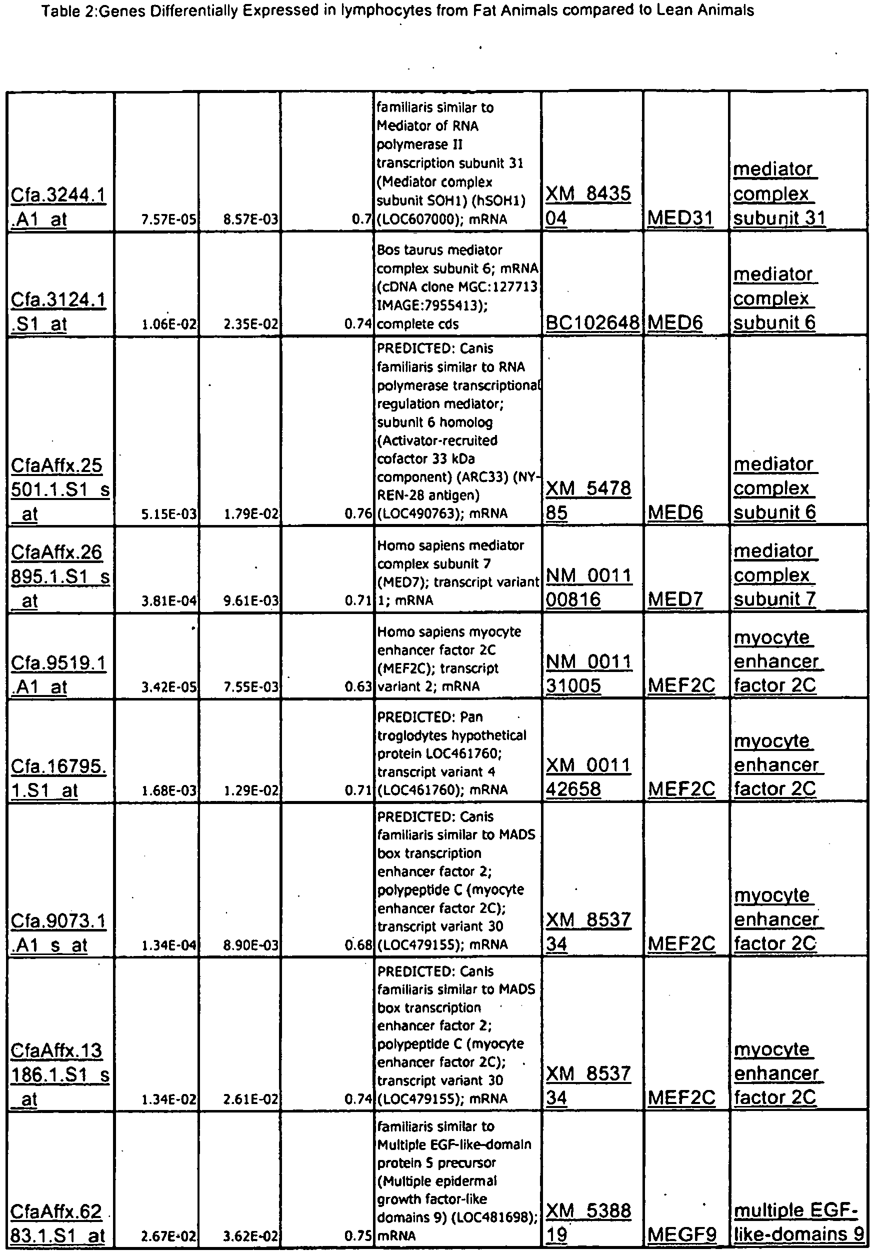

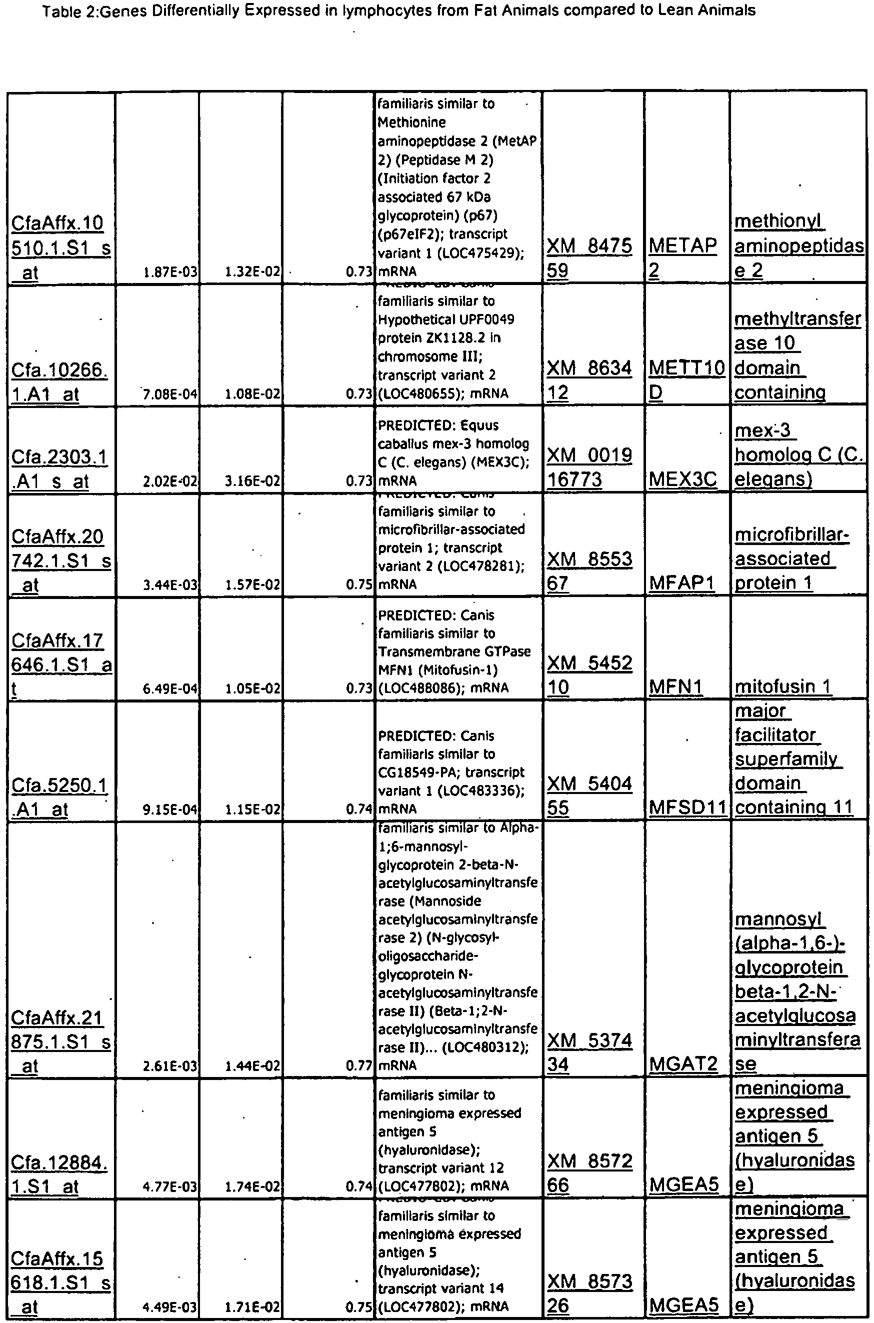

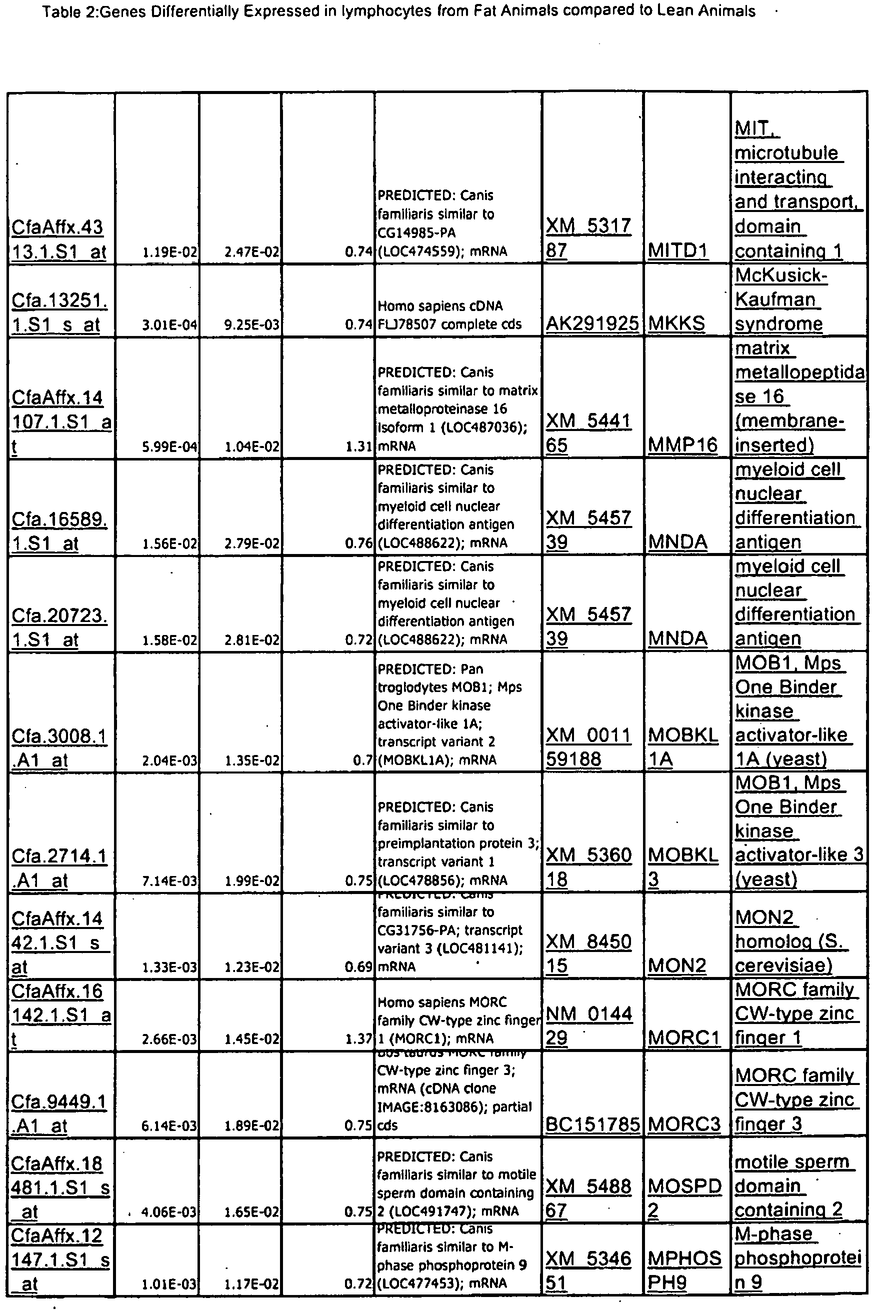

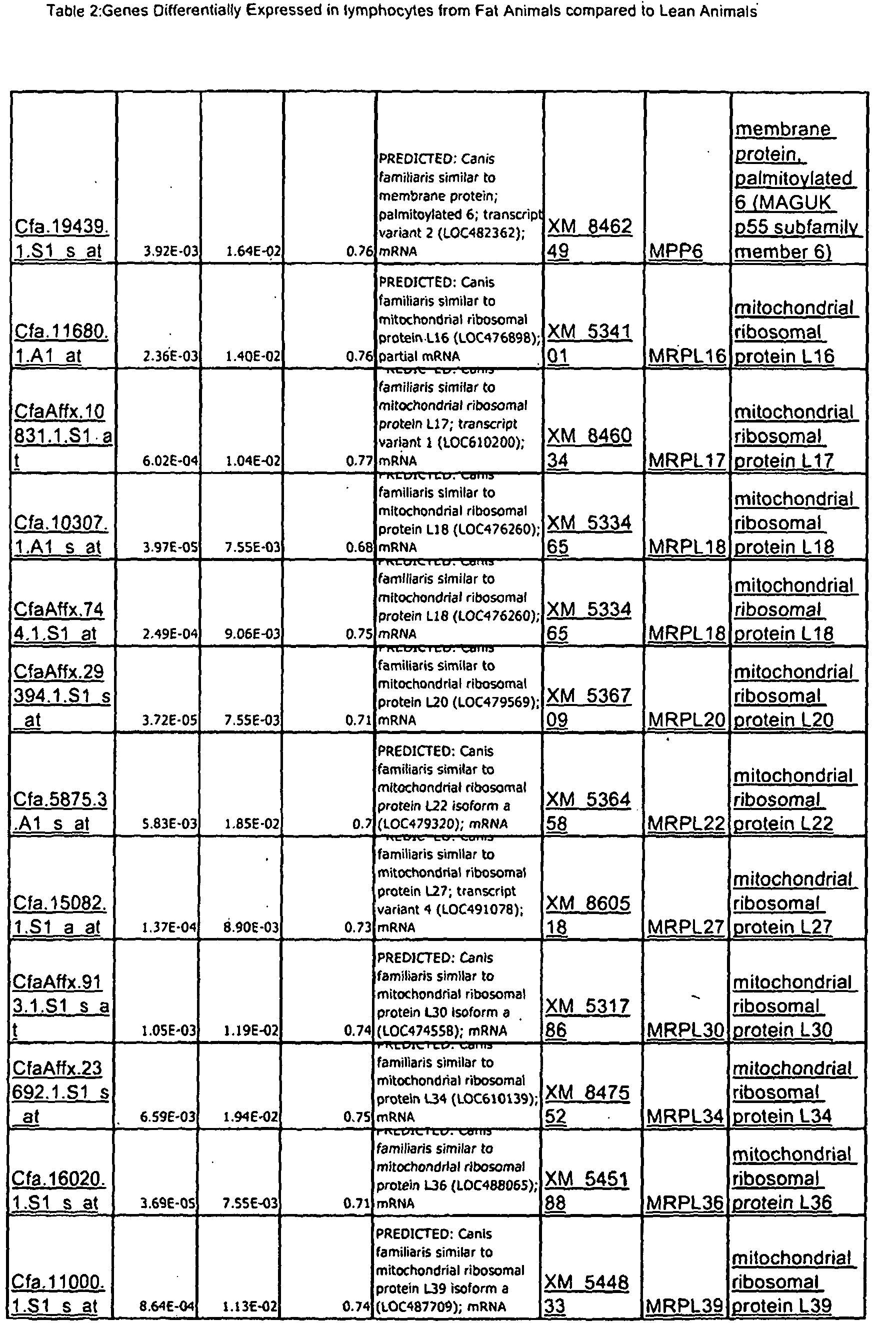

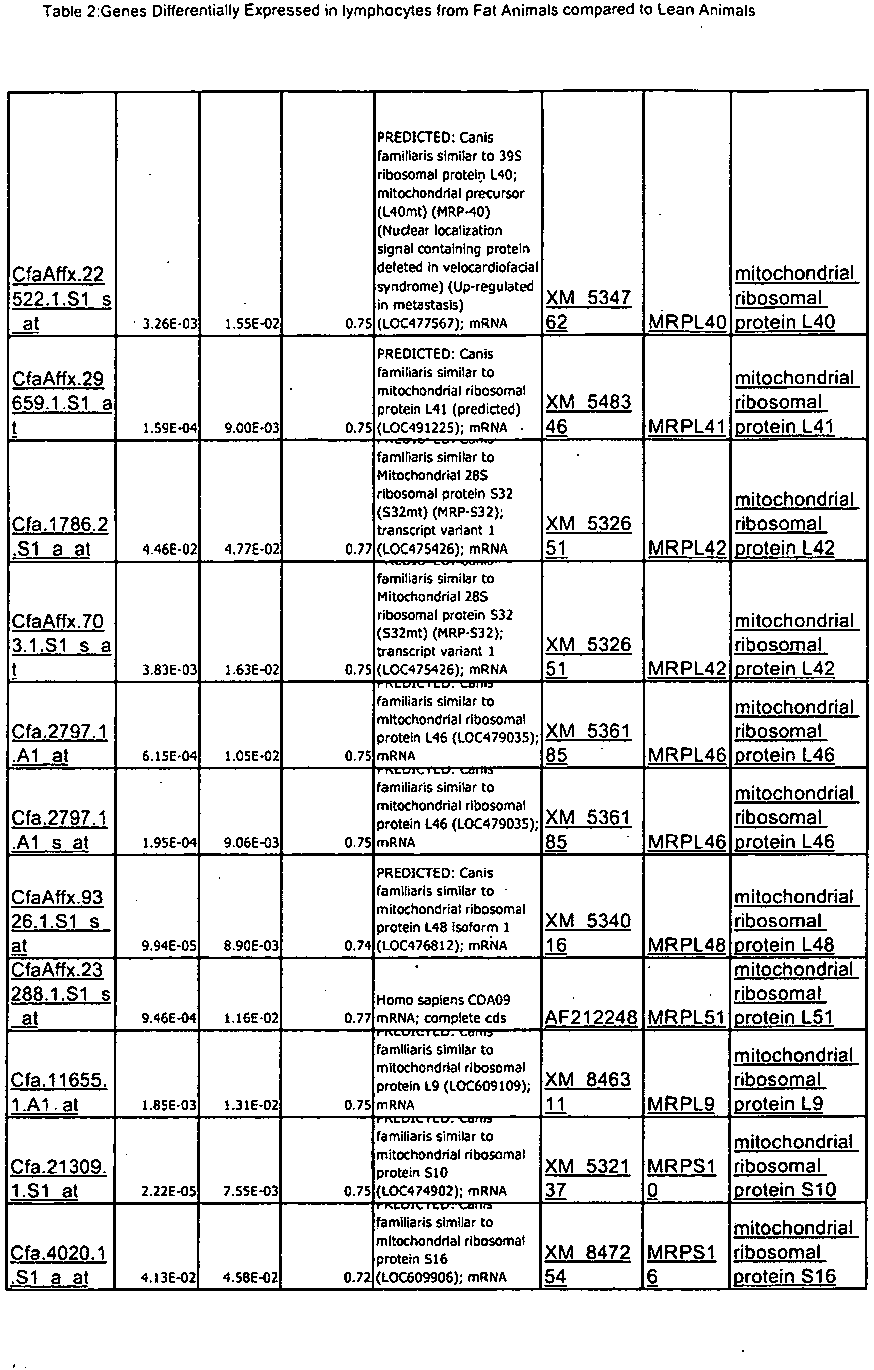

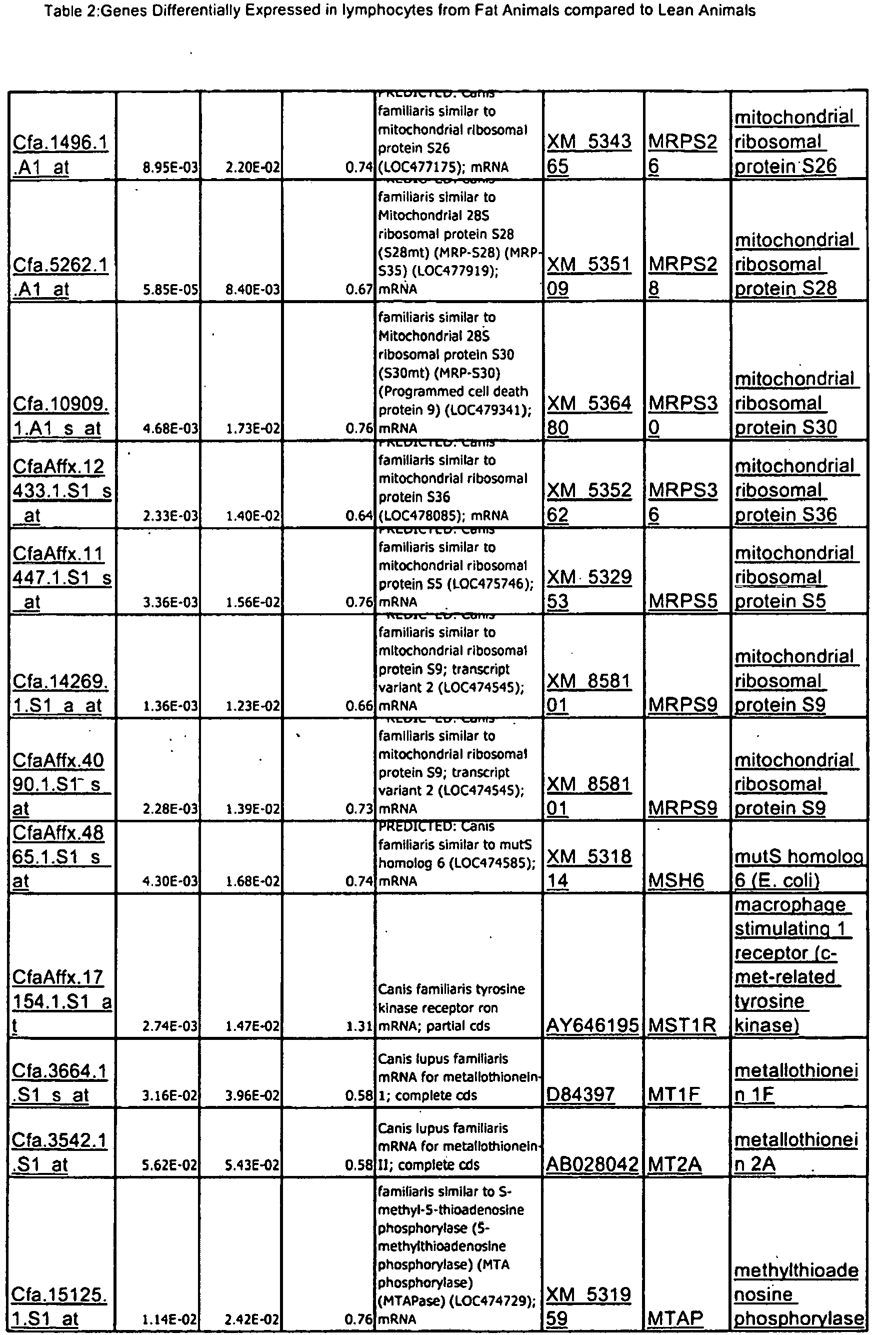

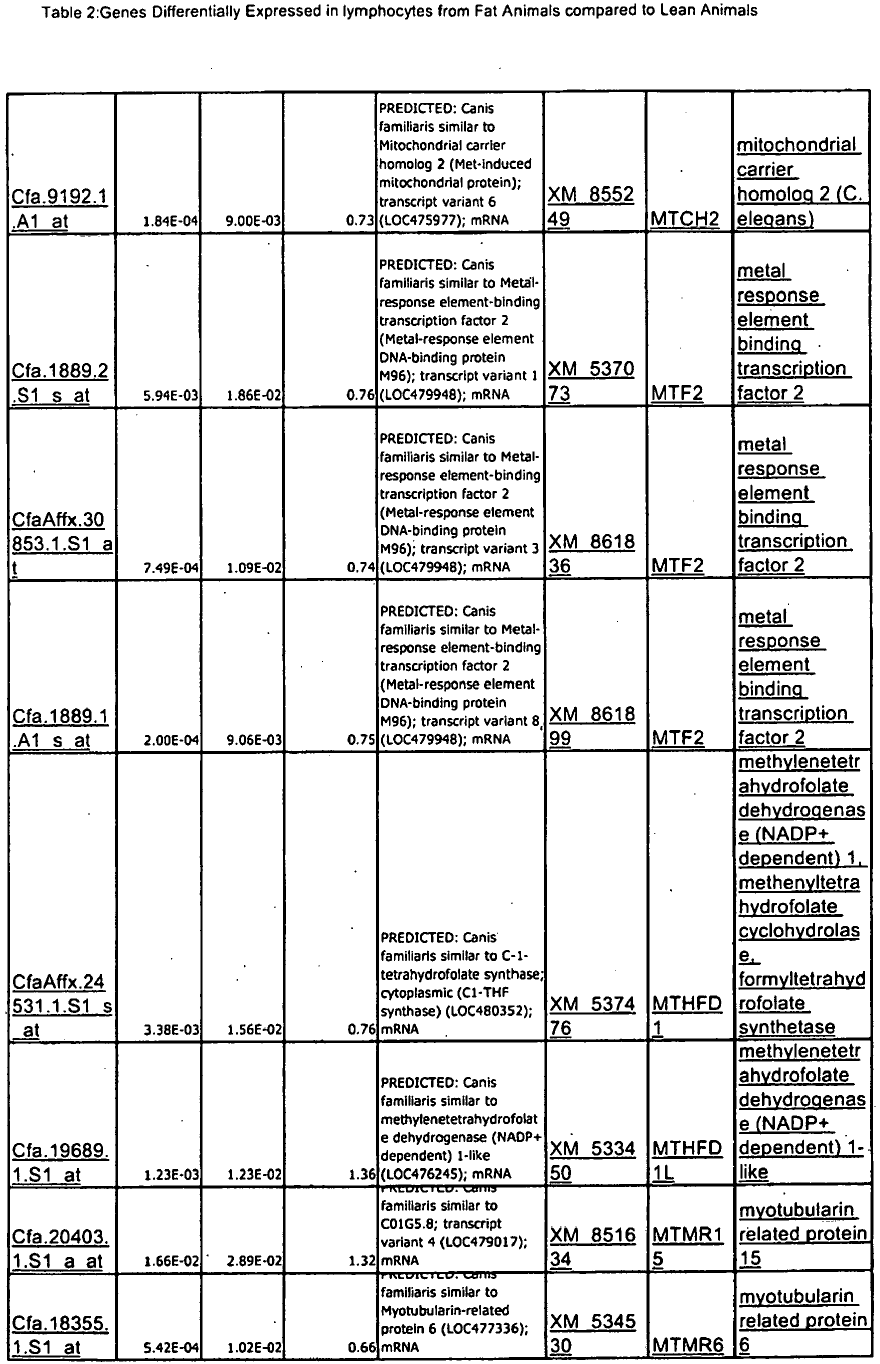

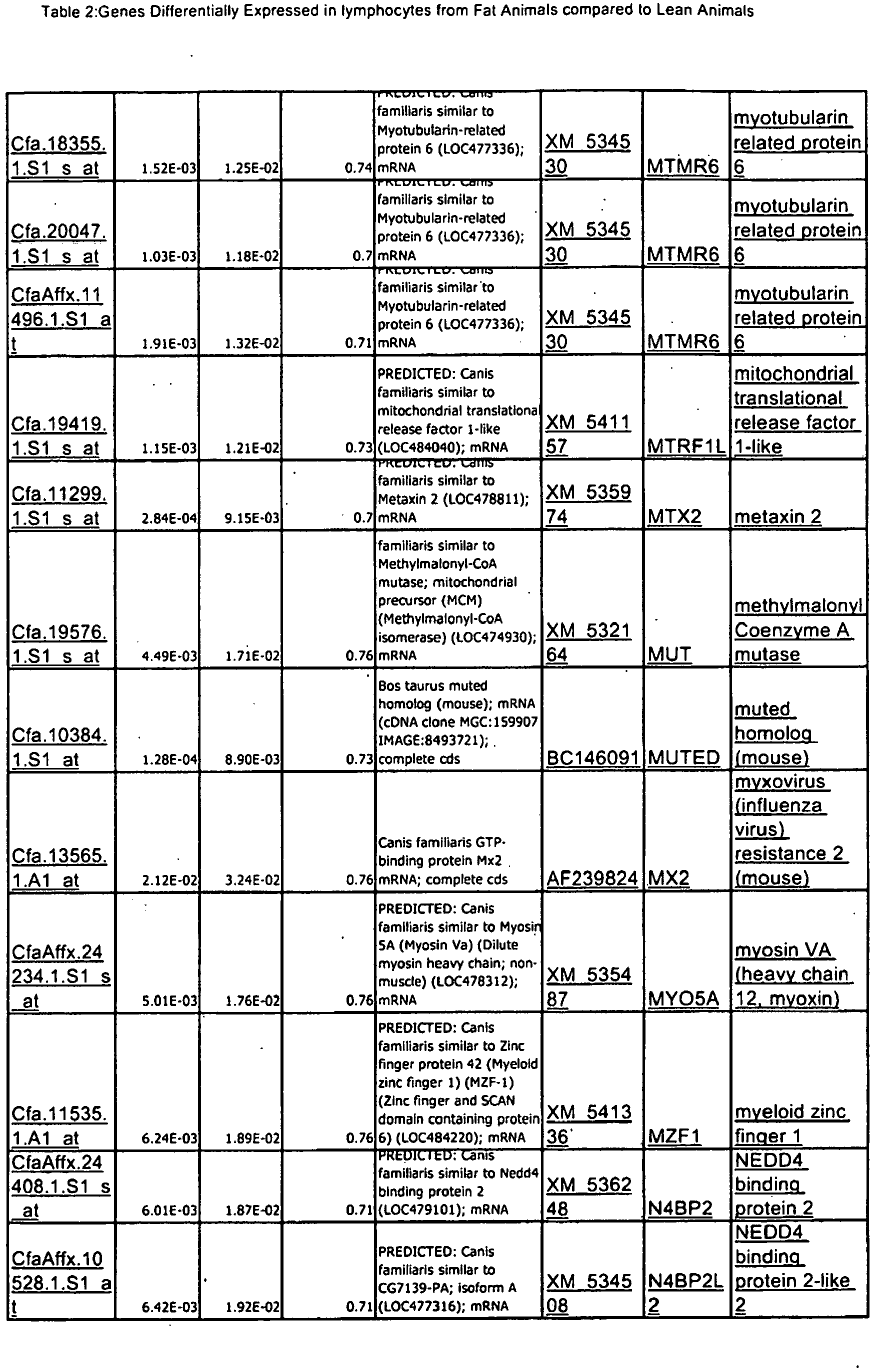

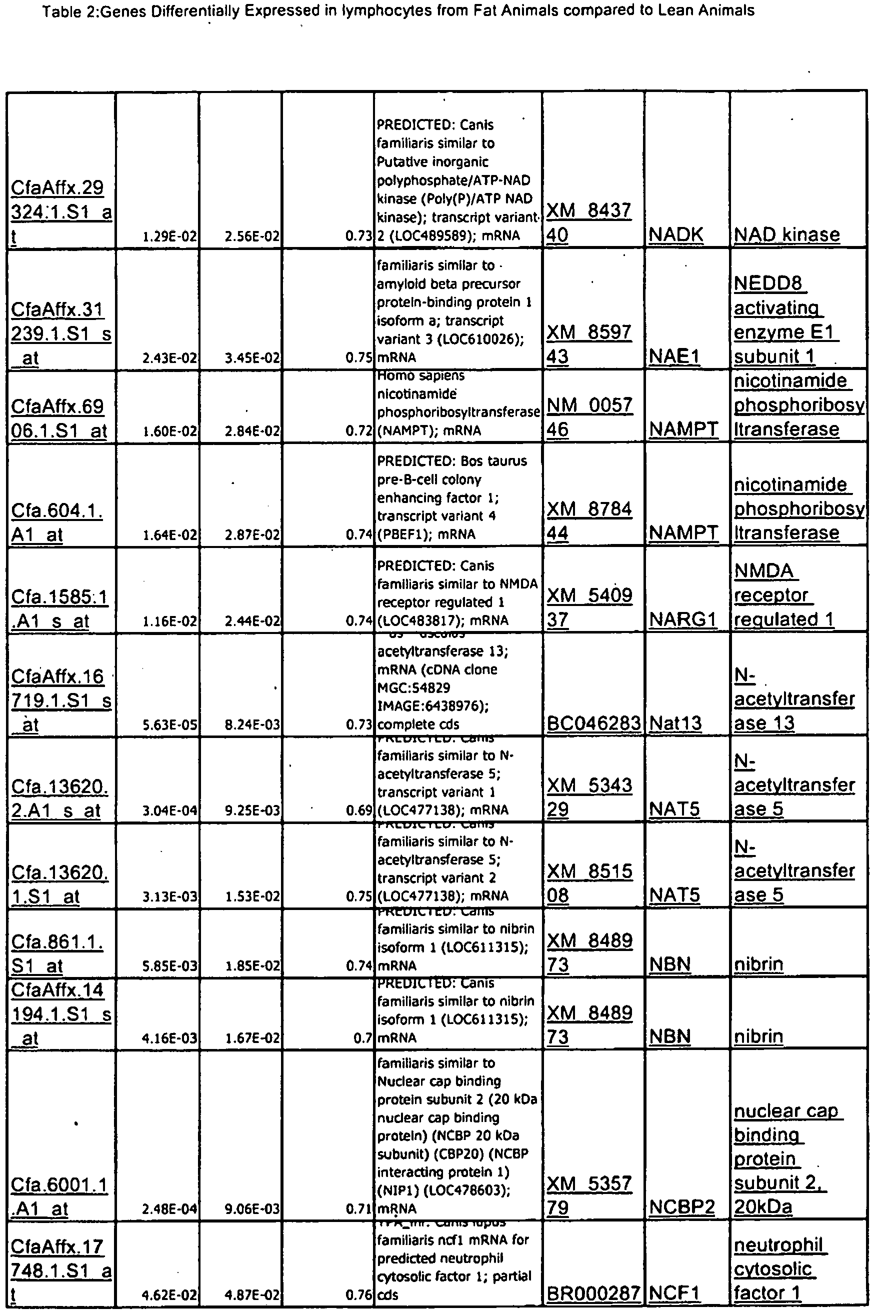

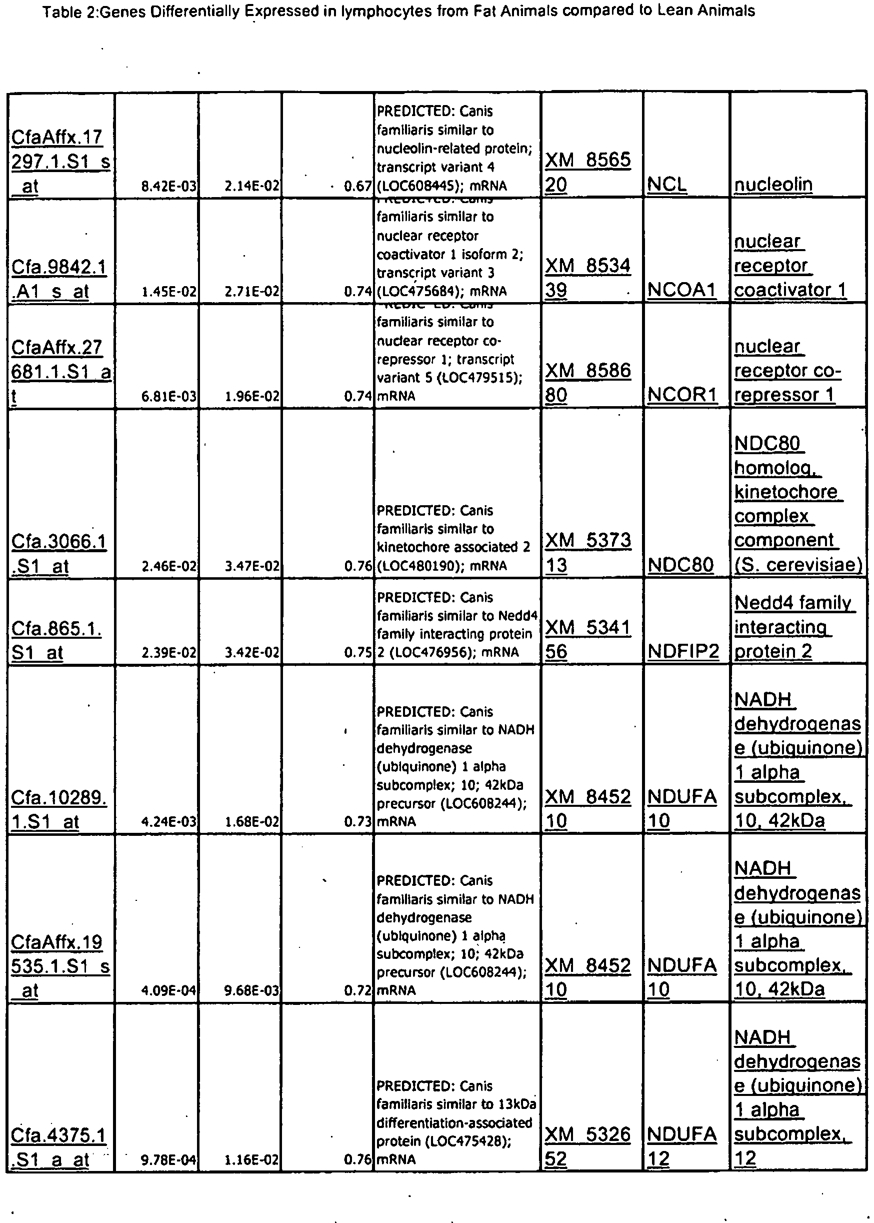

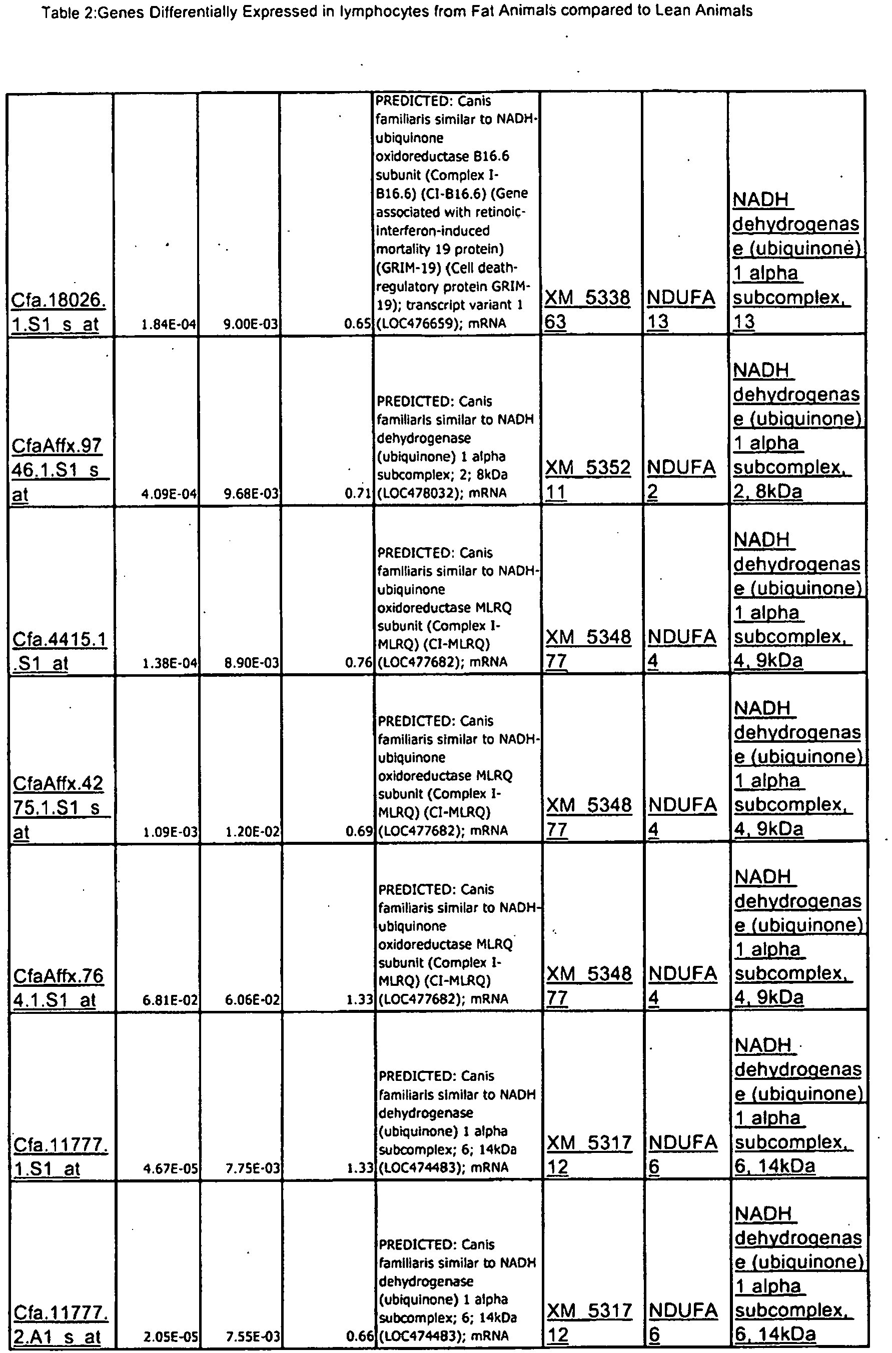

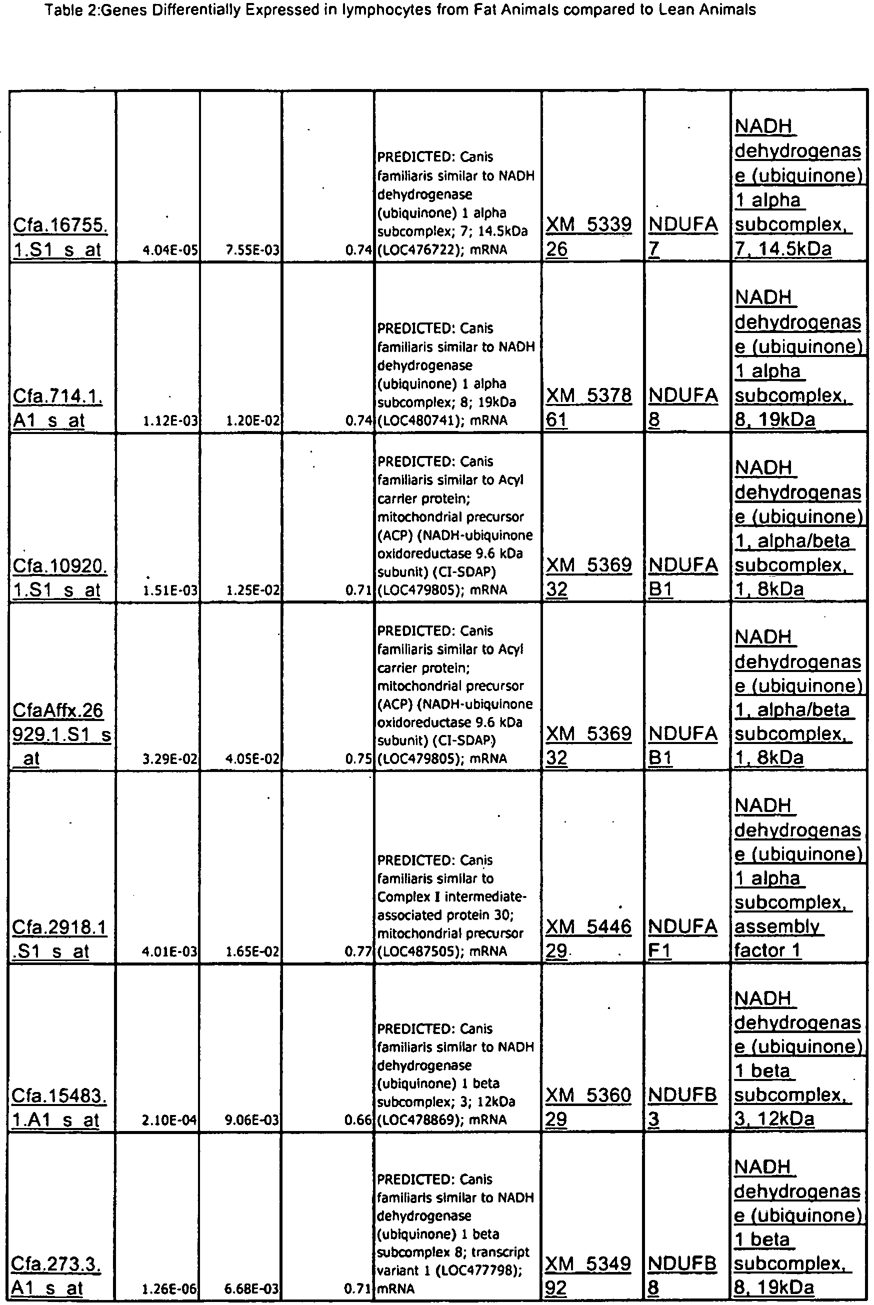

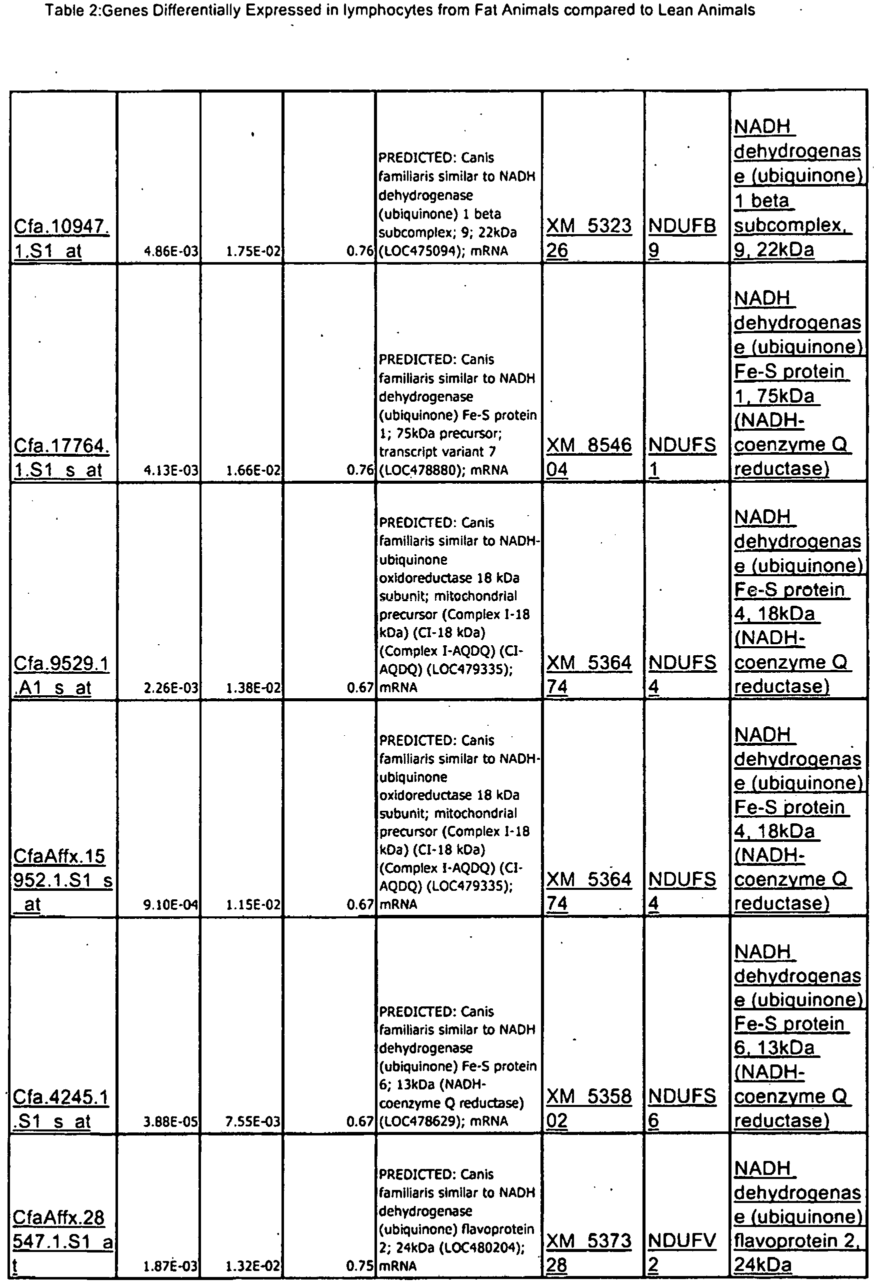

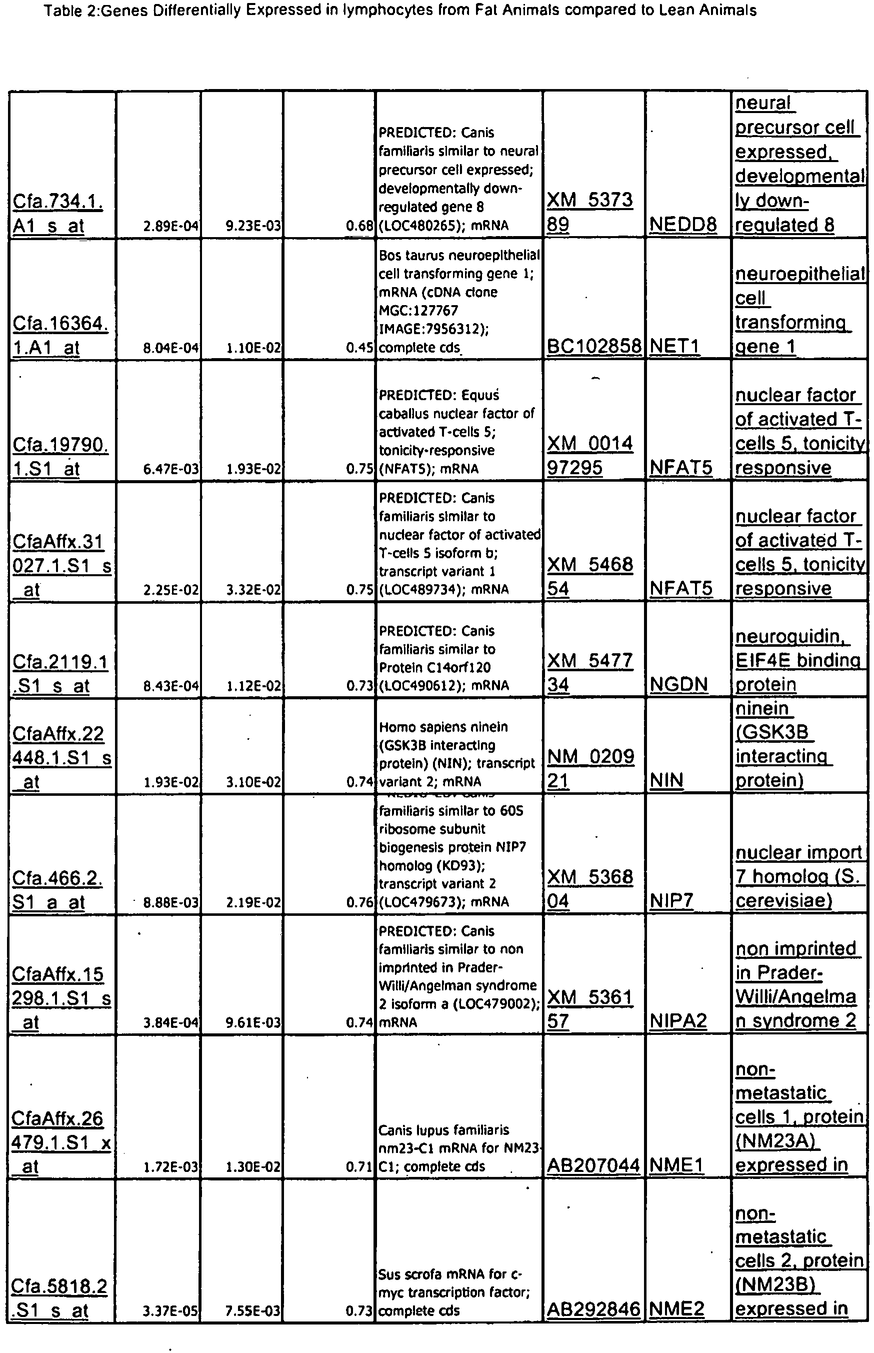

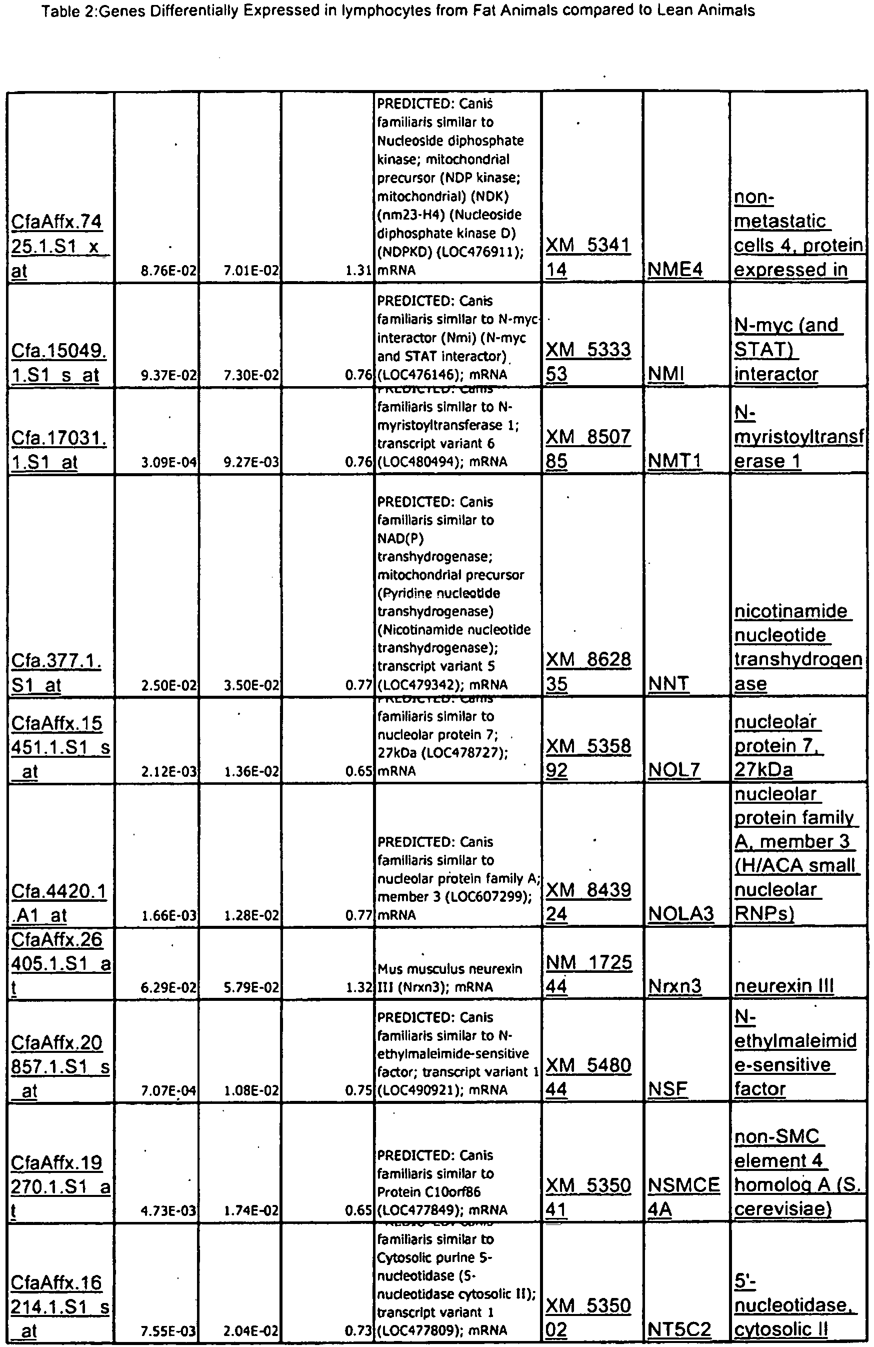

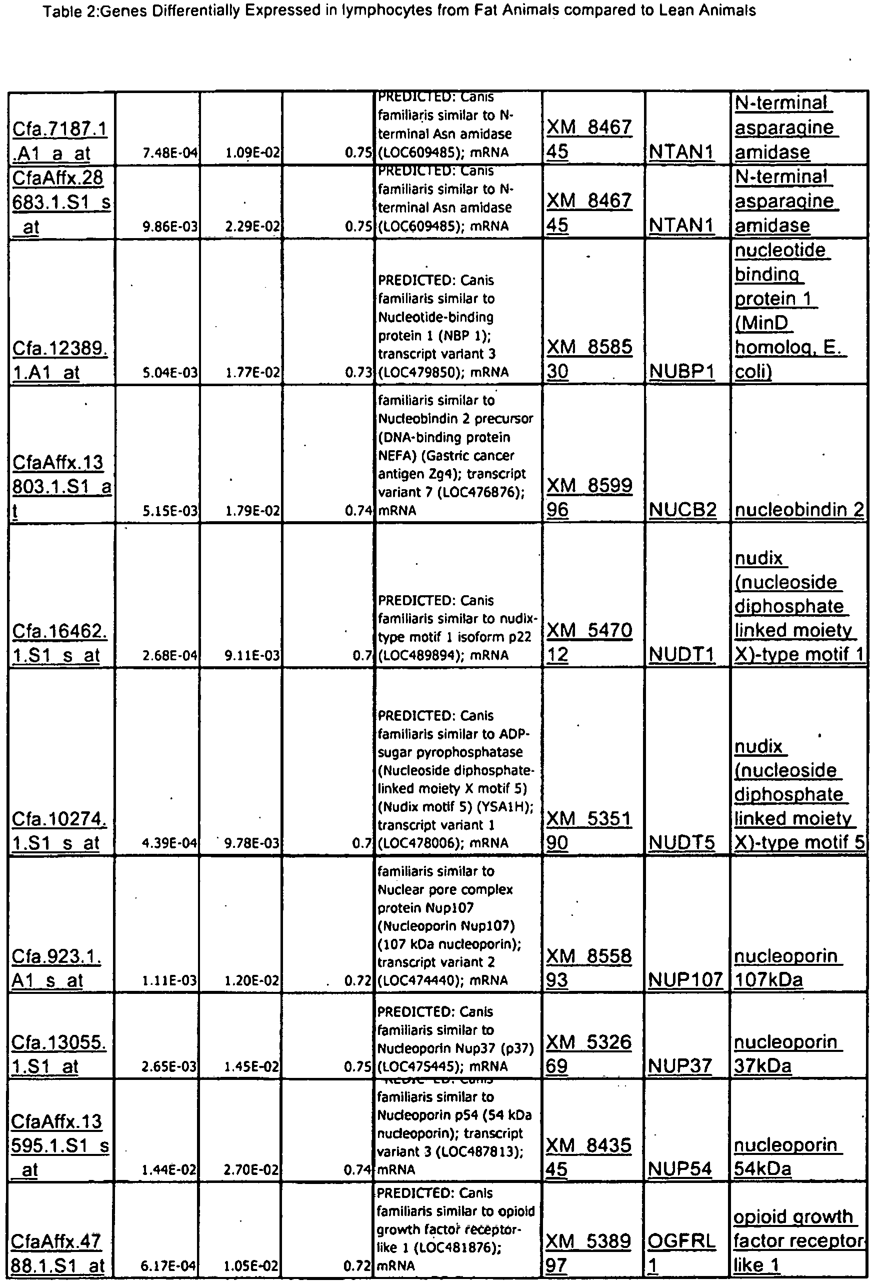

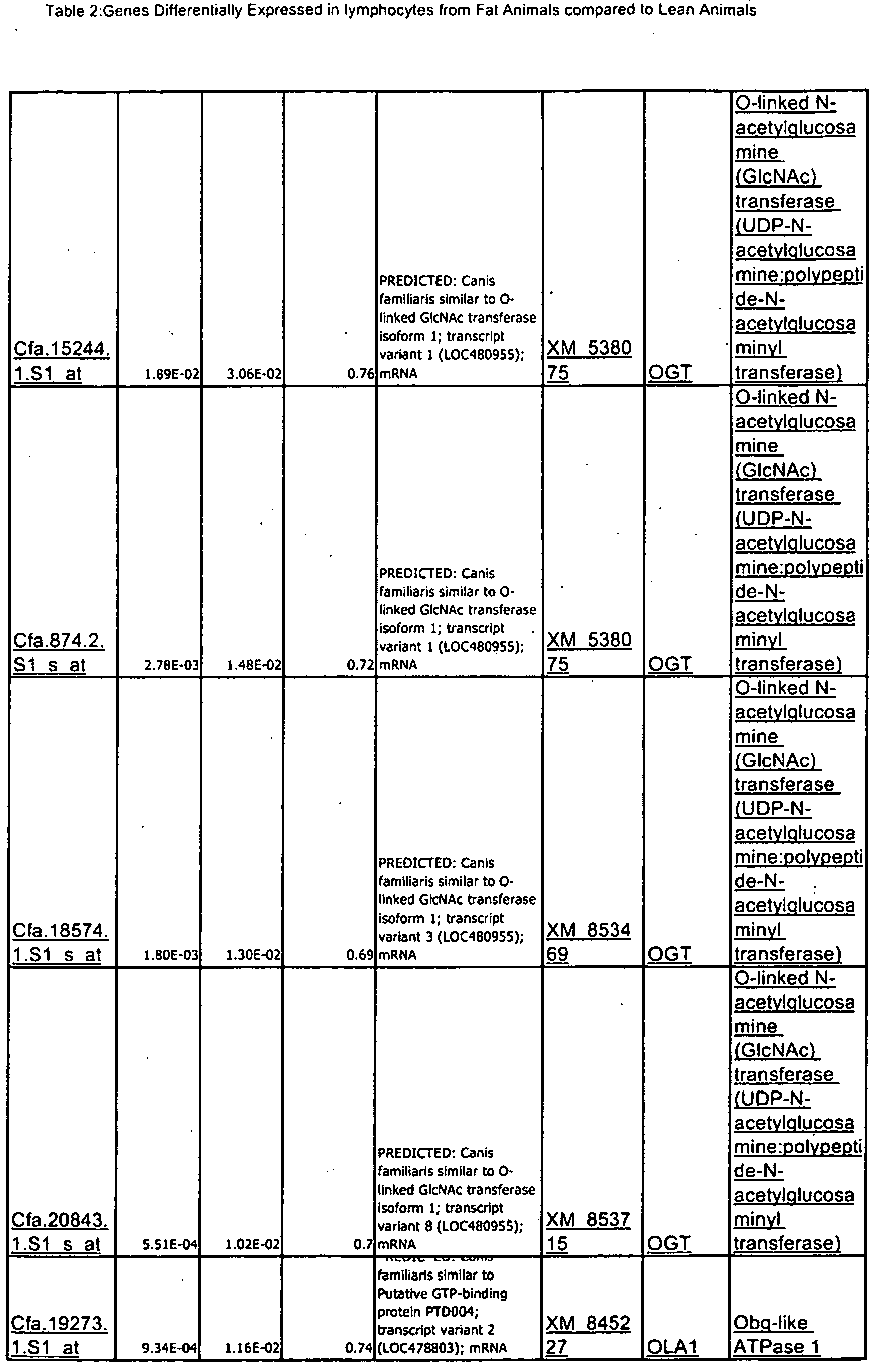

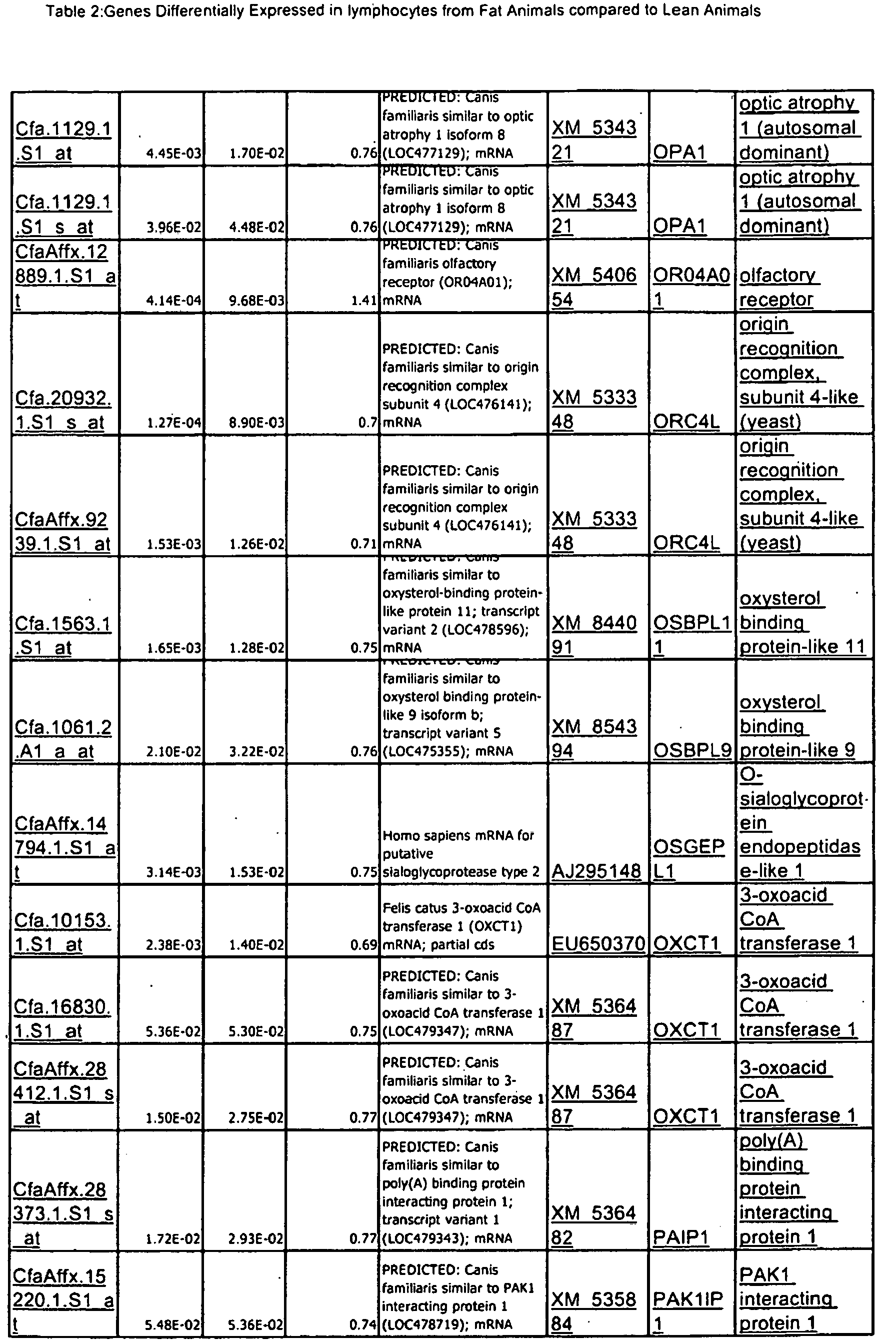

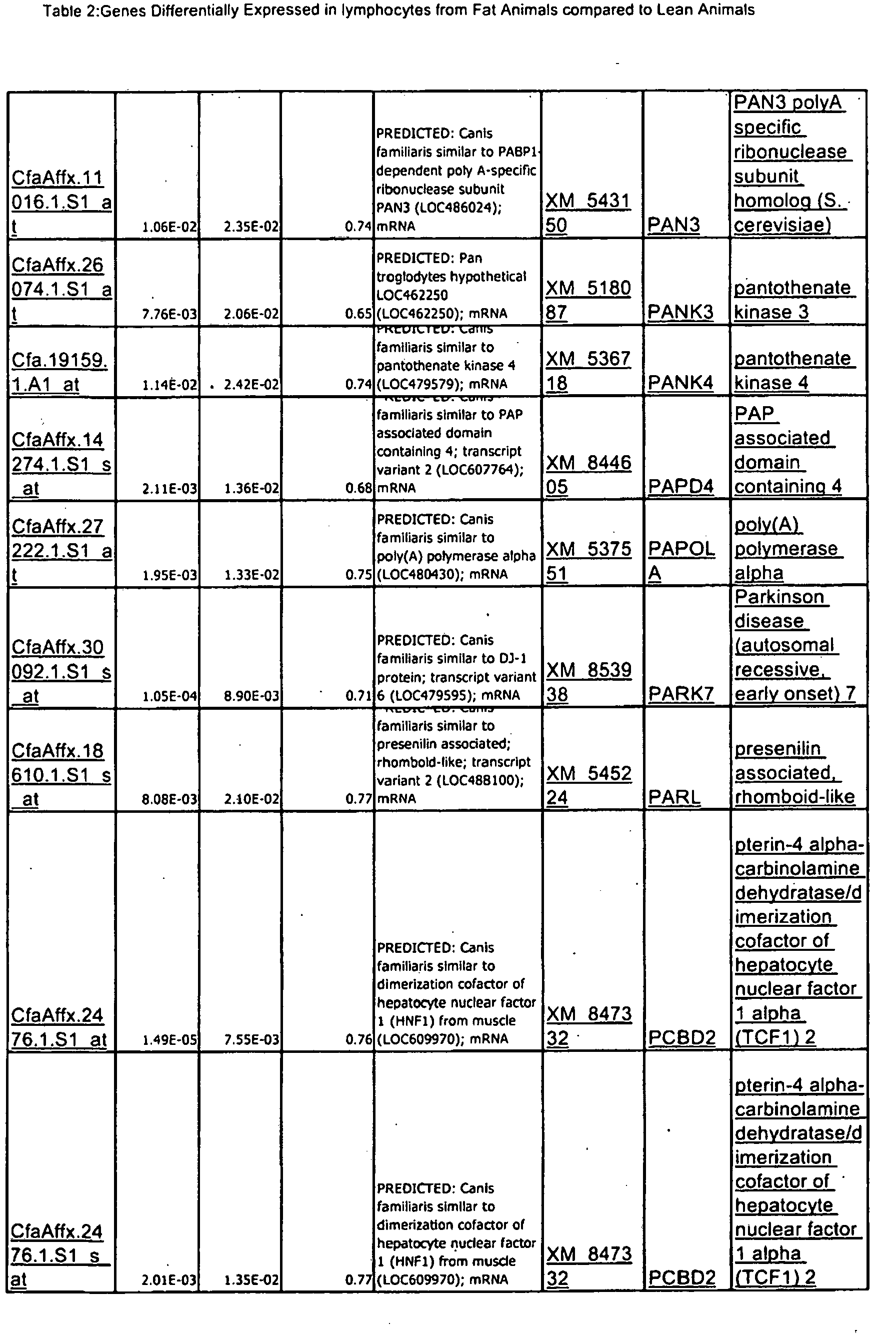

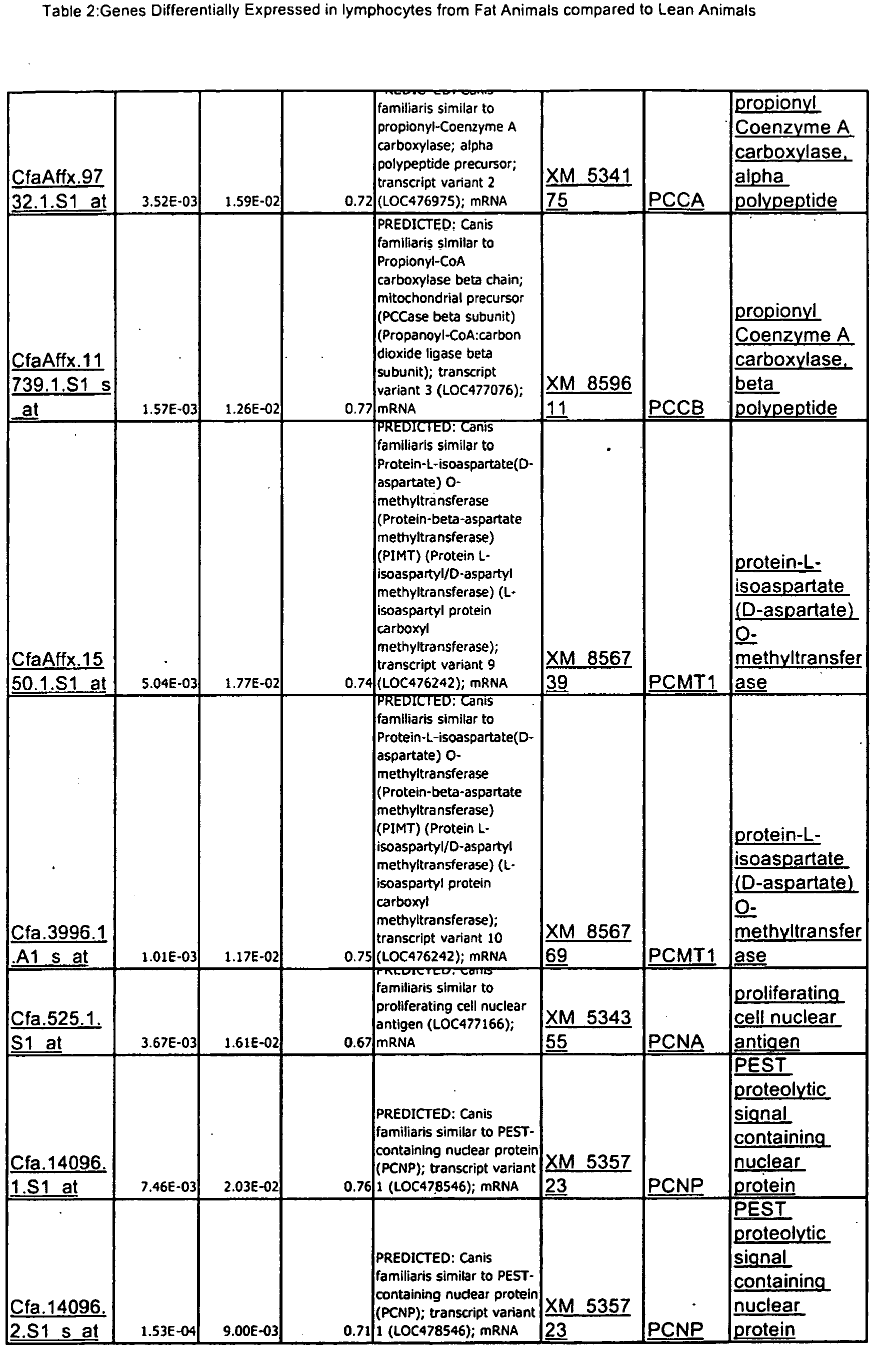

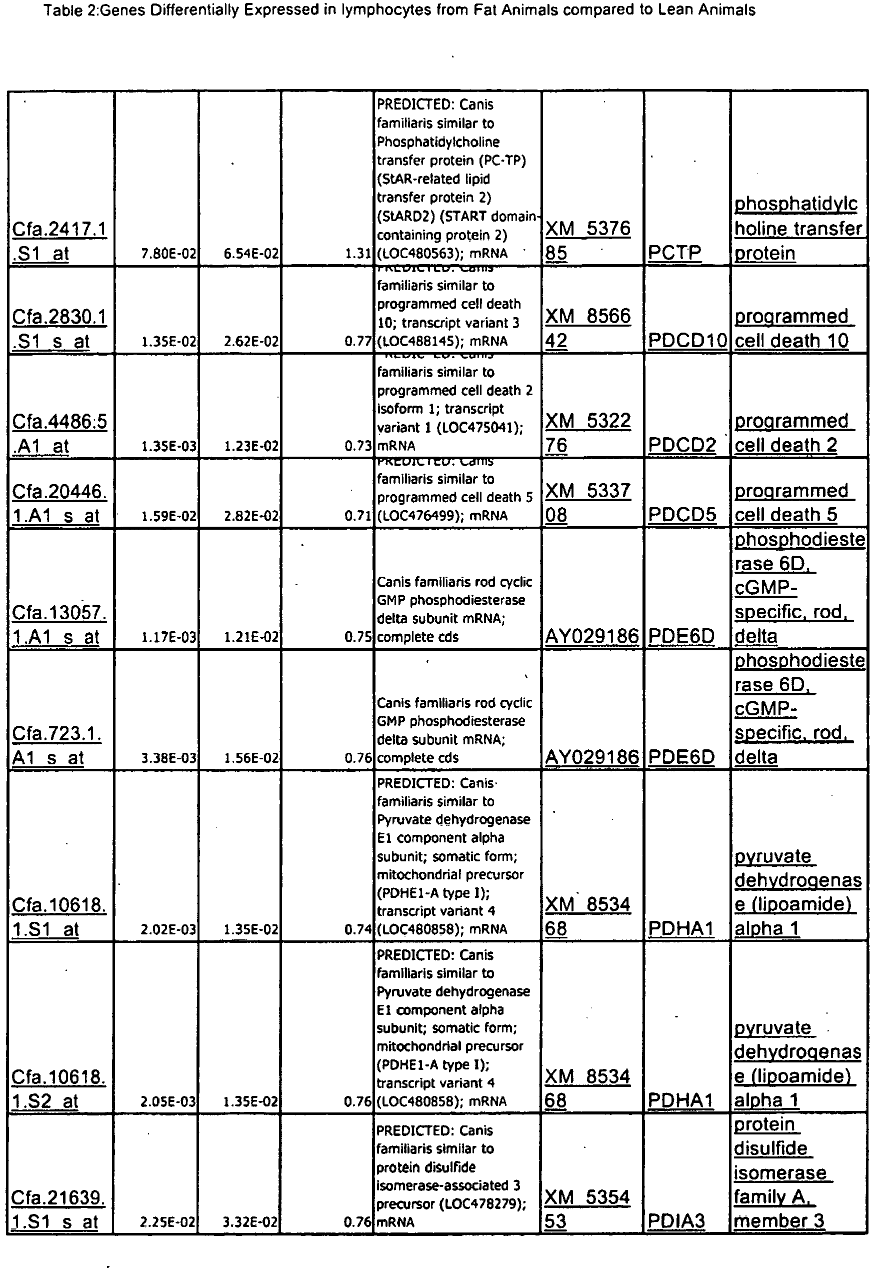

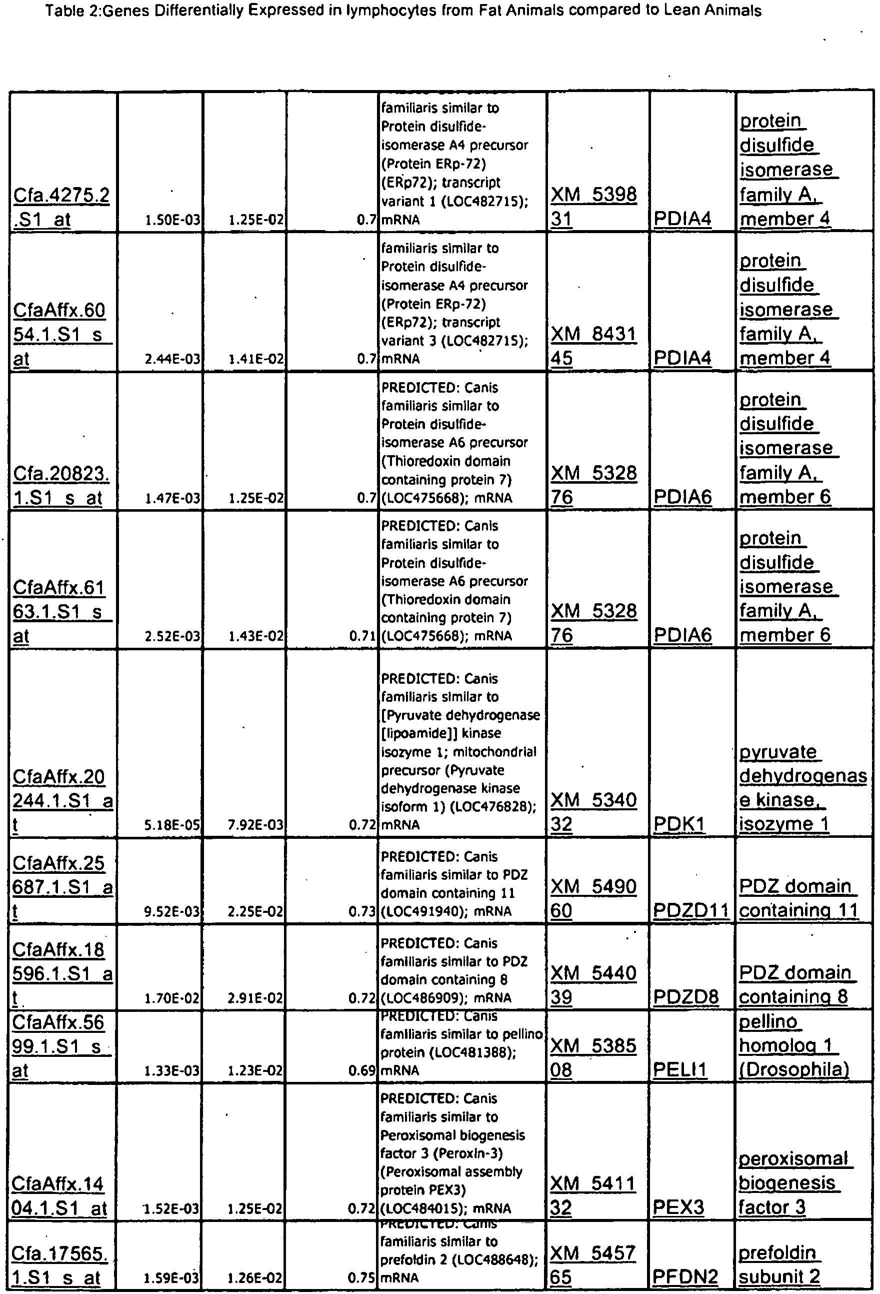

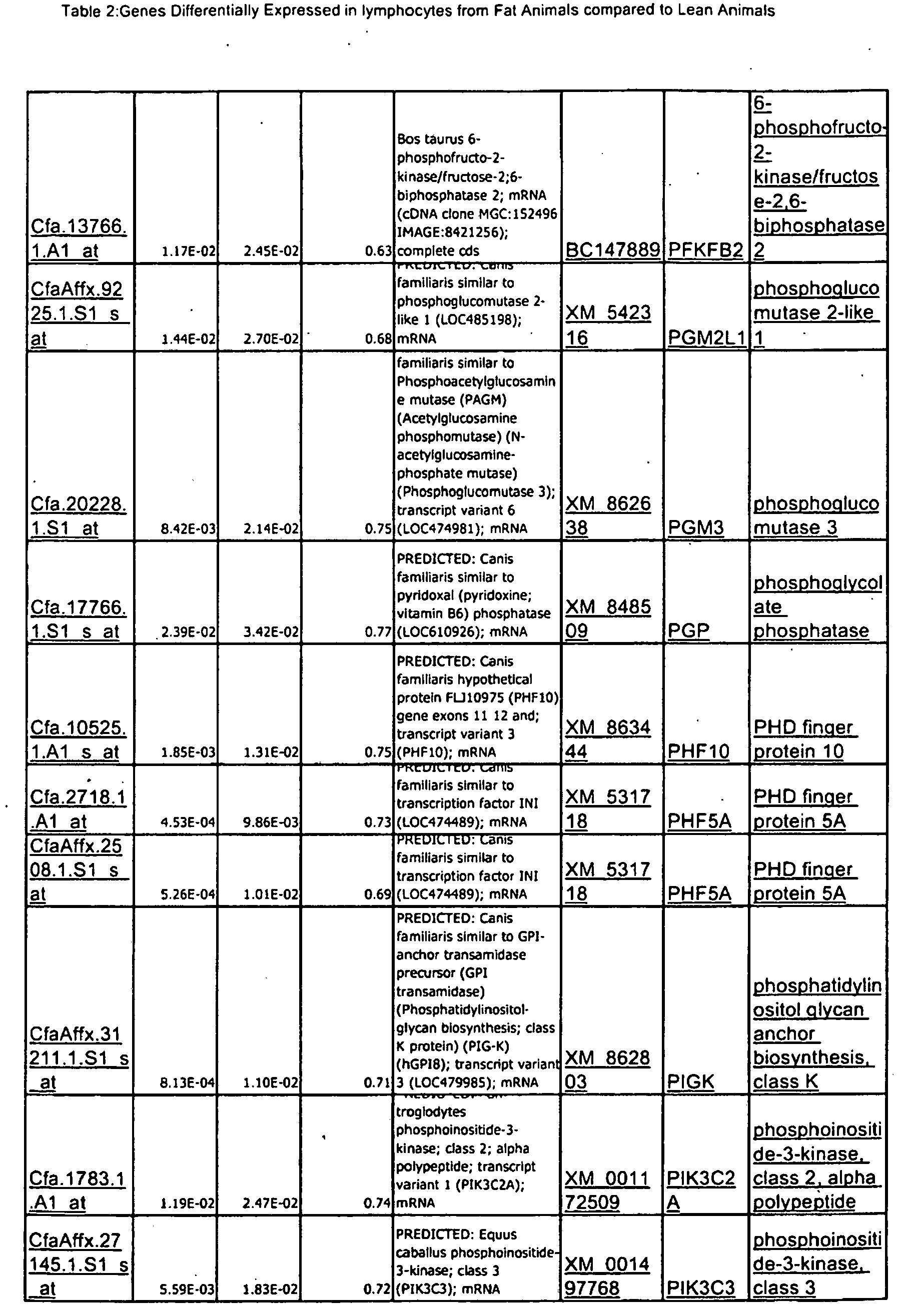

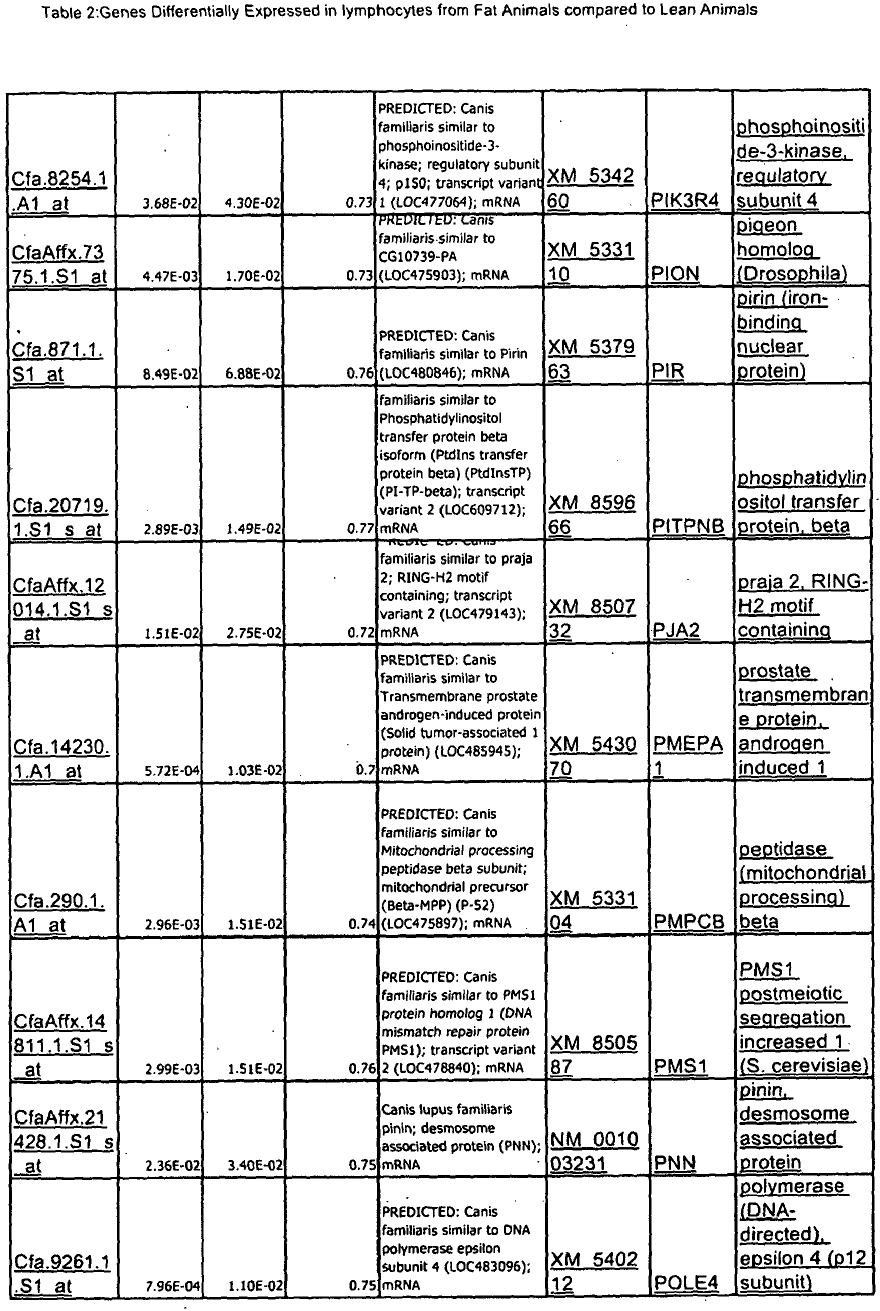

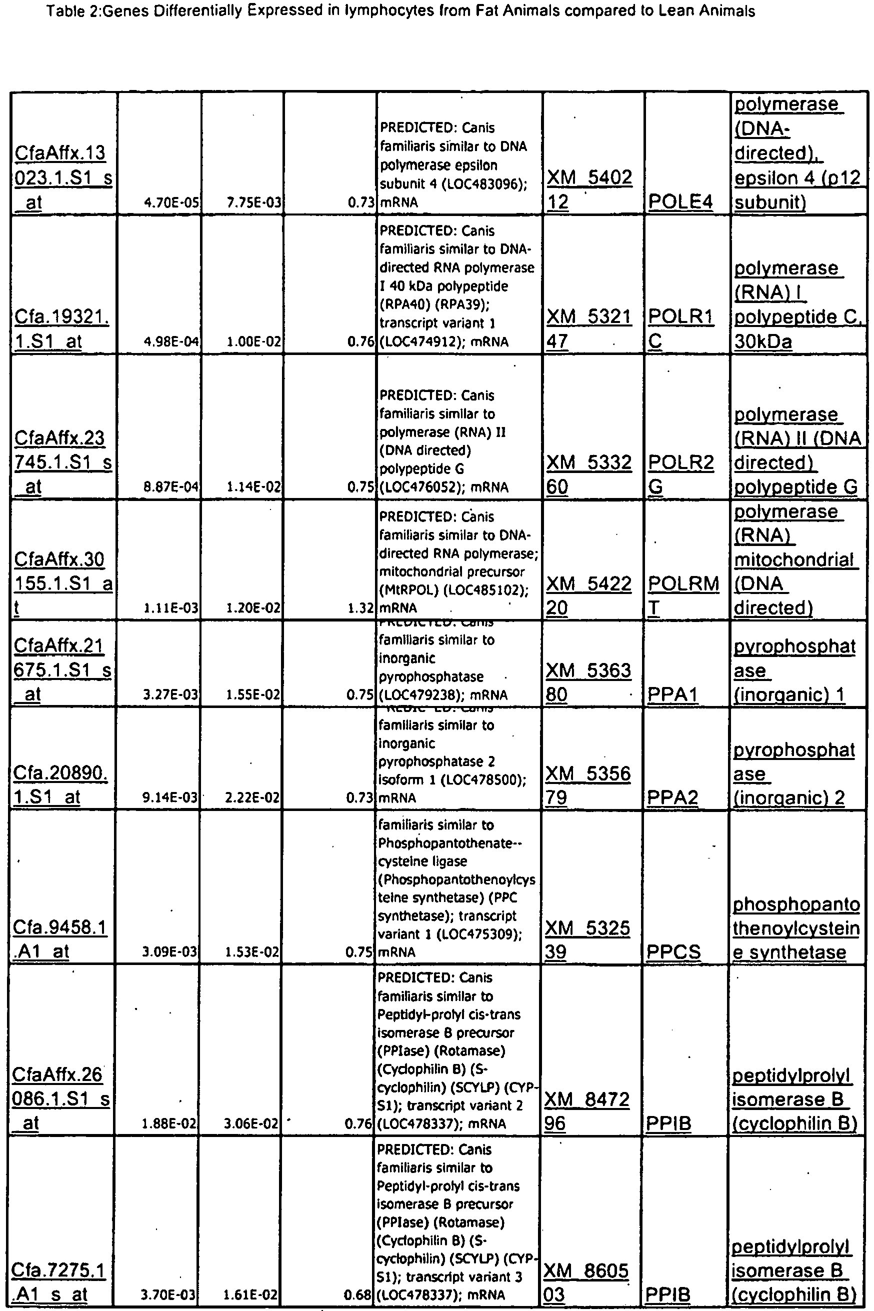

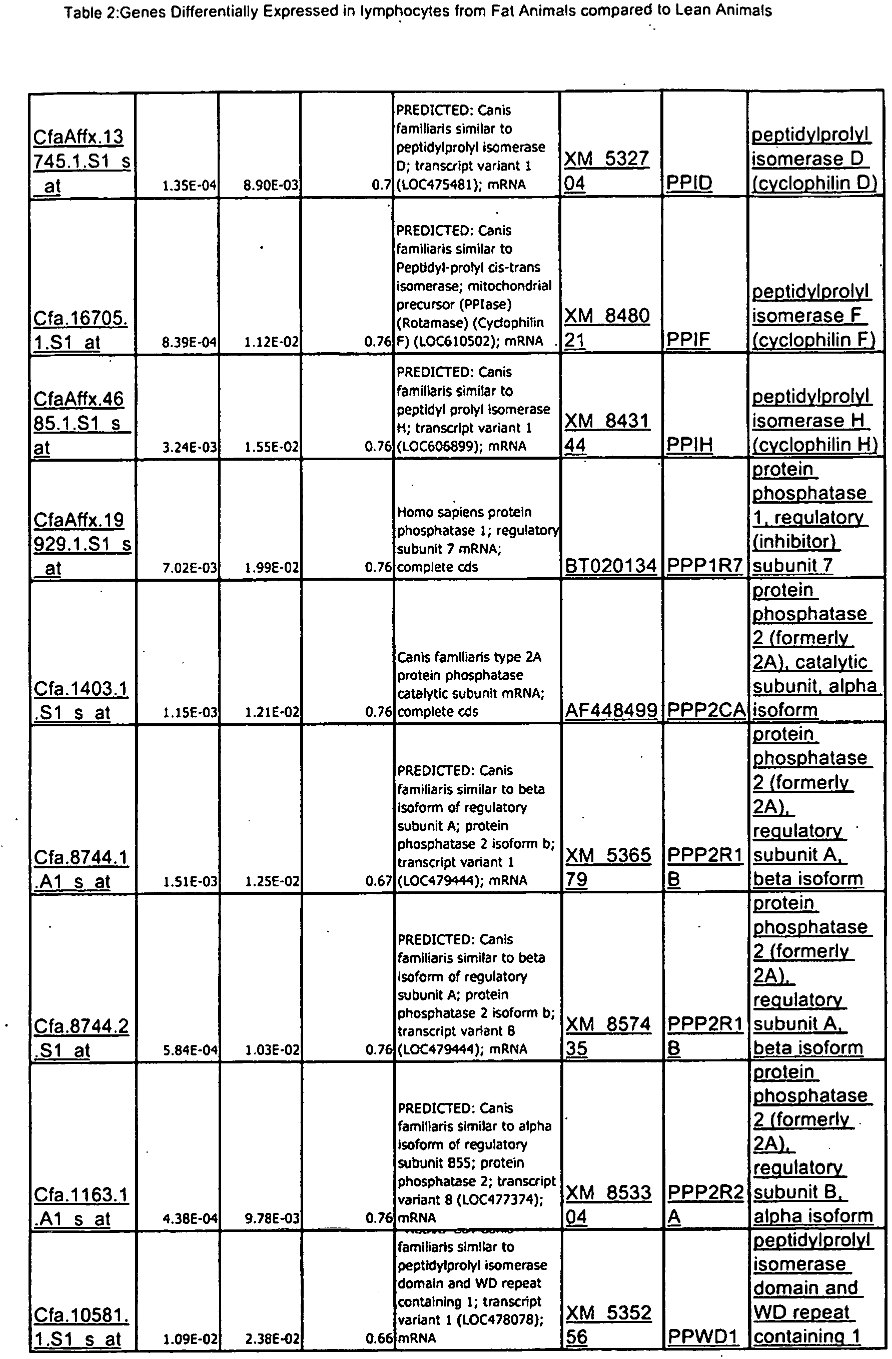

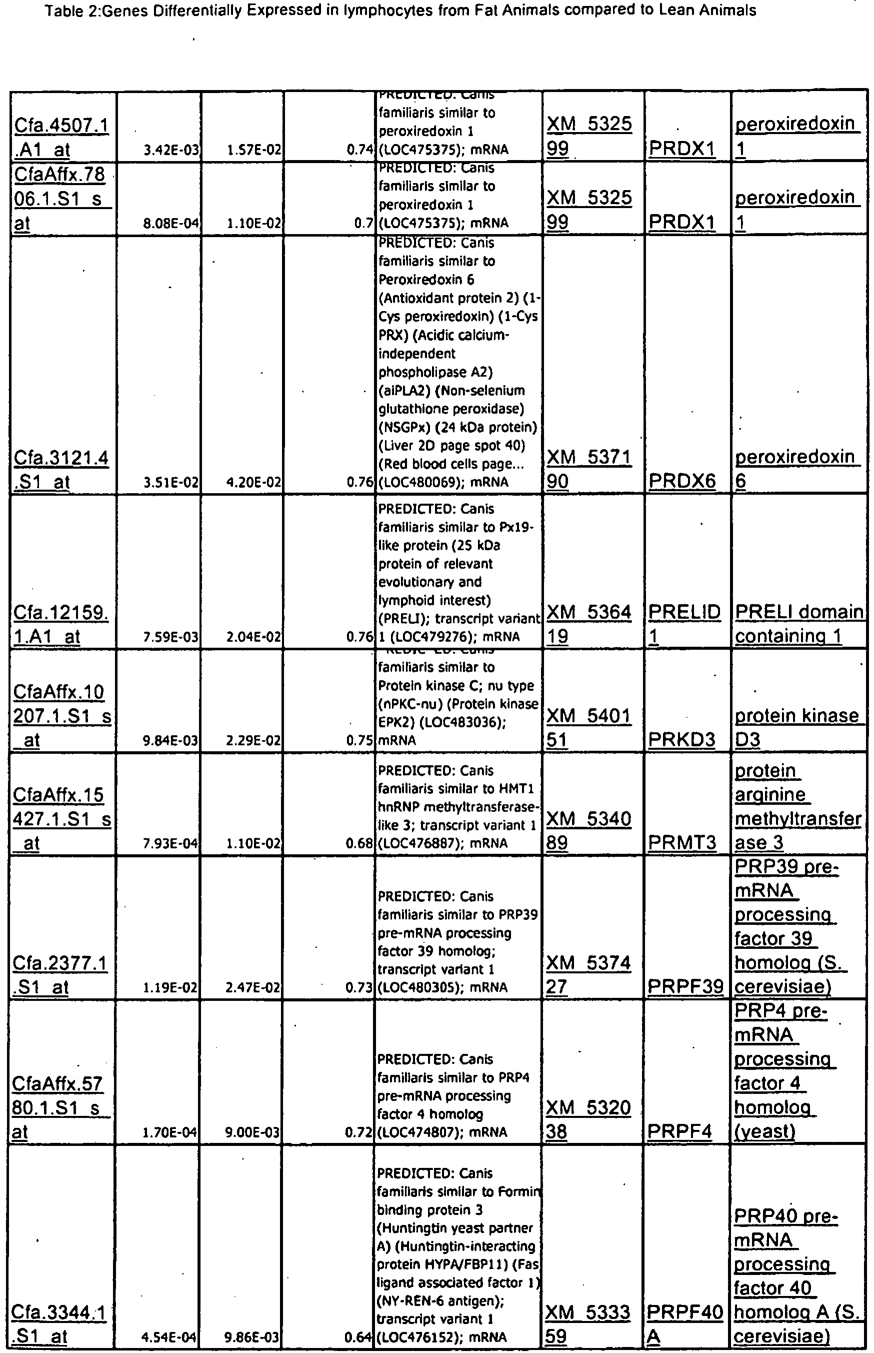

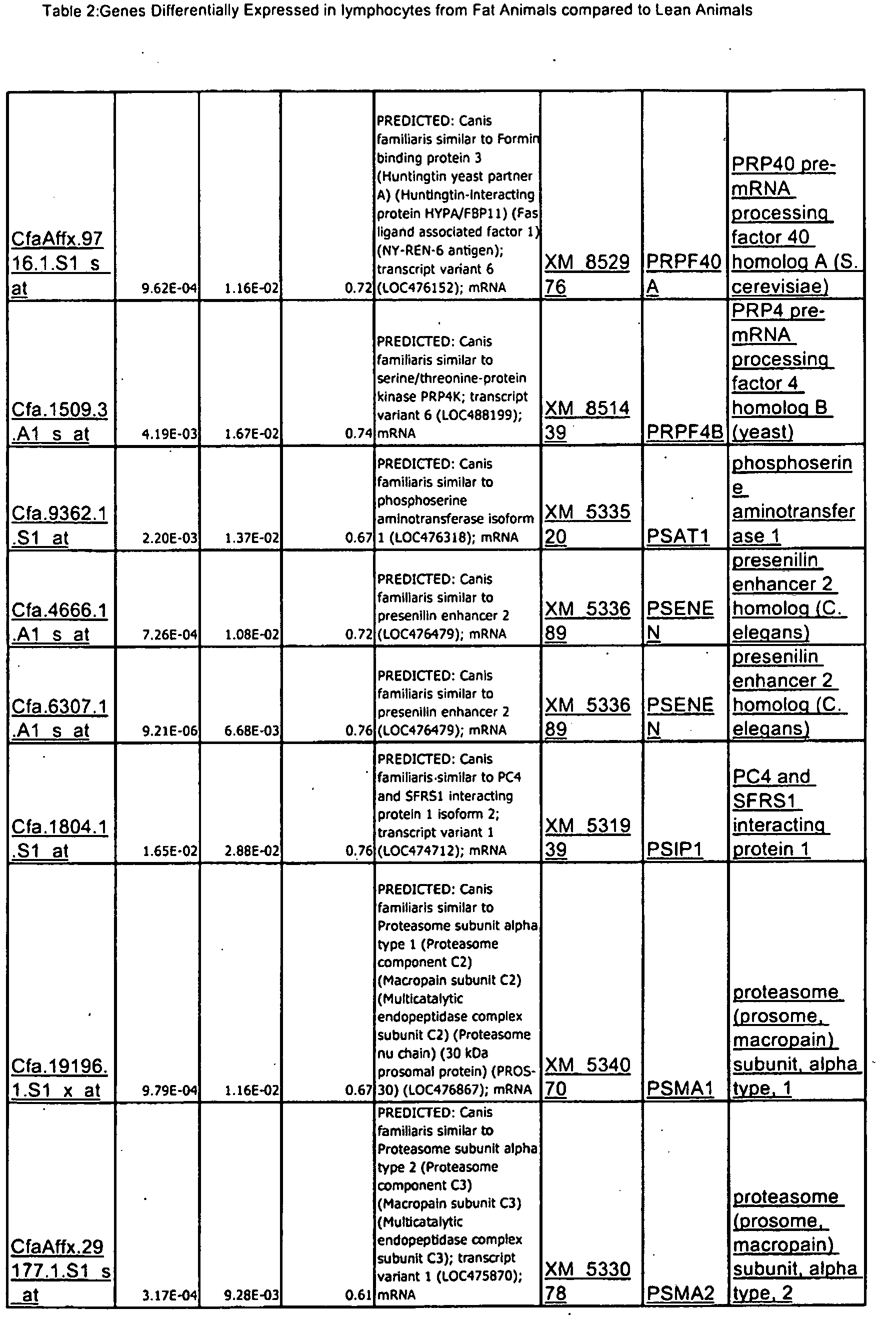

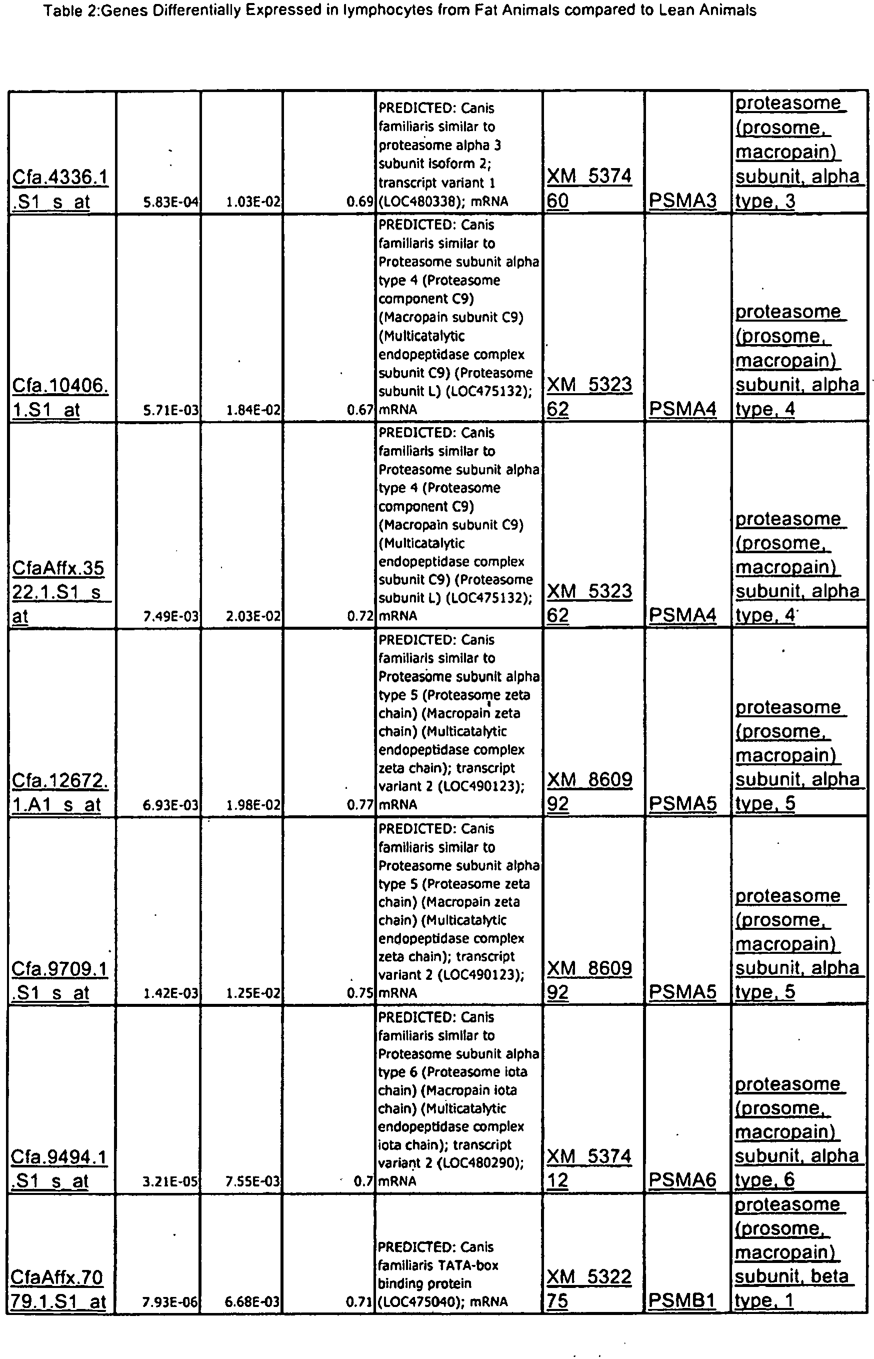

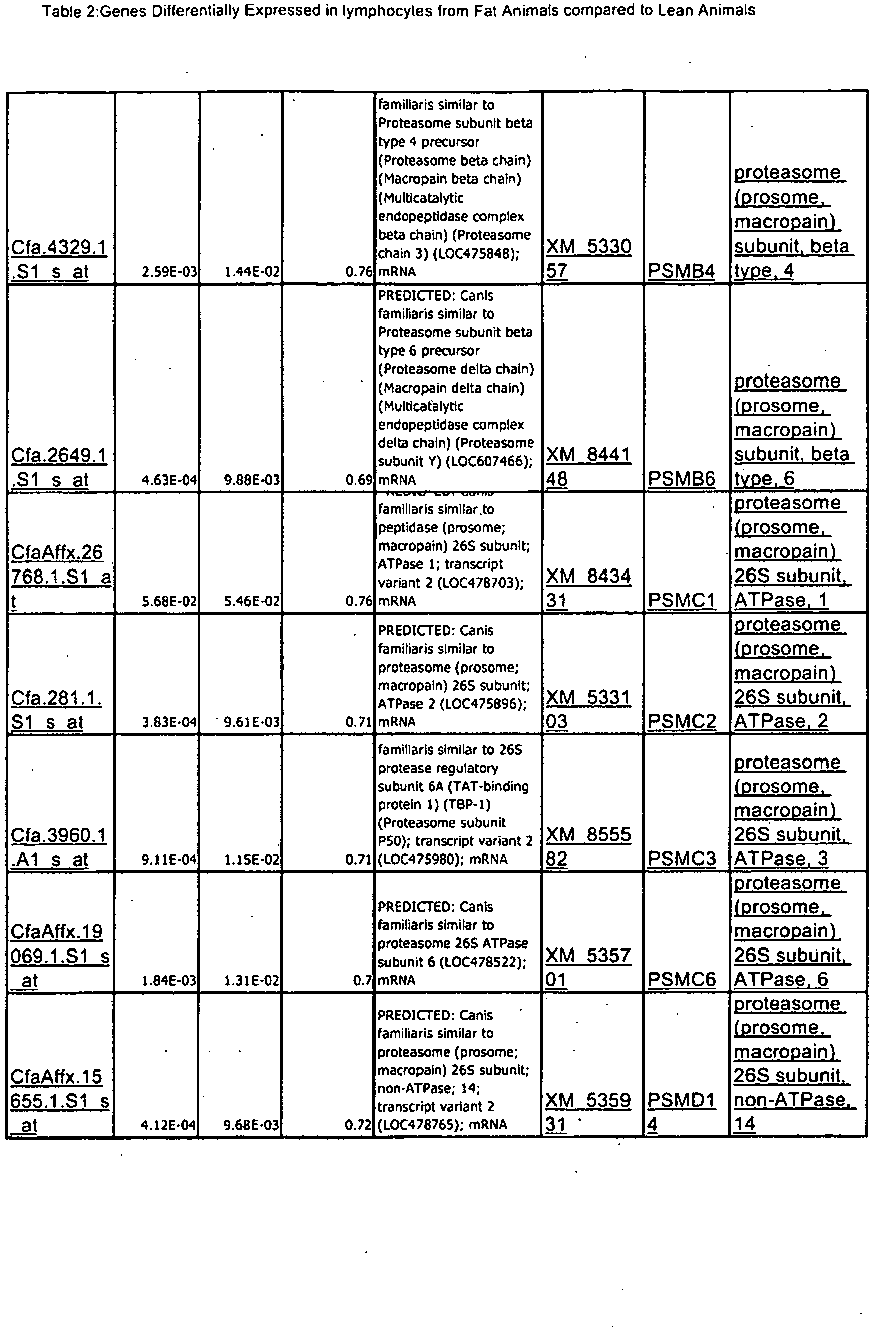

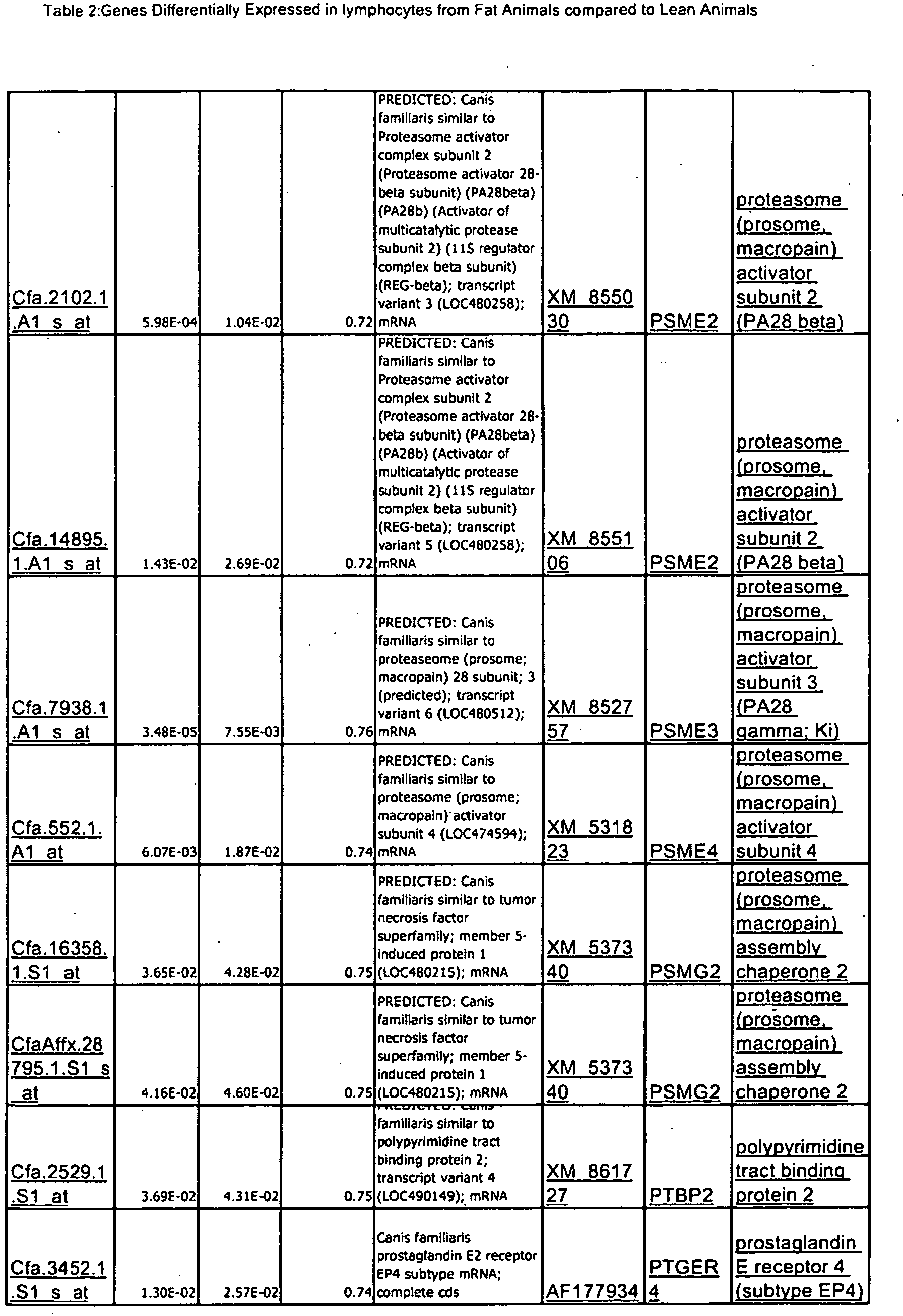

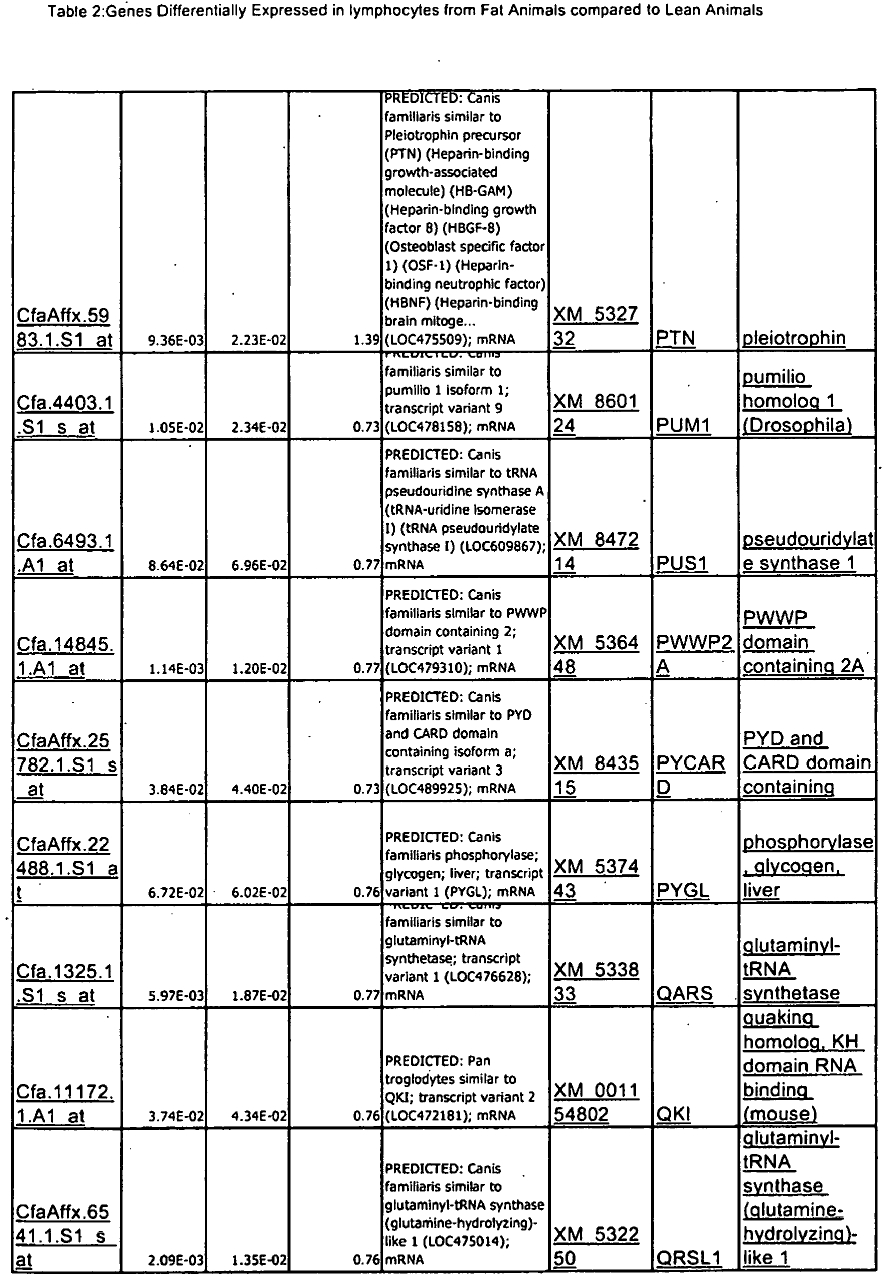

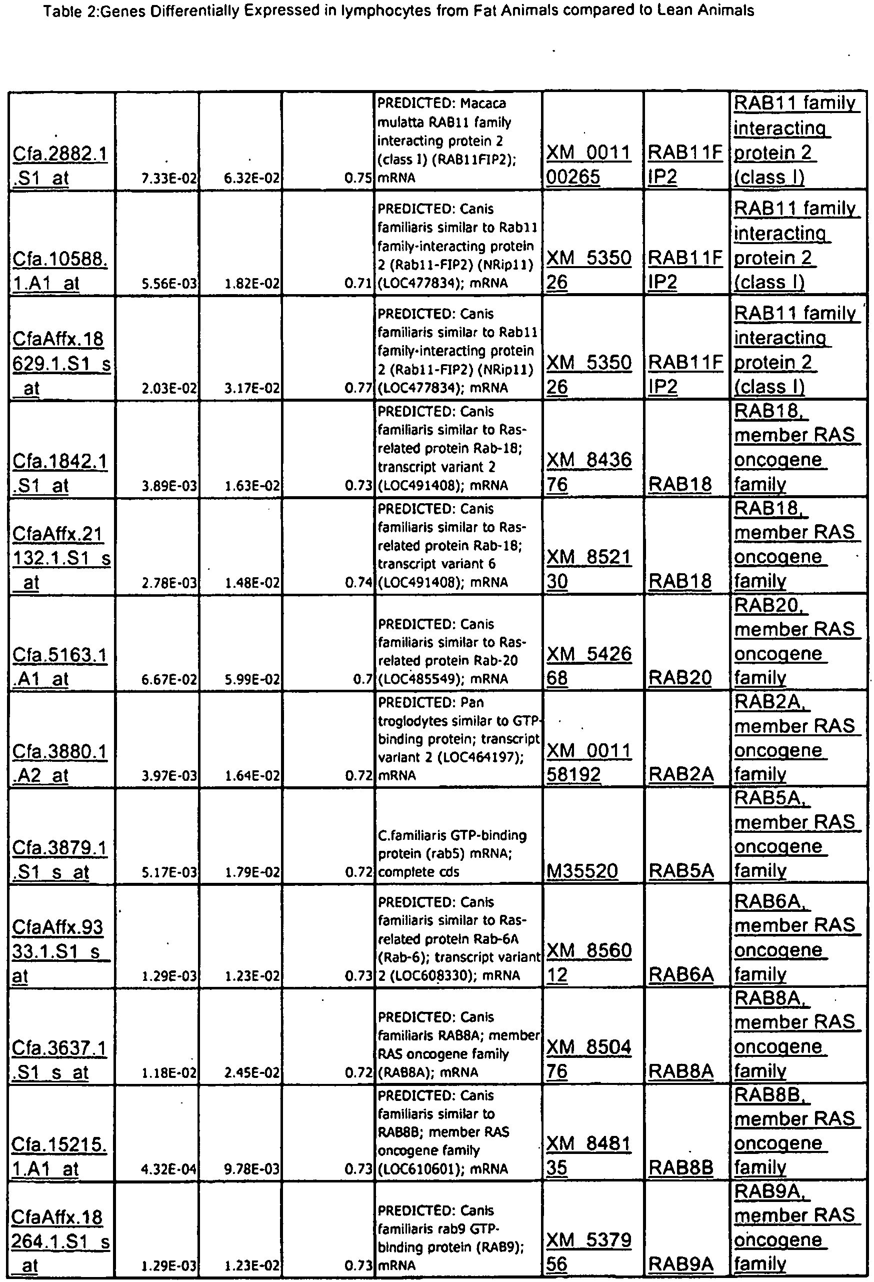

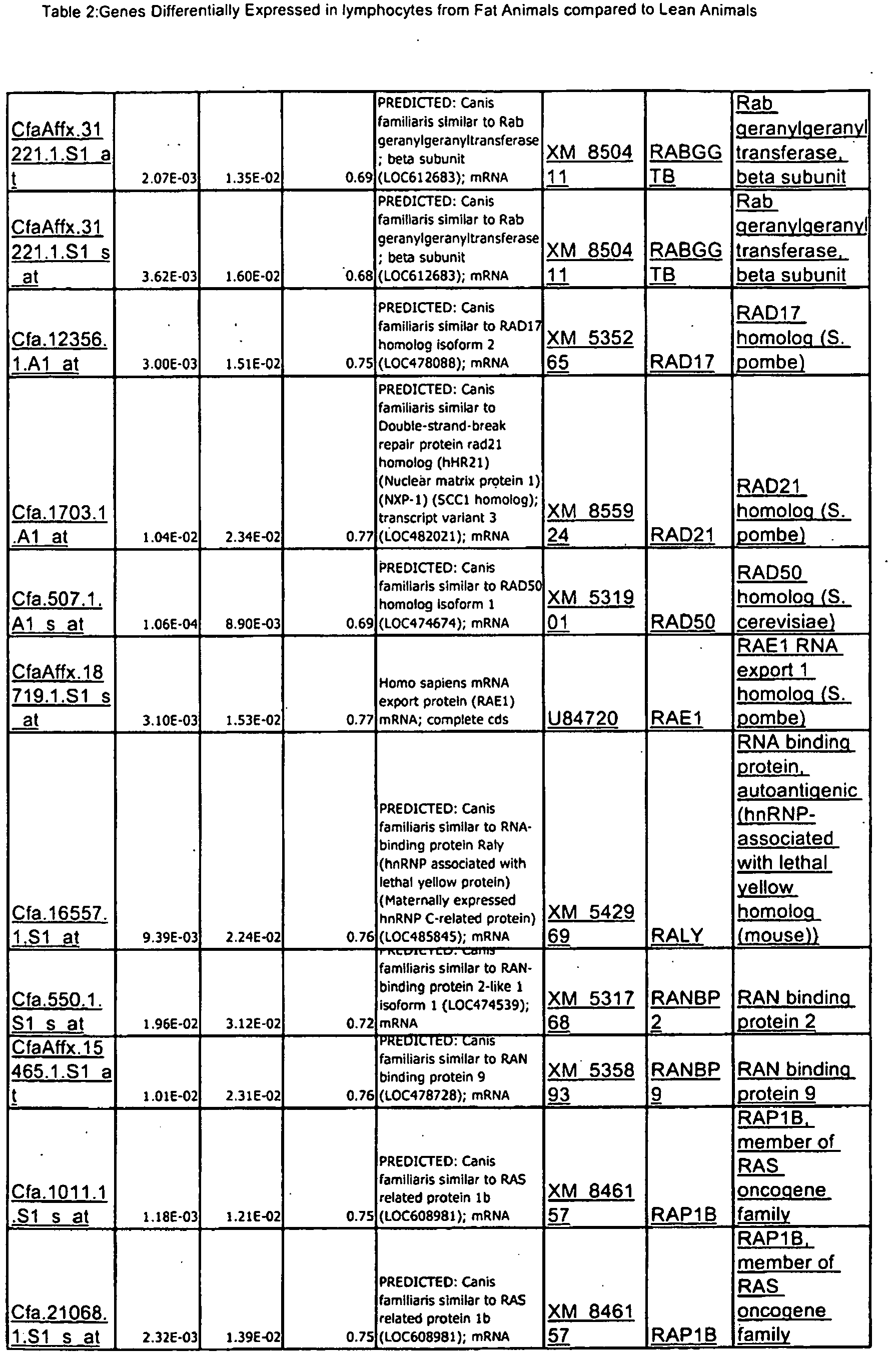

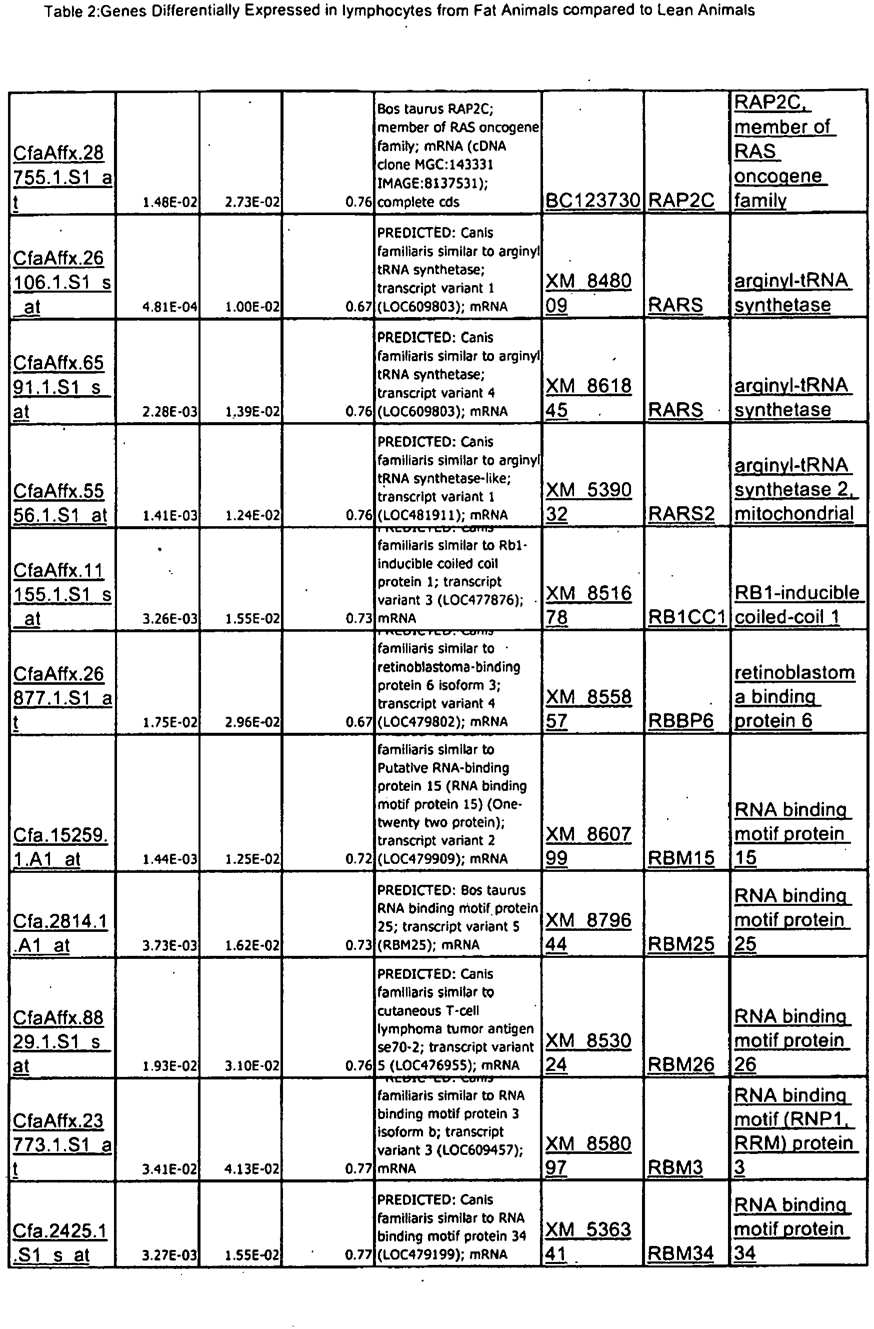

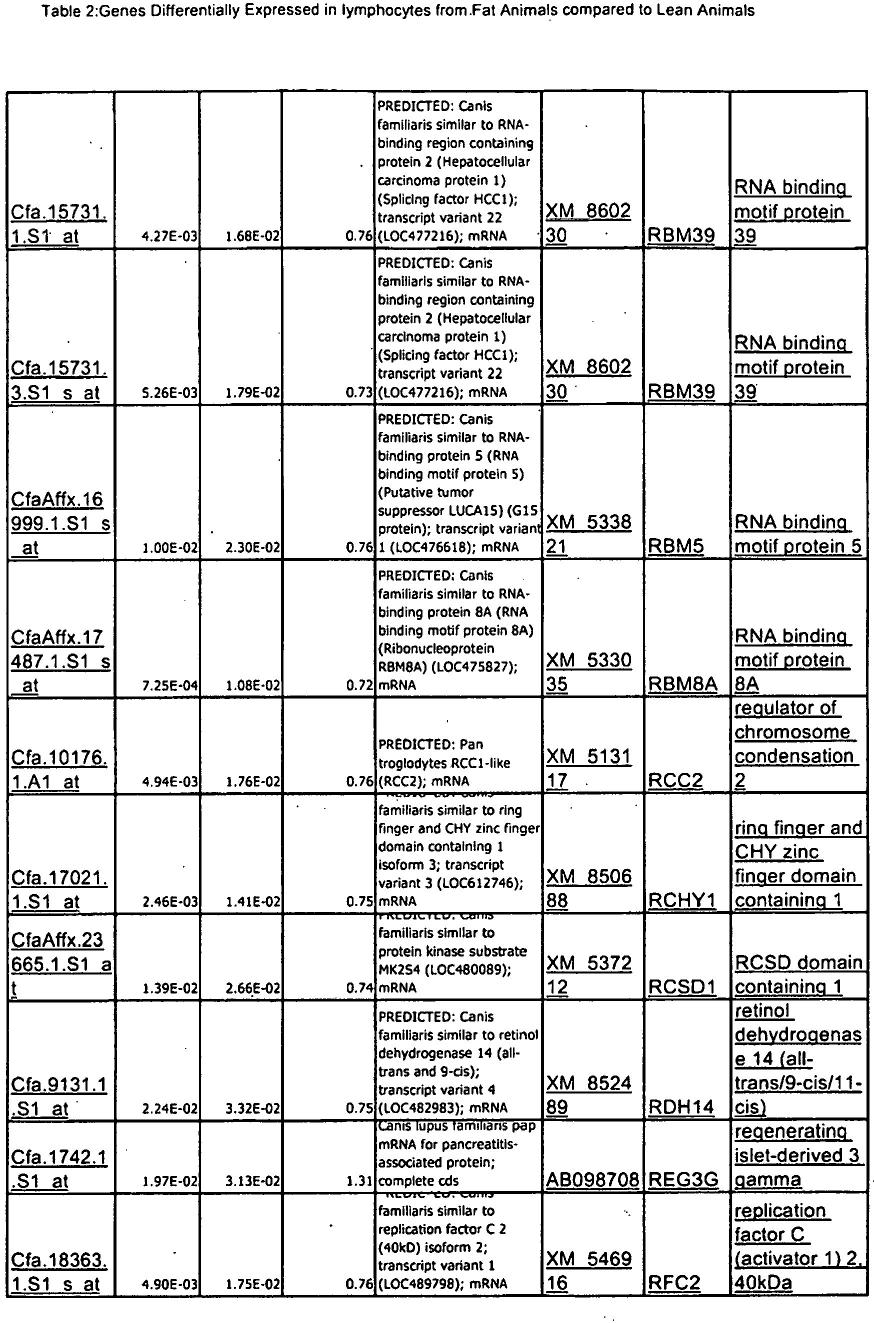

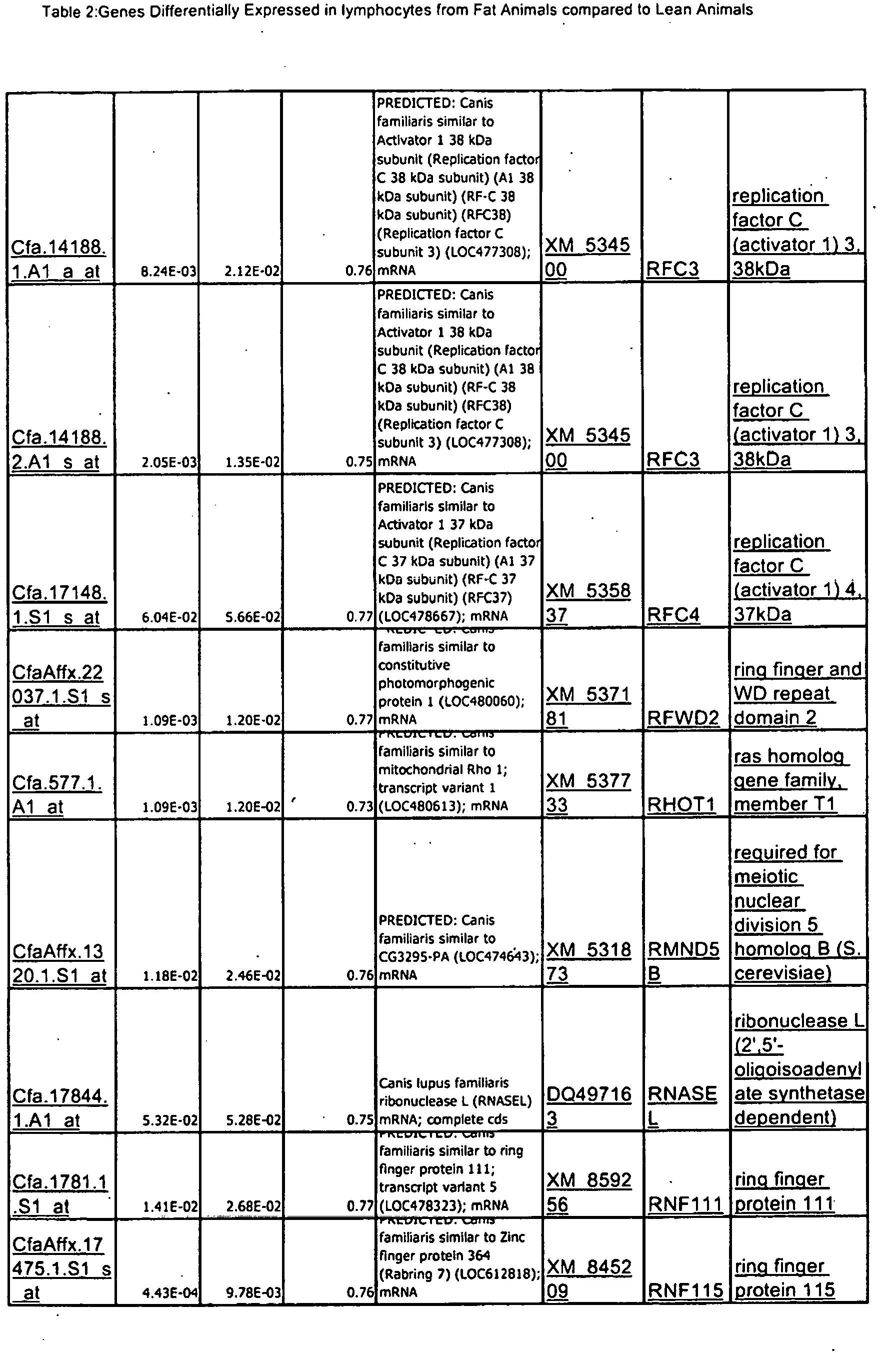

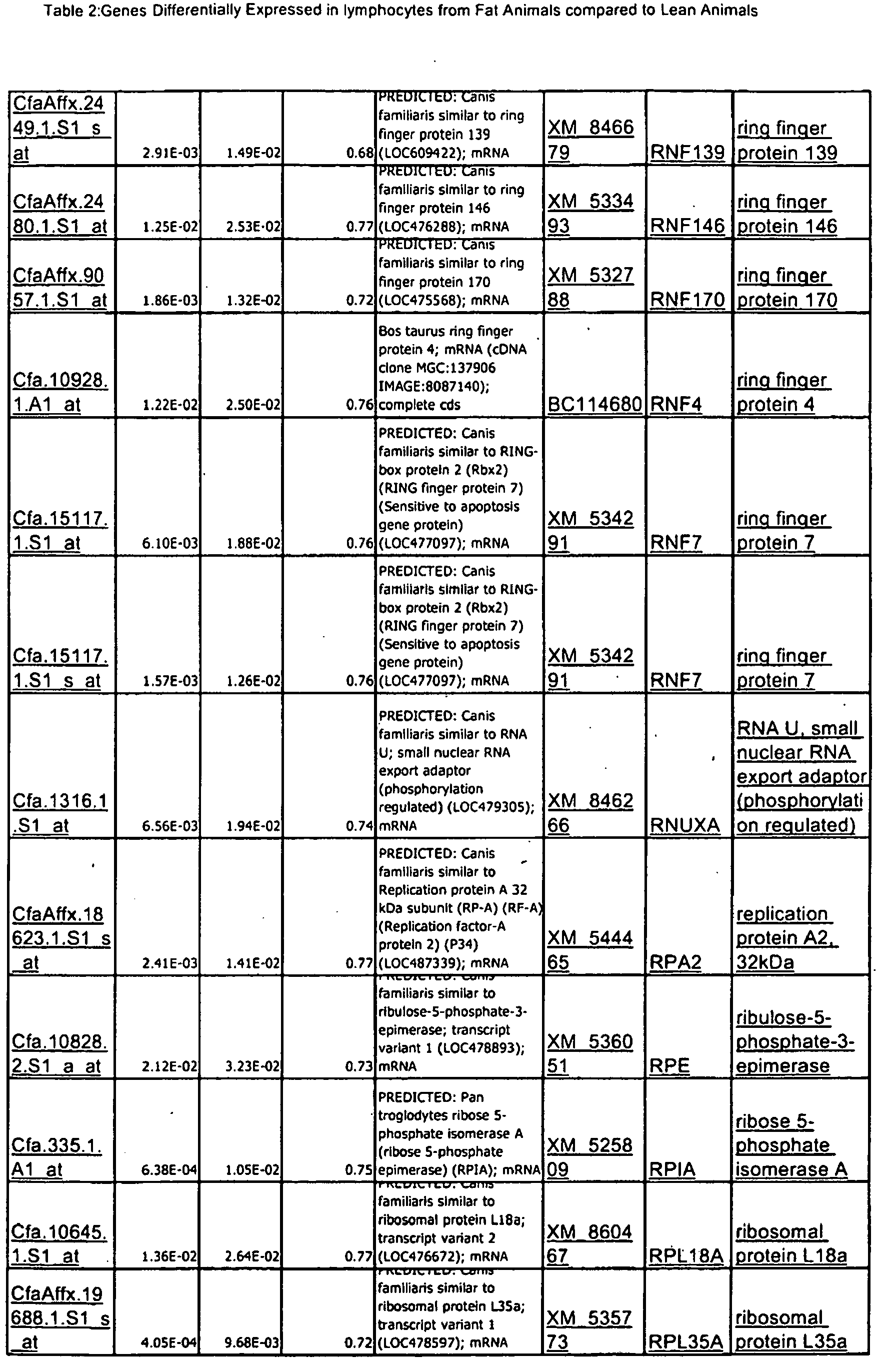

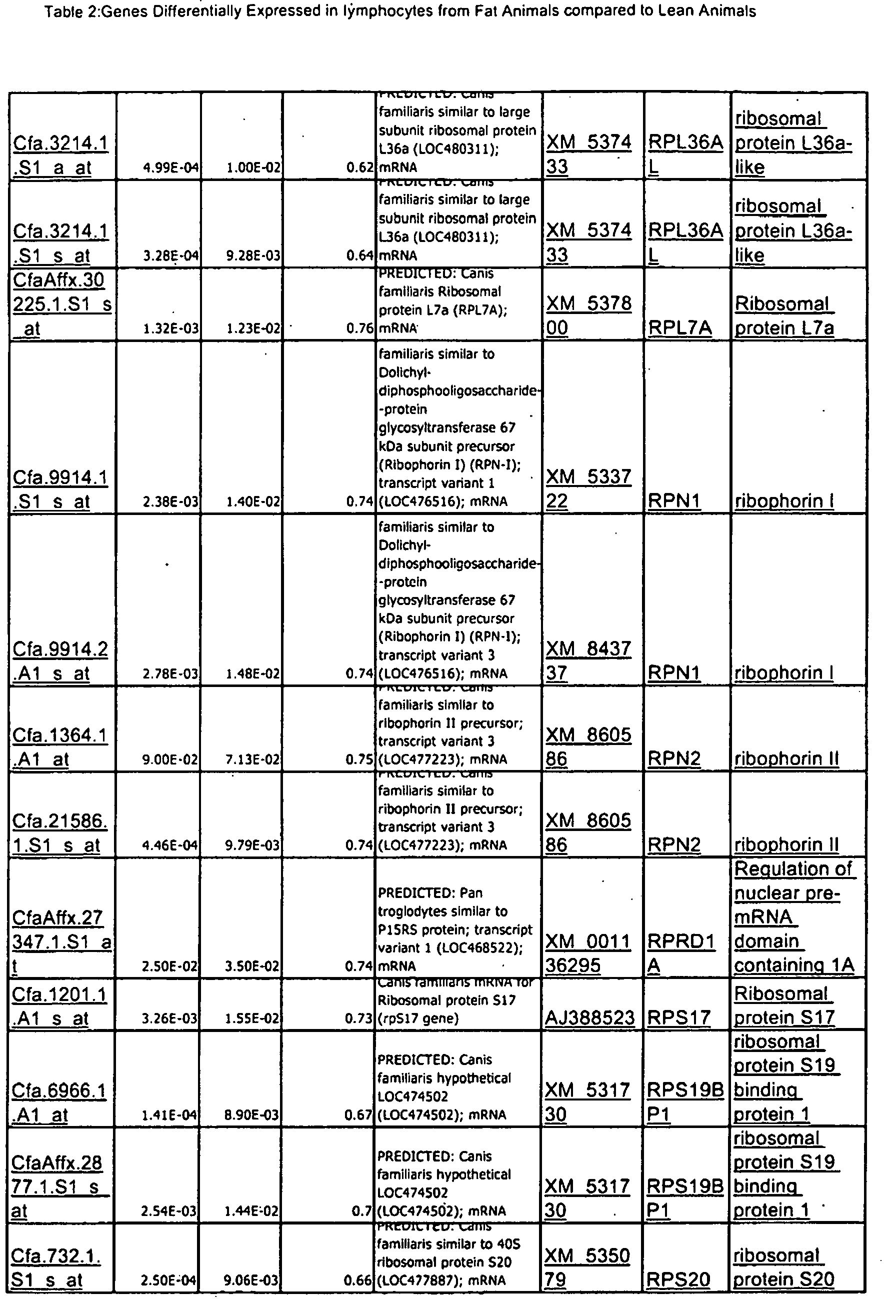

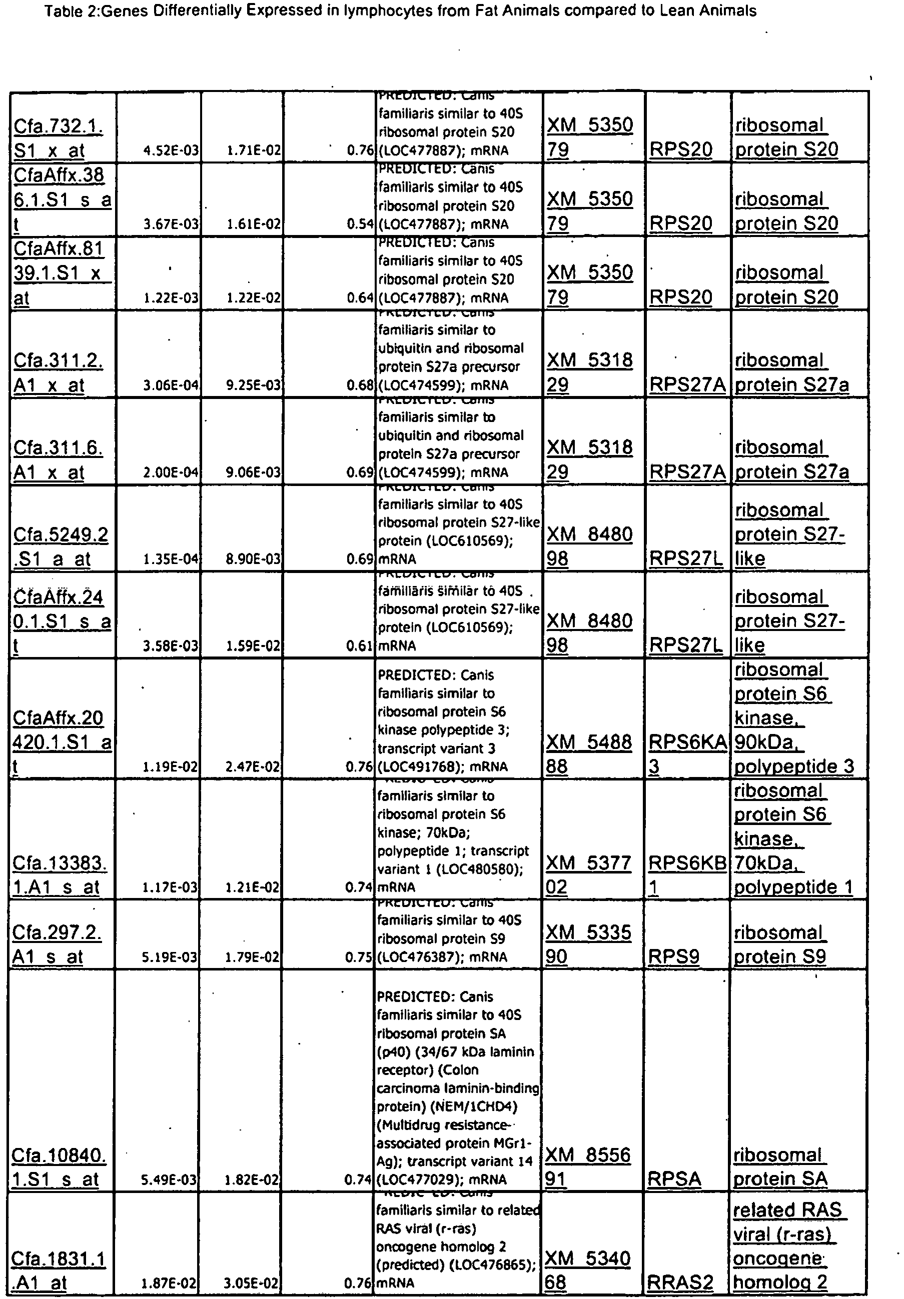

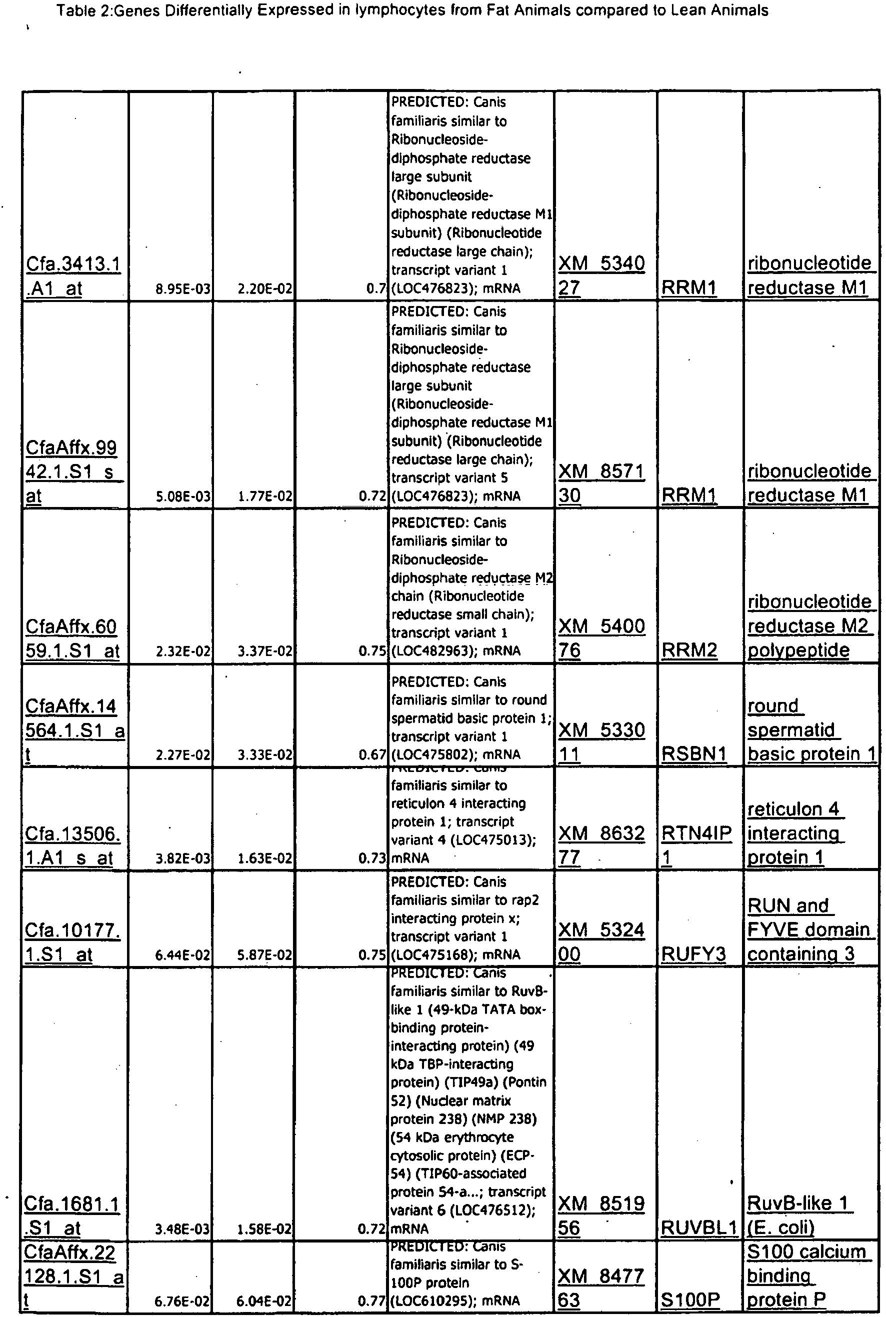

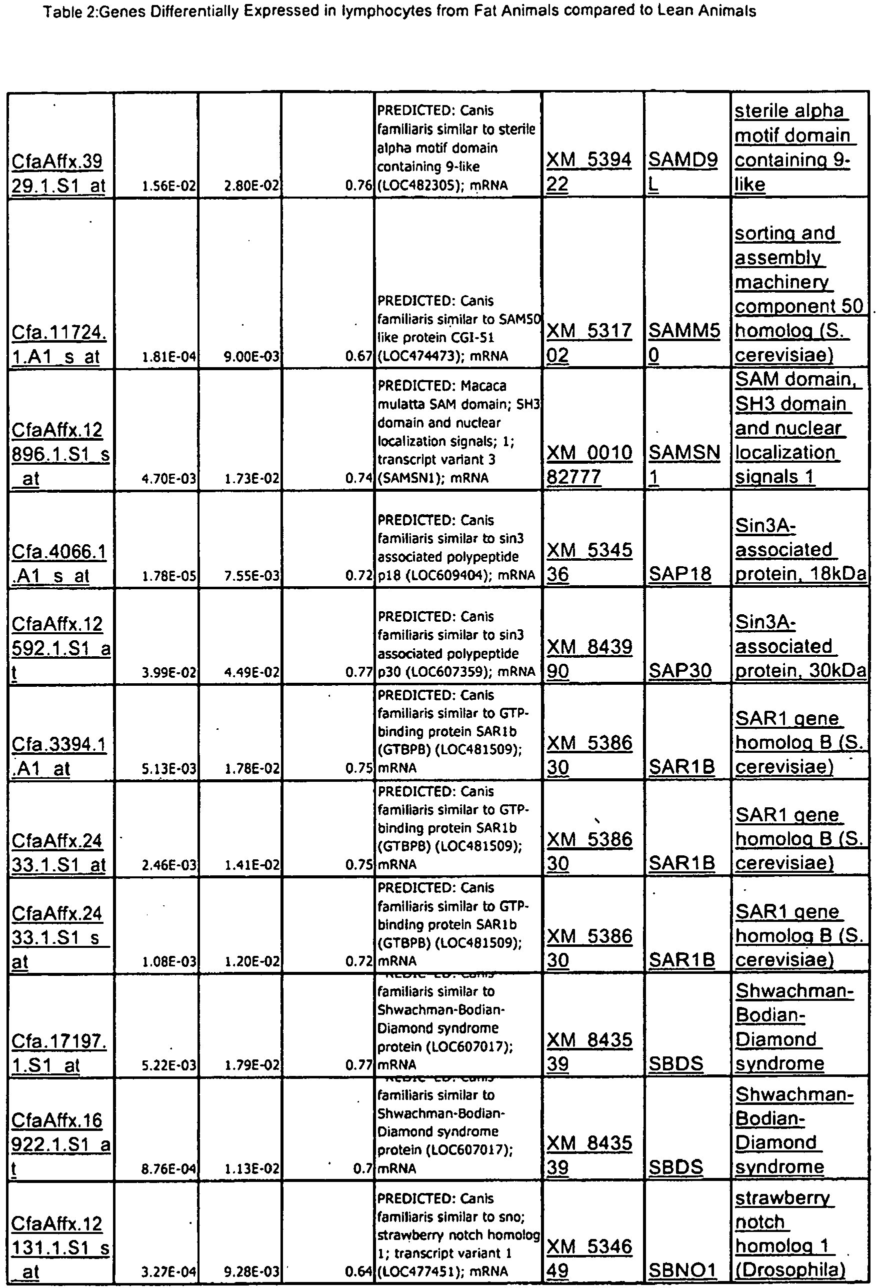

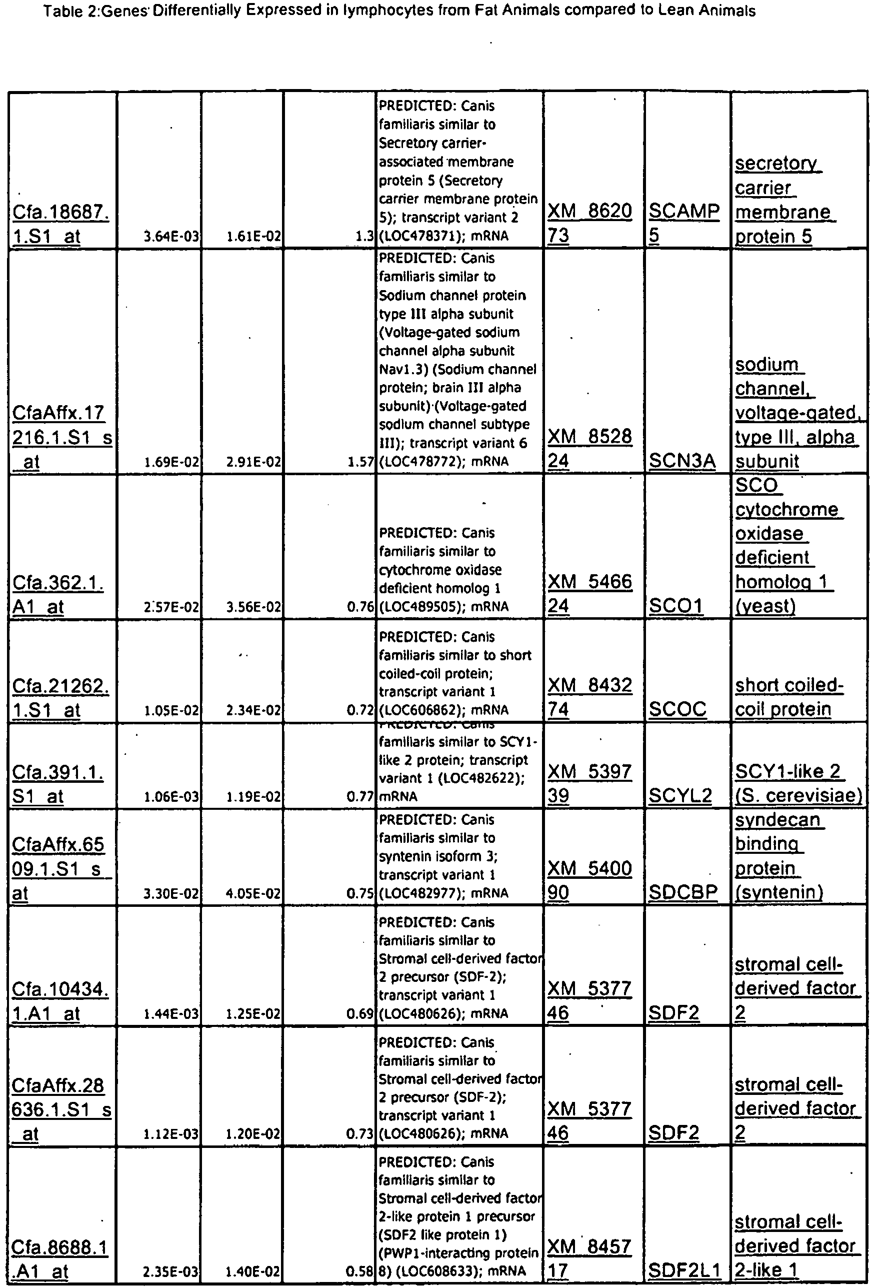

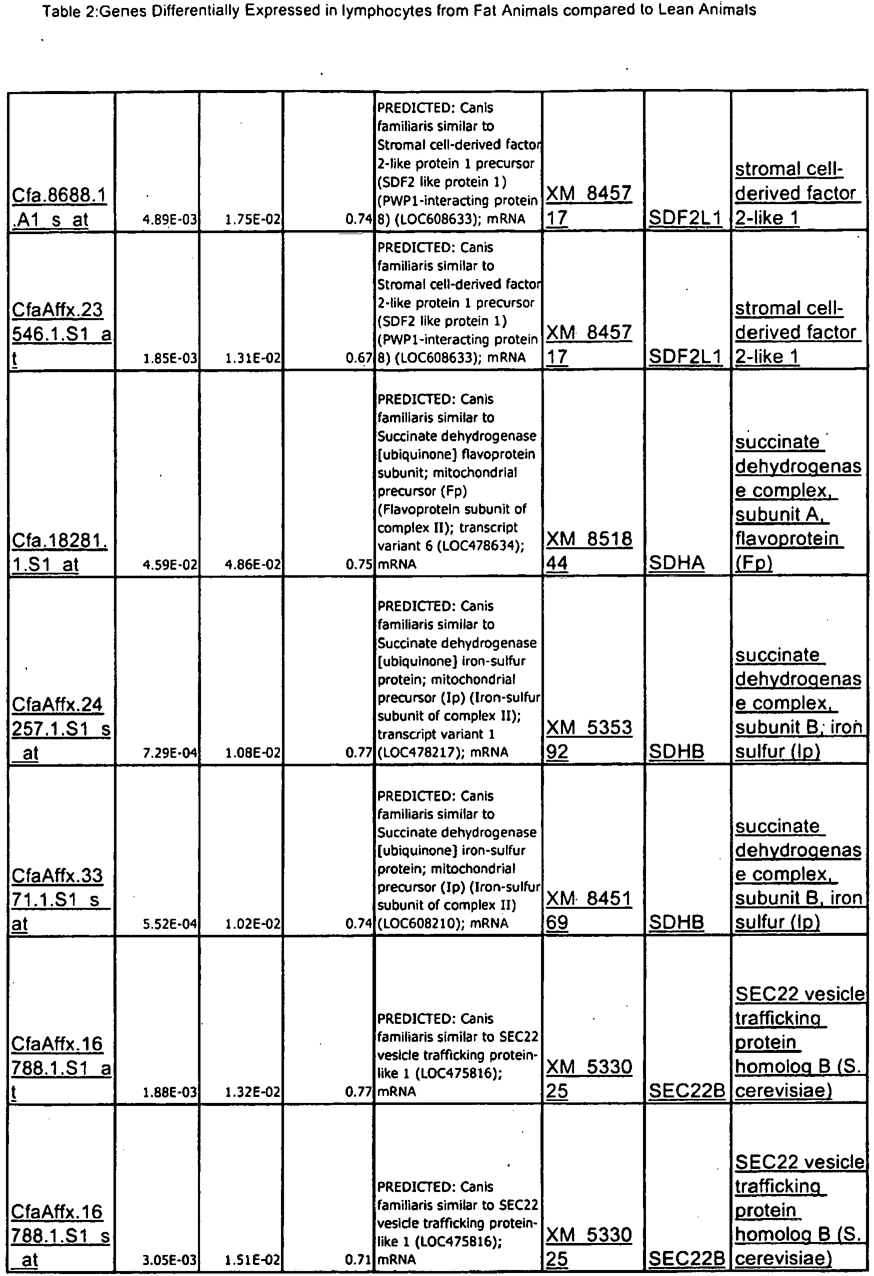

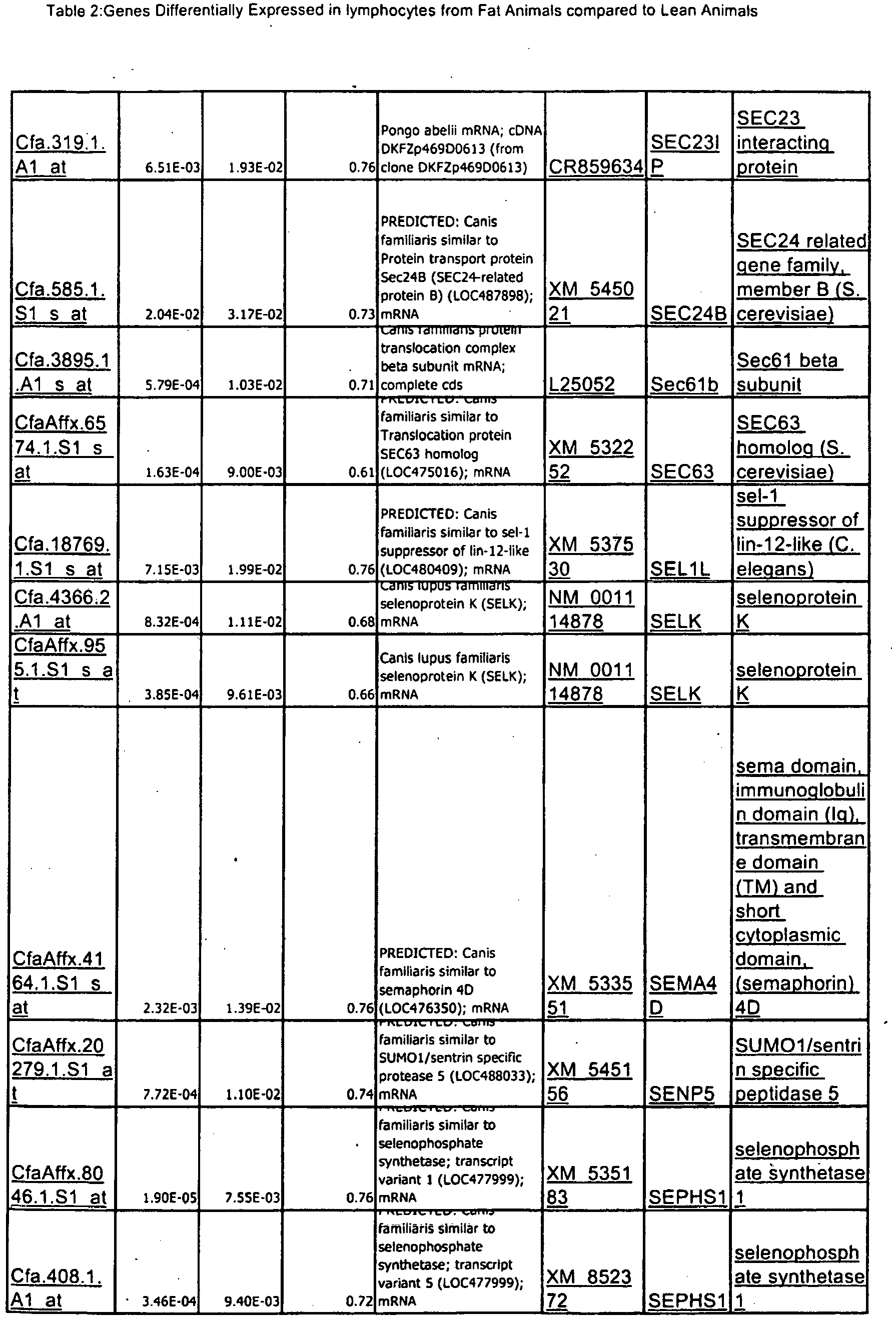

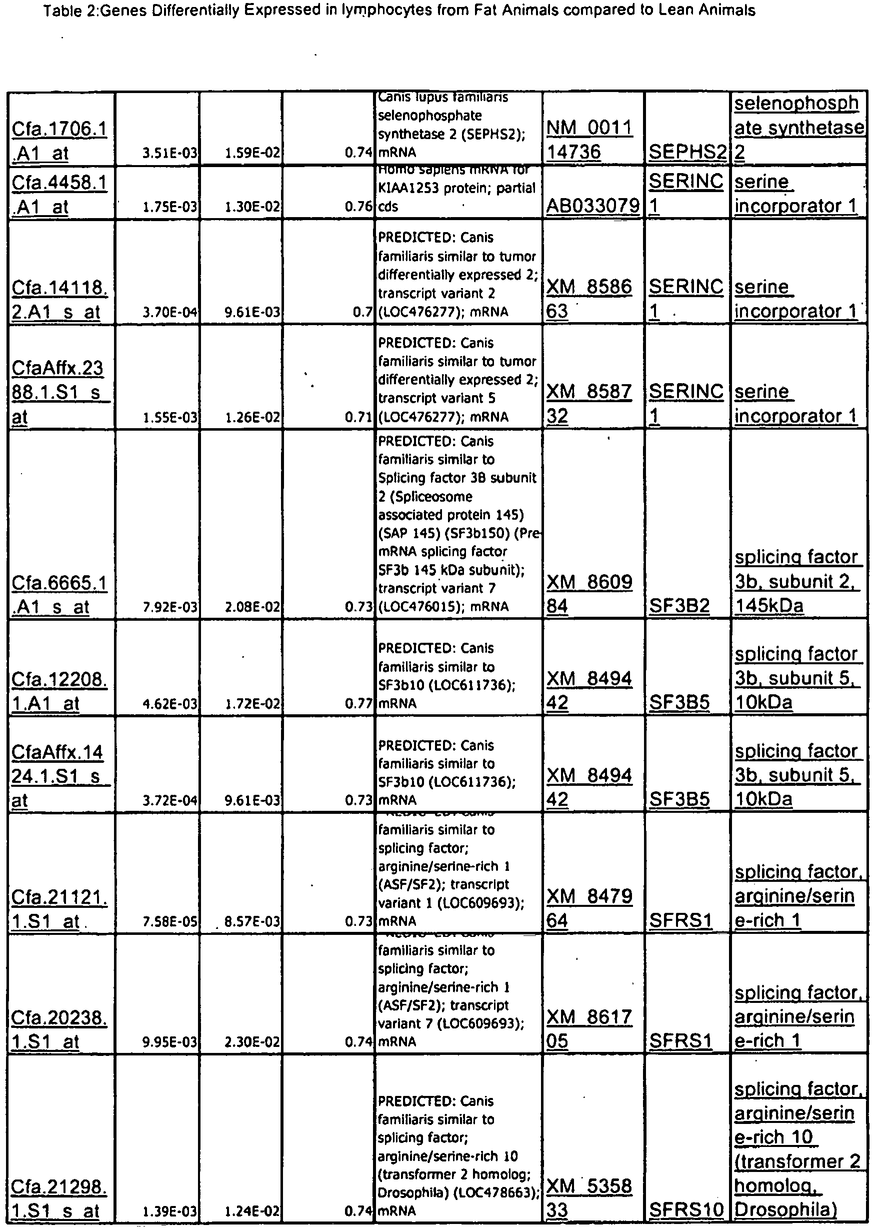

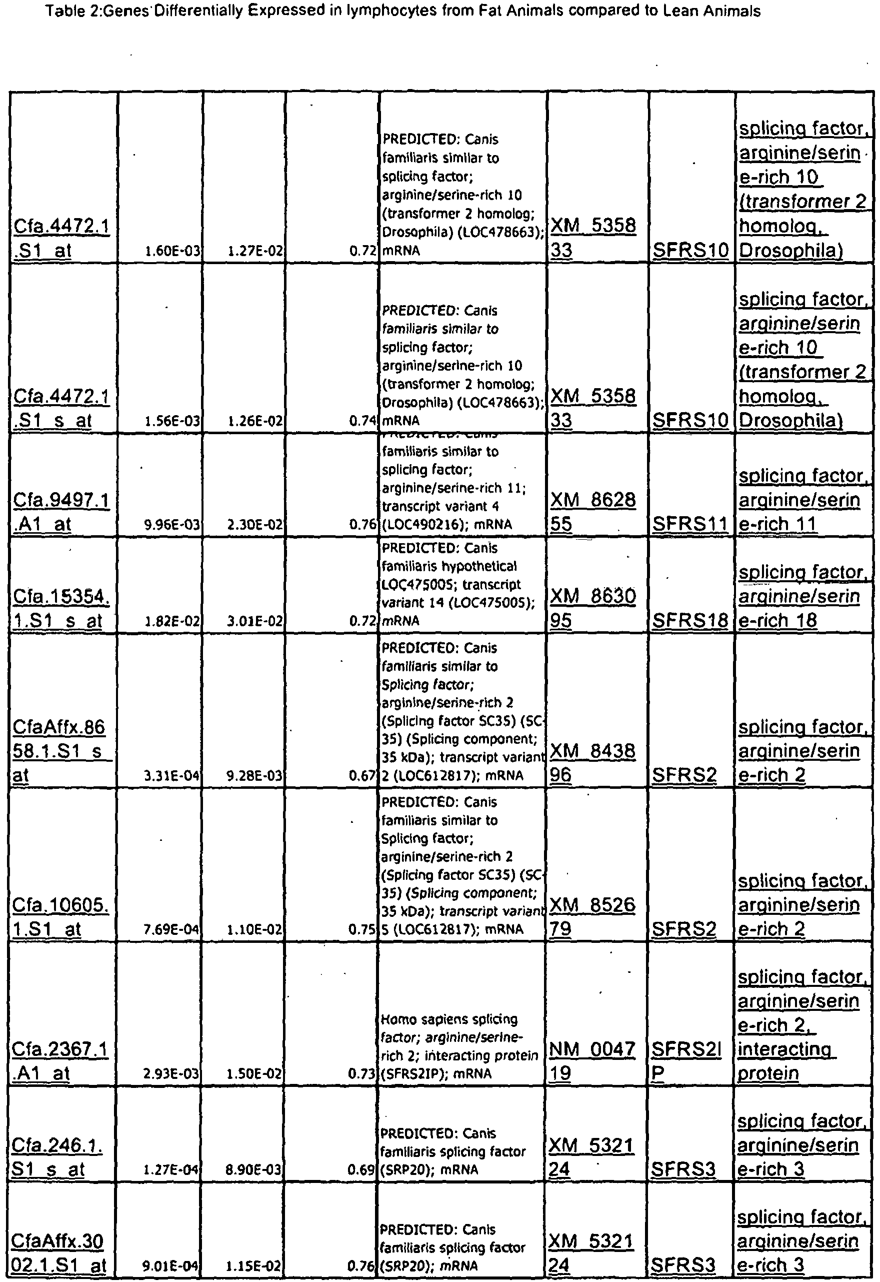

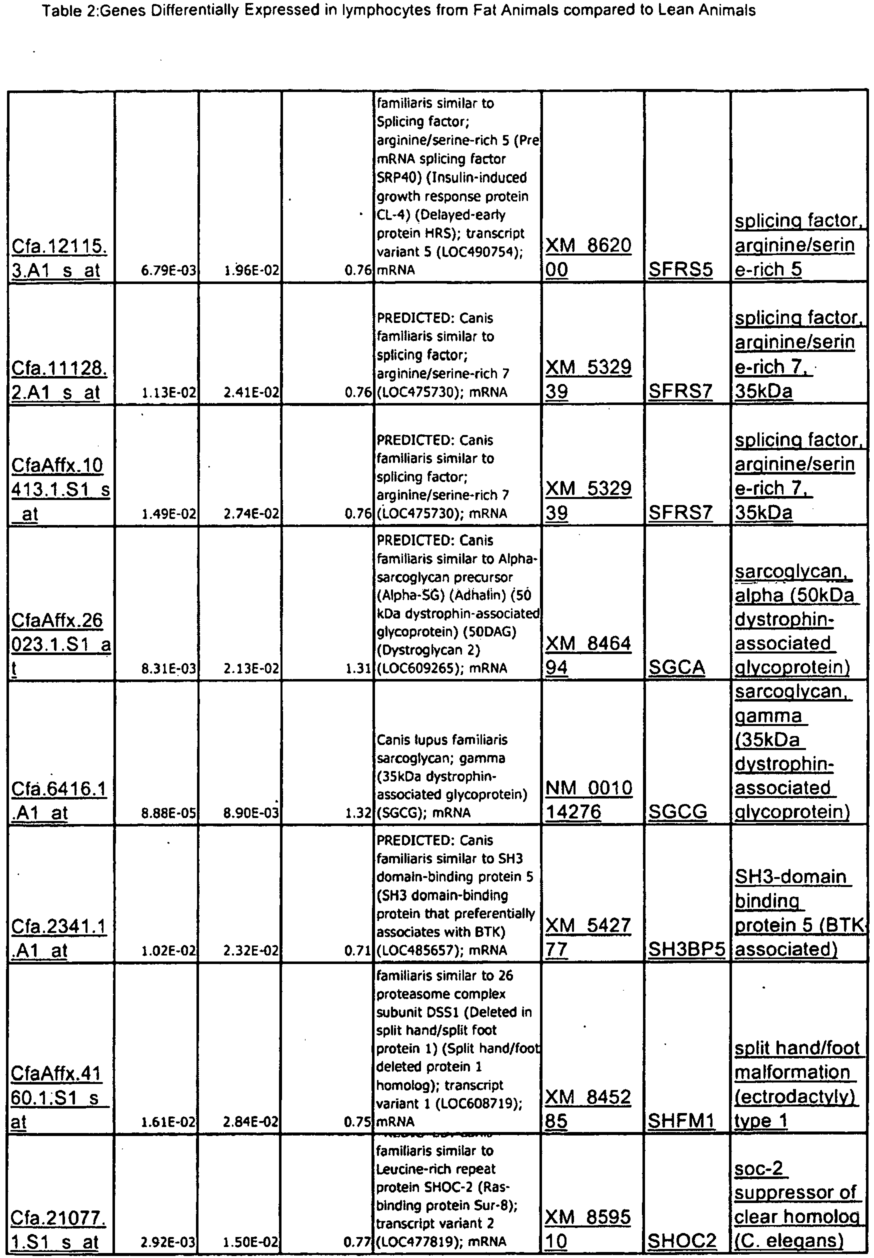

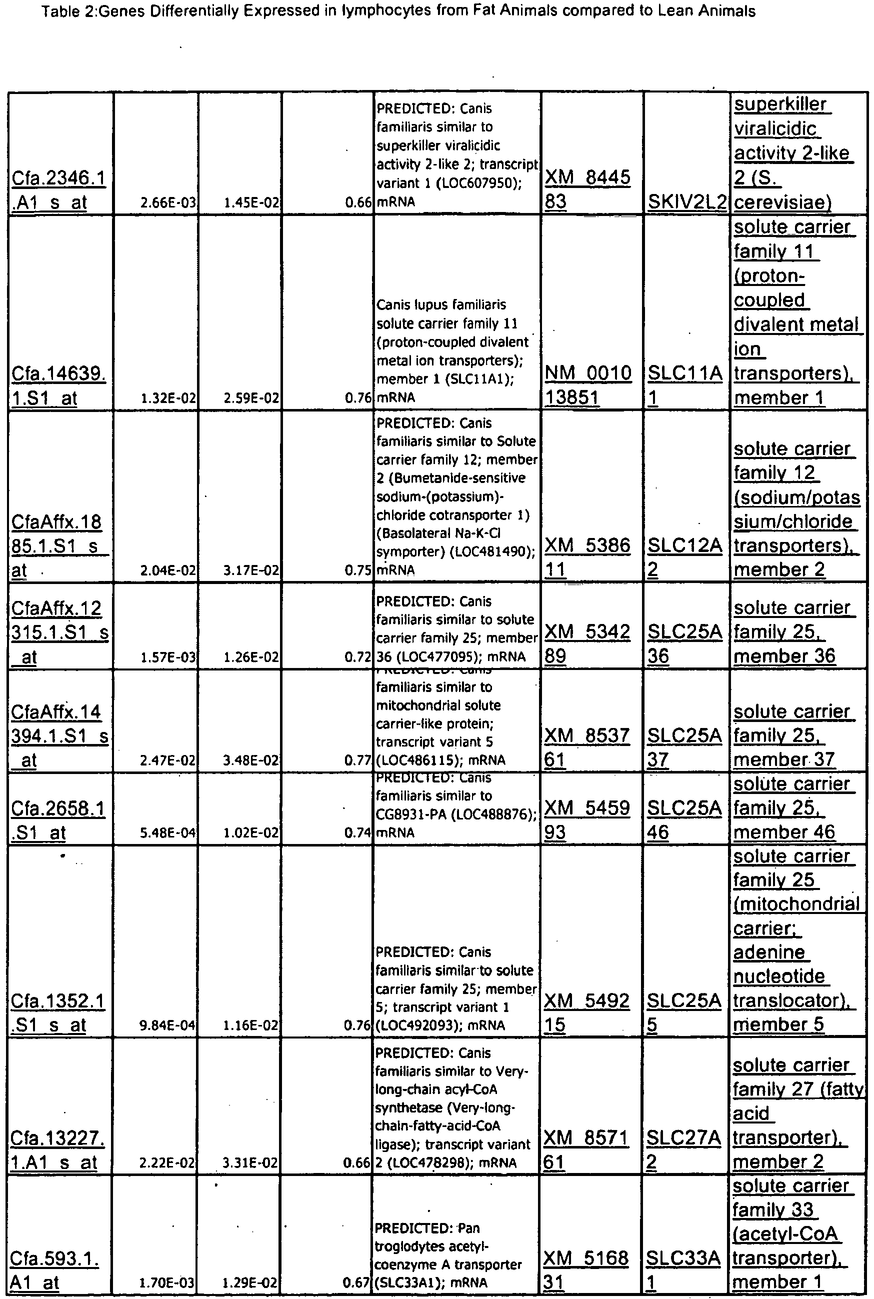

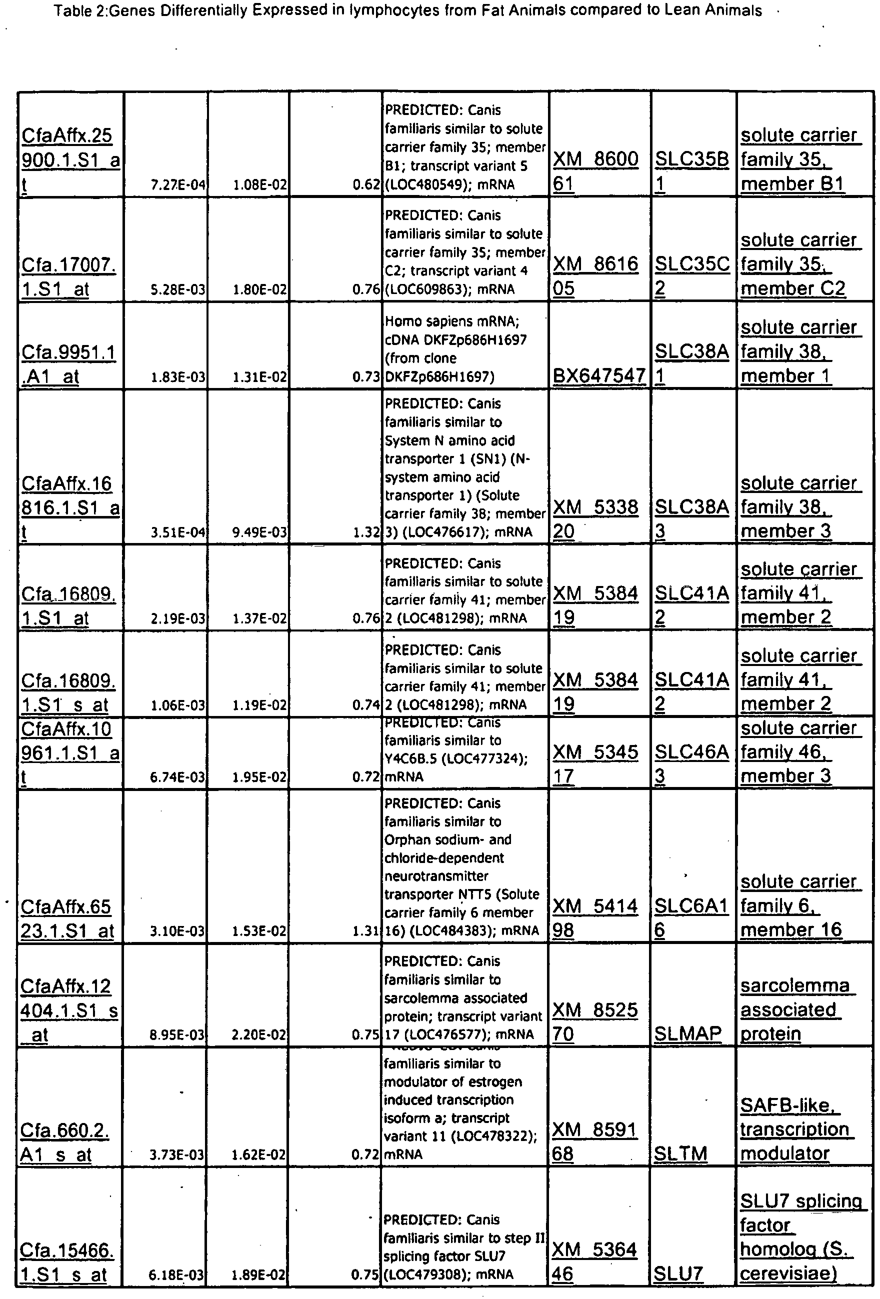

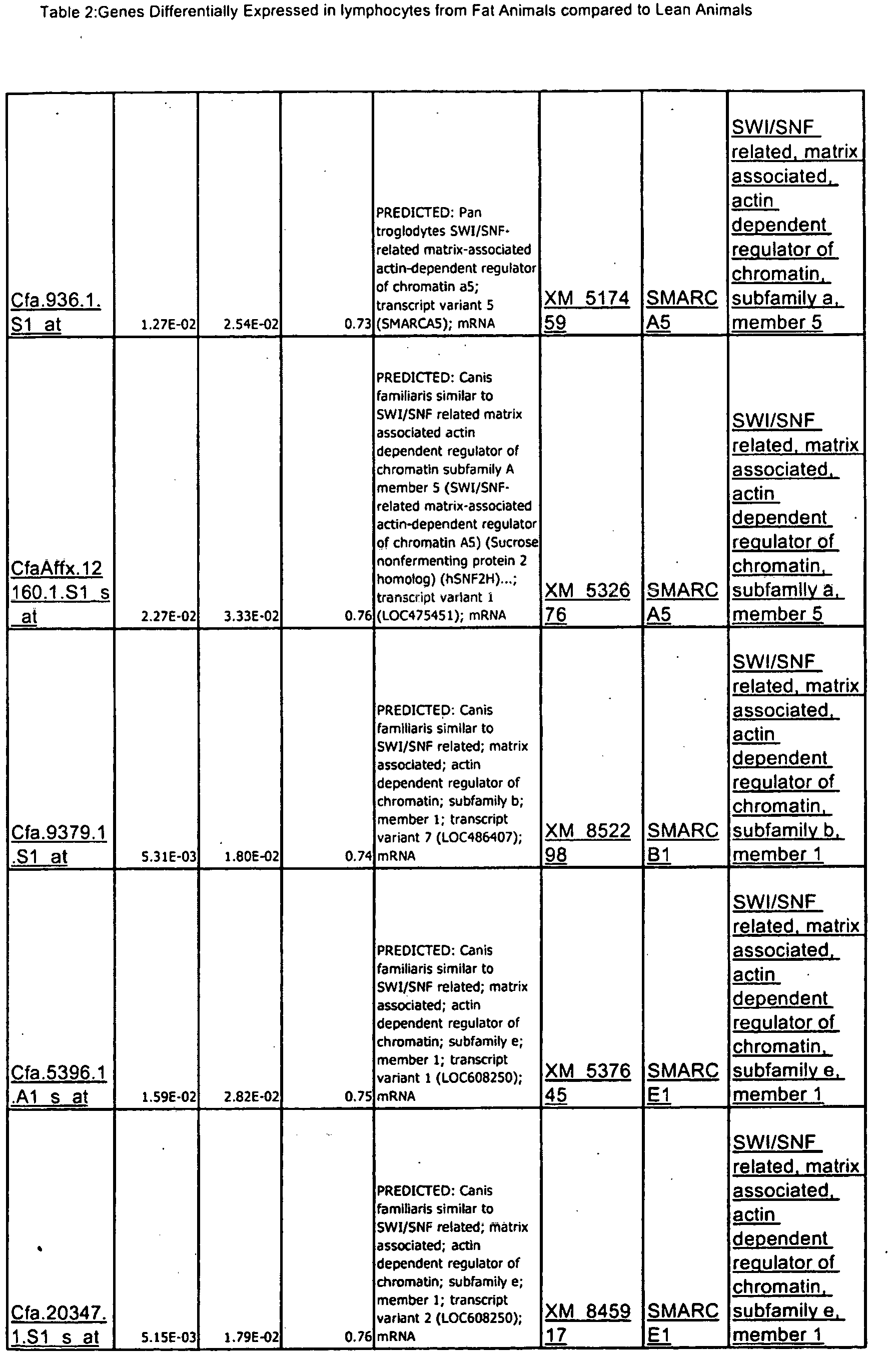

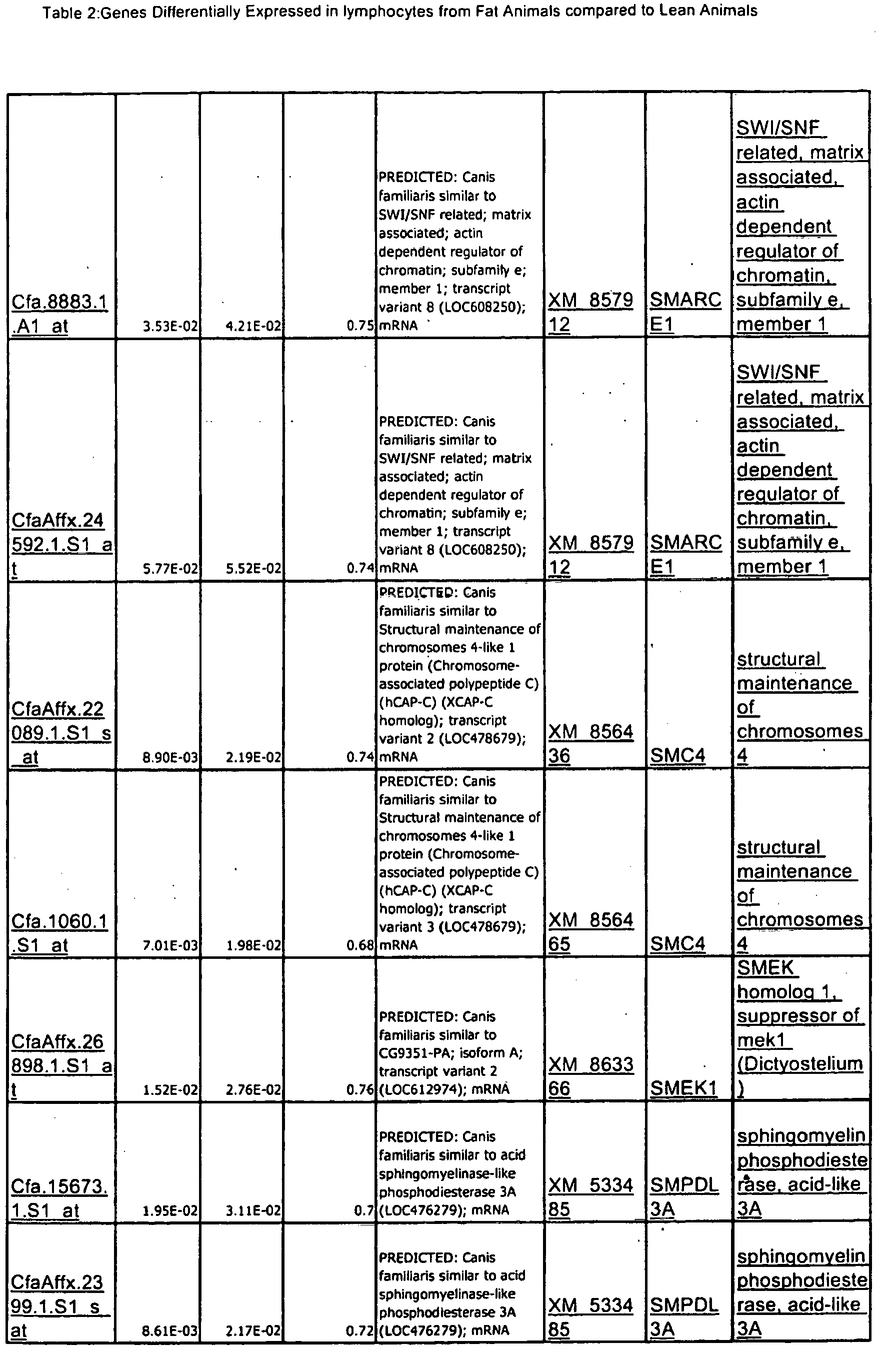

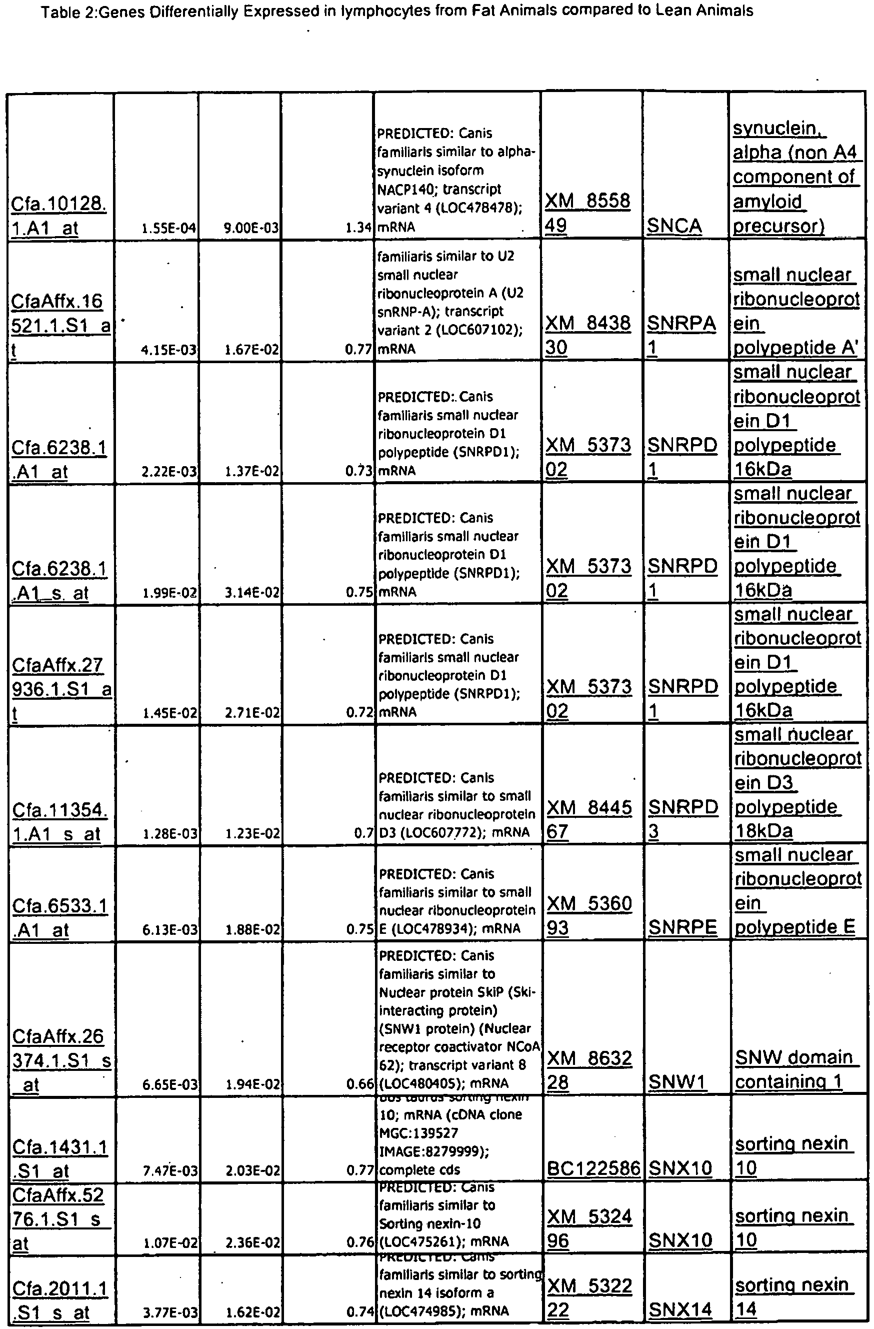

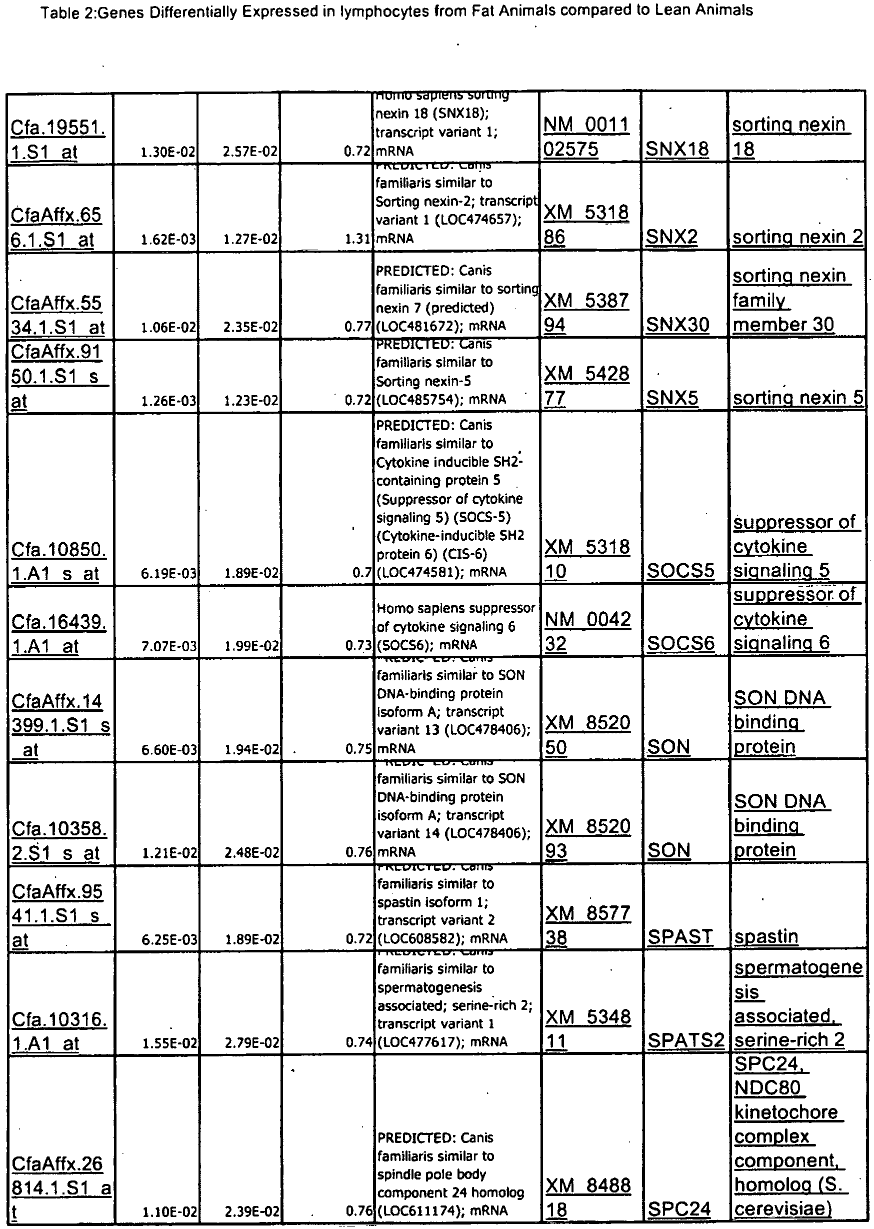

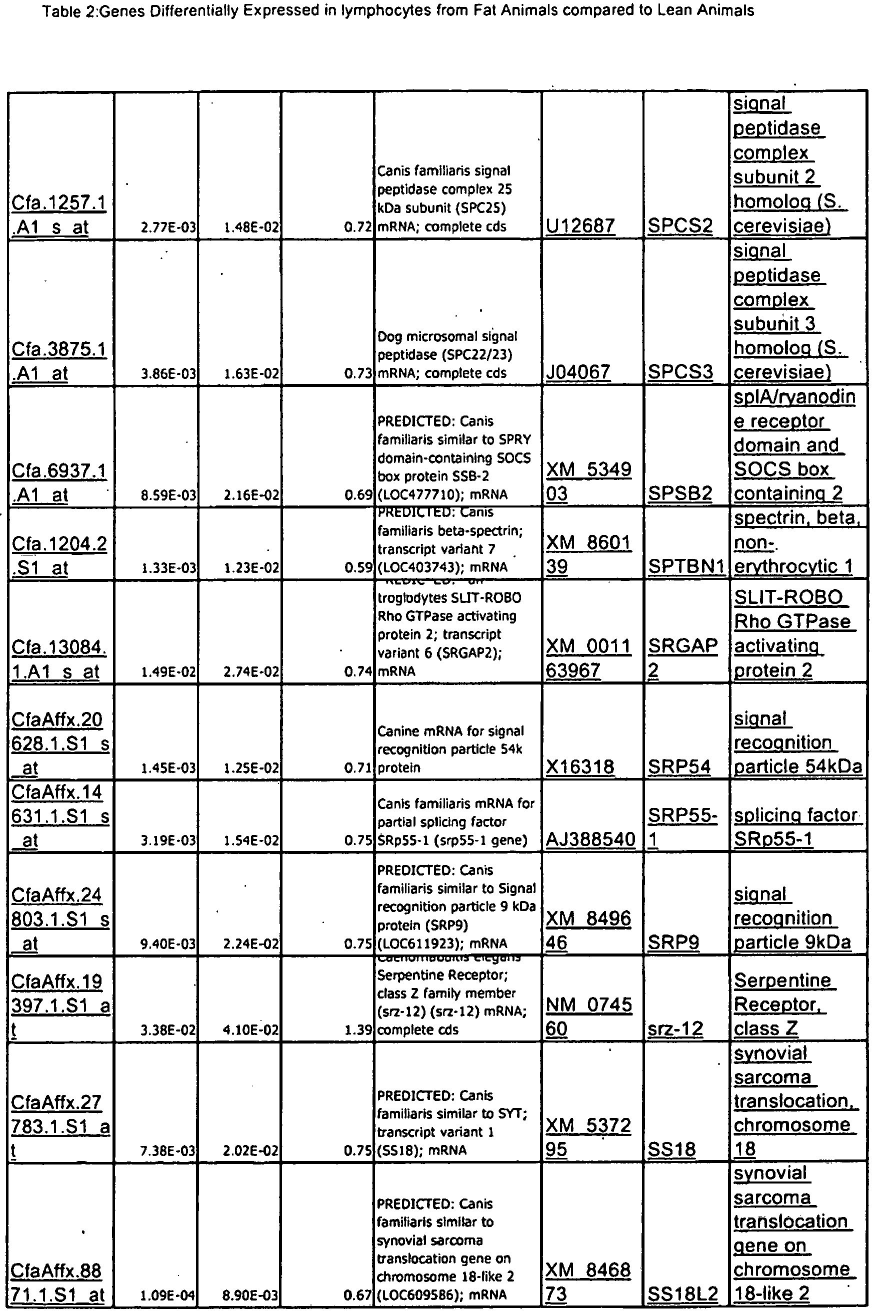

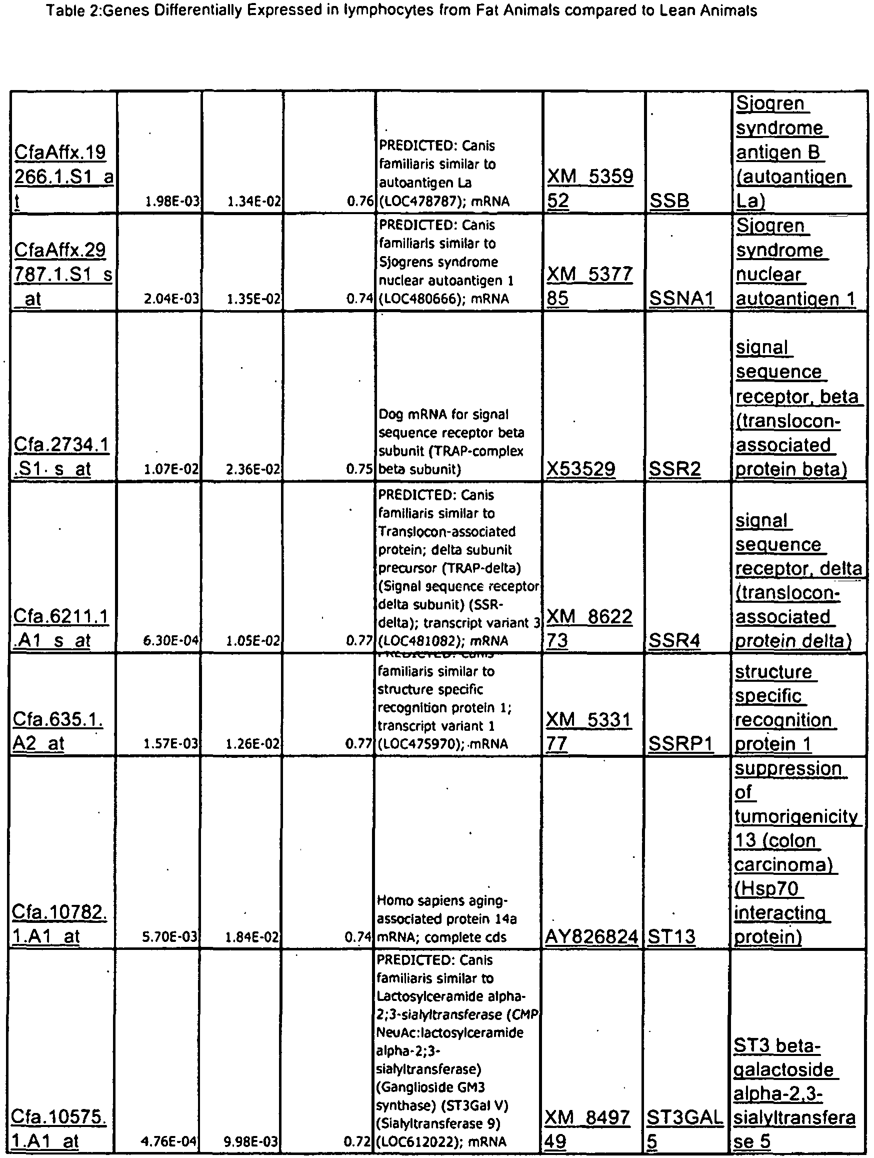

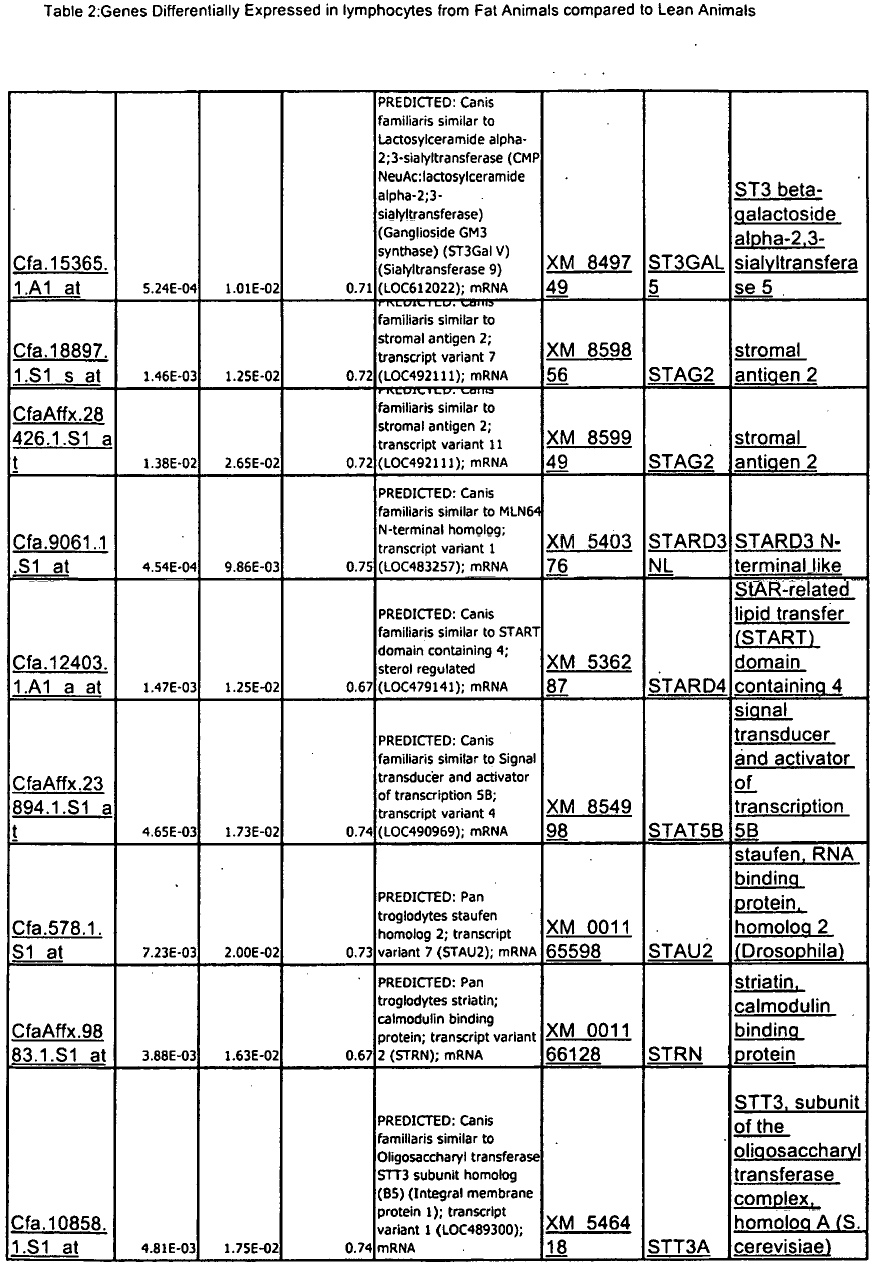

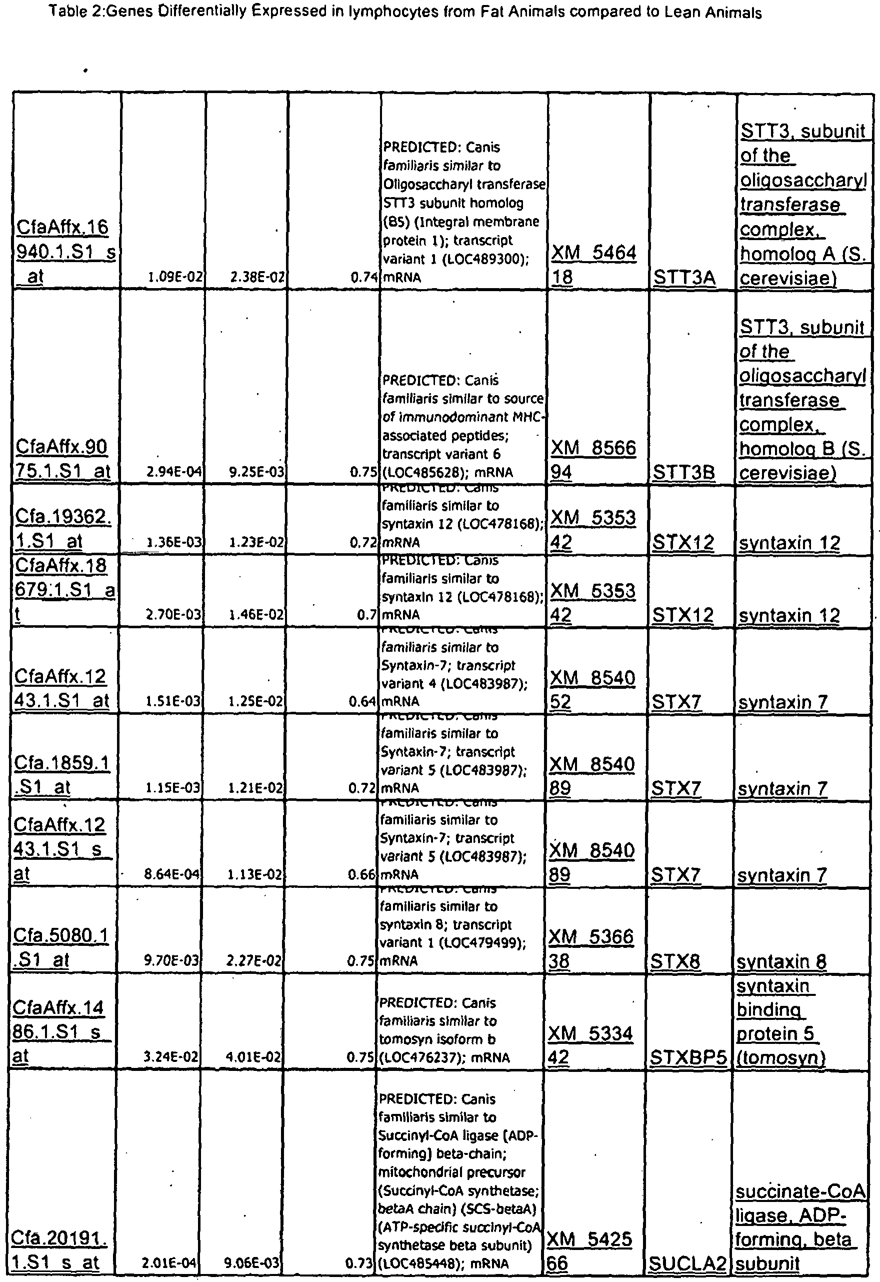

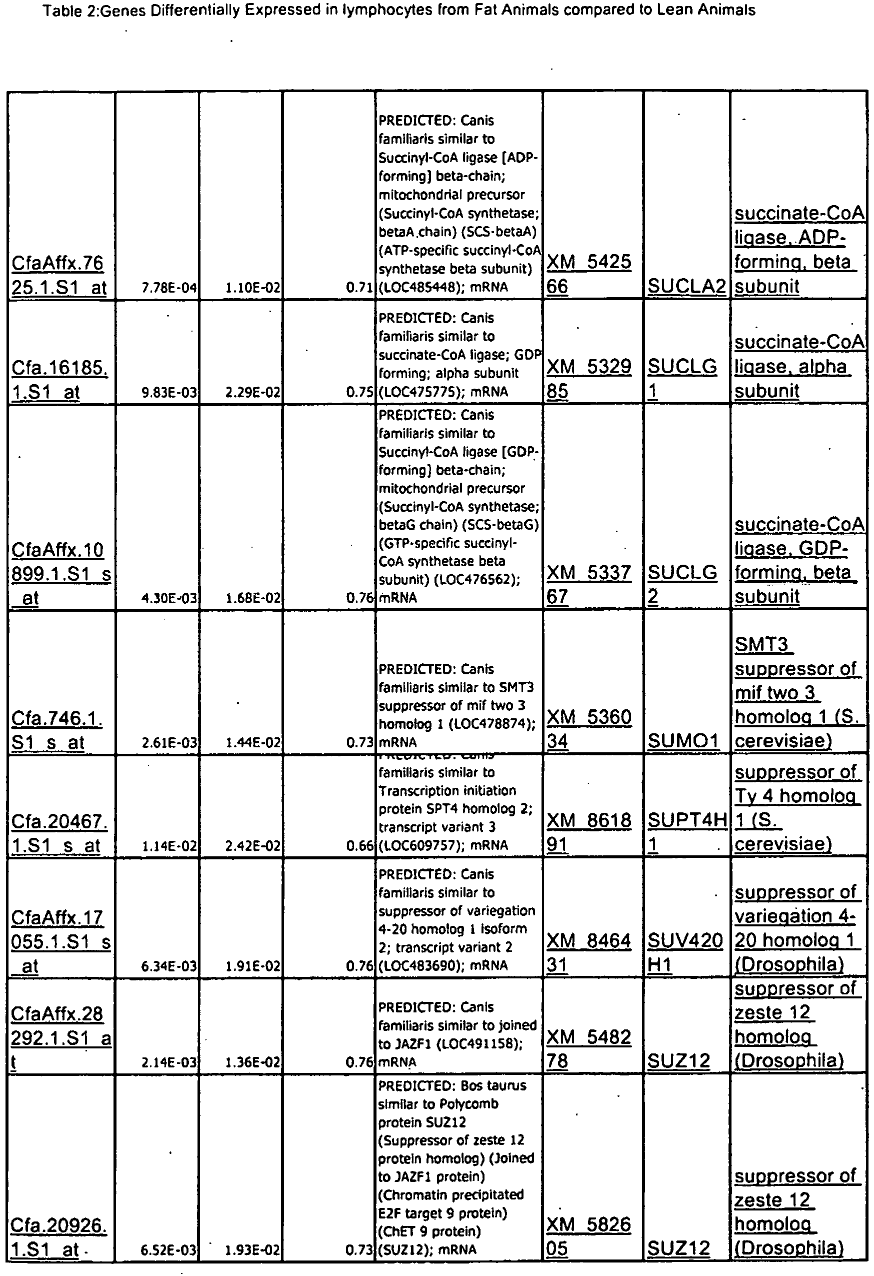

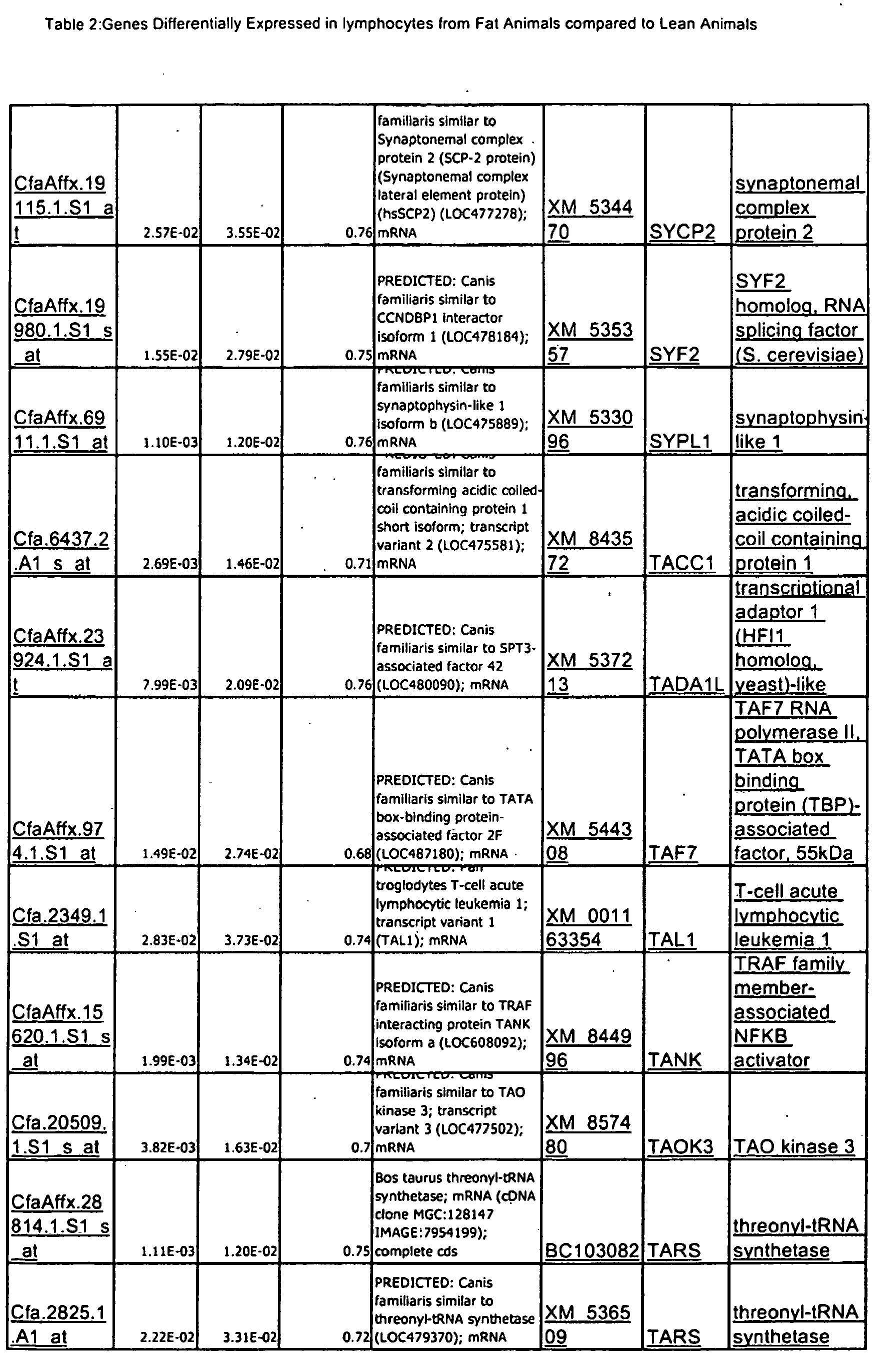

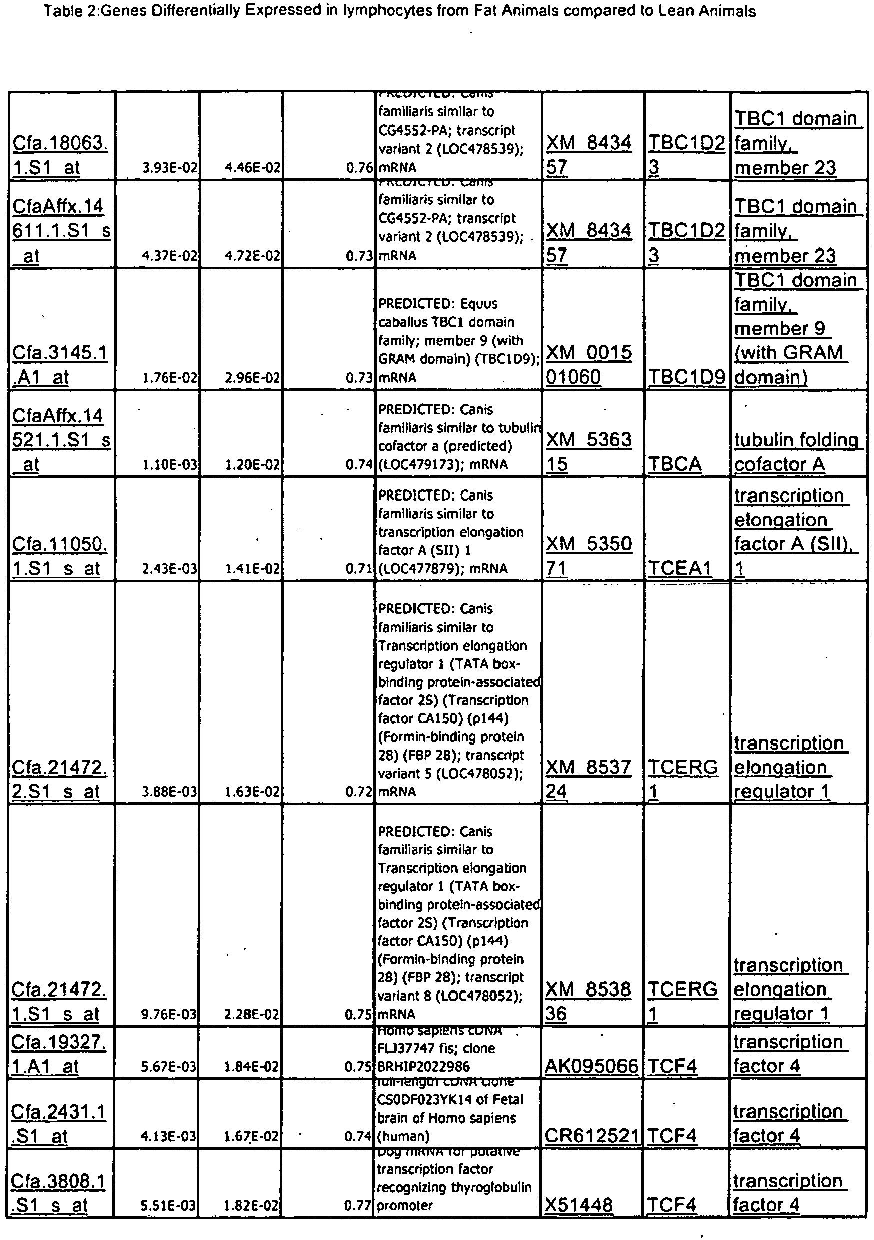

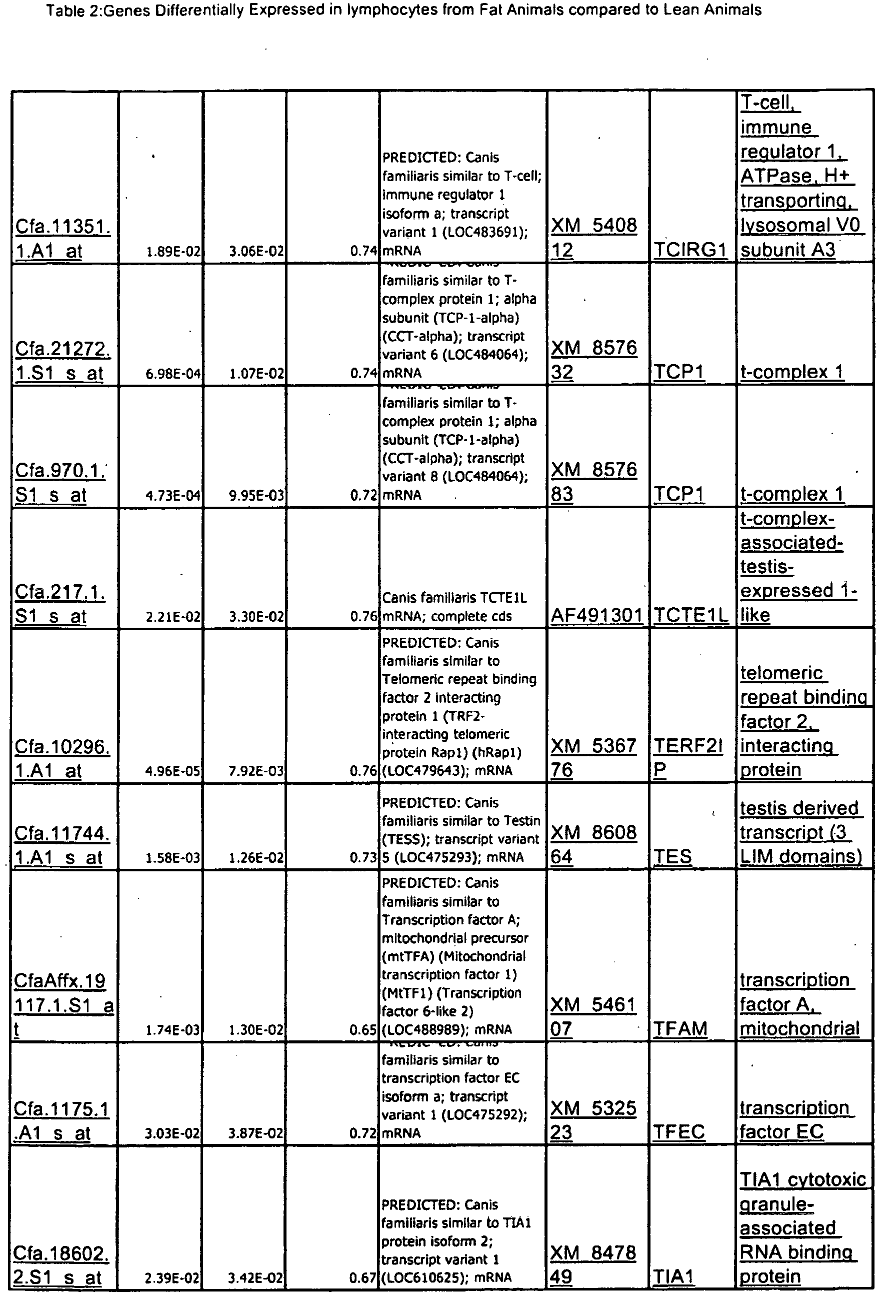

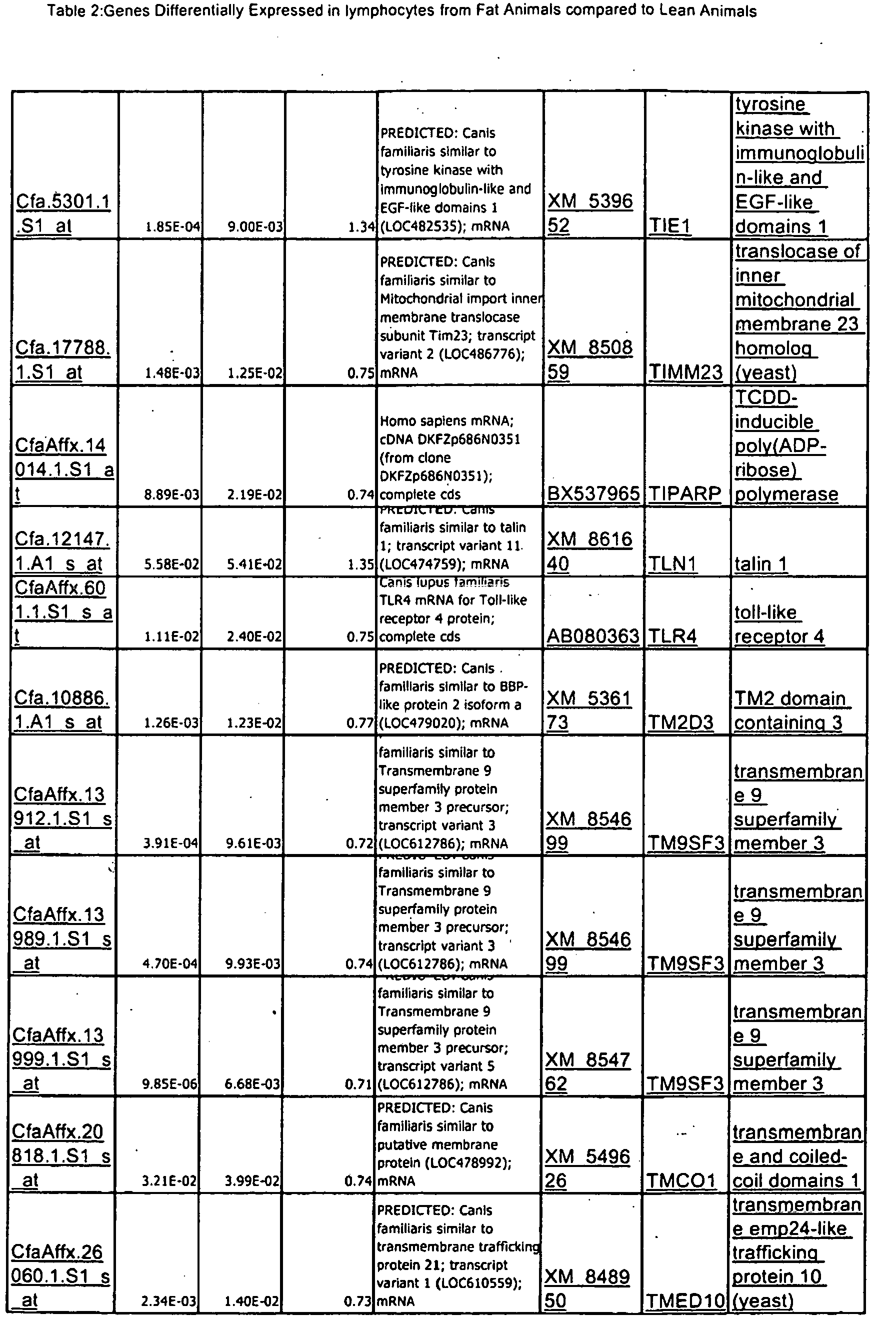

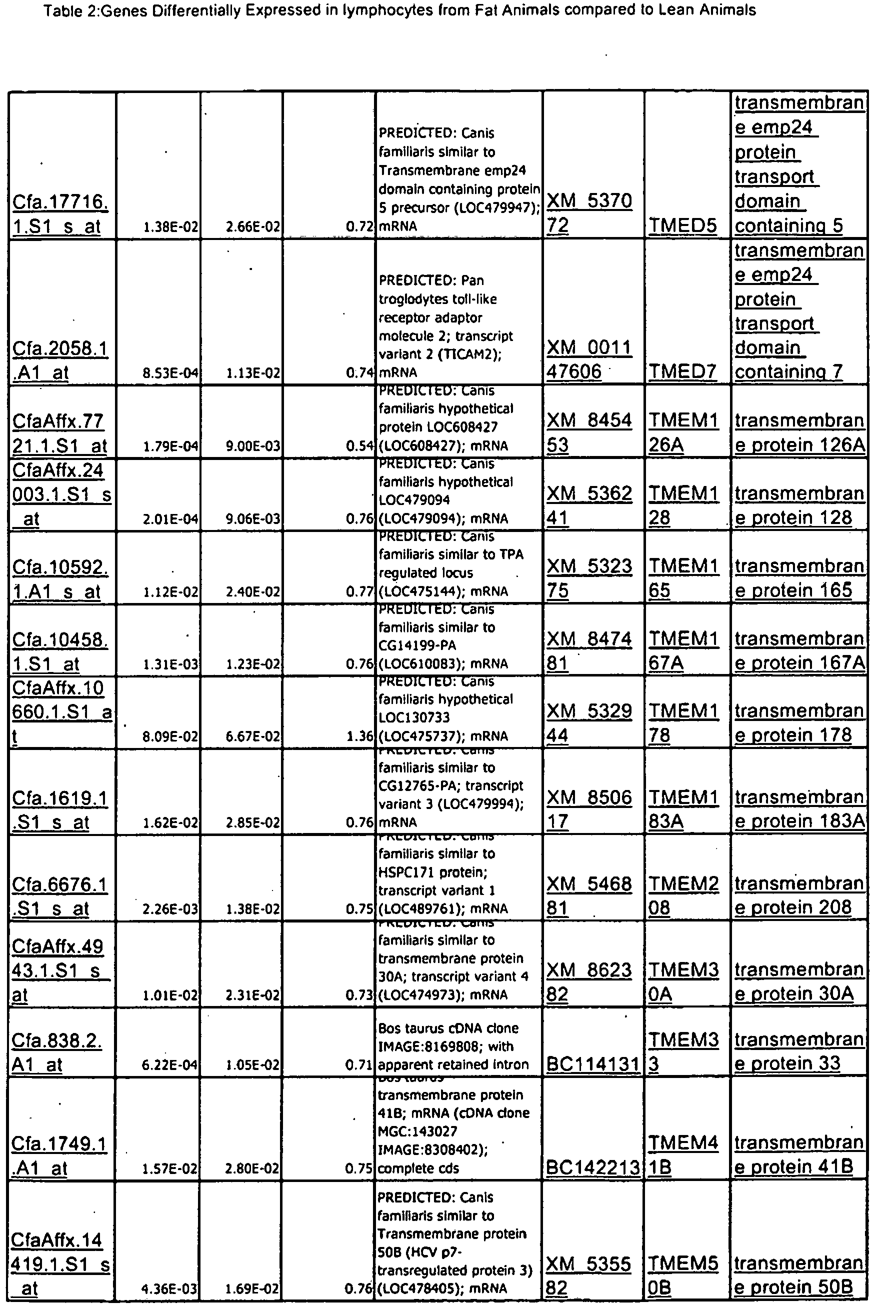

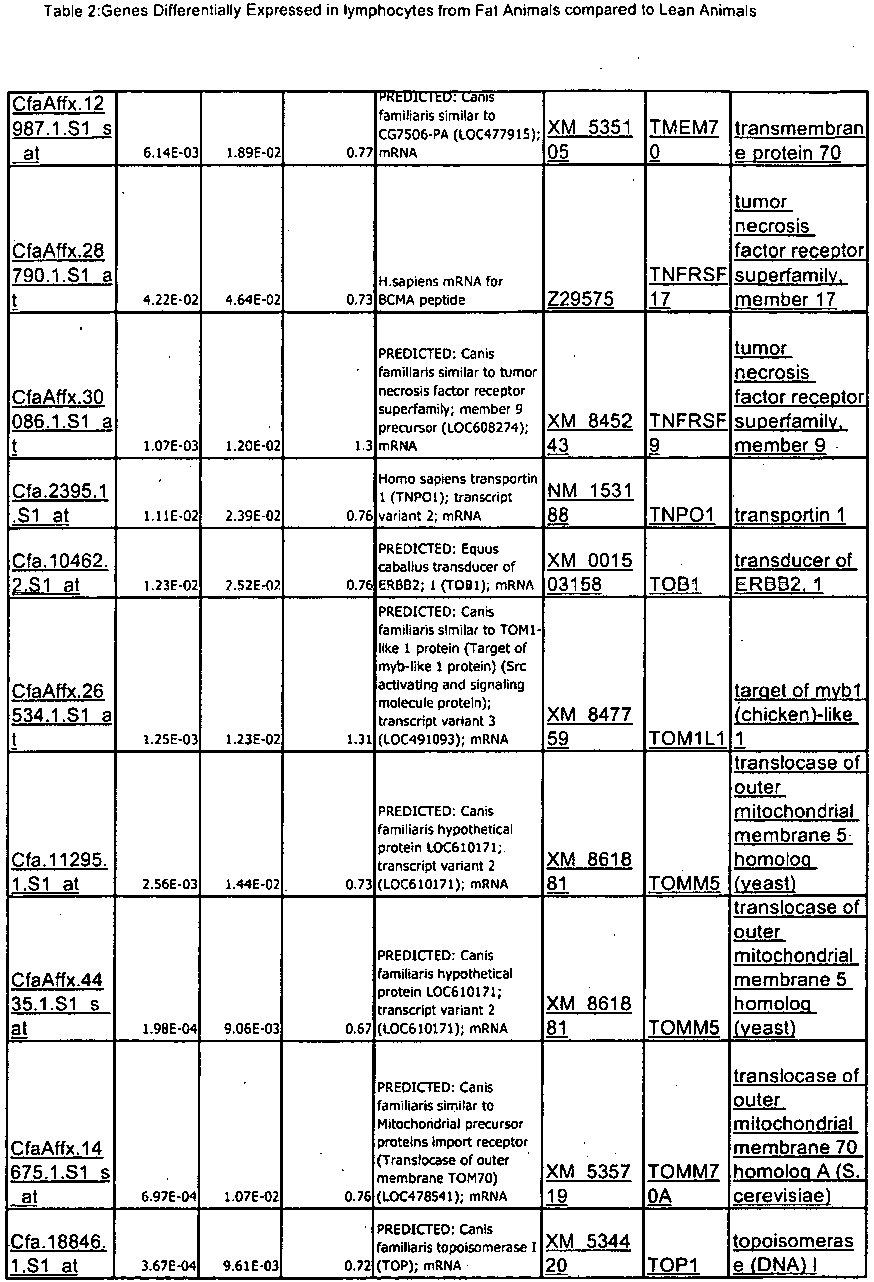

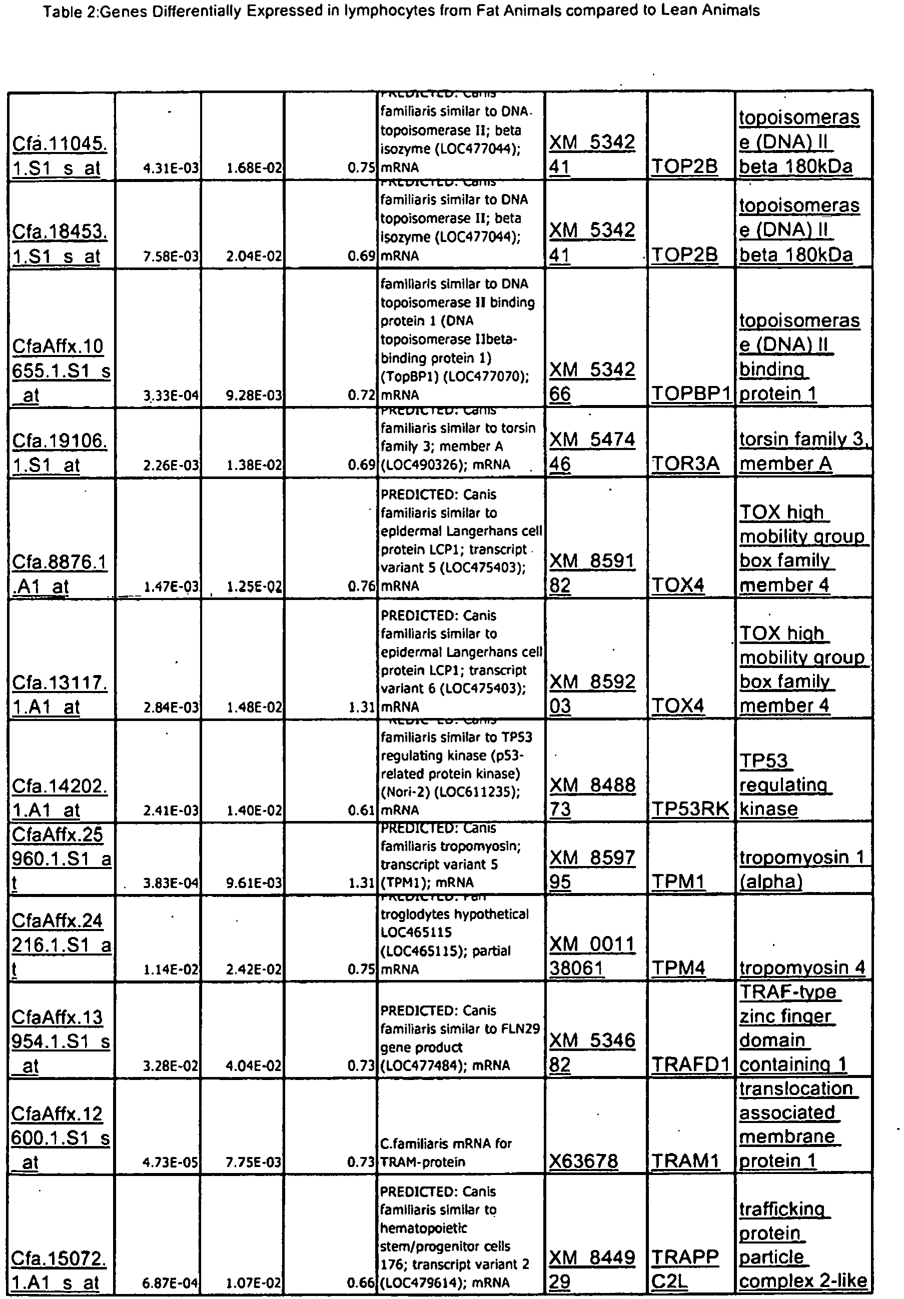

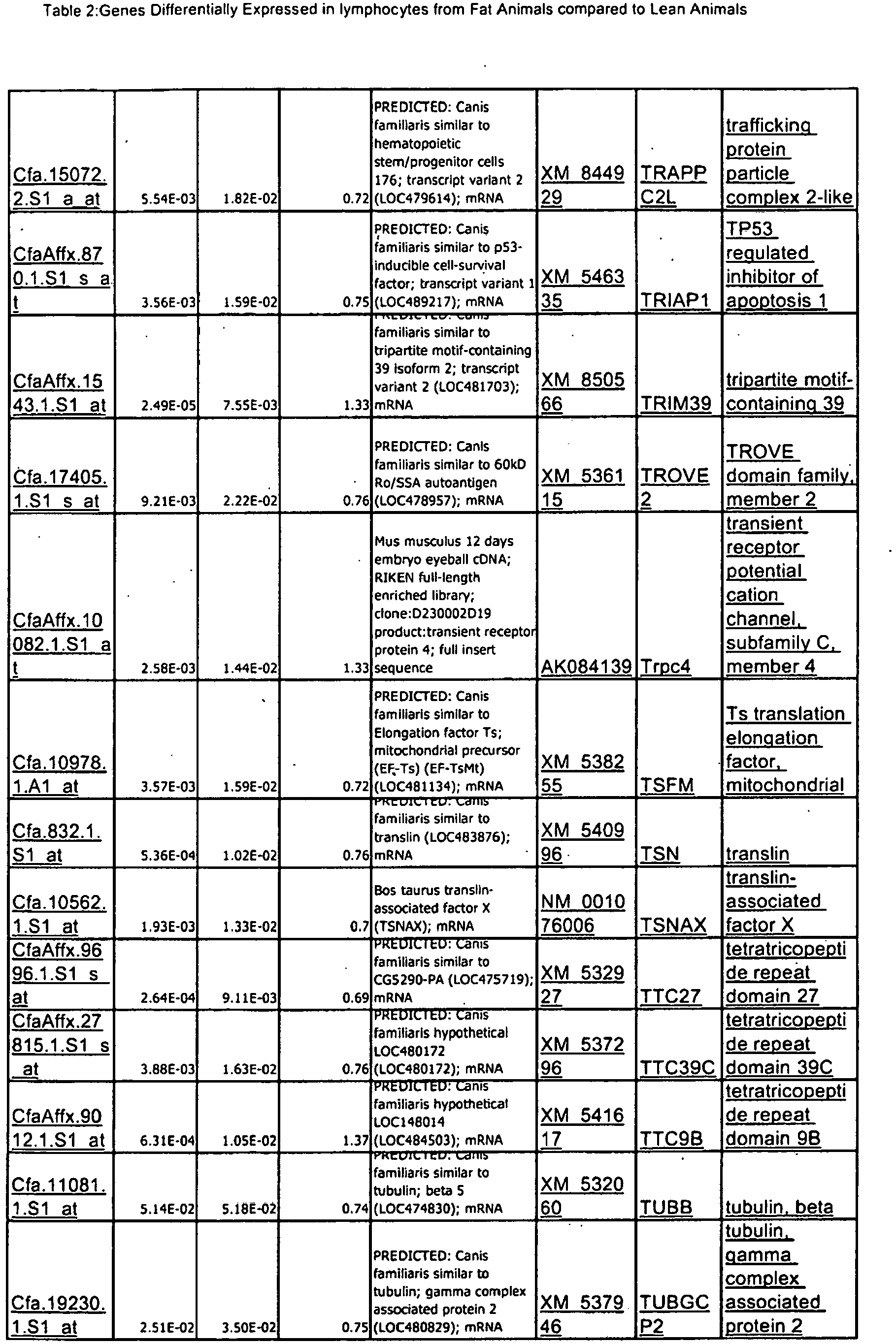

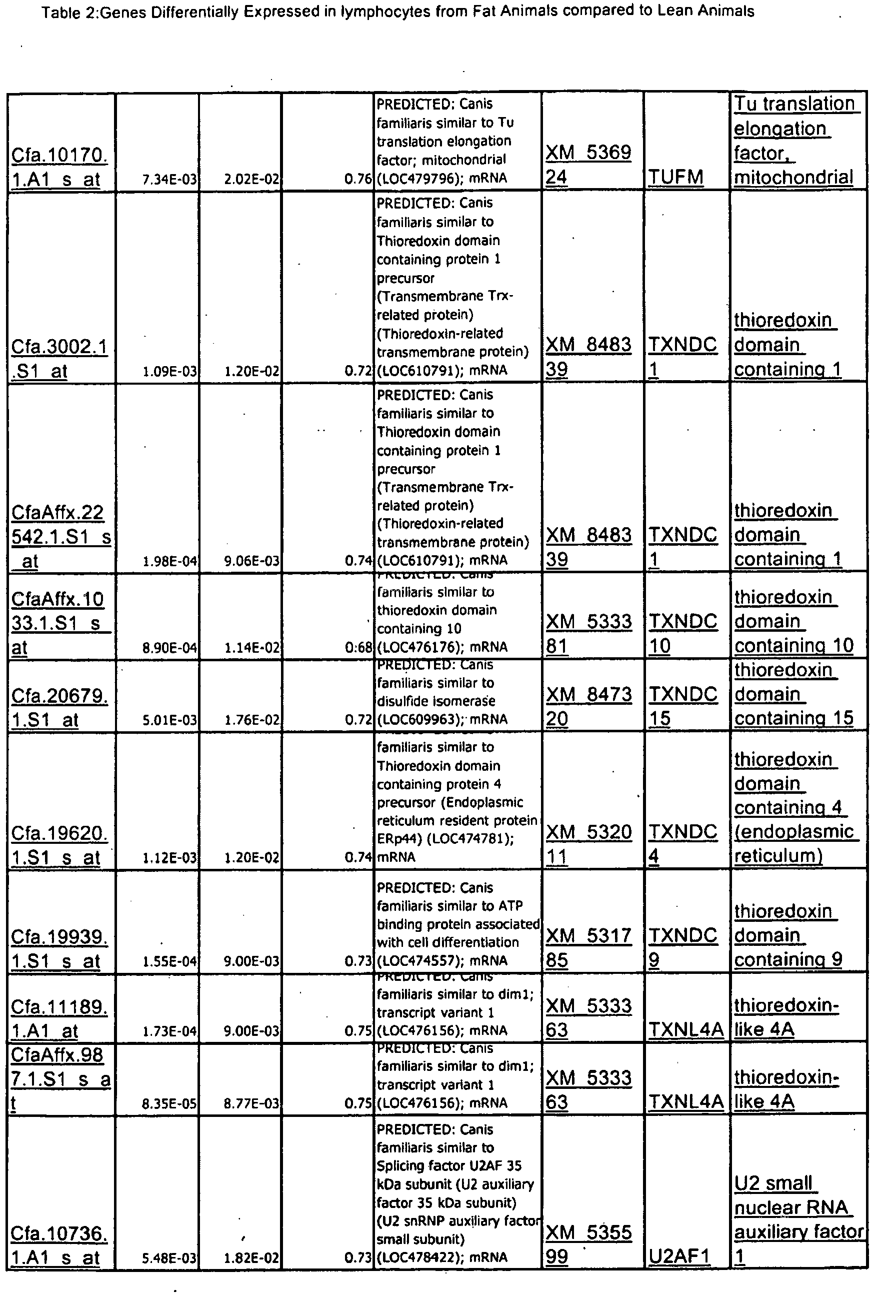

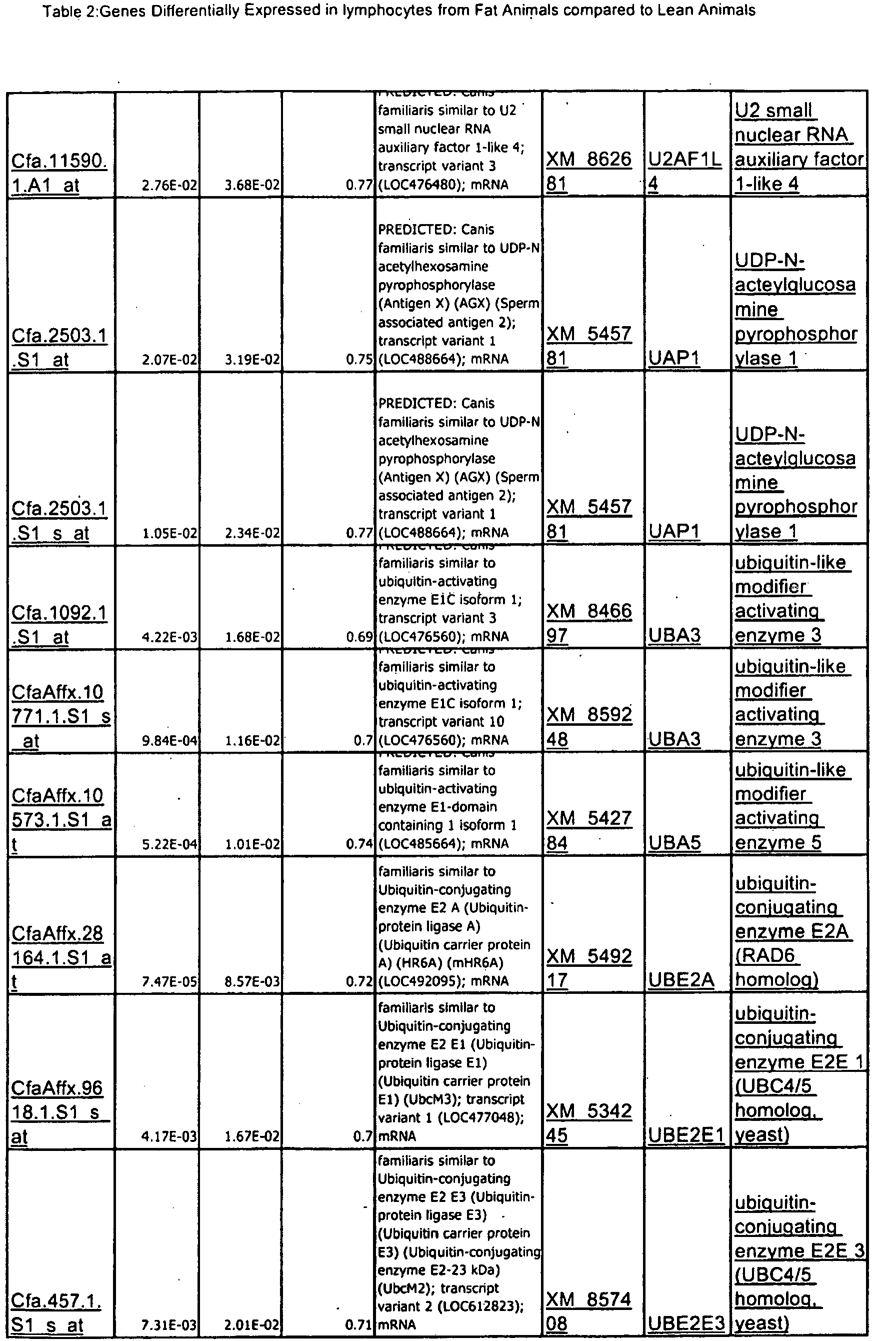

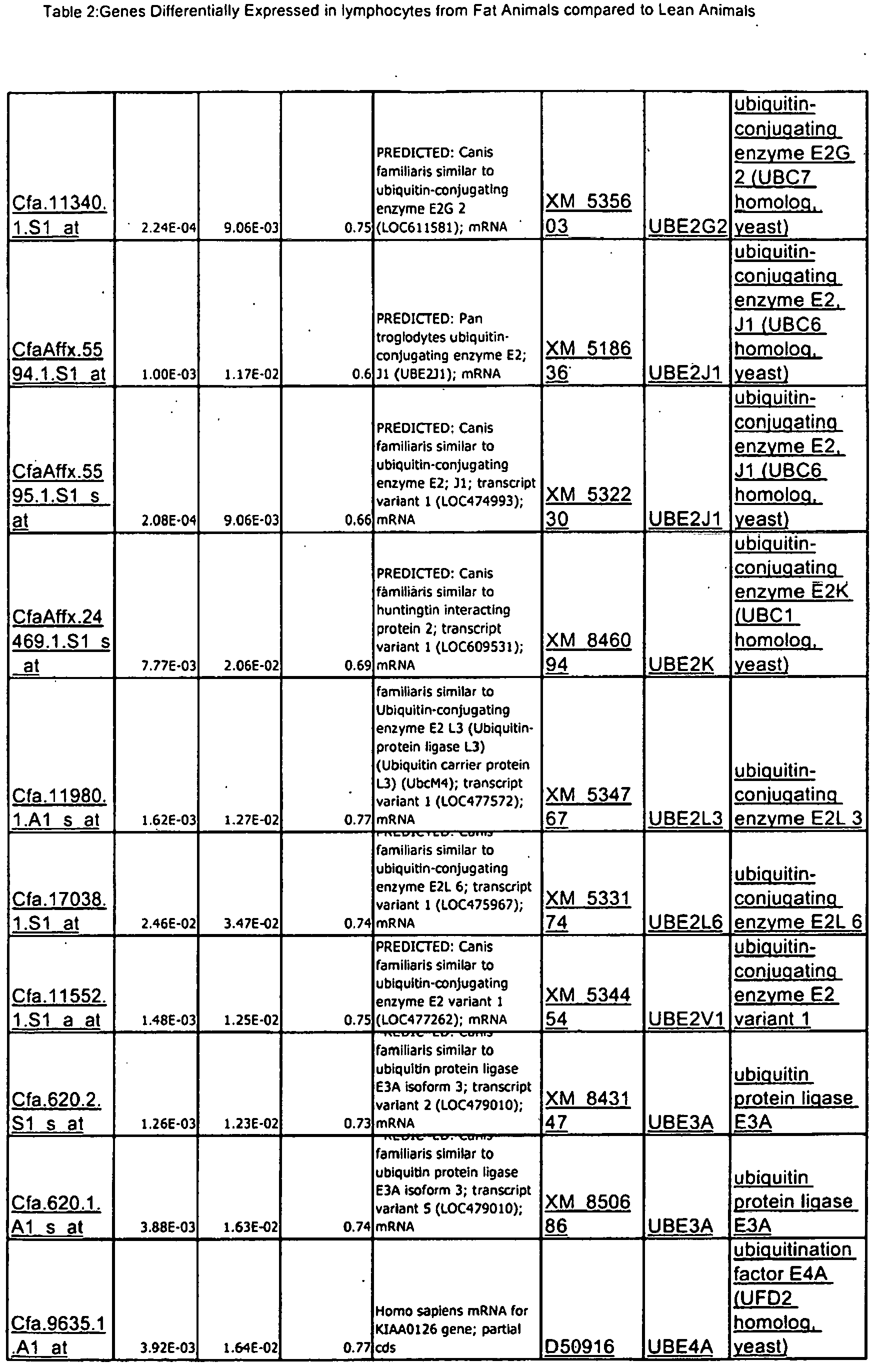

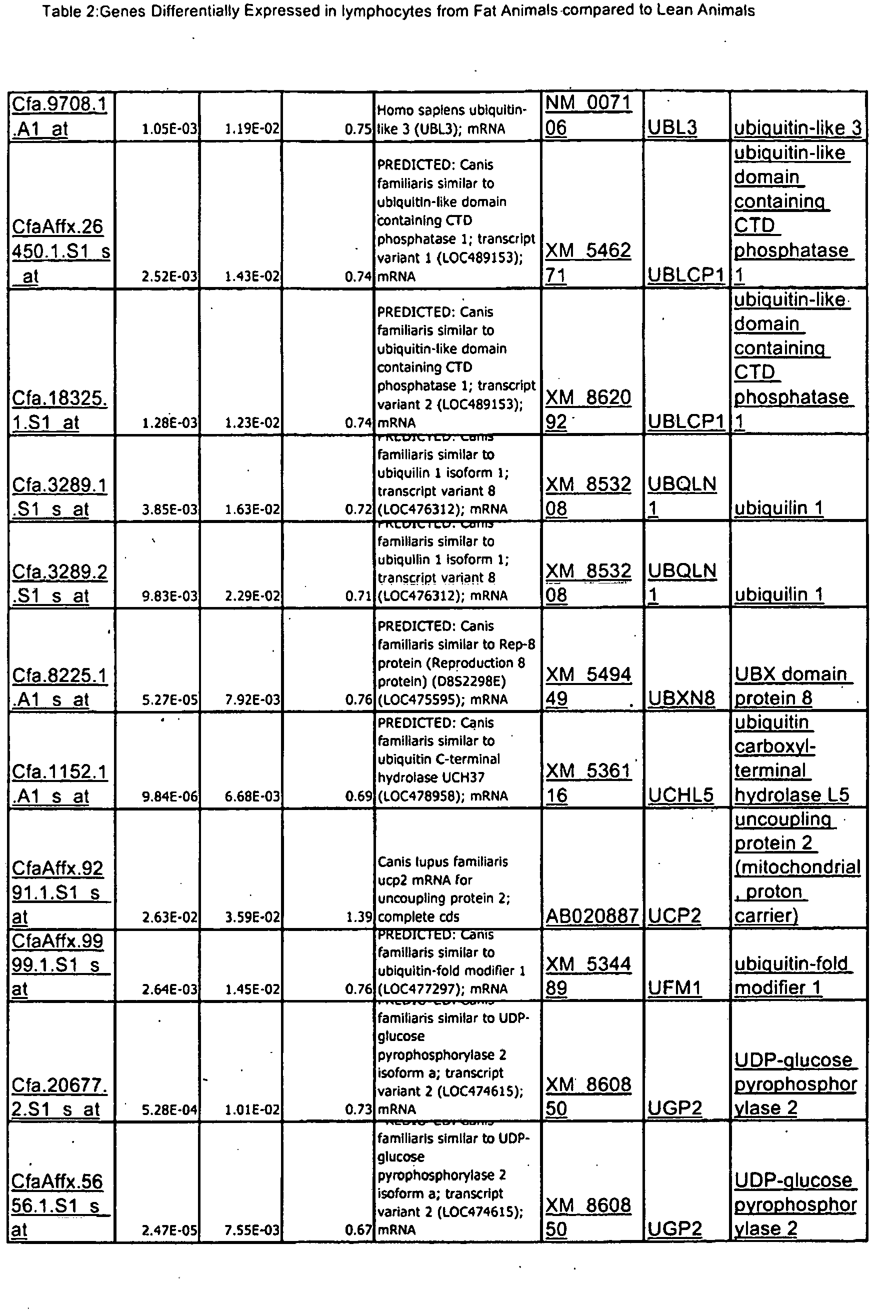

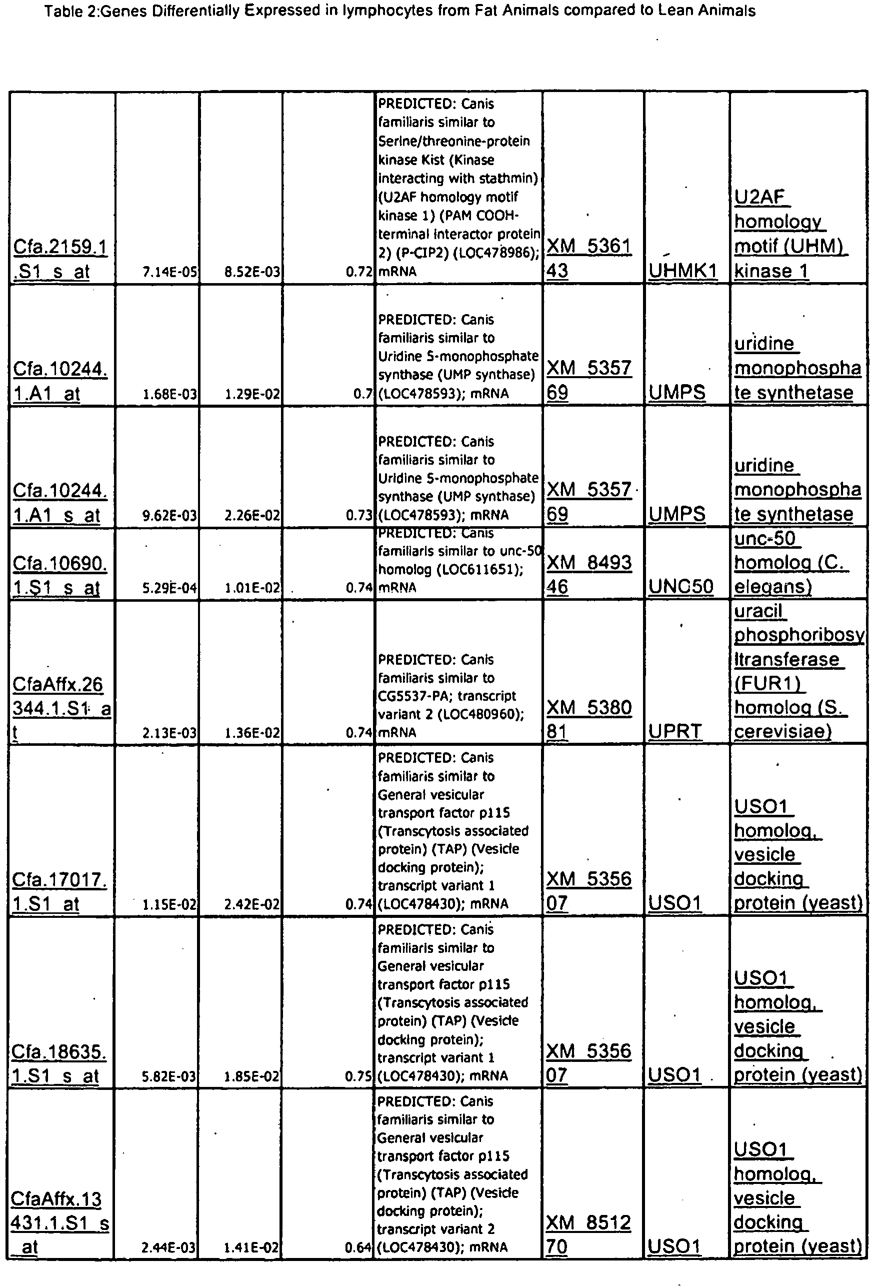

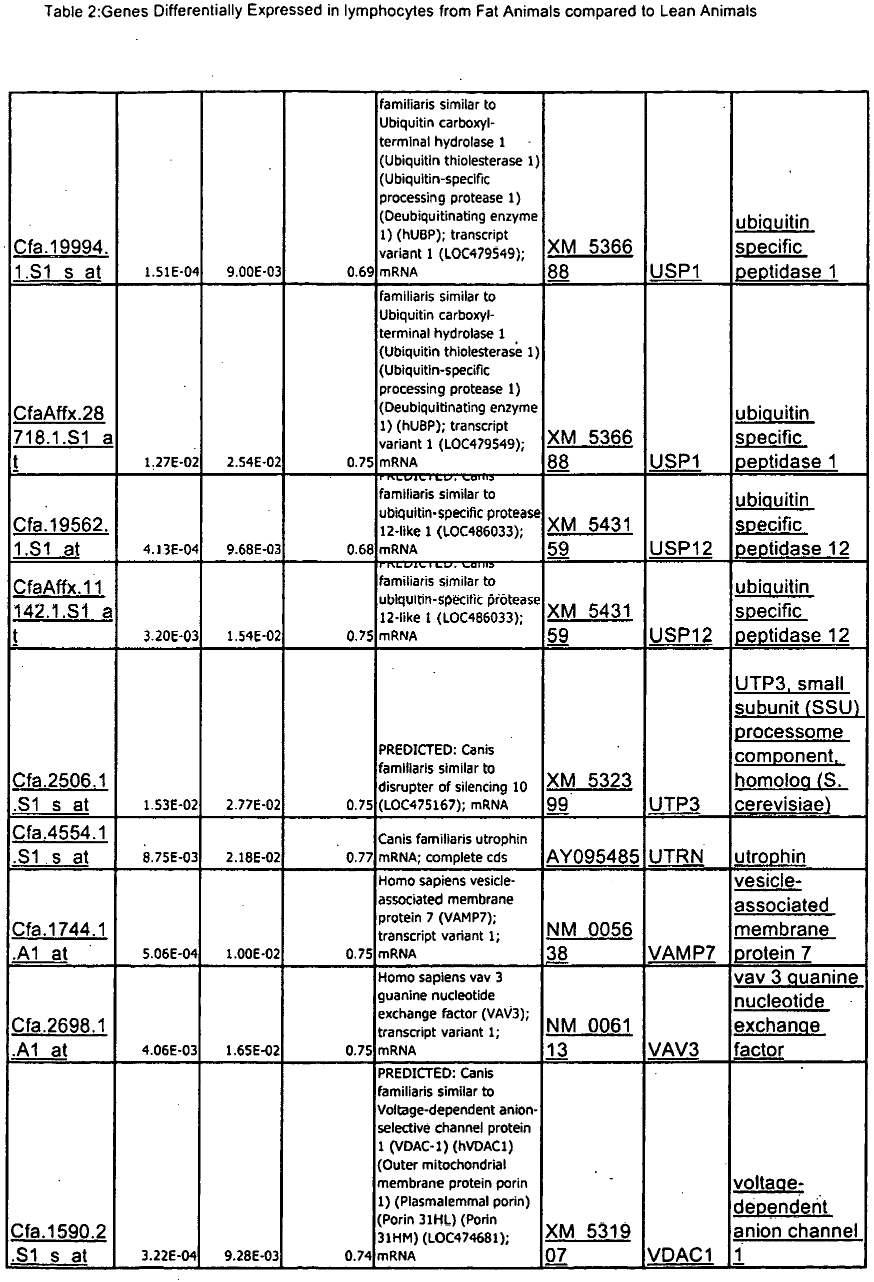

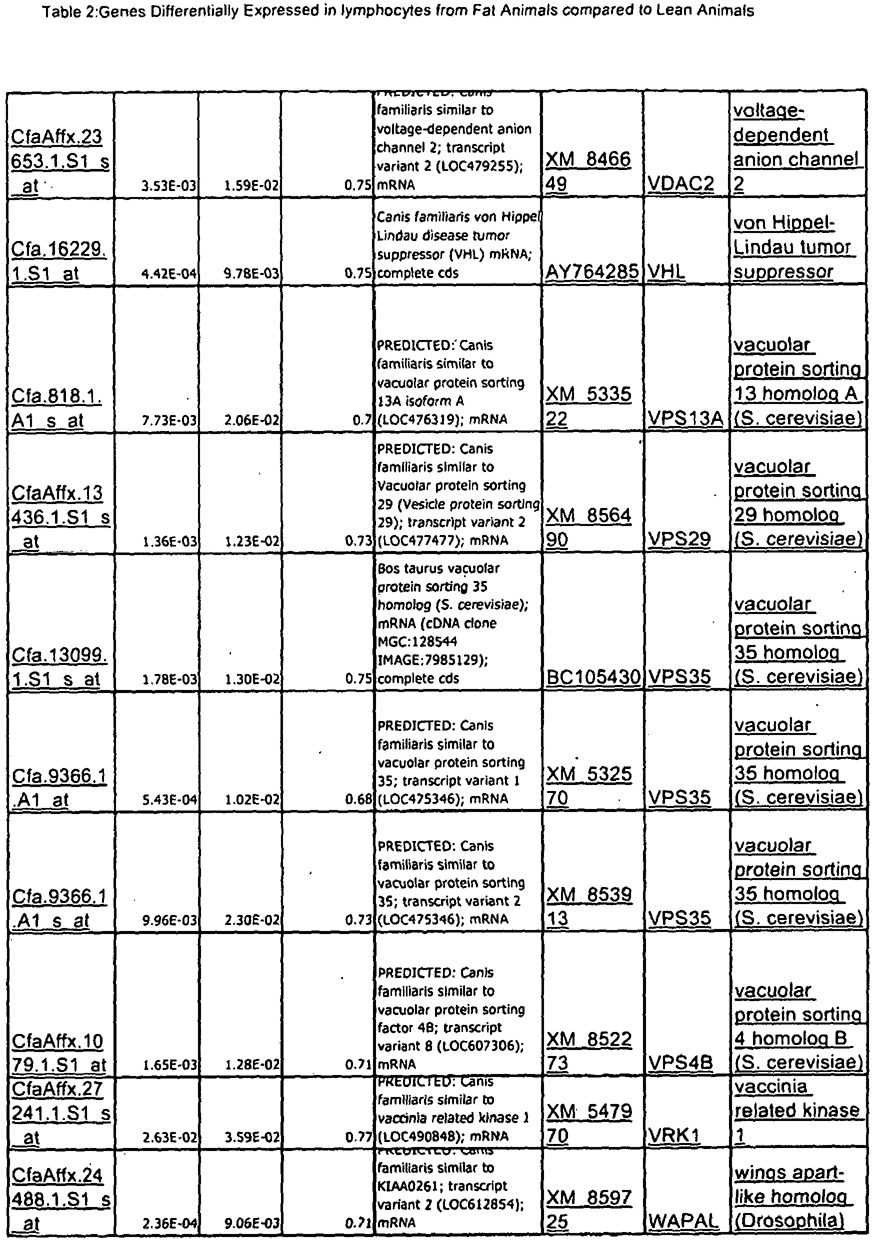

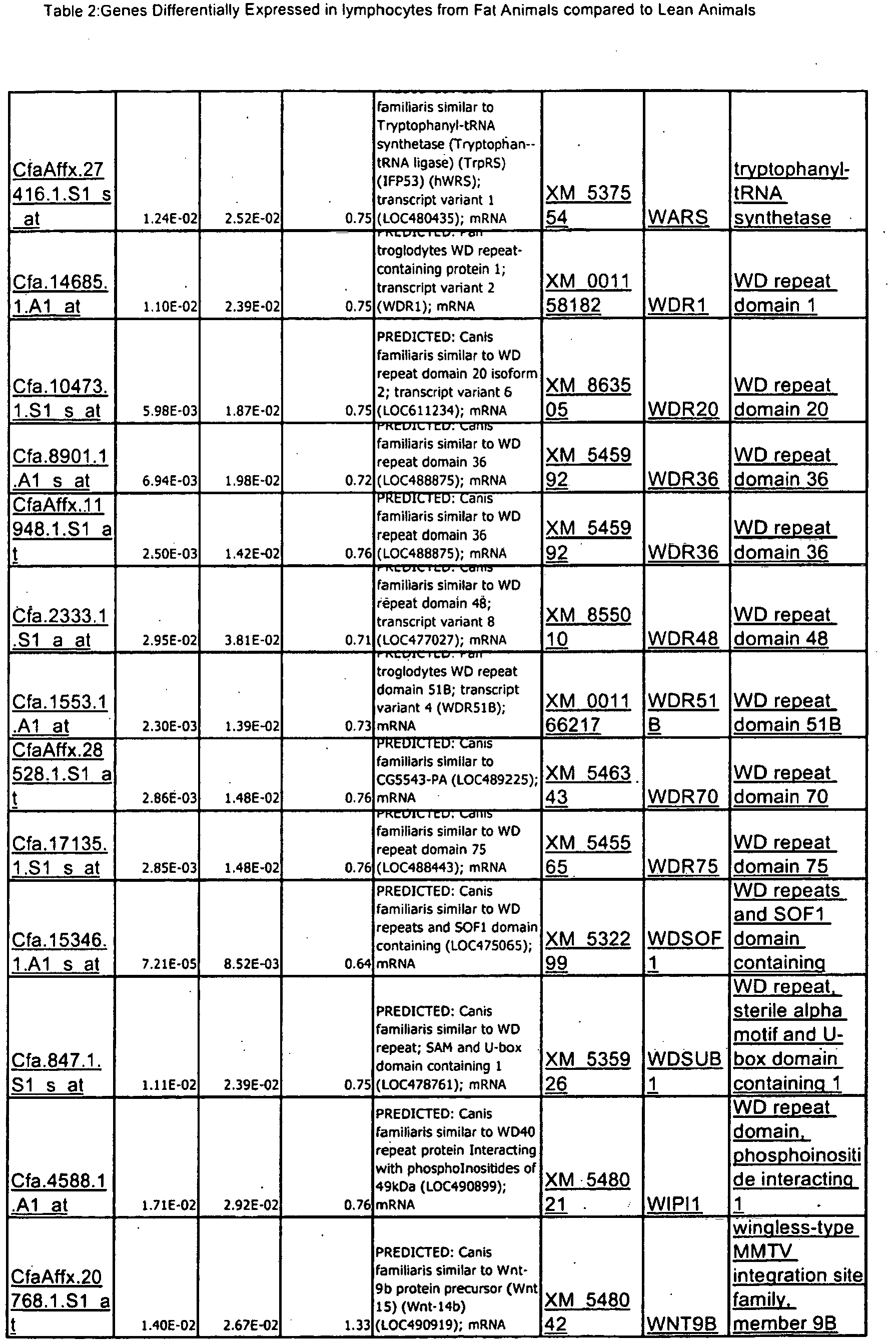

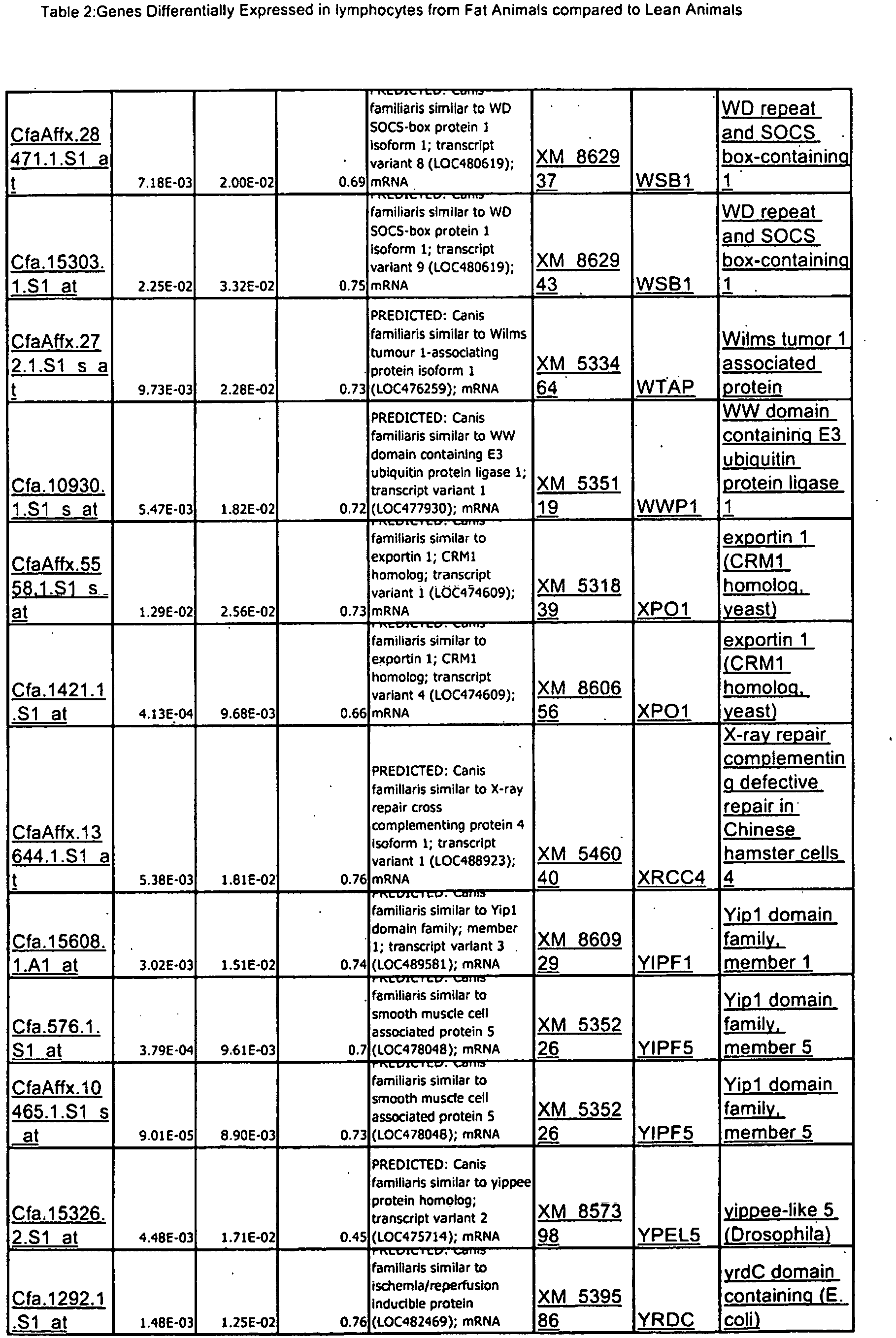

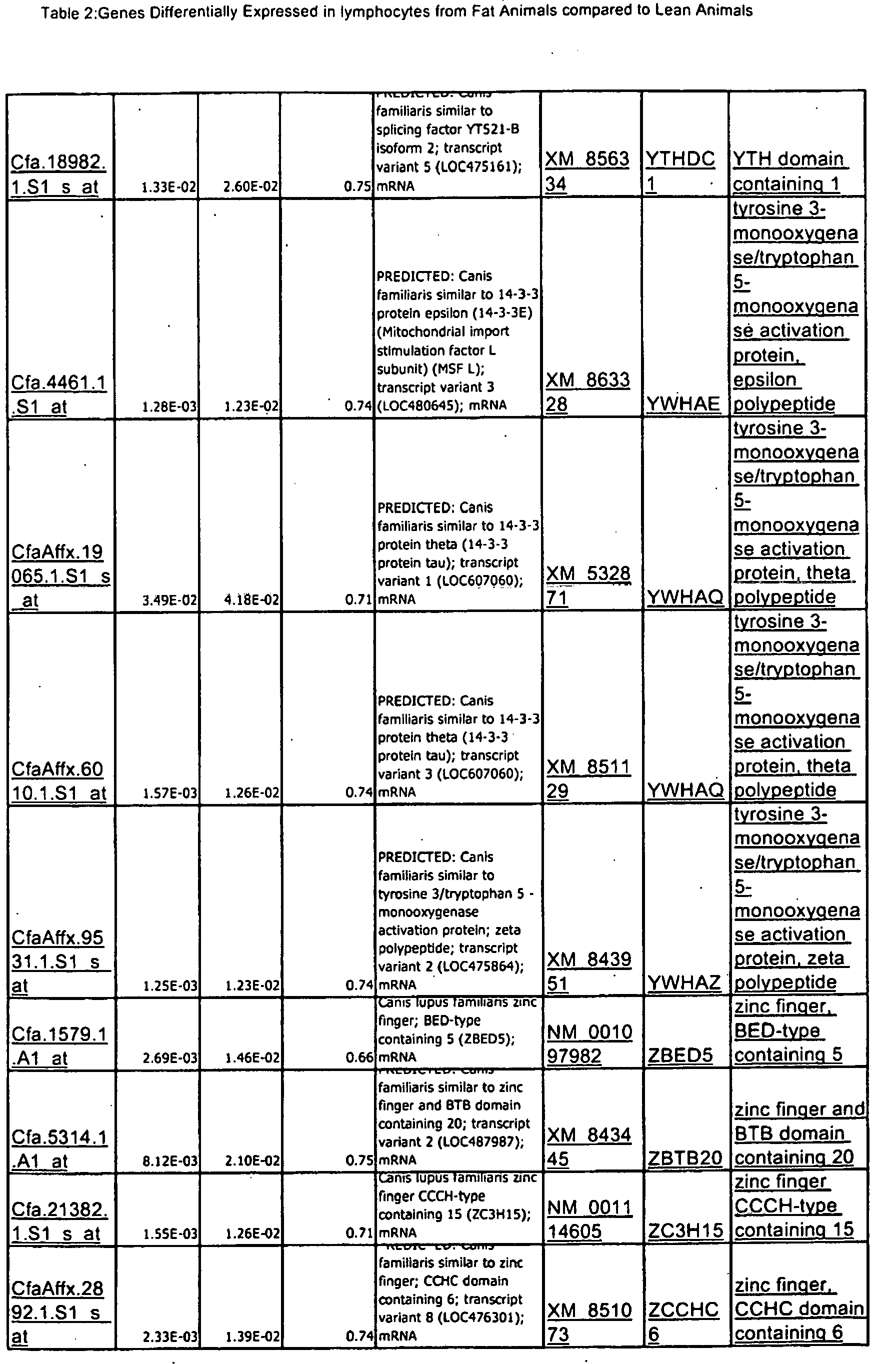

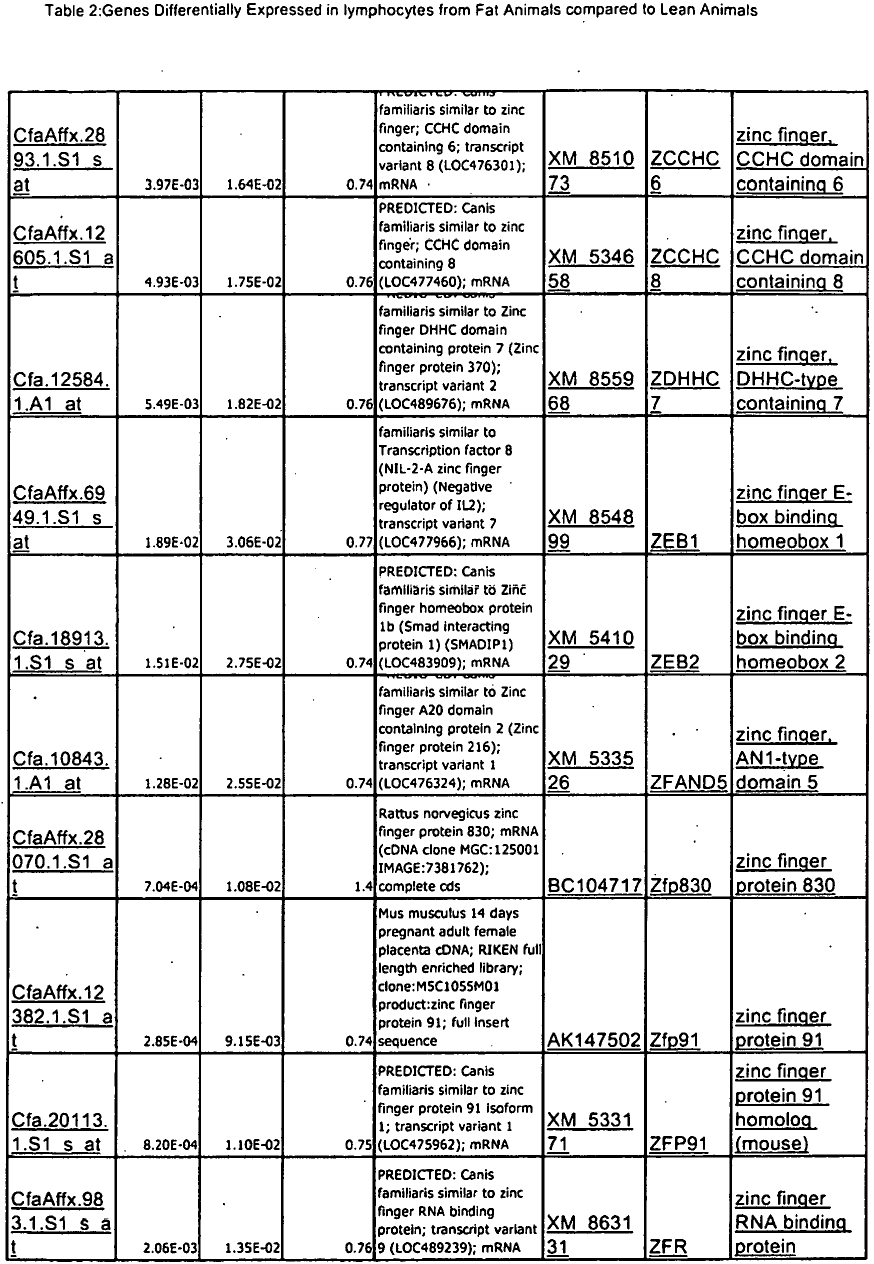

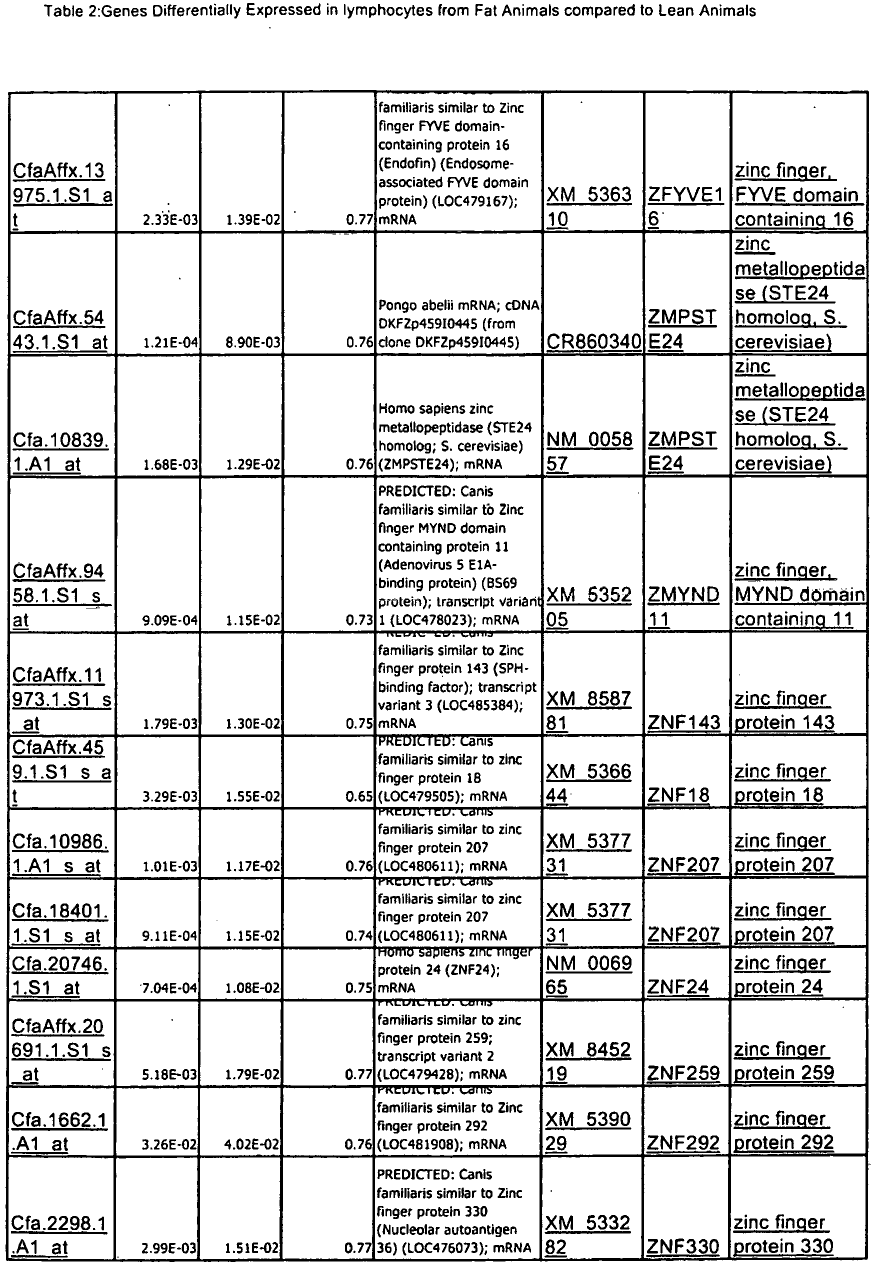

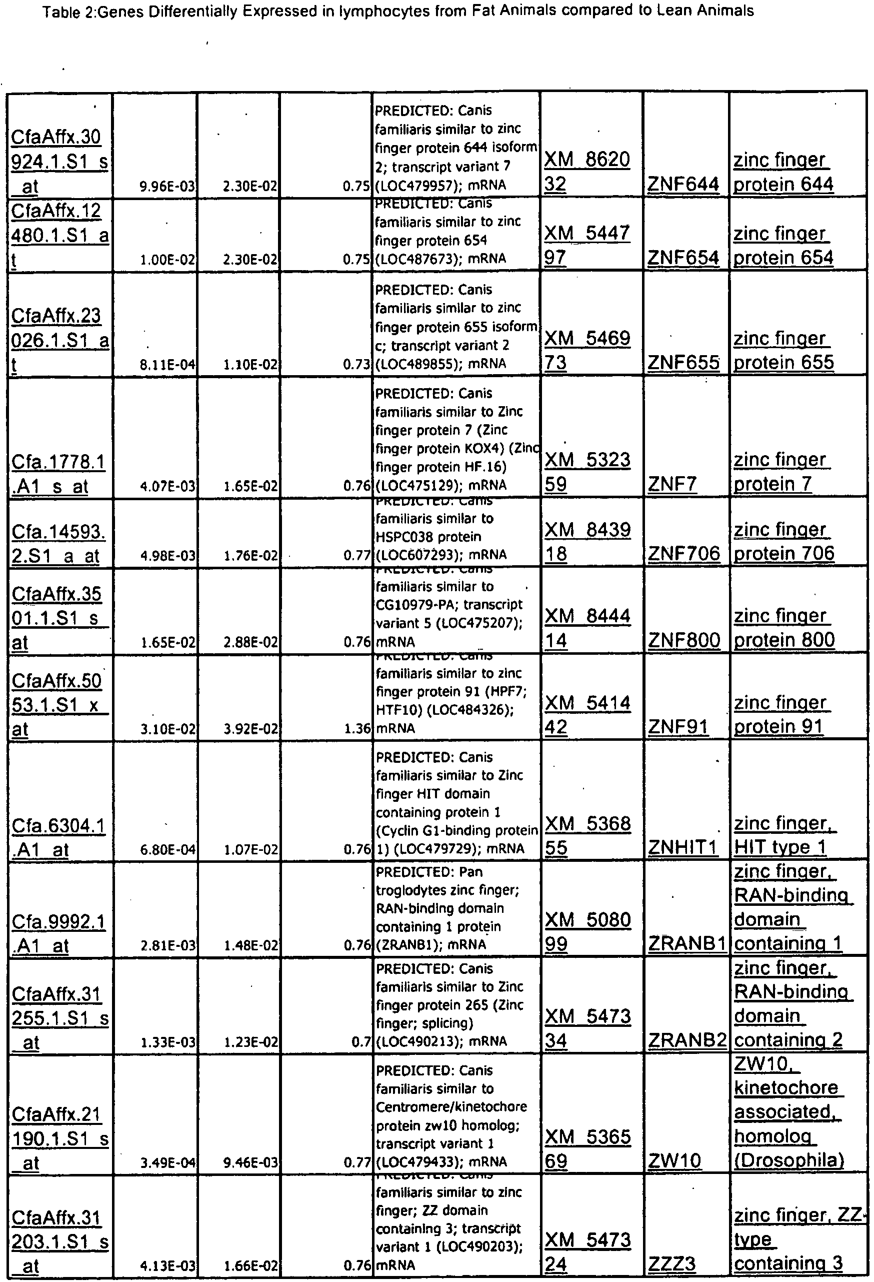

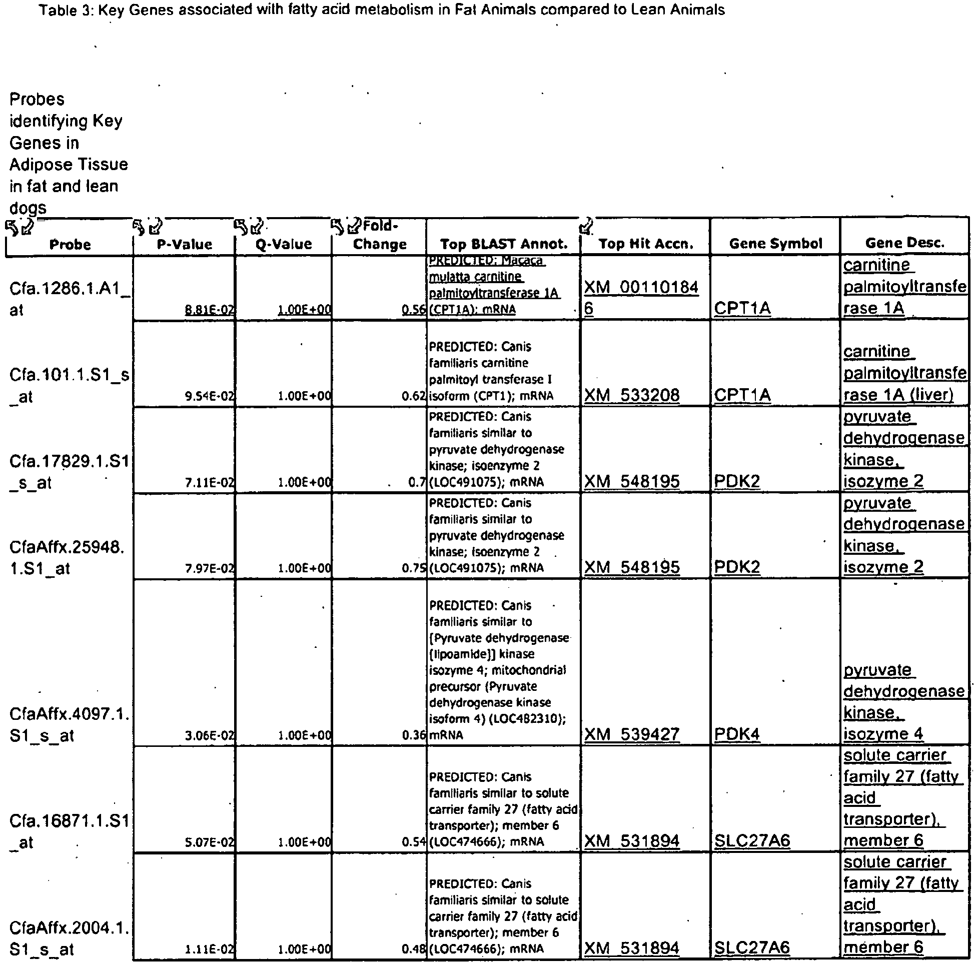

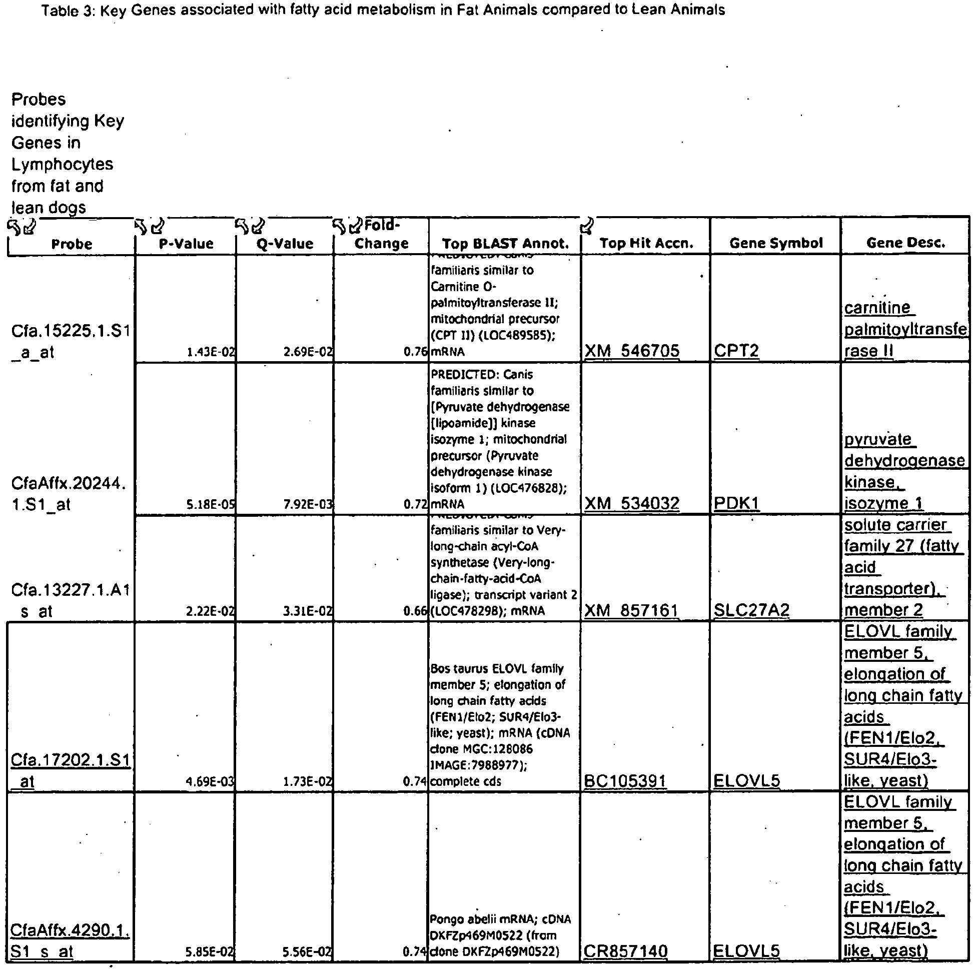

### Example 2

### Determining the Effect of Various Substances or Ingredients on Gene Expression in Canine Cell Lines

Affymetrix canine GeneChips^{®} Canine-1 and Canine-2 were used (Canine-2 replaced Canine-1) to determine the effect of various test substances or ingredients such as MCTs; TAGs; ALA; EPA; DHA; linoleic acid (LA); Arachidonic Acid (ARA); stearic acid (SA), Myristic acid (MA), conjugated linoleic acid (CLA), GLA; arachidonic acid; lecithin; vitamin A, vitamin D, vitamin E, vitamin K, riboflavin, niacin, pyridoxine, pantothenic acid, folic acid, biotin vitamin C, catechin, quercetin, theaflavin; ubiquinone; lycopene, lycoxanthin; resveratrol; α-lipoic acid; L-carnitine; D-limonene; glucosamine; S-adenosylmethionine; chitosan, various materials containing one or more of these compounds, and various combination thereof on gene expression in four canine cell lines and appropriate controls. Each ingredient is tested in two concentrations as illustrated for selected sample ingredients shown in Table 6. The solvent at the higher of the two concentrations is used as a control. Four canine cell lines are used: CCL34 (kidney), CRL1430 (thymus), CCL183 (bone) (obtained from The American Tissue Culture Collection) and CTAC (thyroid) (See, Measurement of NK Activity in Effector Cells Purified from Canine Peripheral Lymphocytes, Veterinary Immunology and Immunopathology, 35 (1993) 239-251). A cell line treated with an ingredient at a specific concentration is referred to as "treatment" and an untreated sample is referred to as "control." The words "genes" and "probes" are used synonymously in this method. Gene expression is measured for the treatment cell lines and controls using the instructions provided with the Affymetrix chips.

A sample of the data obtained from these experiments is shown in Table 5. Table 5 shows the probe id, the p-value, the fold change, the annotation of the top BLAST hit, the top BLAST hit accession number, the gene symbol and finally the gene description is given in the last column.
Based upon the physiological condition of the canines (a diagnosis as fat) and a comparison of the information from the Tables 1, 2, 3 and 5, i.e, noting genes that are influenced by a test substance or ingredient and are also differentially expressed in overweight canines compared to lean canines, a nutritional formula useful for selecting and preparing a food composition for overweight canines would be believed to contain one or more of the following ingredients in the following amounts (in vivo amounts in milligrams per kilogram of body weight per day (mg/kg/day) are based upon extrapolation from amounts used in vitro, for example: DHA - from 1 to 30; EPA - from 1 to 30; EPA/DHA Combo (1.5:1 ratio) - from 4/2 to 30/45; ALA - from 10 to 100; LA - from 30 to 600; ARA - from 5 to 50; SA - from 3 to 60 and MA - from 3 to 60. Based upon these data, a food composition and related diet containing one or more of these ingredients can be prepared and used to regulate the genes that are differentially expressed in overweight animals compared to lean animals. Such regulation will cause the modulation of the amount of adipose tissue on the animal and, therefore, in one embodiment, promote a shift to a desirable or normal (more lean) status and promote better health and wellness of the animal.

**Table 4: Ingredients Tested in Canine Cell Lines**

| **Substance** | **Concentration 1** | **Concentration 2** | **Solvent** |
|---|---|---|---|
| DHA | 0.005 mg/ml (5 micro g/ml) | 0.025 mg/ml (25 micro g/ml) | ETOH |
| EPA | 0.005 mg/ml (5 micro g/ml) | 0.025 mg/ml (25 micro g/ml) | ETOH |
| EPA/DHA Combo 1.5:1 ratio (like in fish oil) | 0.015 mg/ml EPA & 0.010 mg/ml DHA (total is 0.025 mg/ml) | 0.030 mg/ml EPA & 0.02 mg/ml DHA (total is 0.050 mg/ml) | ETOH |
| Alpha linolenic acid (ALA) | 0.05 mg/ml (50 micro g/ml) | 0.1 mg/ml (100 micro g/ml) | ETOH |
| Linoleic acid (LA) | 0.1 mg/ml (100 micro g/ml) | 0.5 mg/ml (500 micro g/ml) | ETOH |
| Arachidonic acid (ARA) | 0.025 mg/ml (25 micro g/ml) | 0.05 mg/ml (50 micro g/ml) | ETOH |
| Stearic acid (SA) | 0.01 mg/ml (10 micro g/ml) | 0.05 mg/ml (50 micro g/ml) | ETOH |
| Myristic Acid (MA) | 0.01 mg/ml (10 micro g/ml) | 0.05 mg/ml (50 micro g/ml) | ETOH |
| Conjugated Linoleic acid | 0.02 mg/ml (20 micro g/ml) | 0.1 mg/ml (100 micro g/ml) | MEOH |

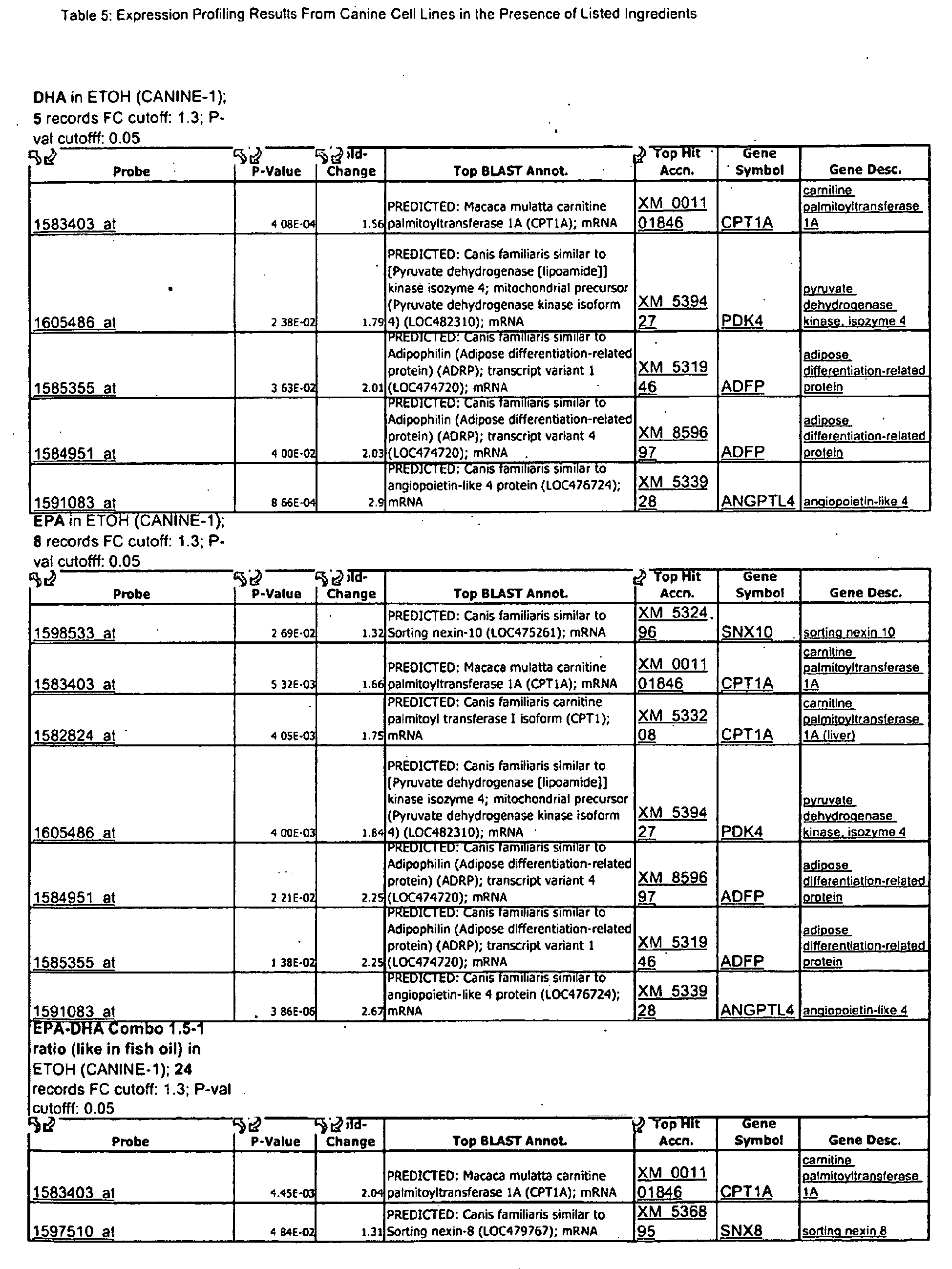

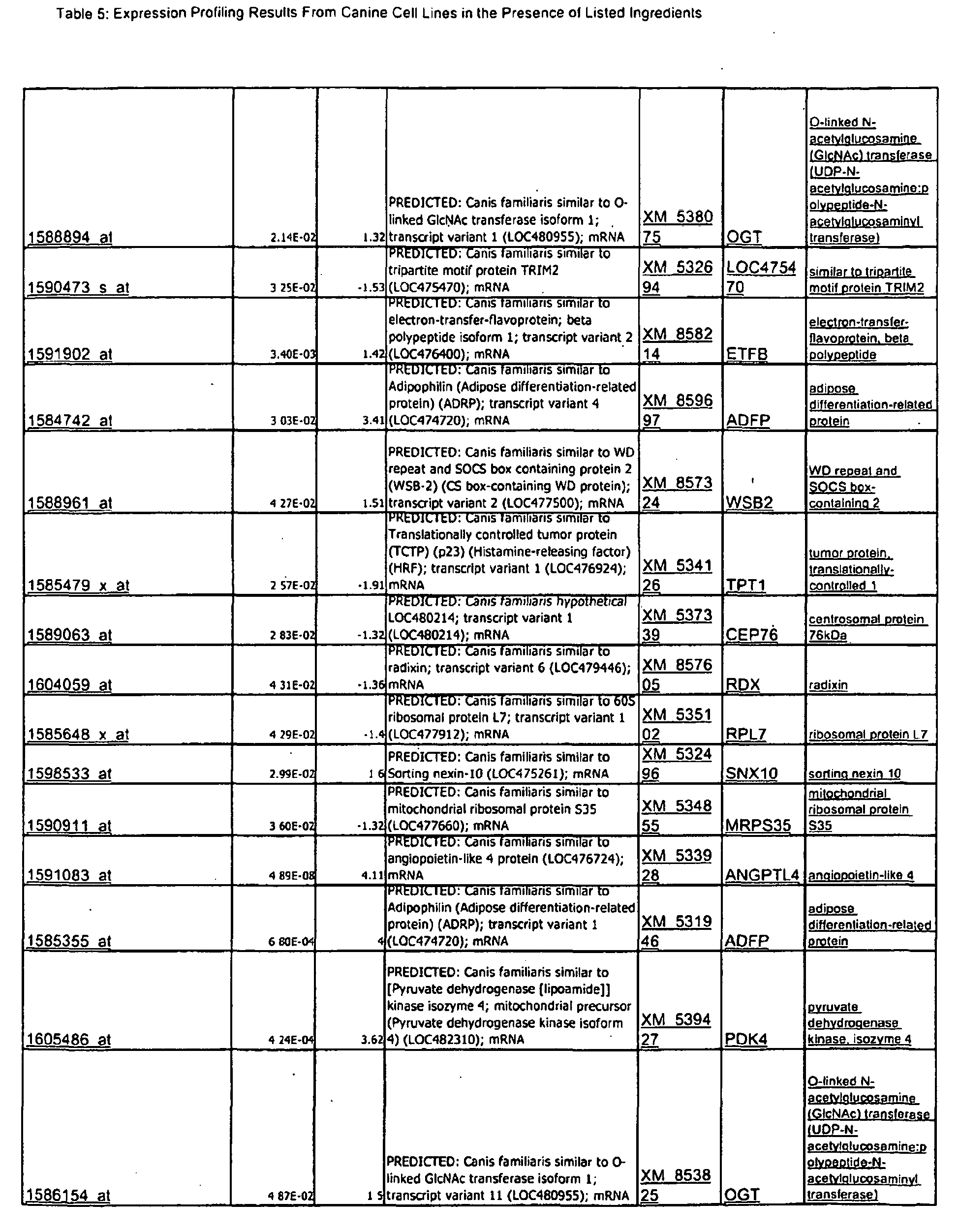

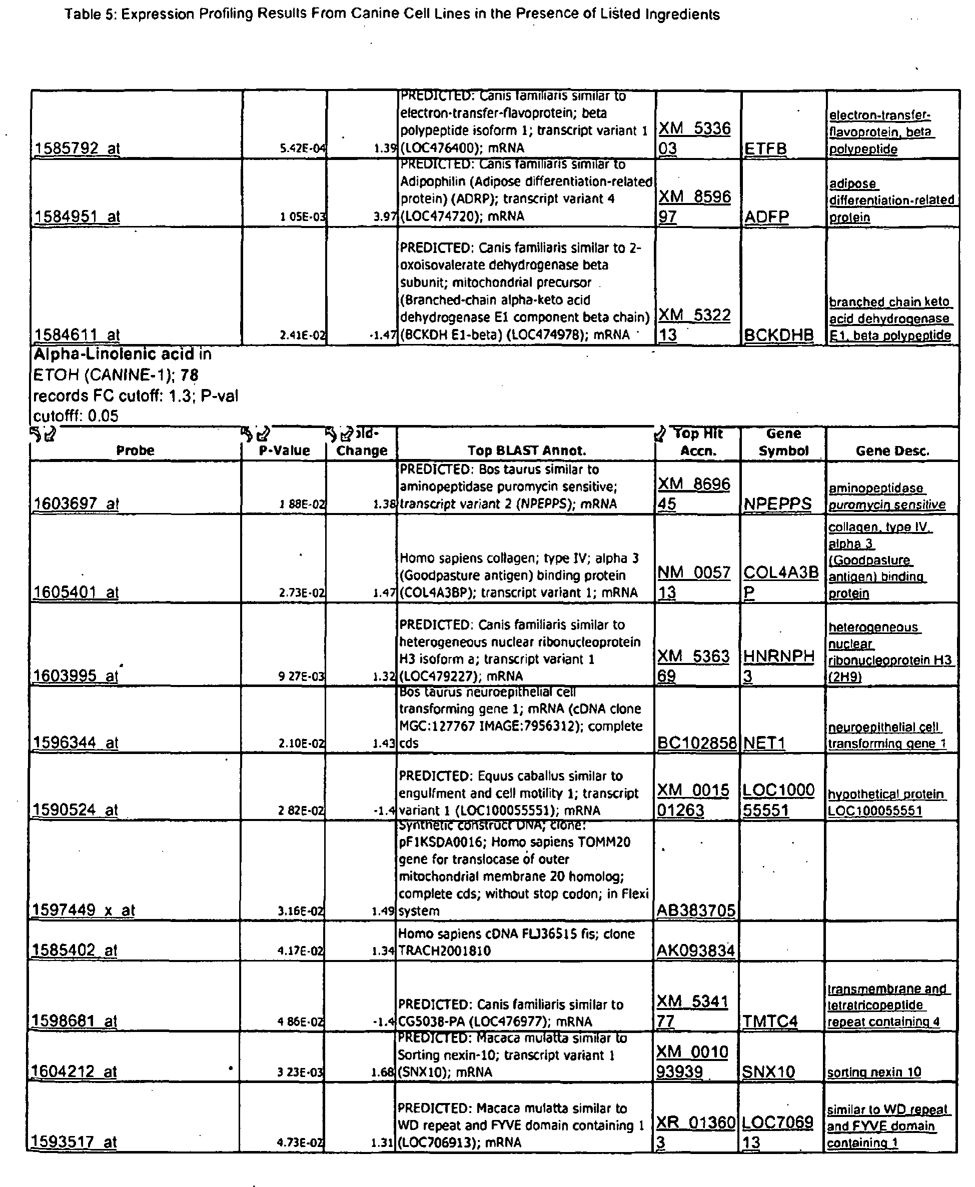

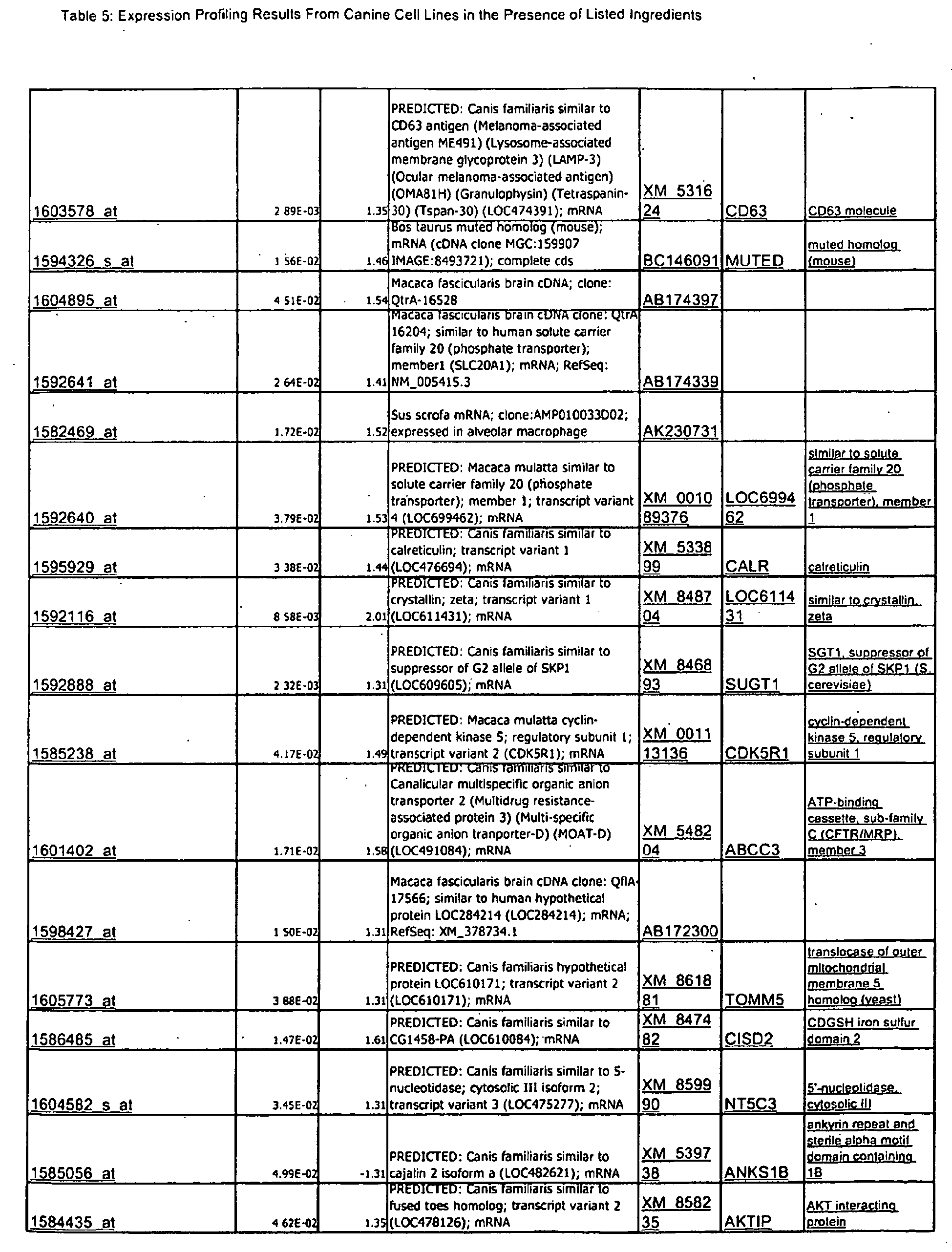

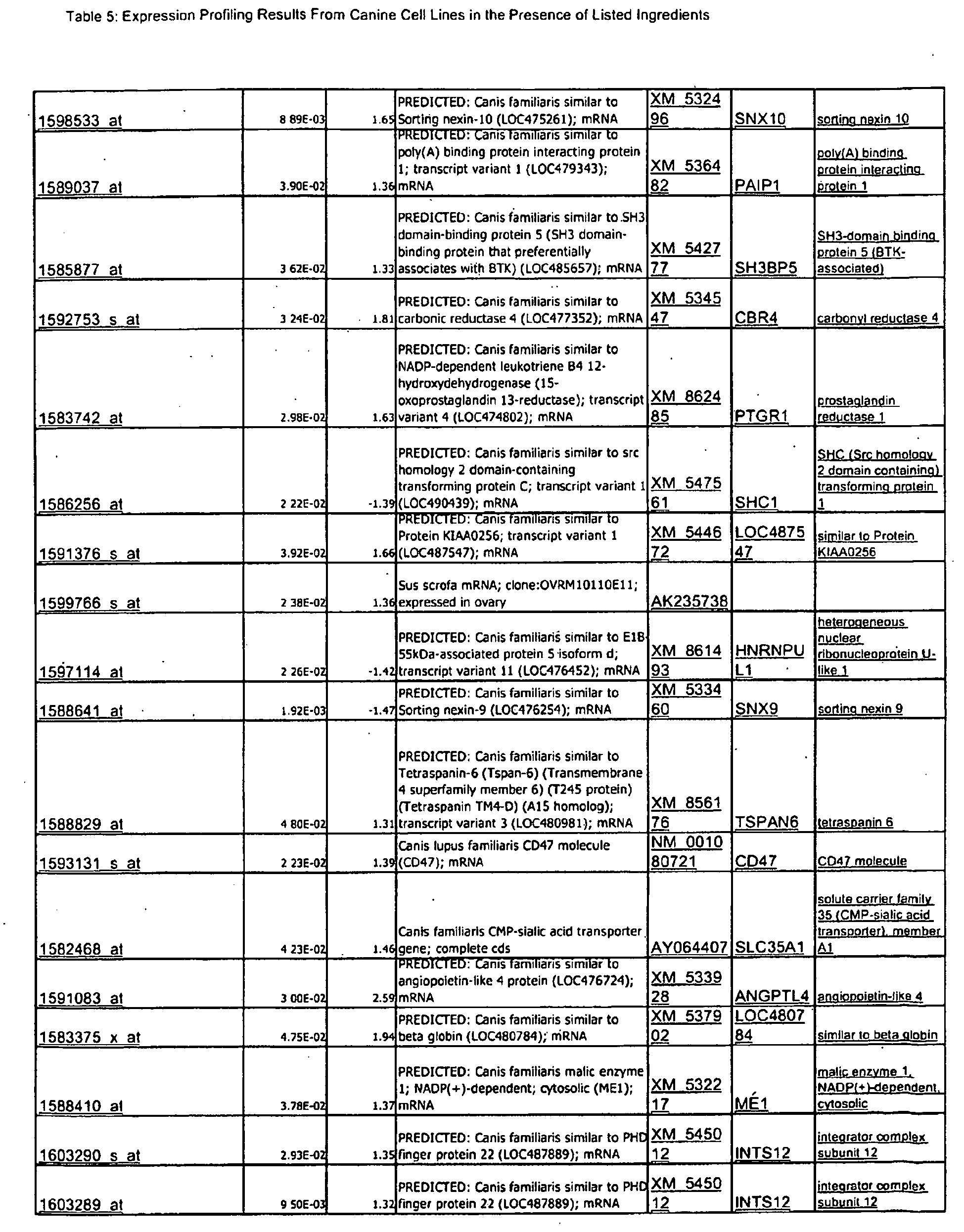

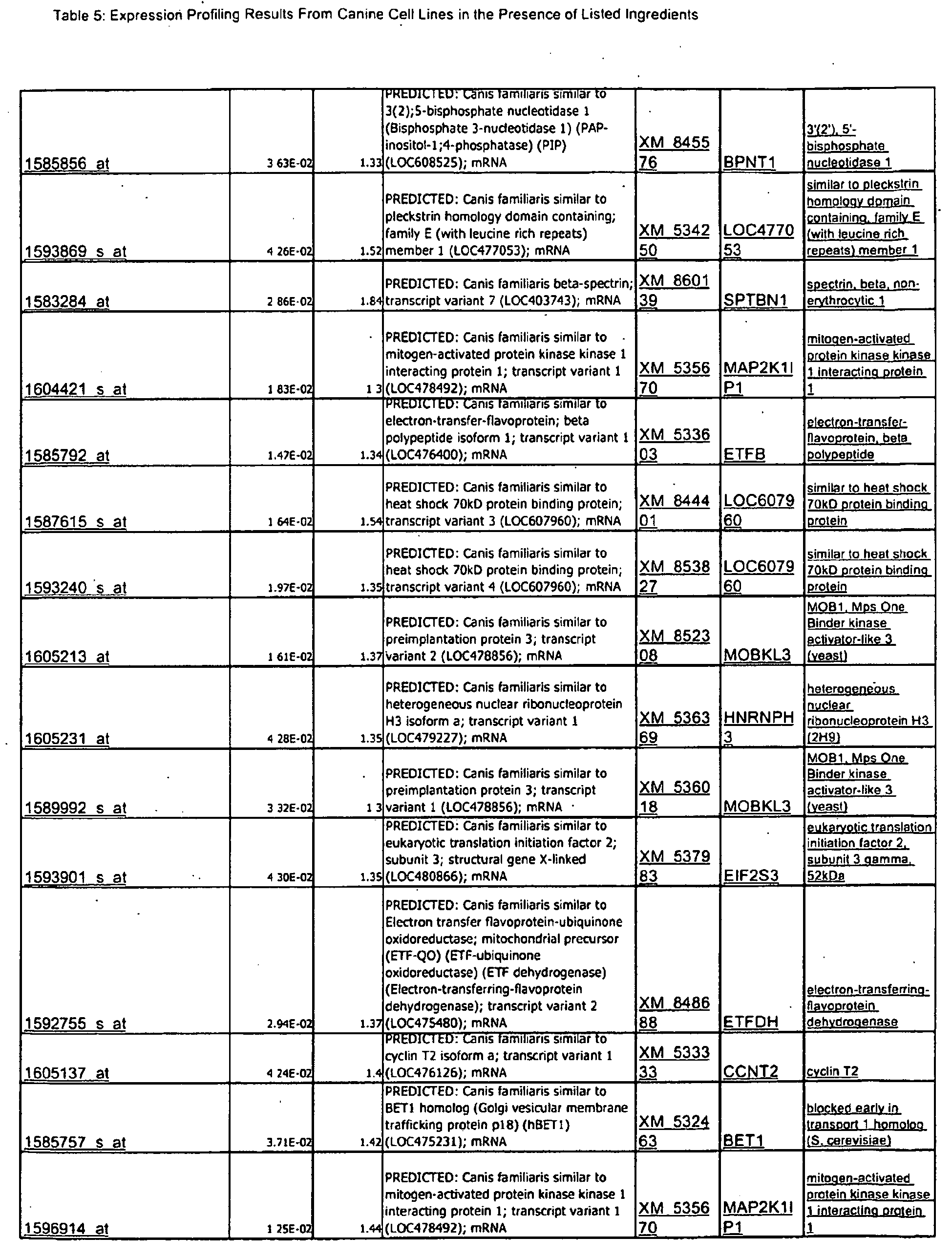

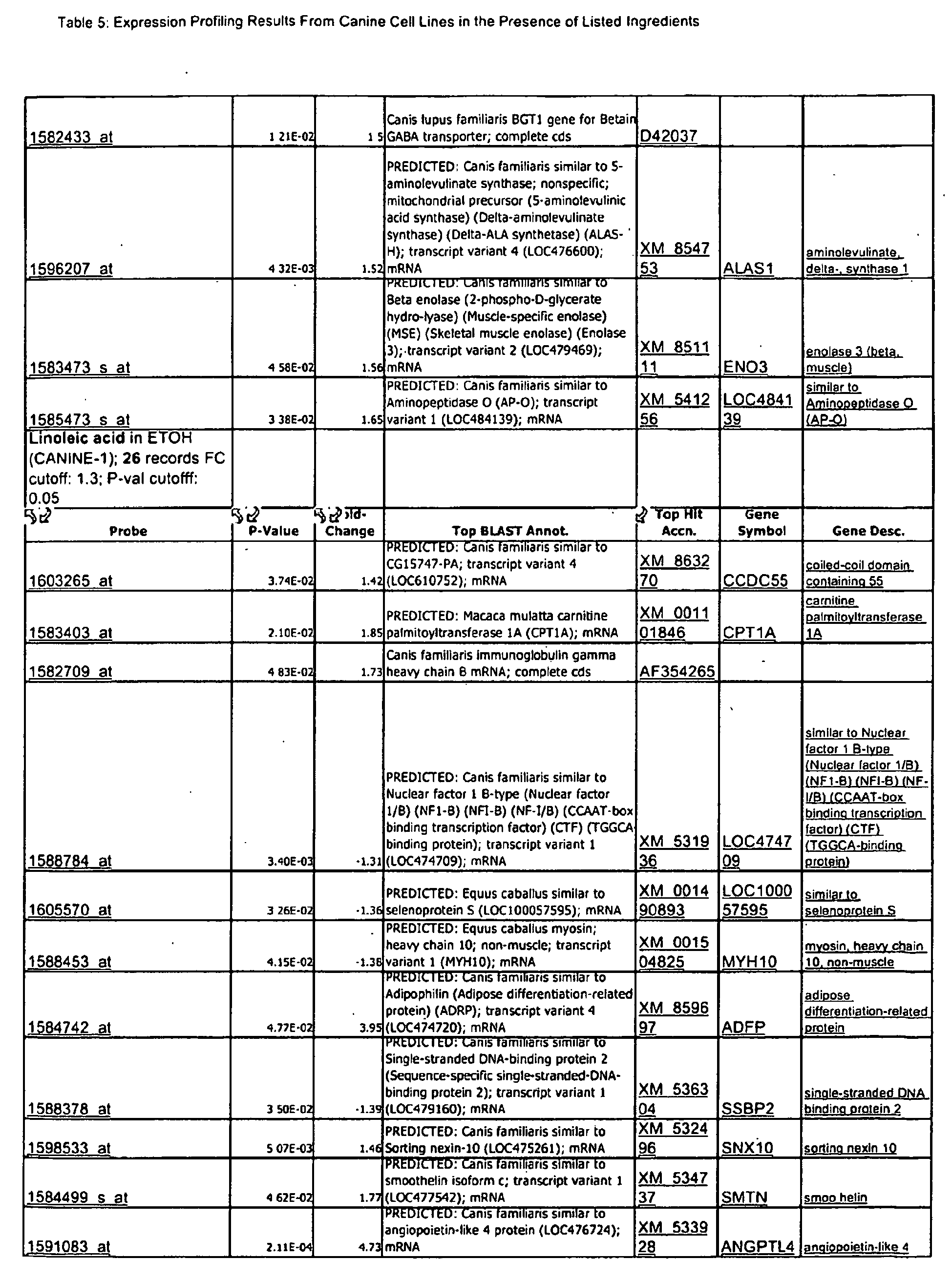

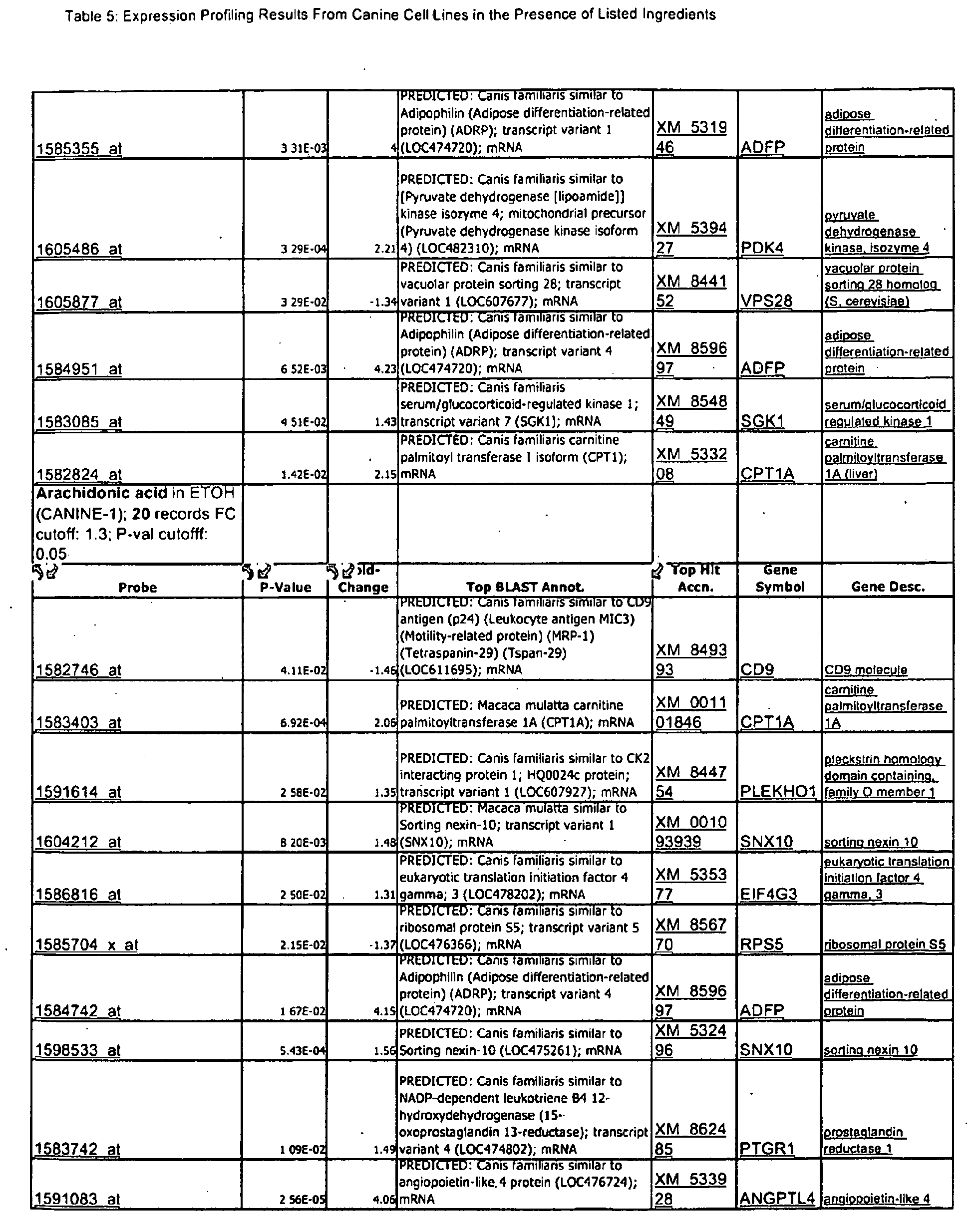

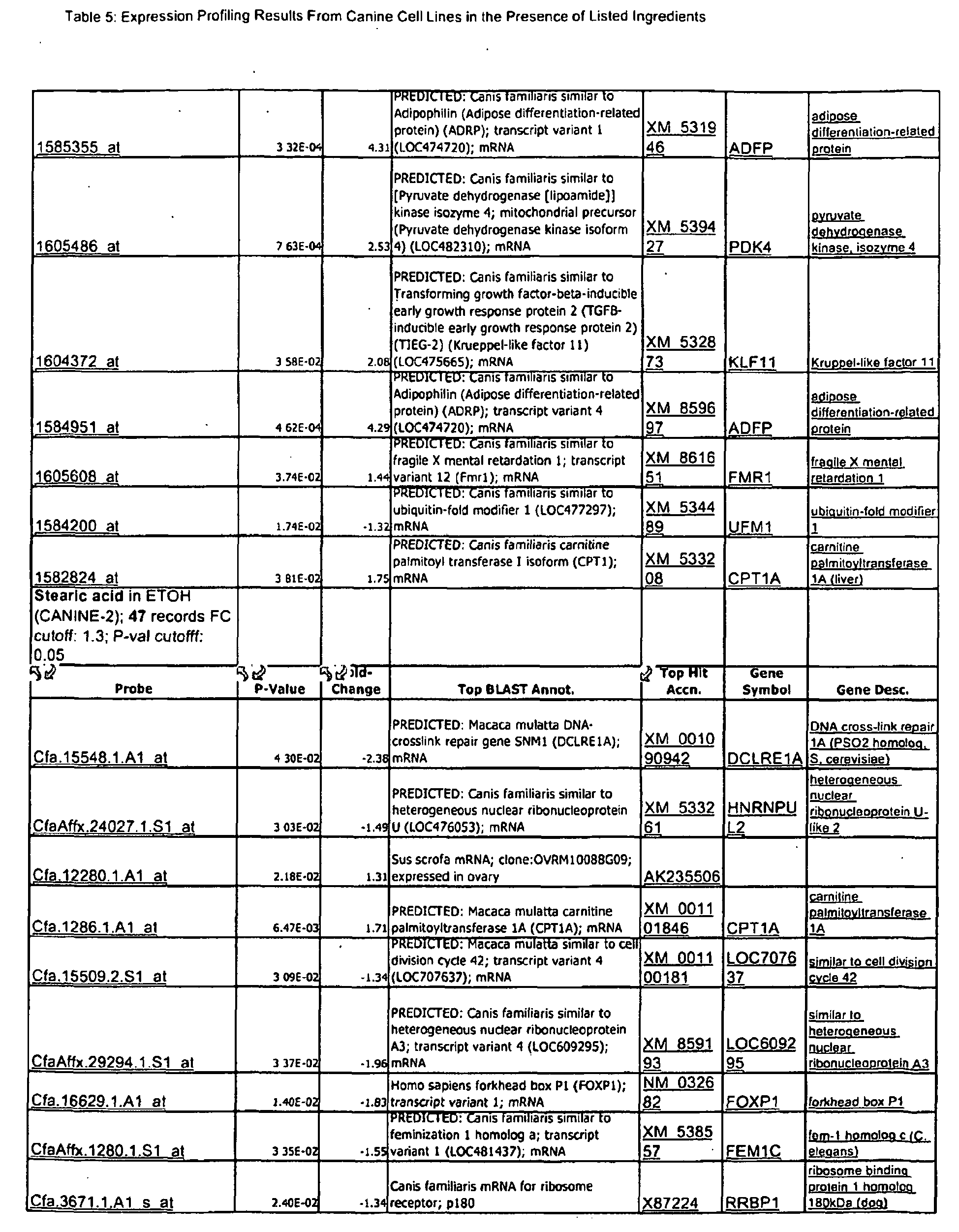

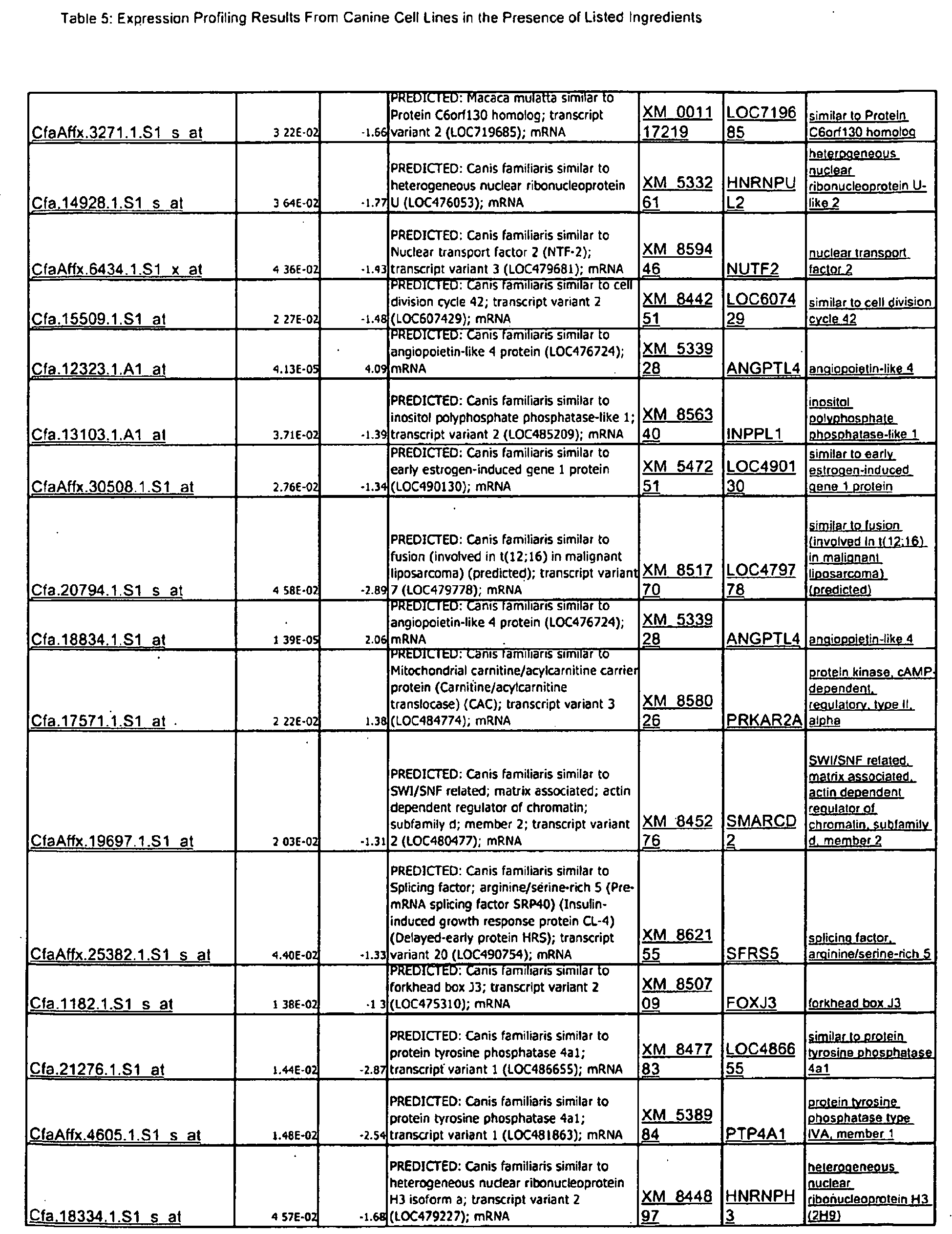

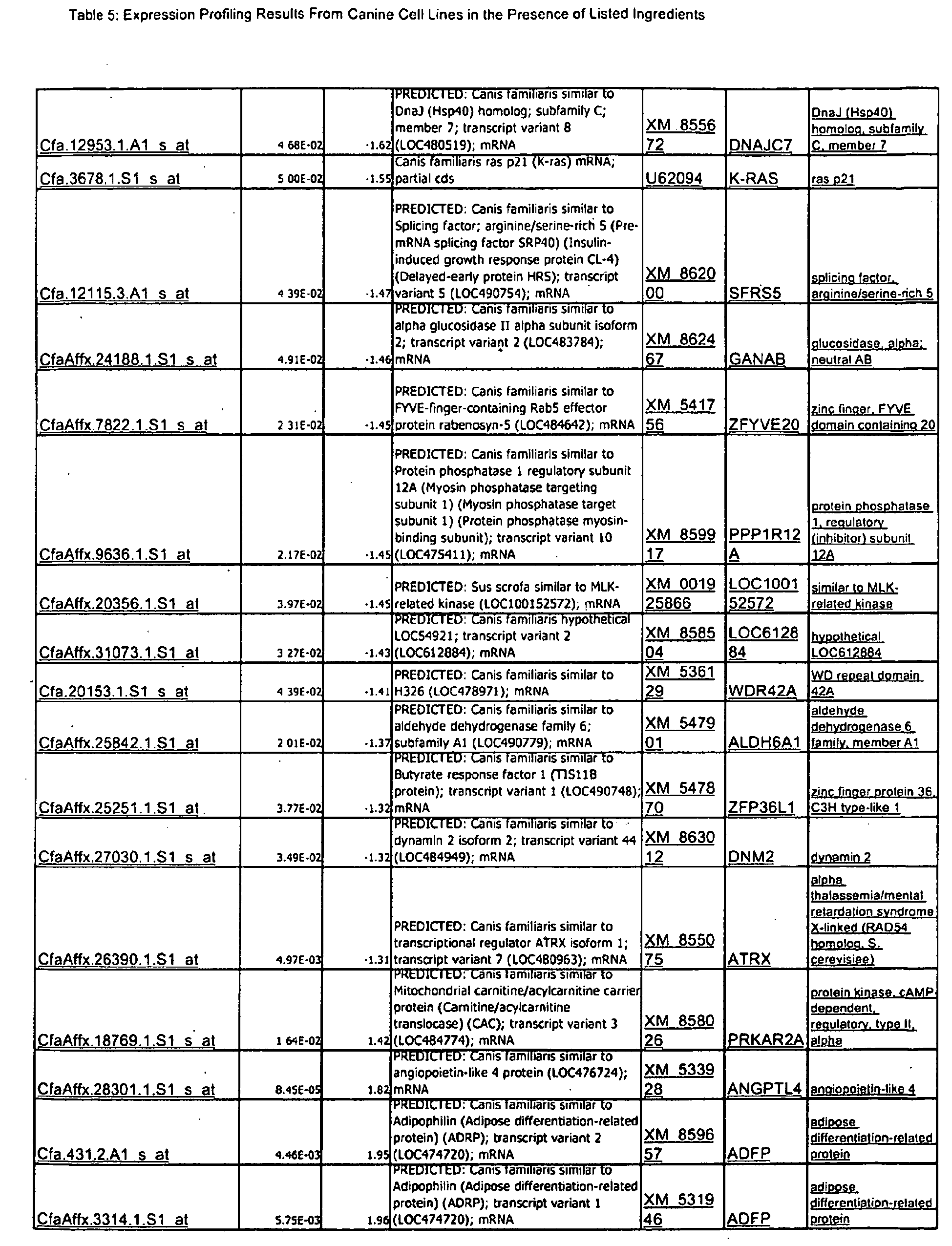

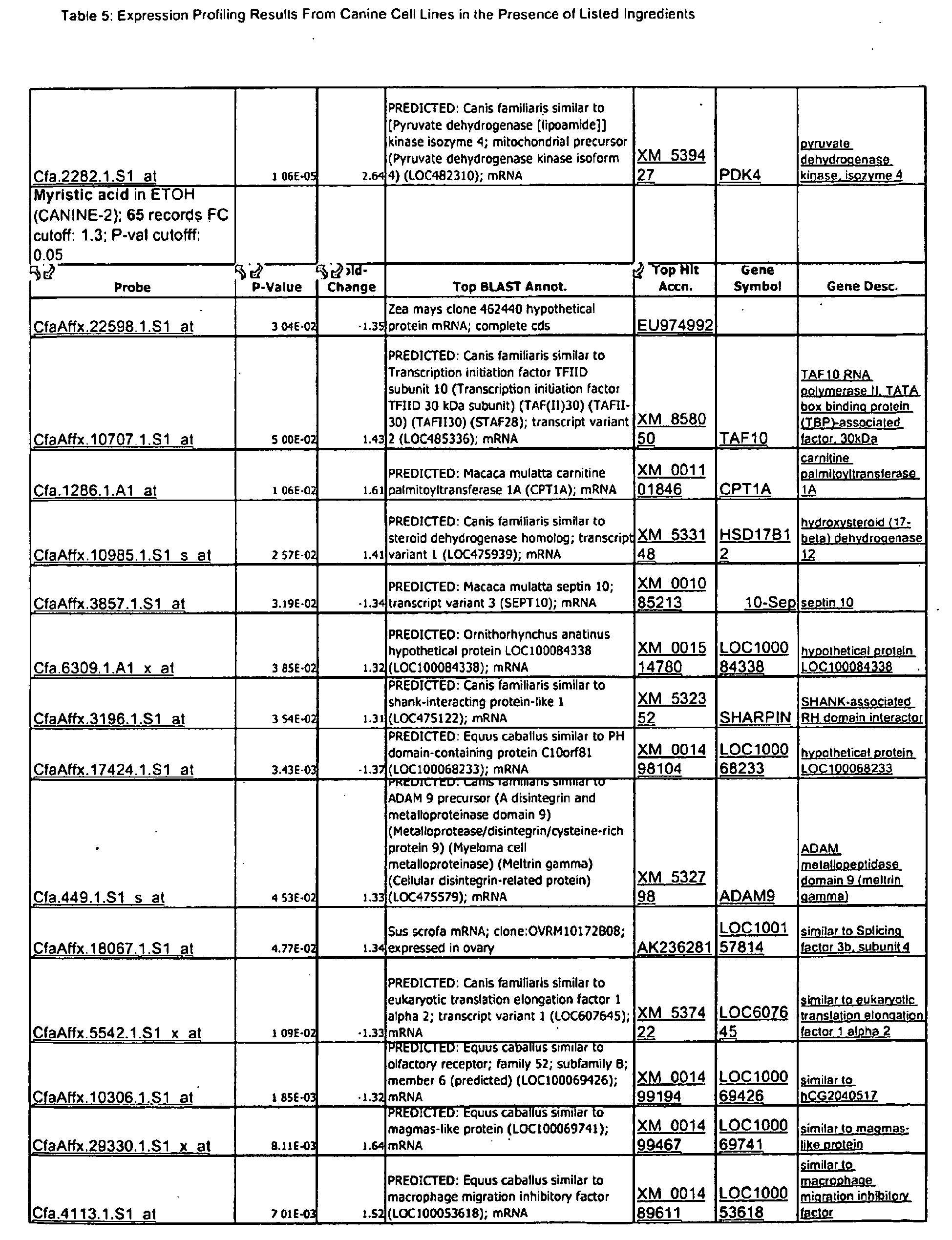

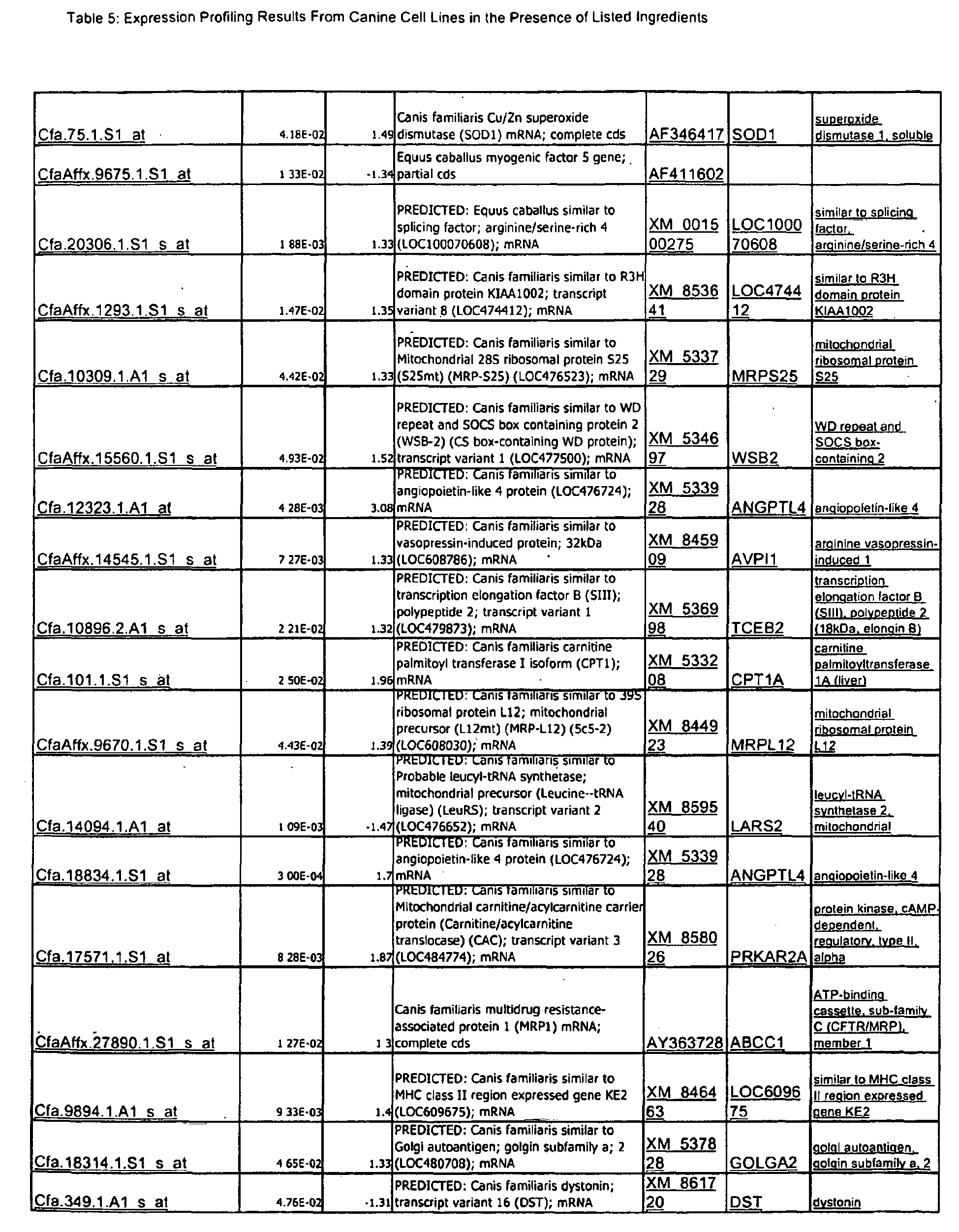

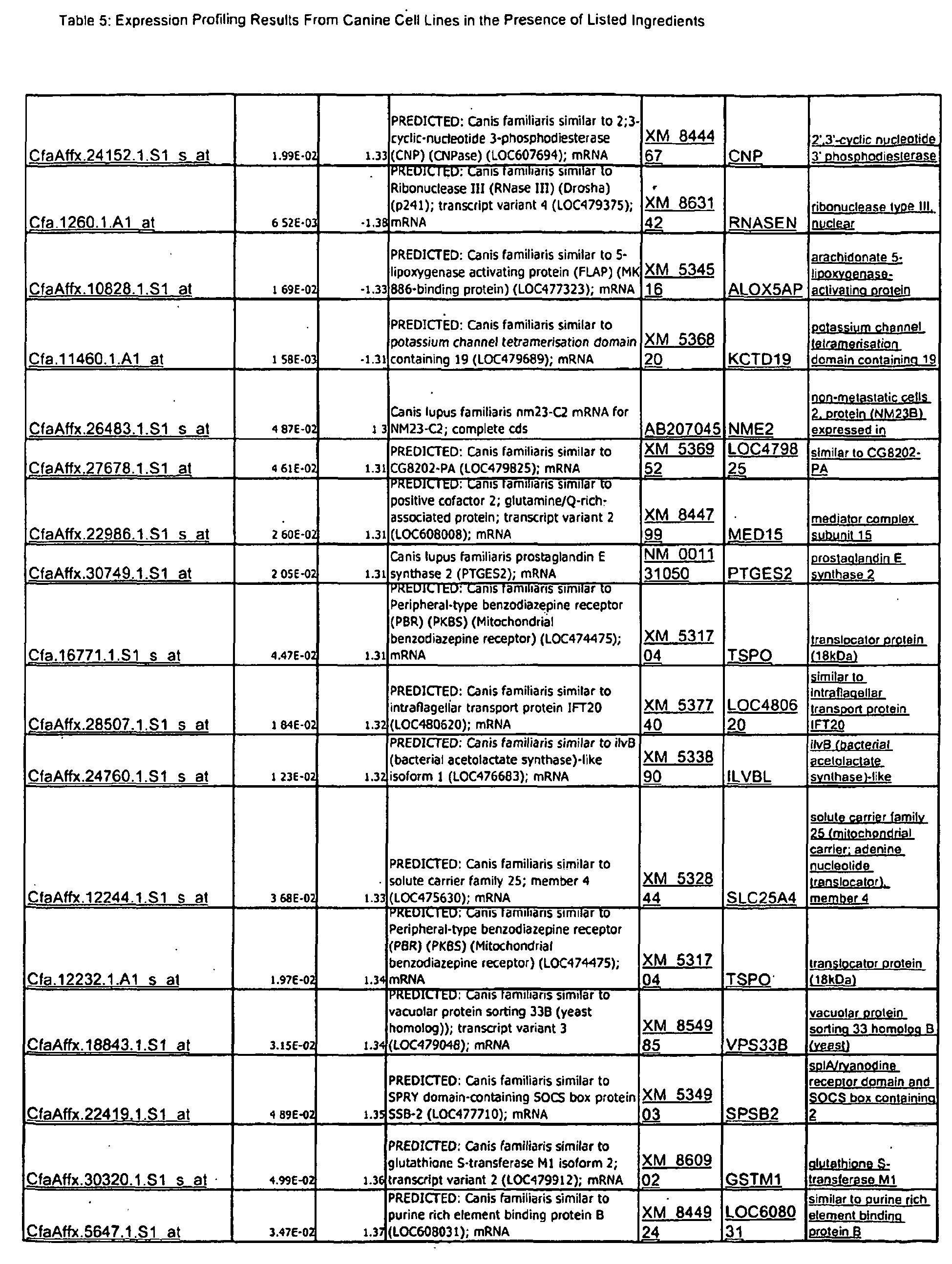

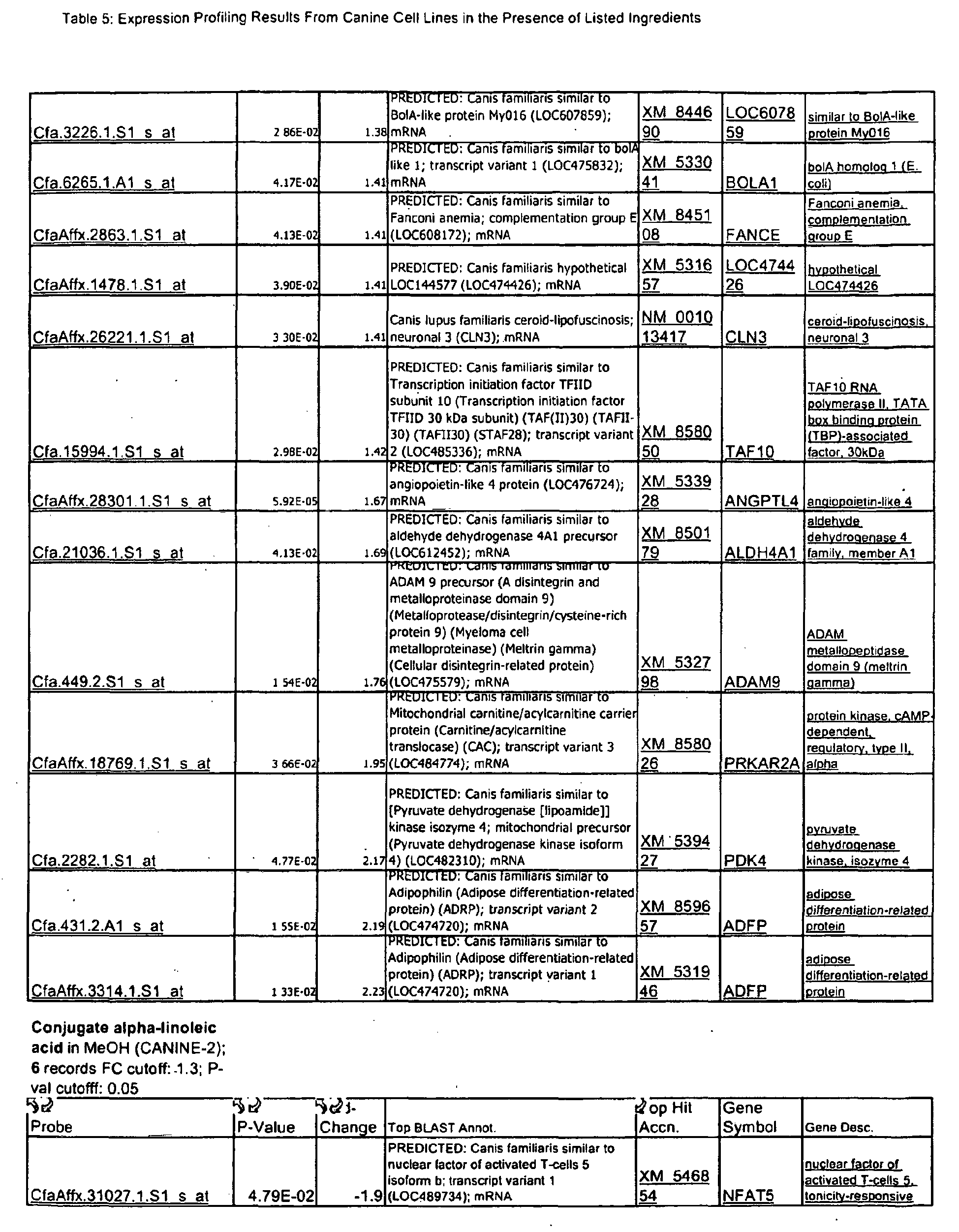

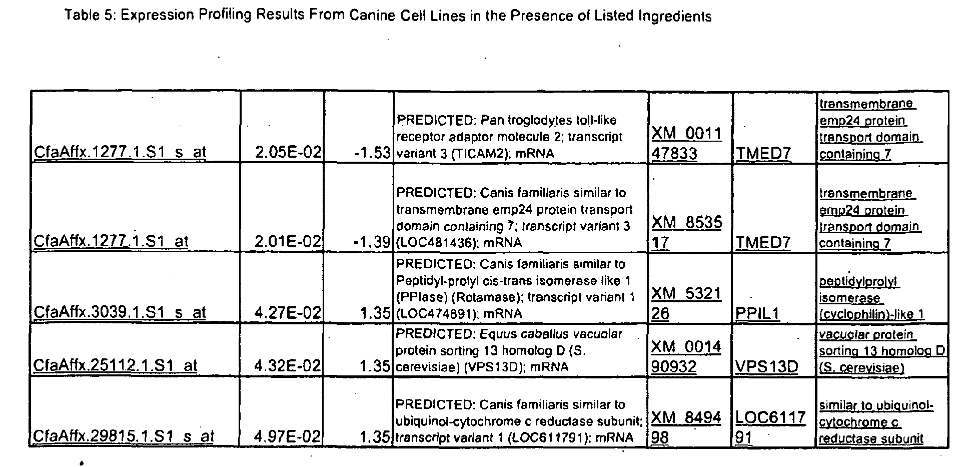

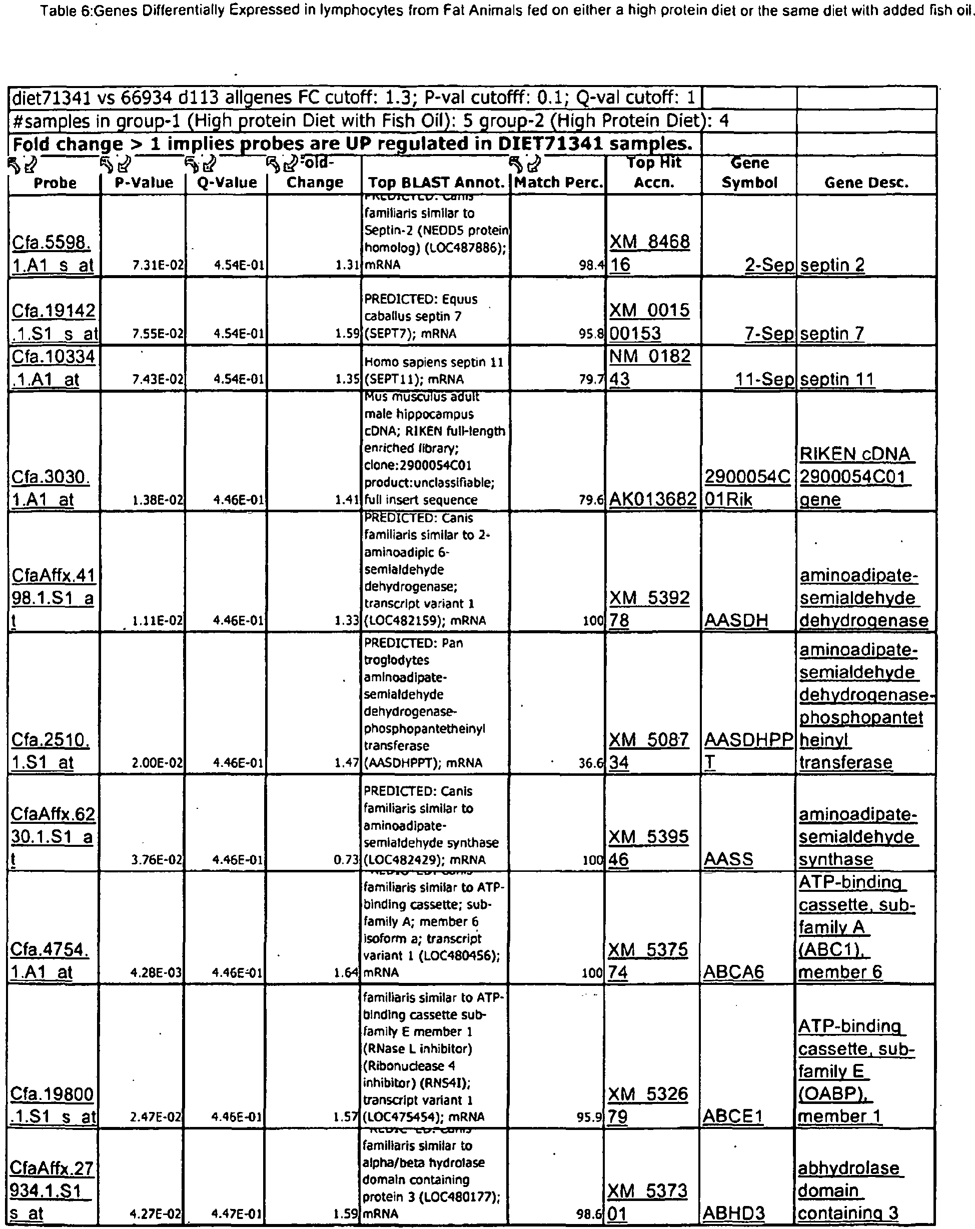

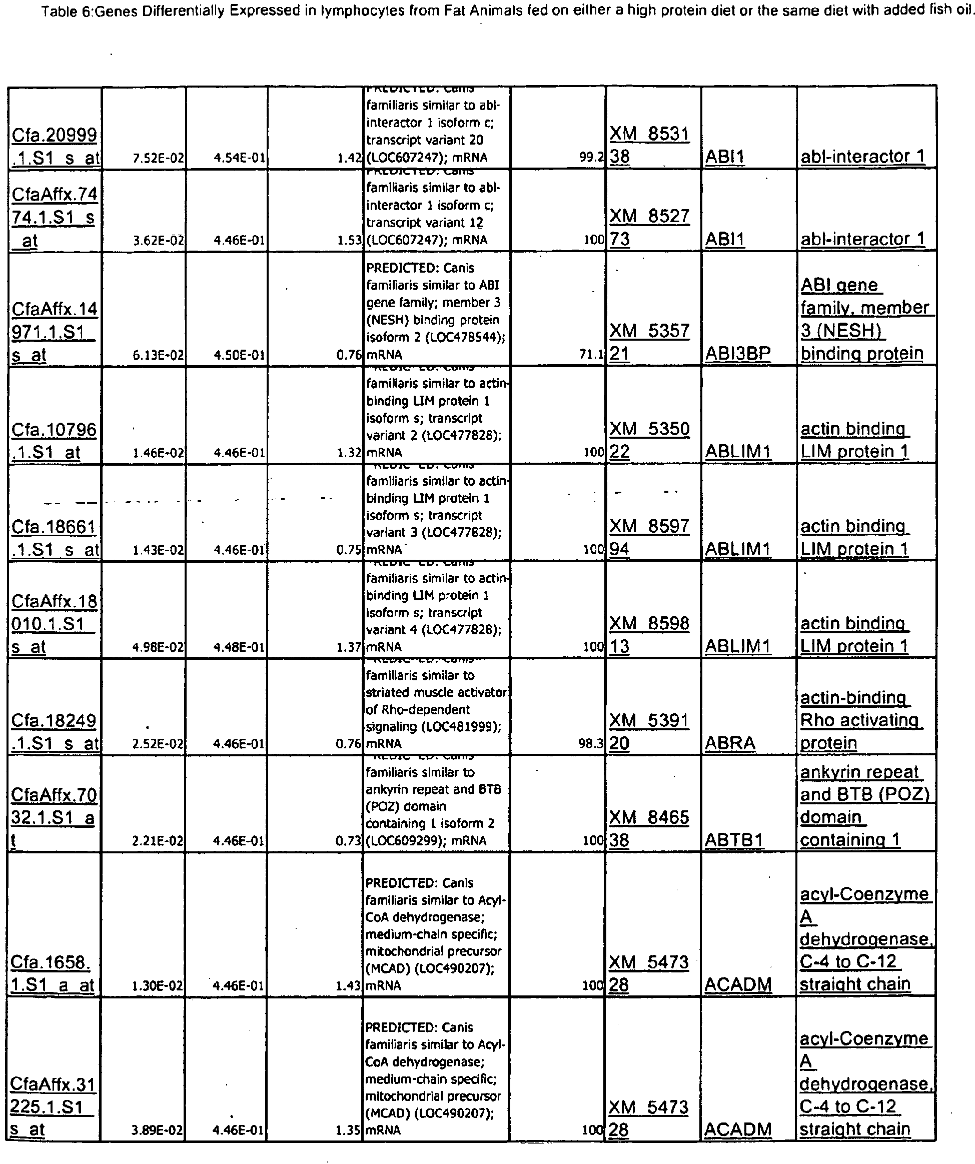

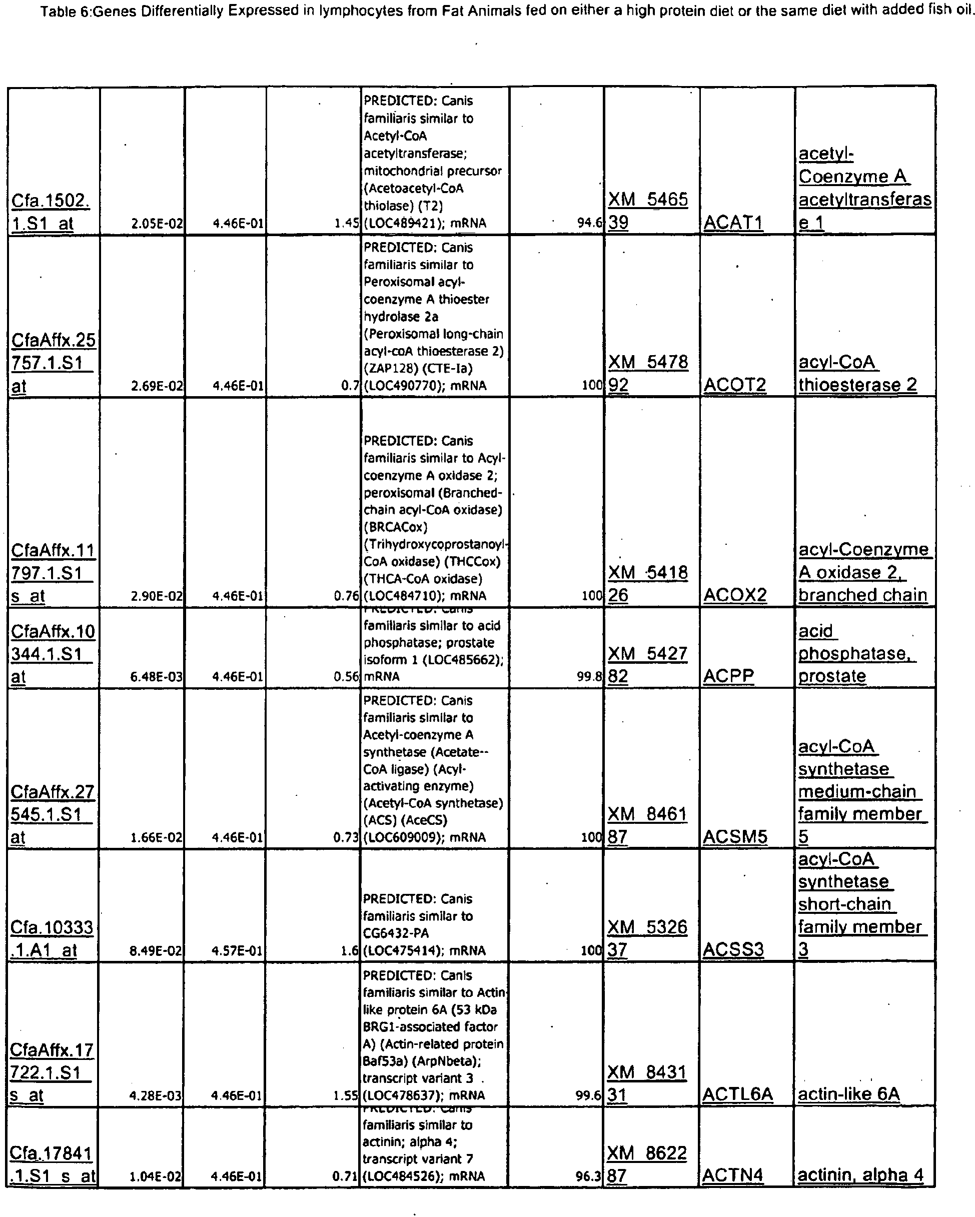

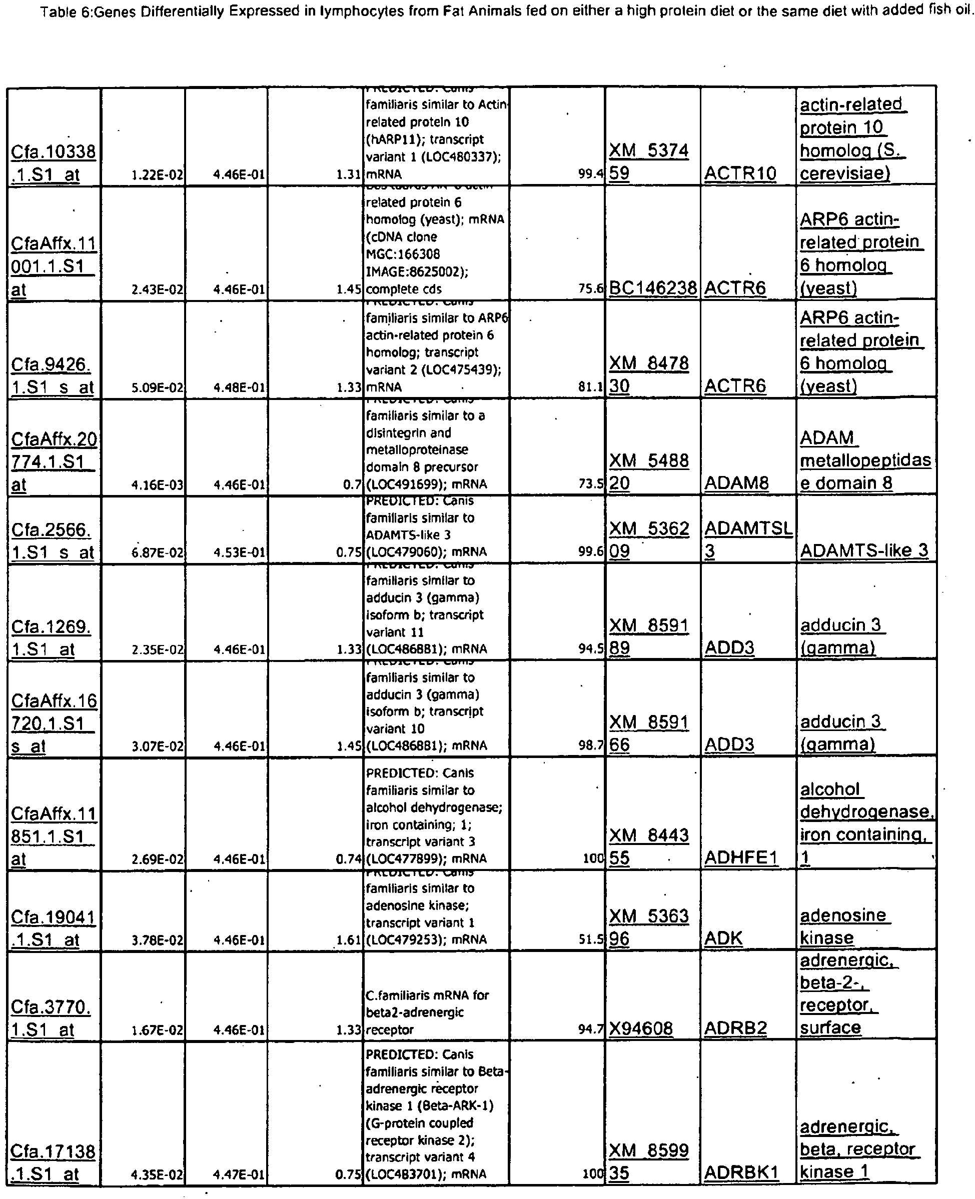

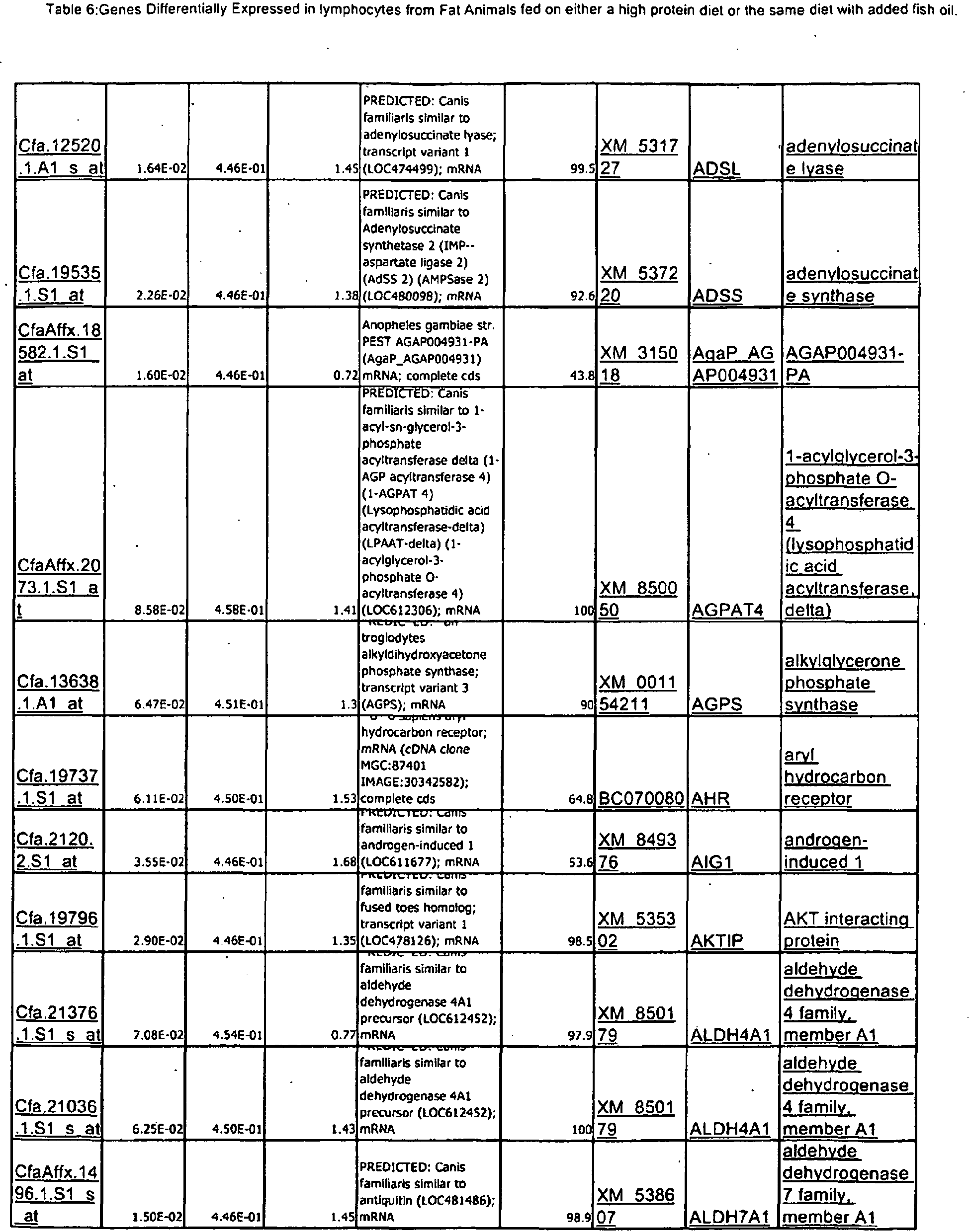

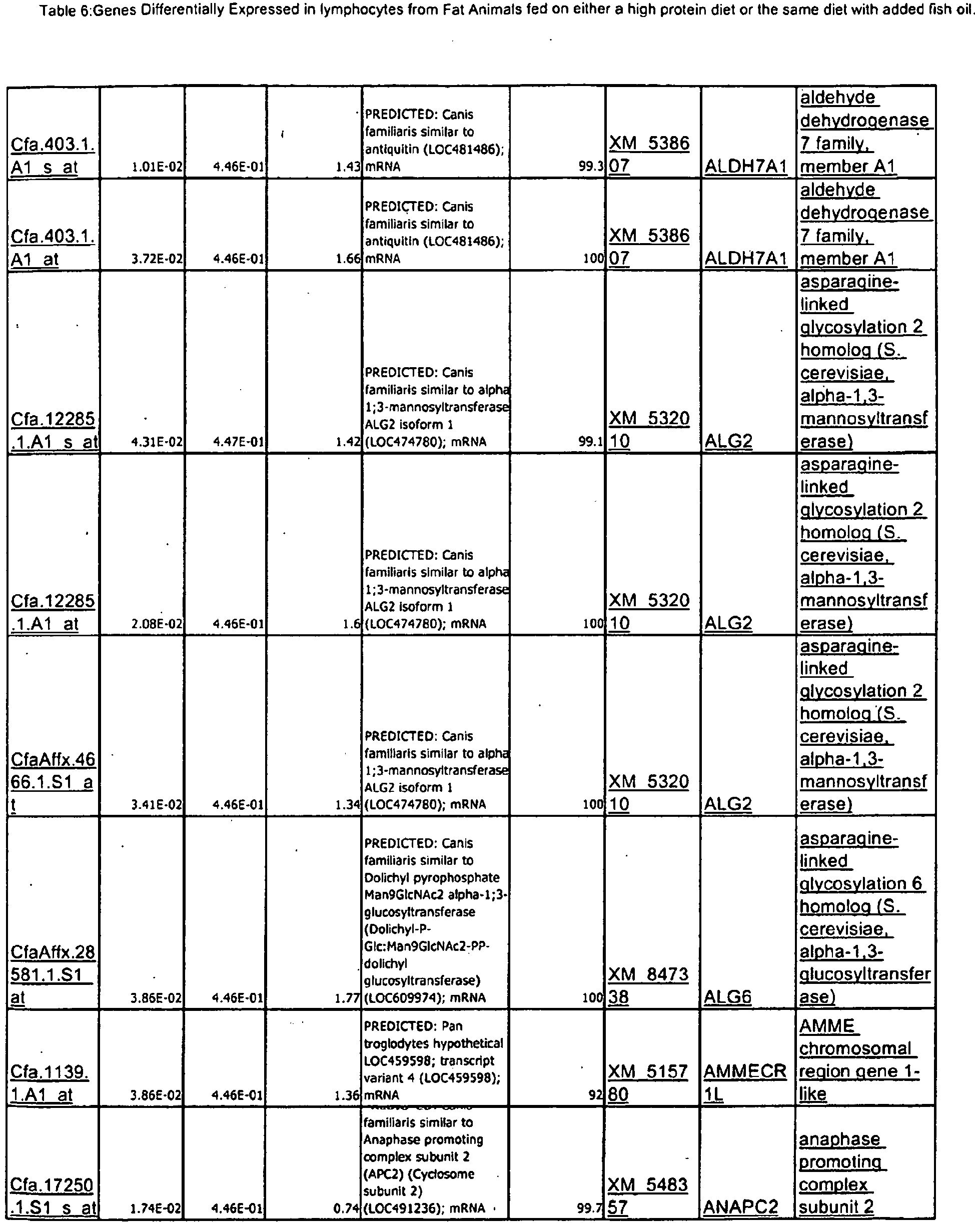

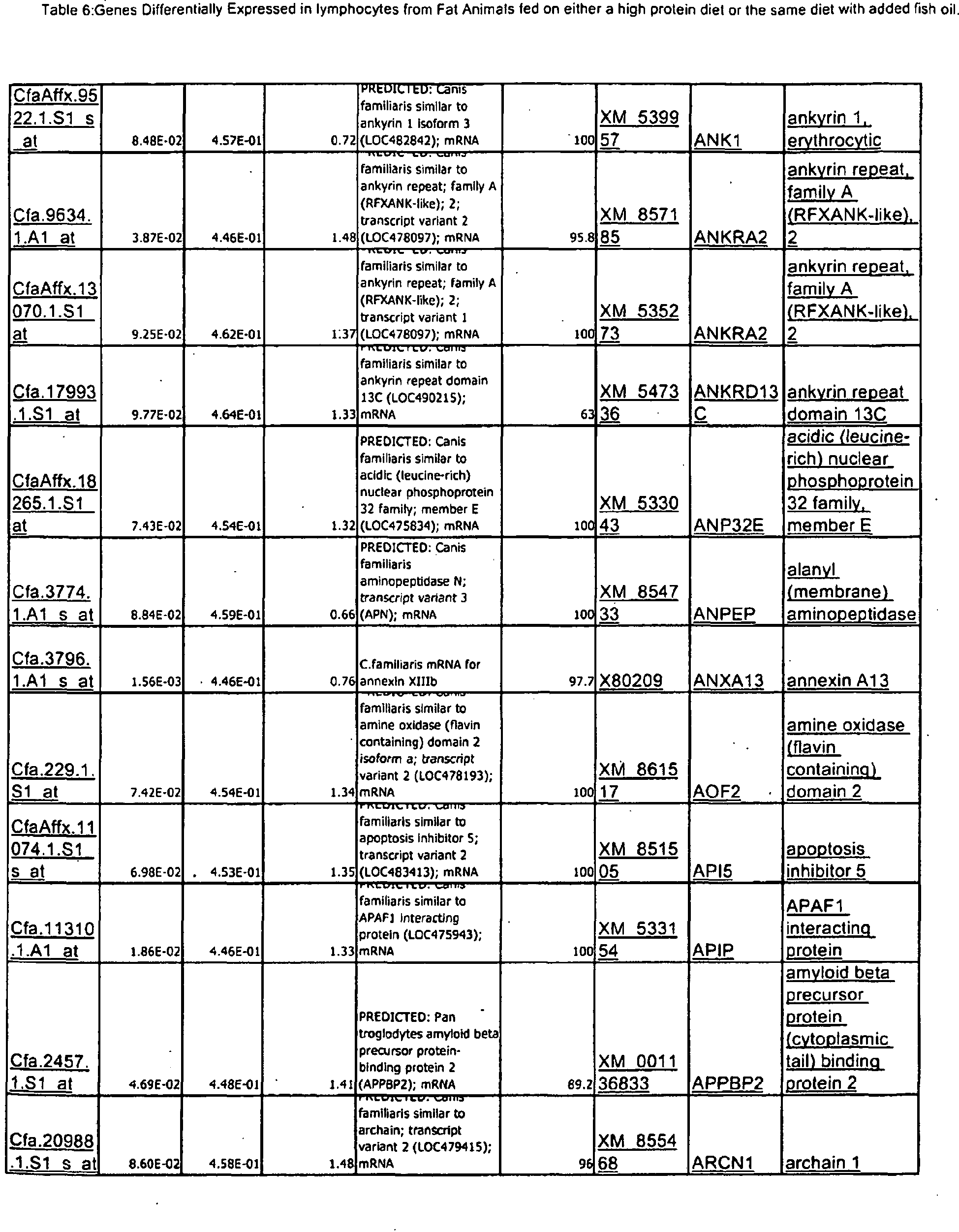

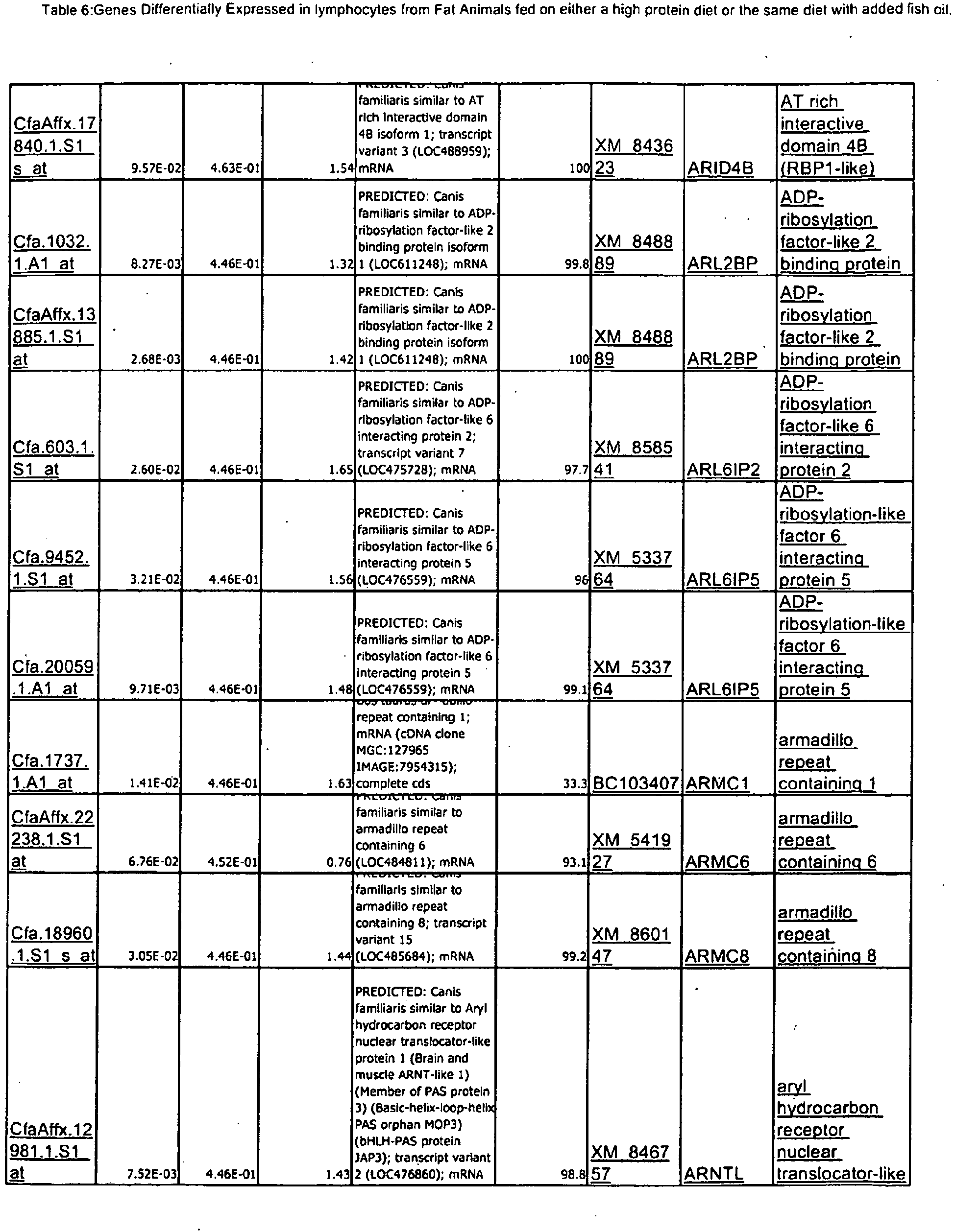

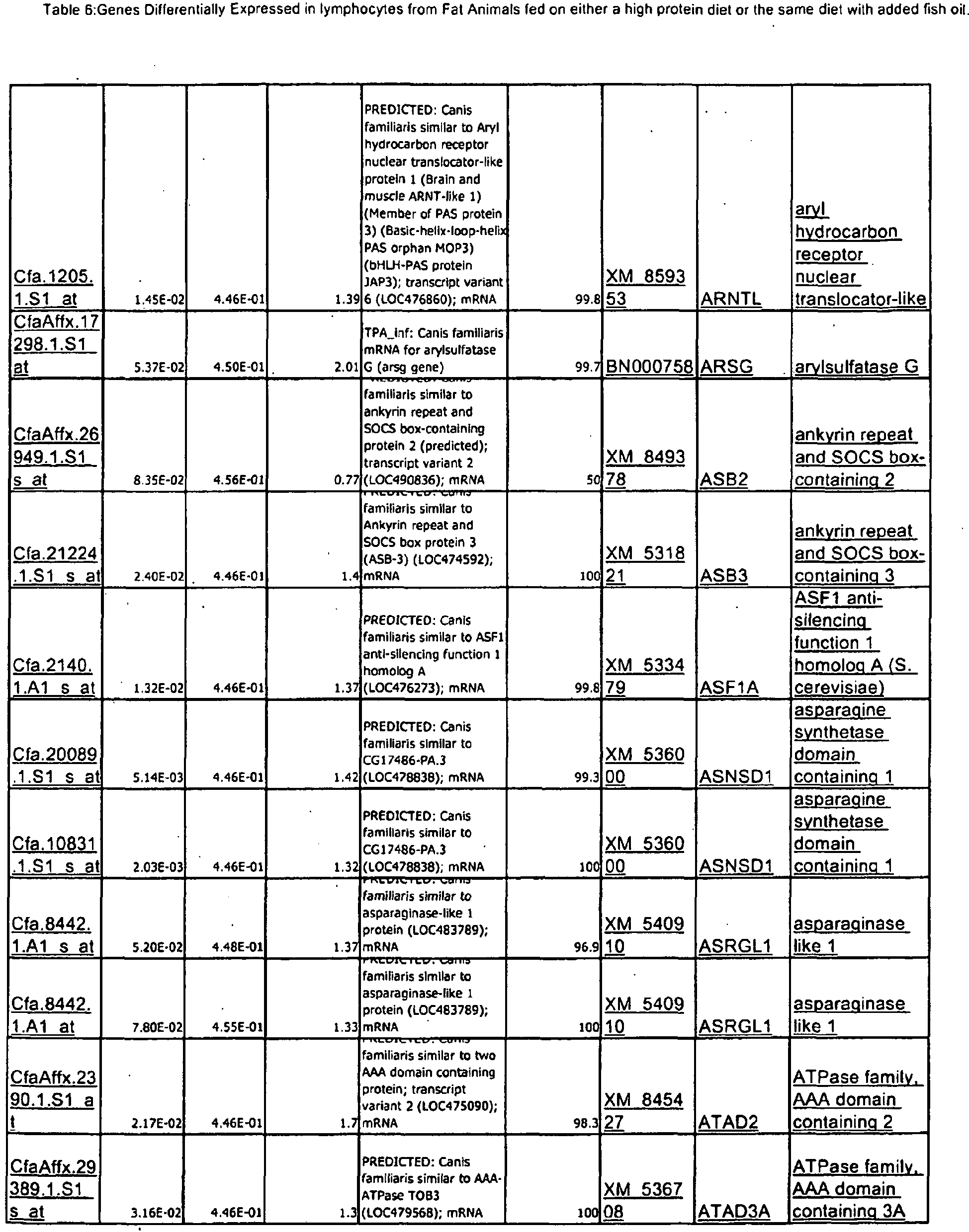

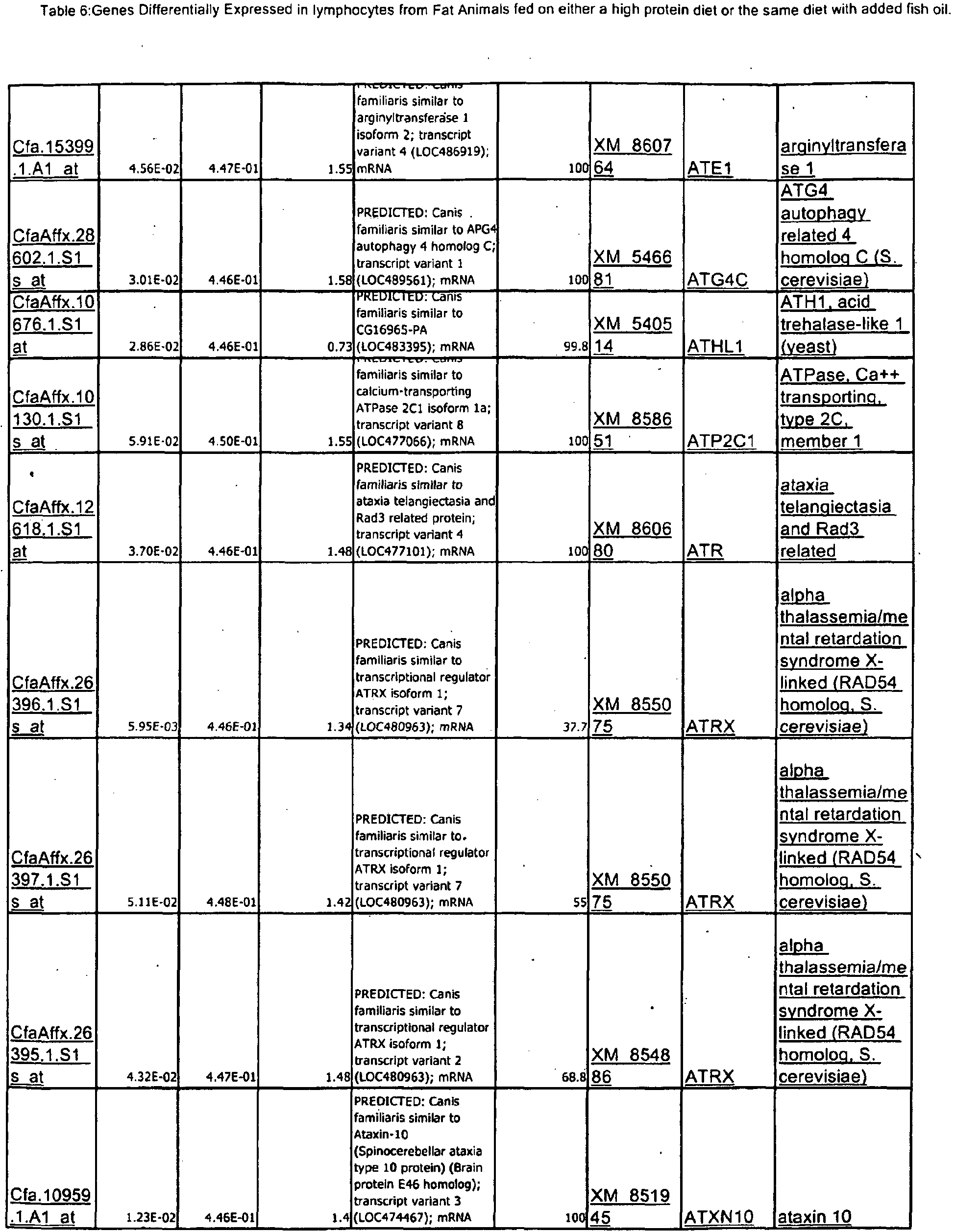

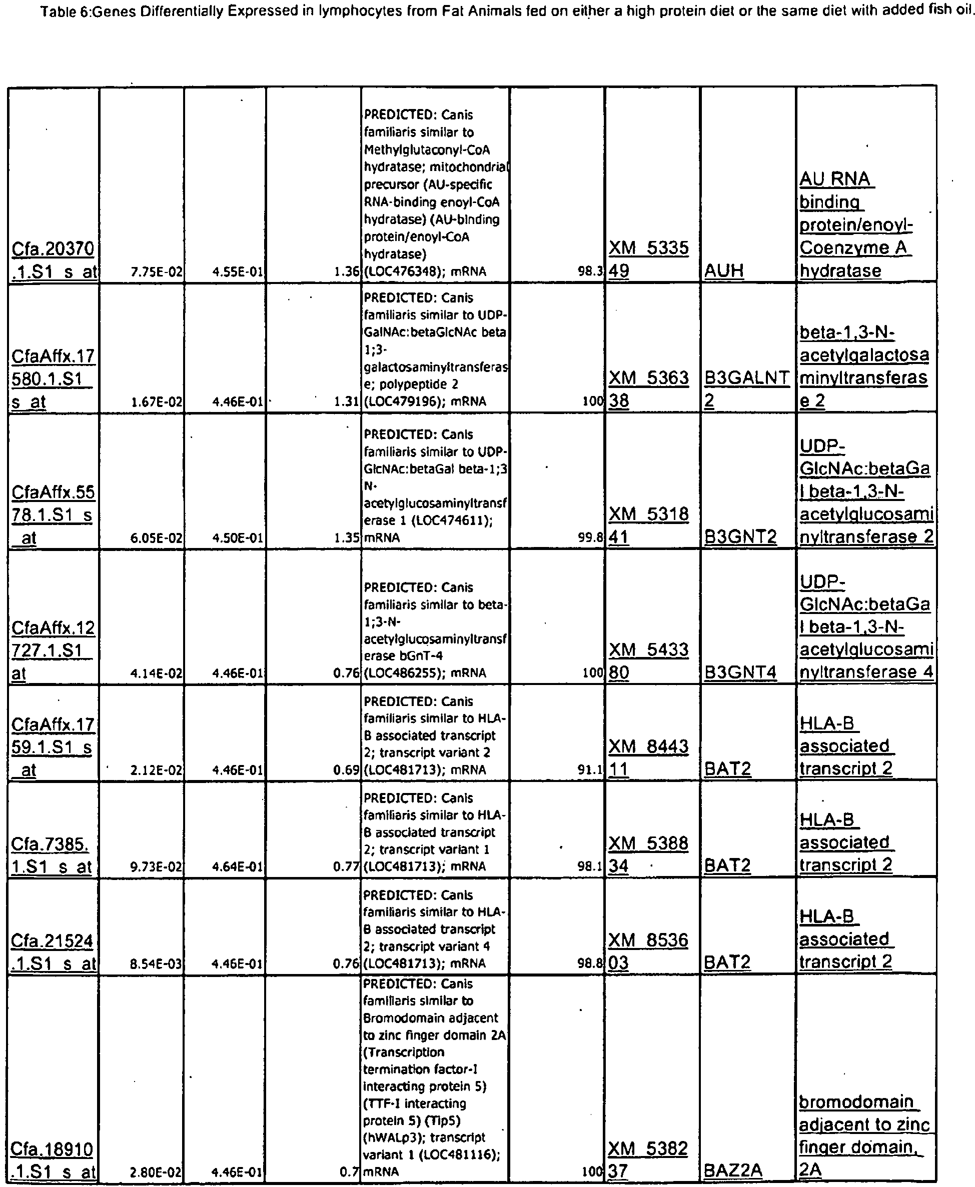

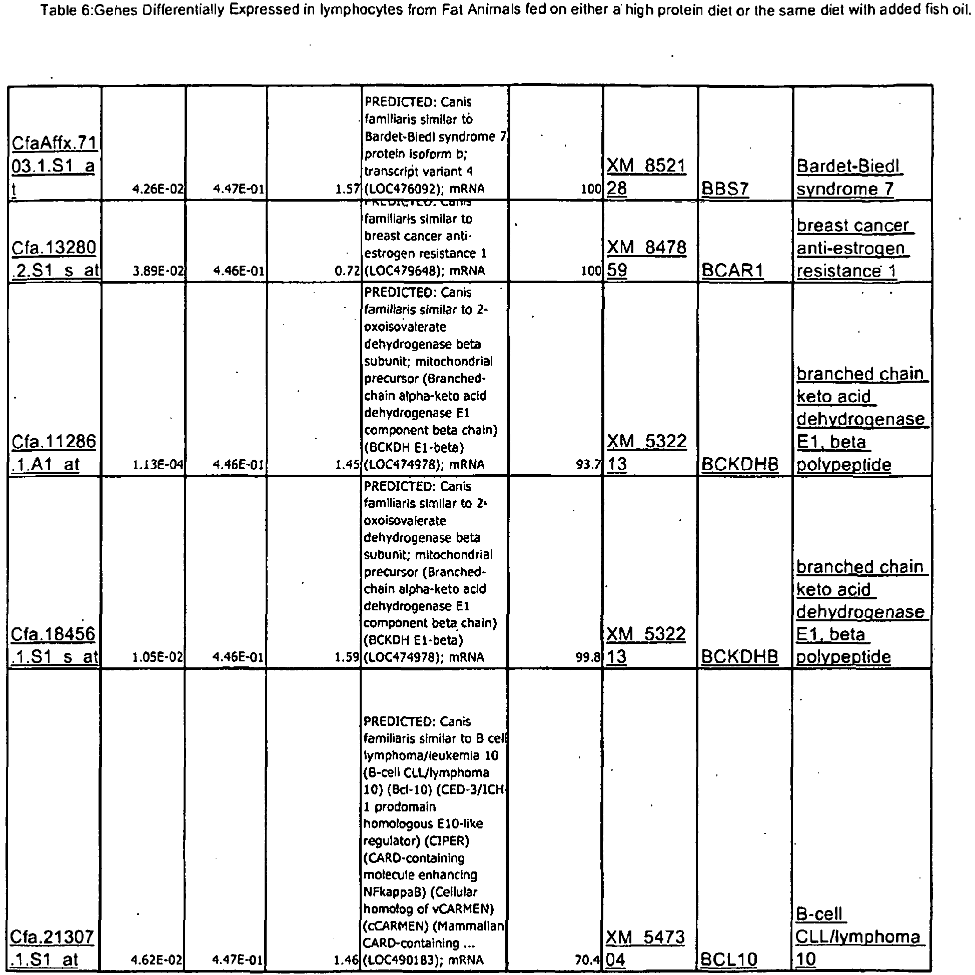

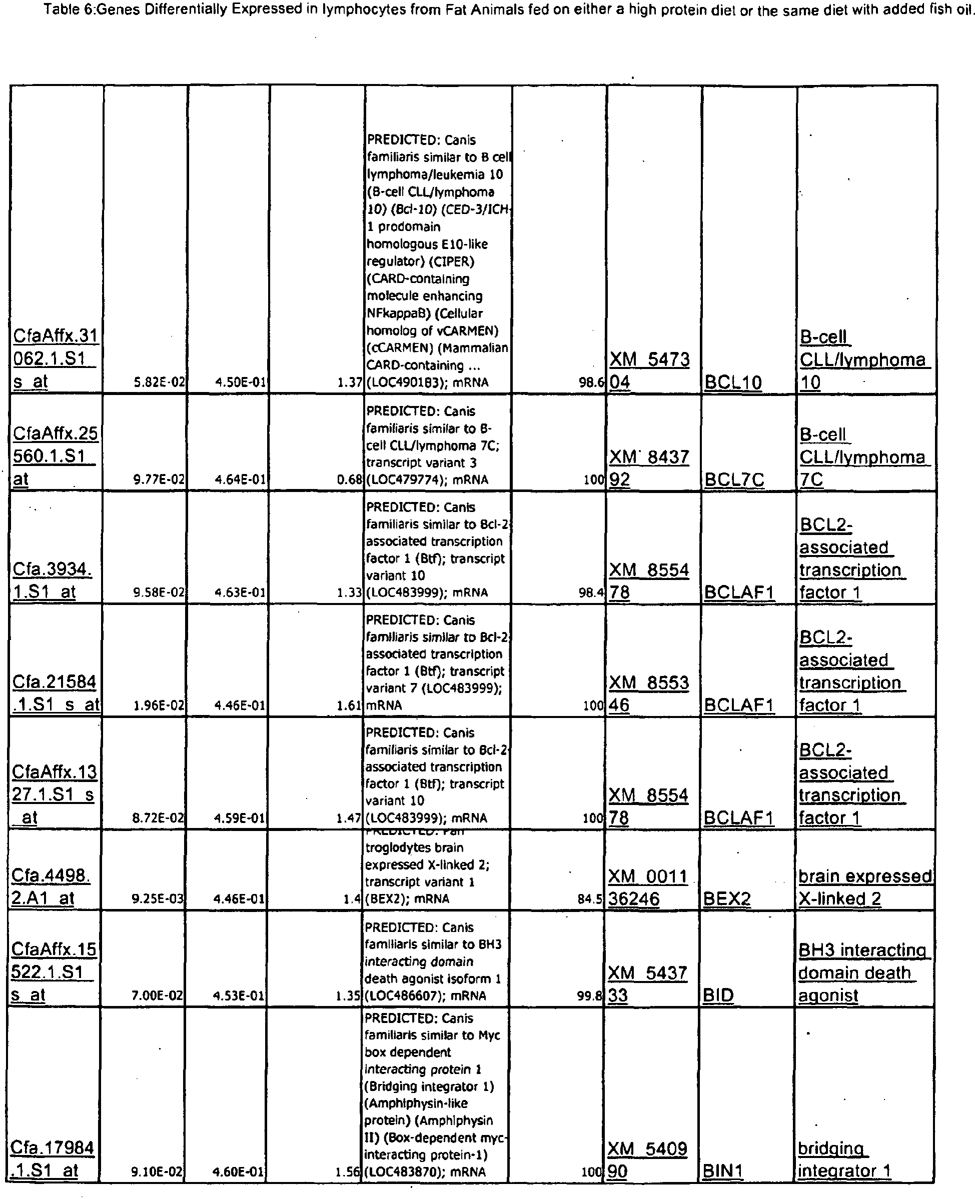

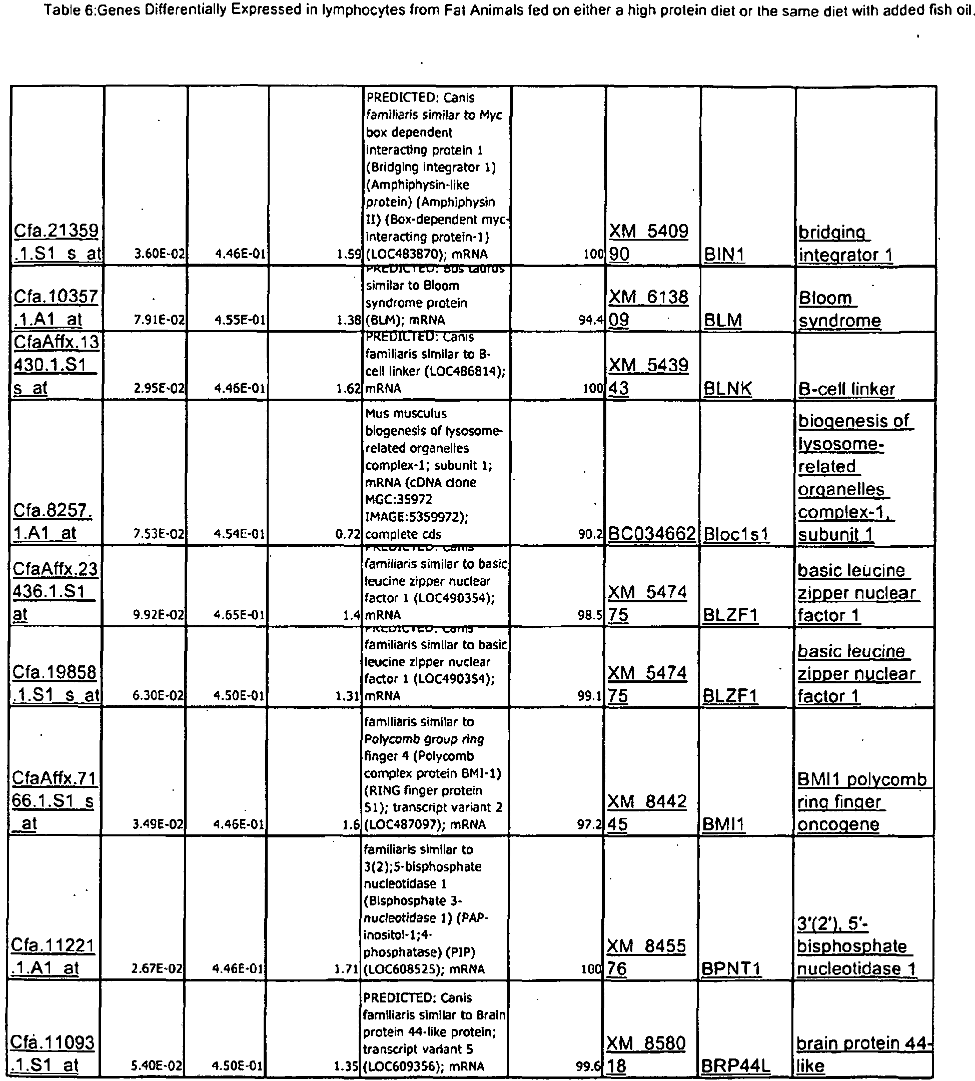

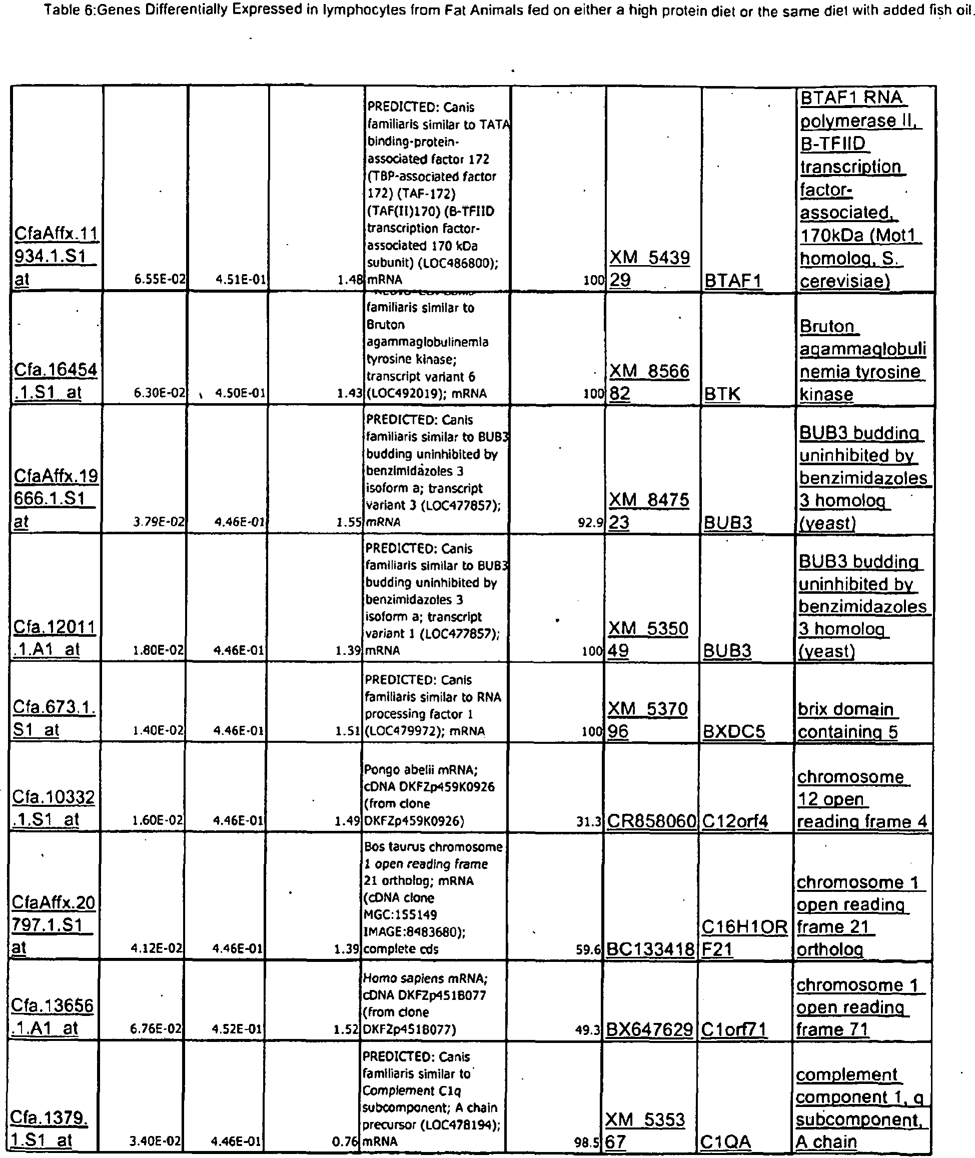

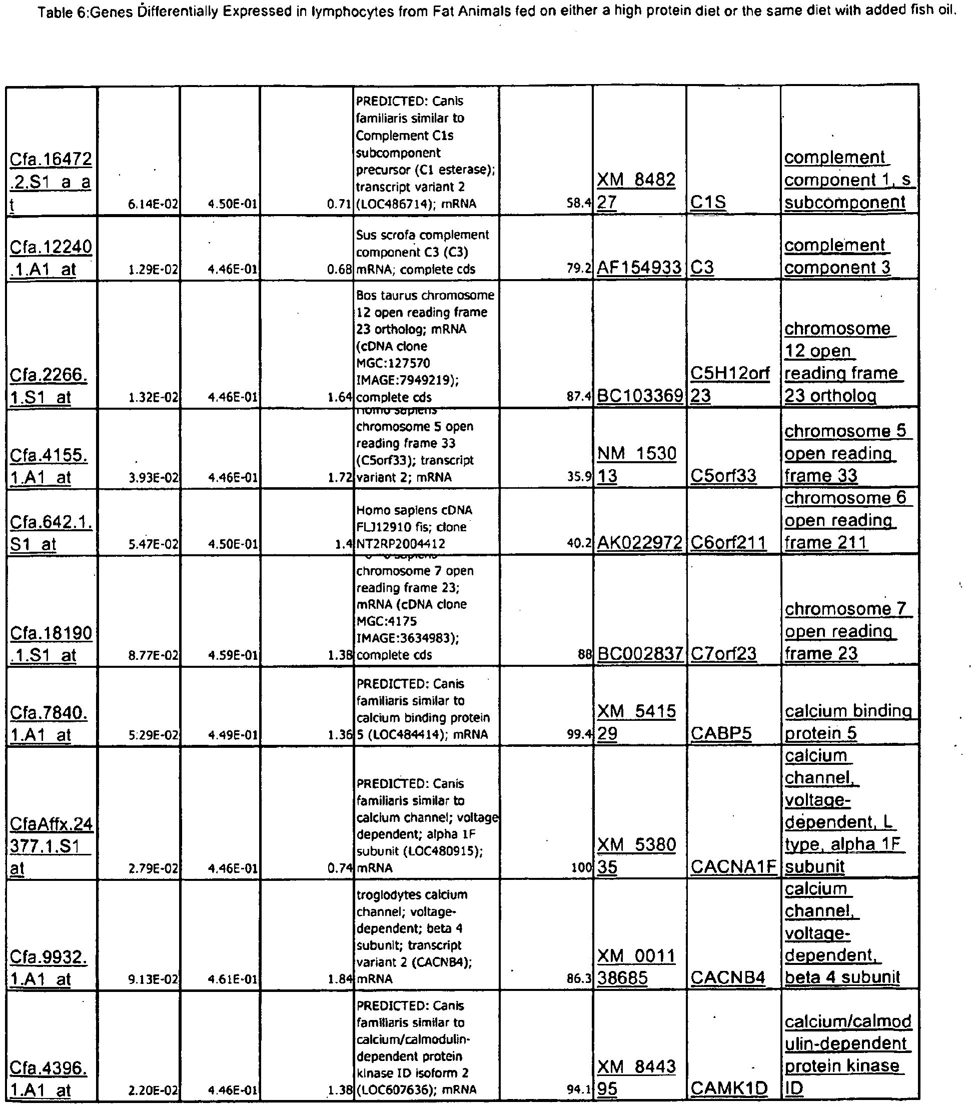

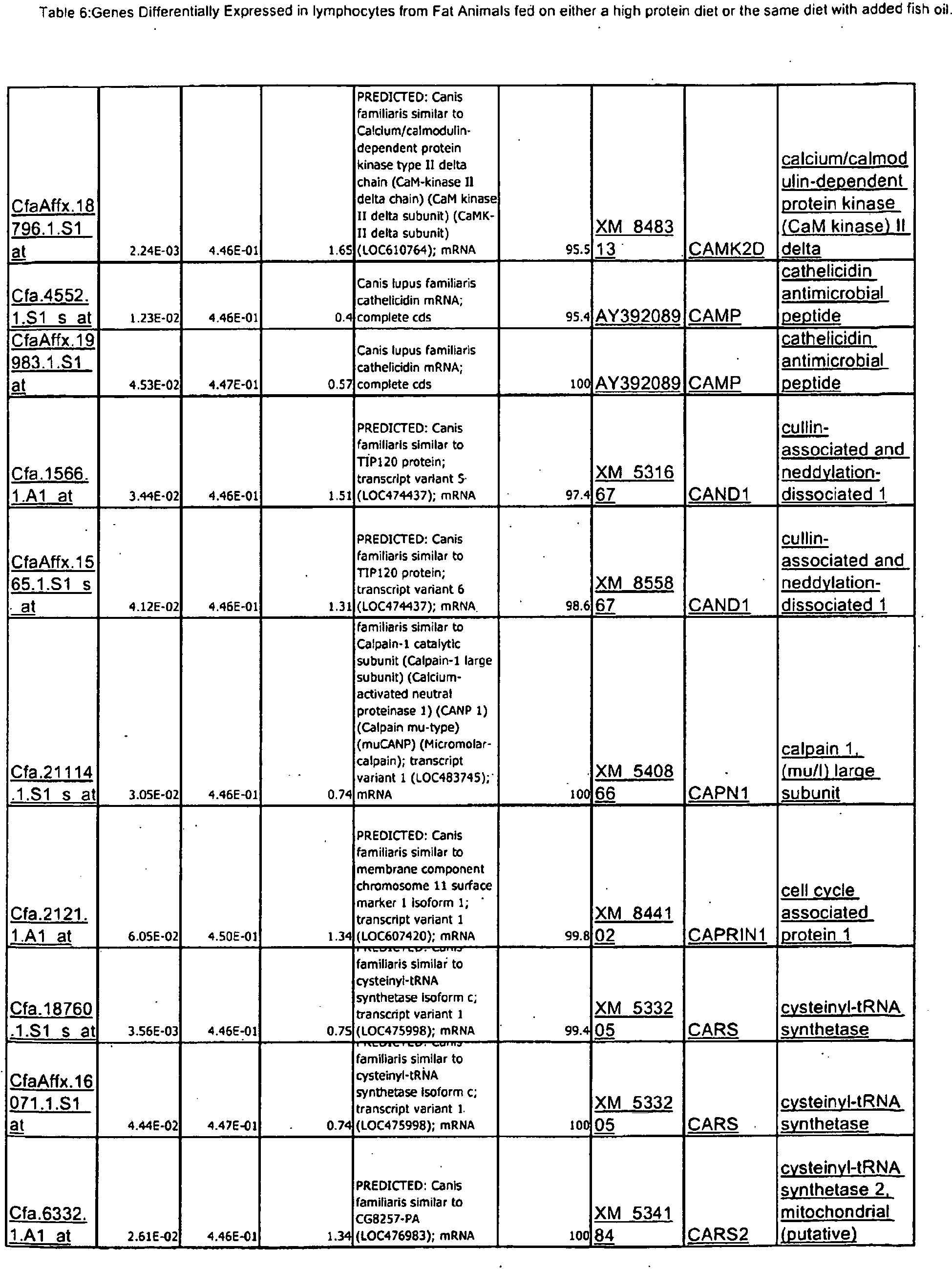

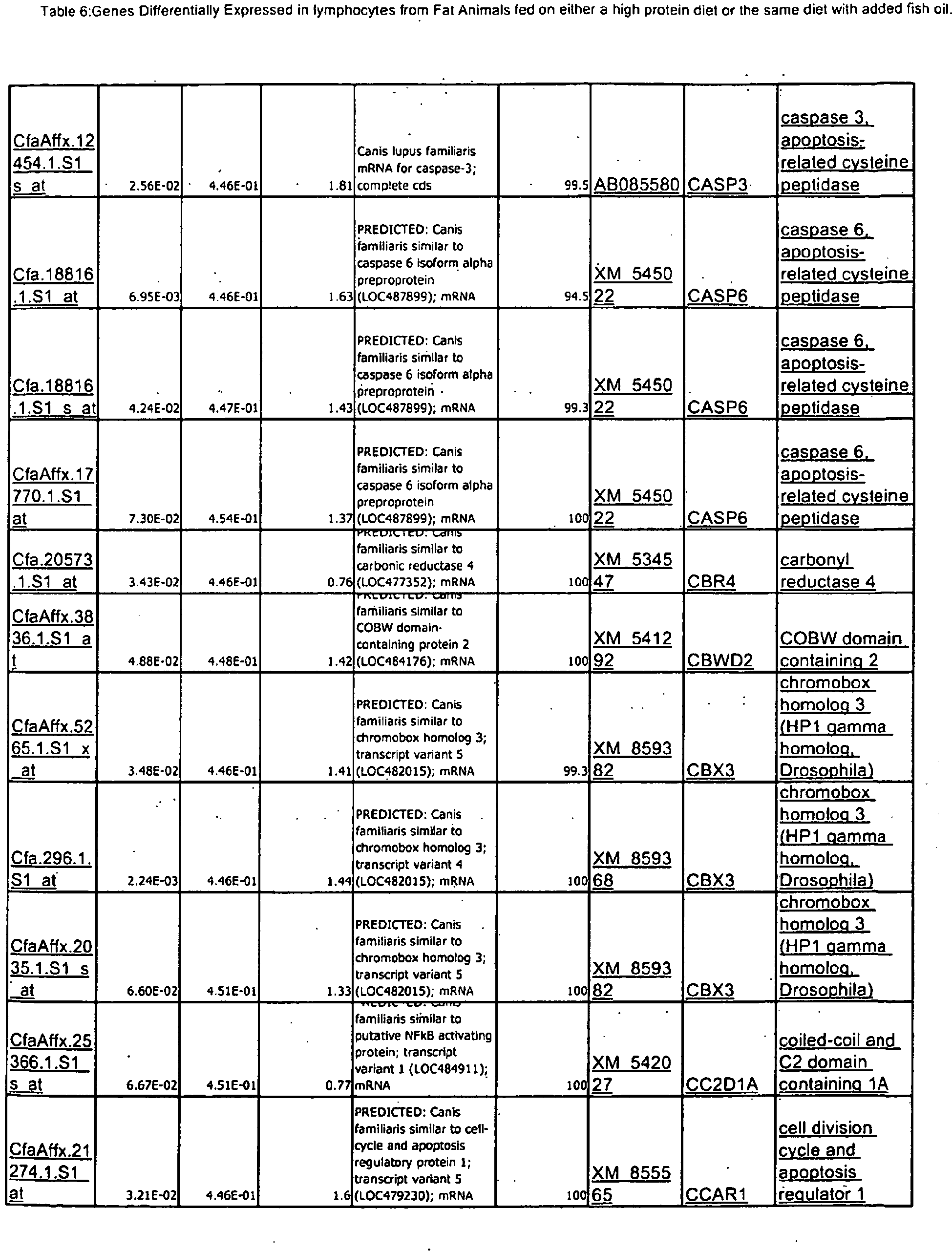

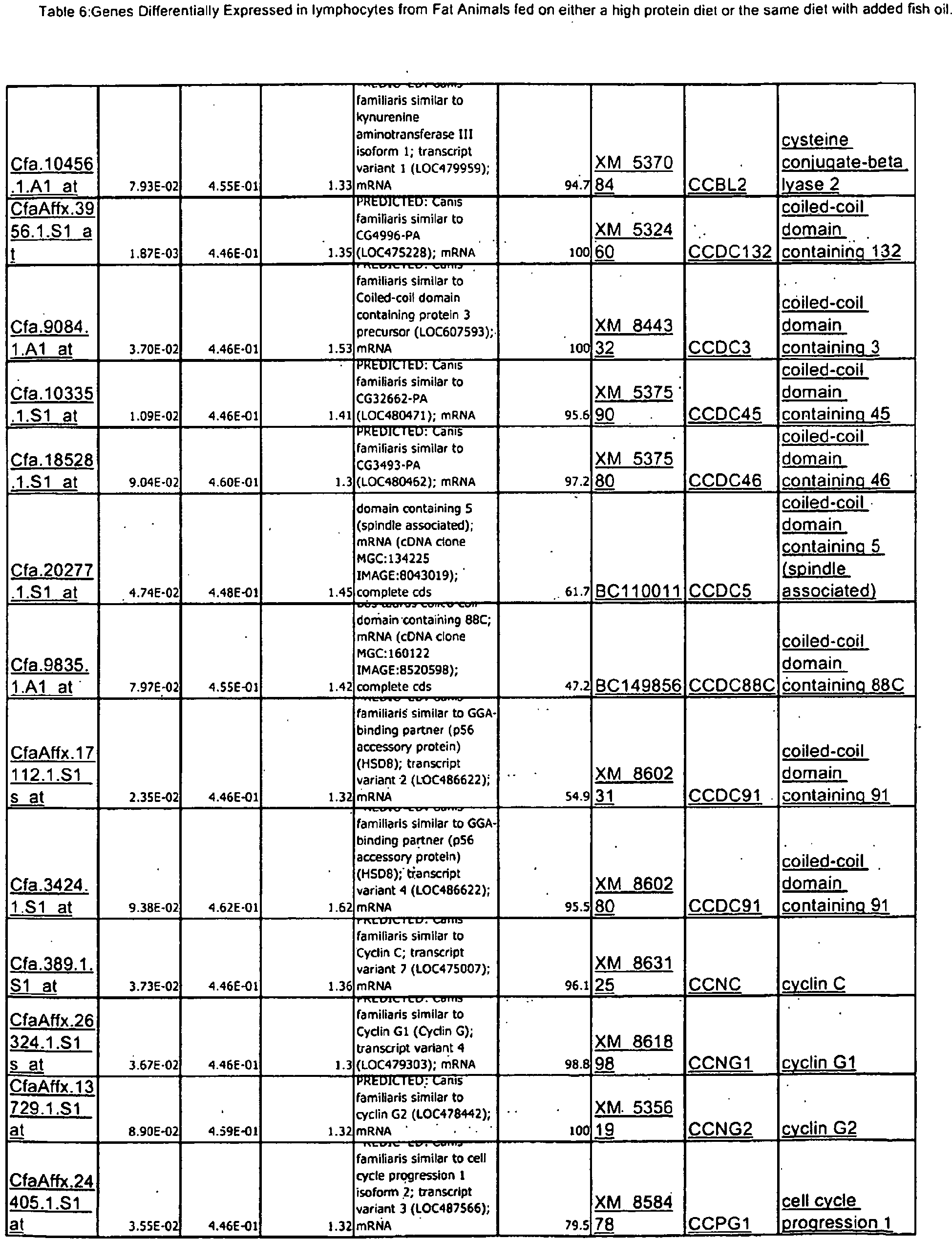

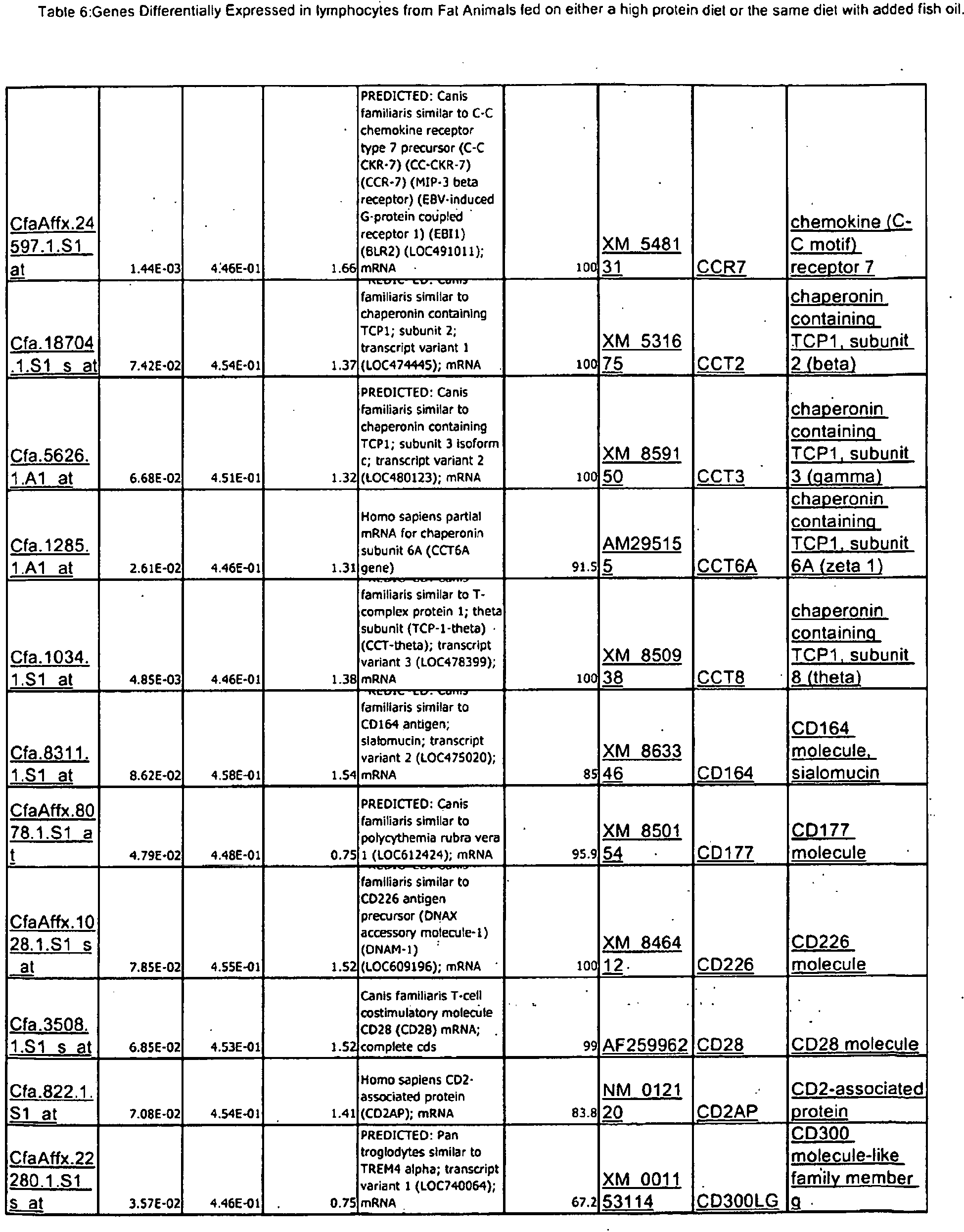

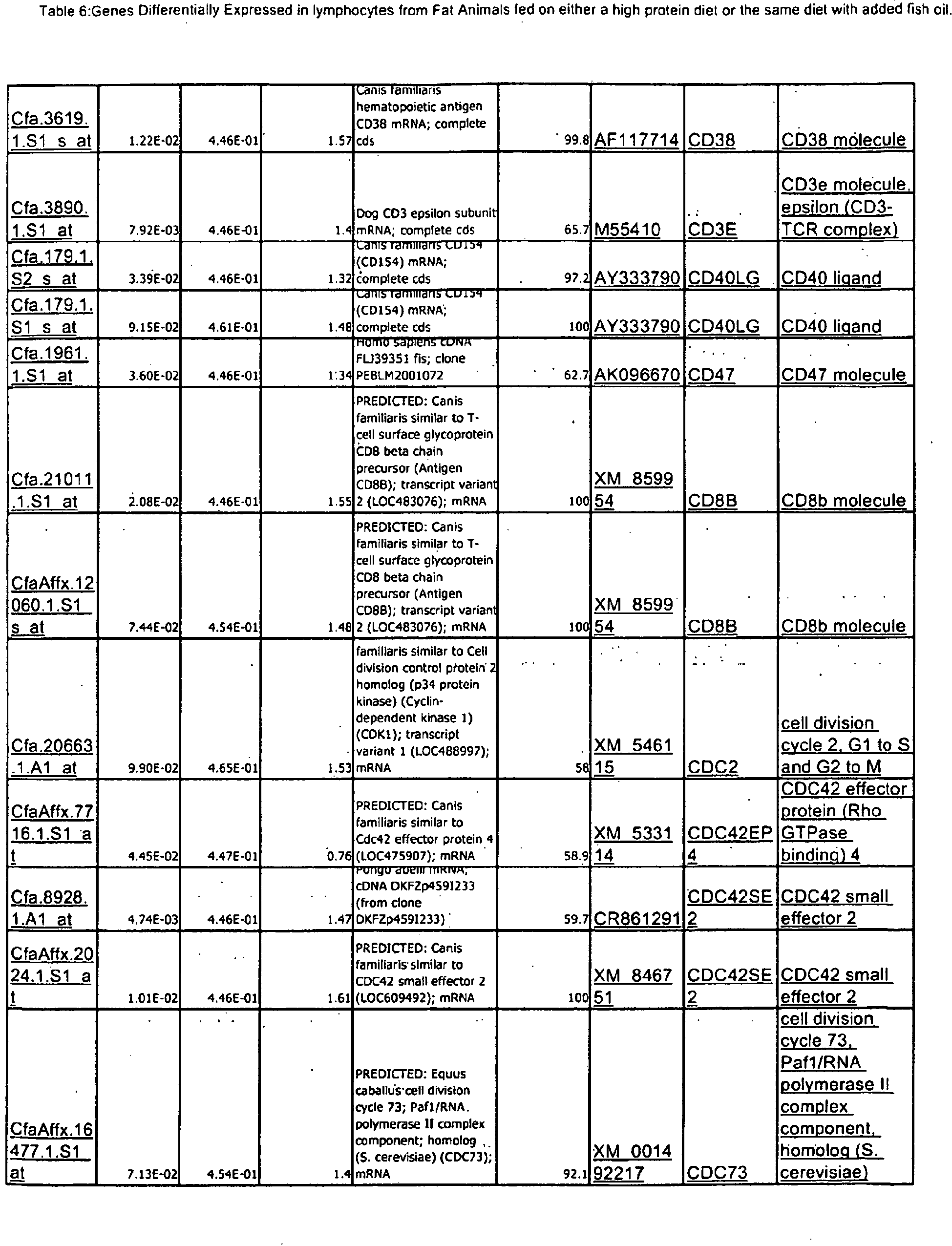

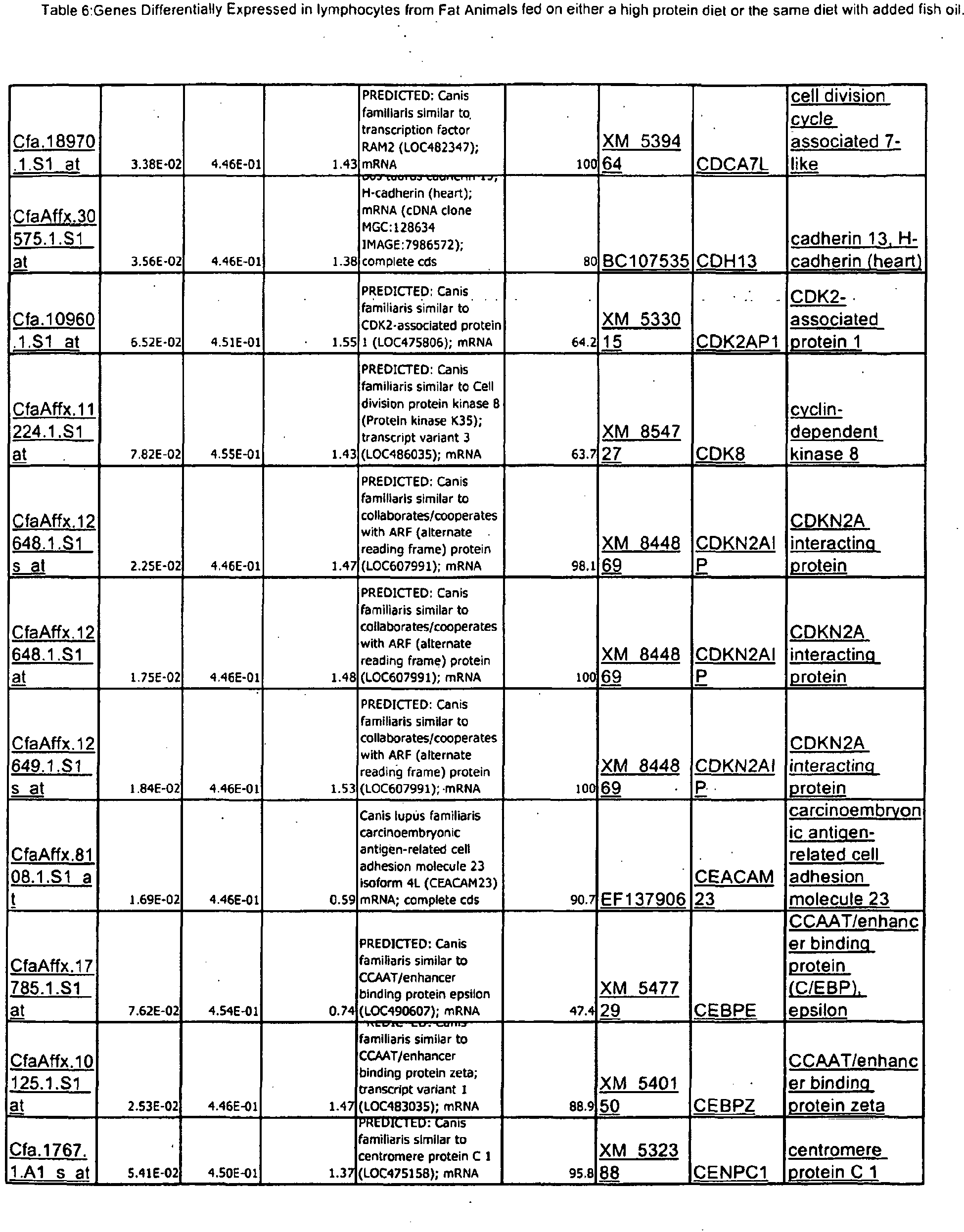

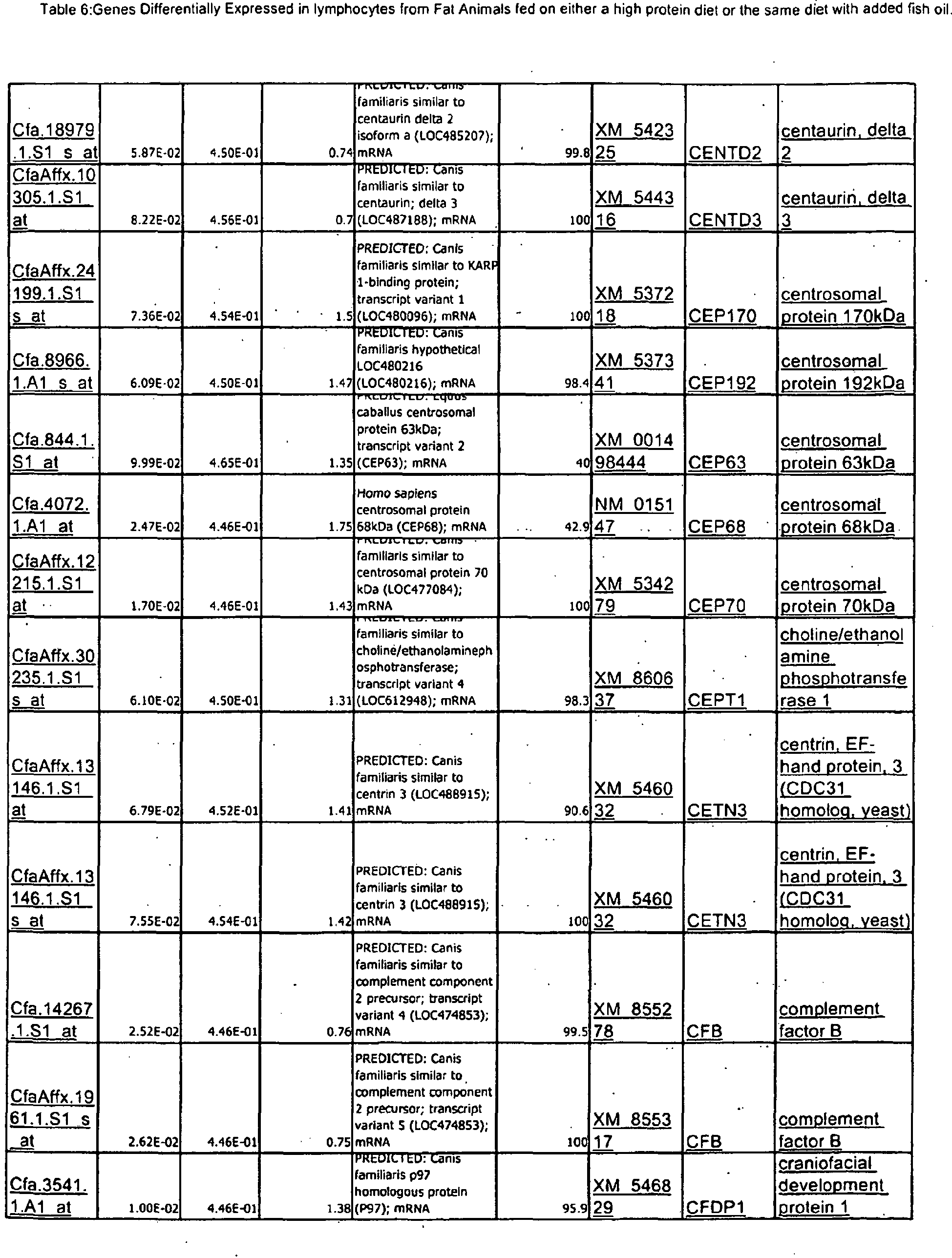

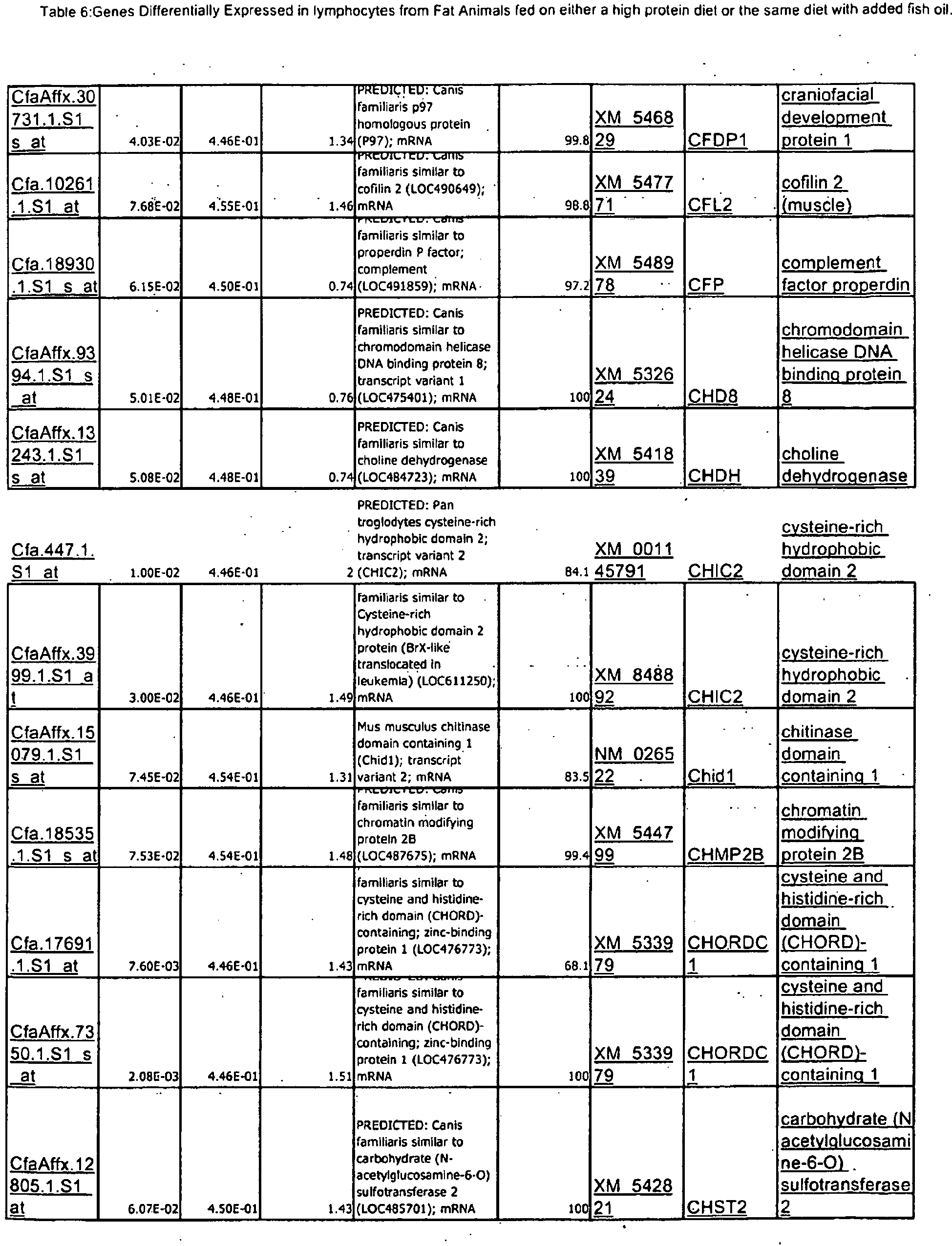

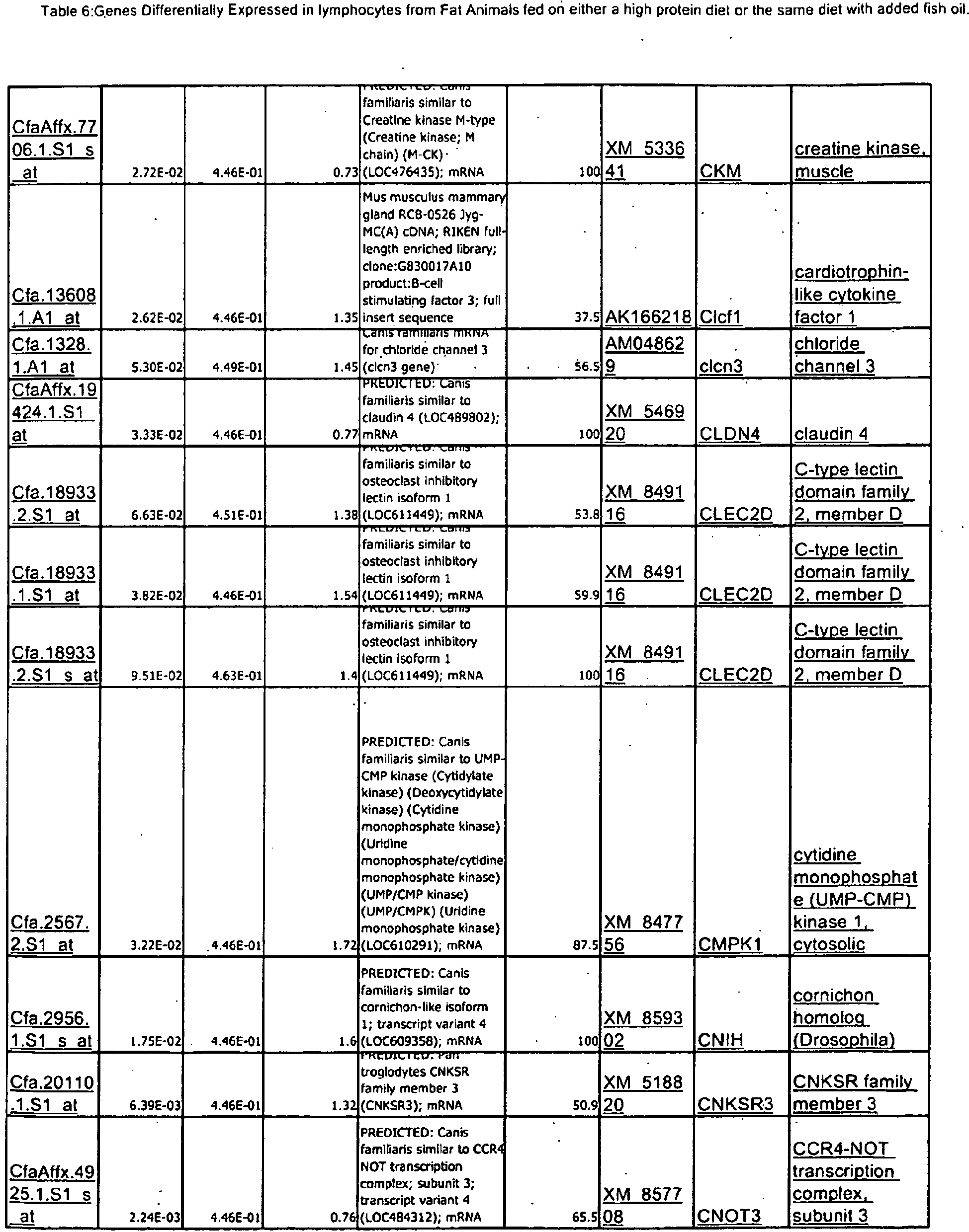

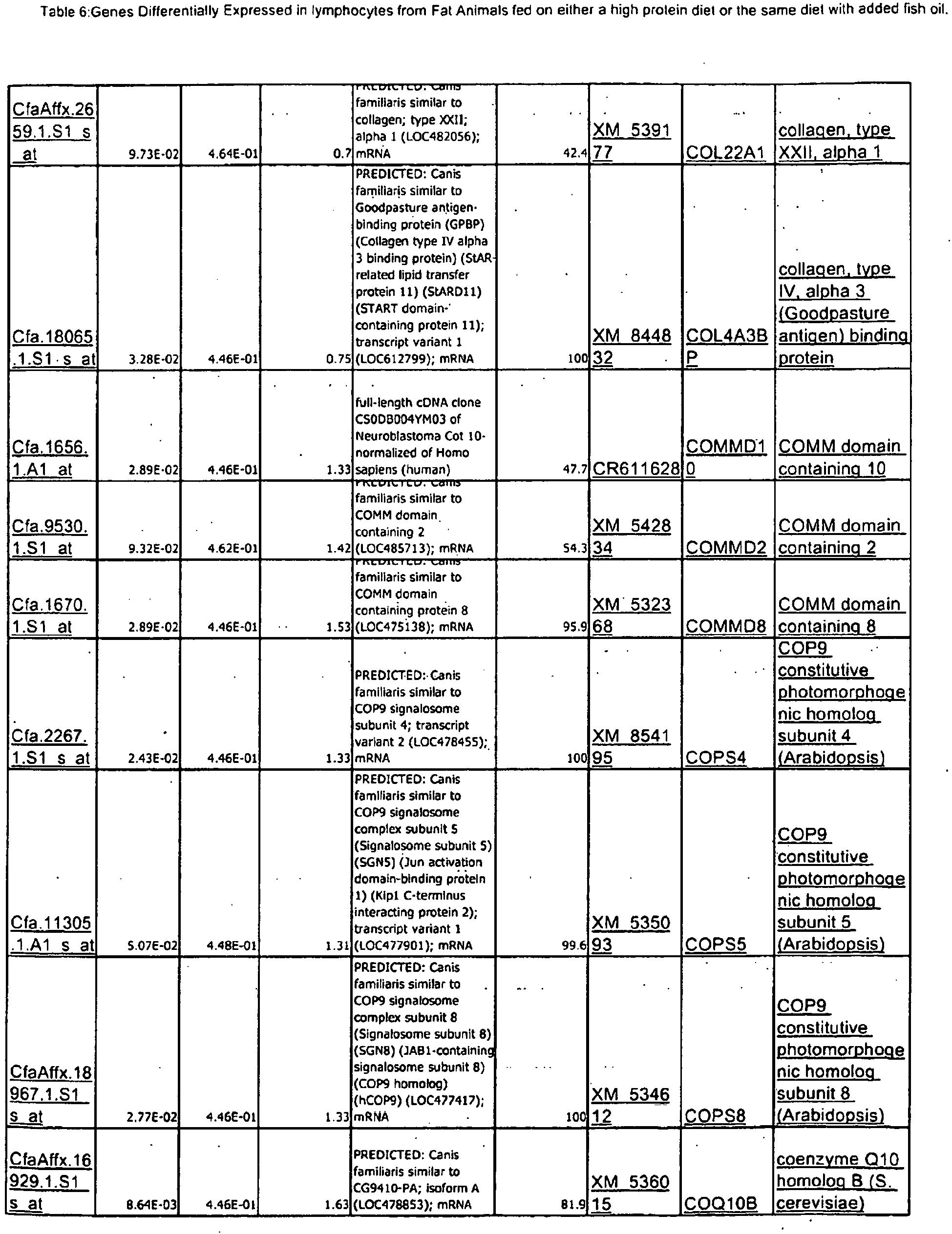

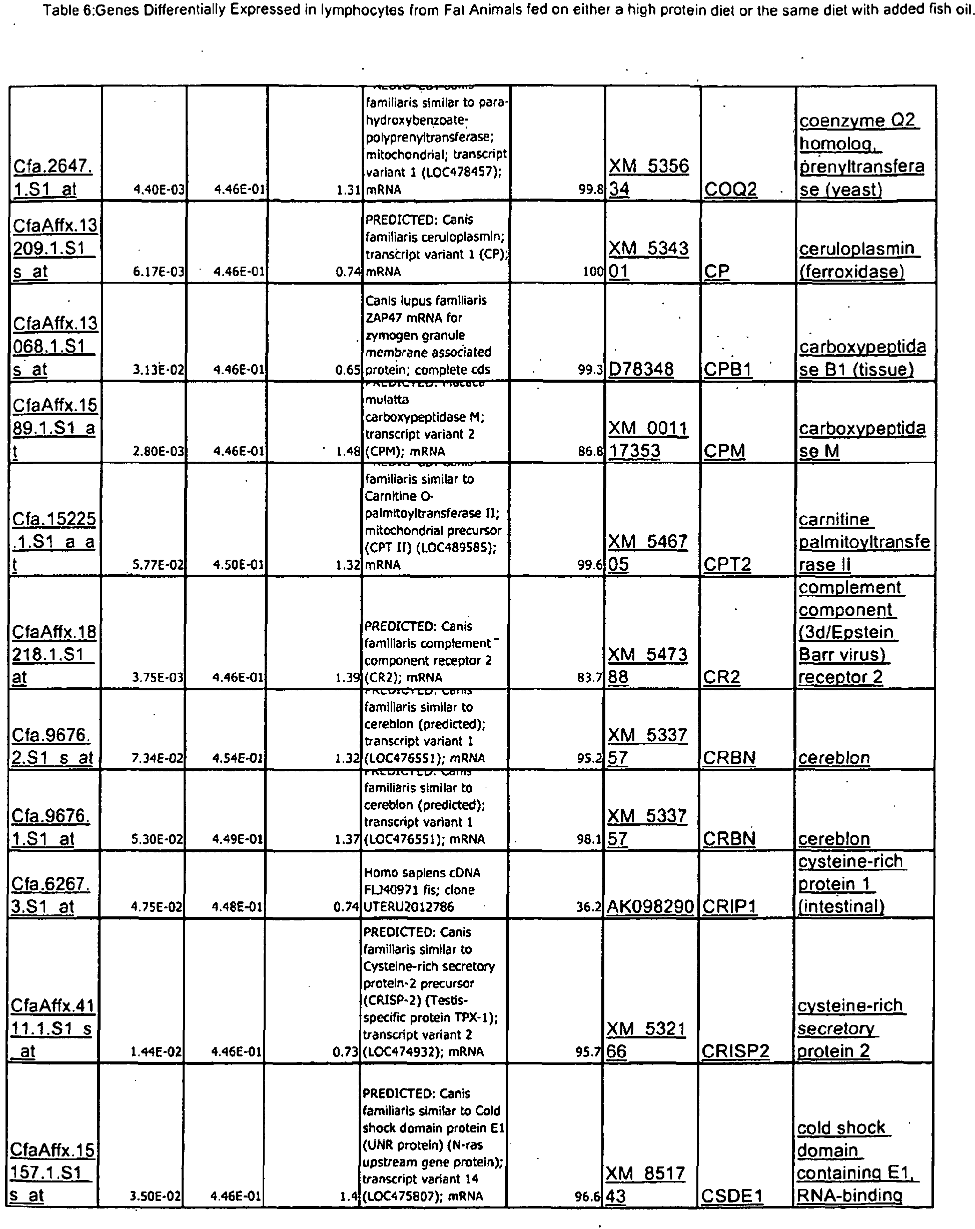

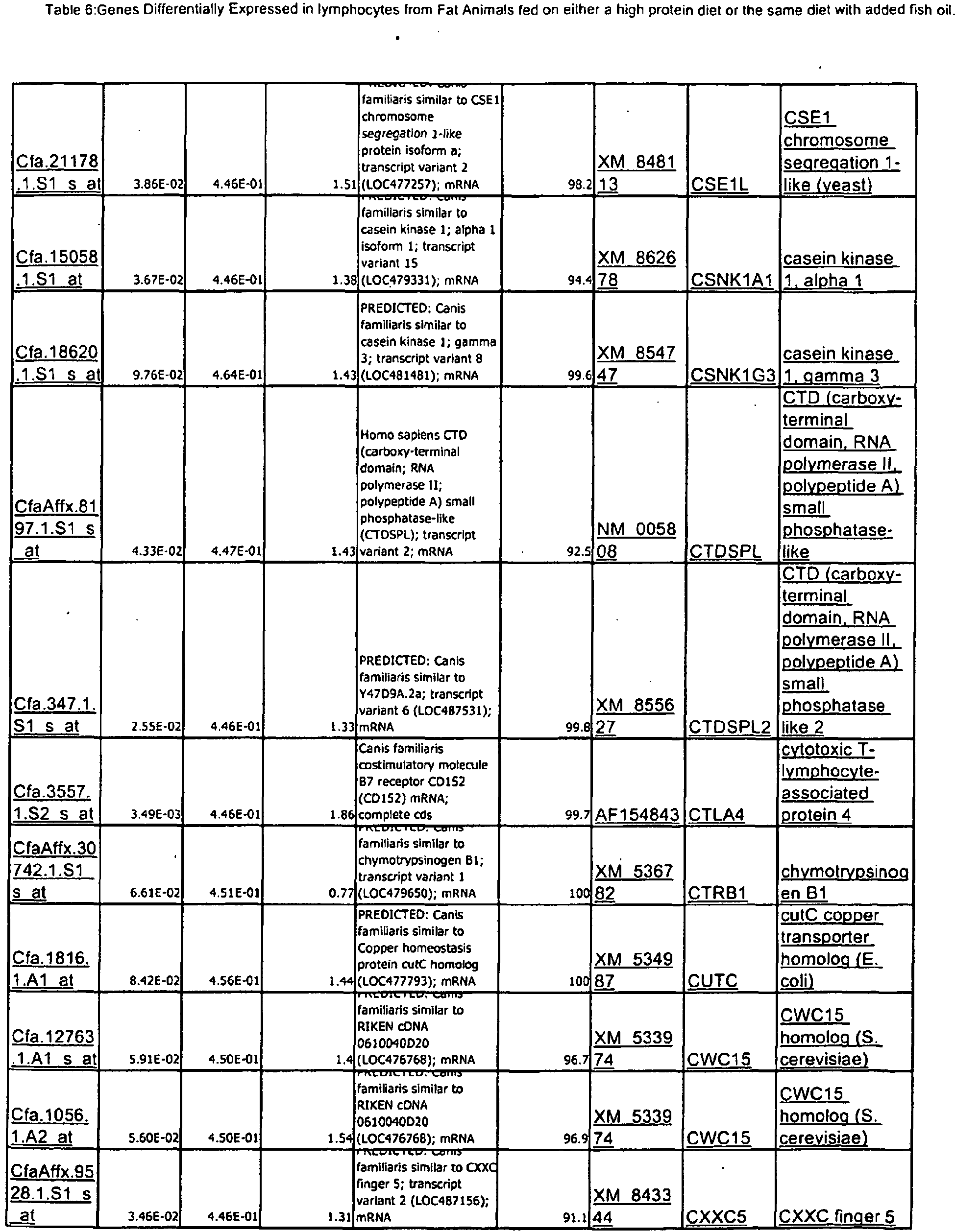

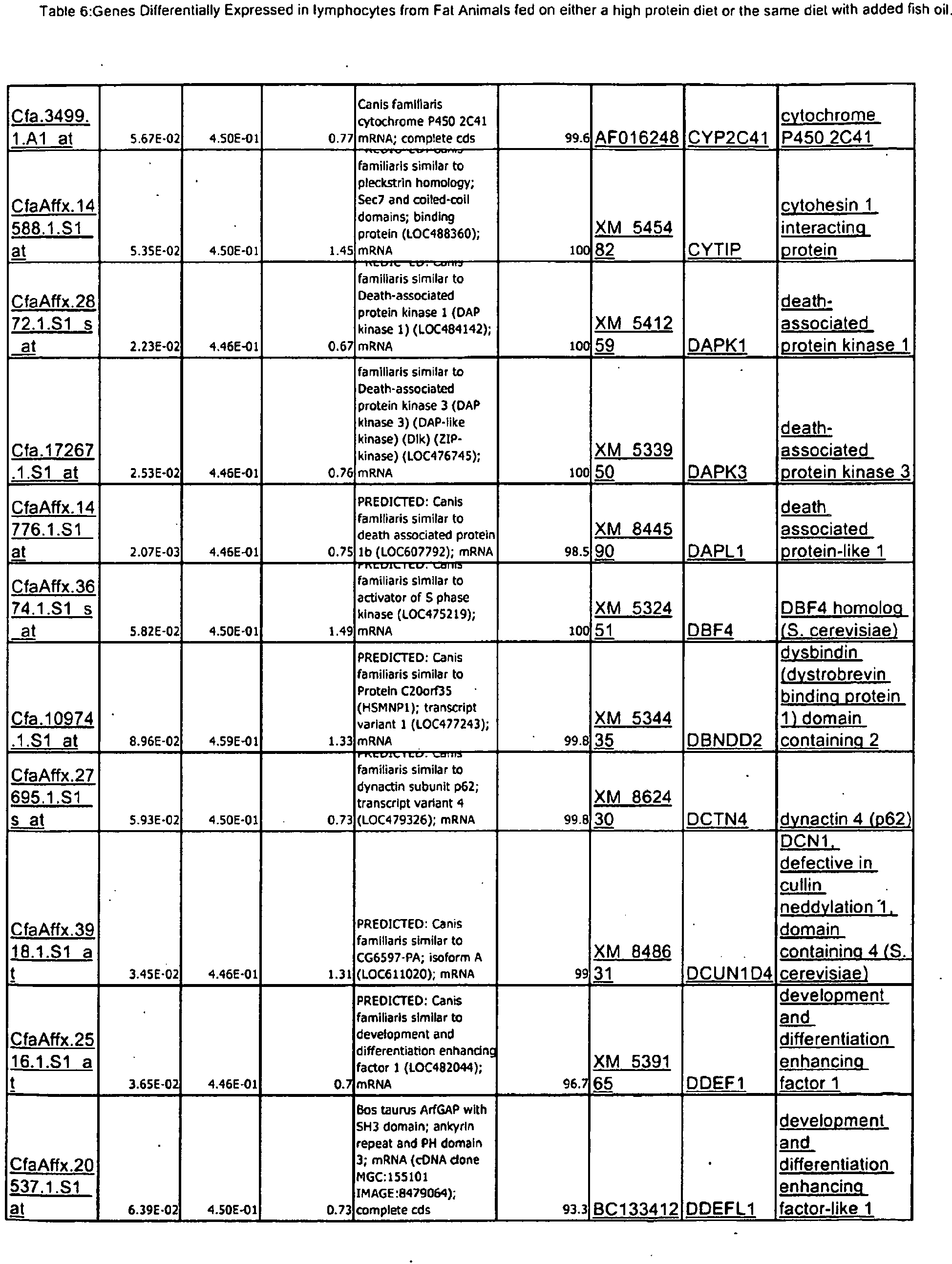

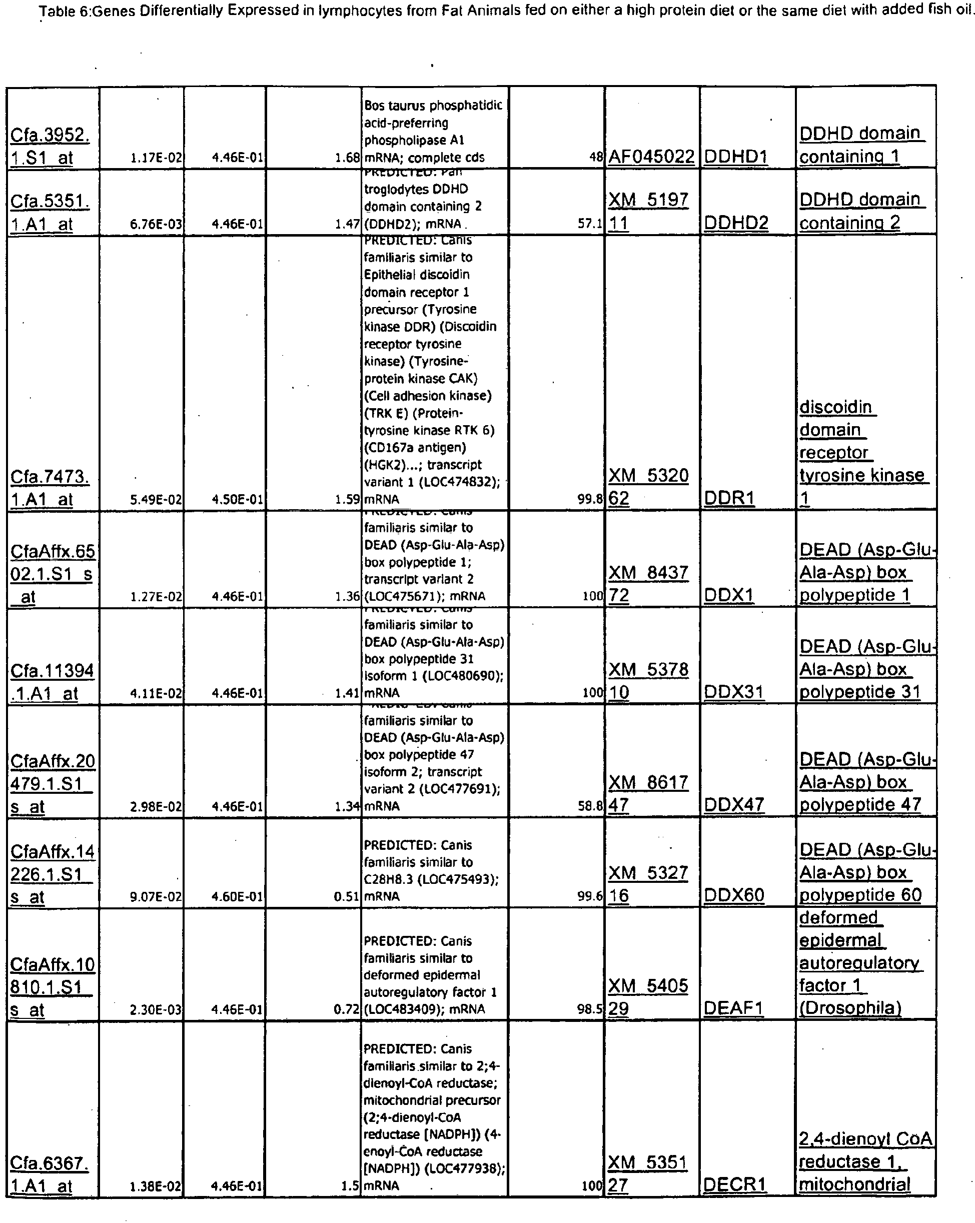

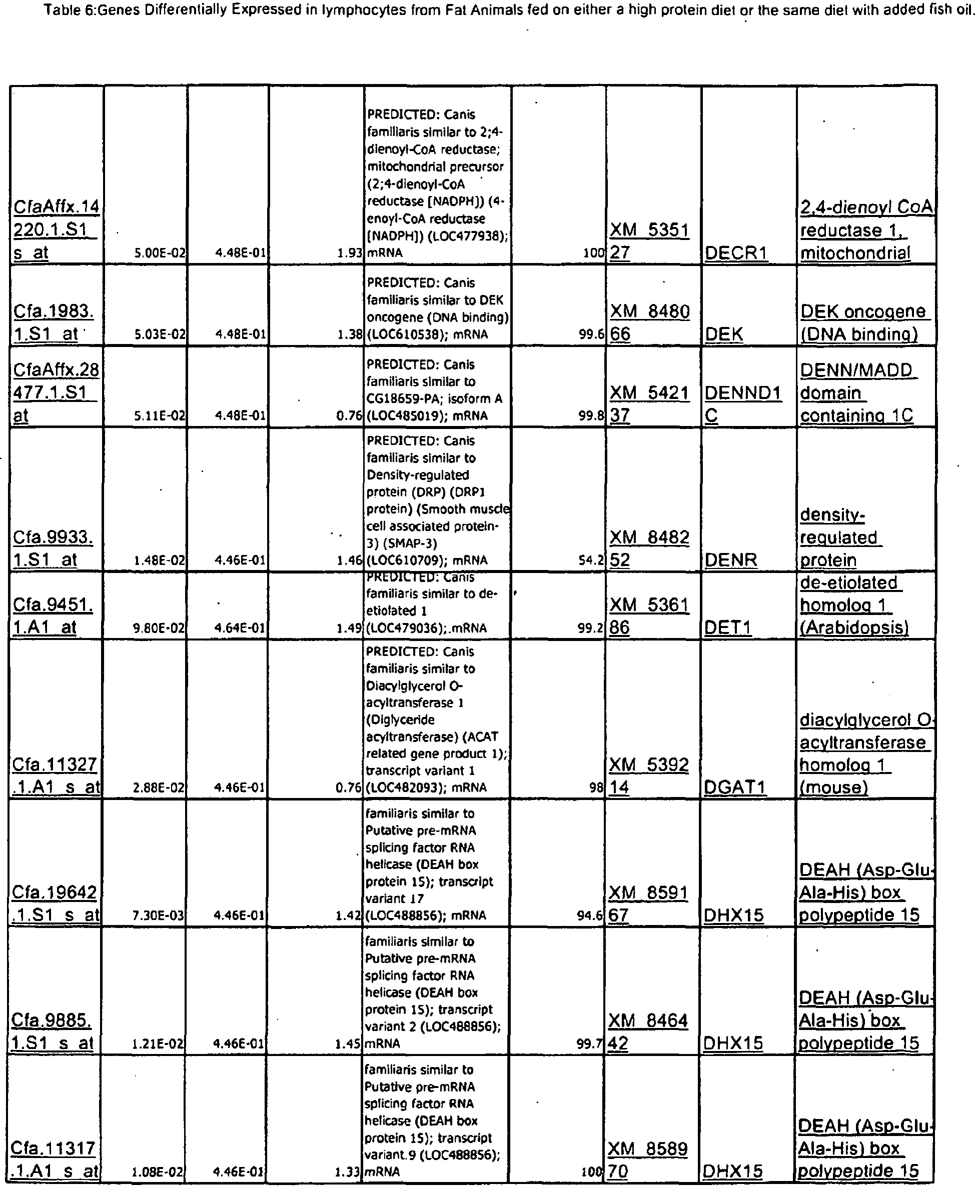

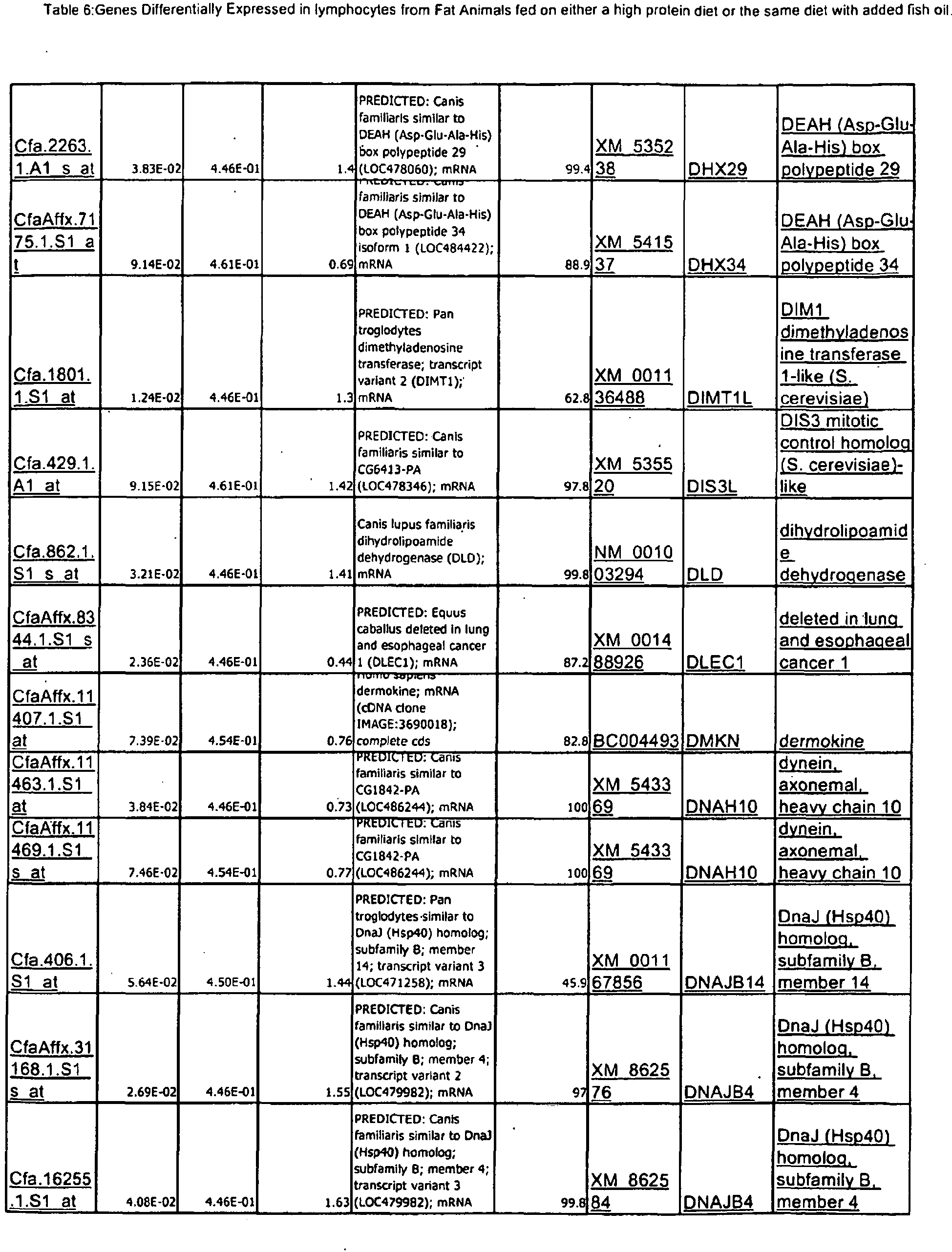

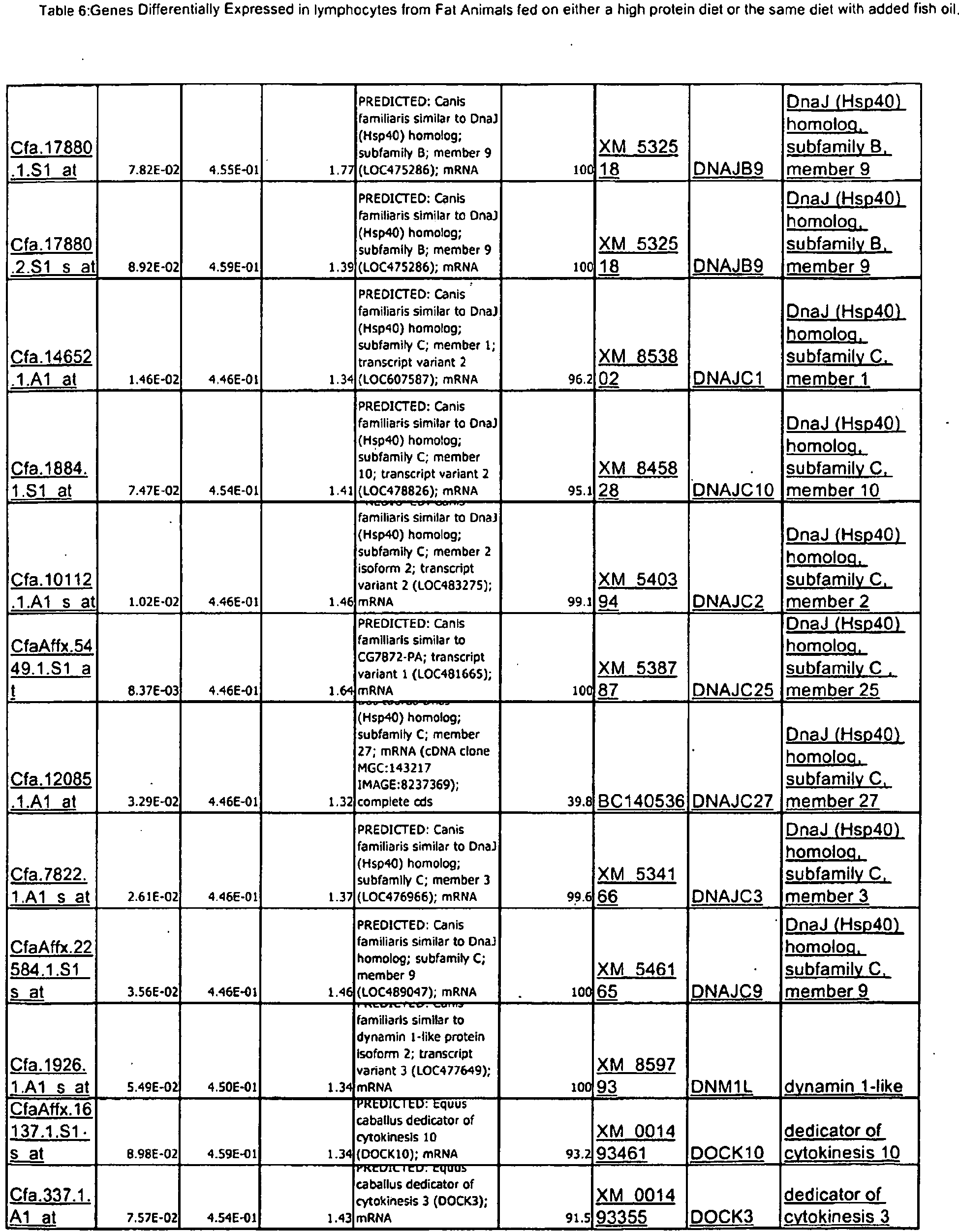

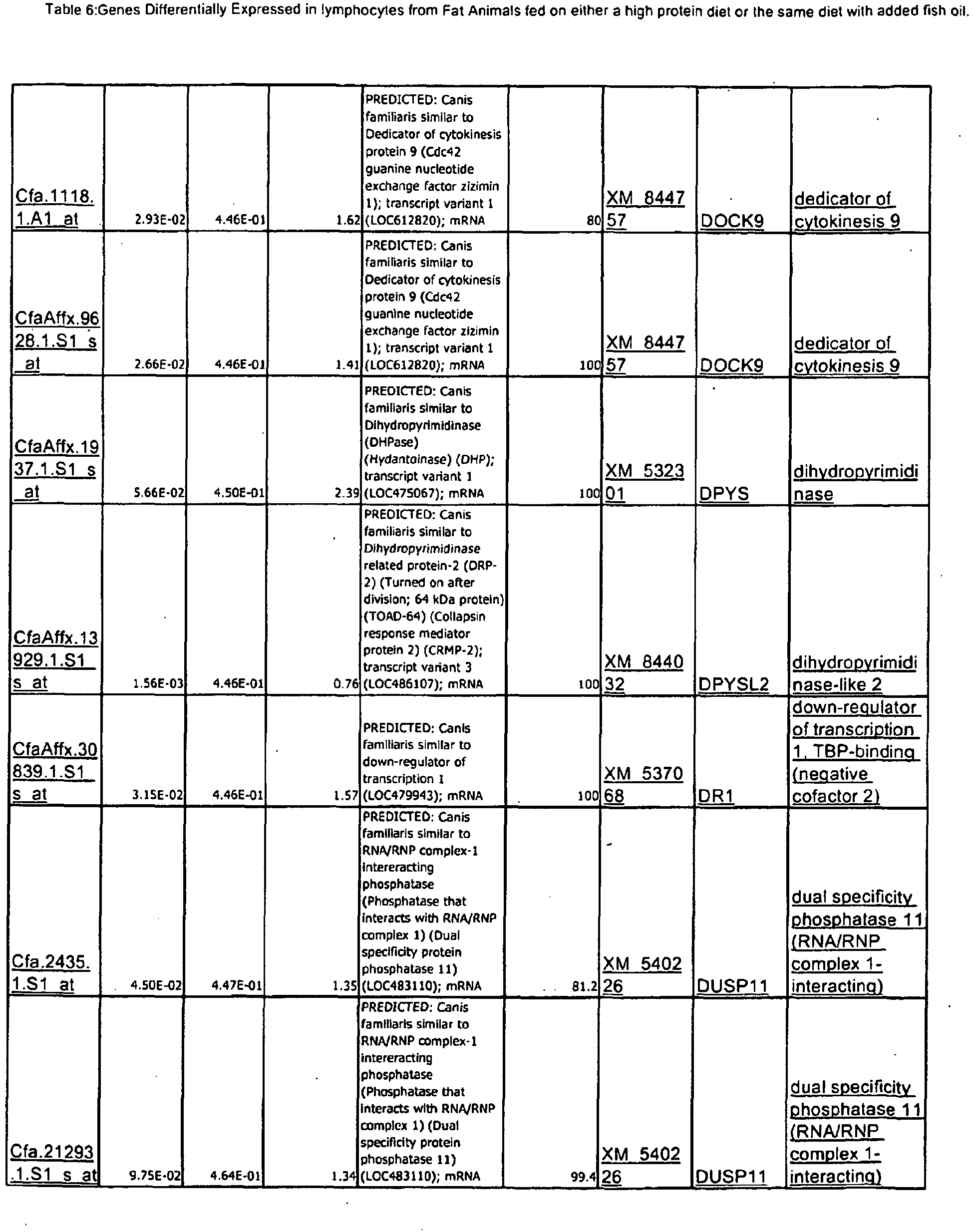

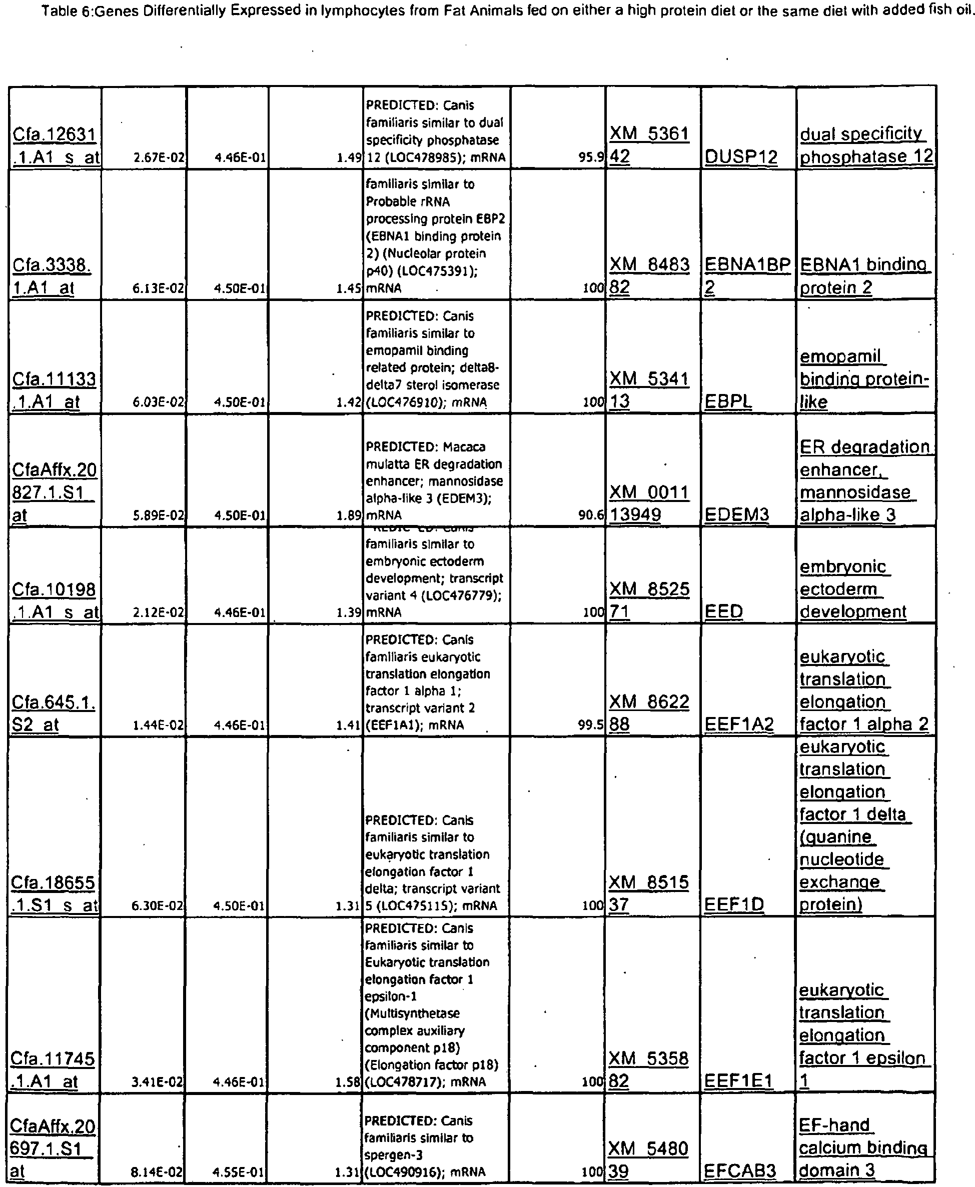

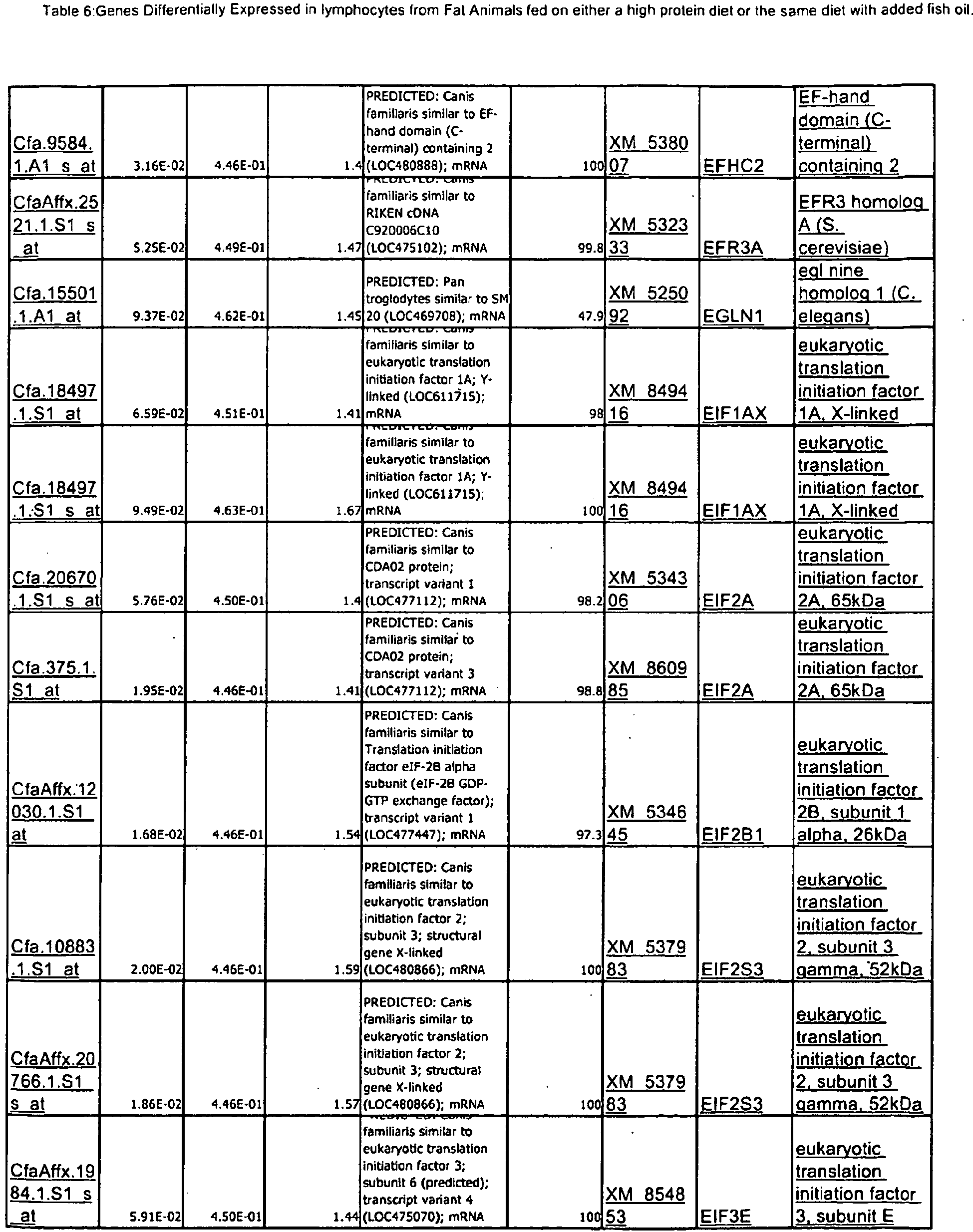

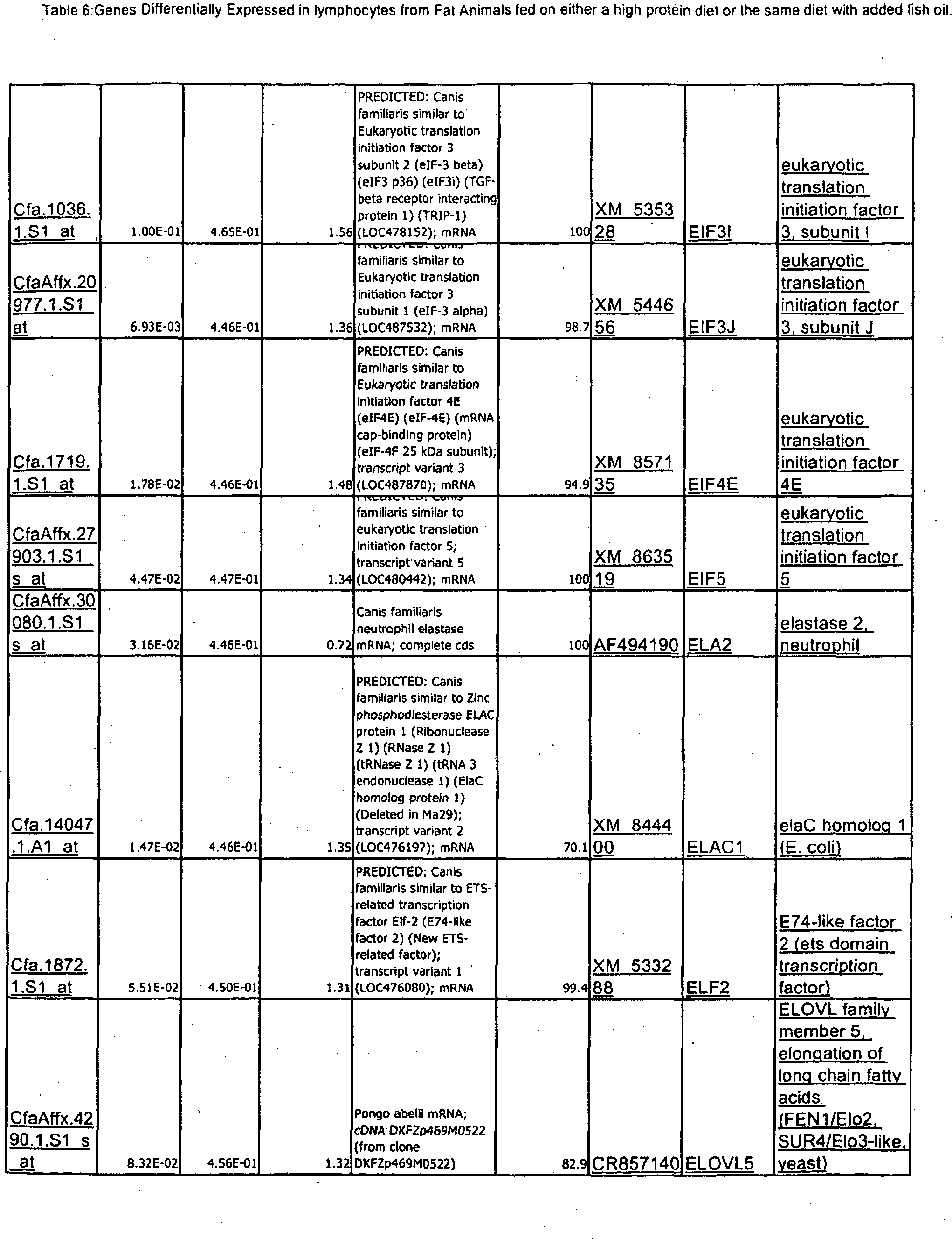

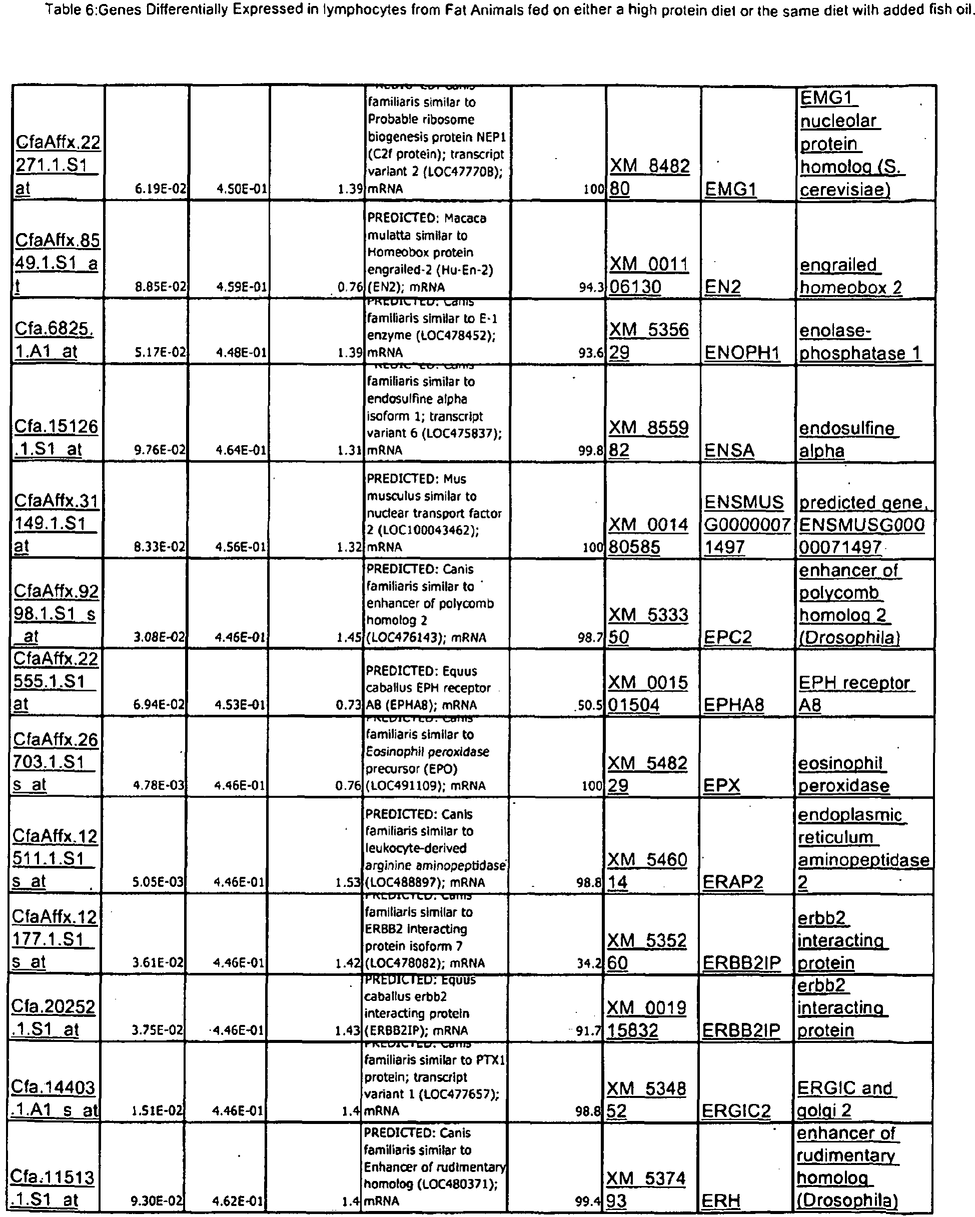

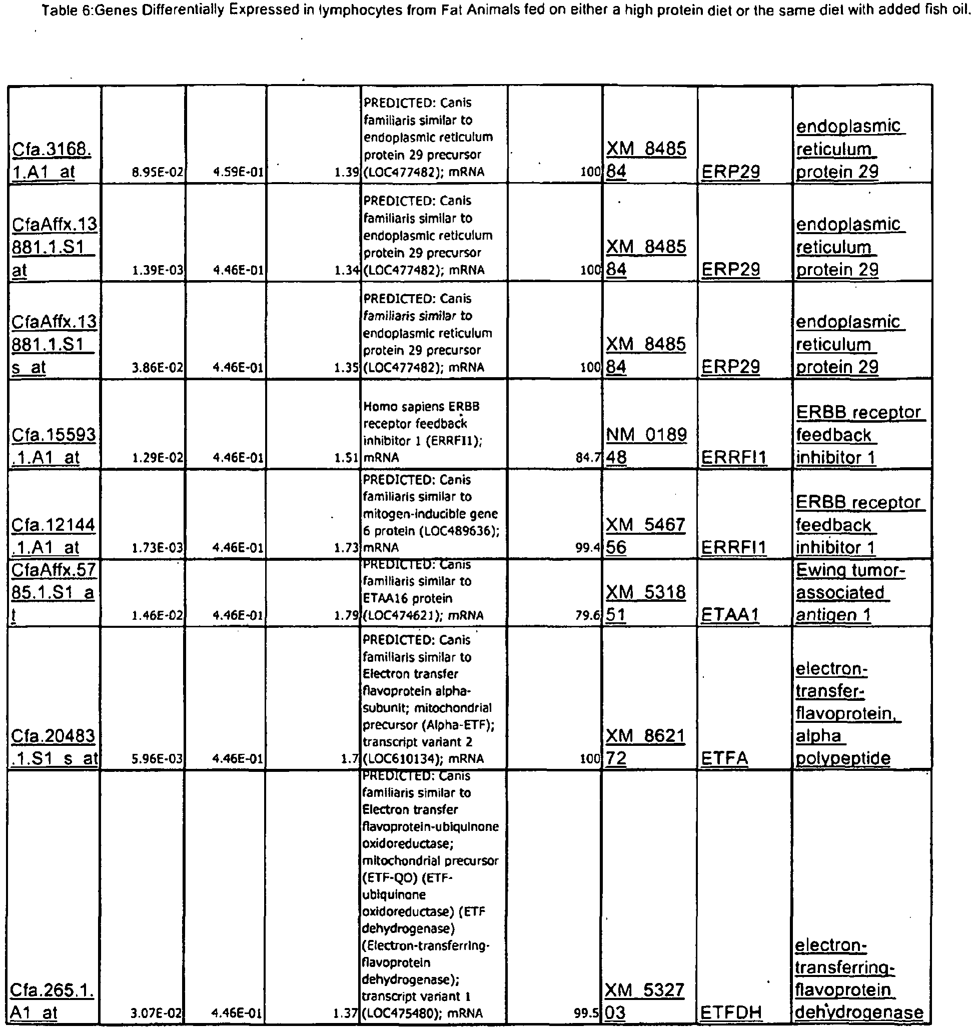

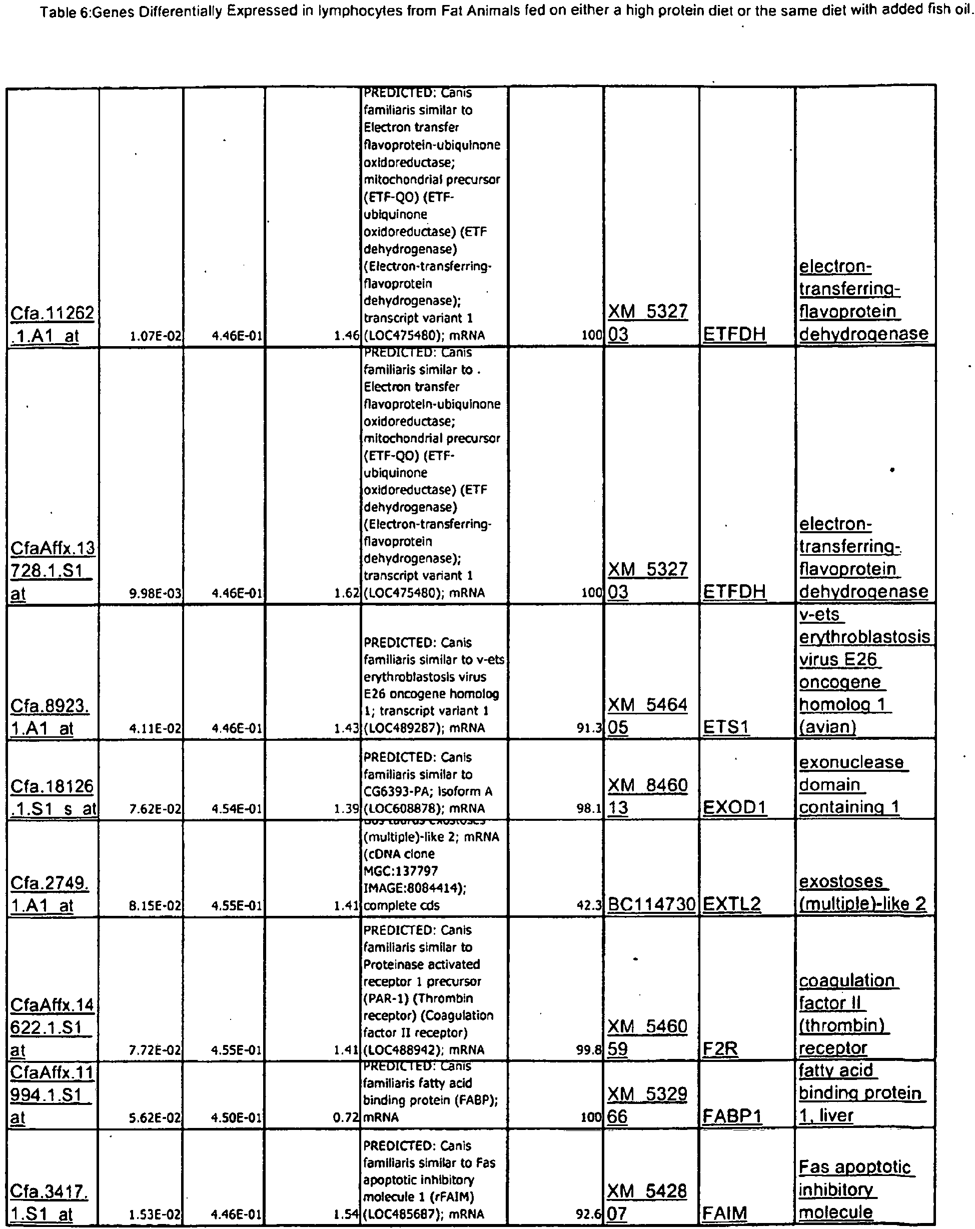

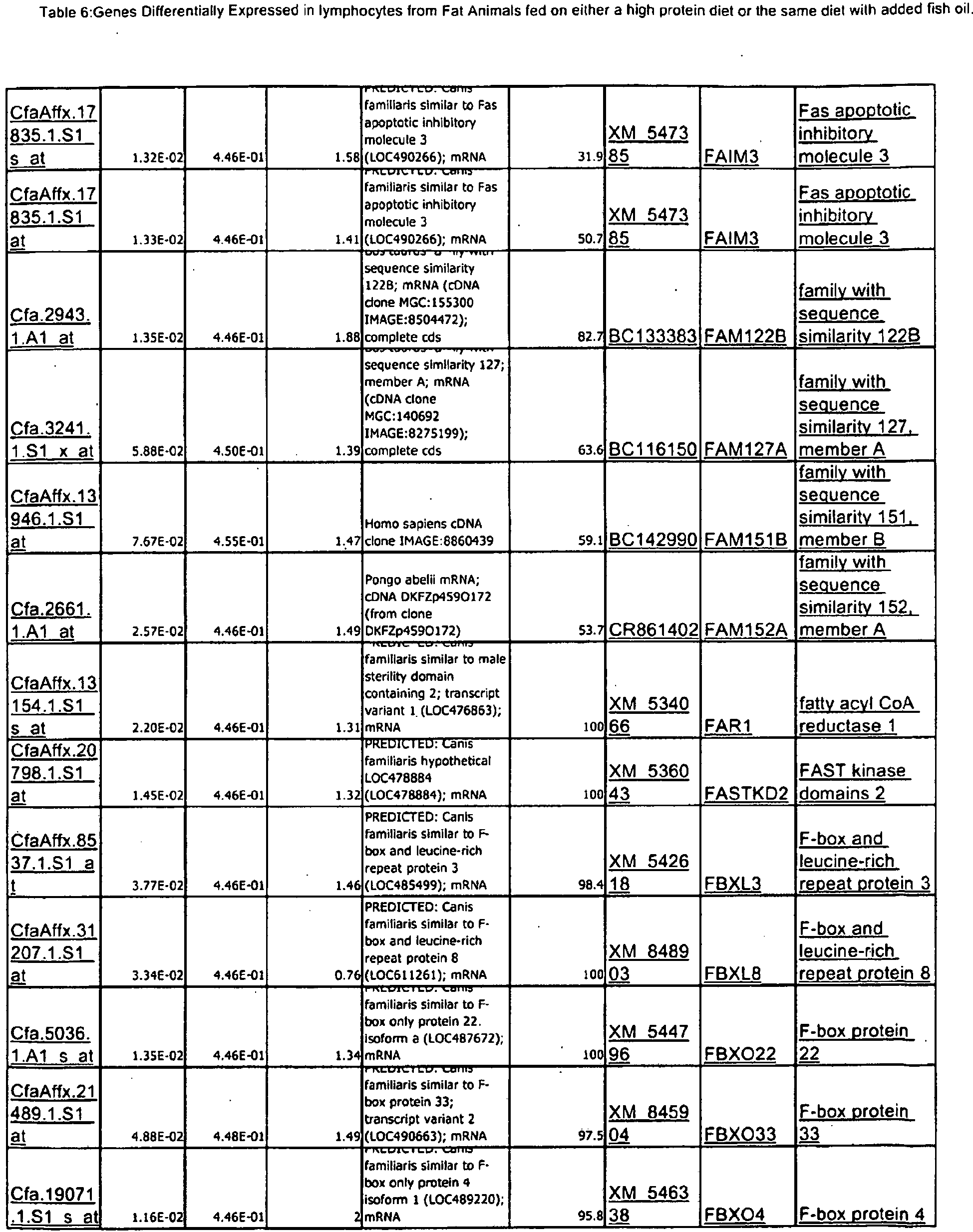

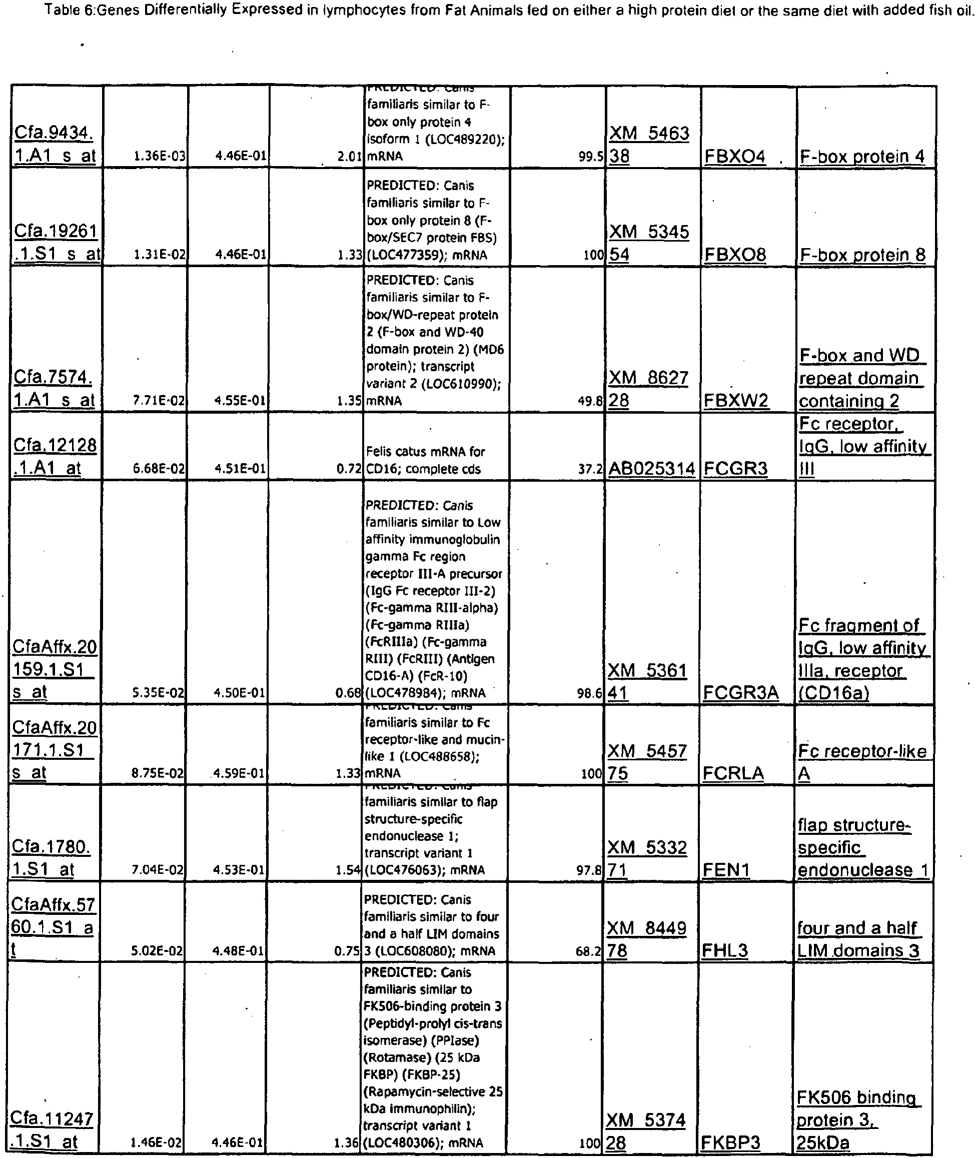

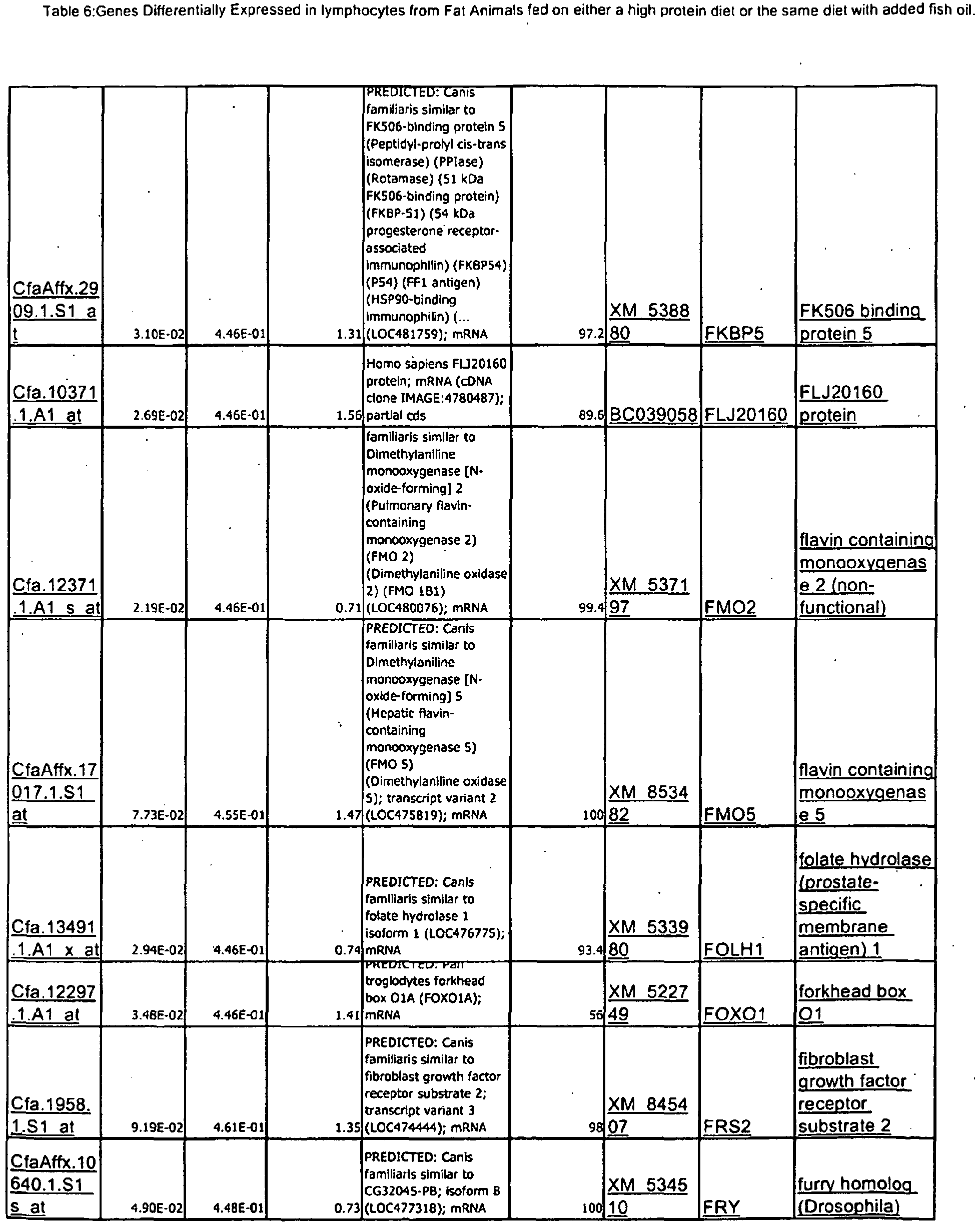

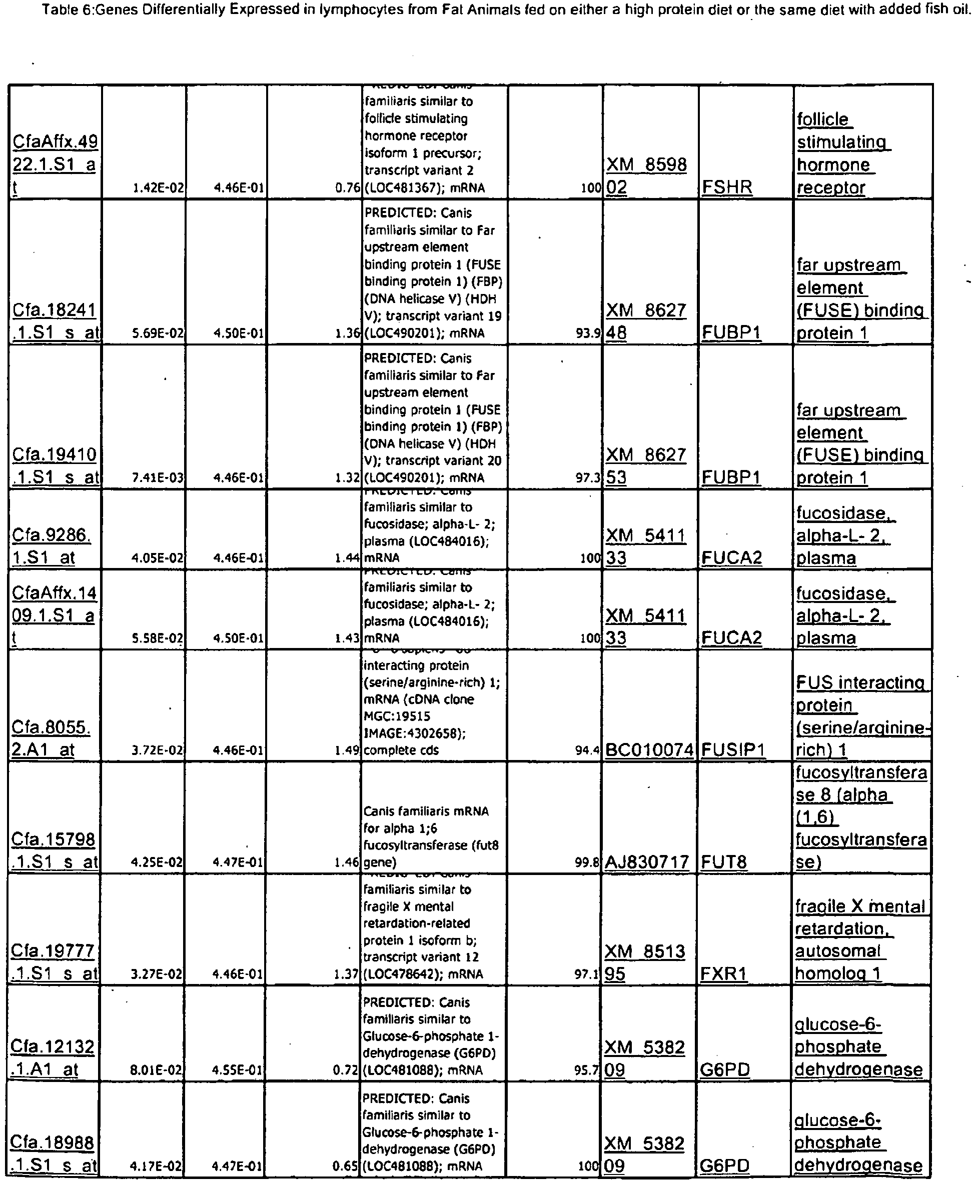

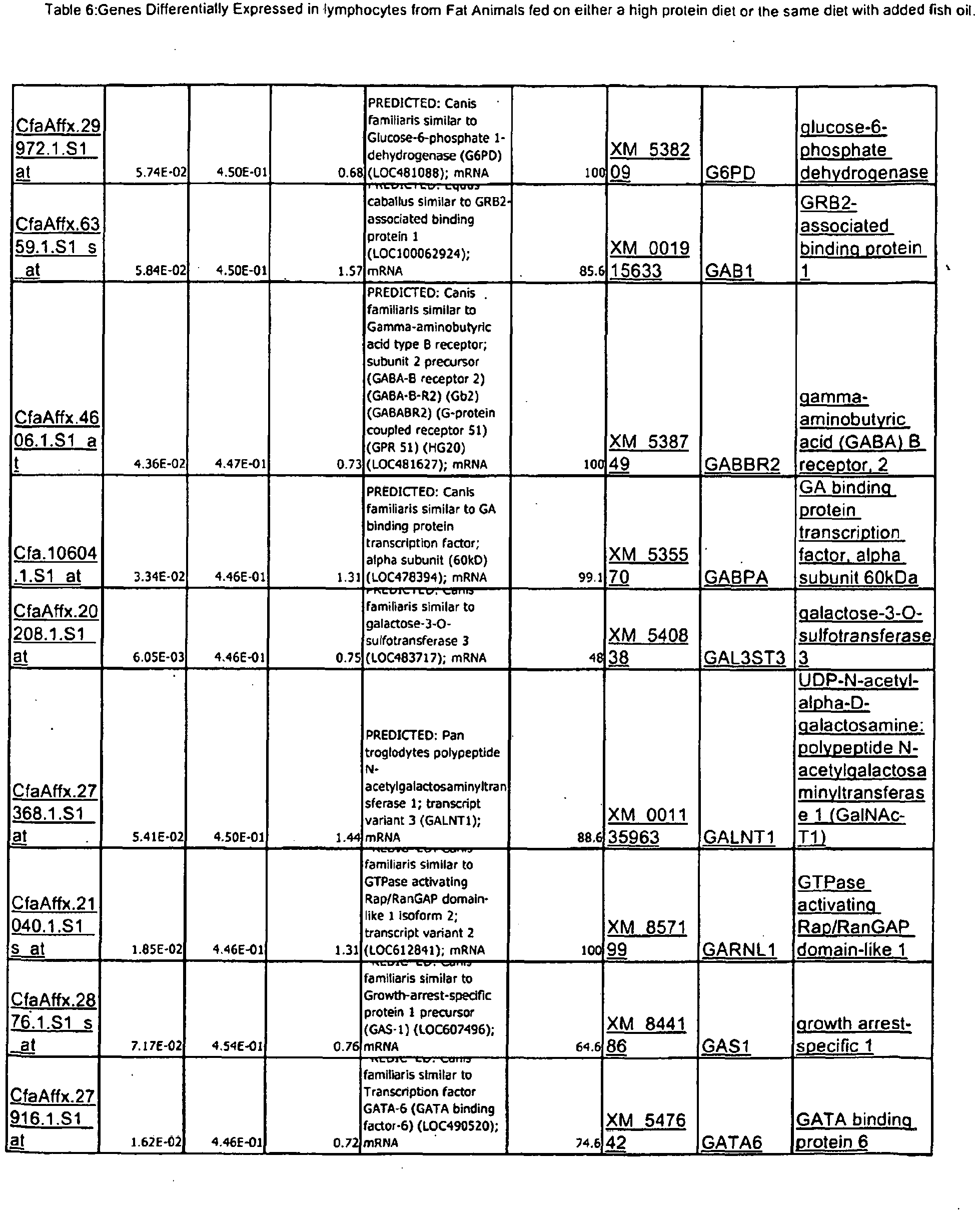

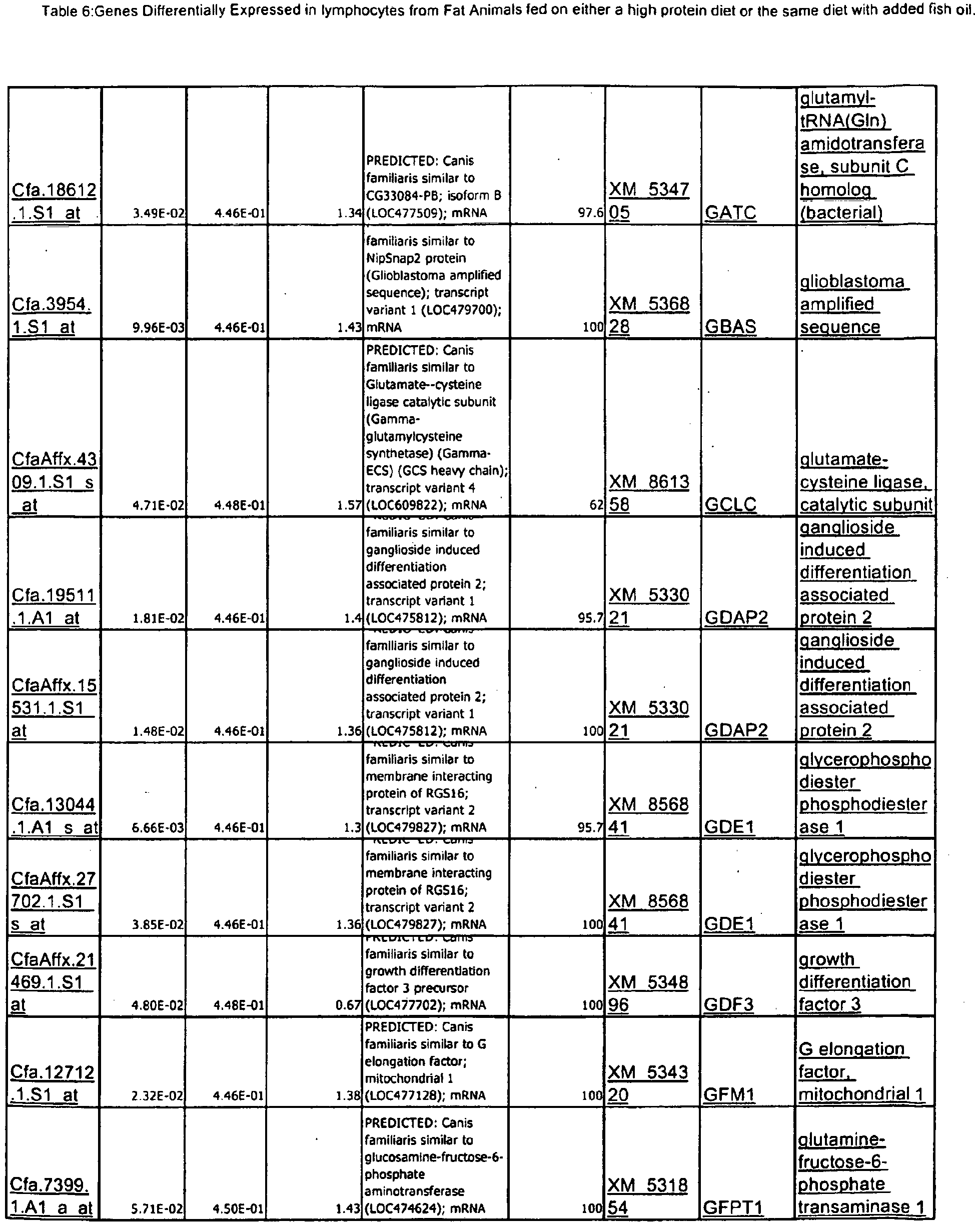

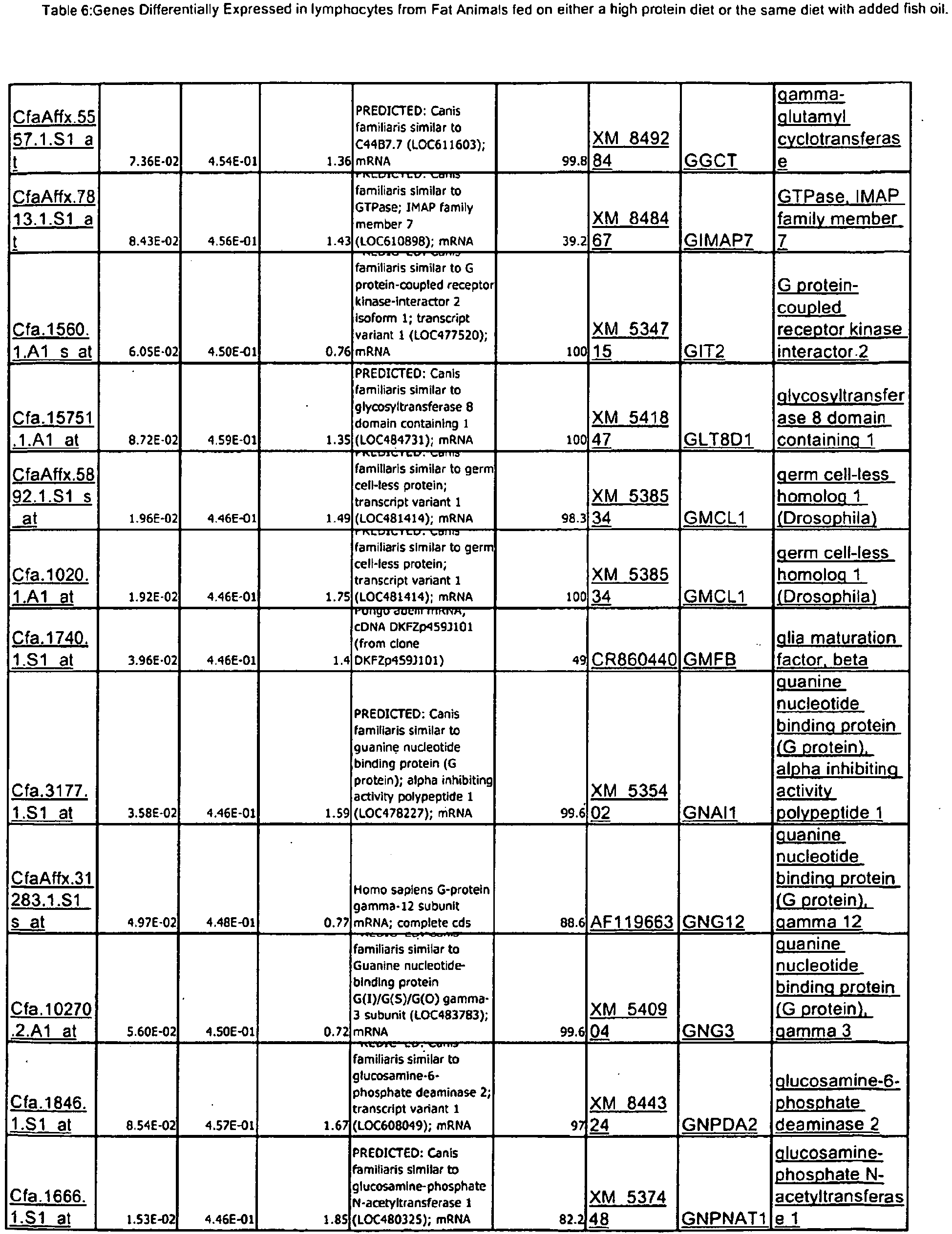

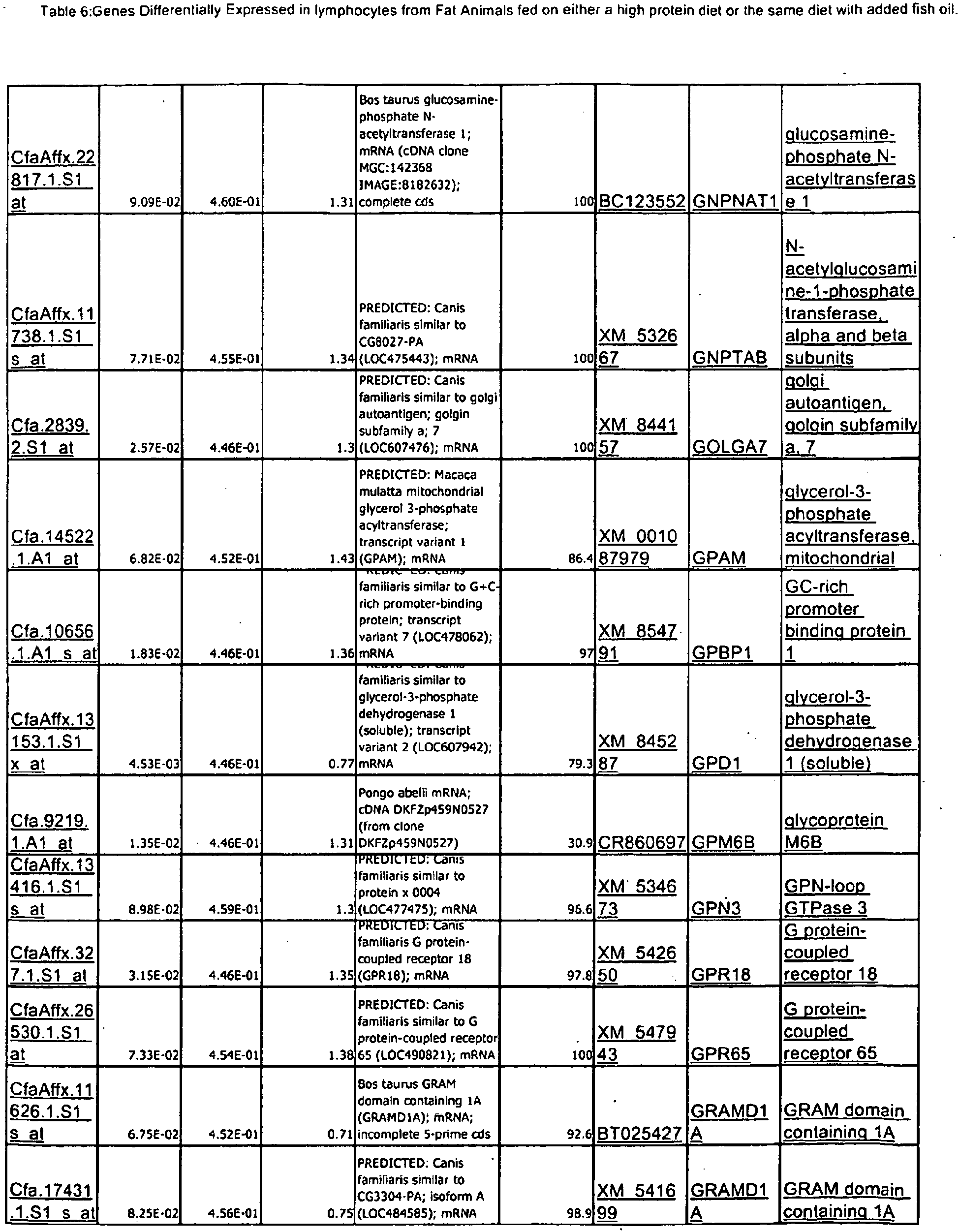

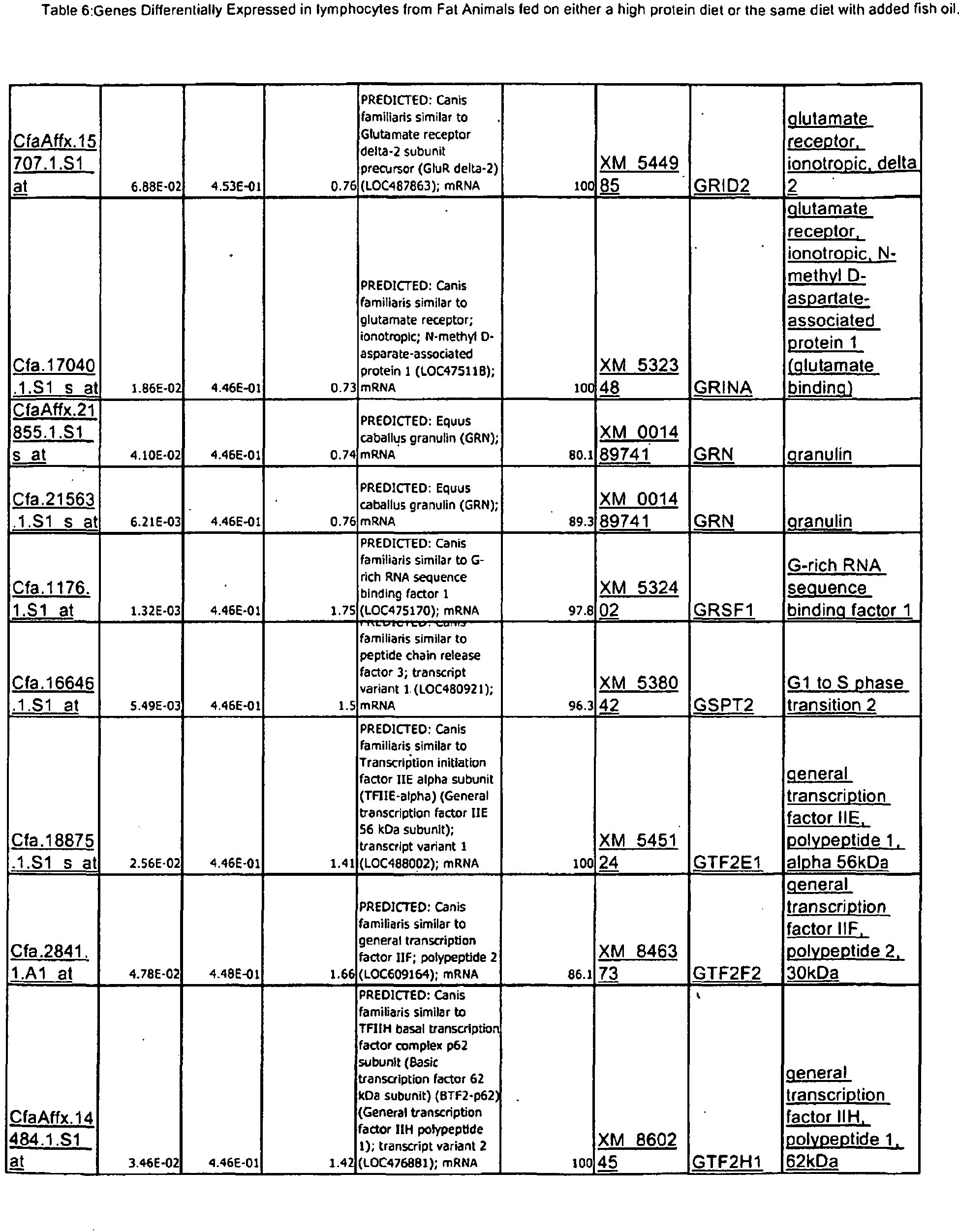

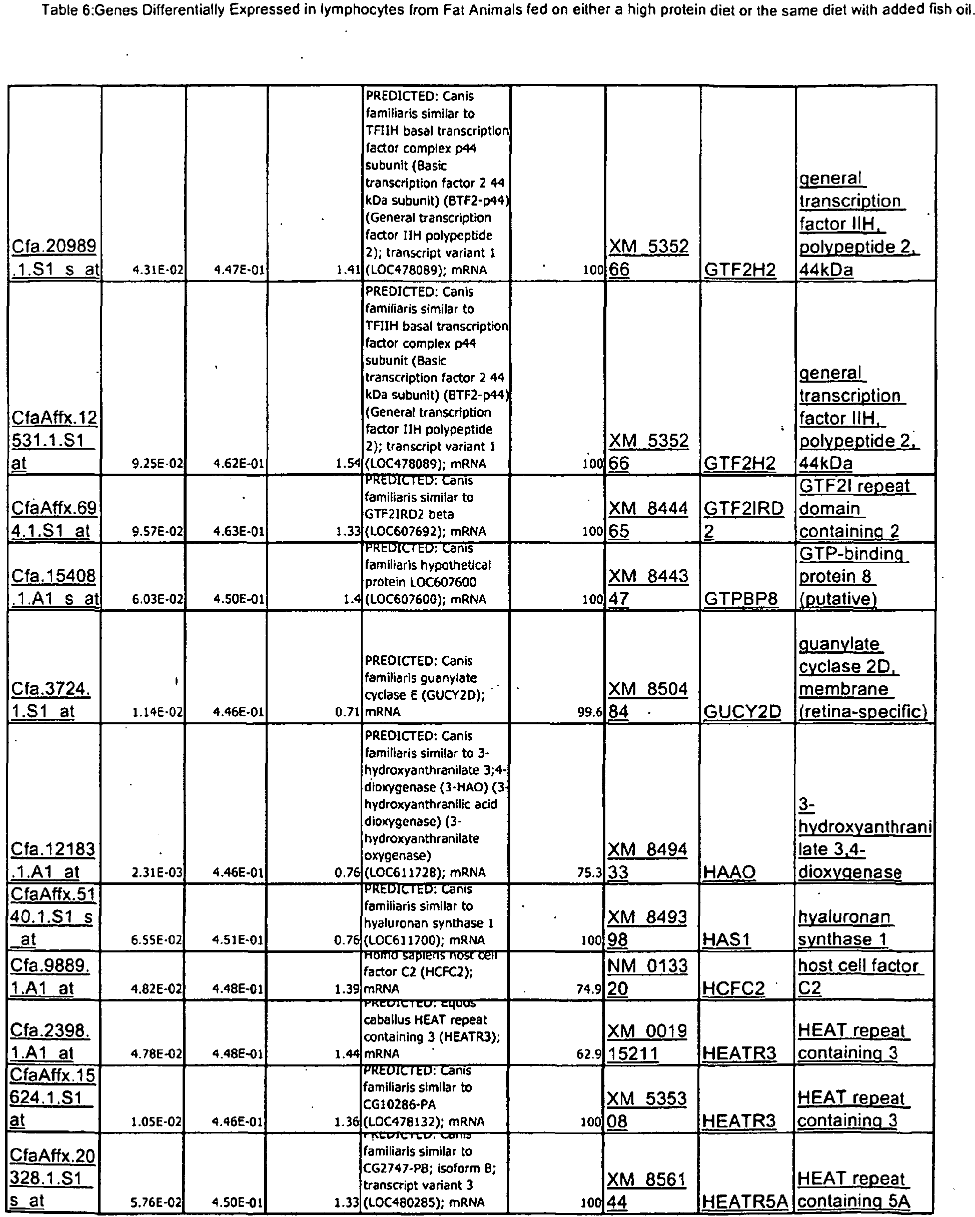

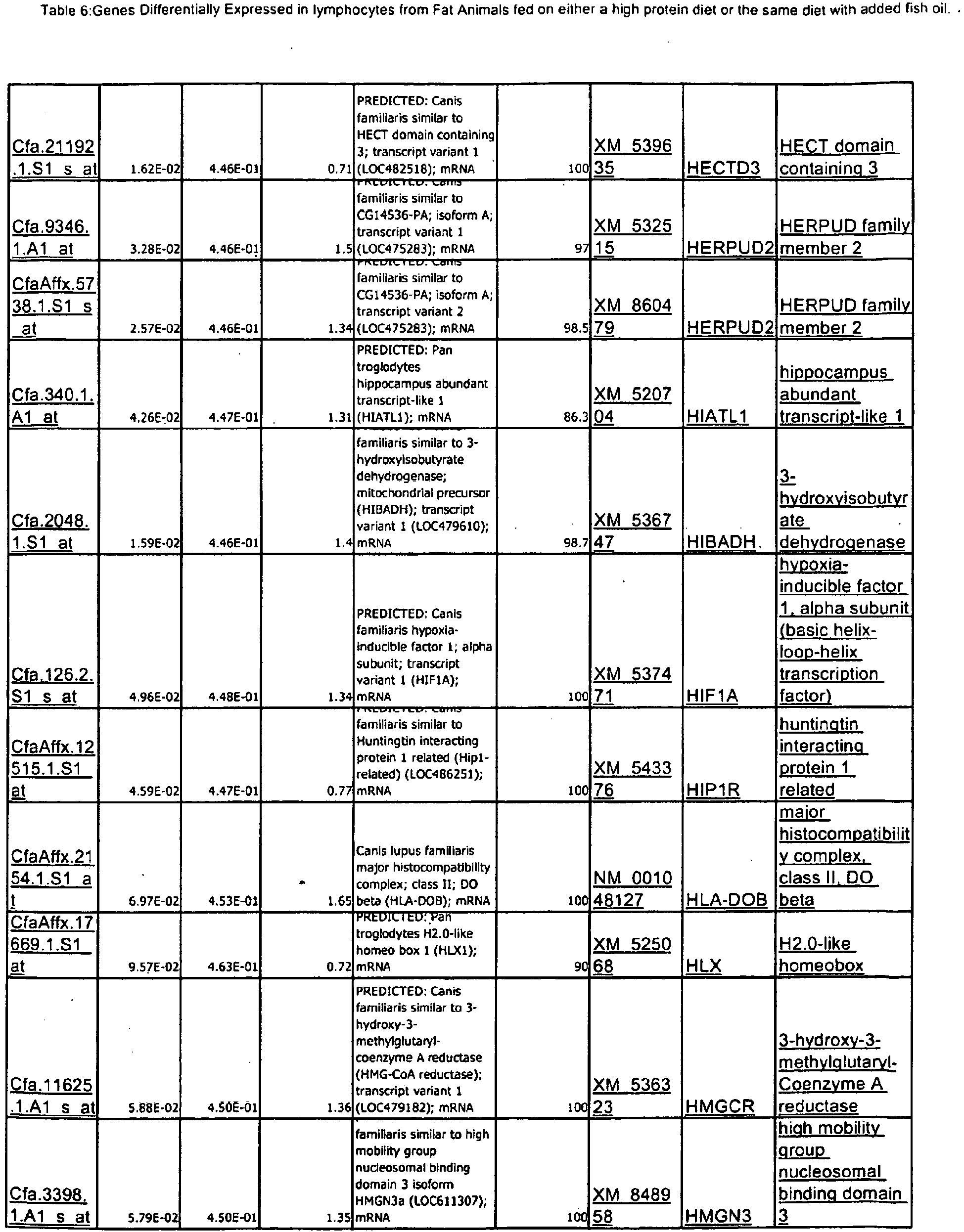

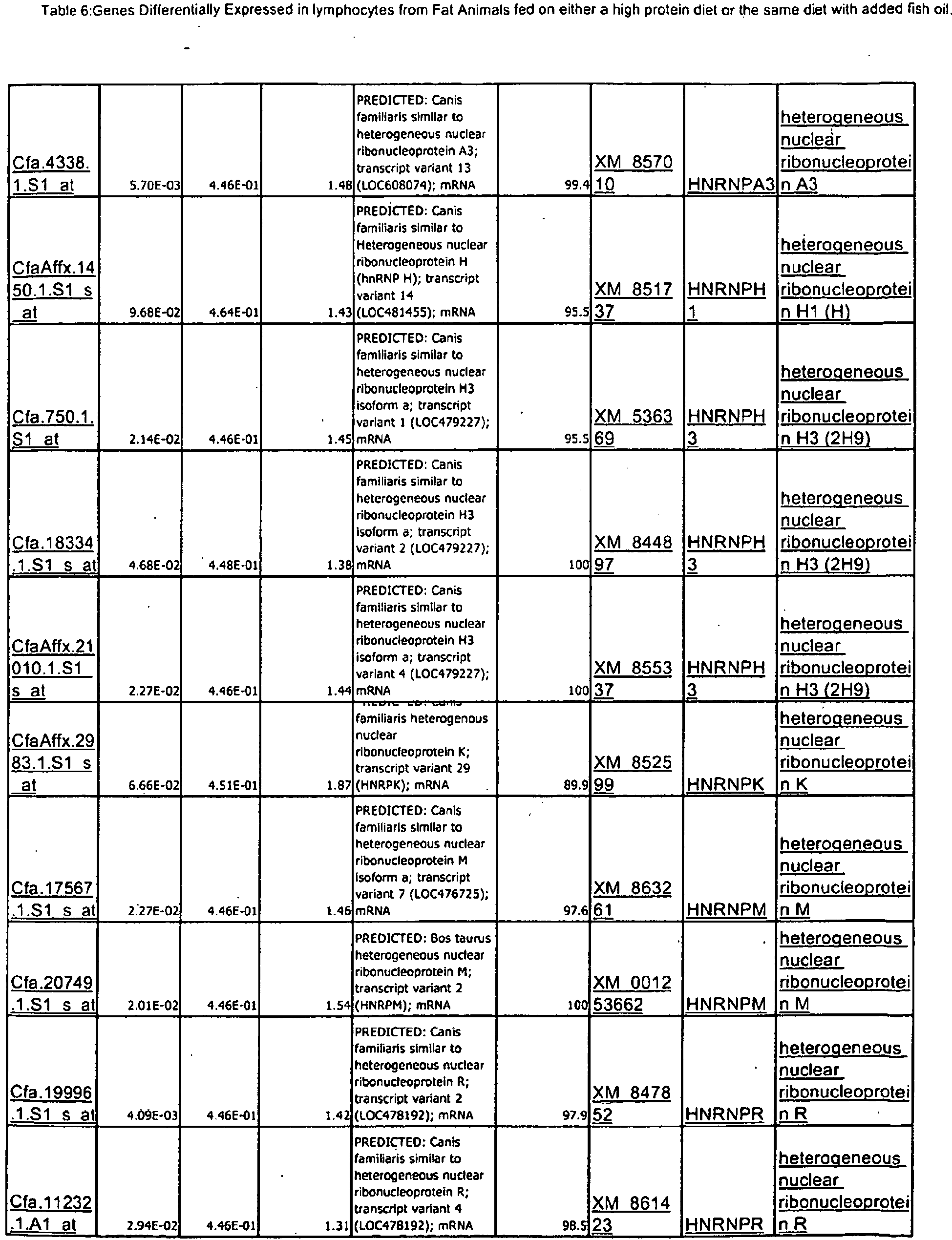

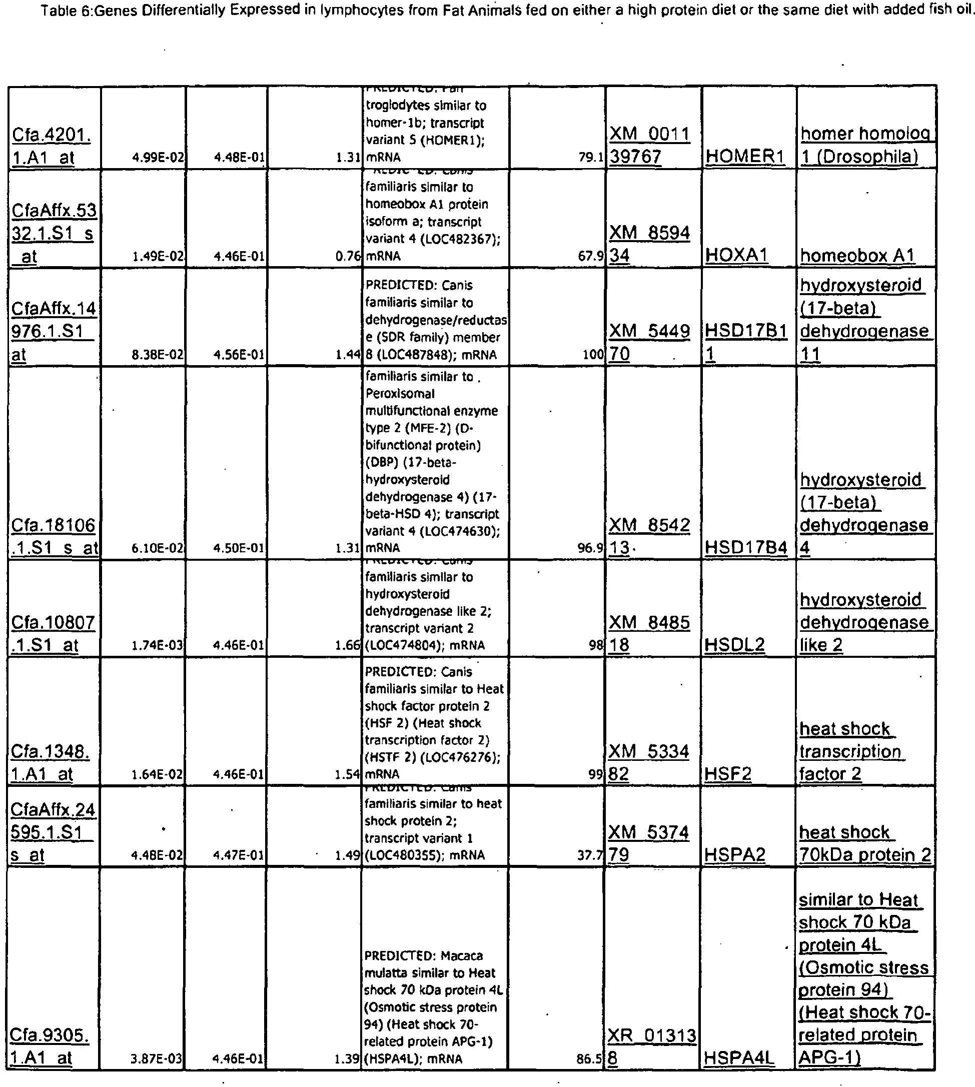

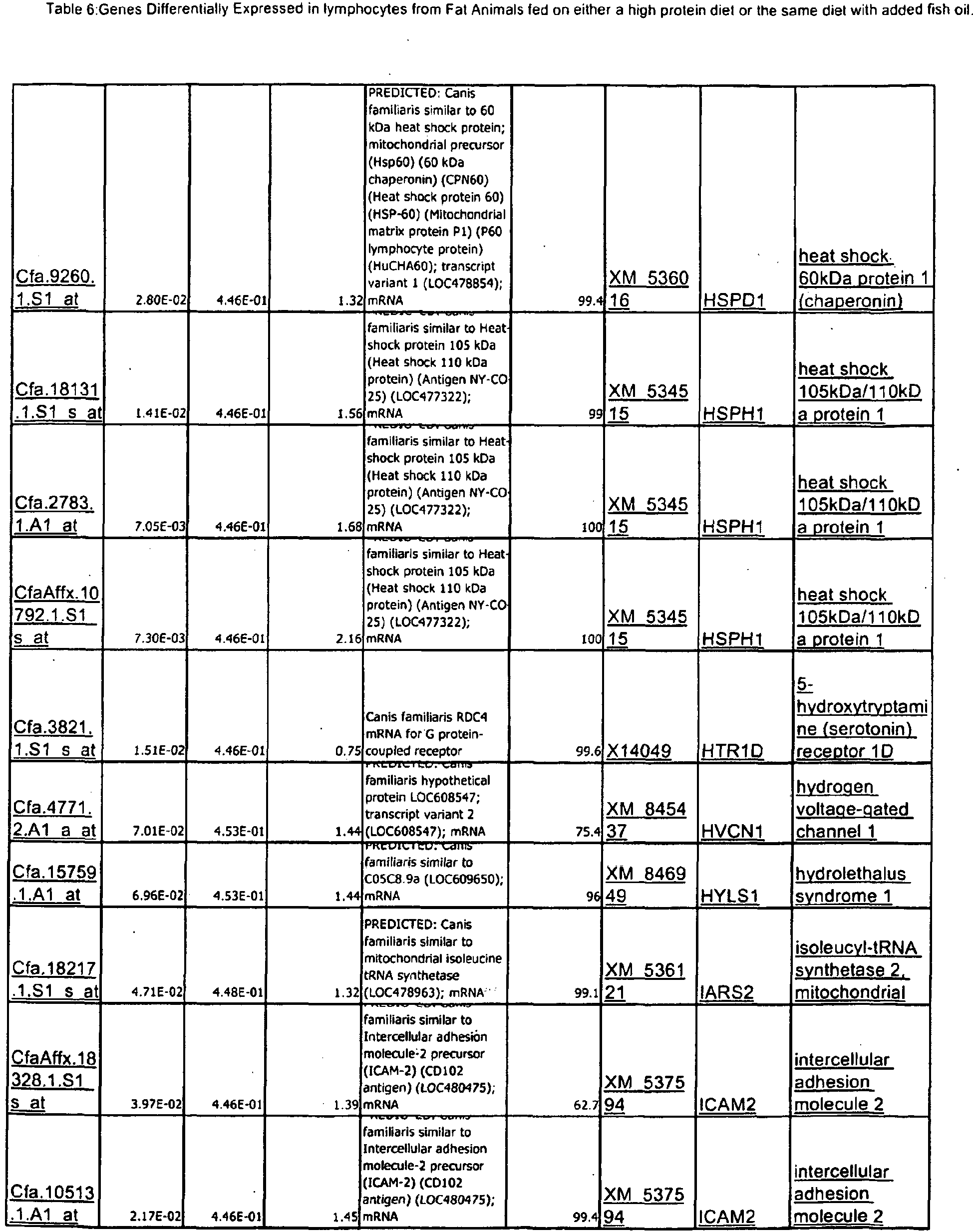

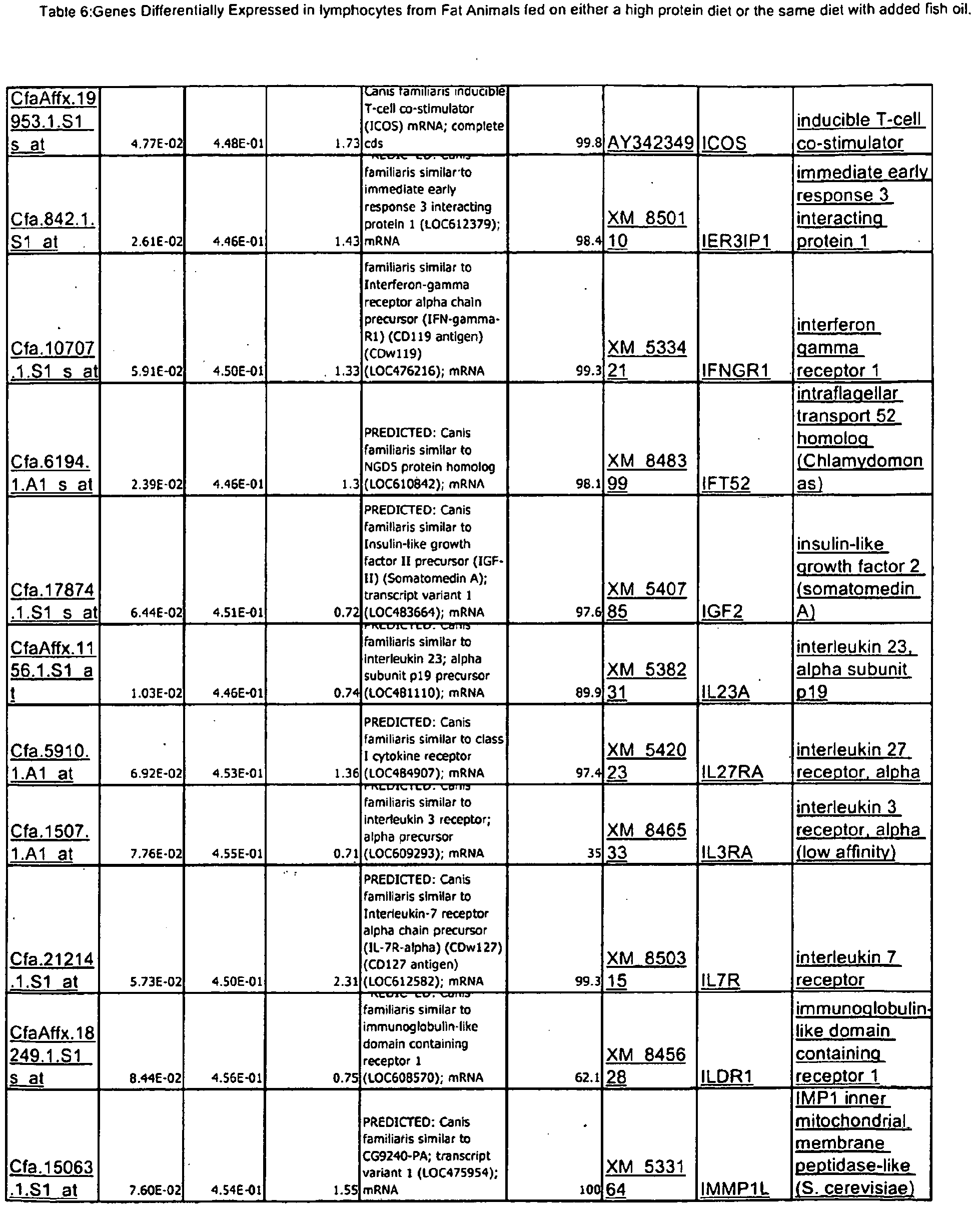

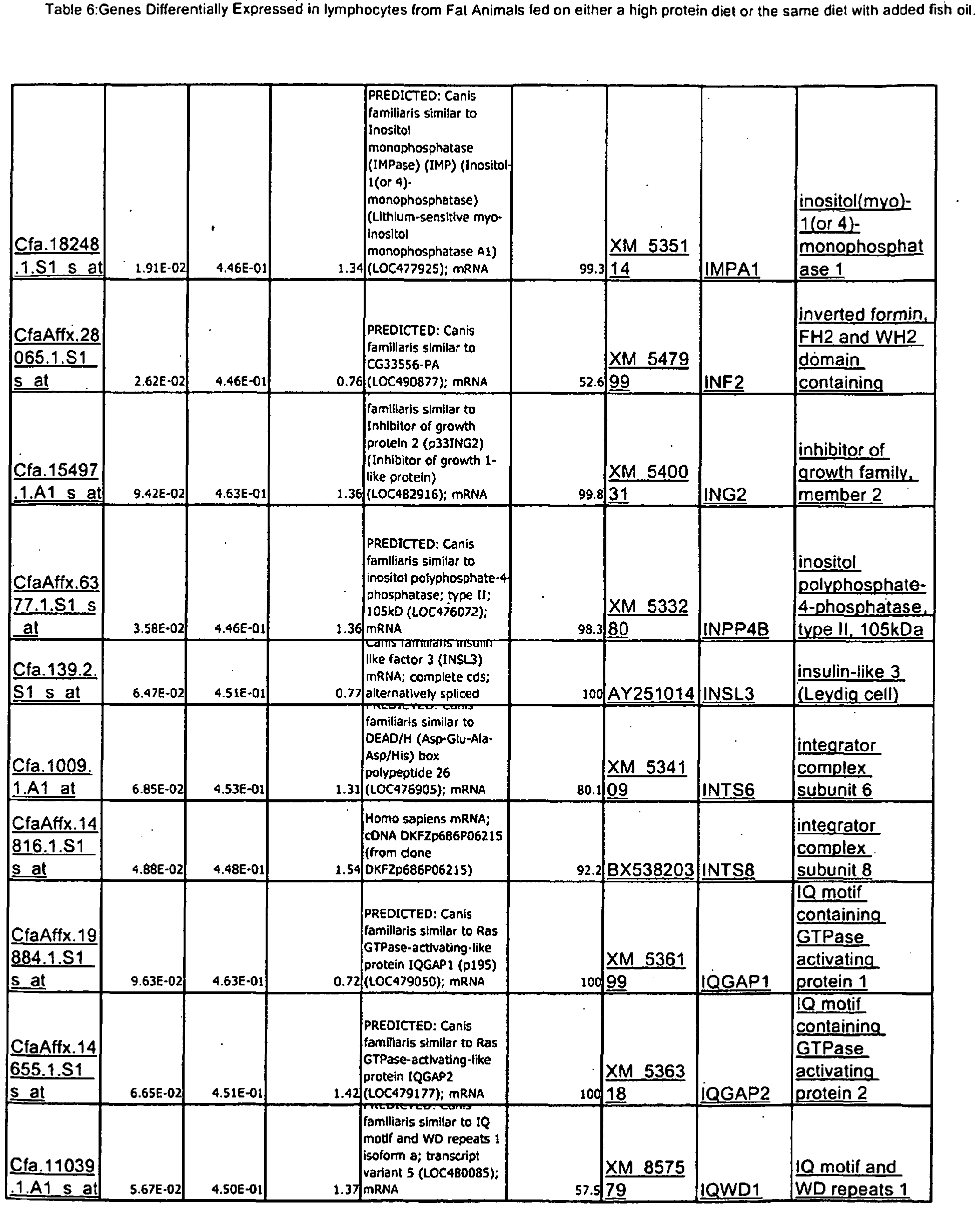

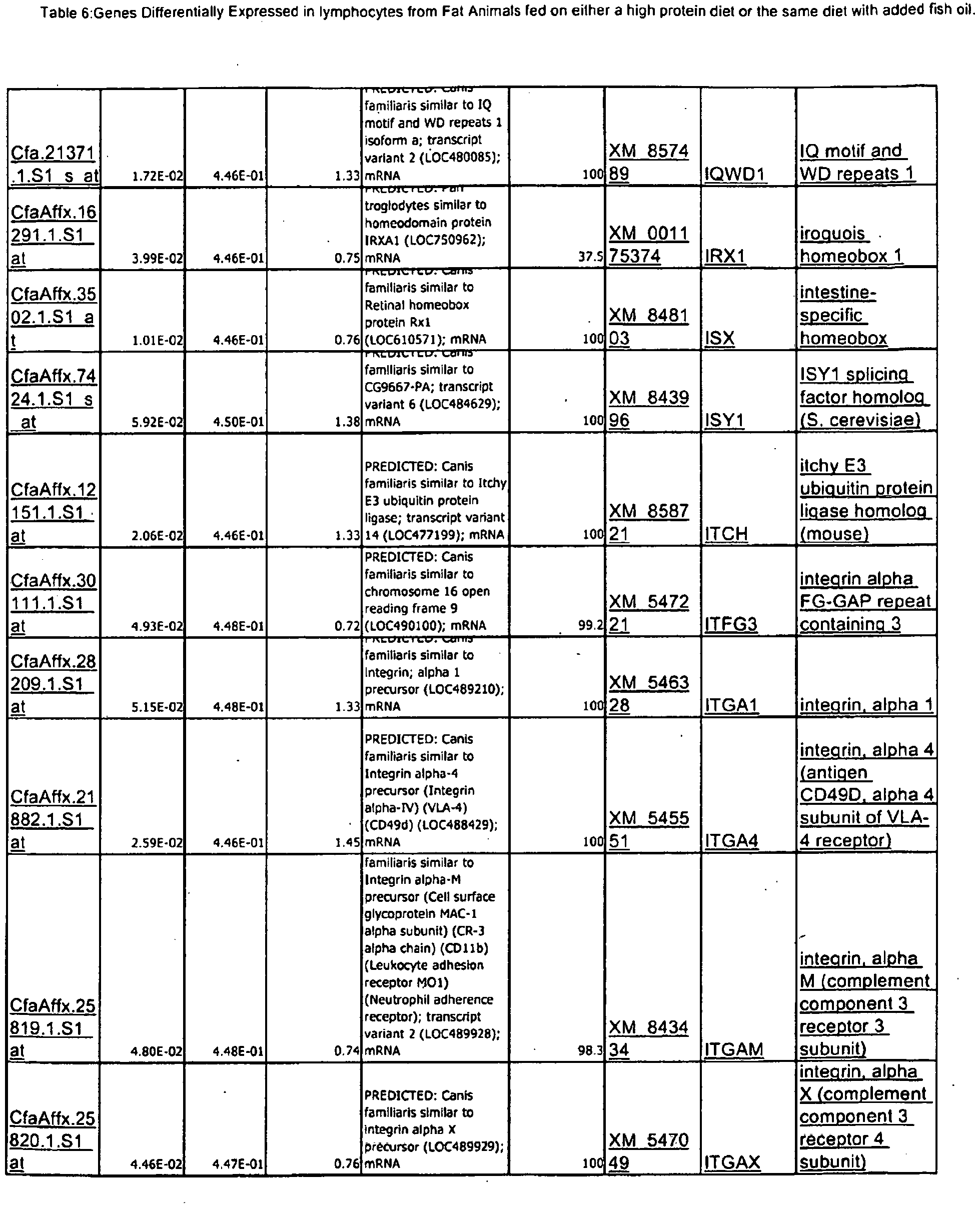

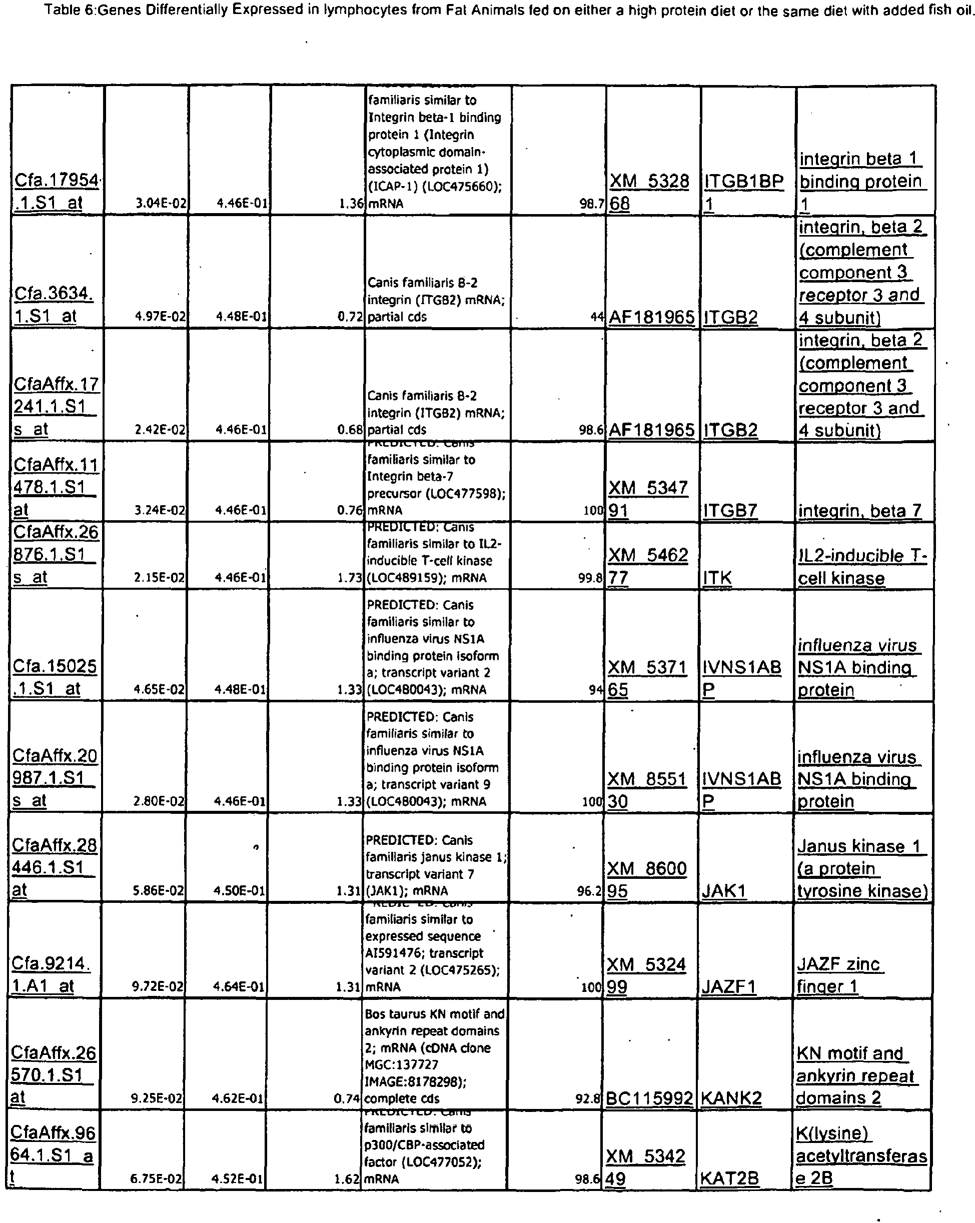

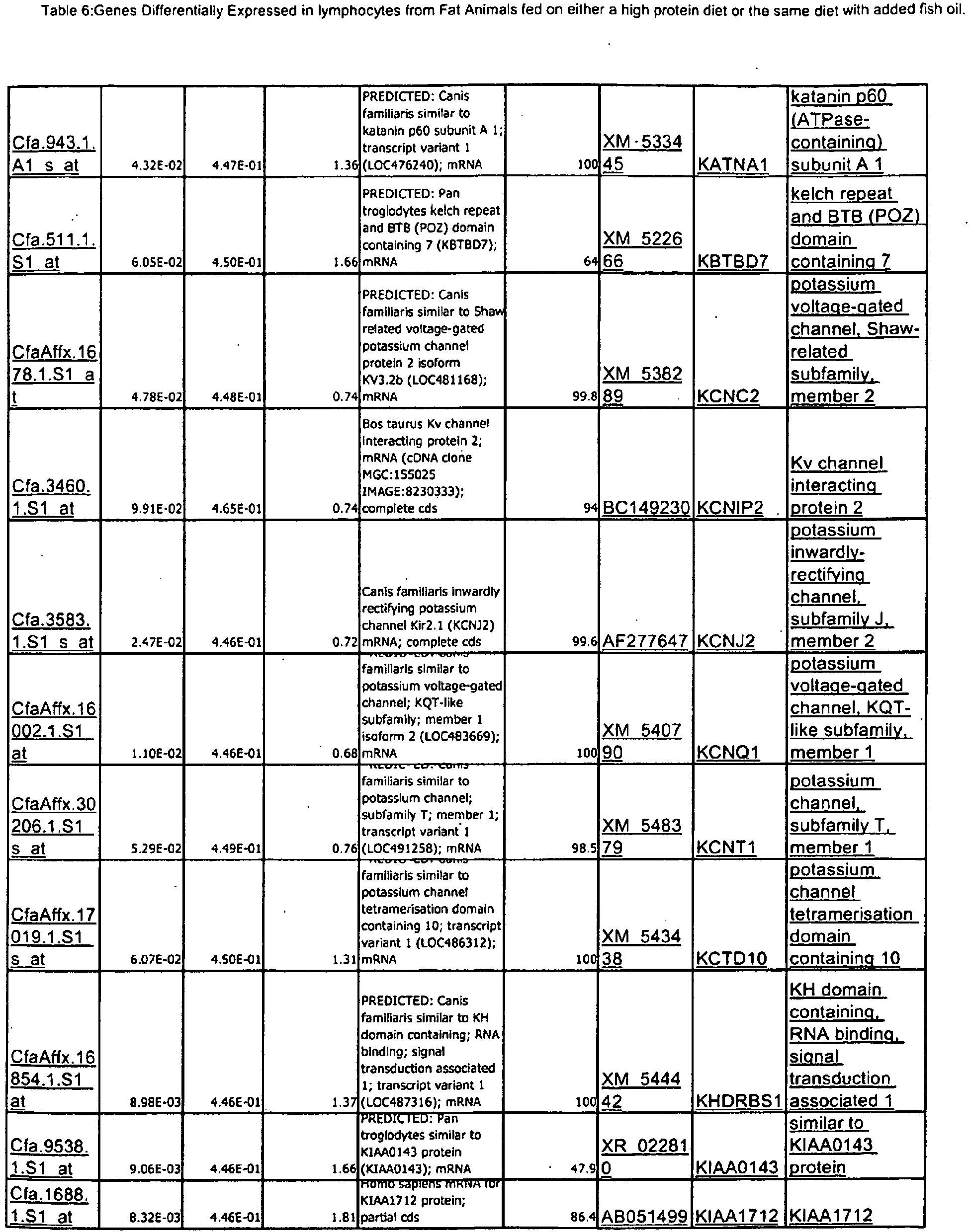

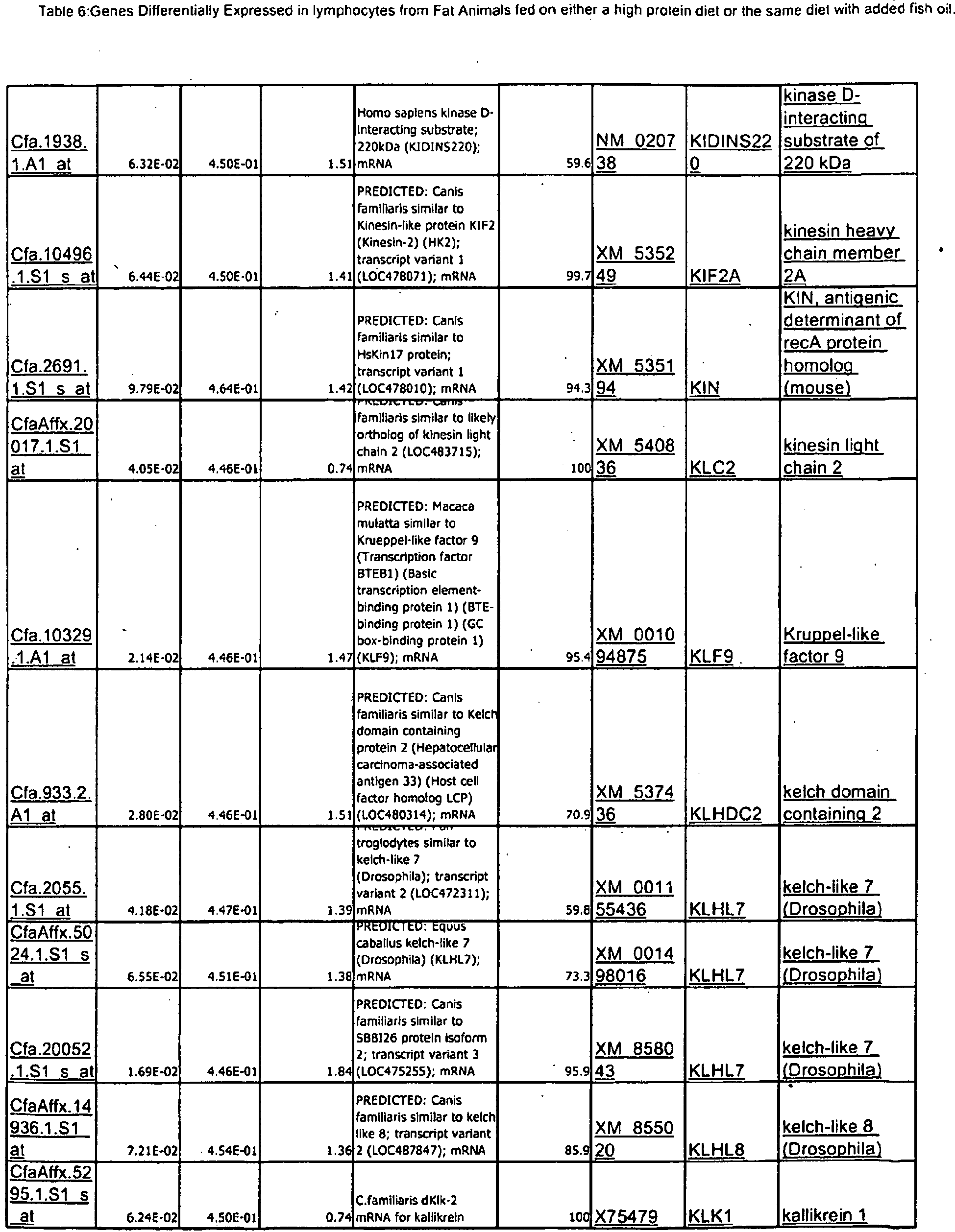

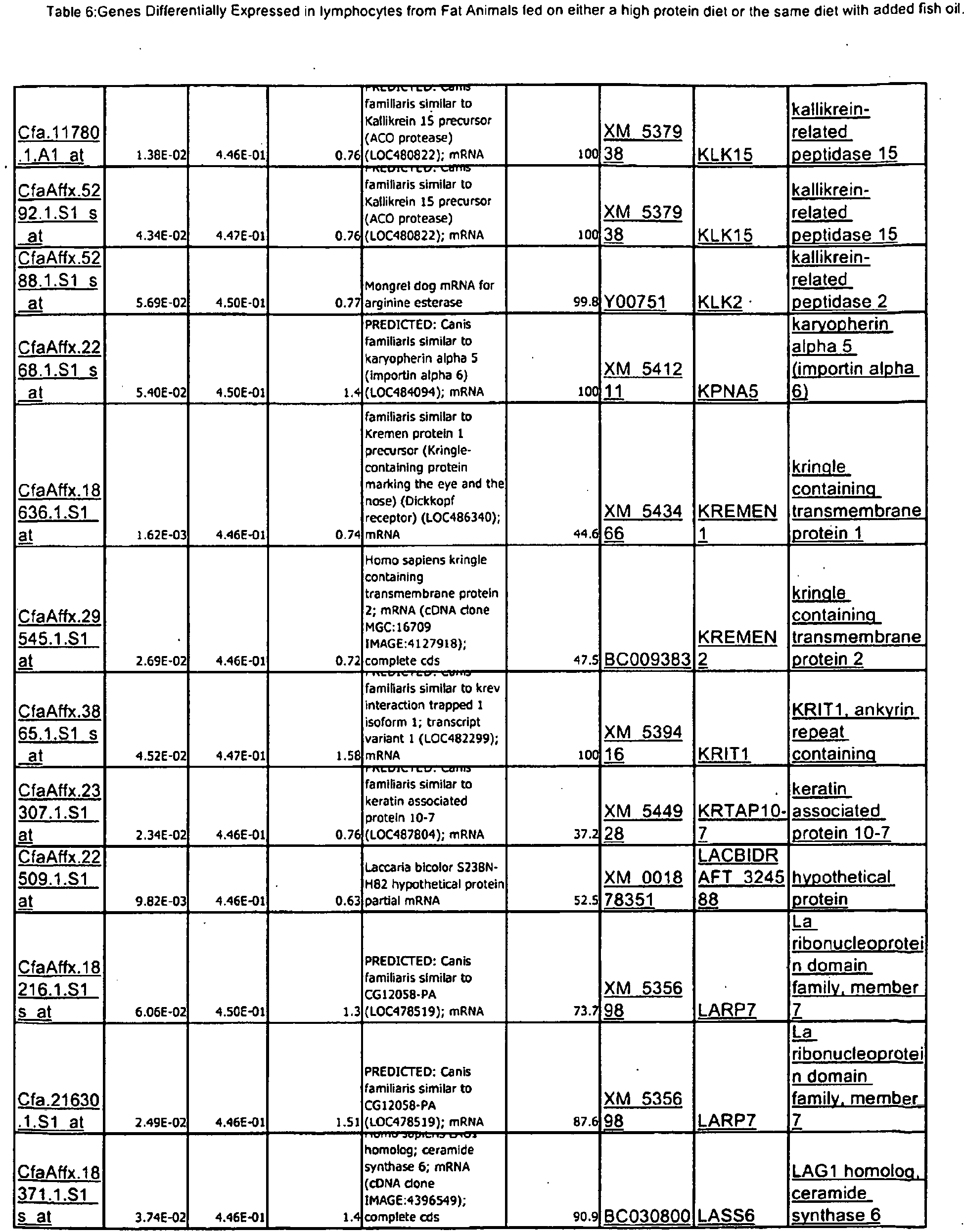

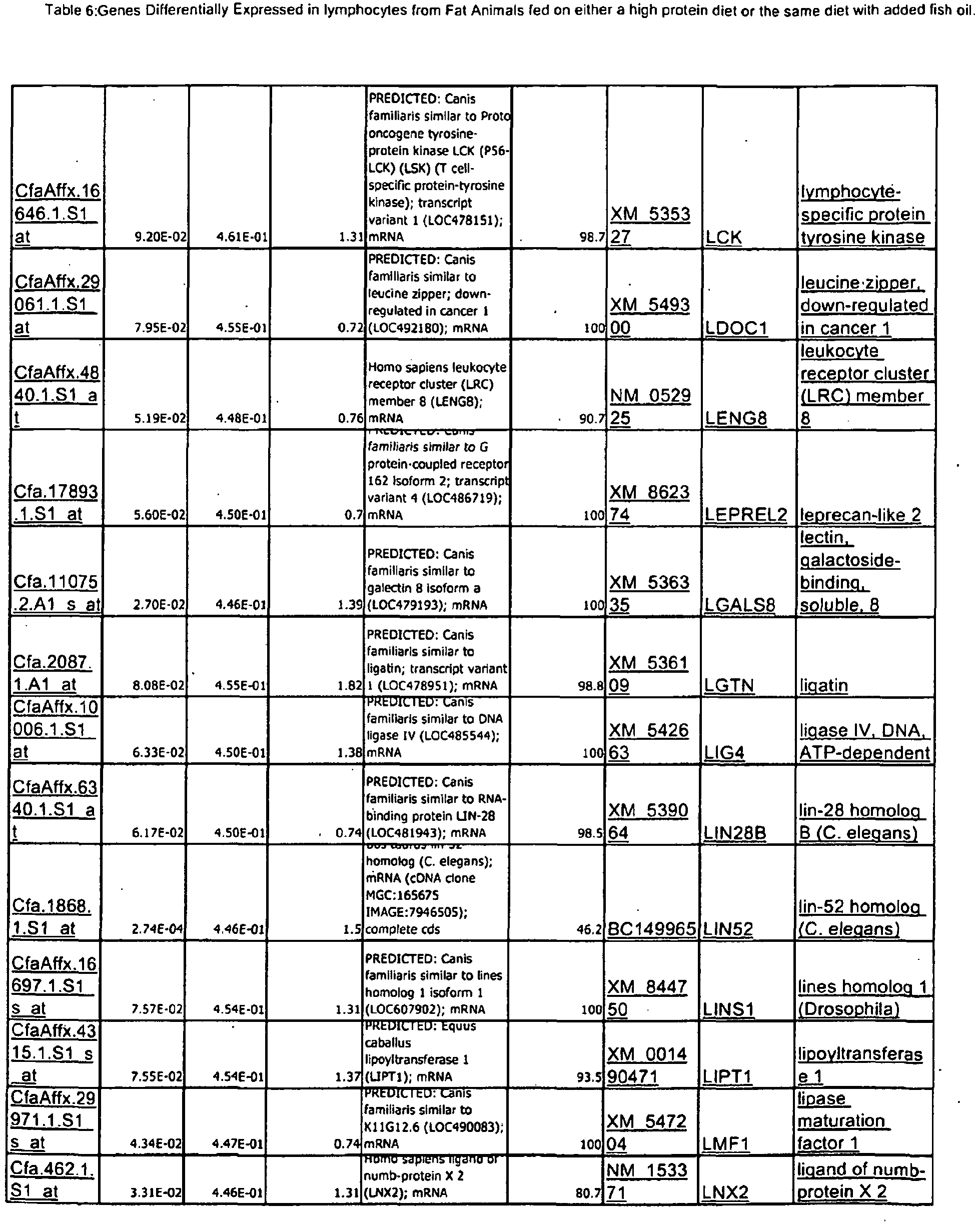

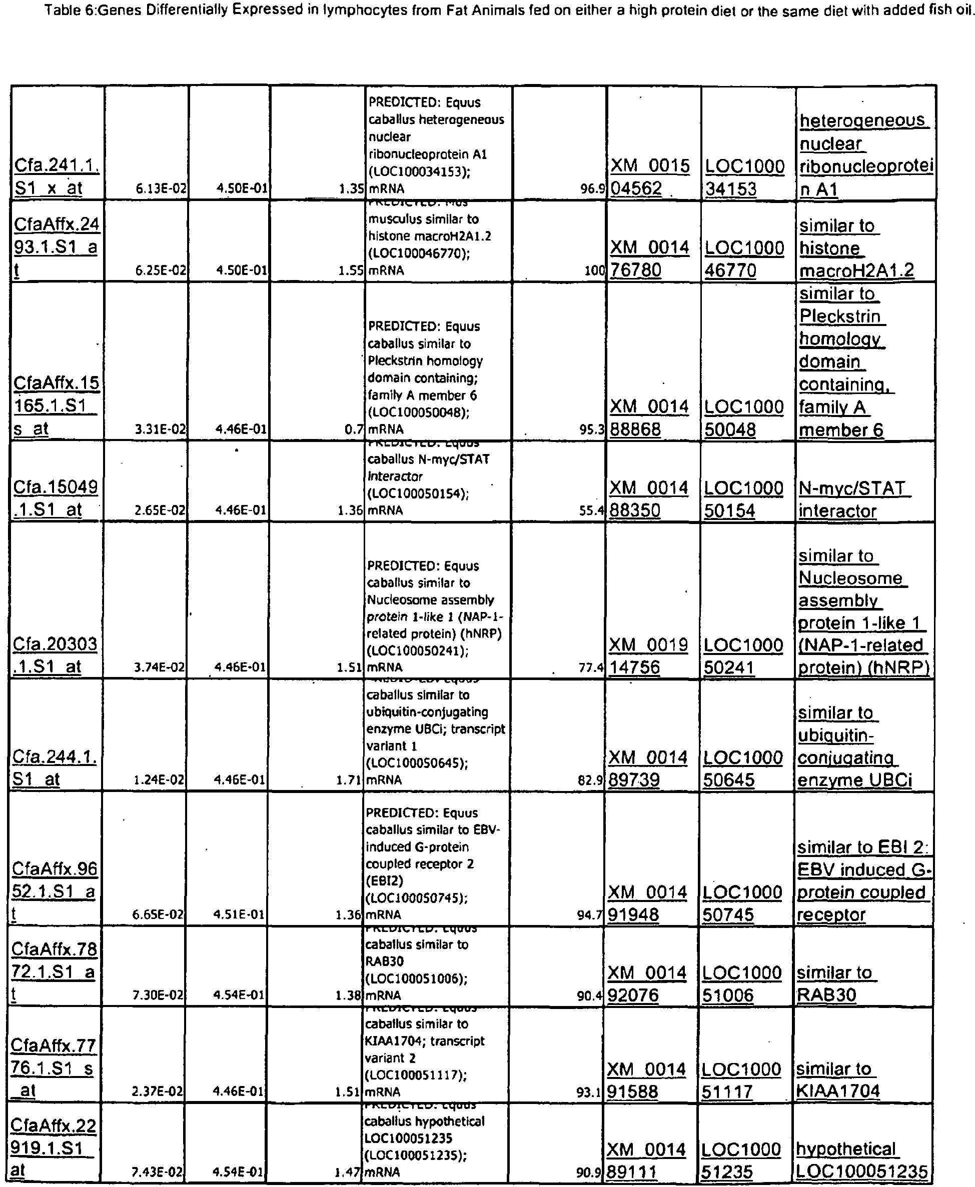

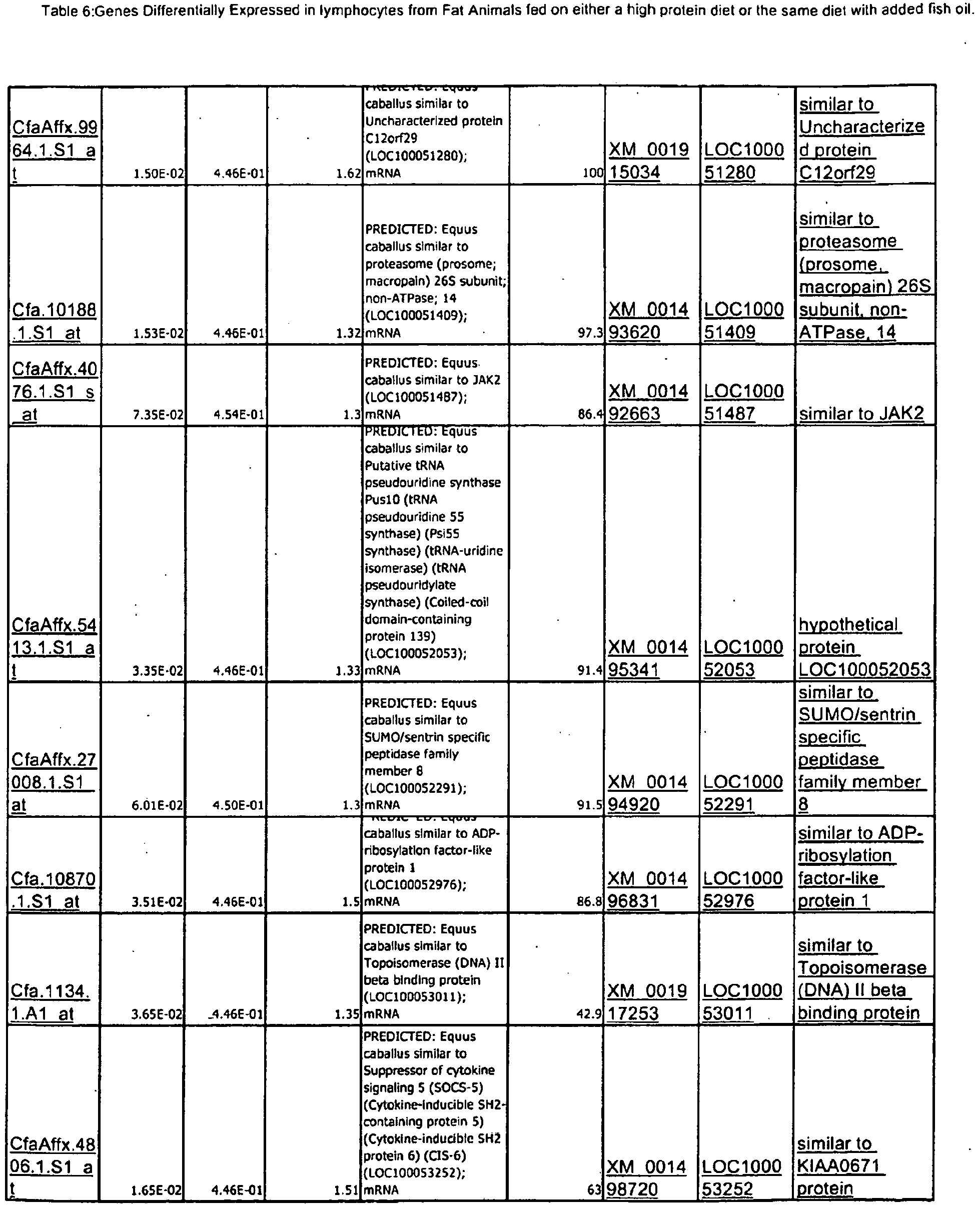

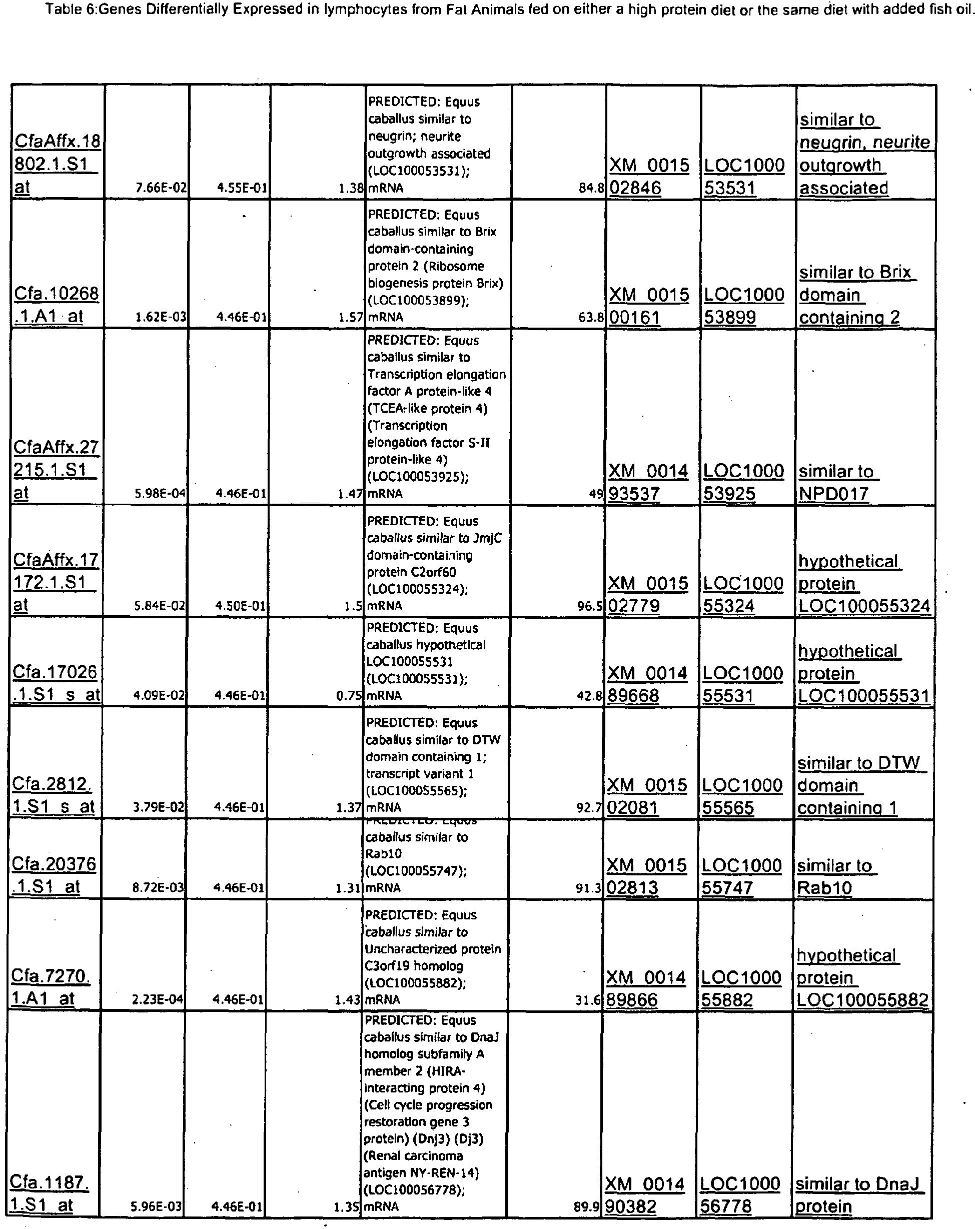

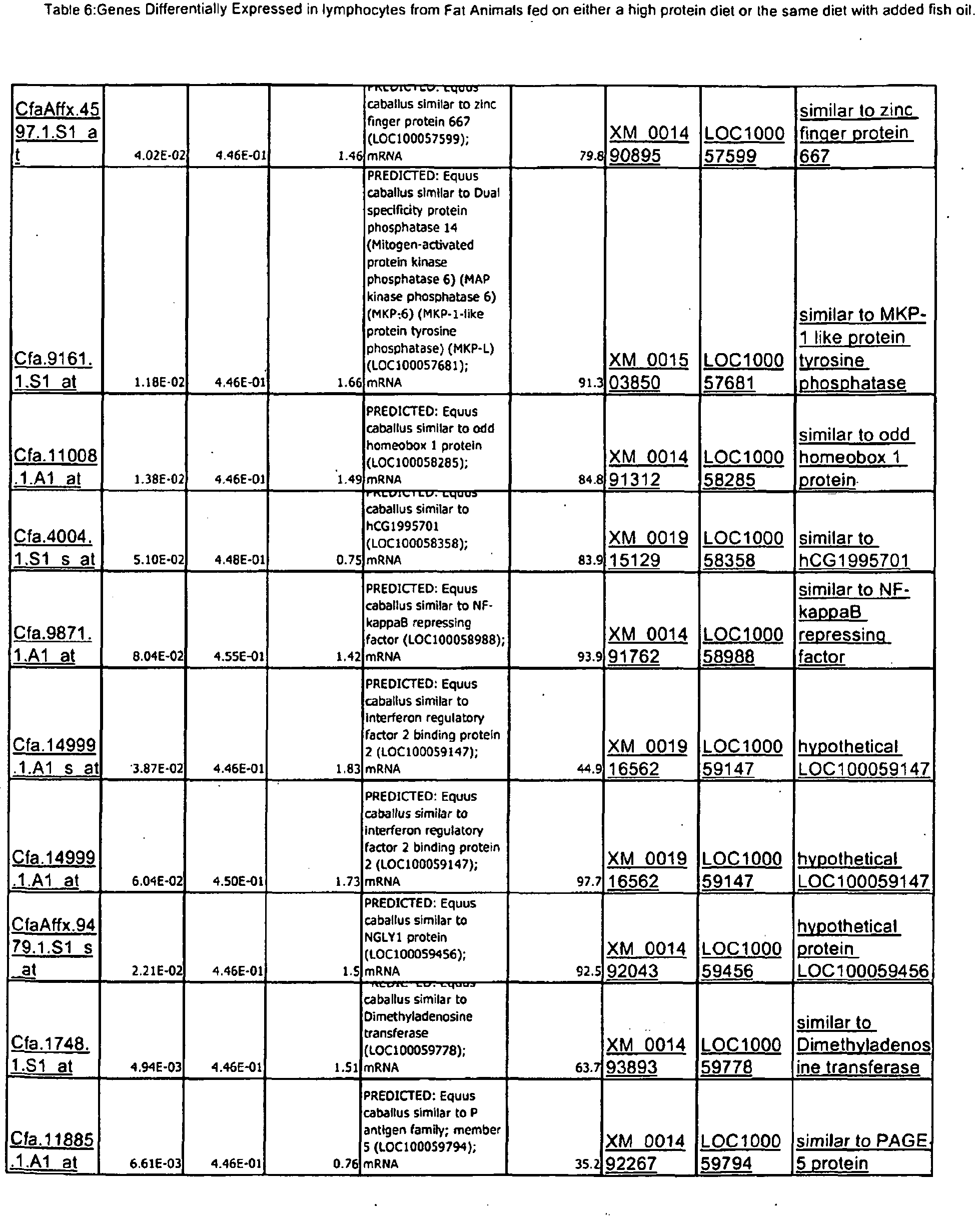

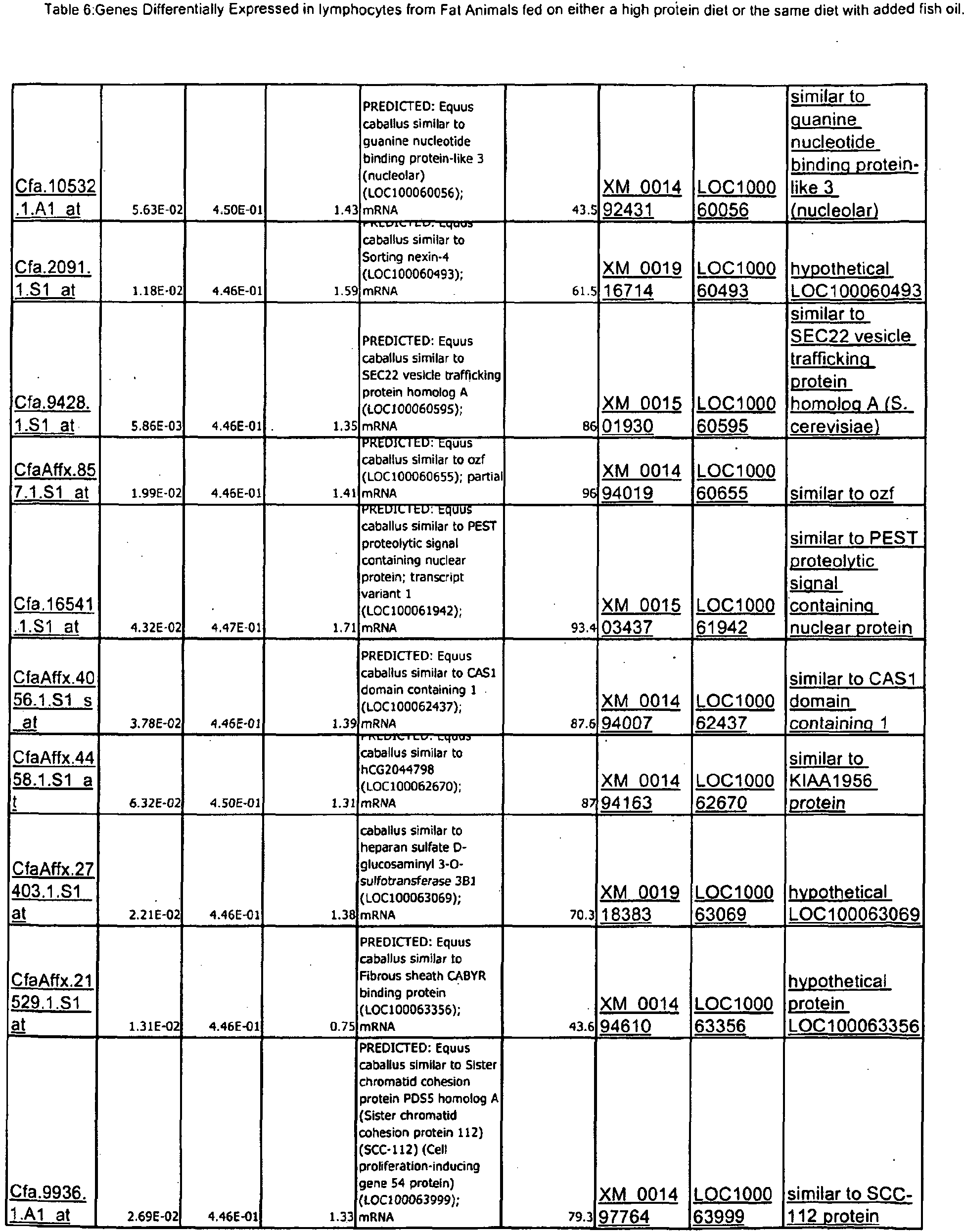

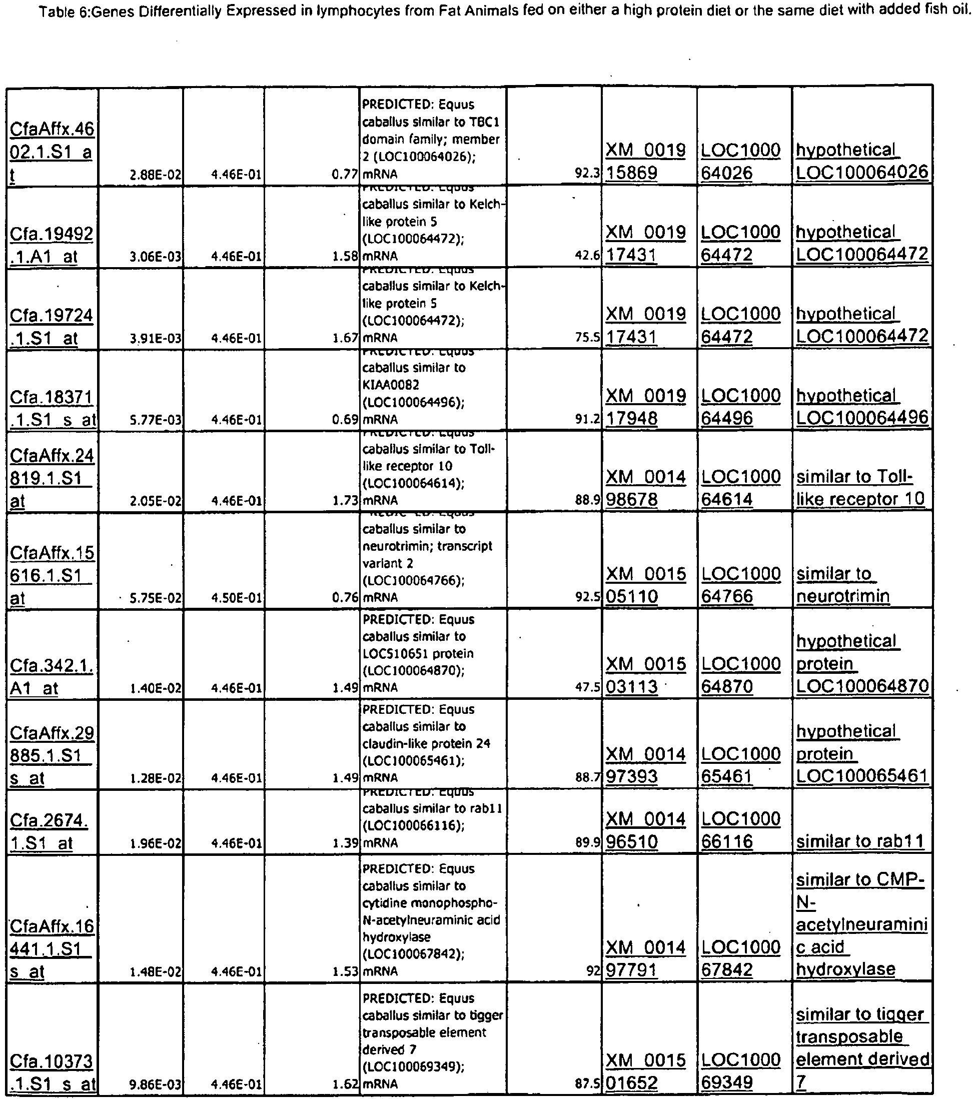

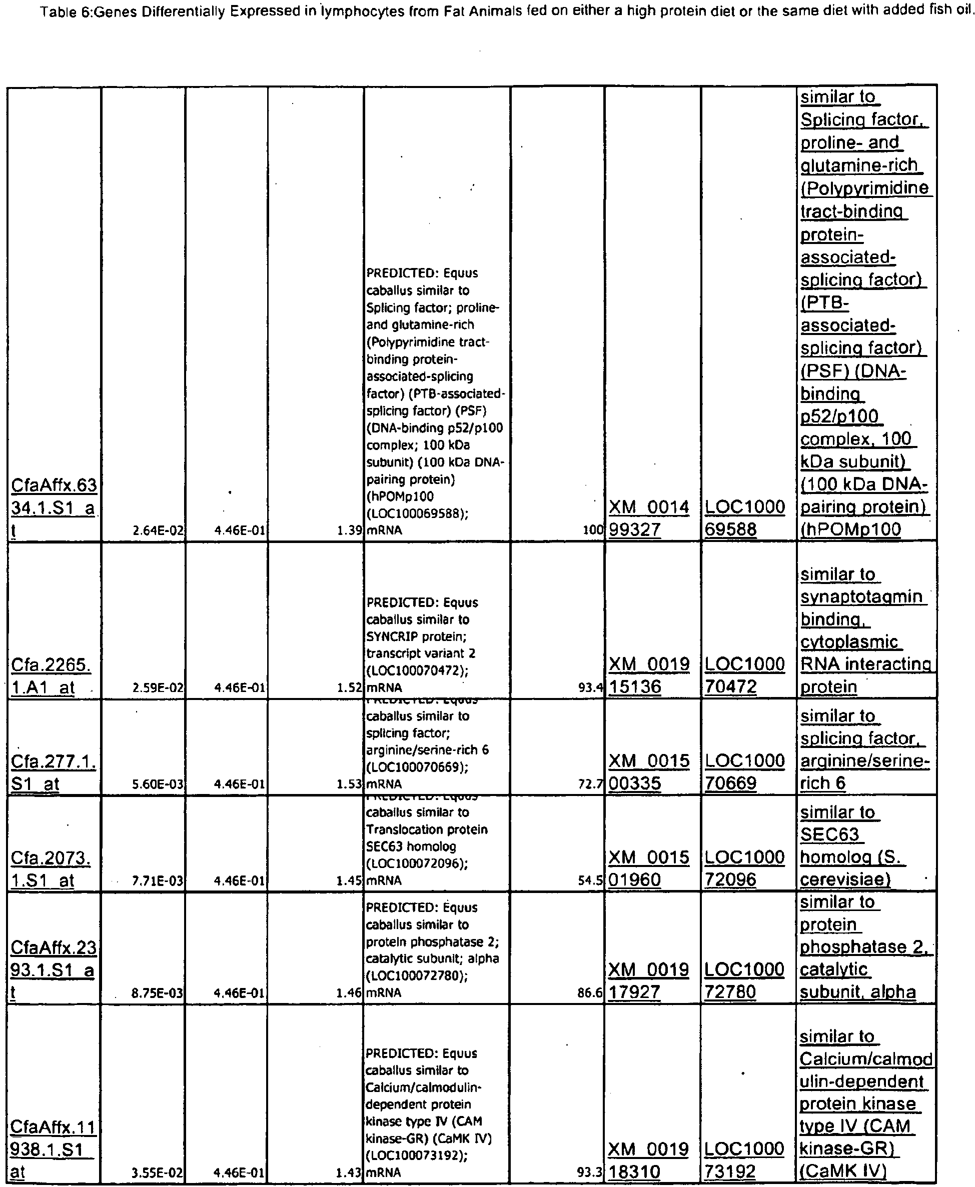

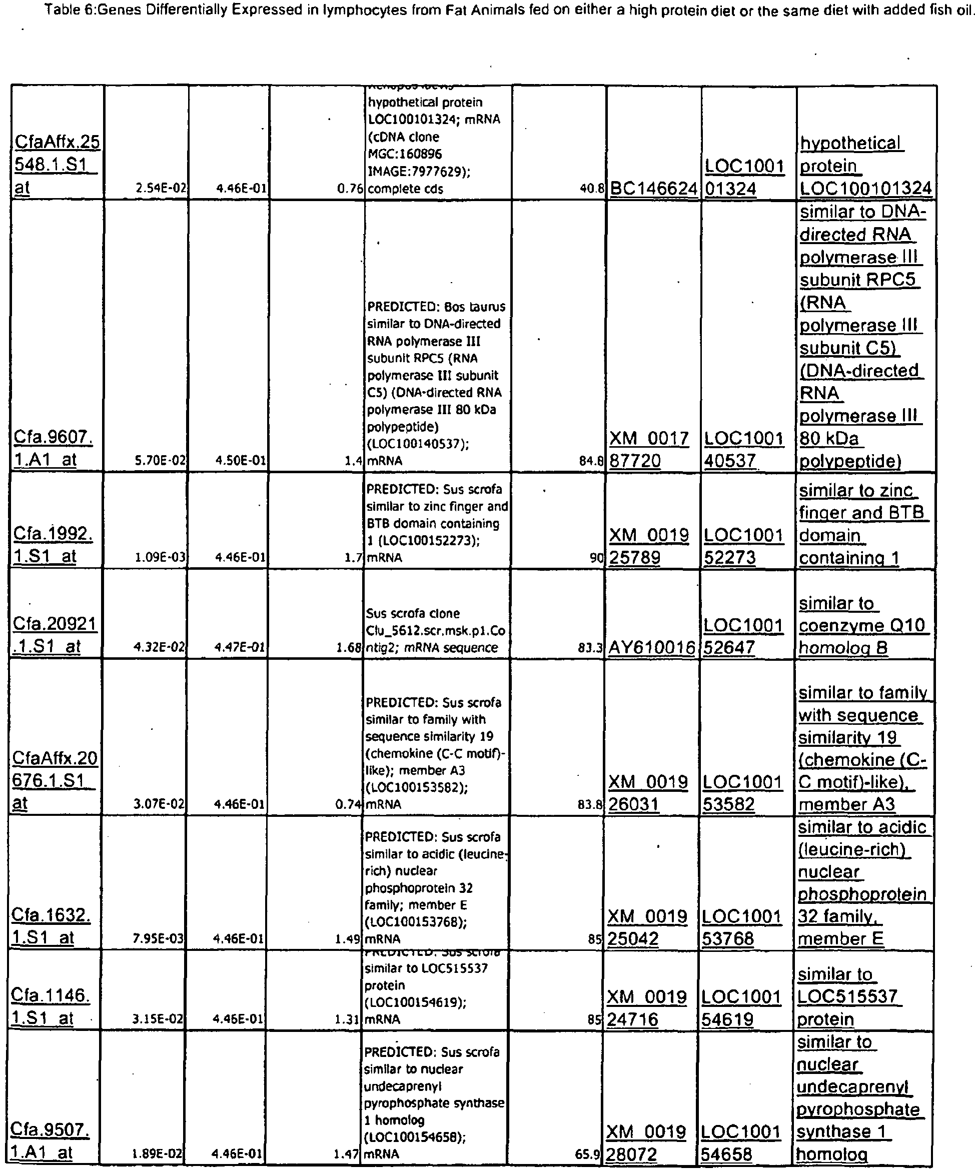

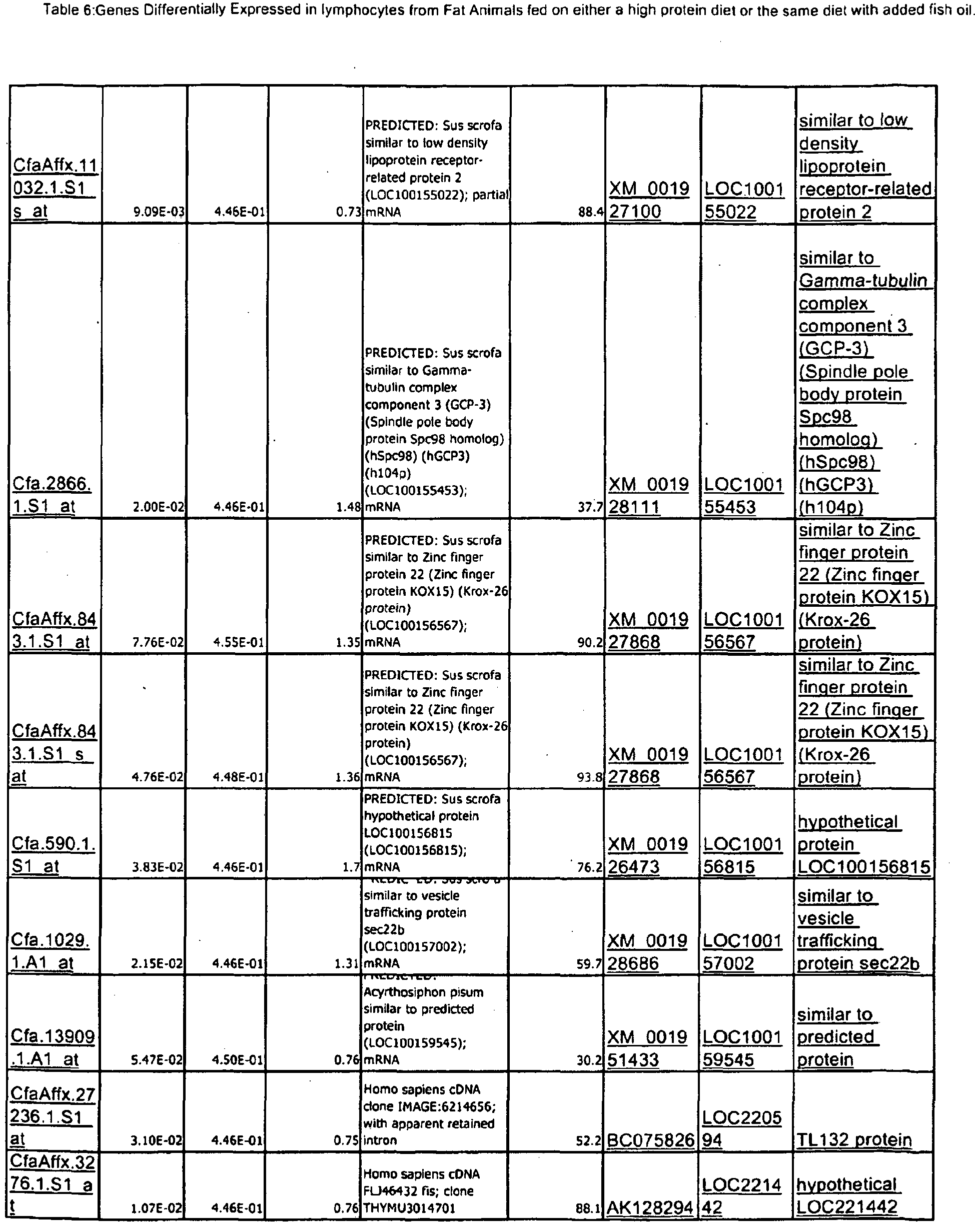

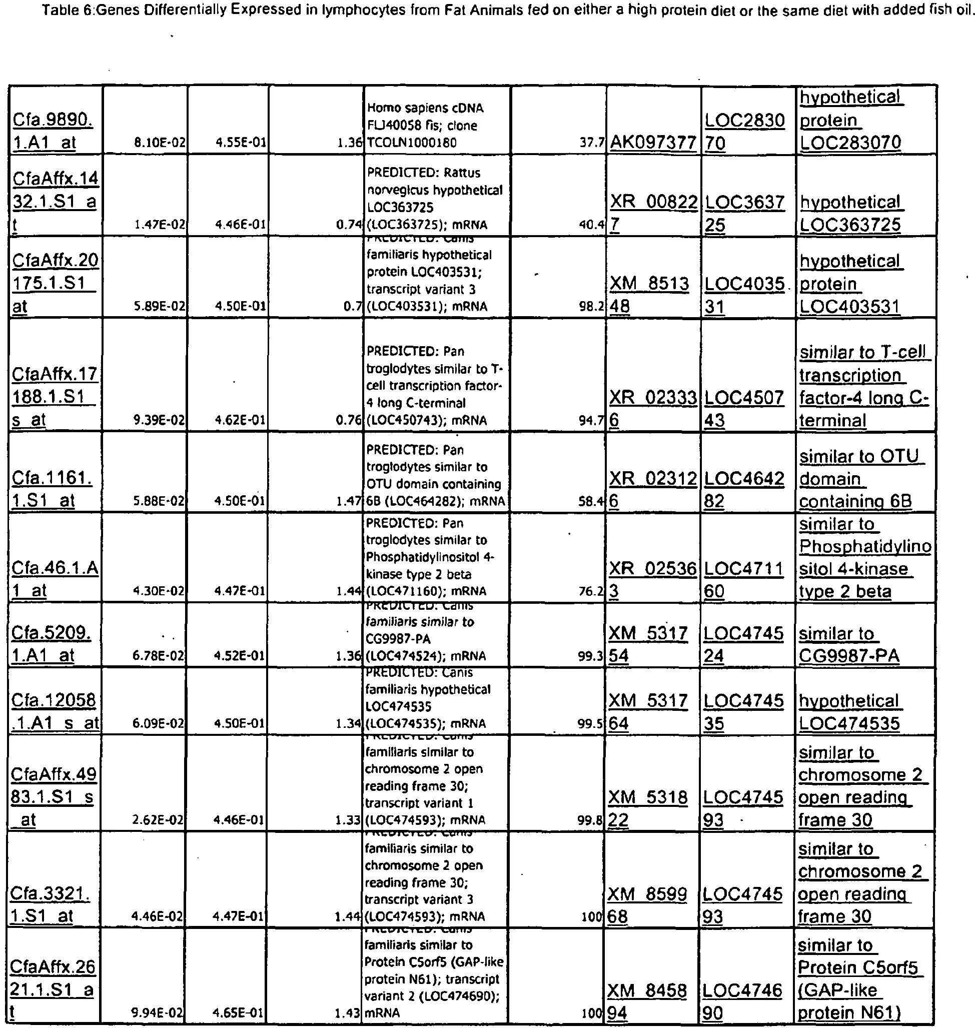

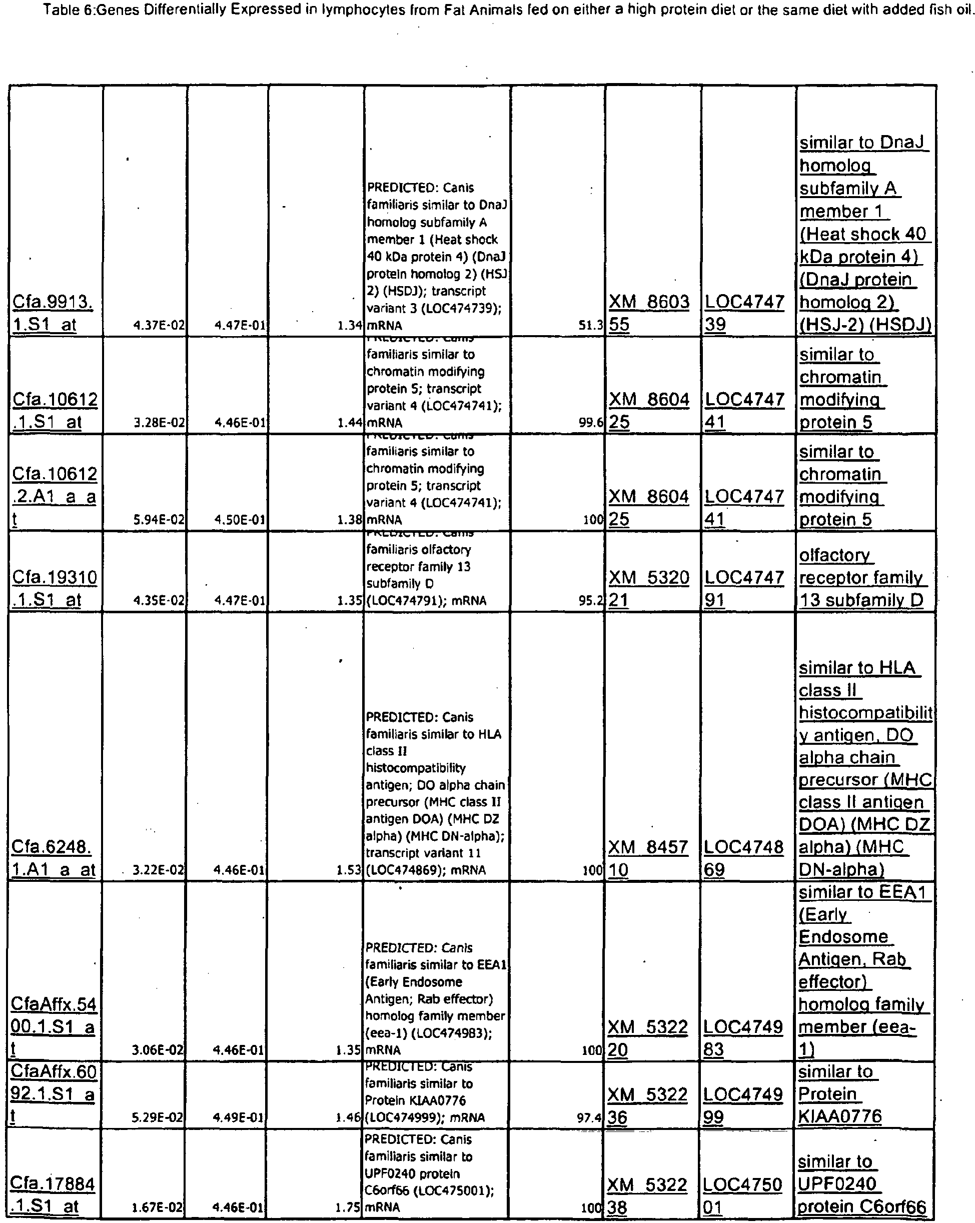

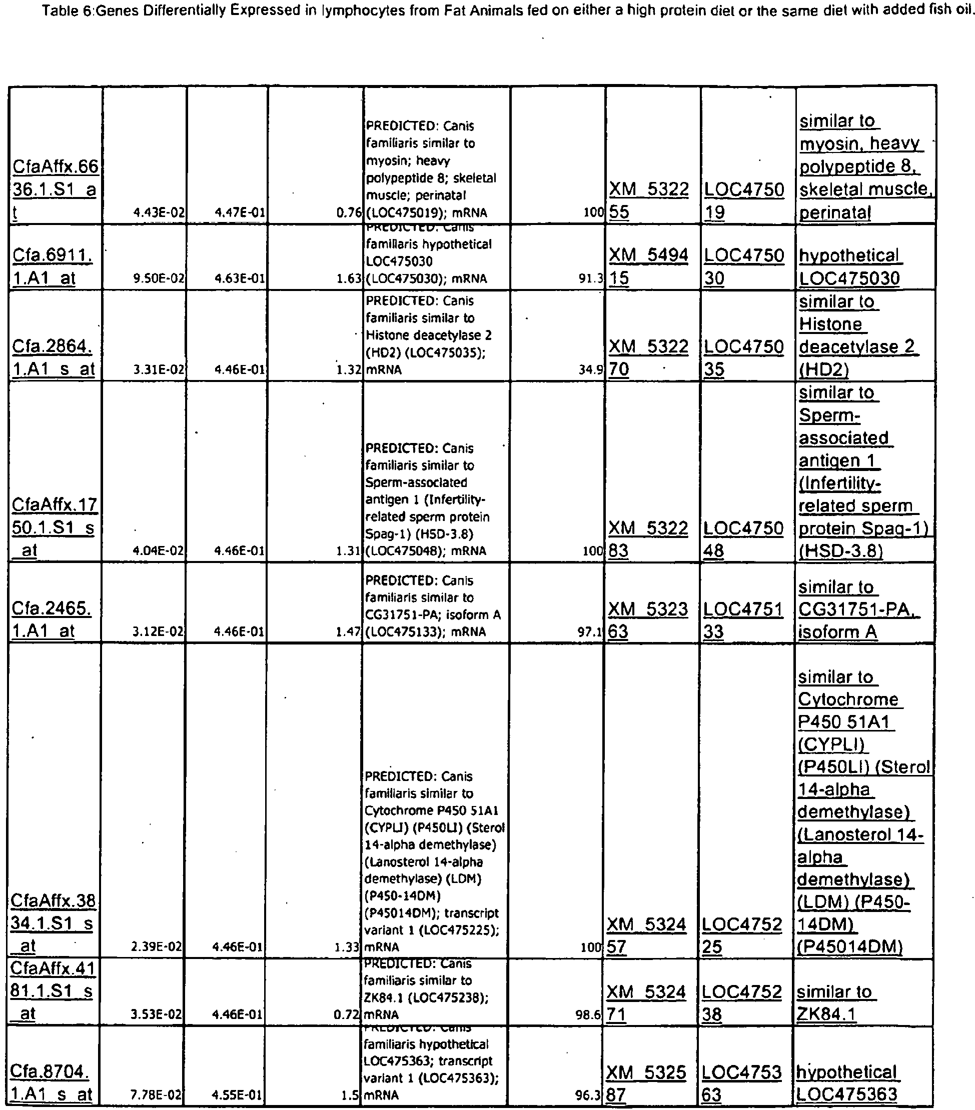

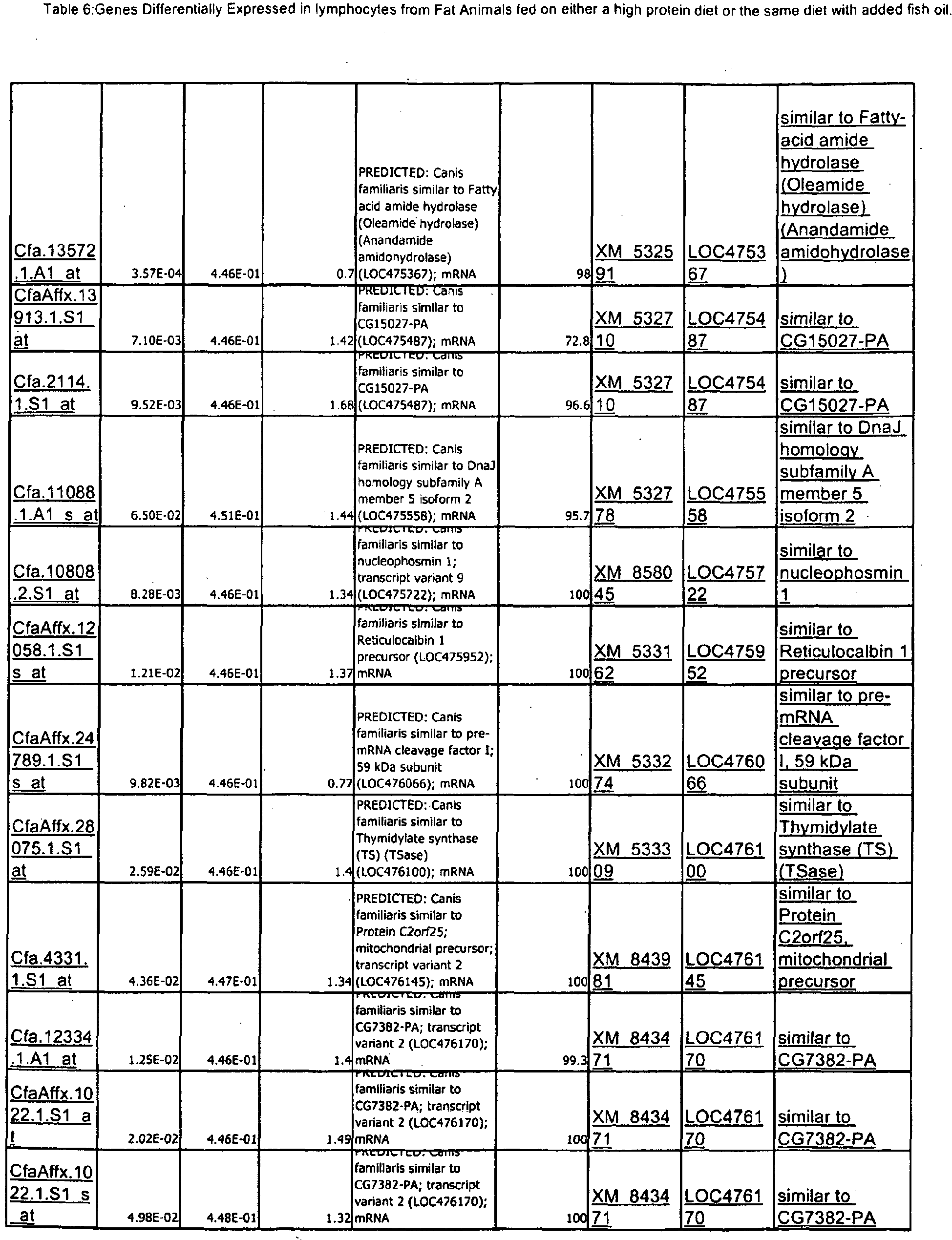

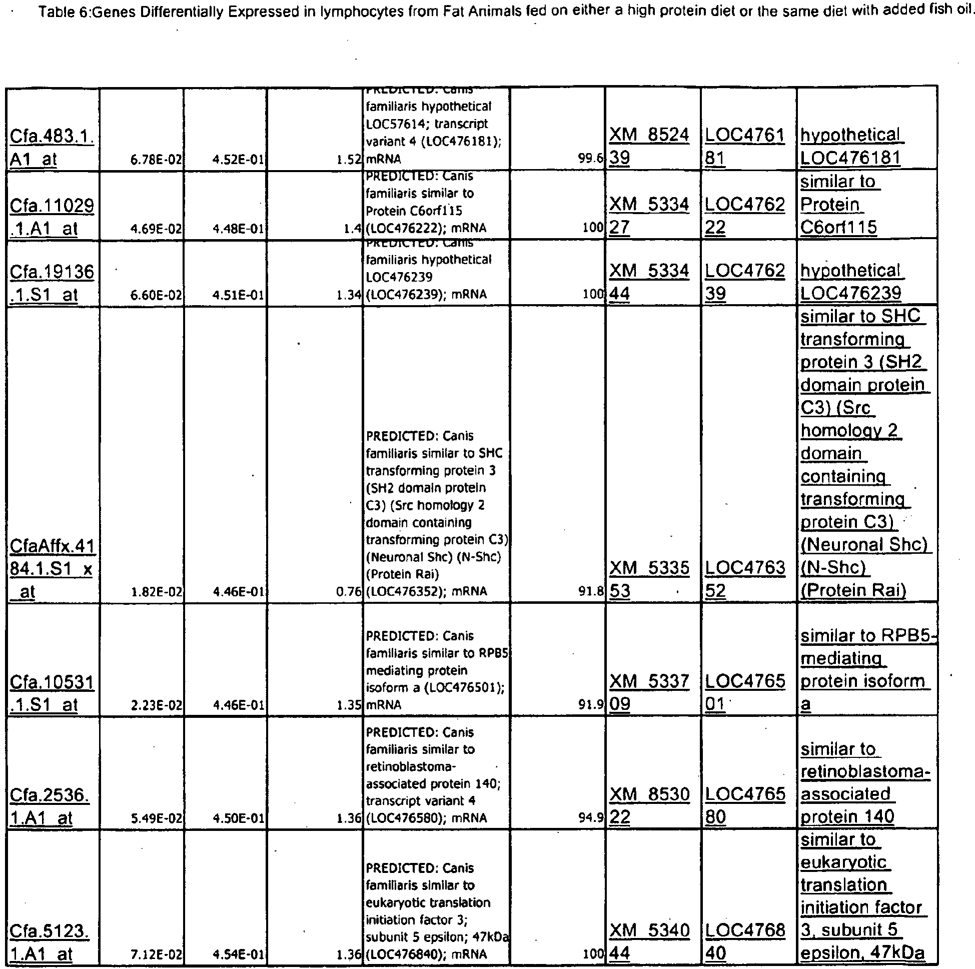

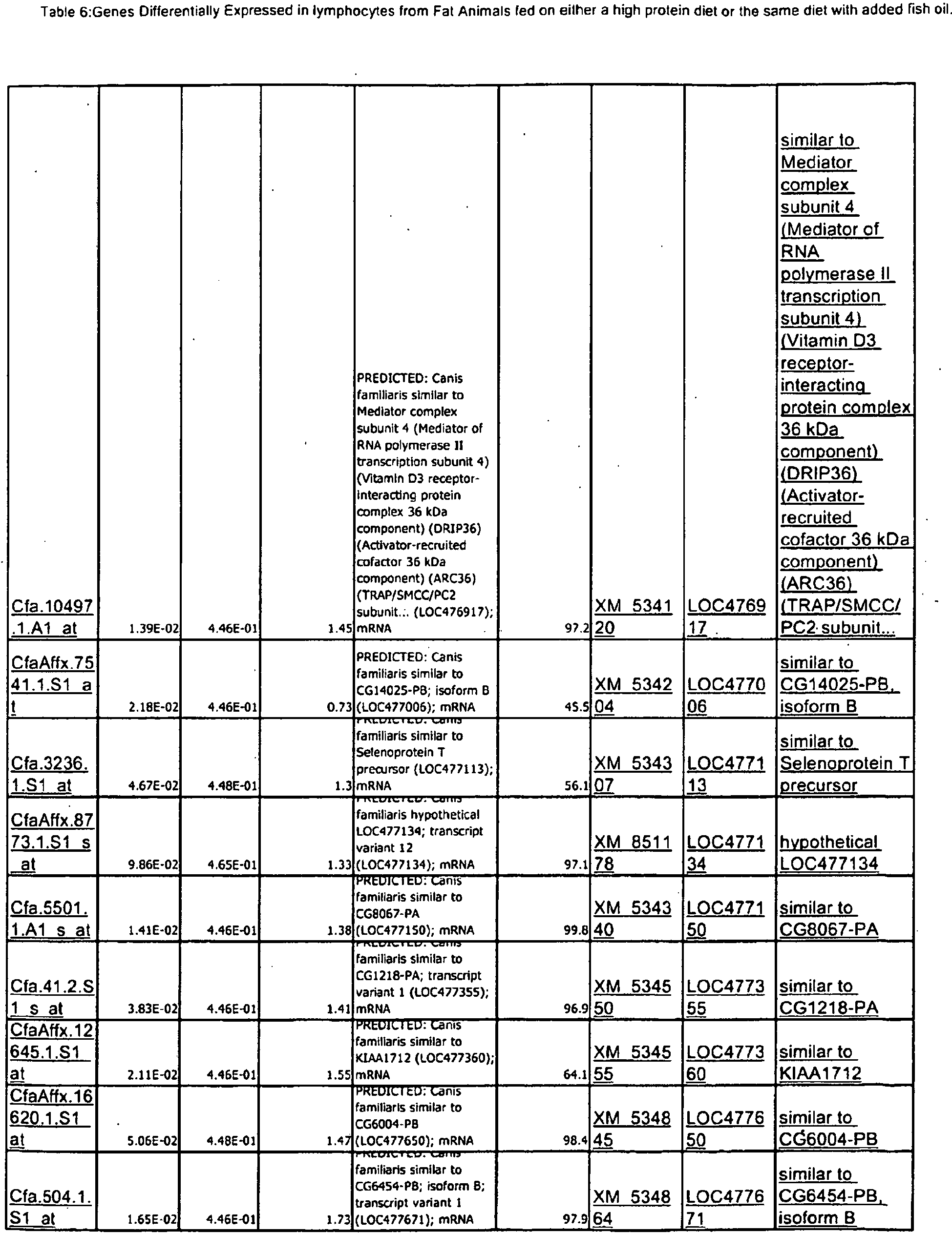

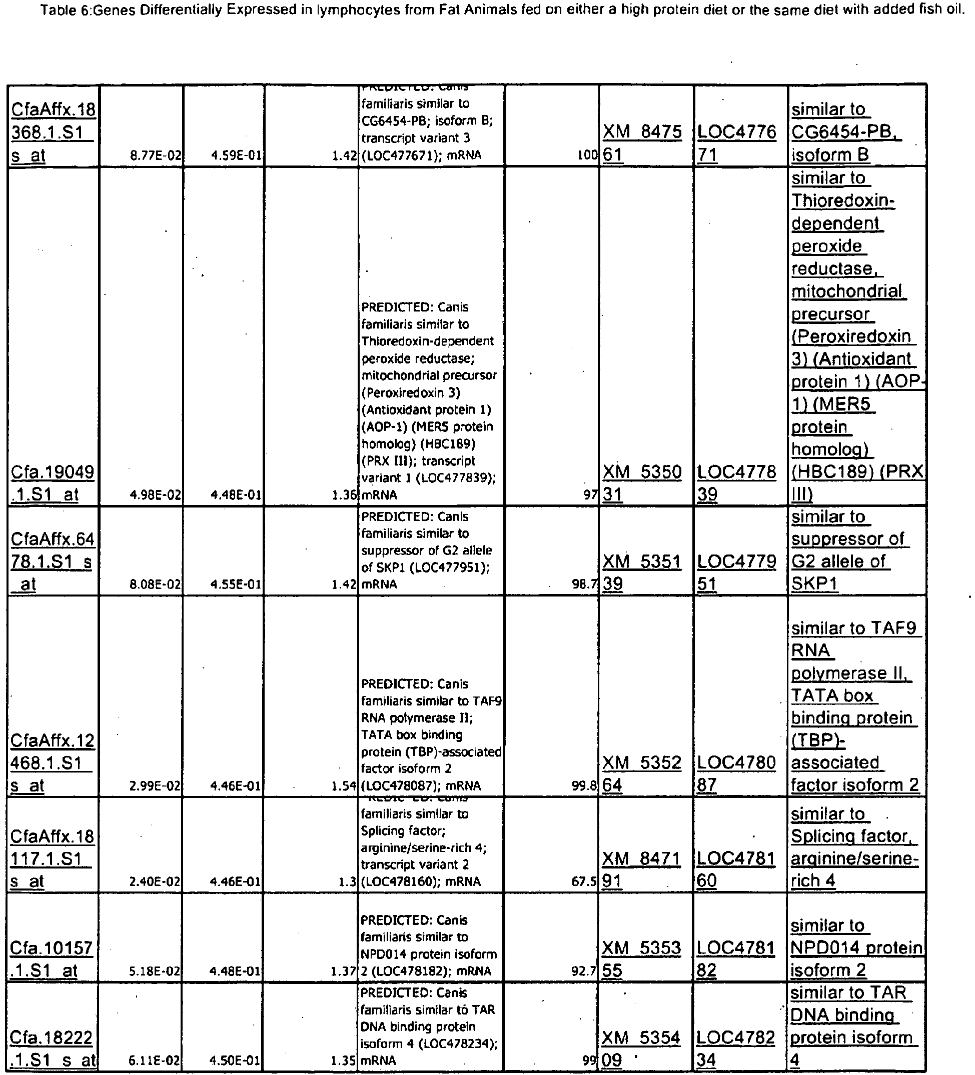

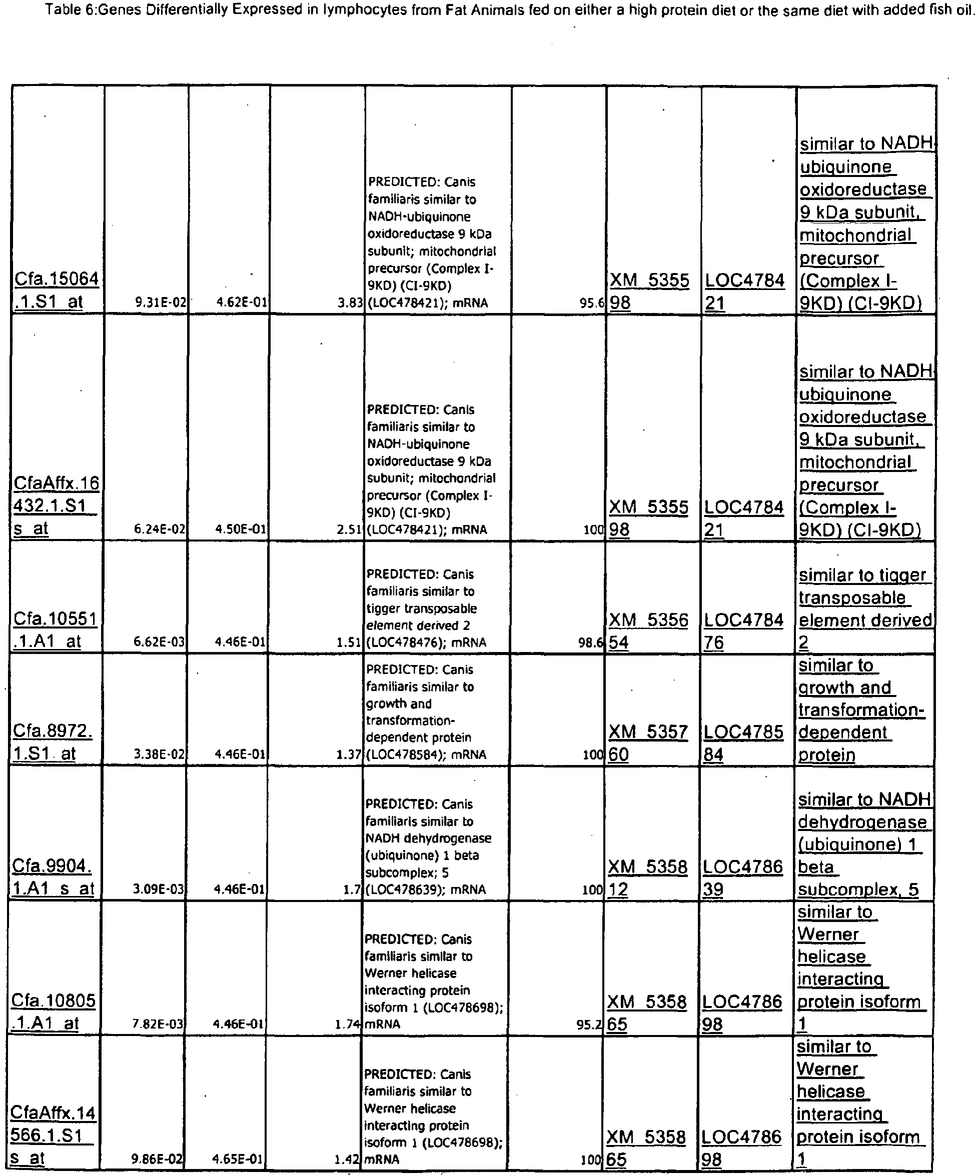

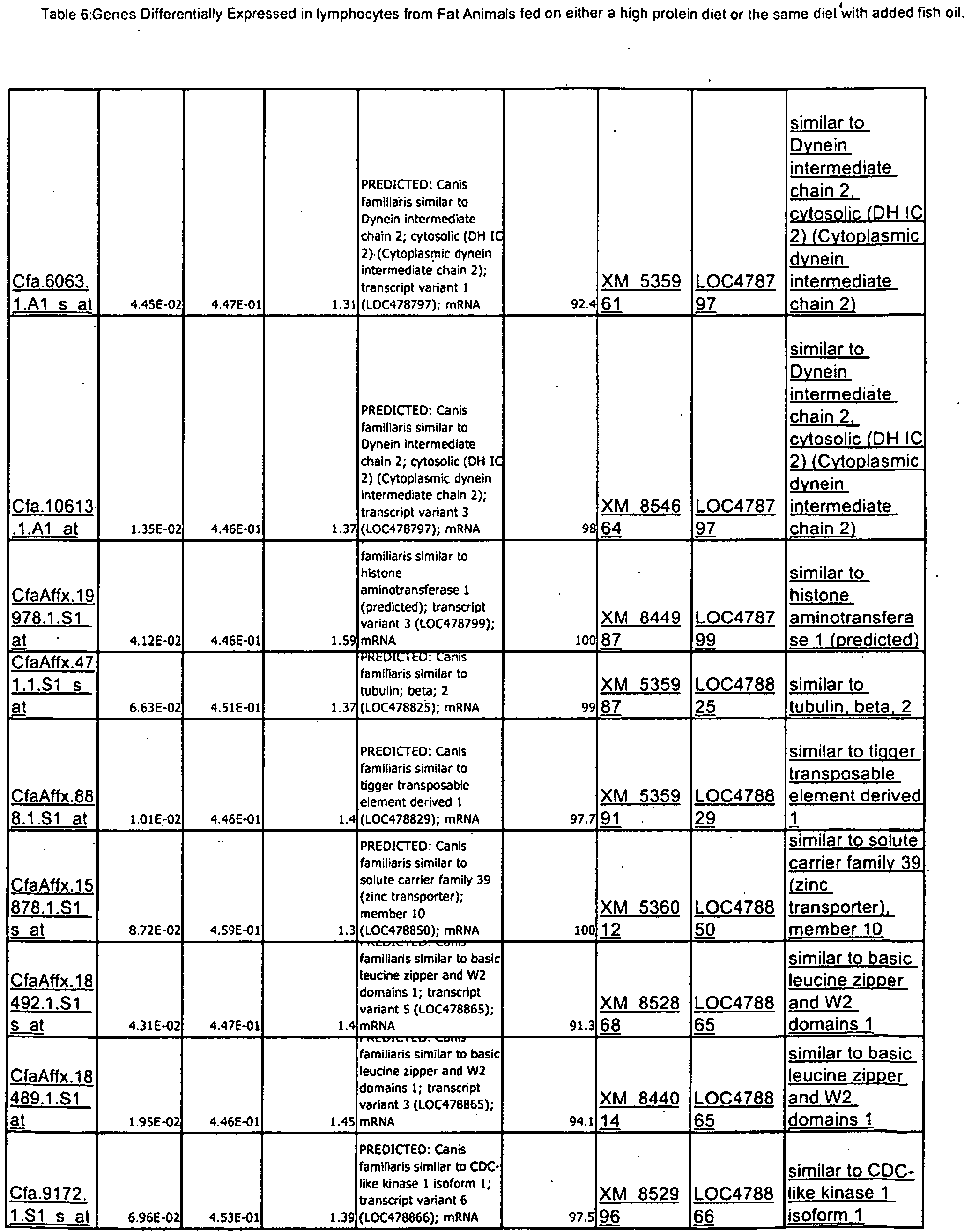

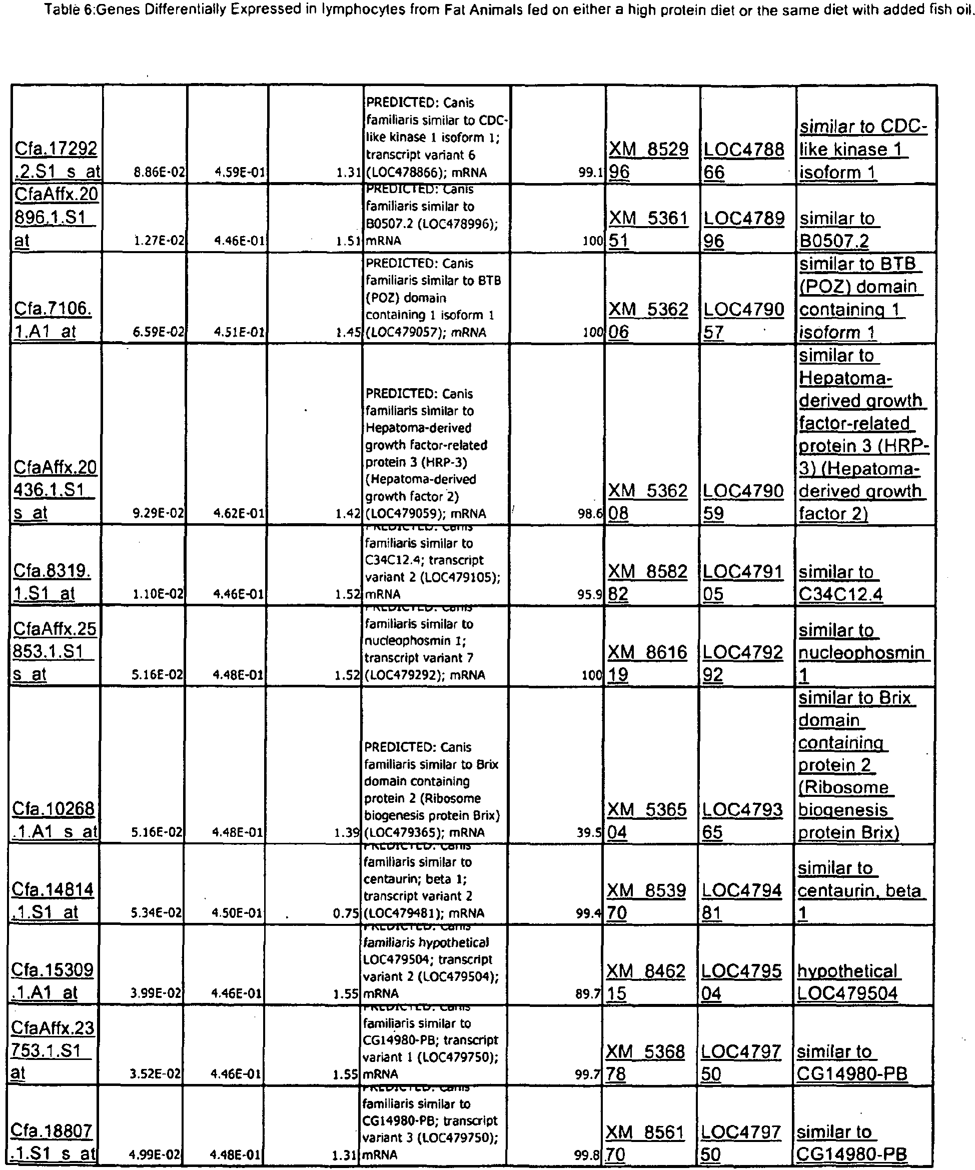

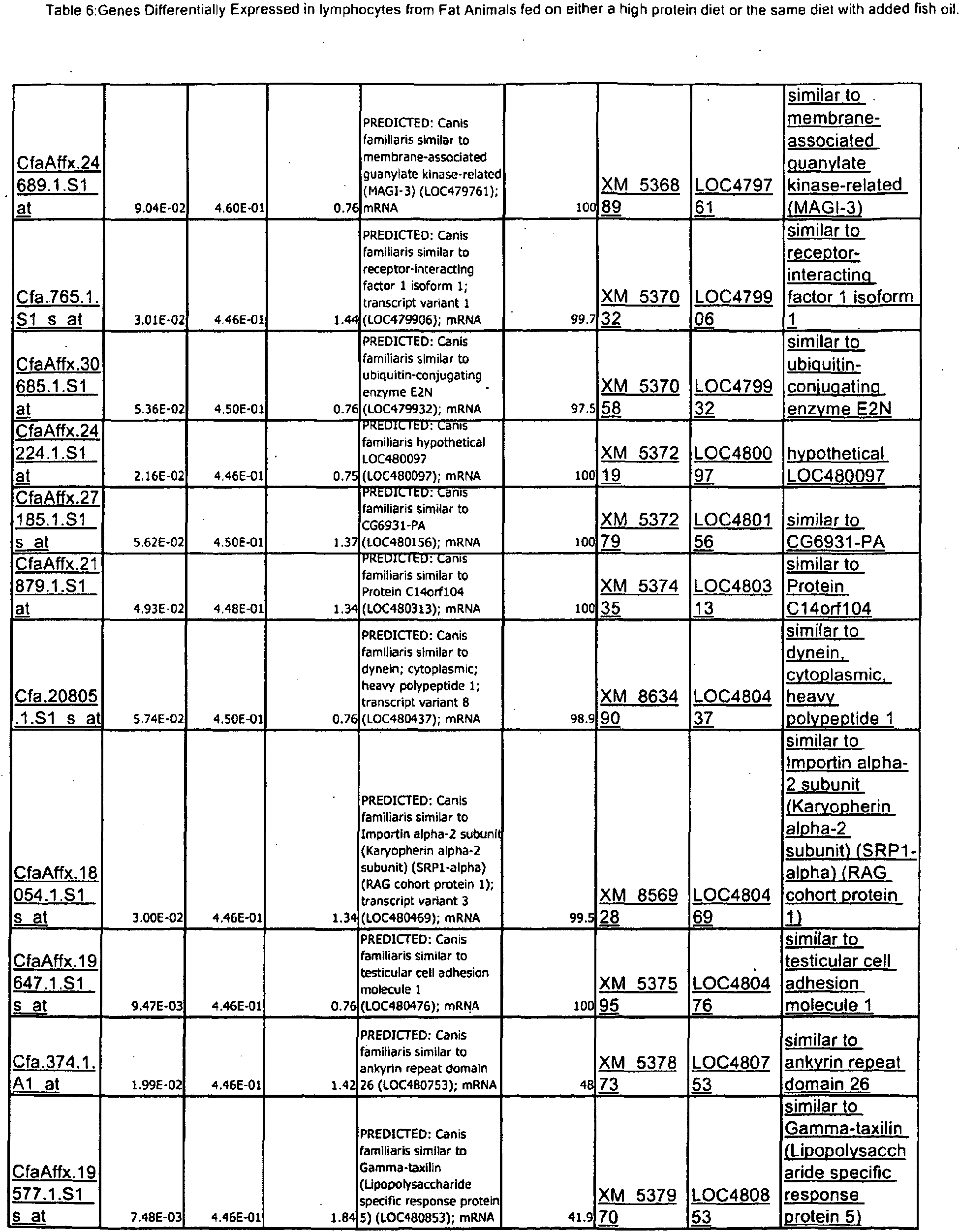

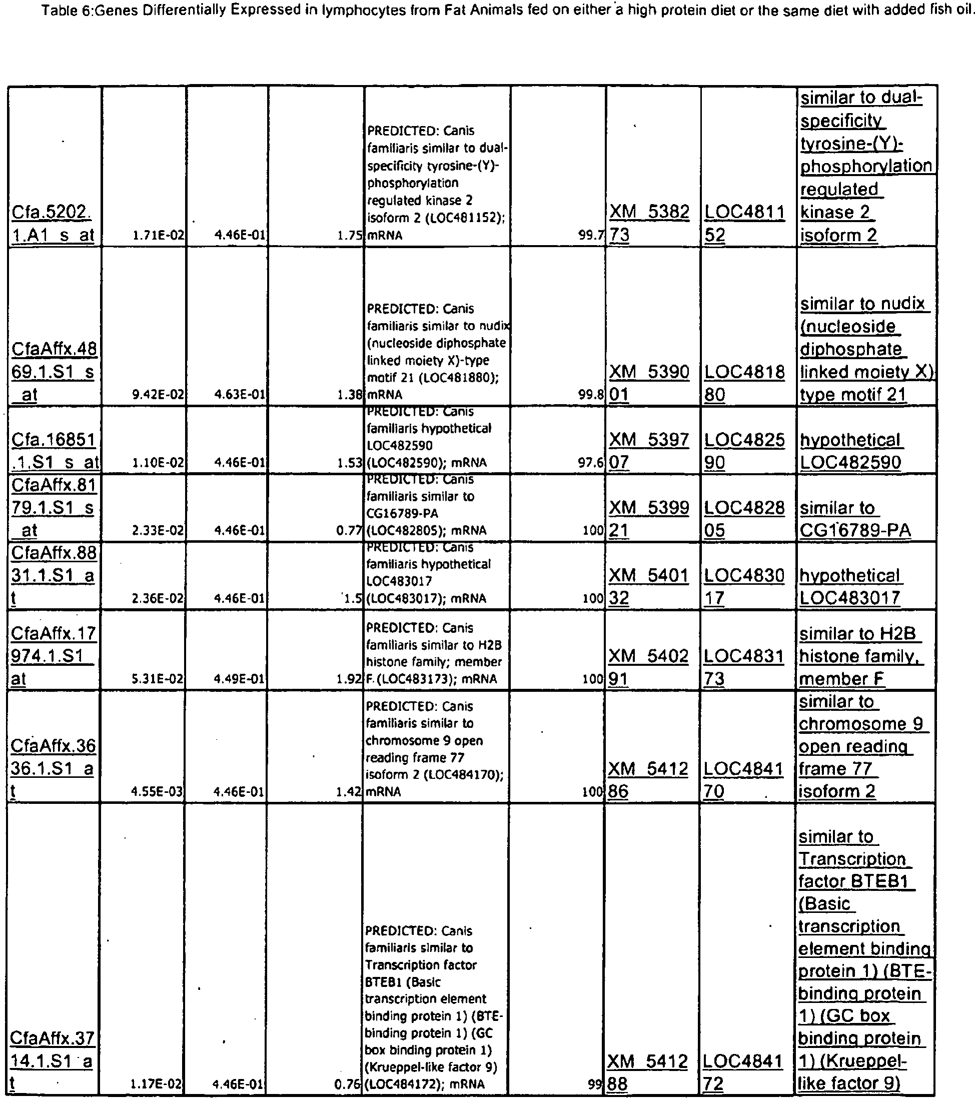

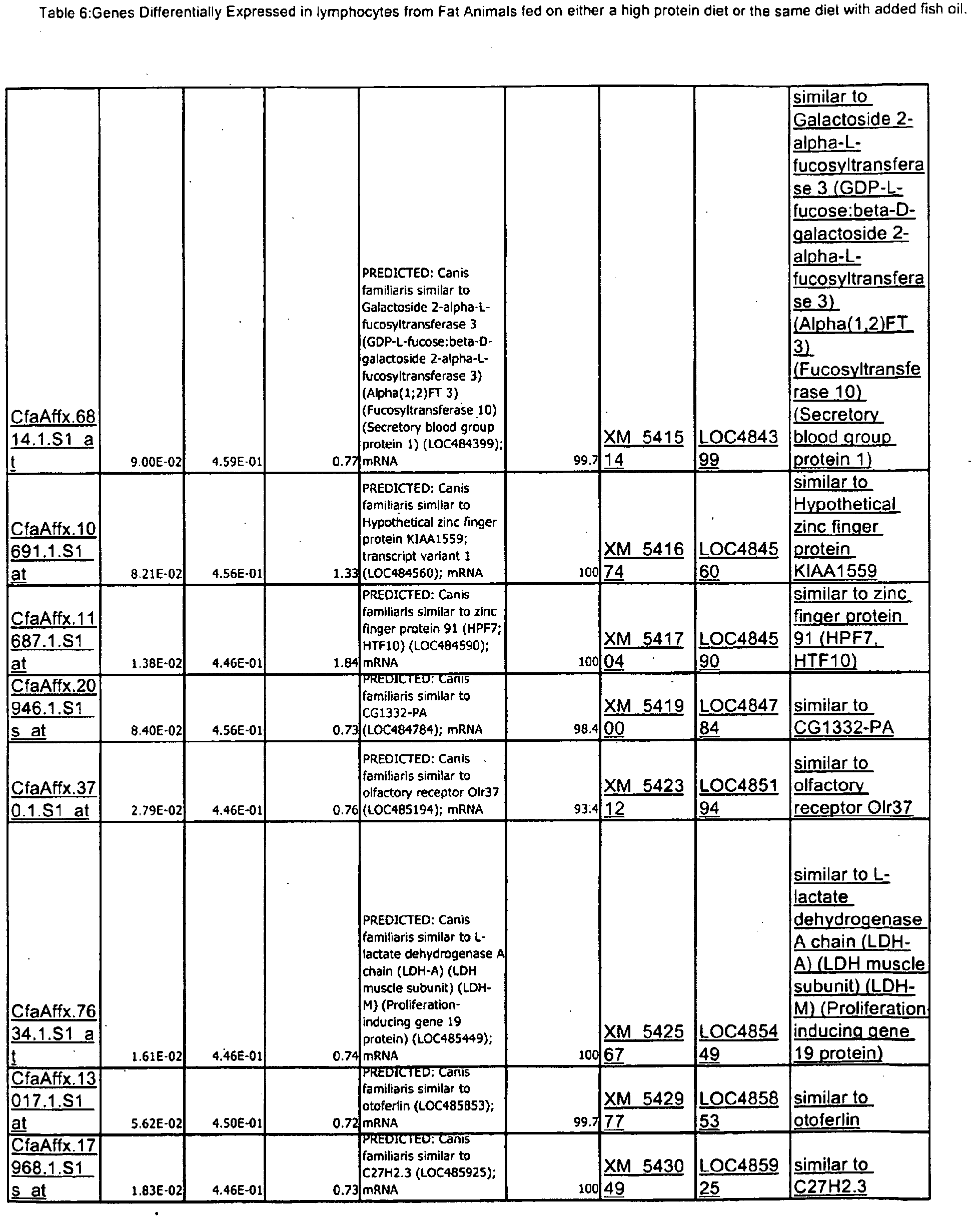

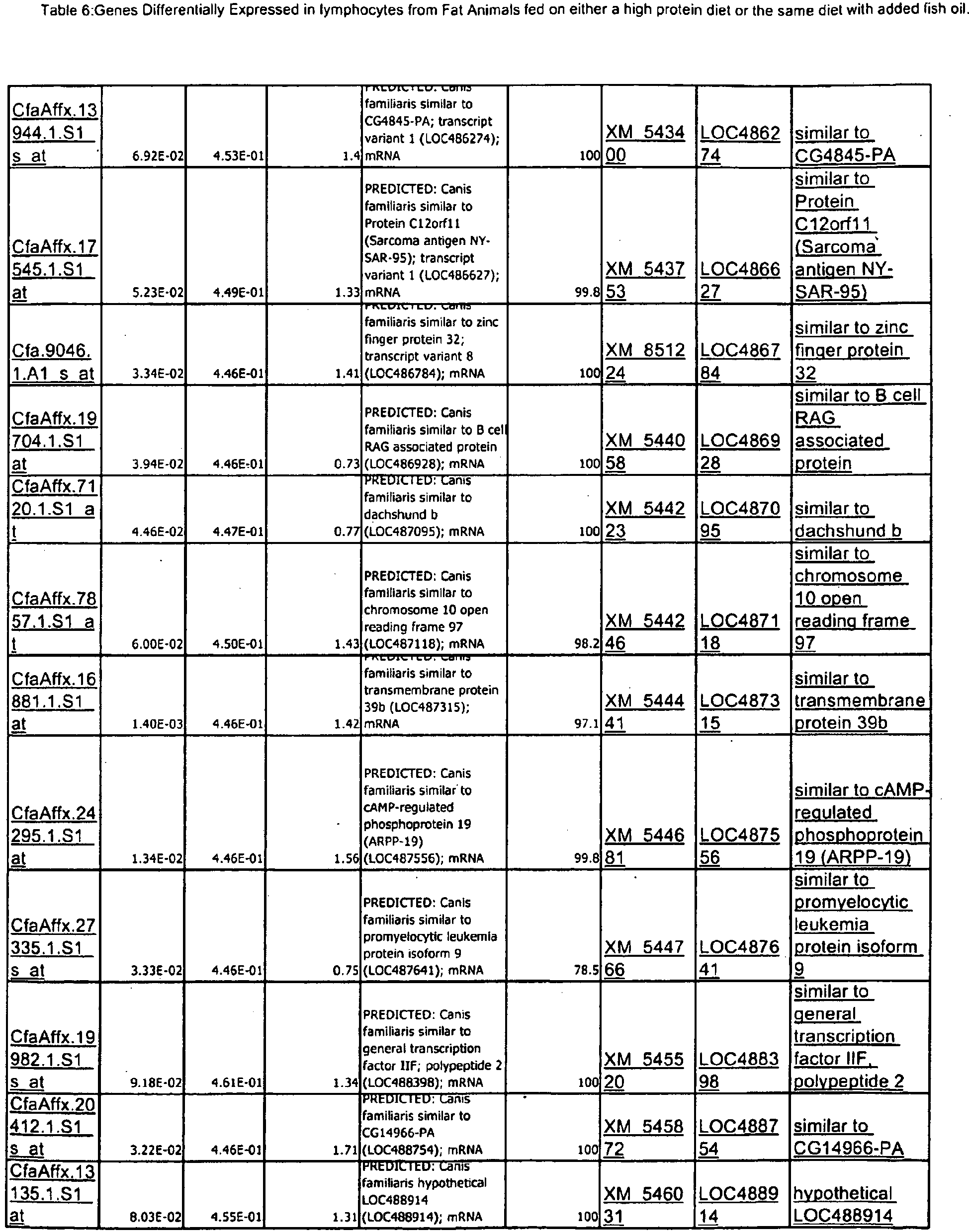

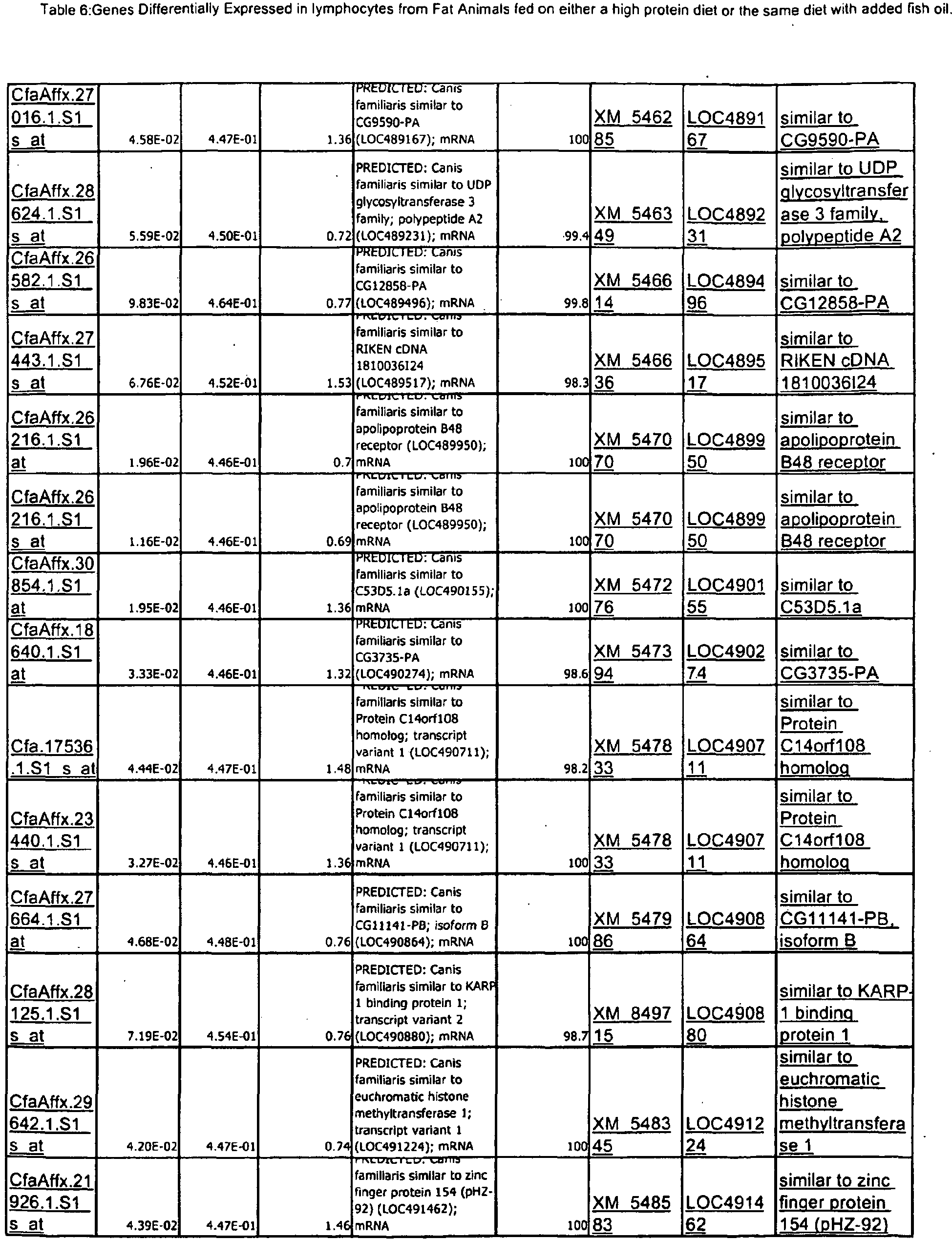

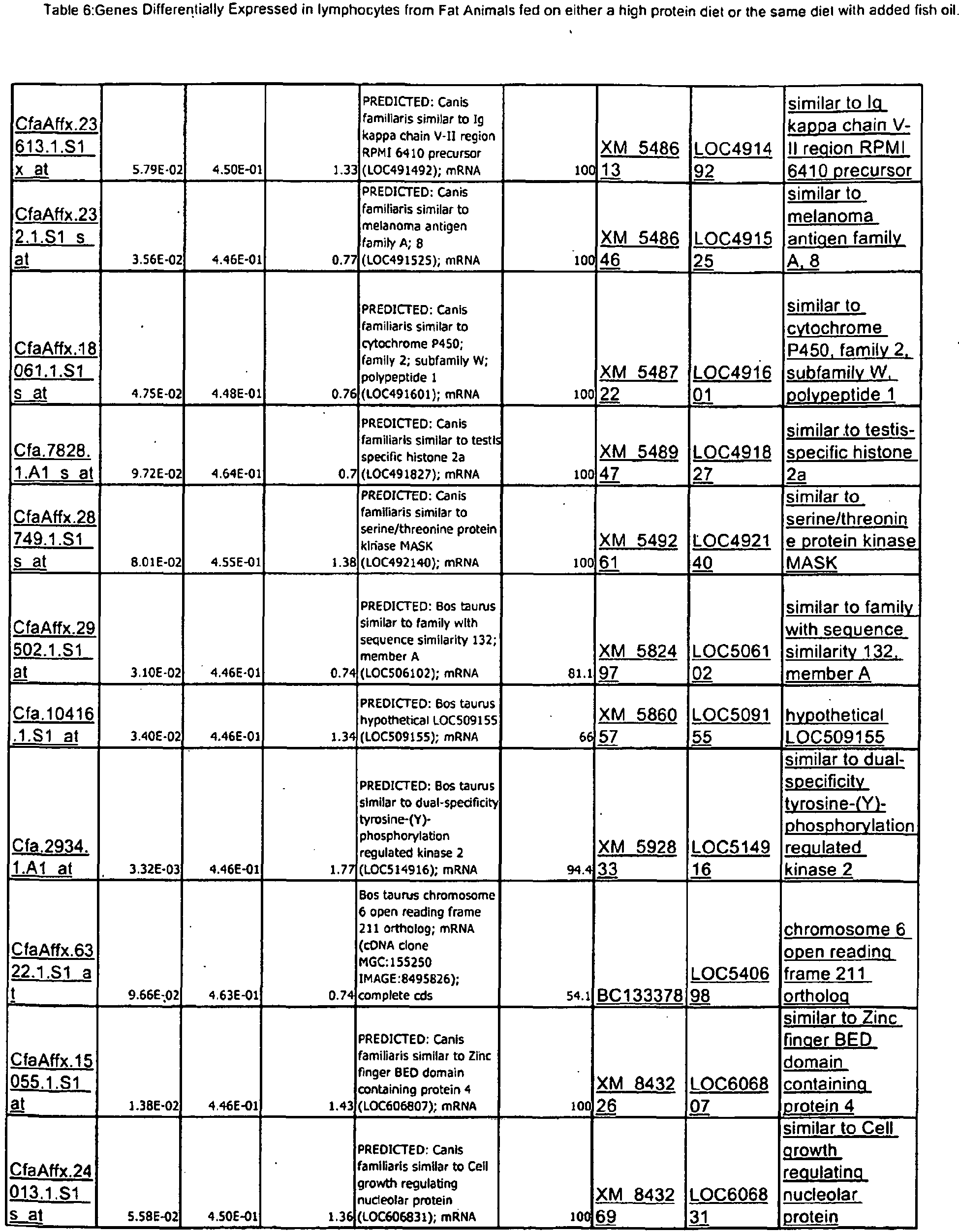

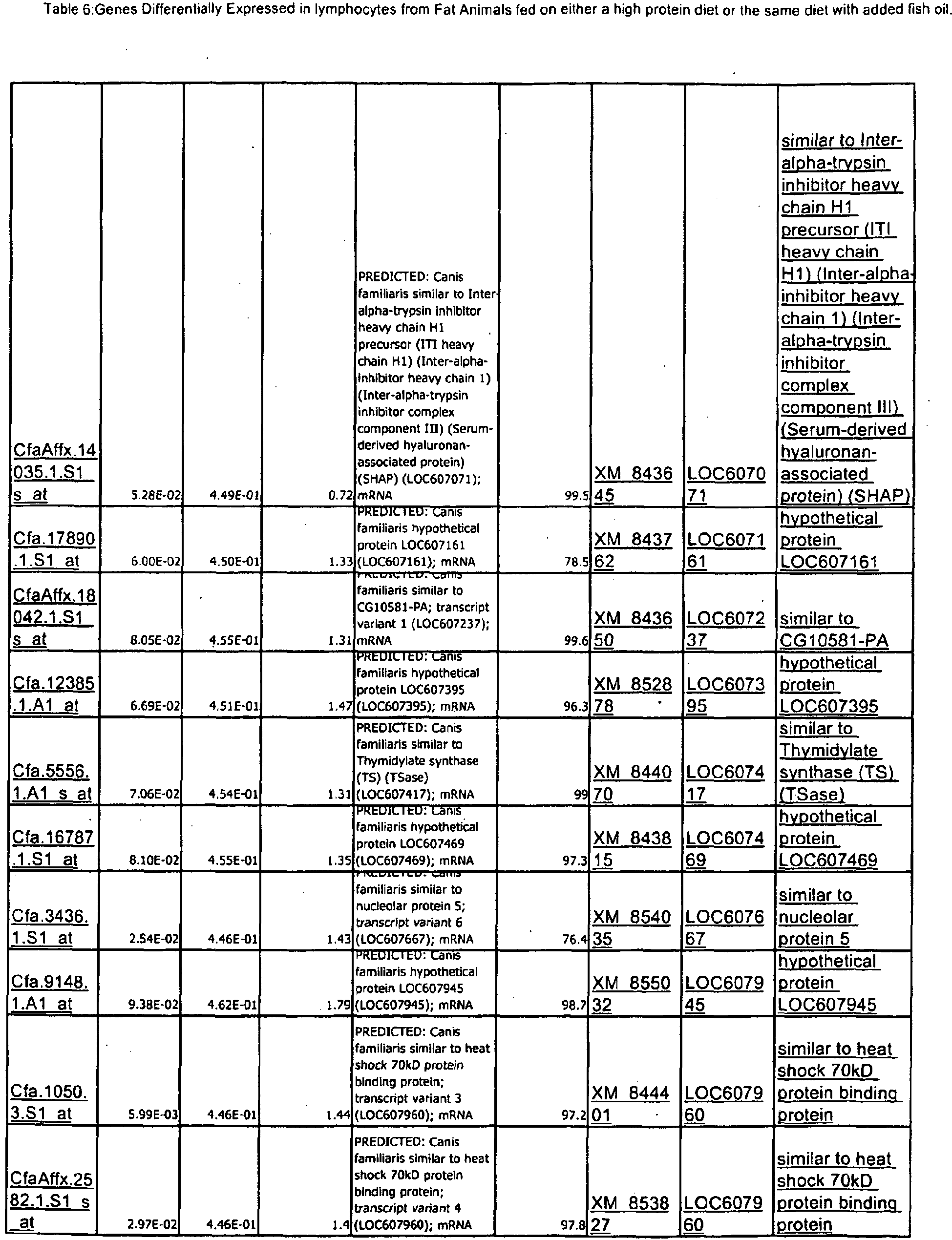

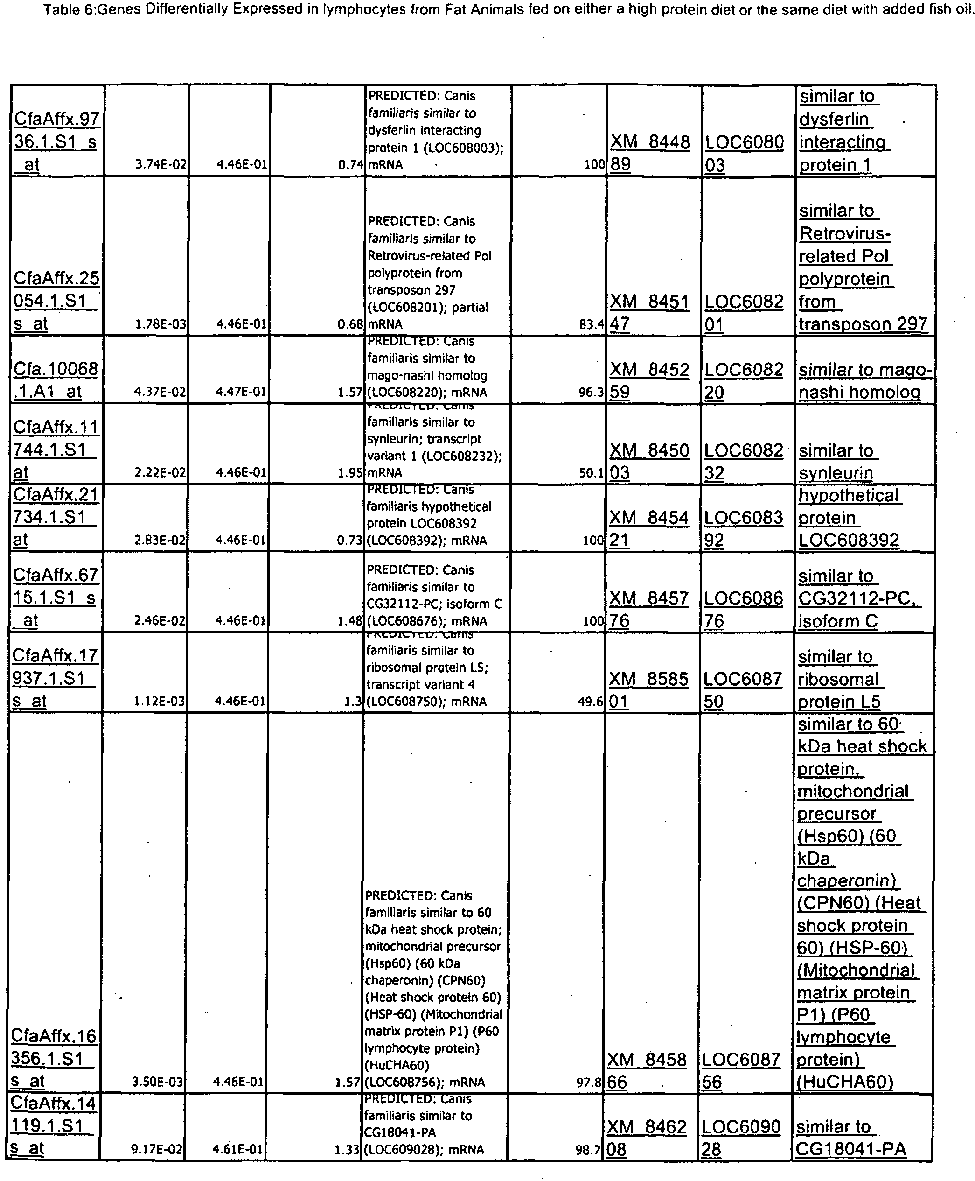

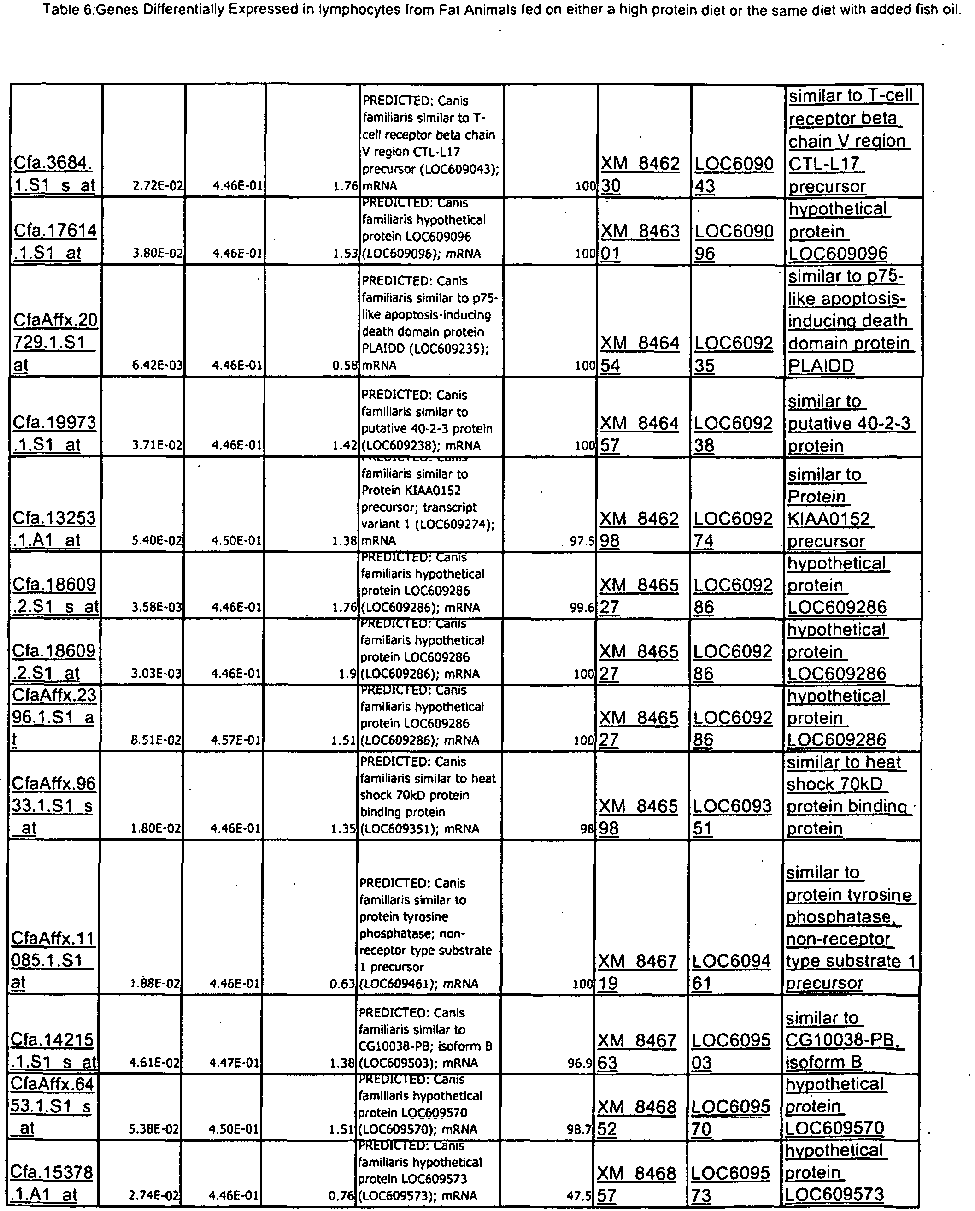

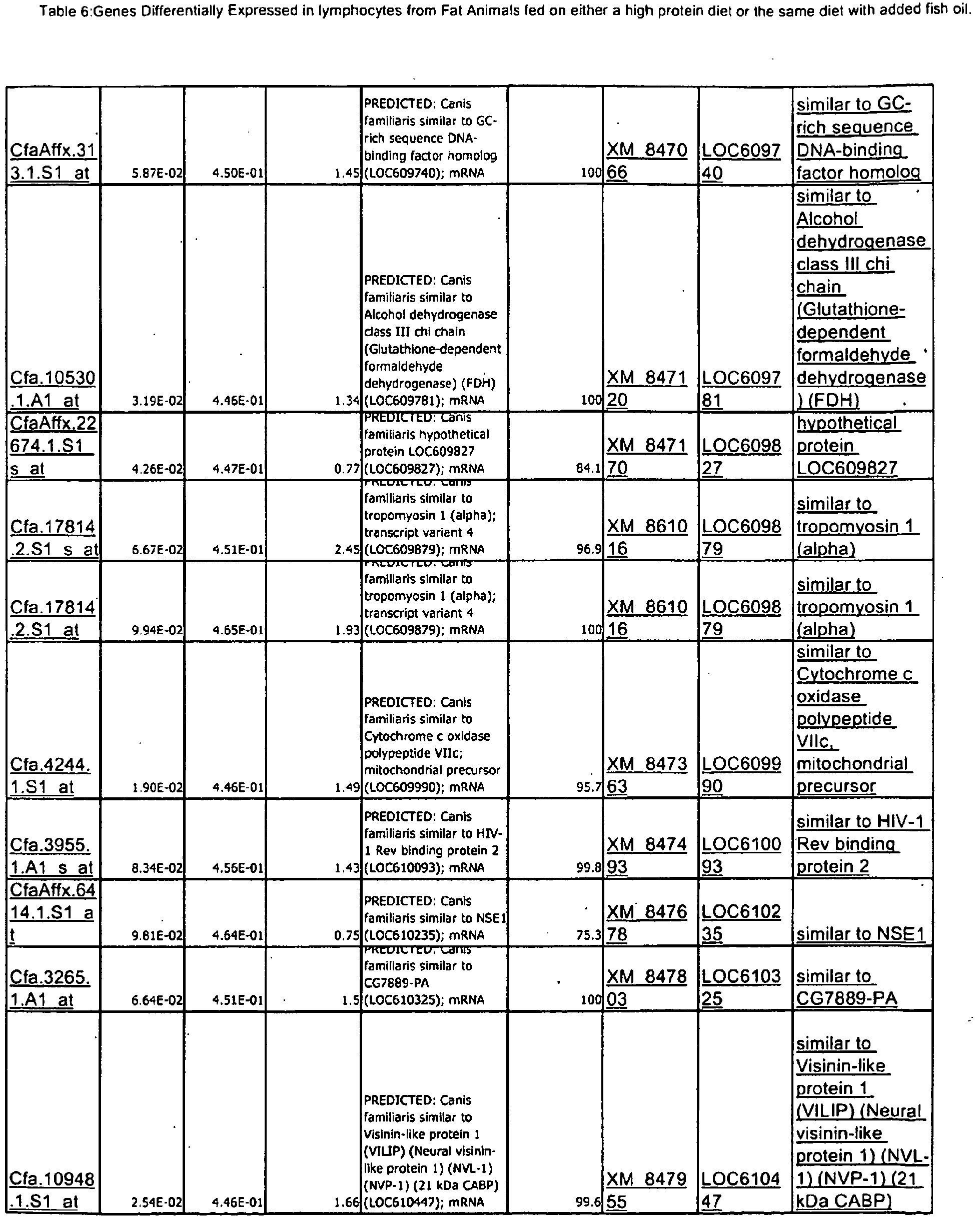

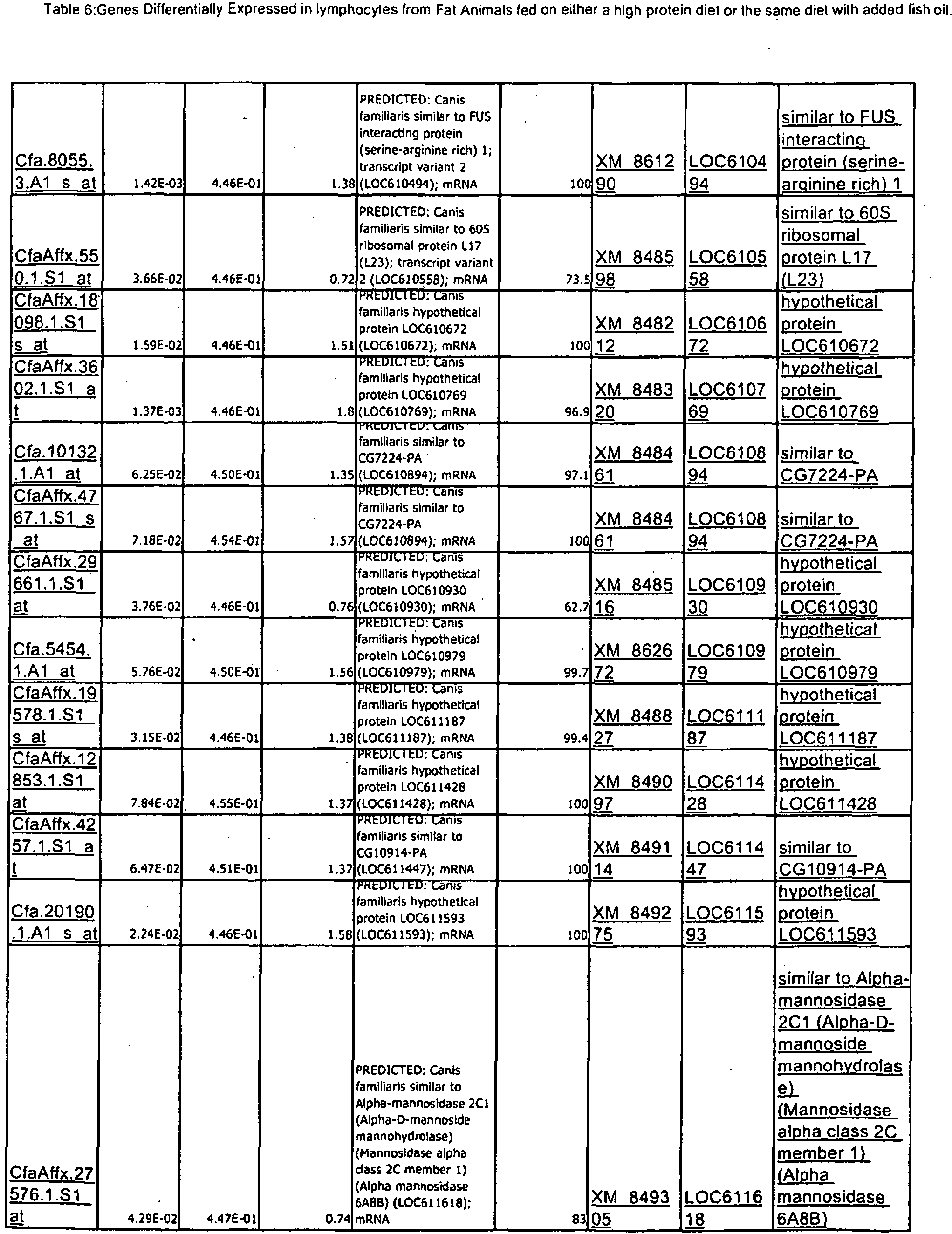

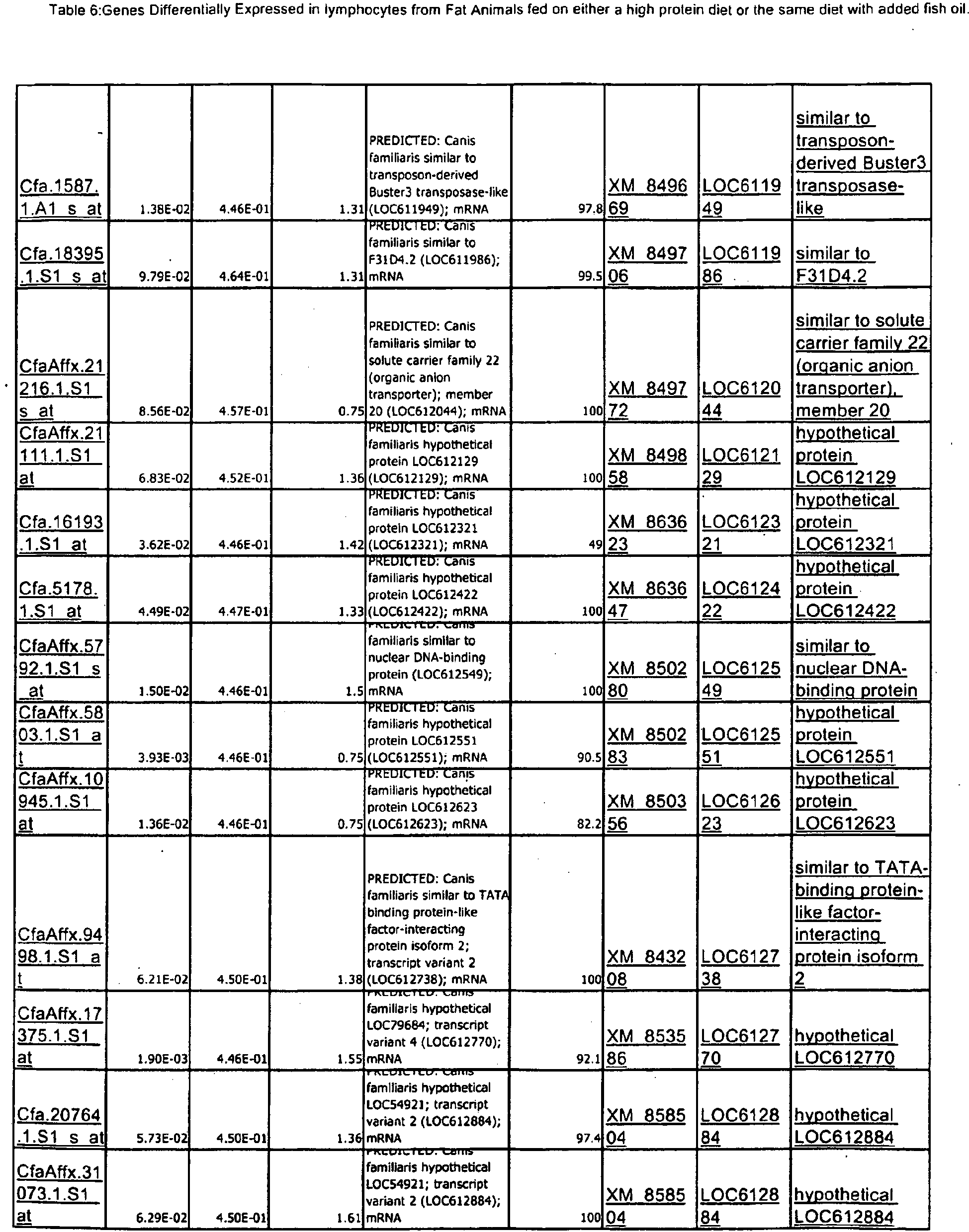

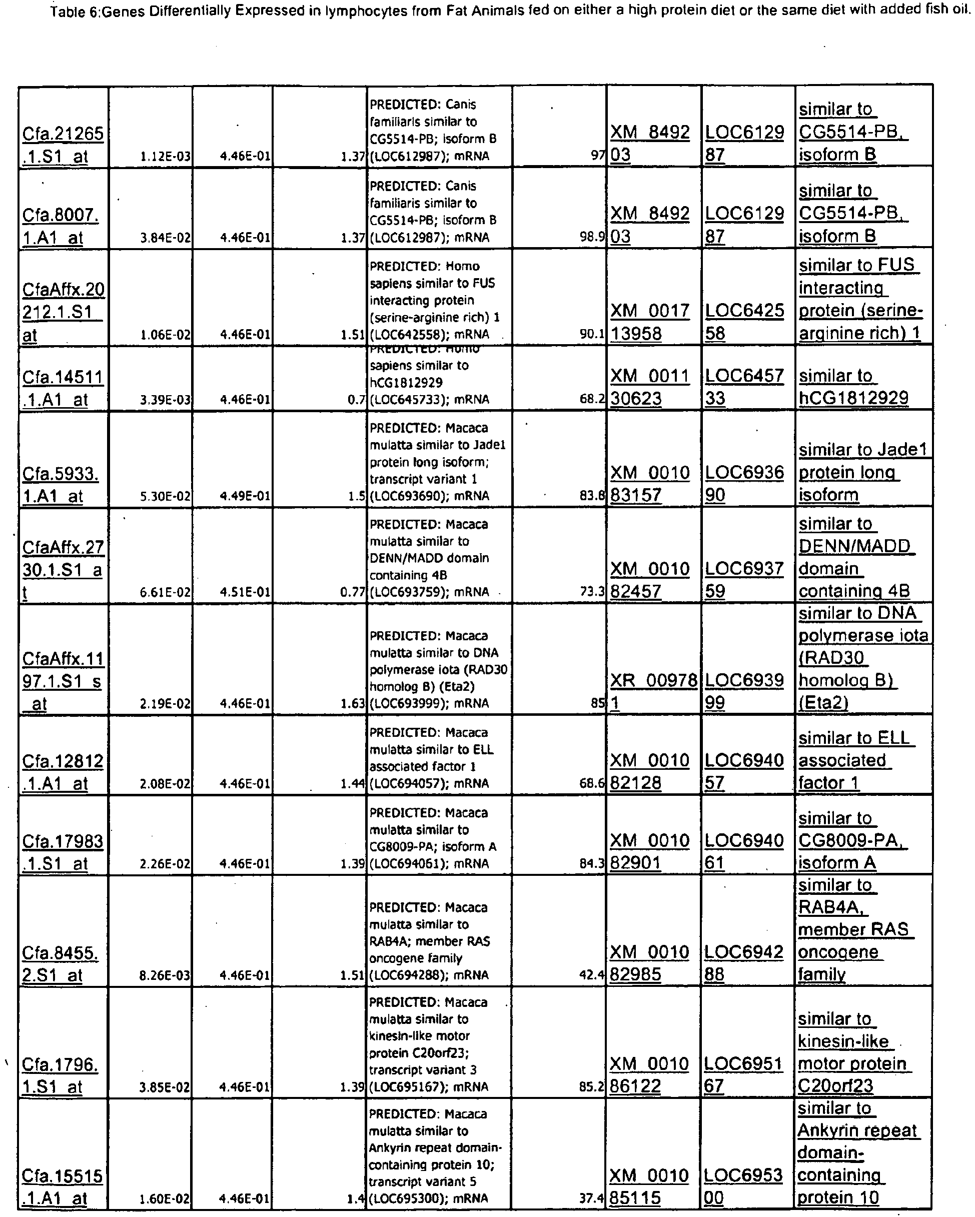

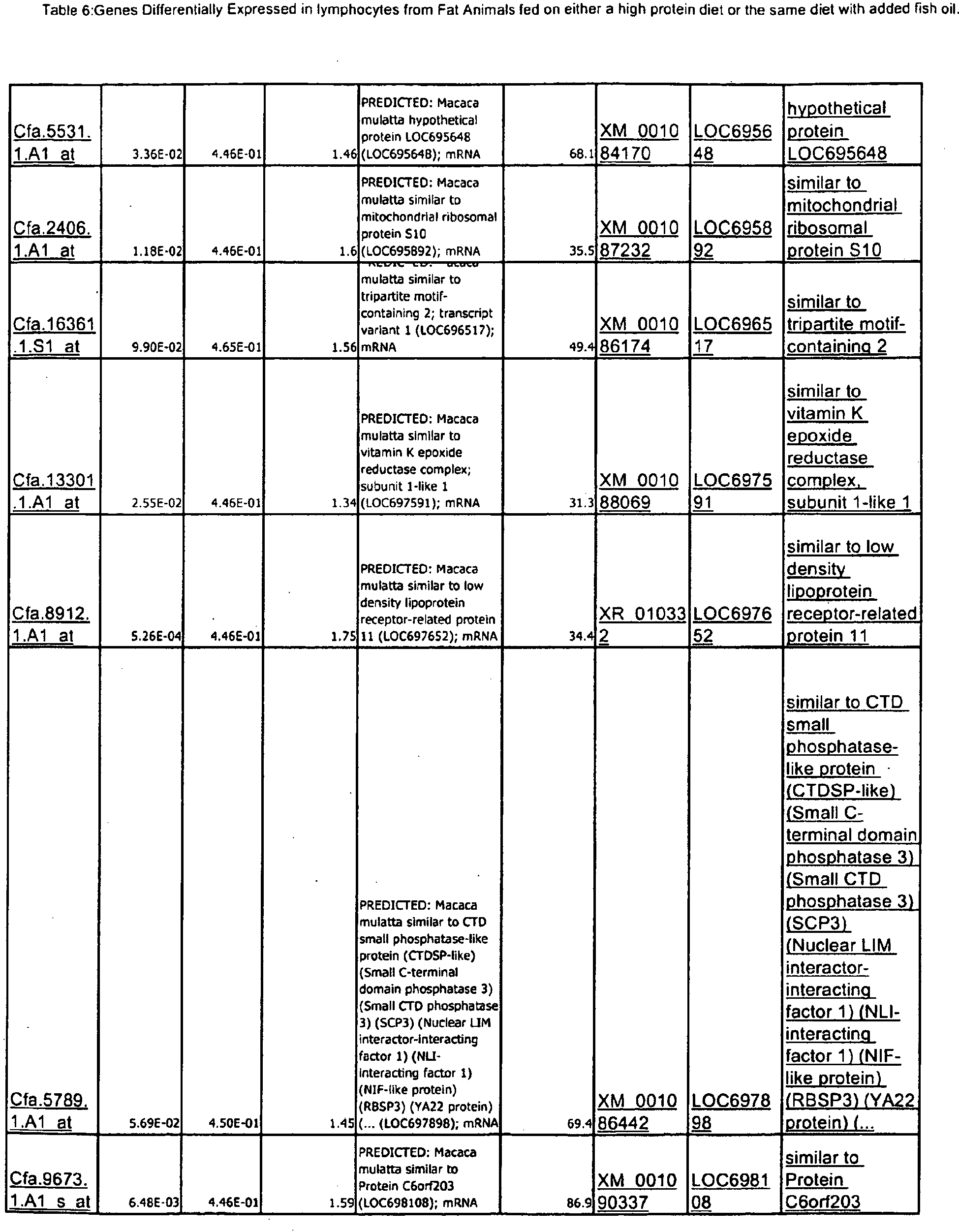

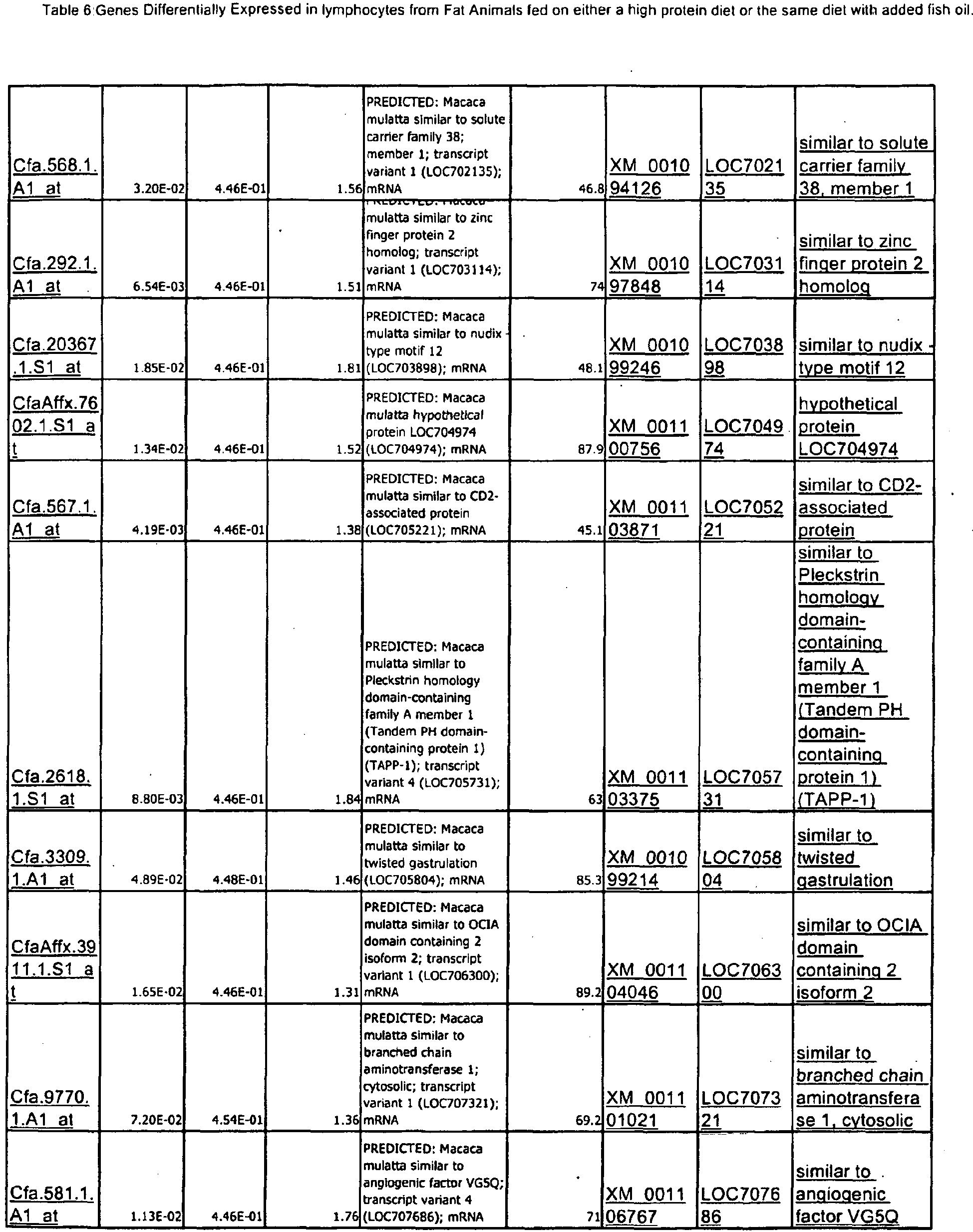

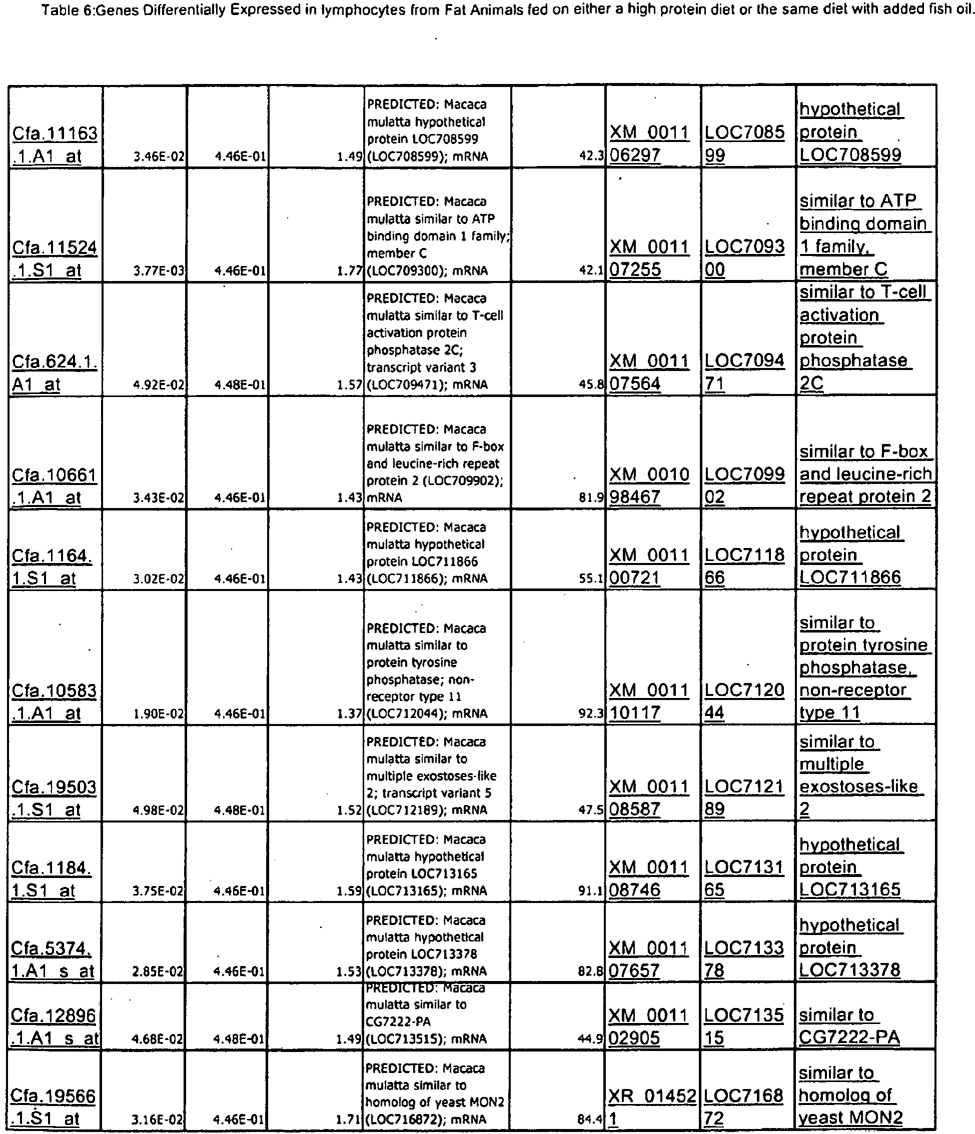

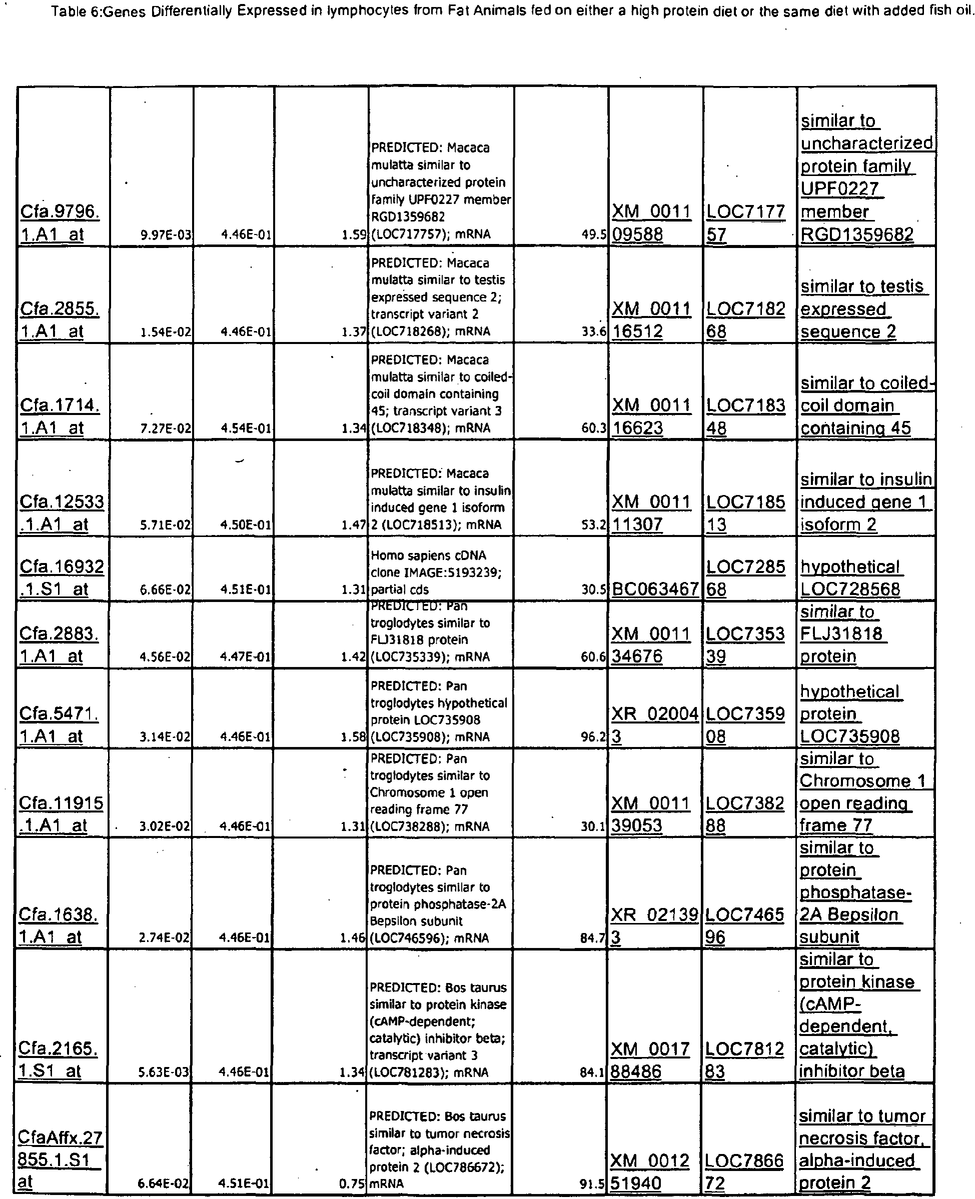

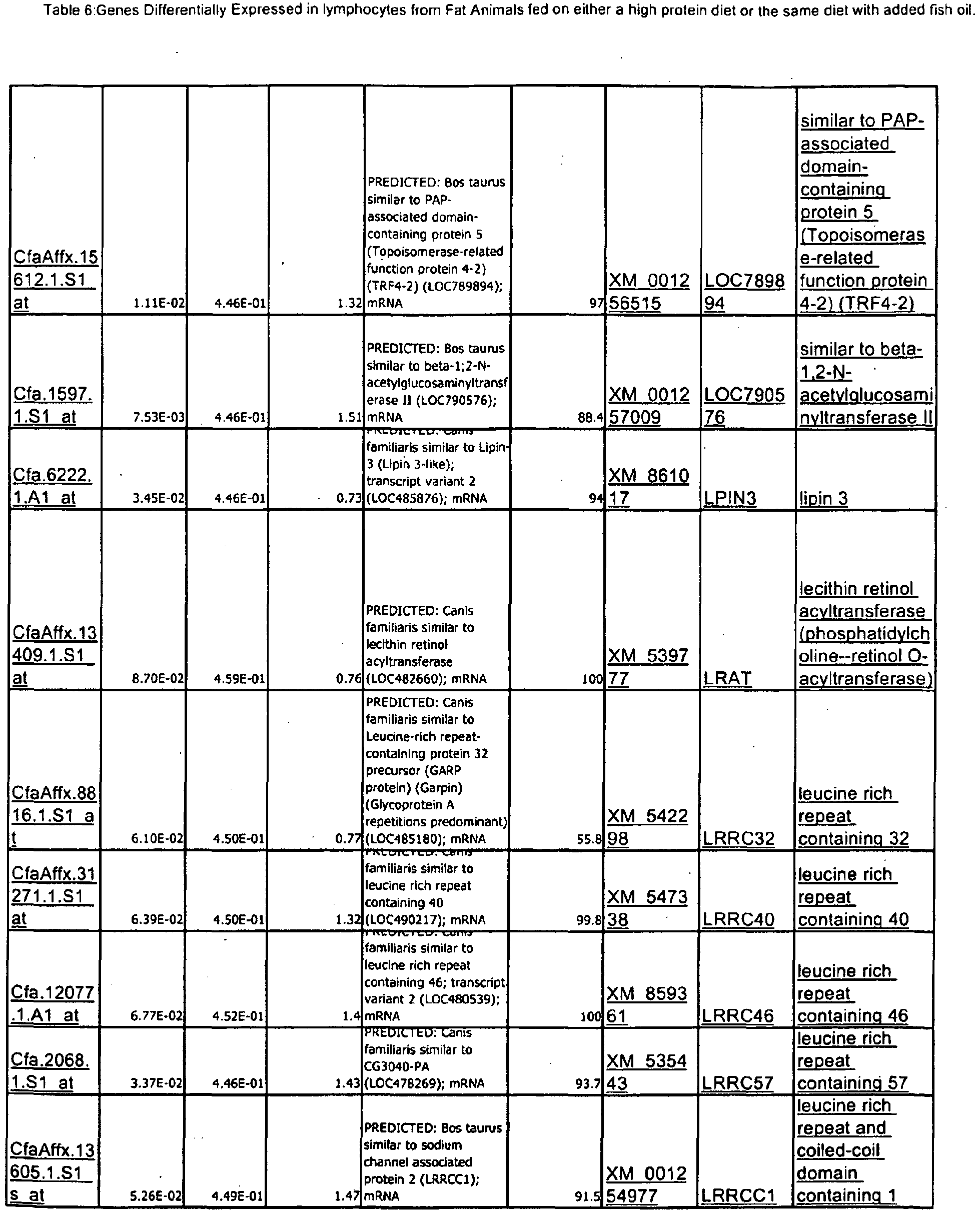

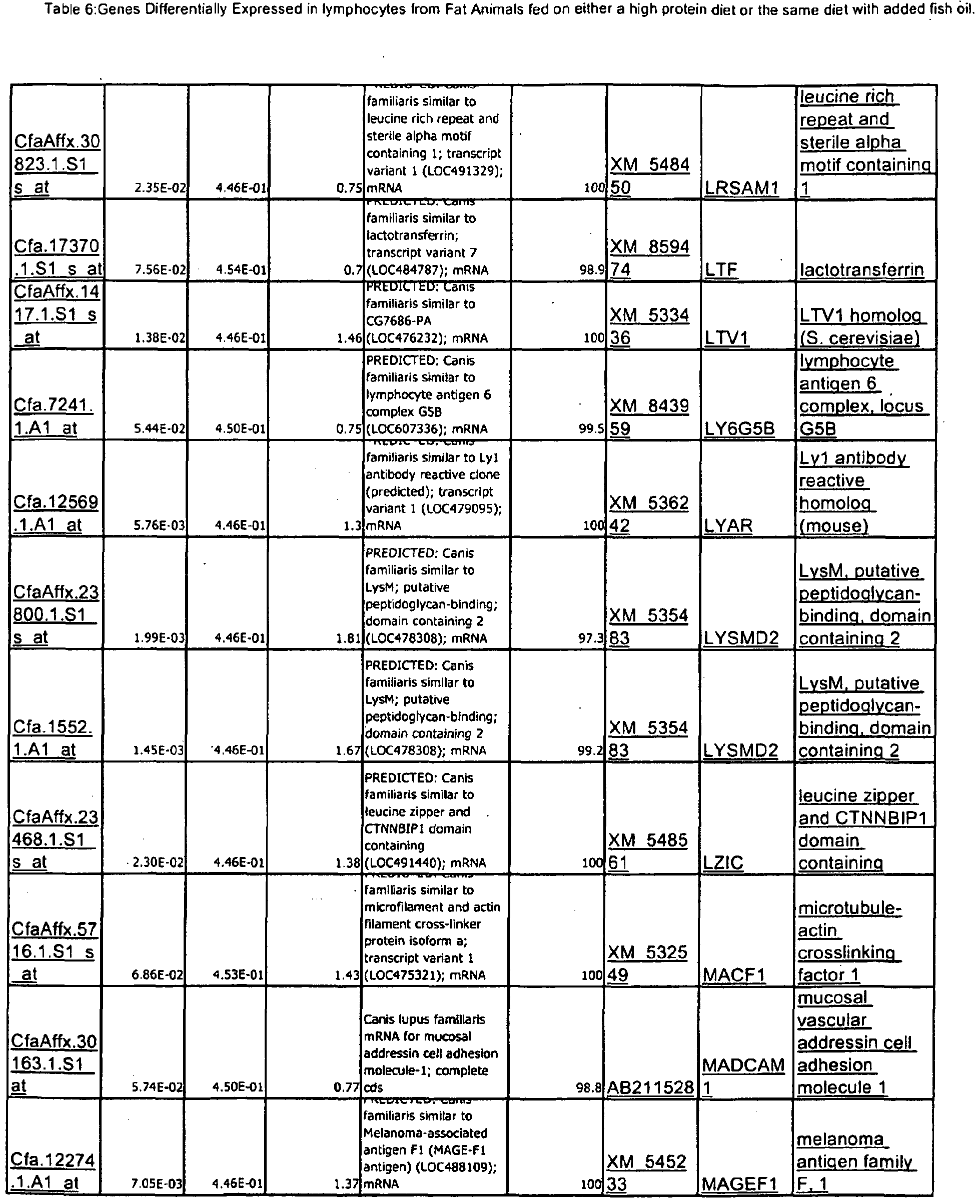

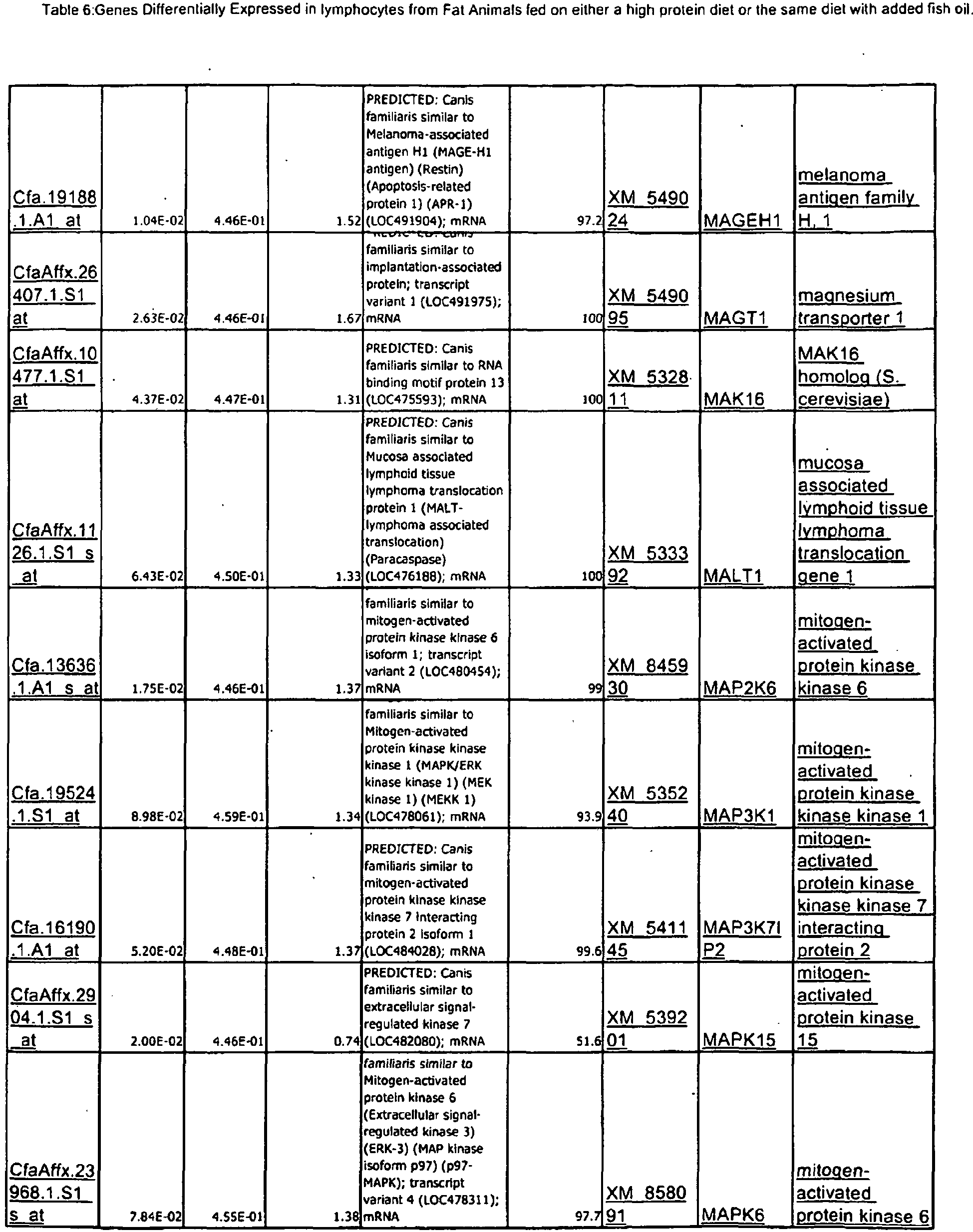

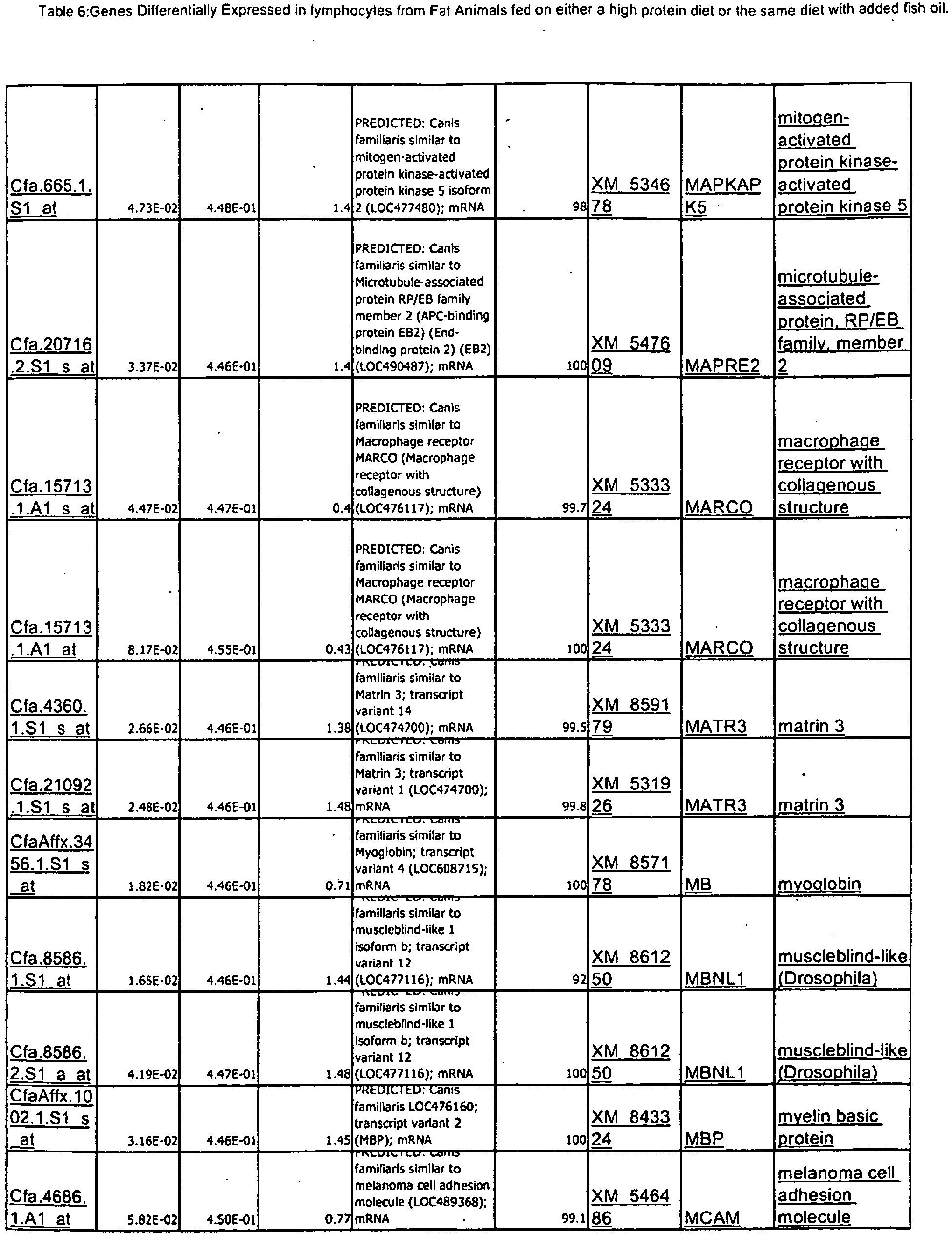

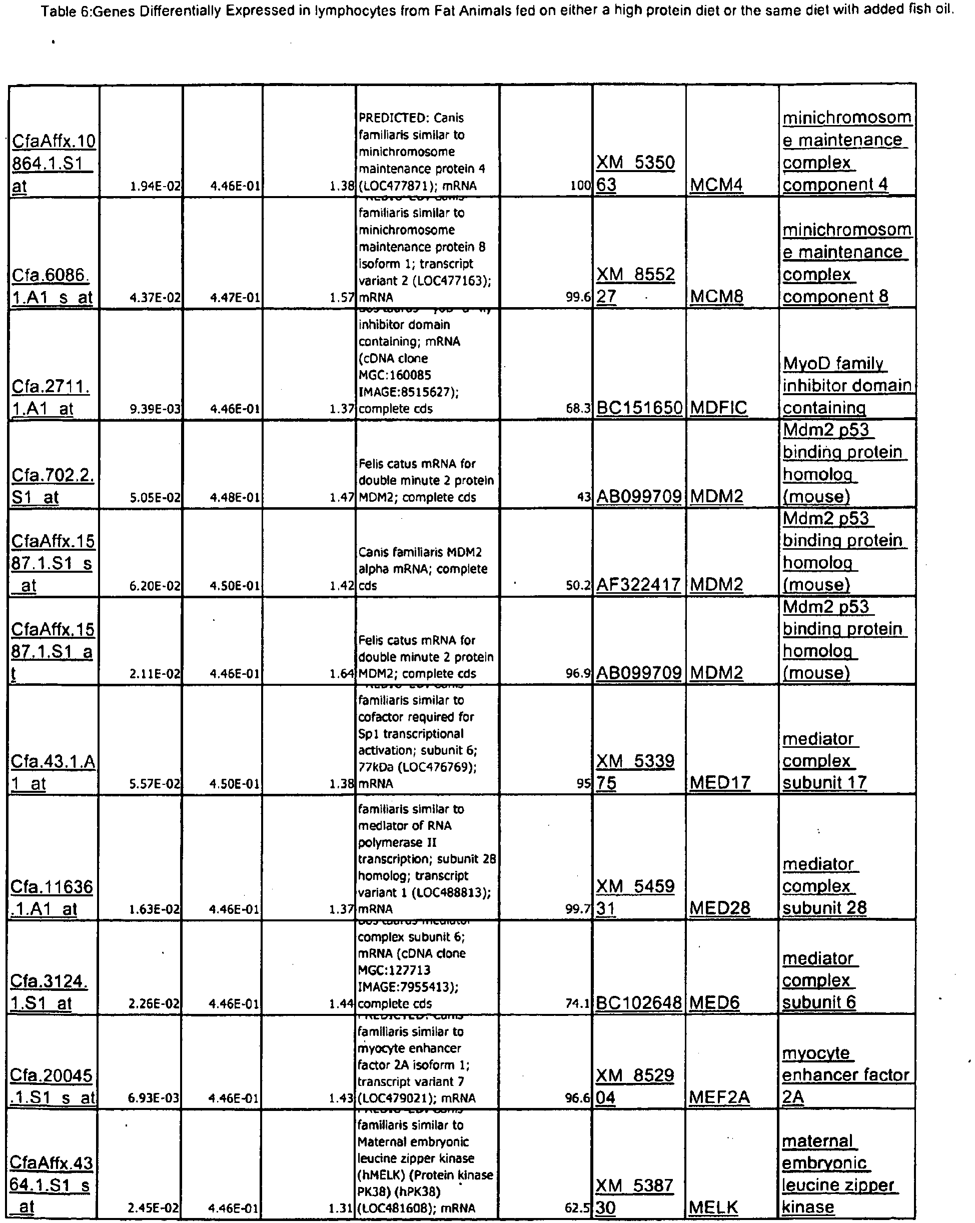

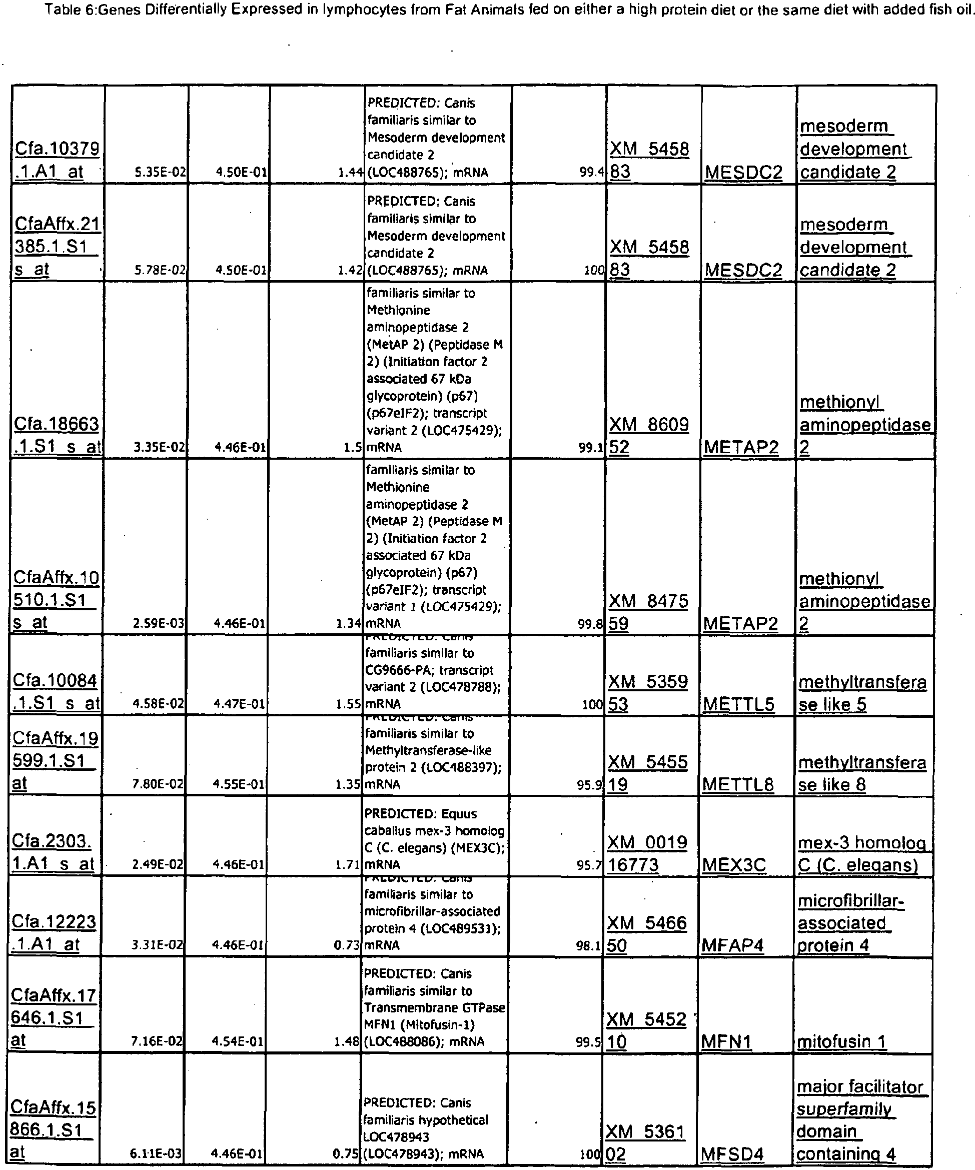

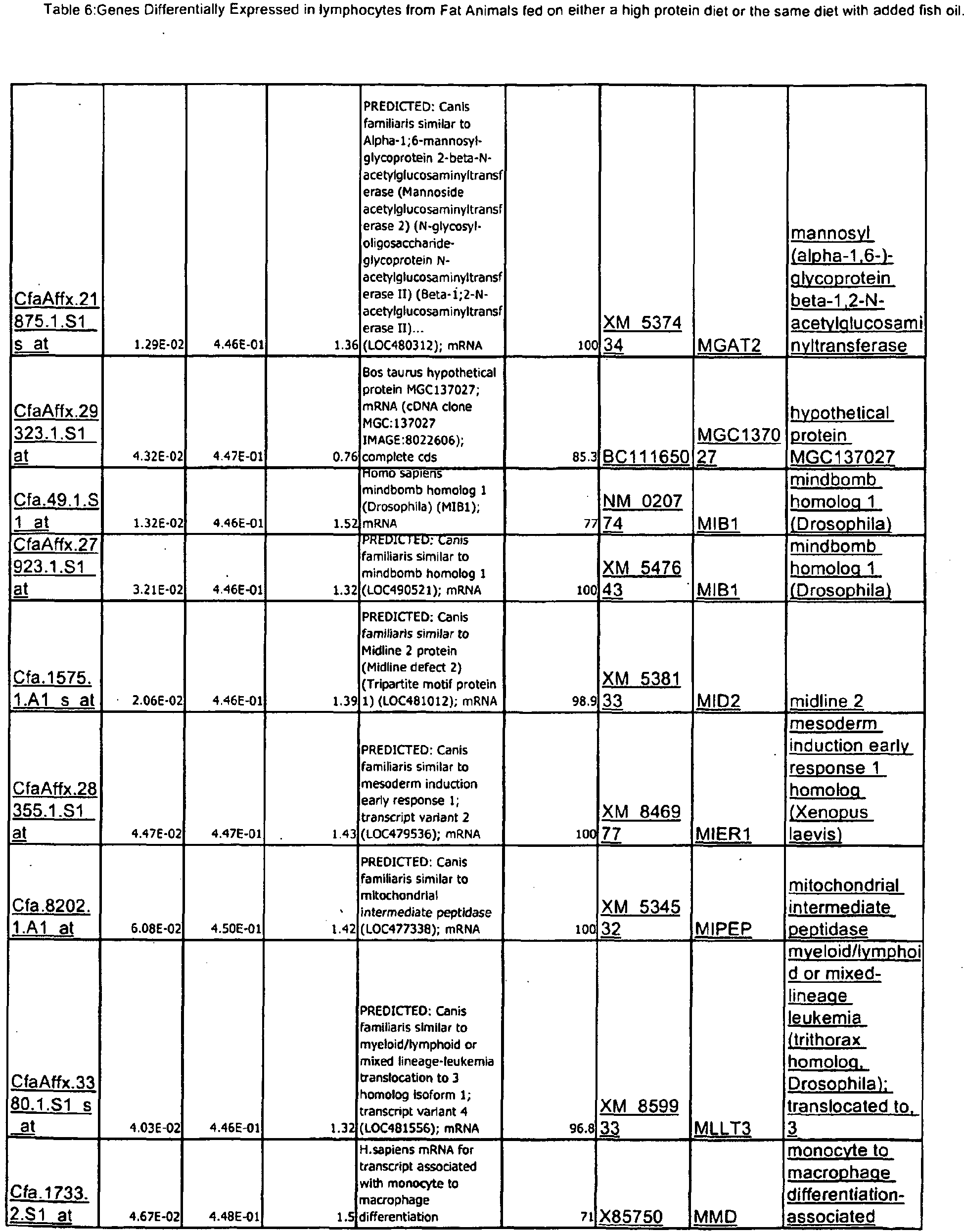

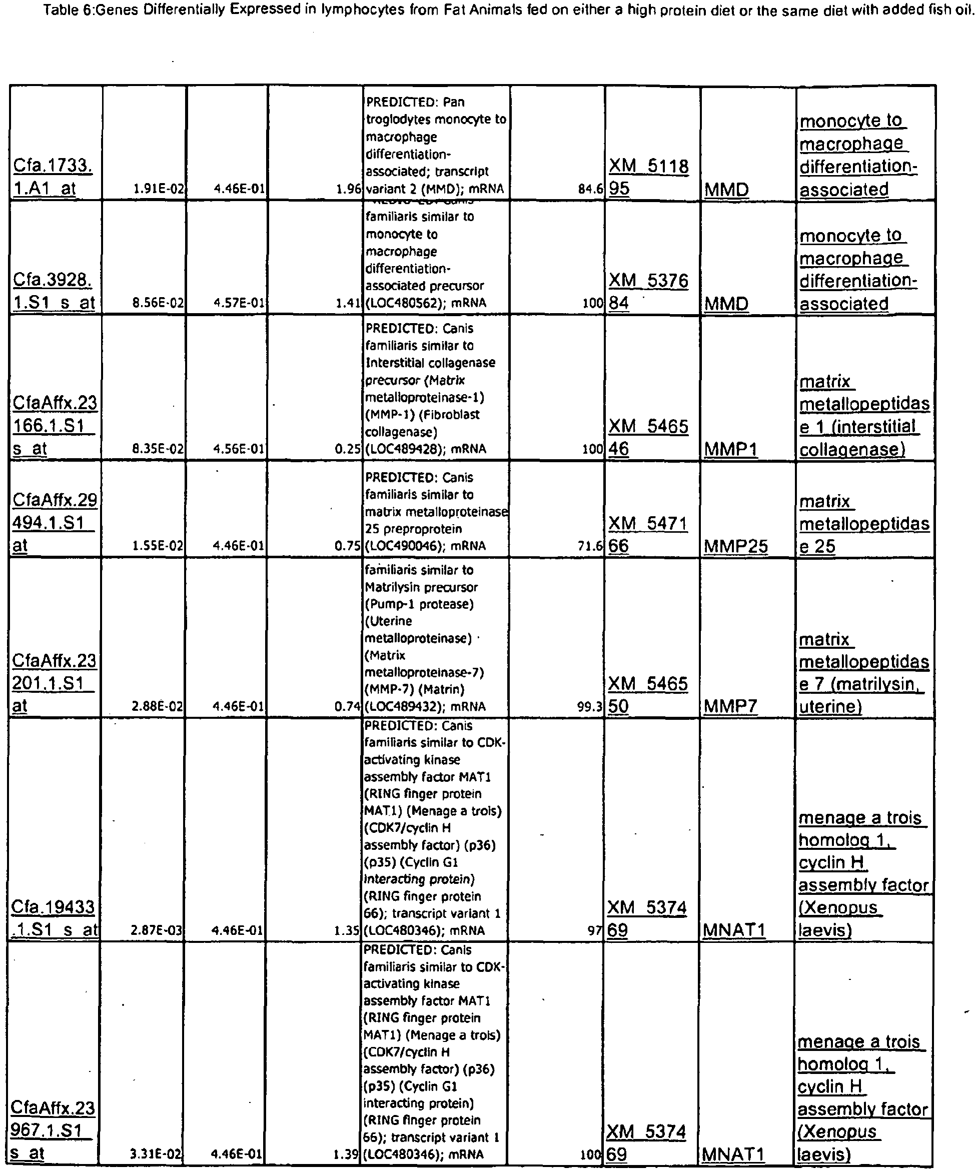

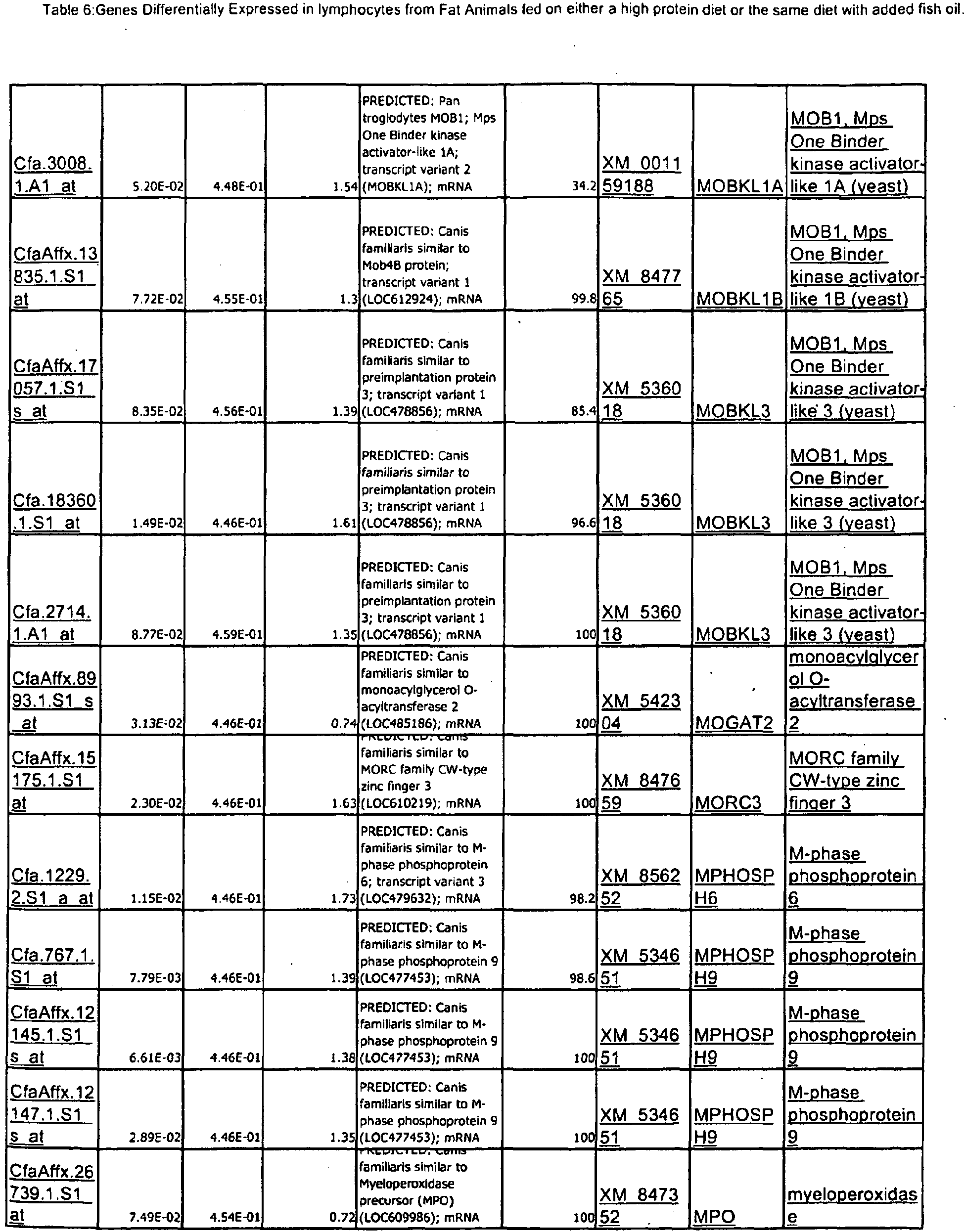

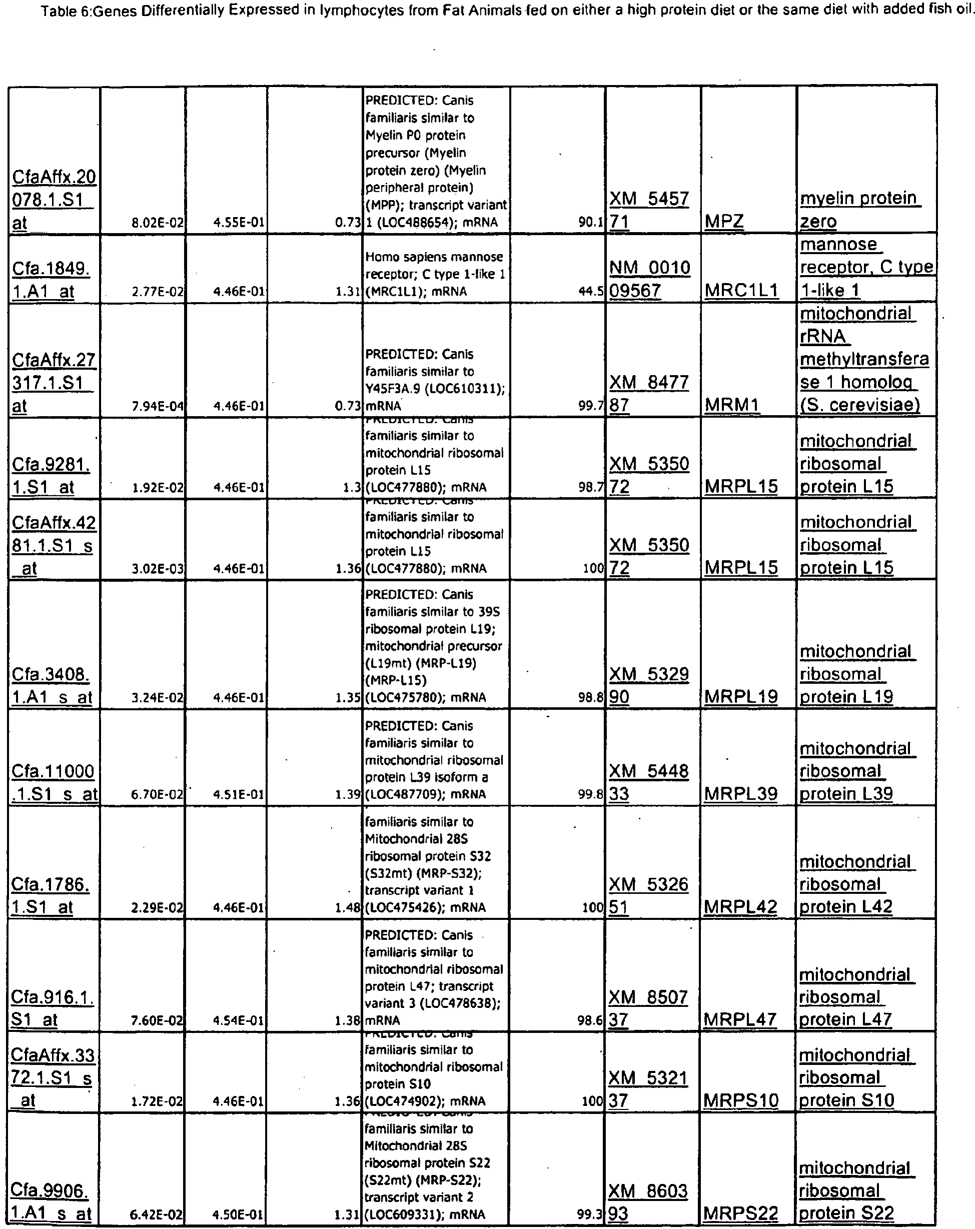

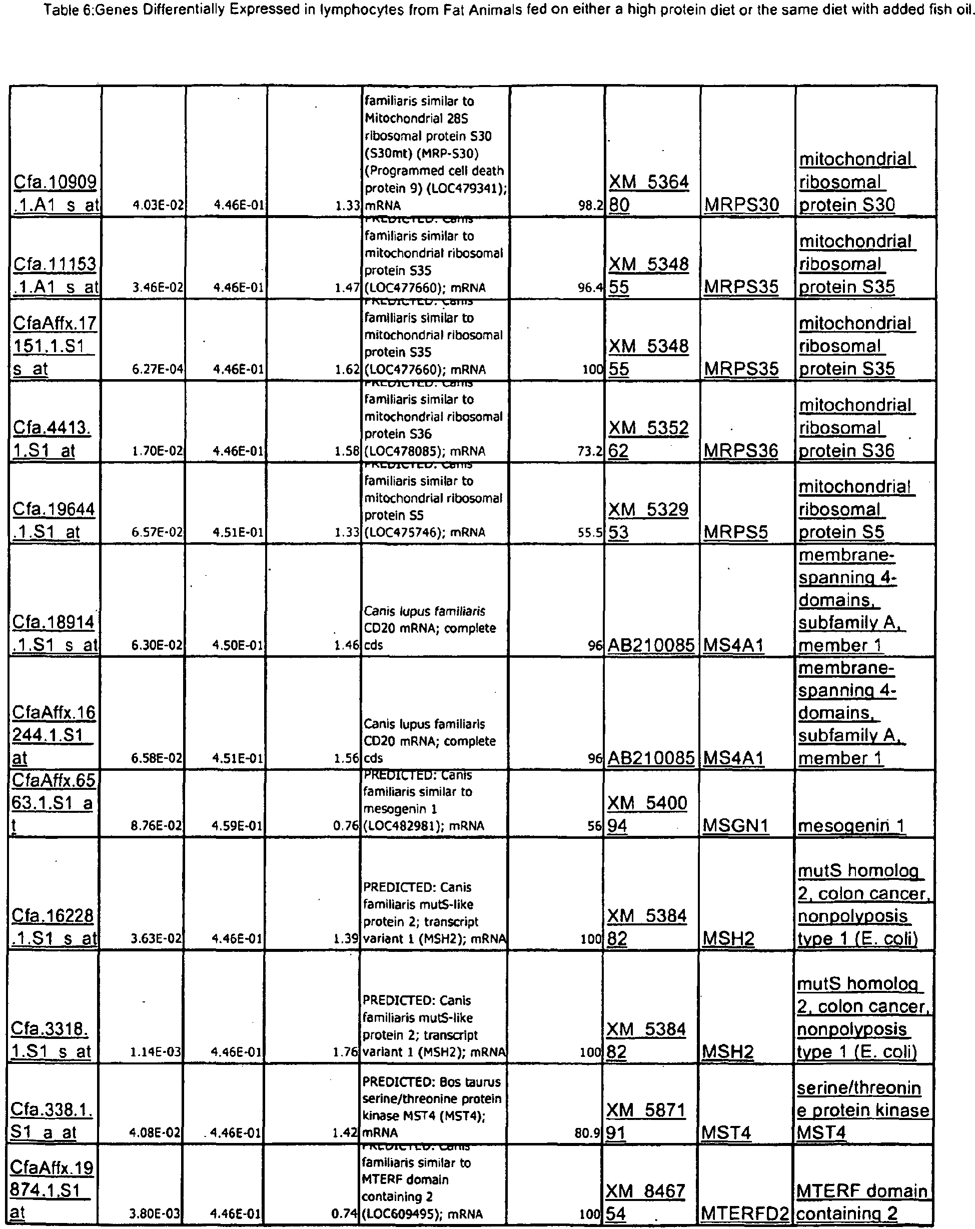

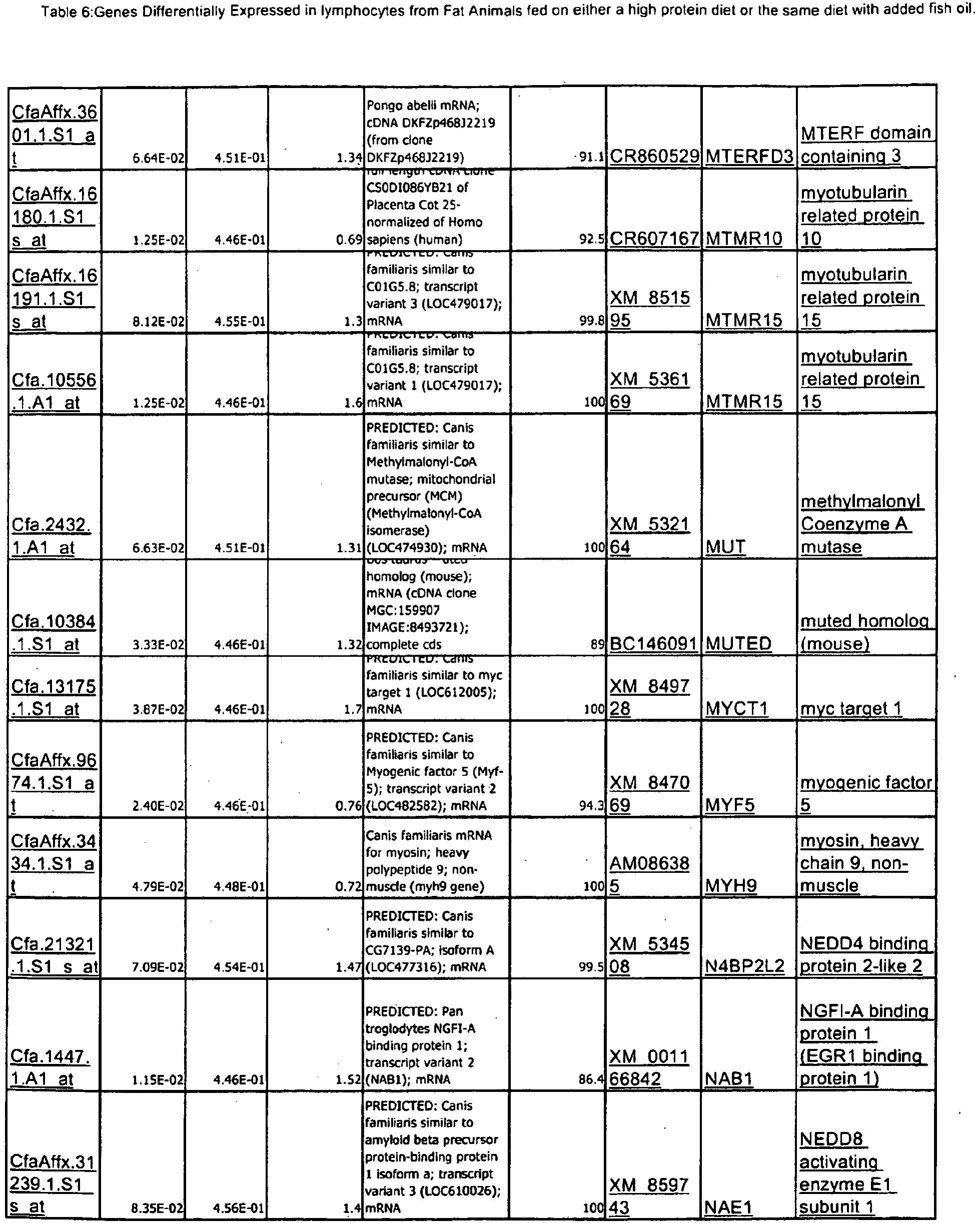

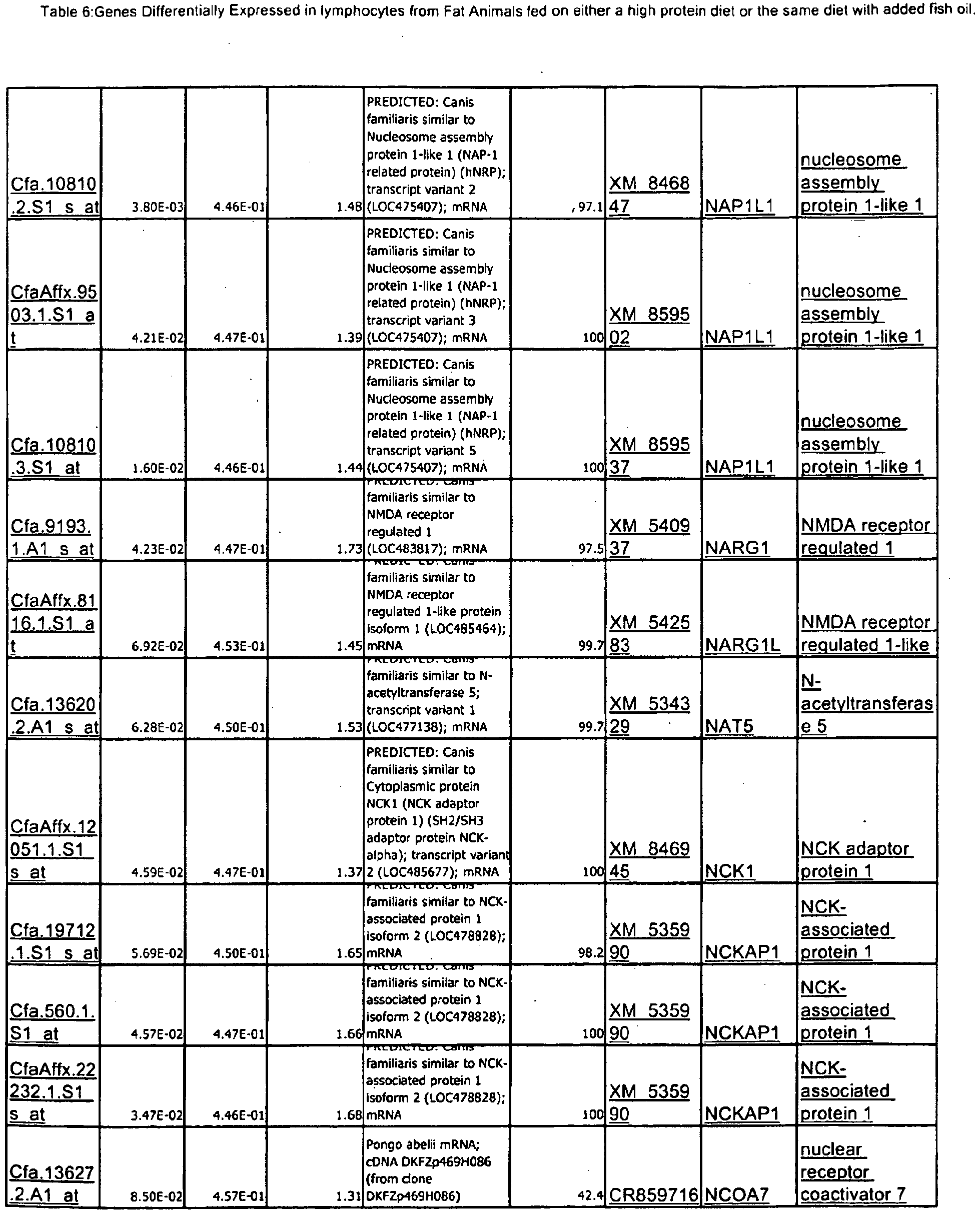

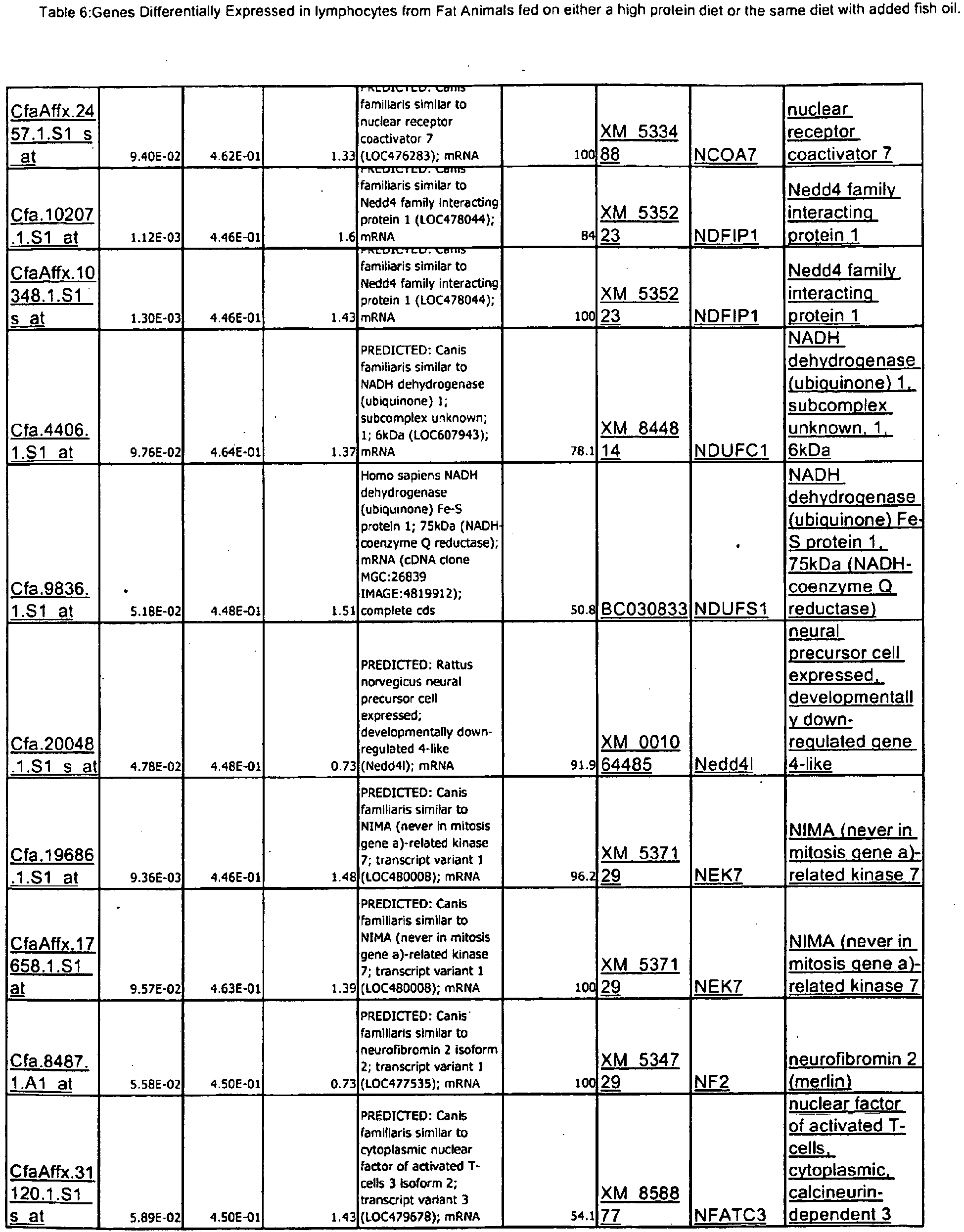

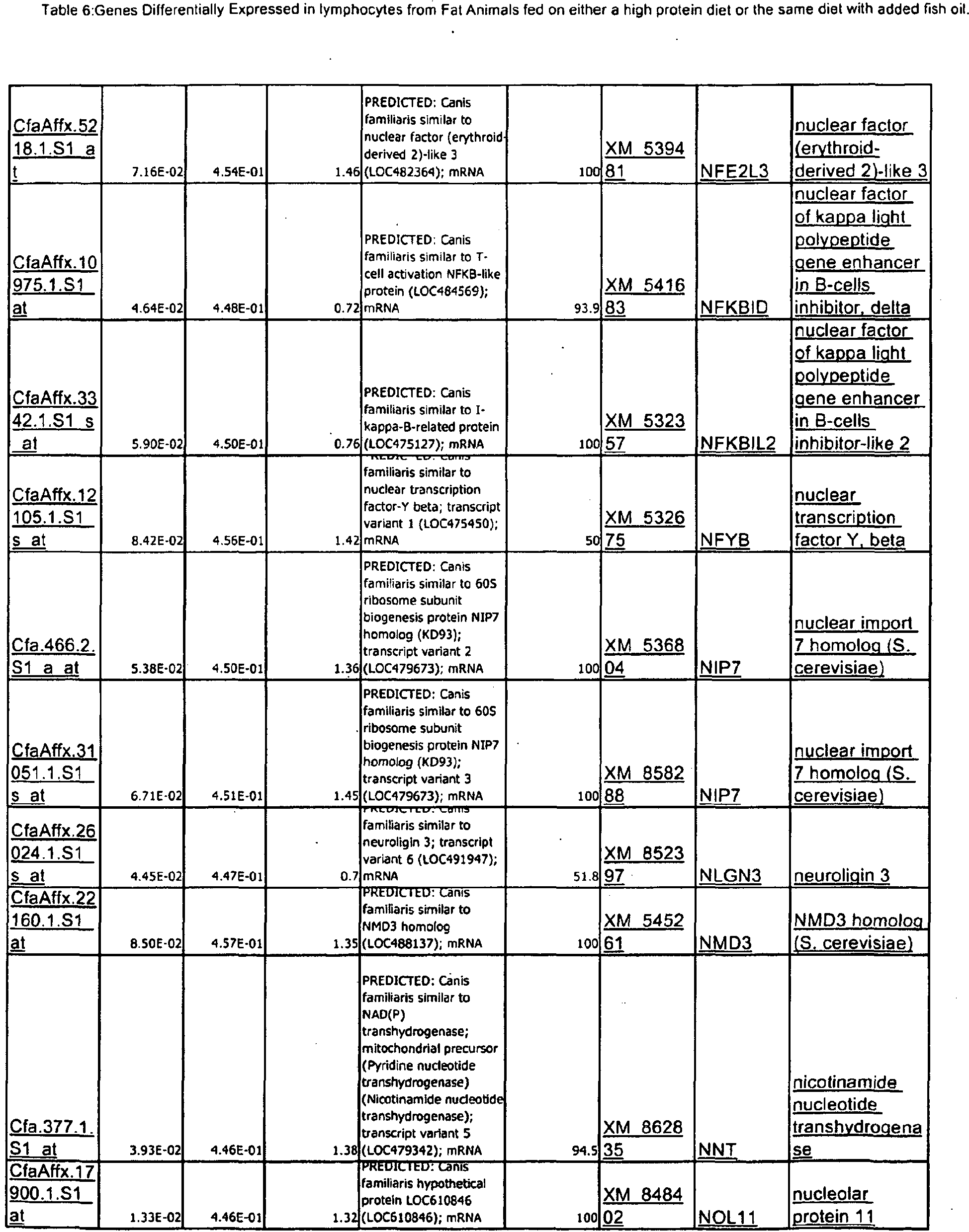

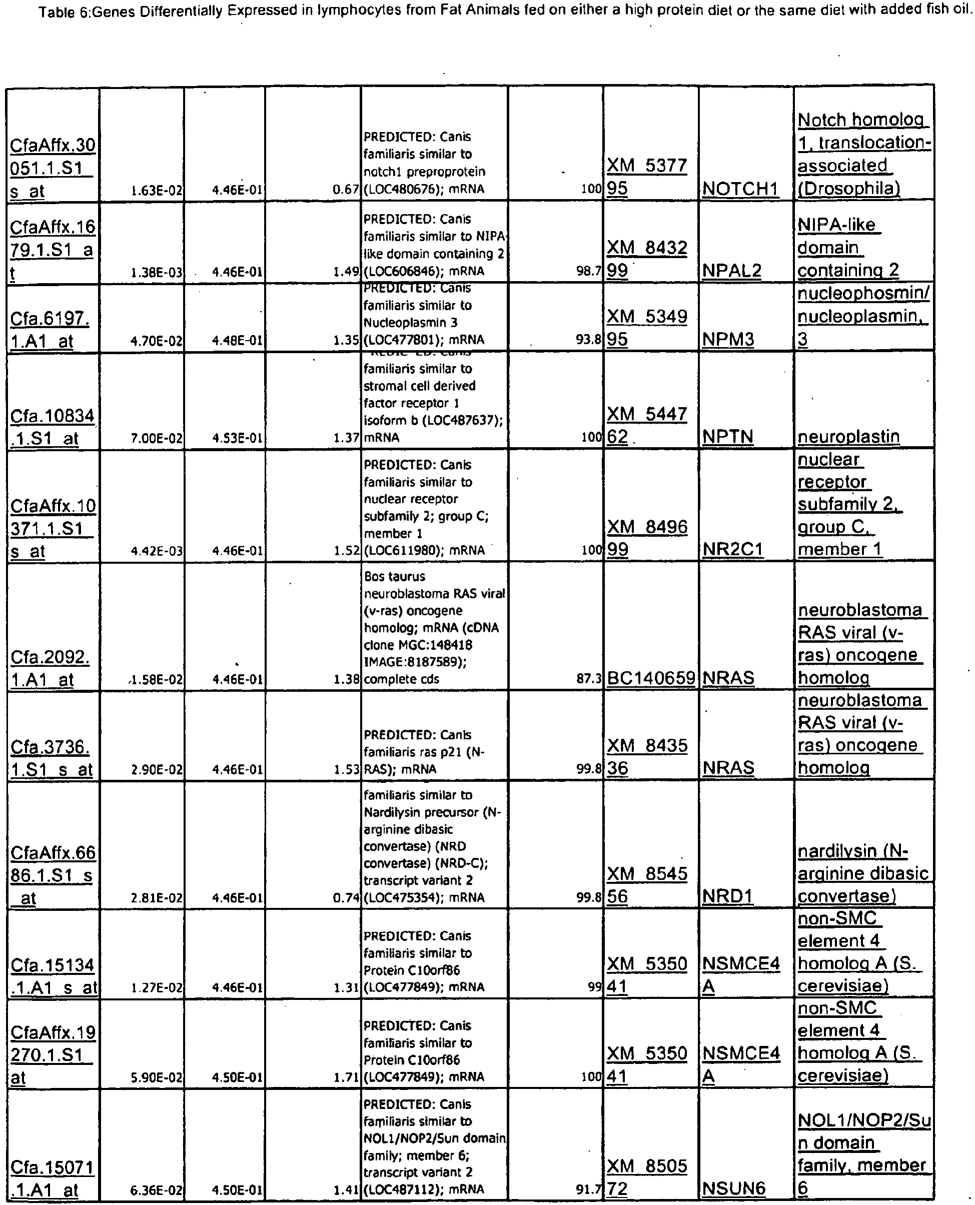

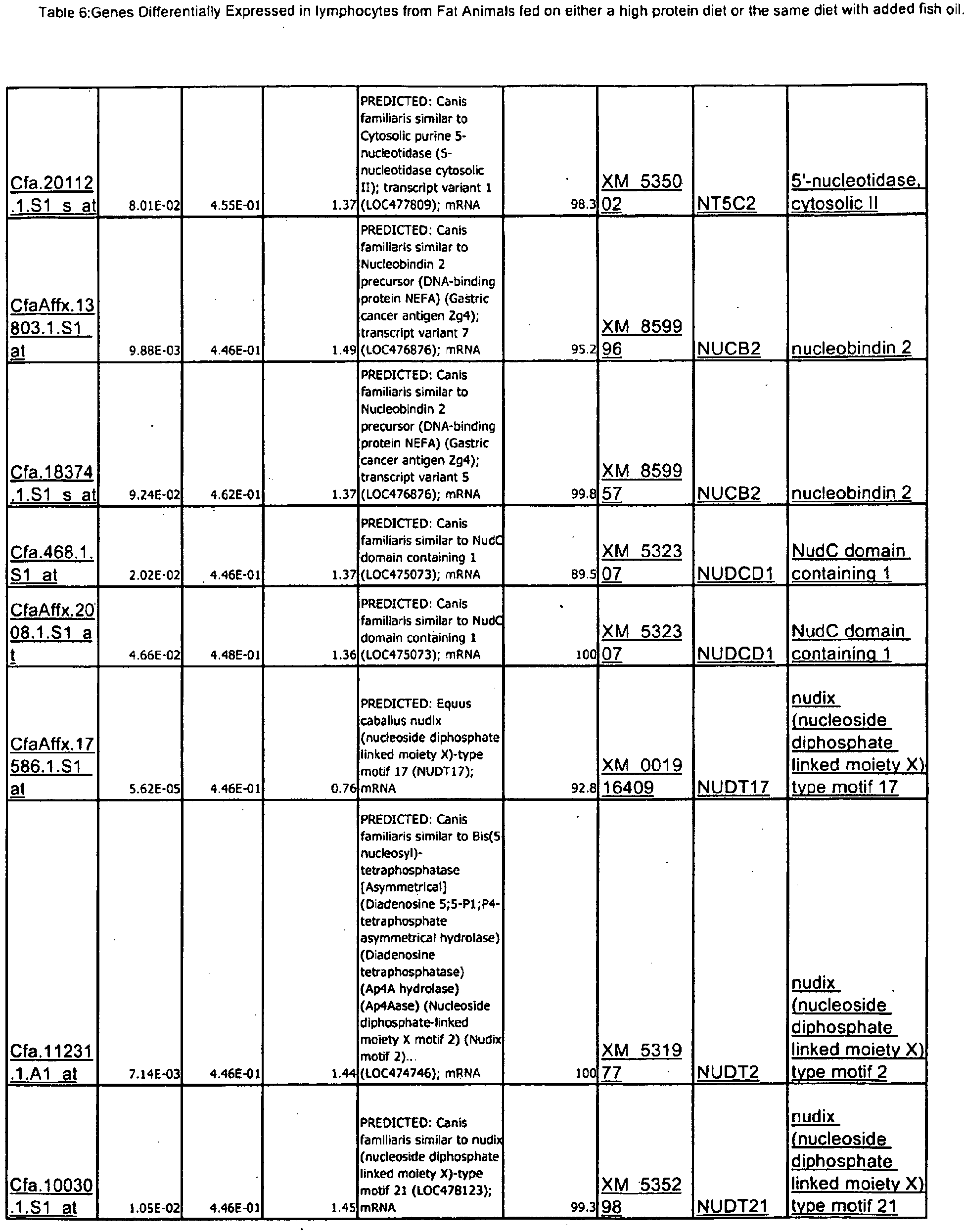

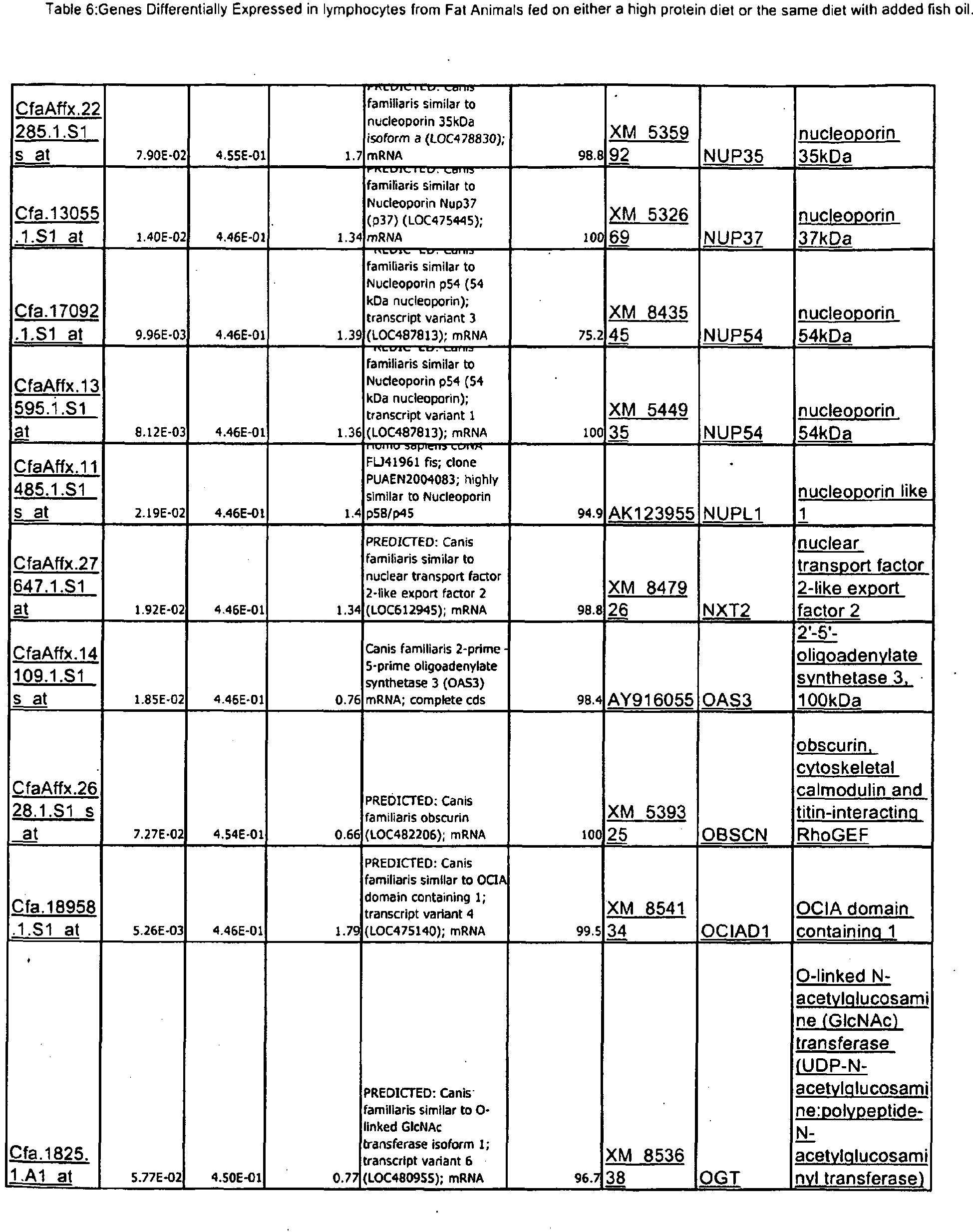

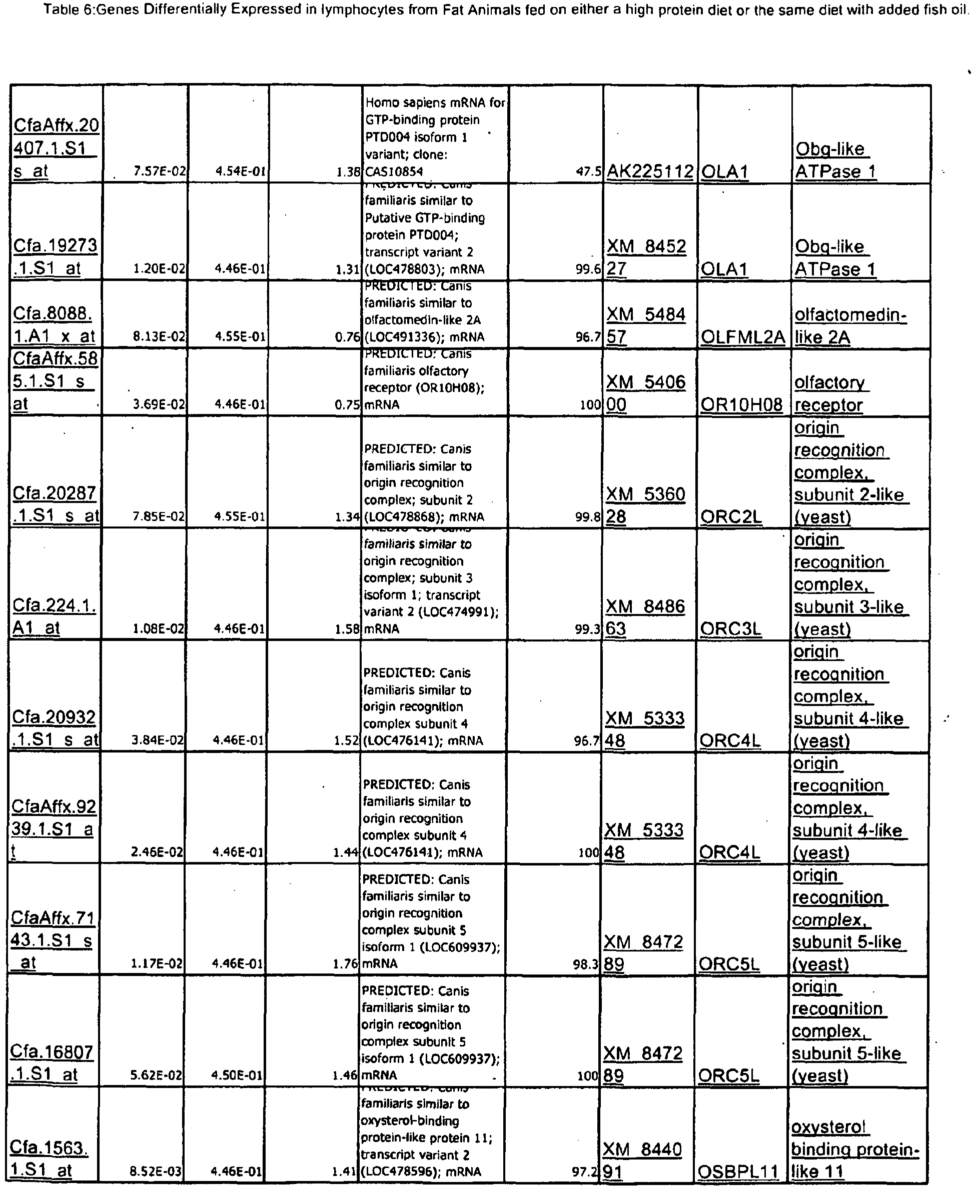

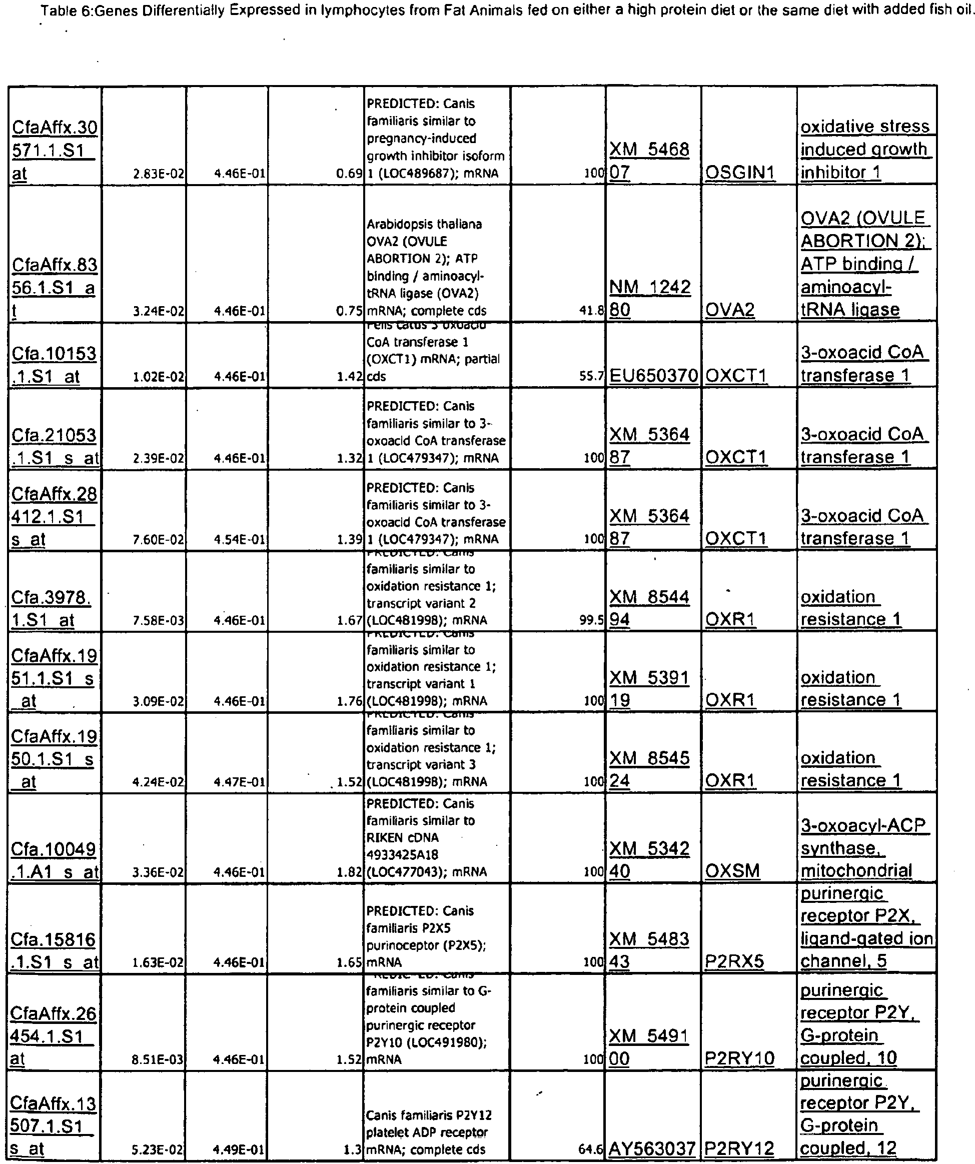

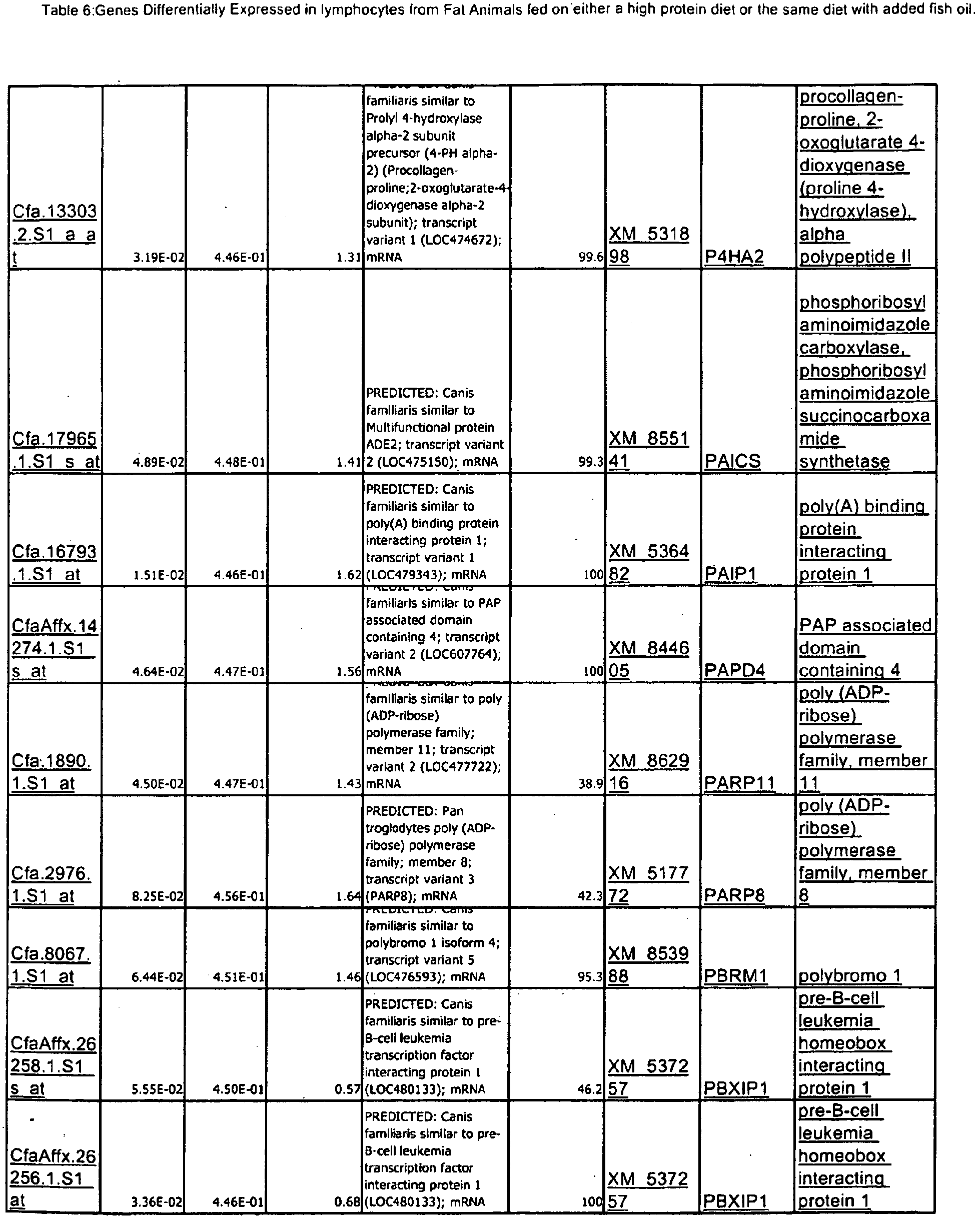

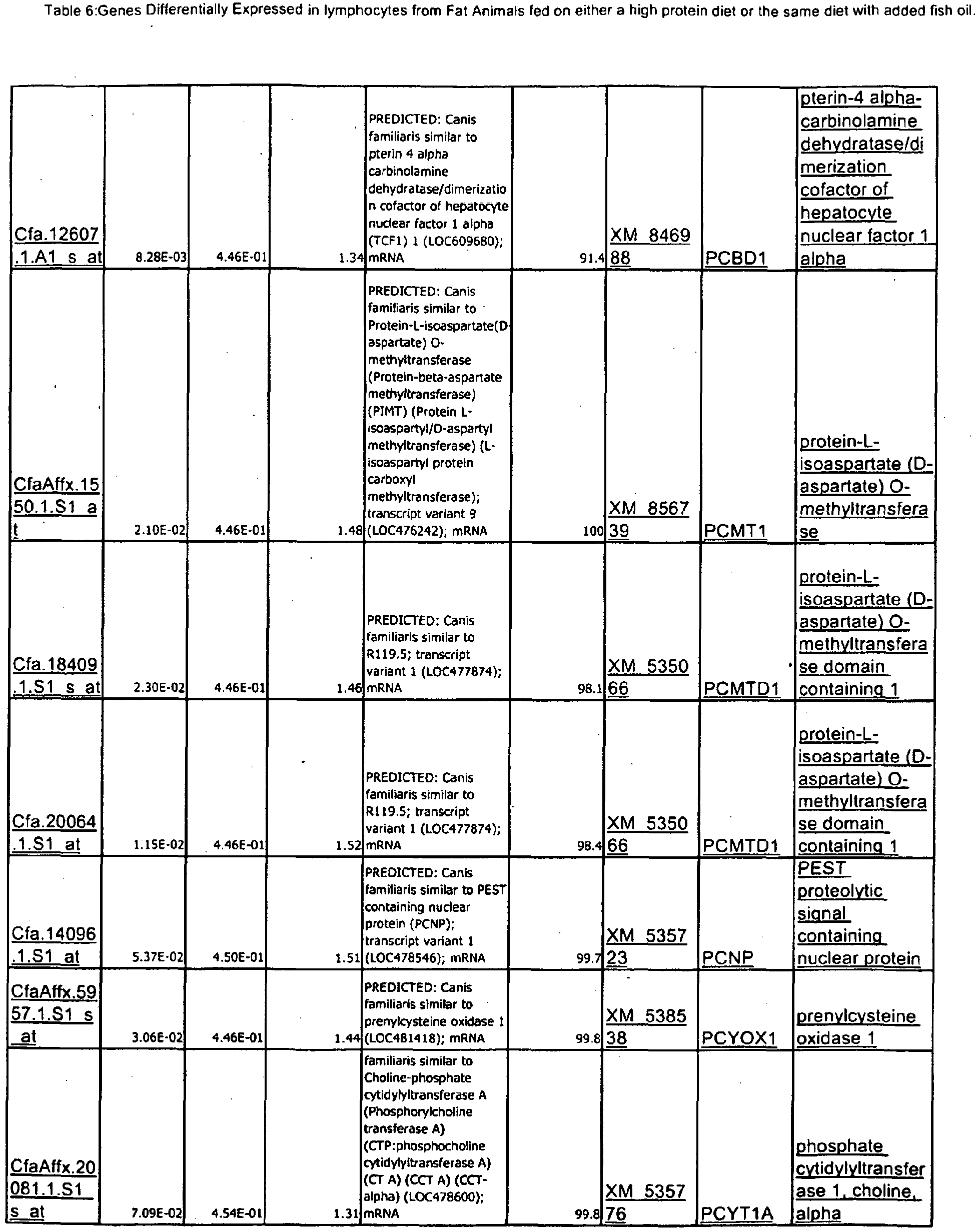

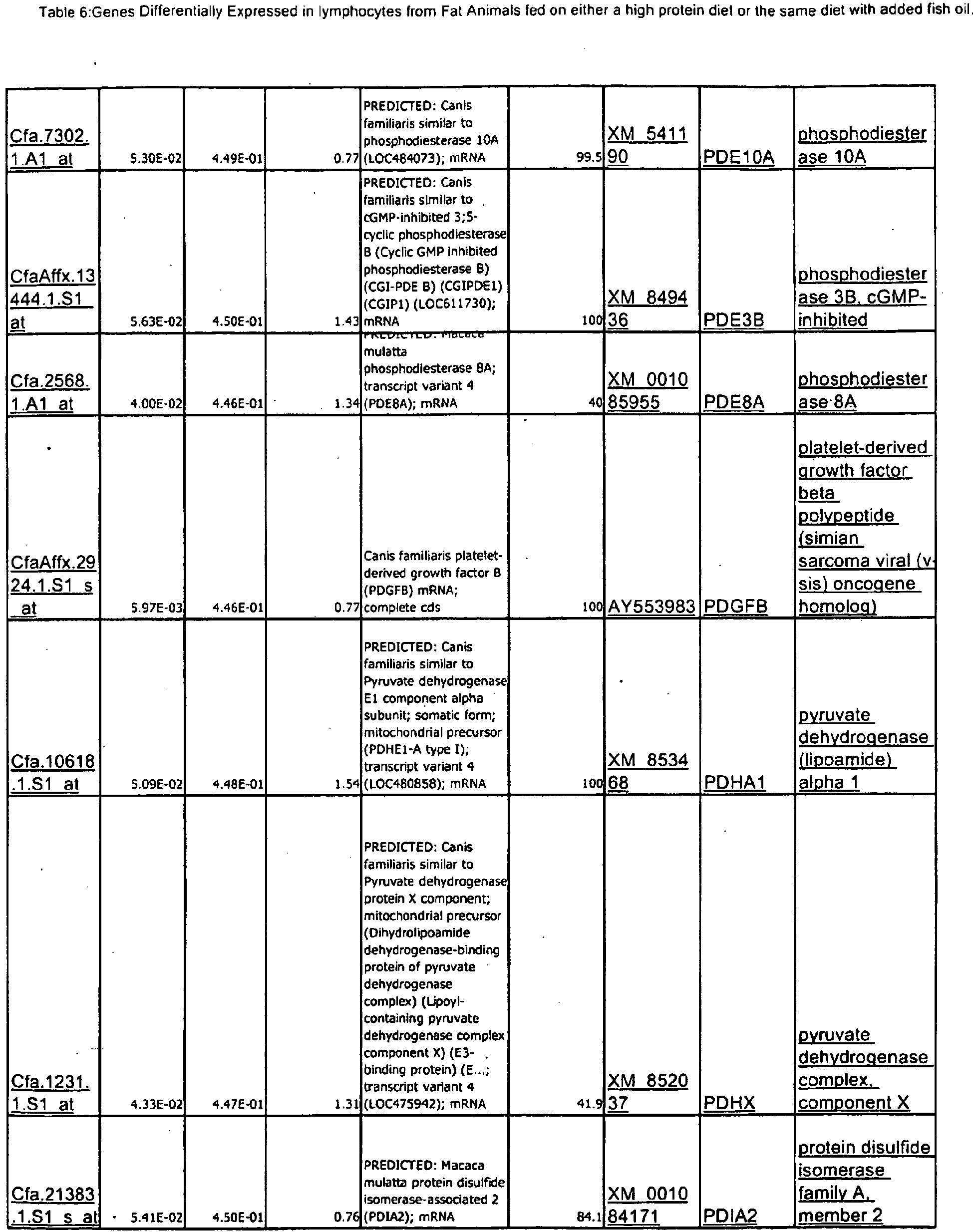

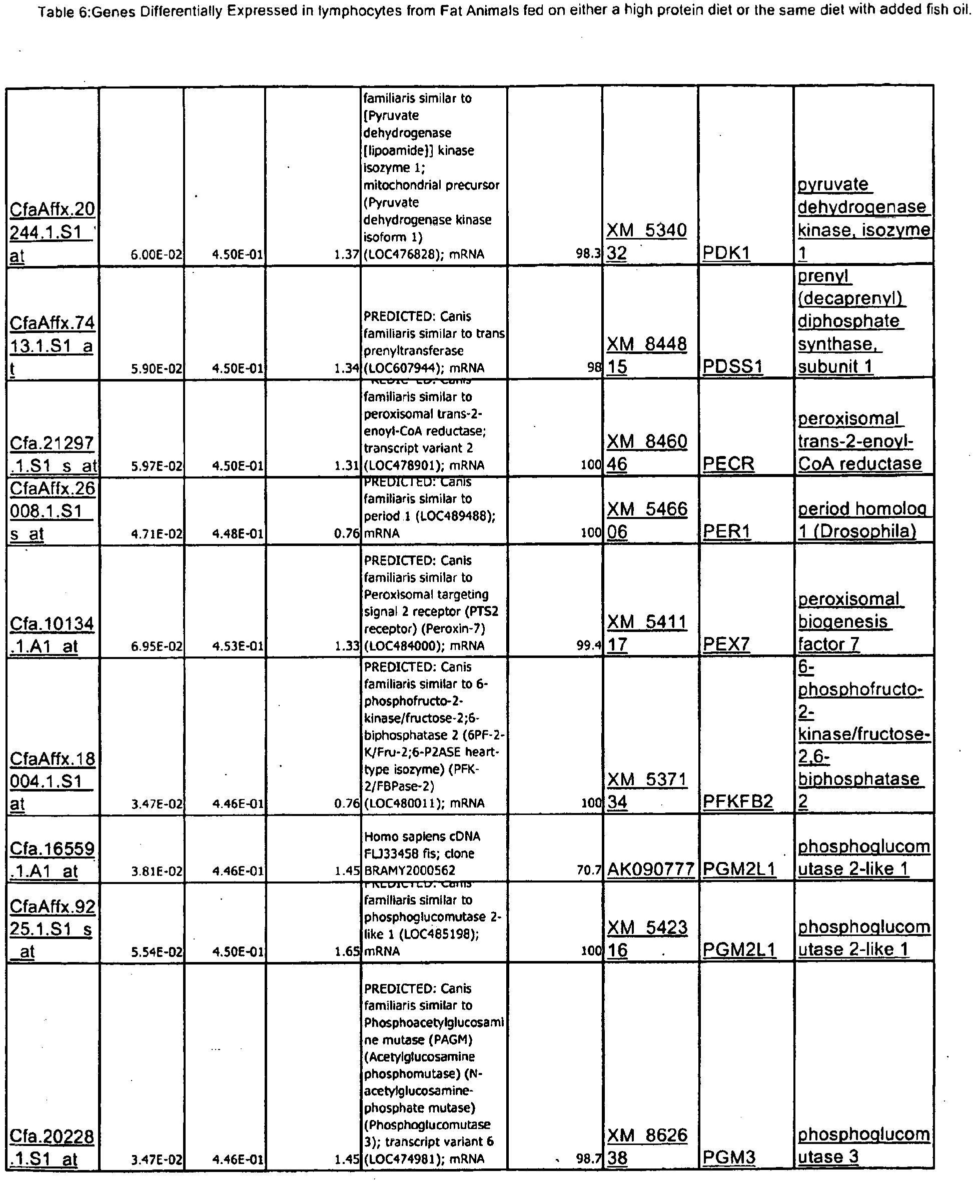

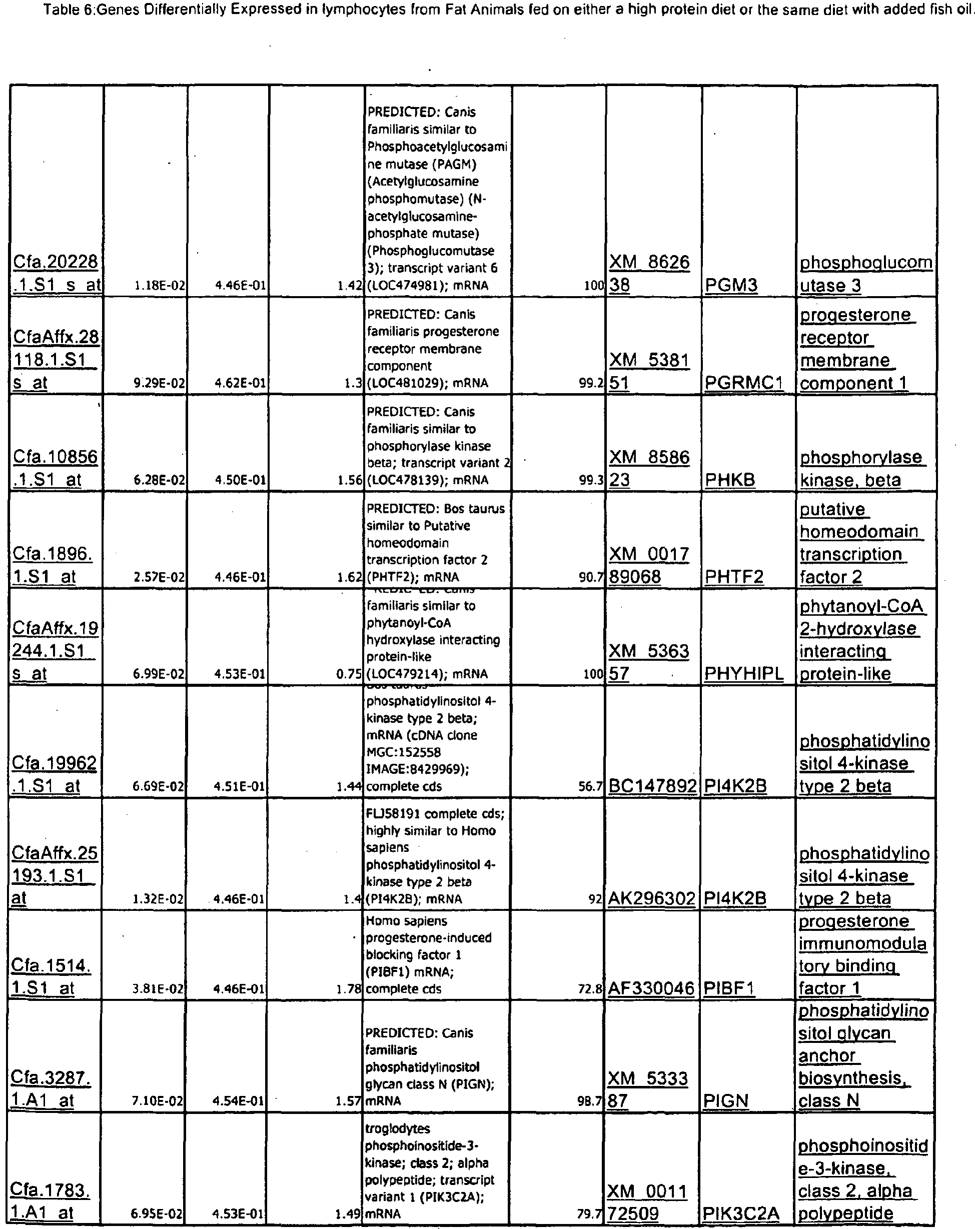

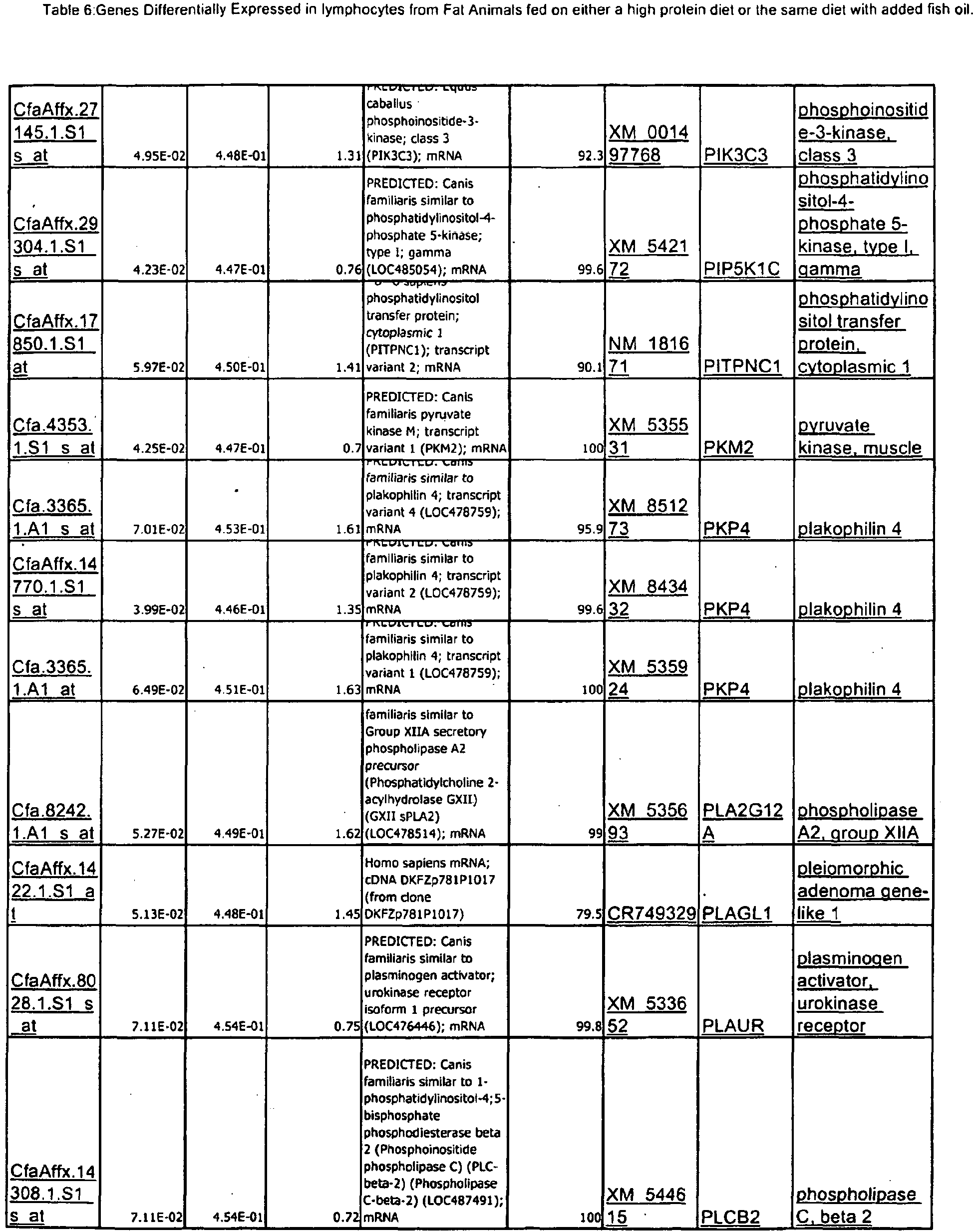

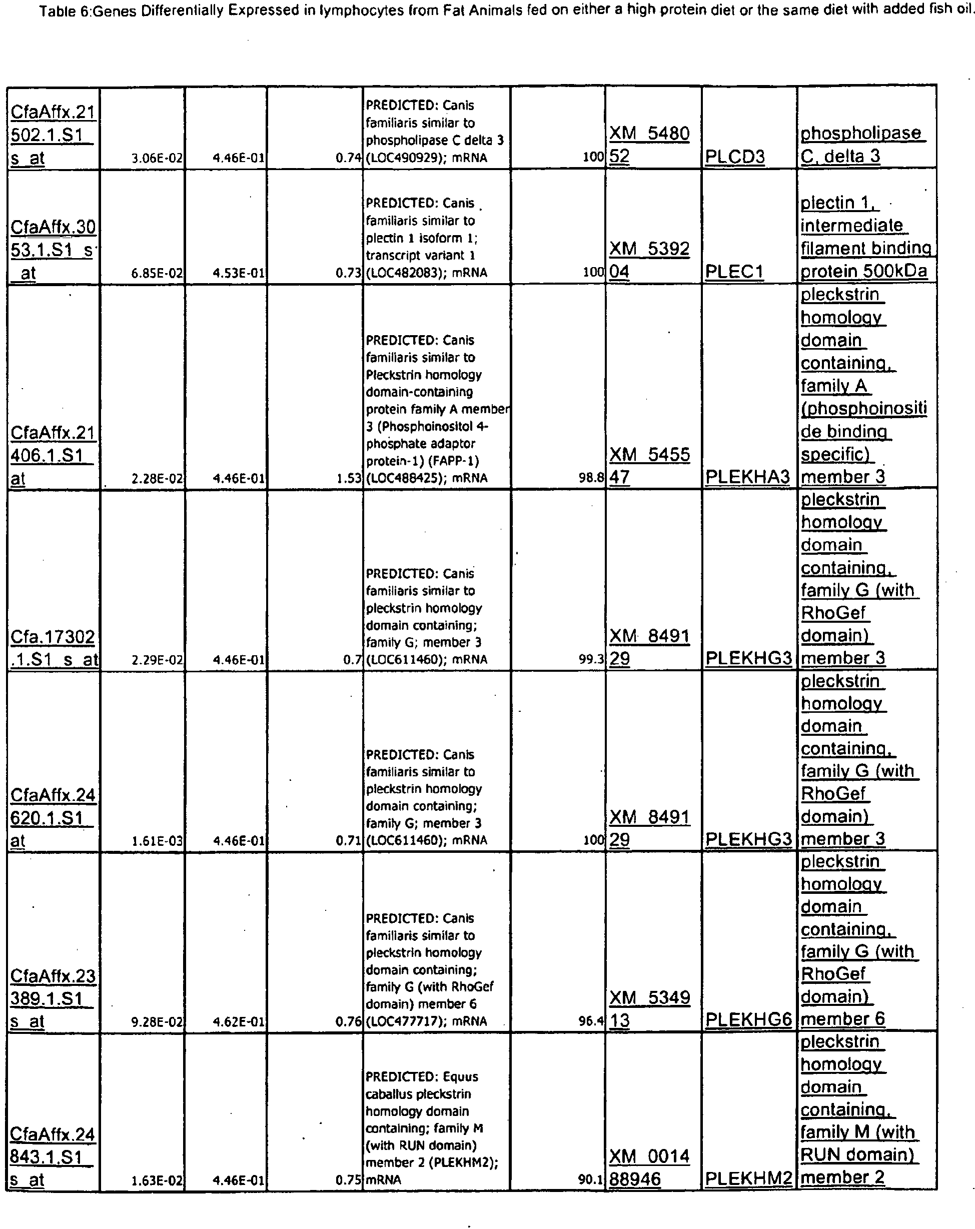

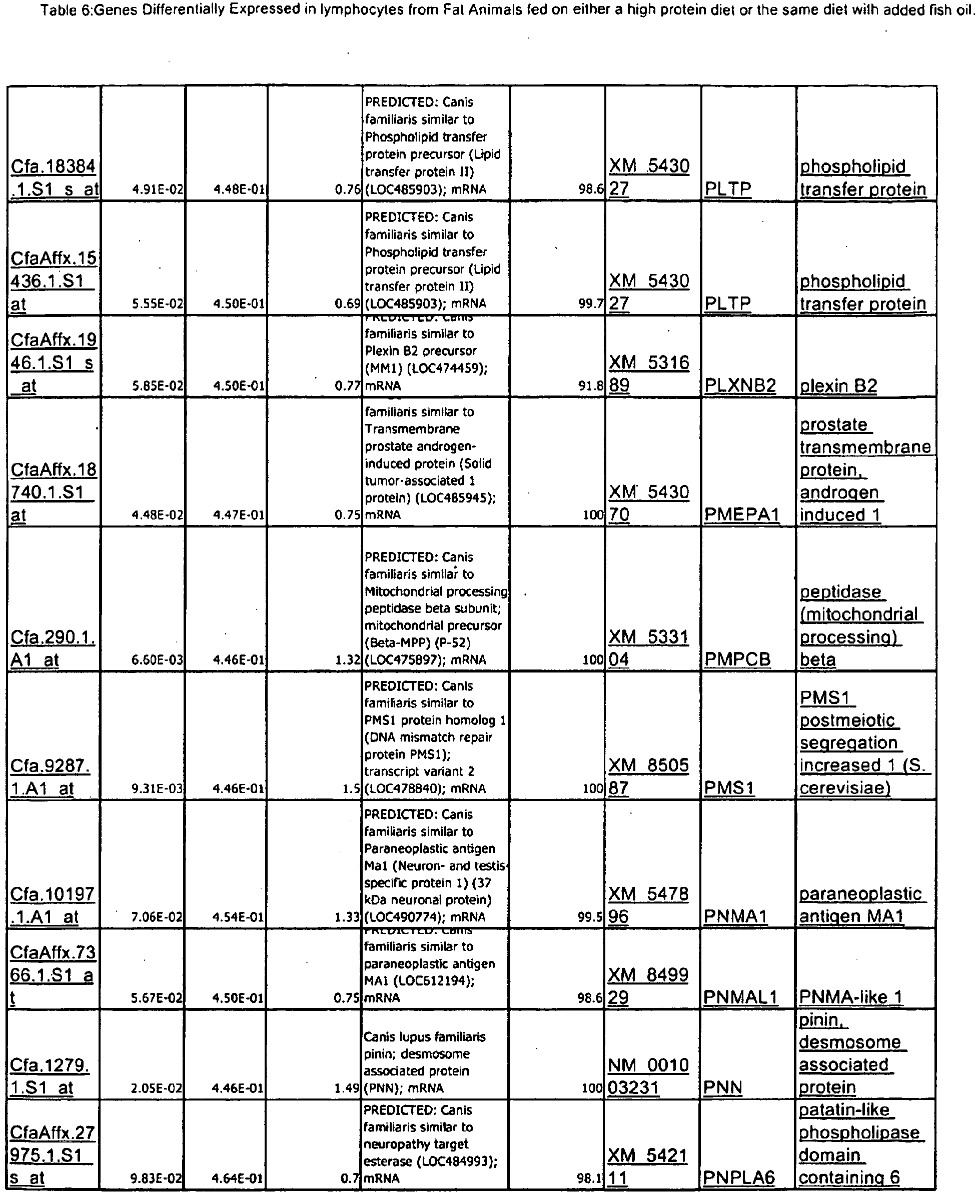

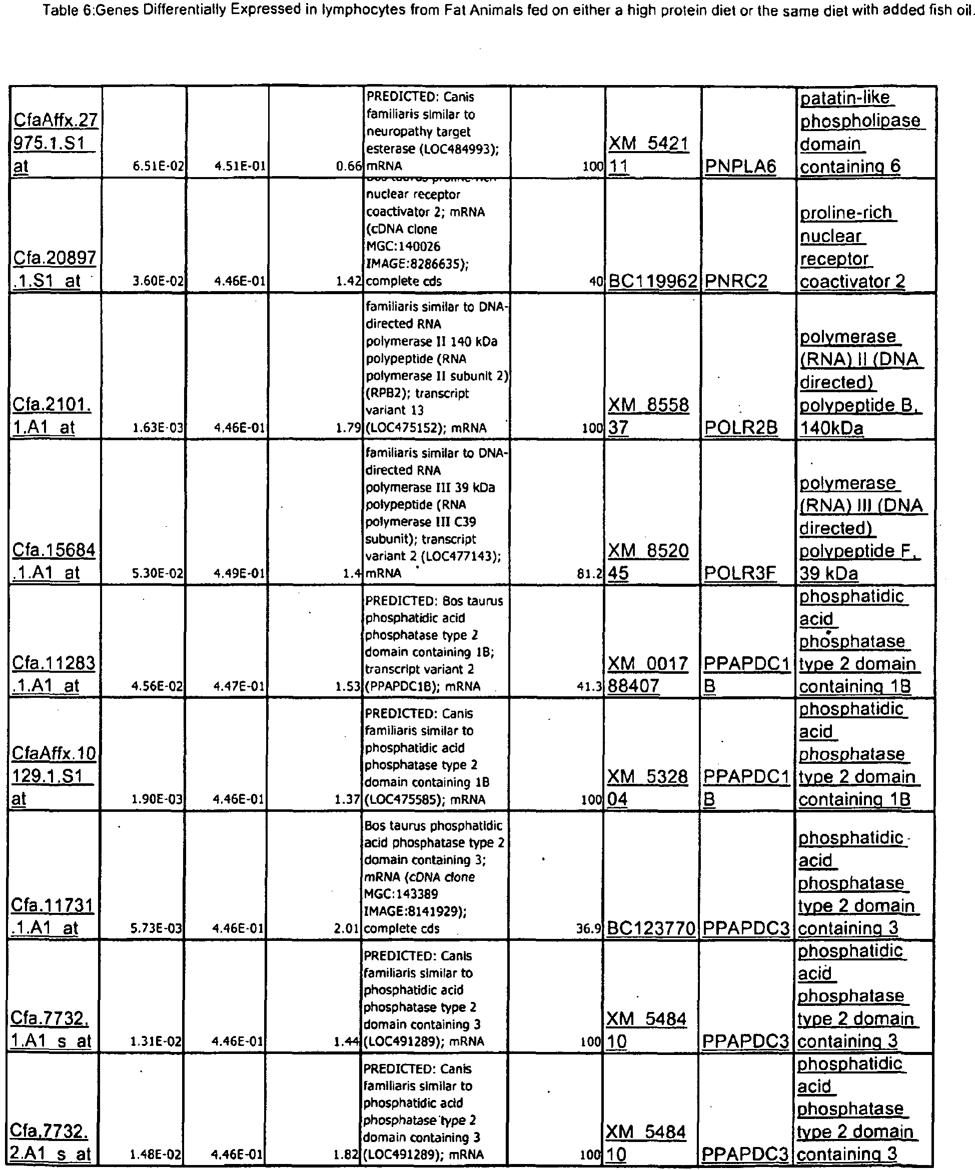

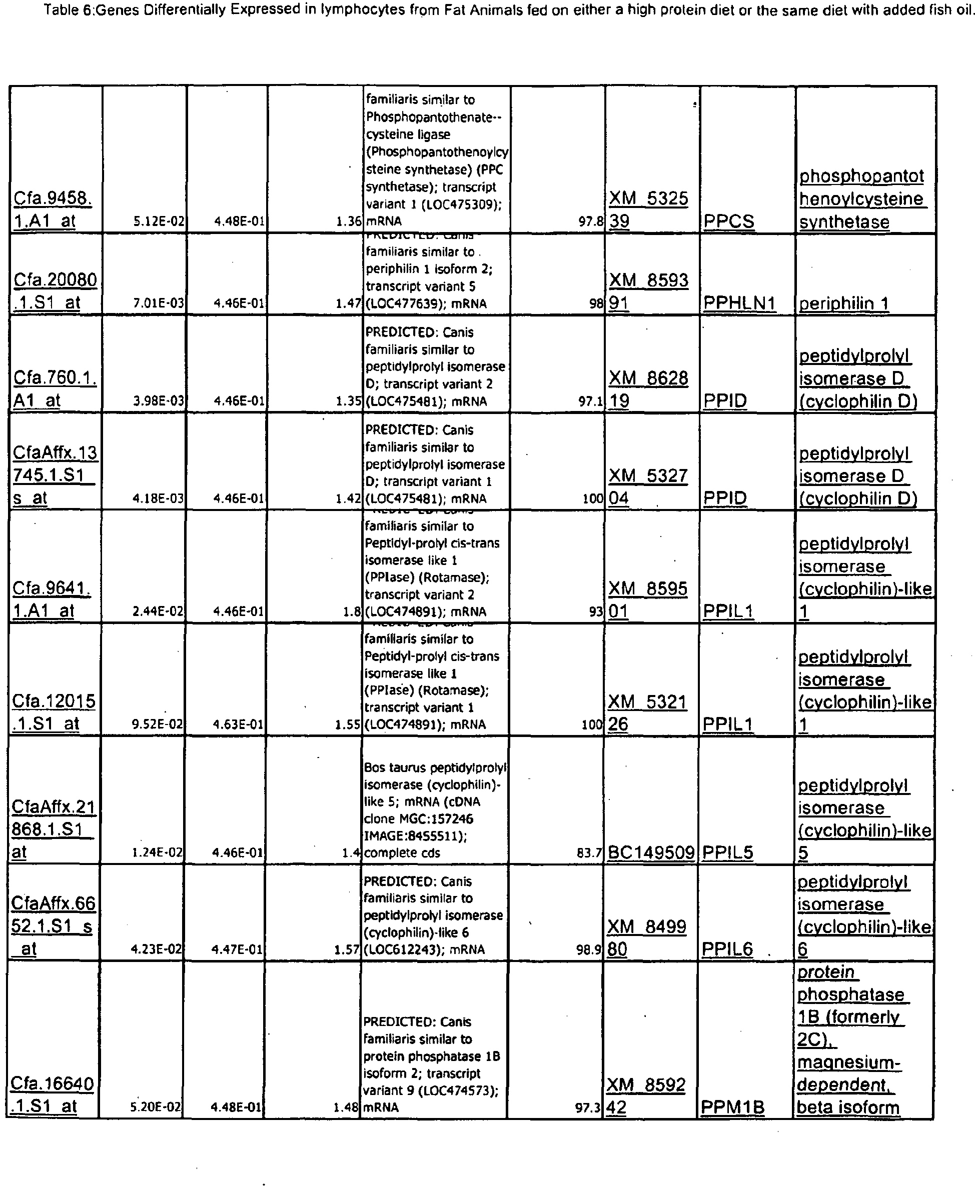

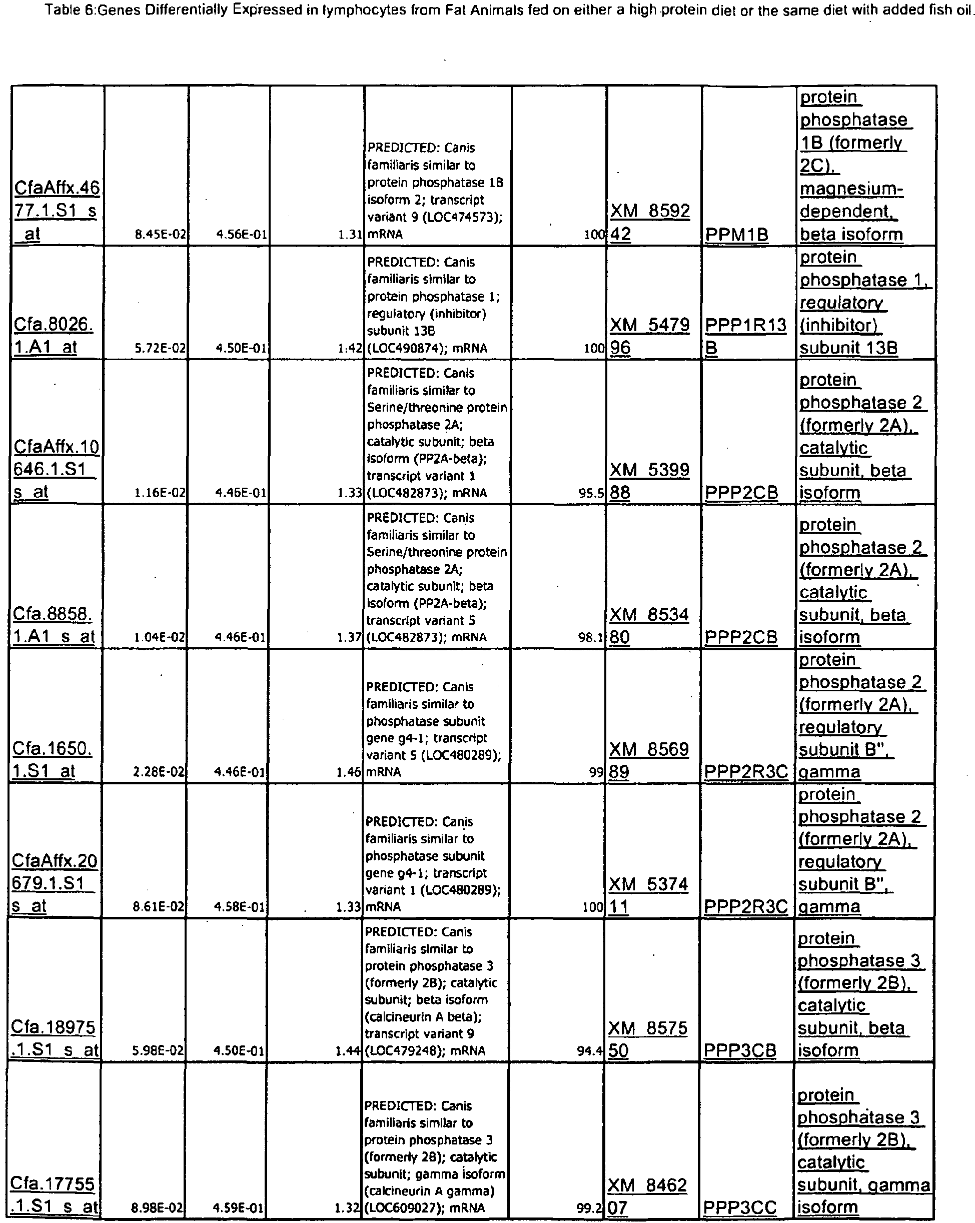

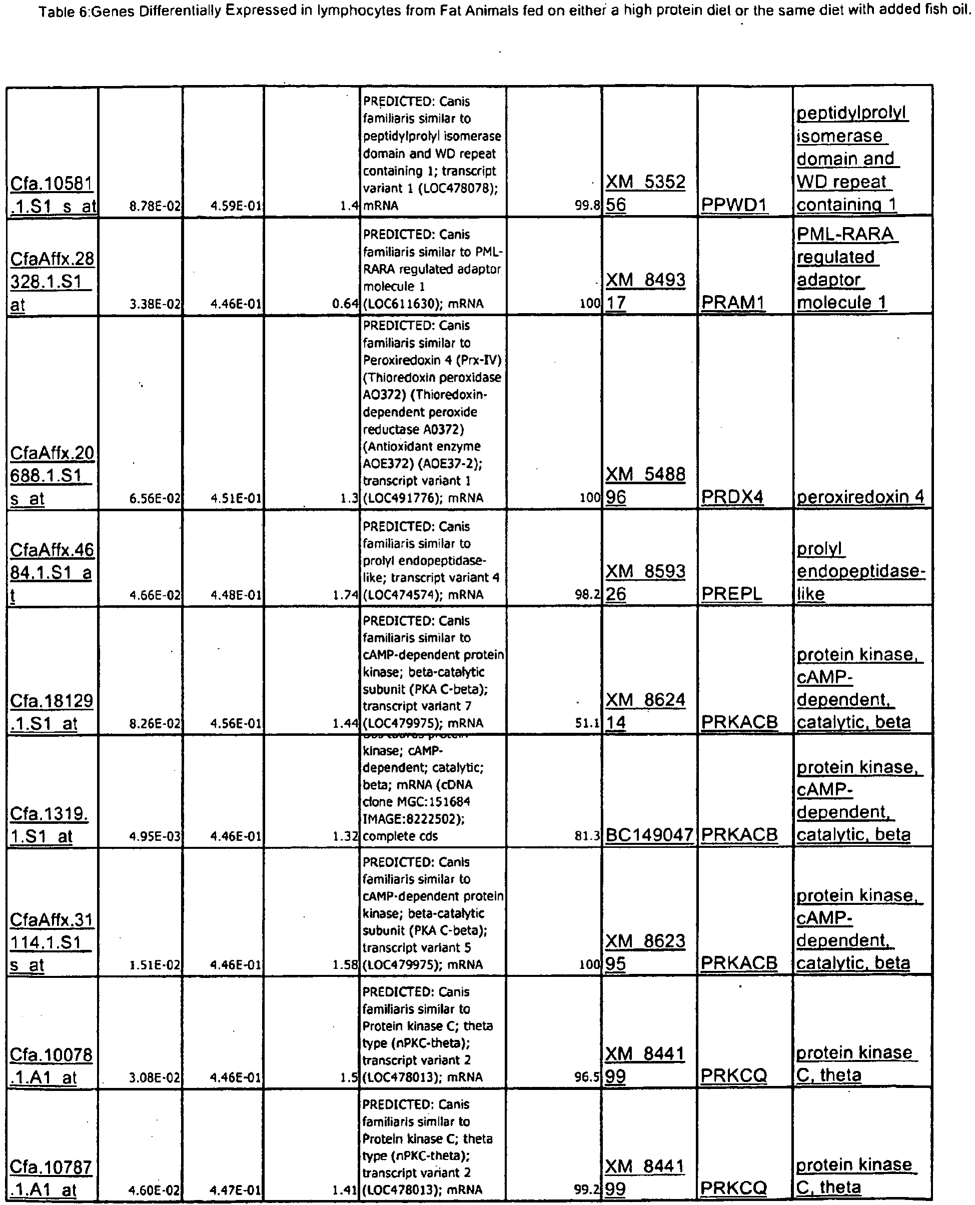

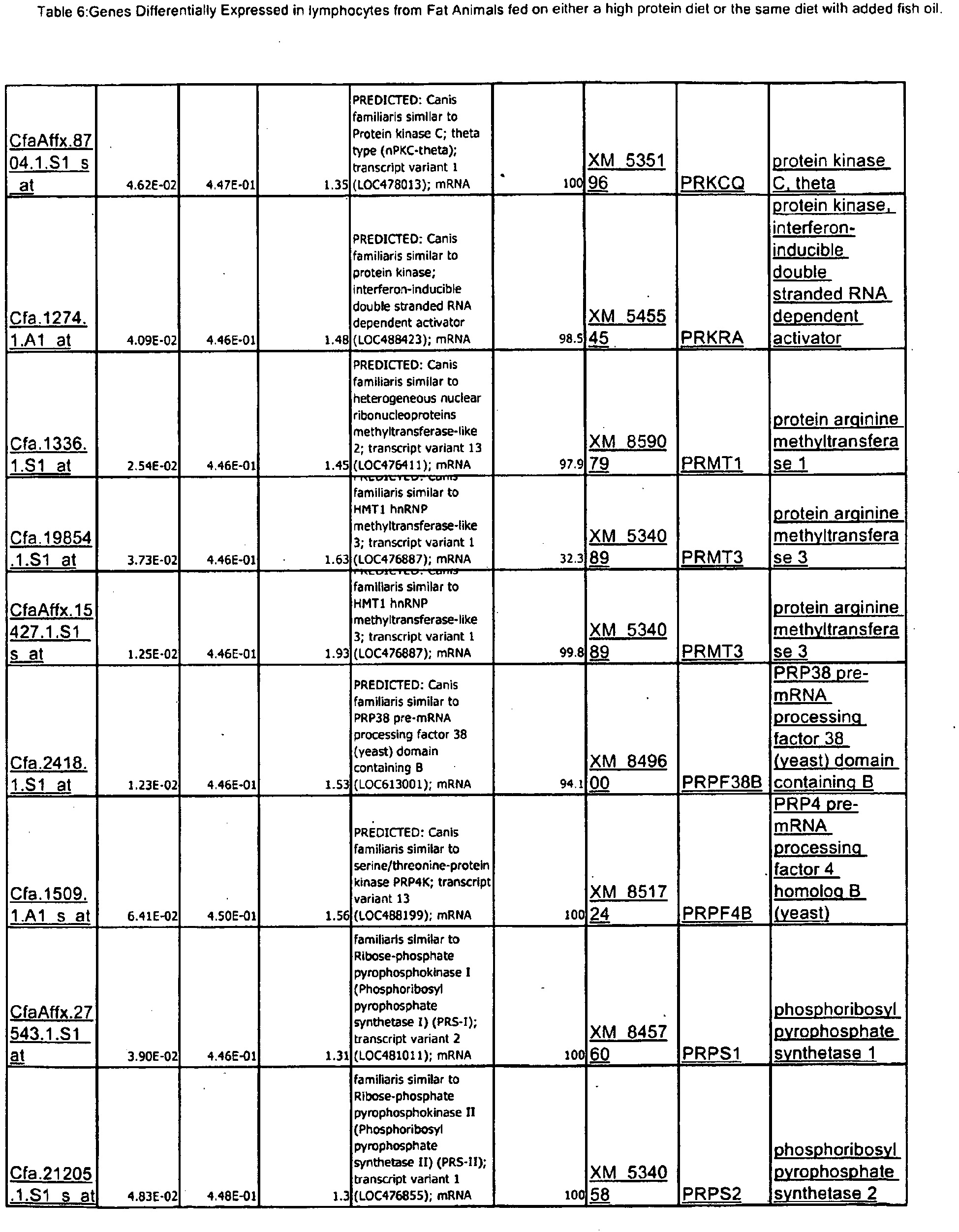

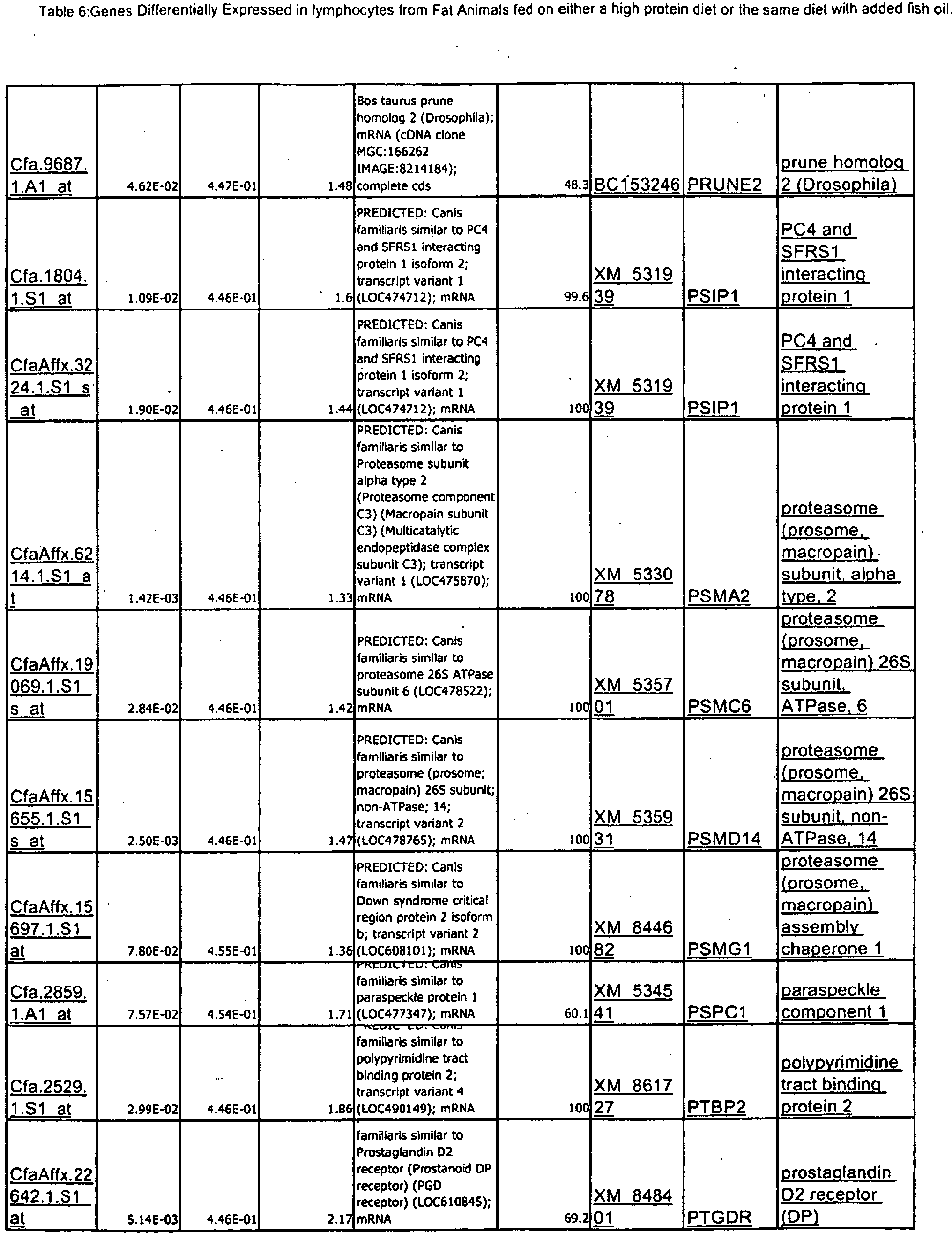

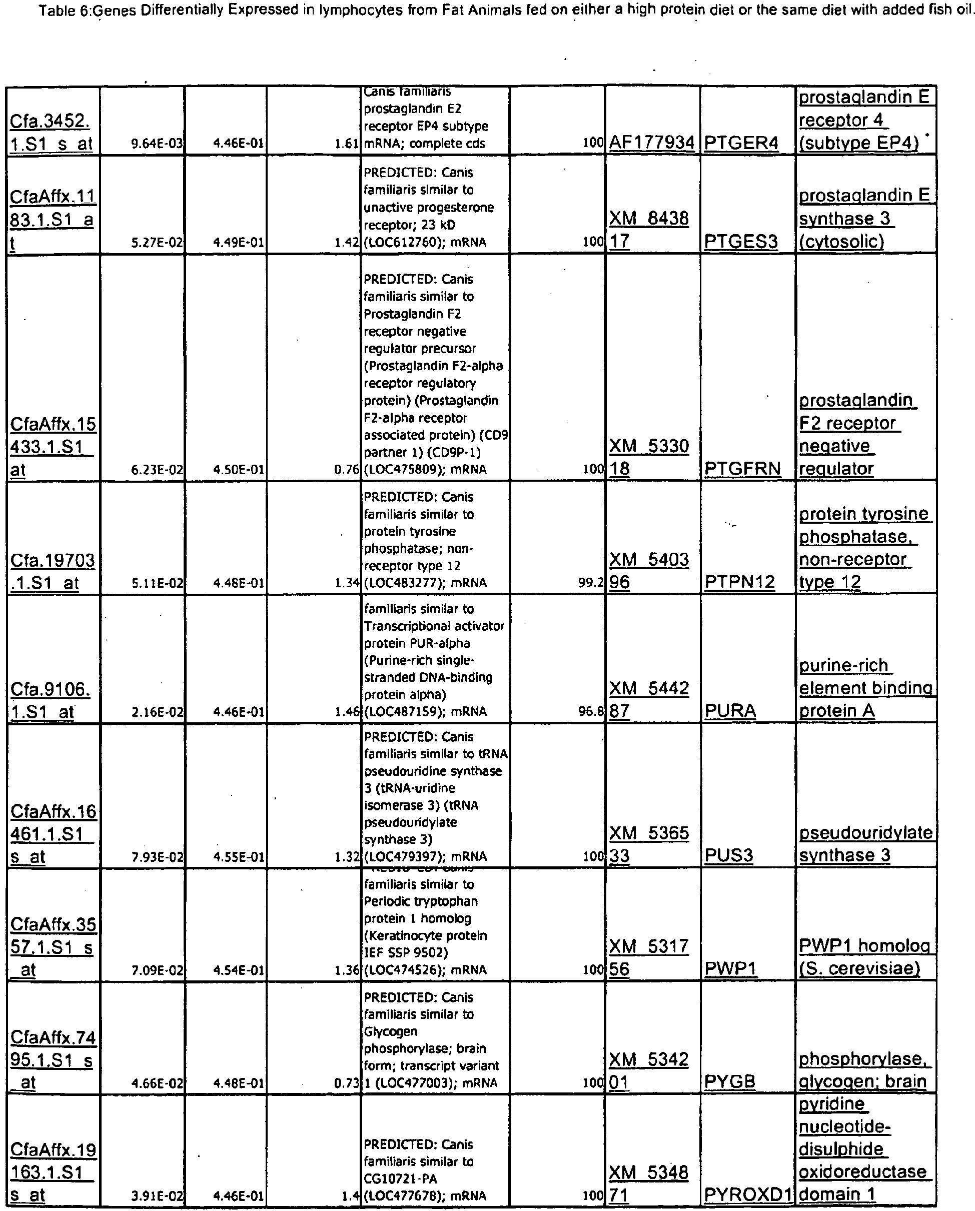

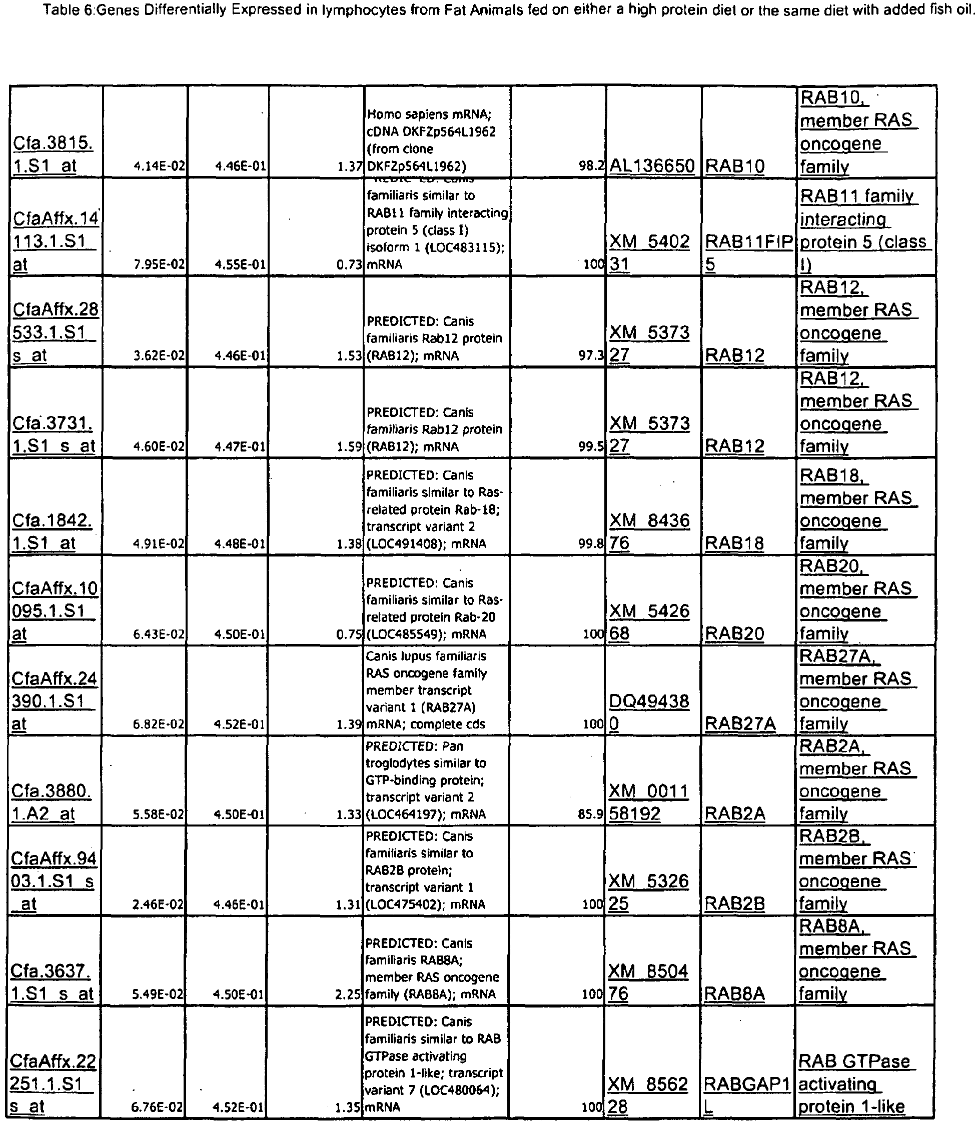

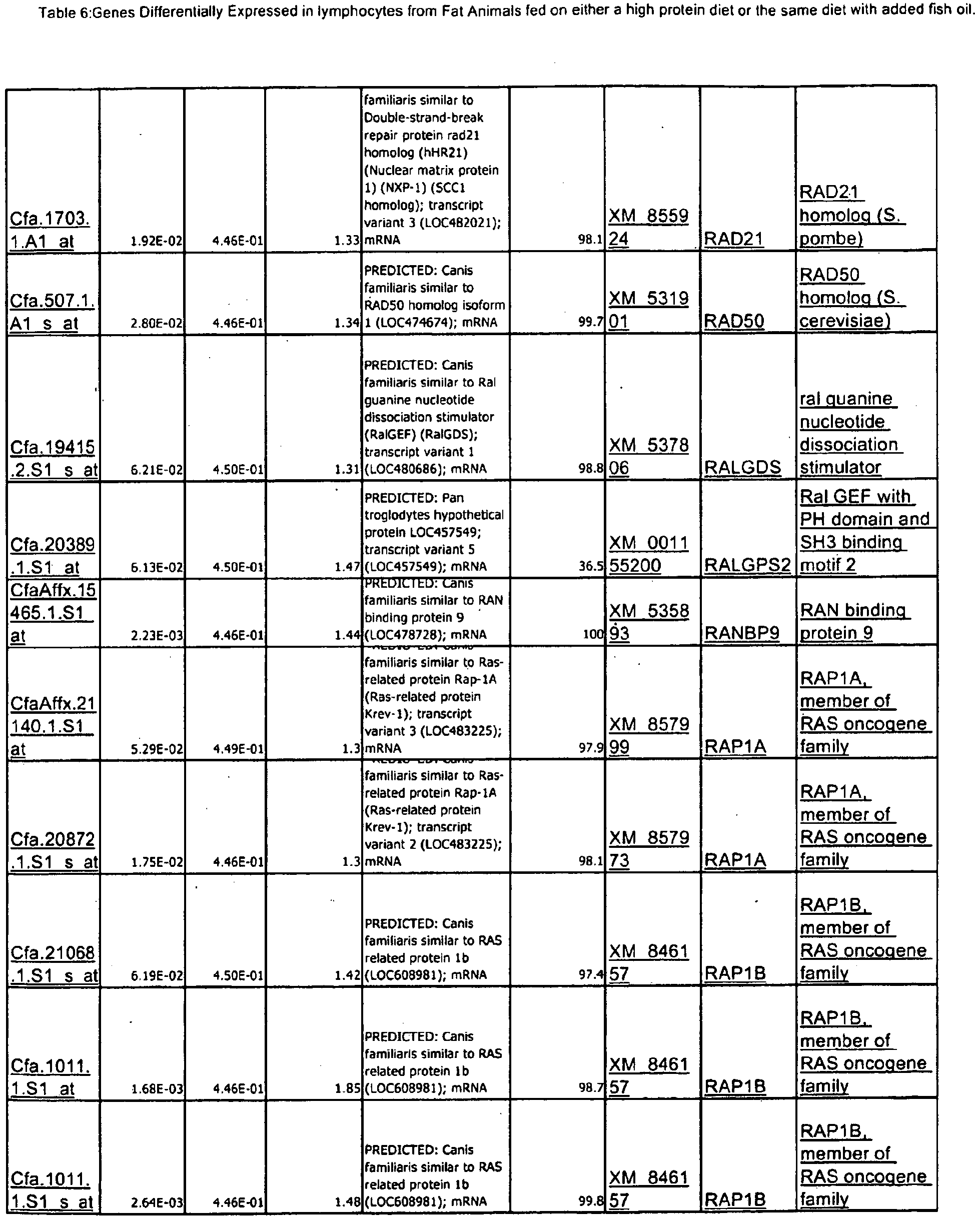

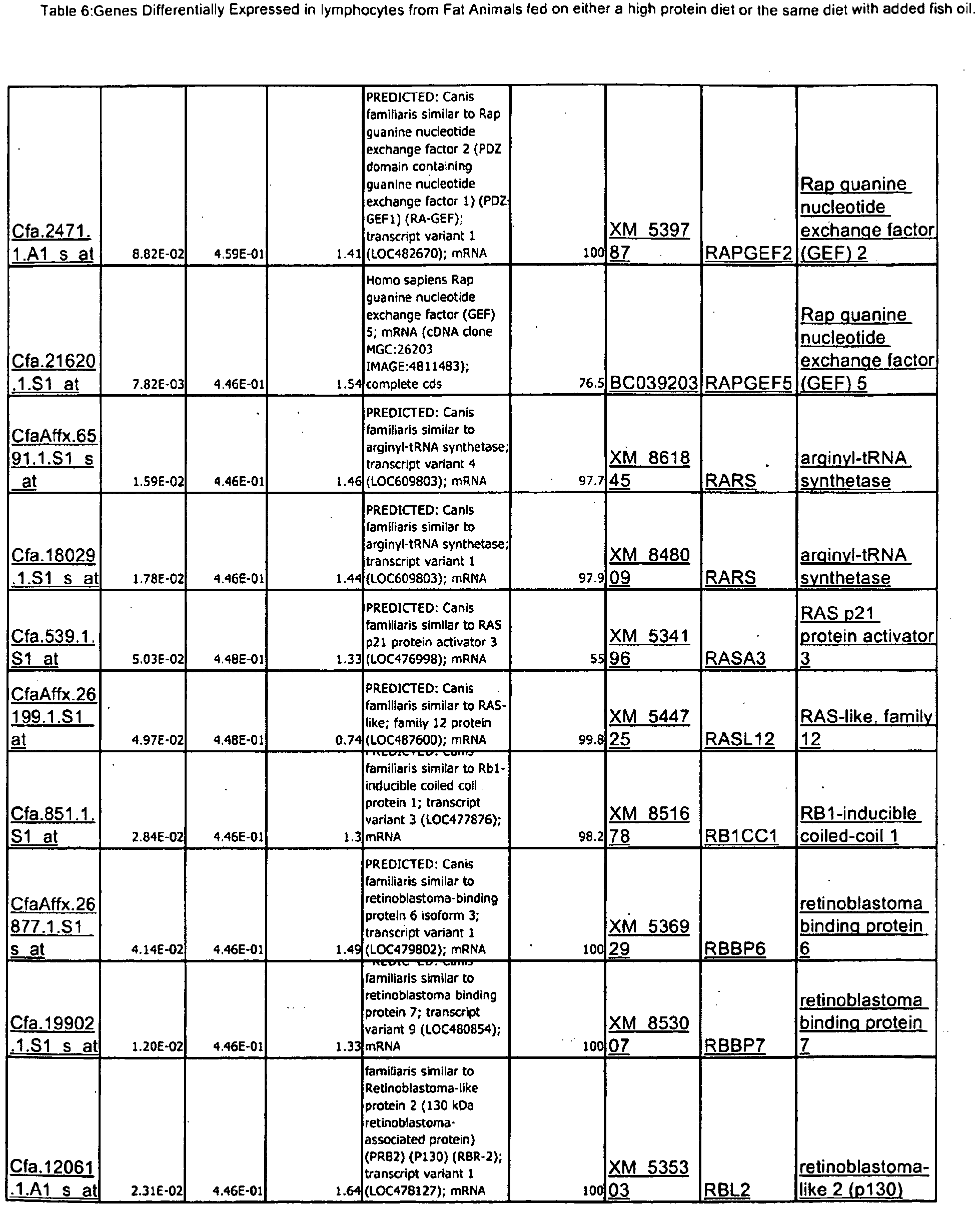

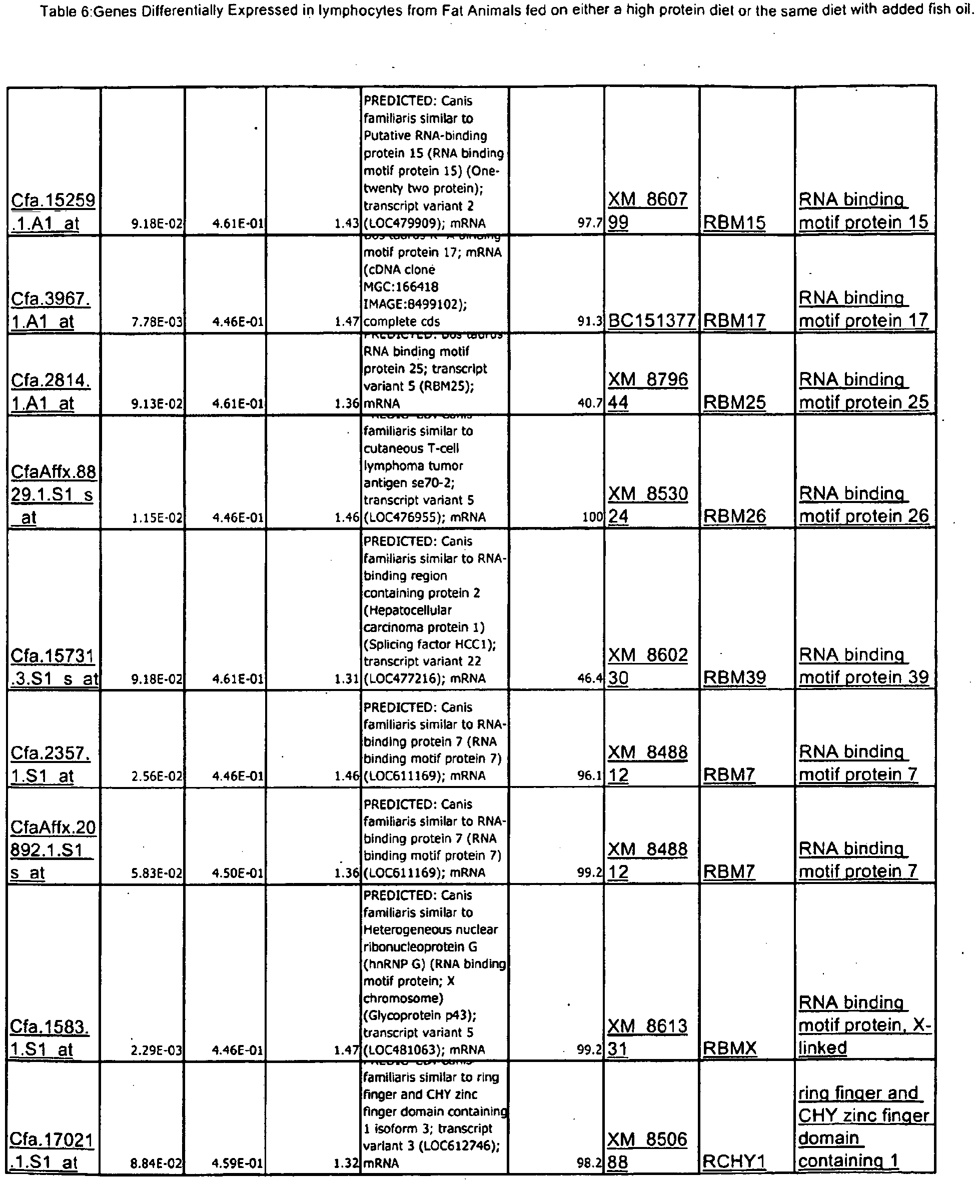

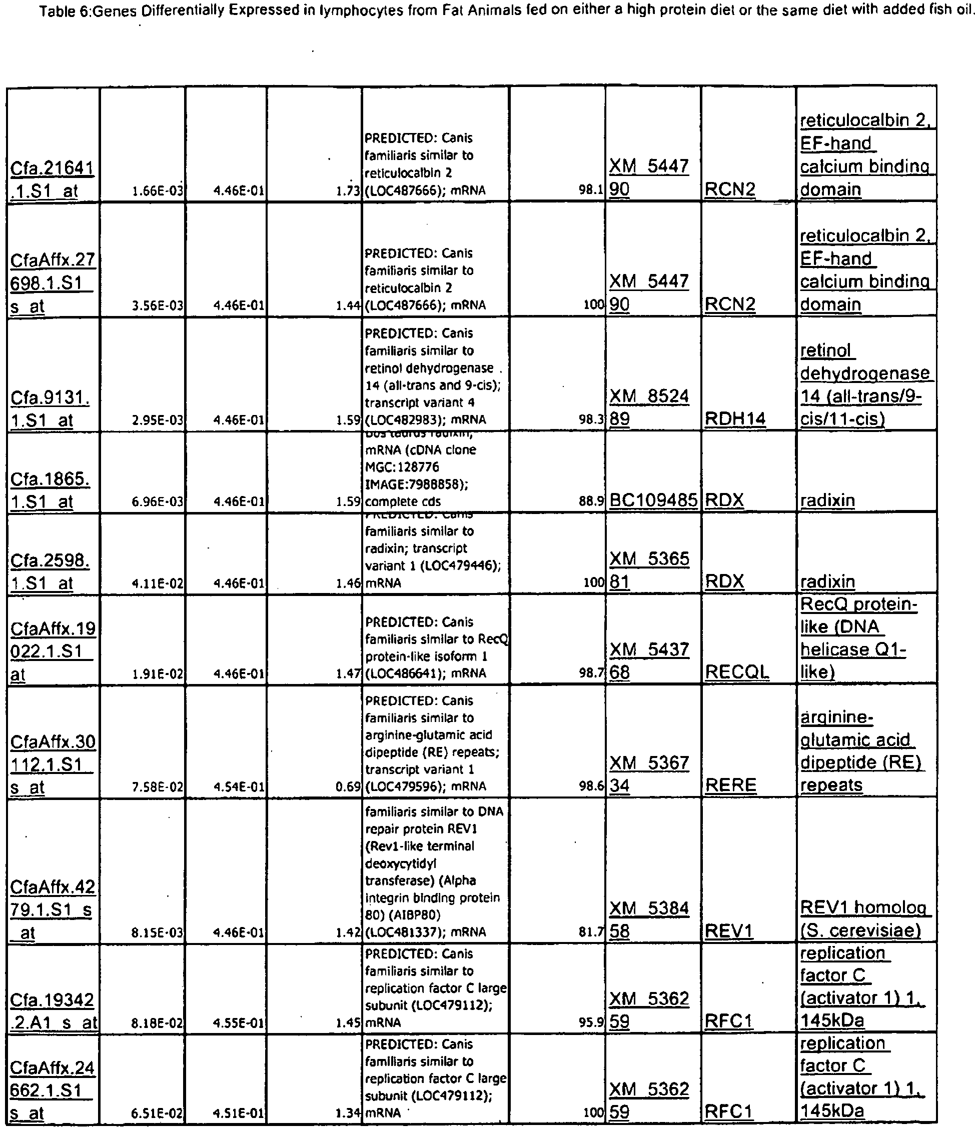

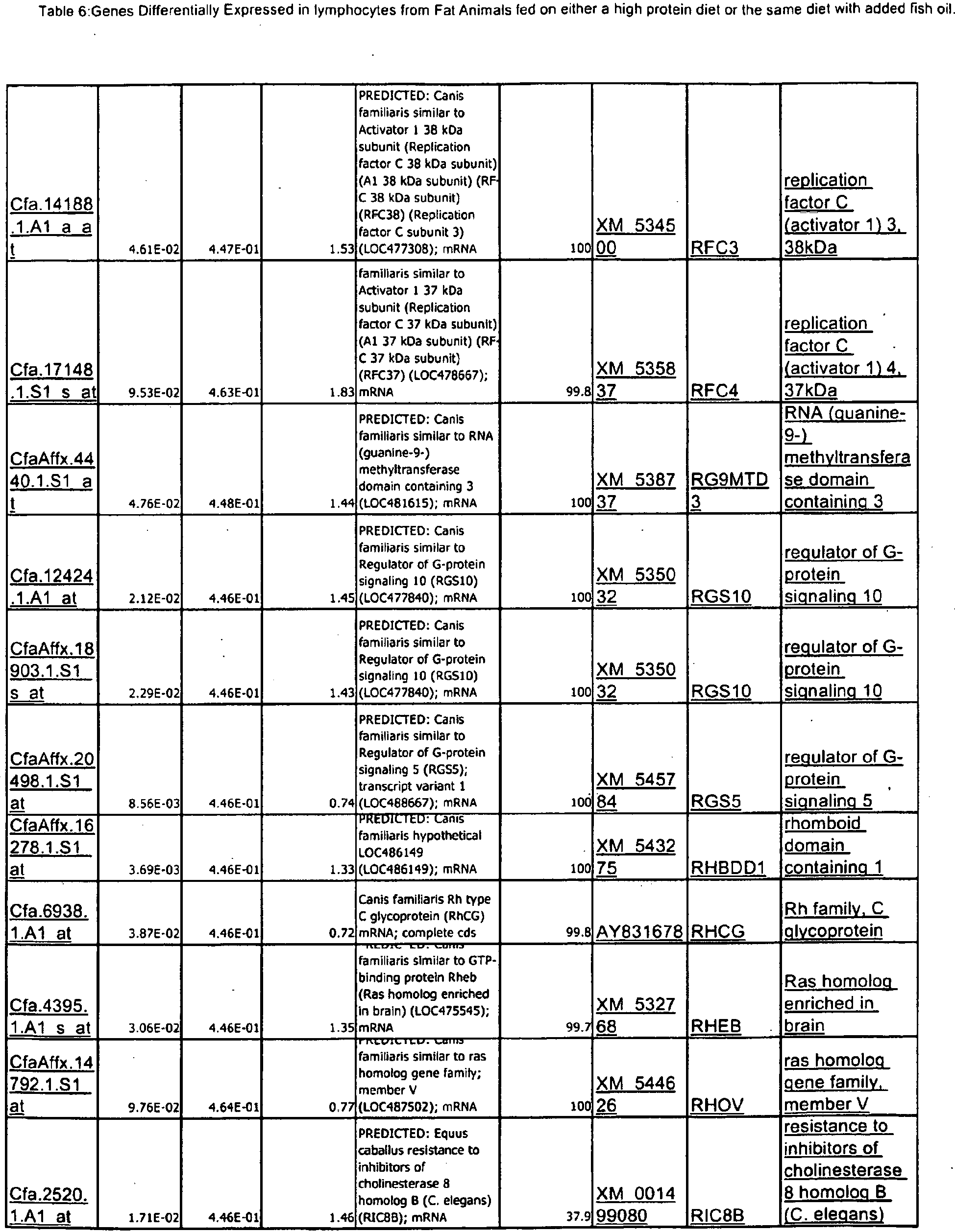

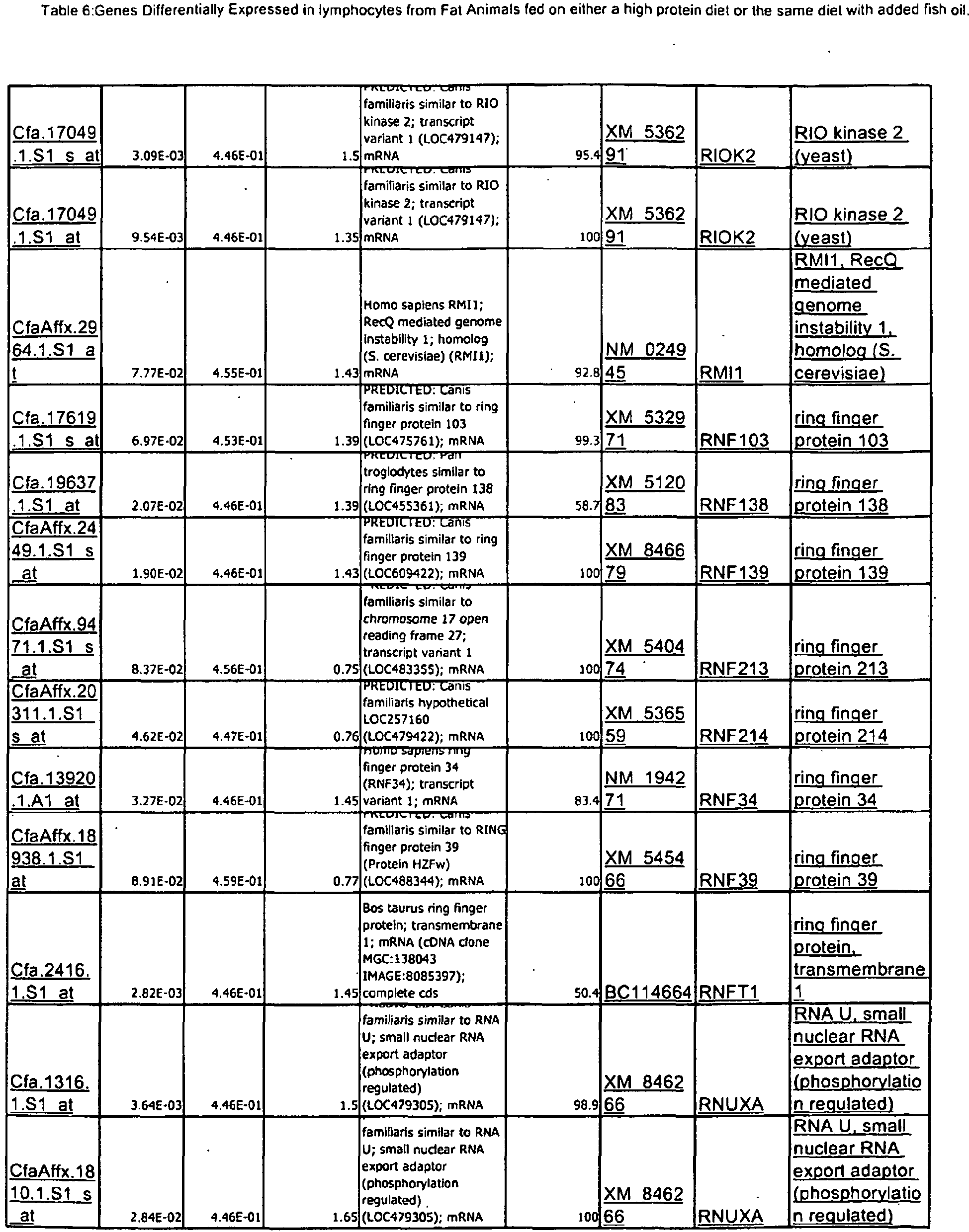

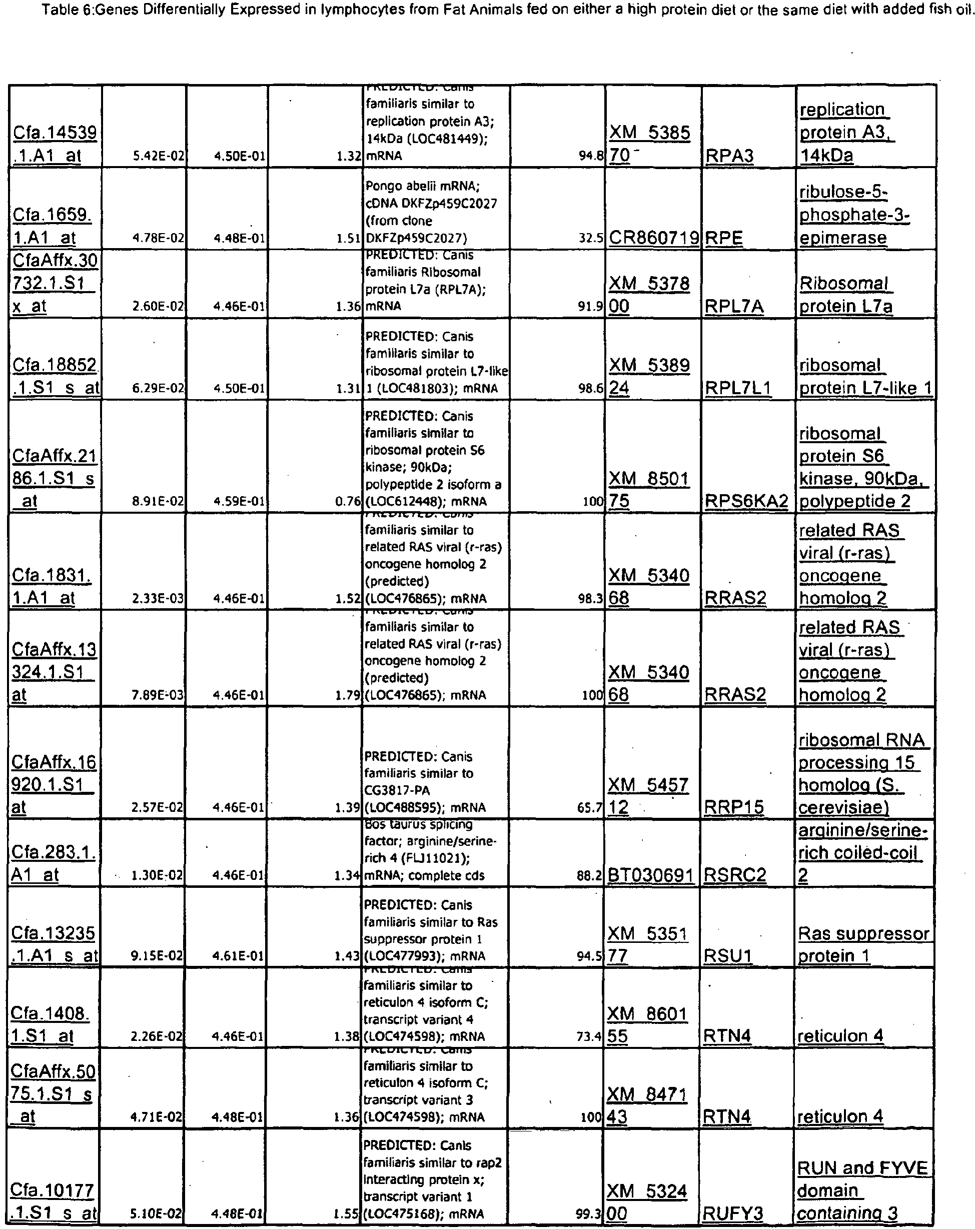

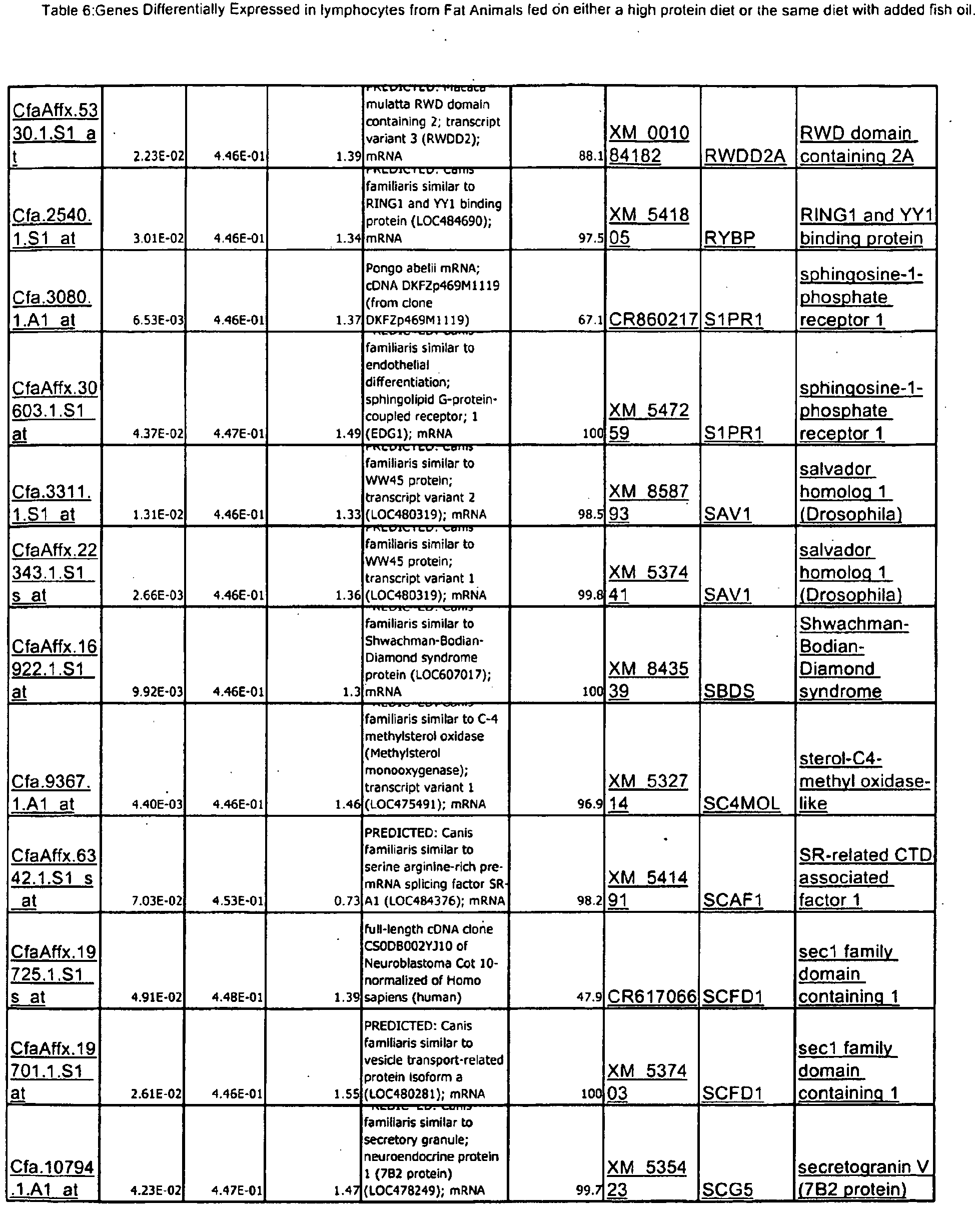

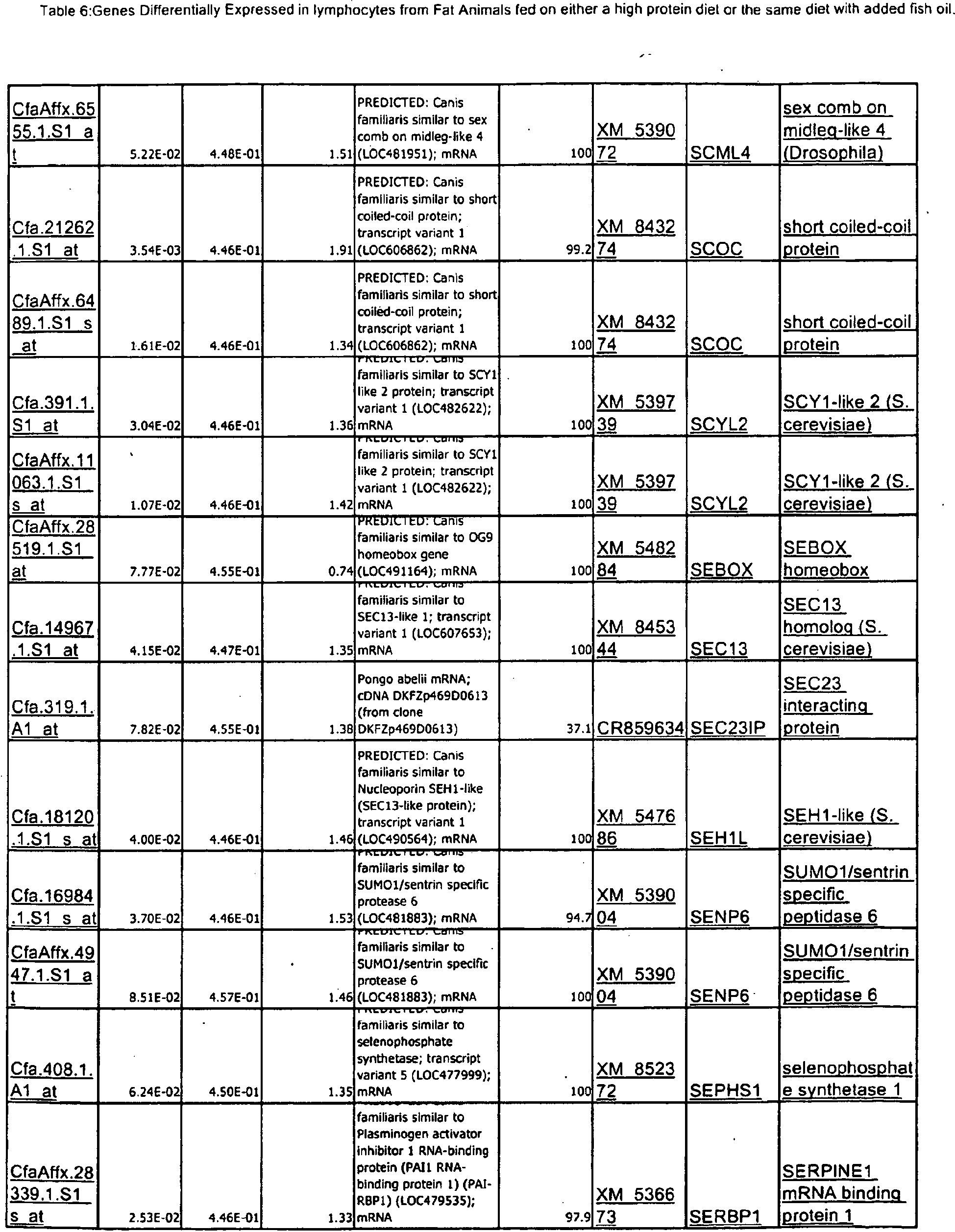

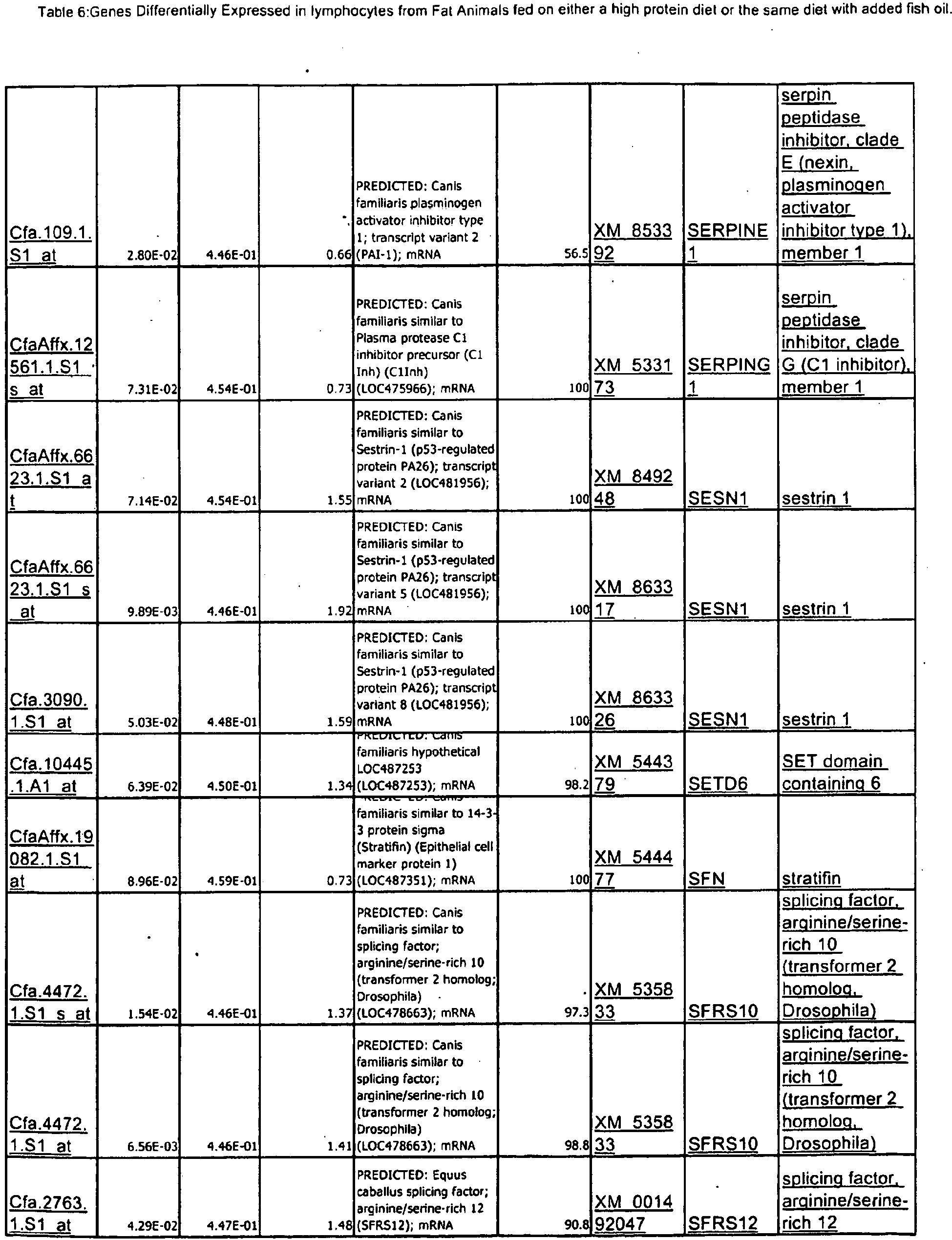

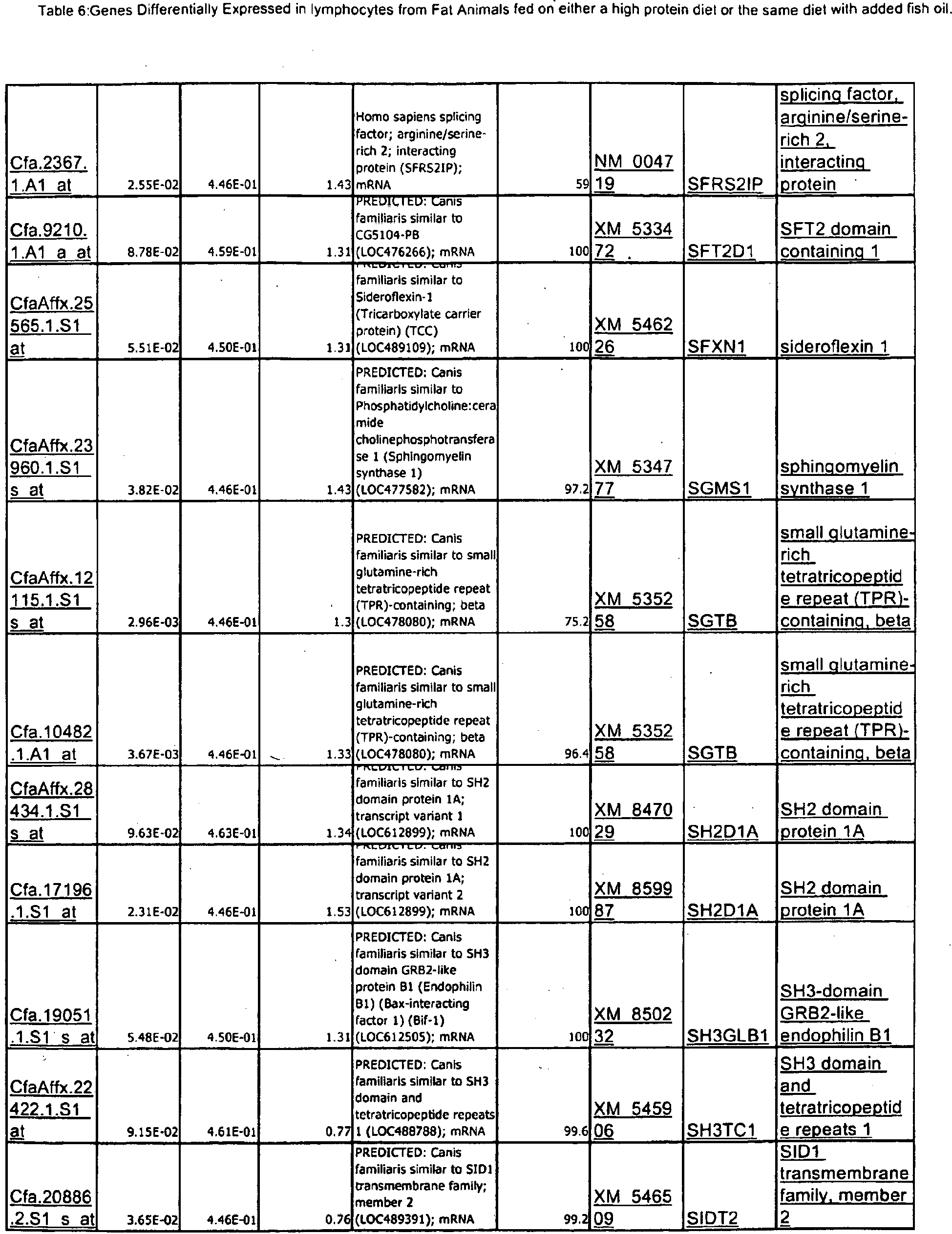

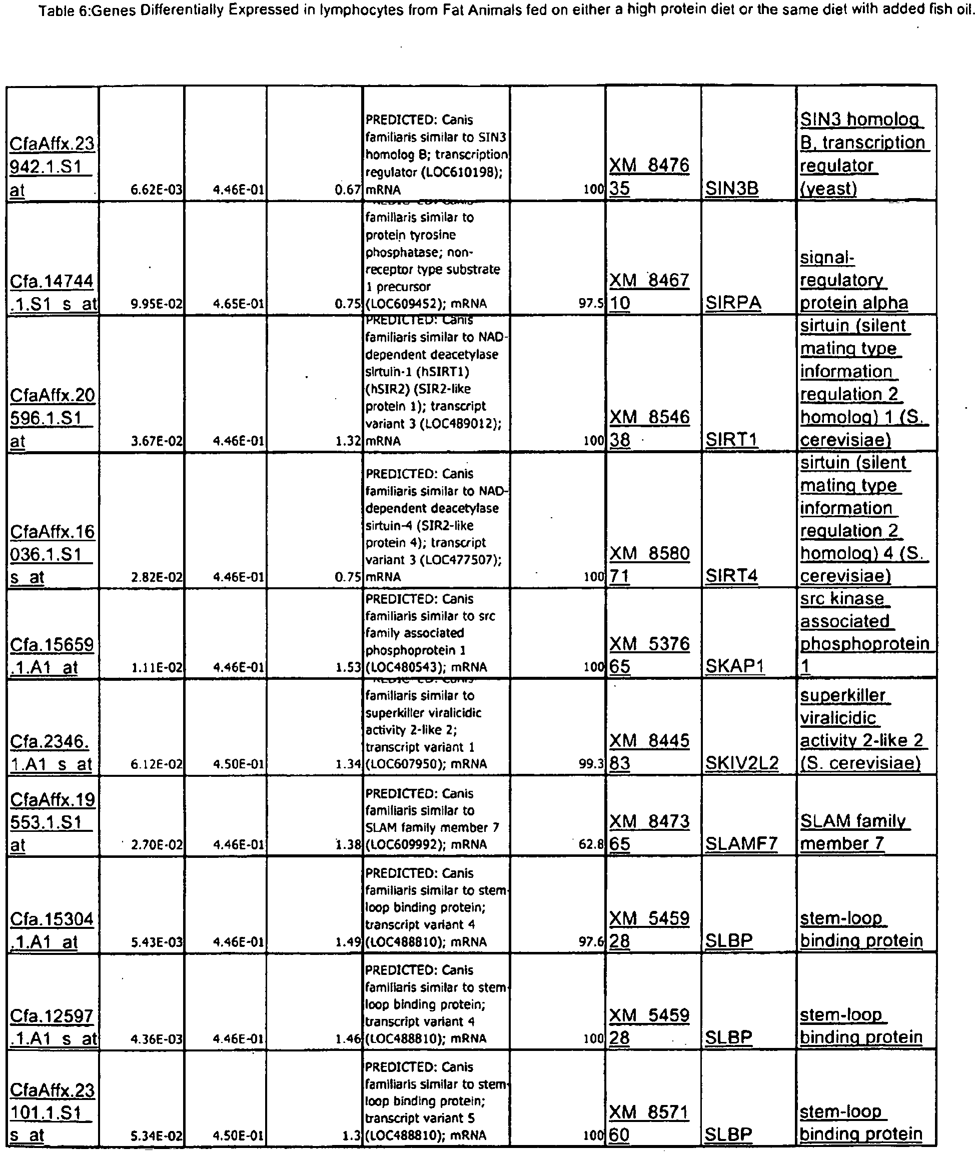

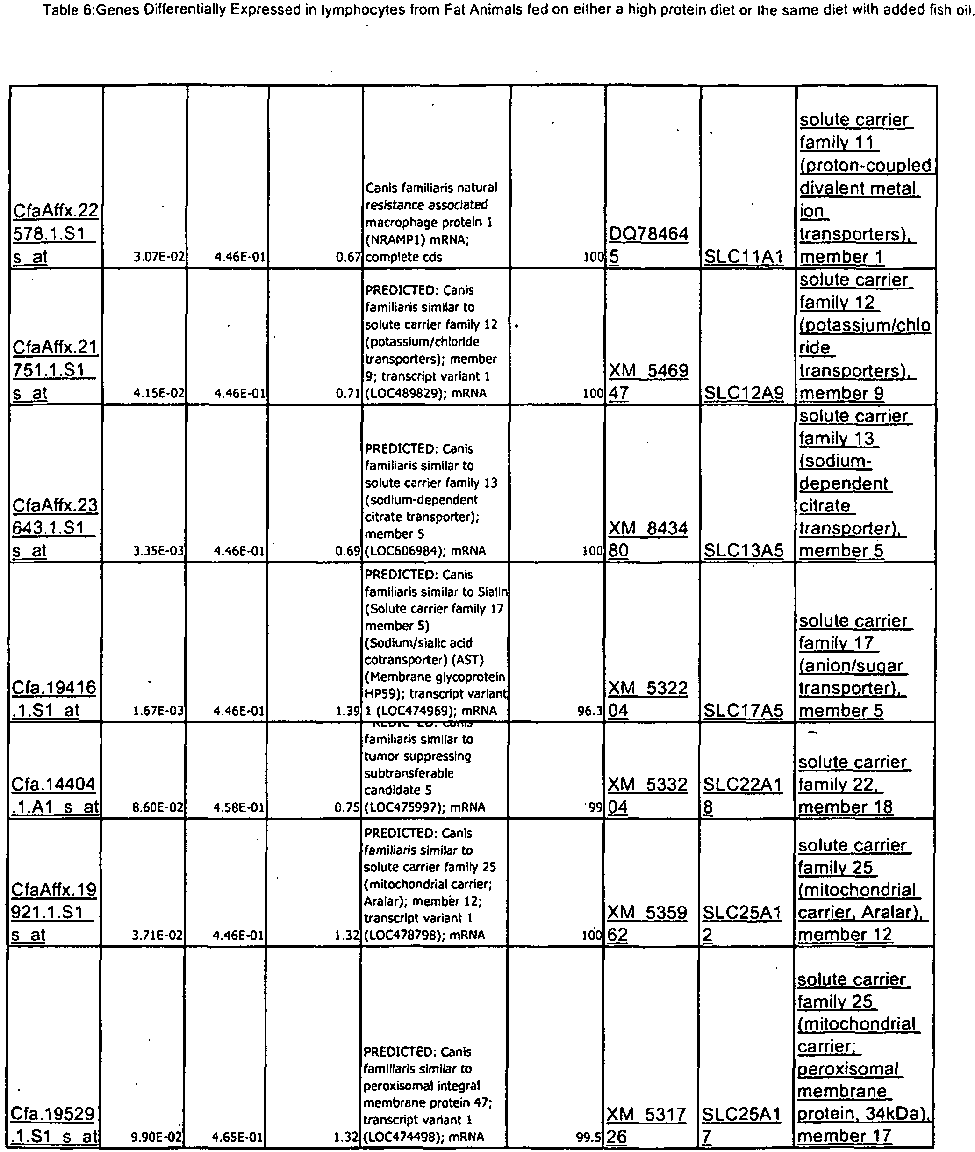

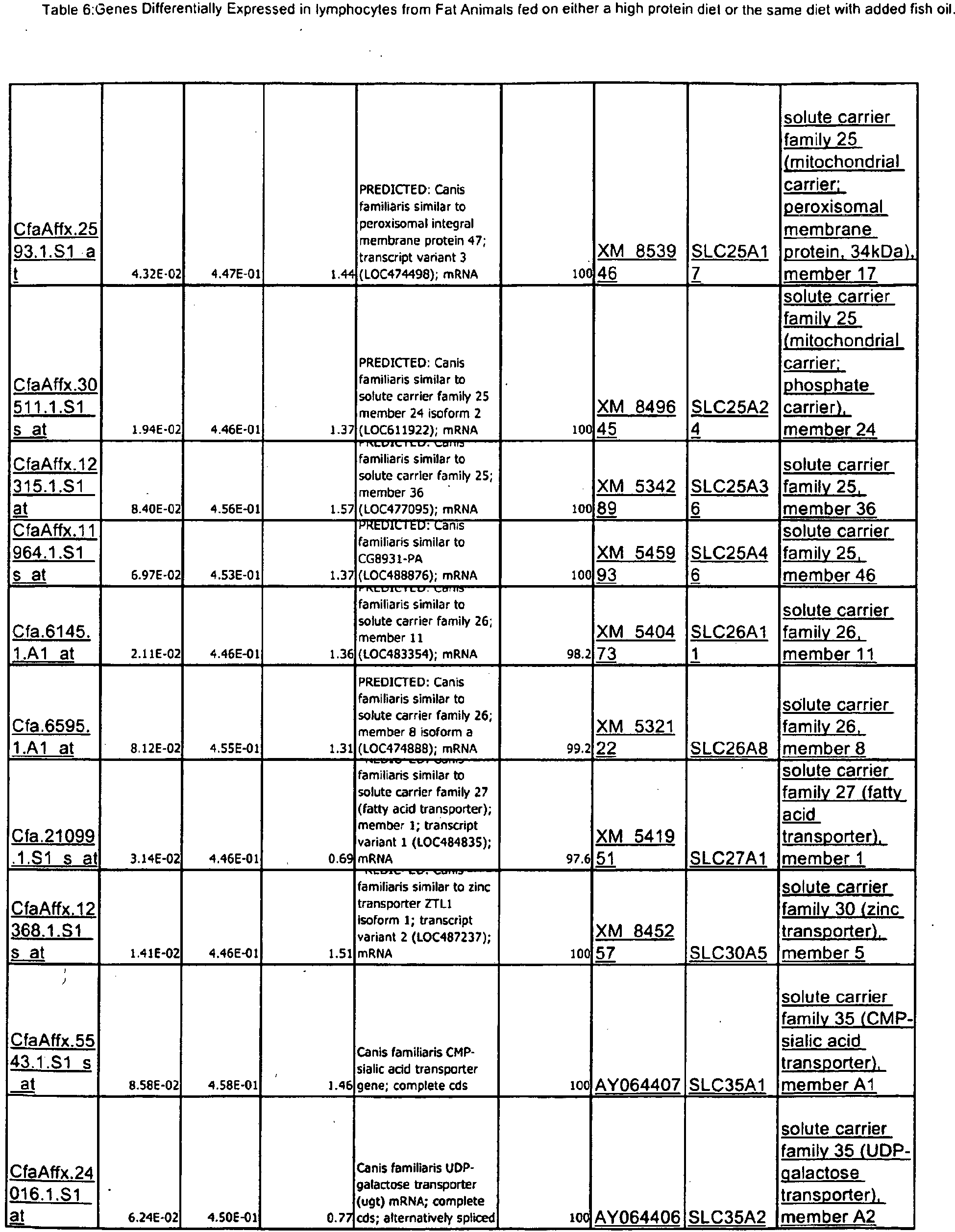

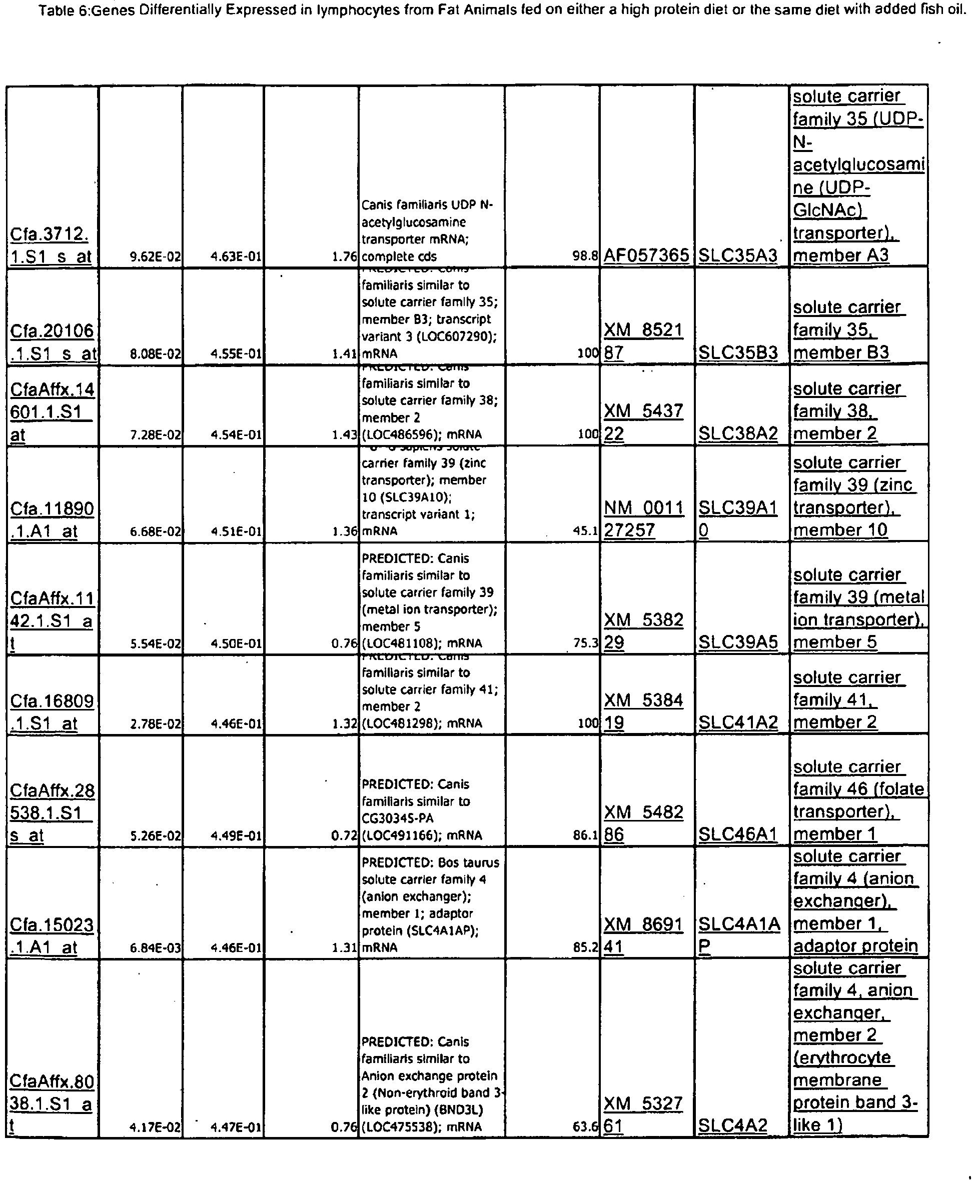

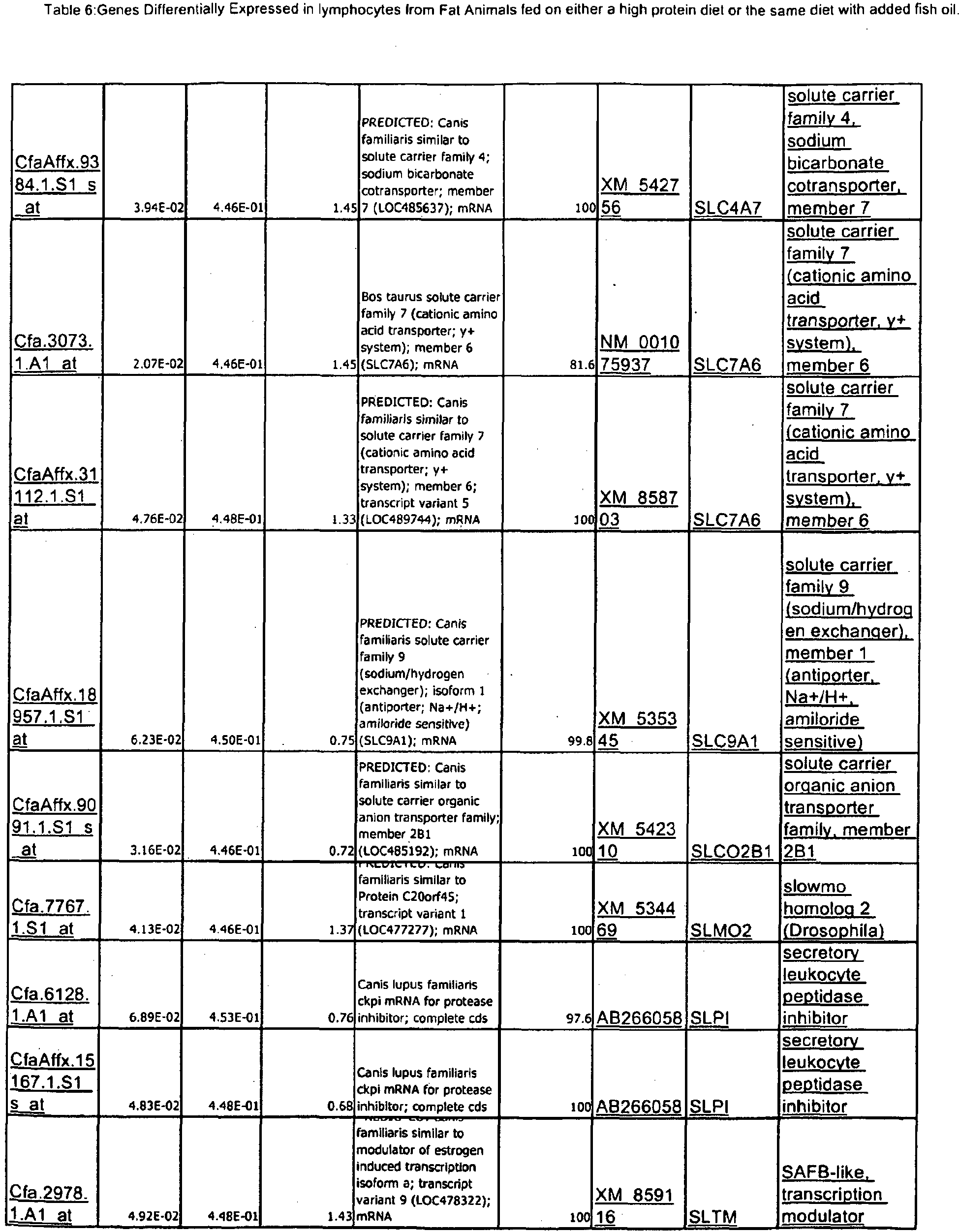

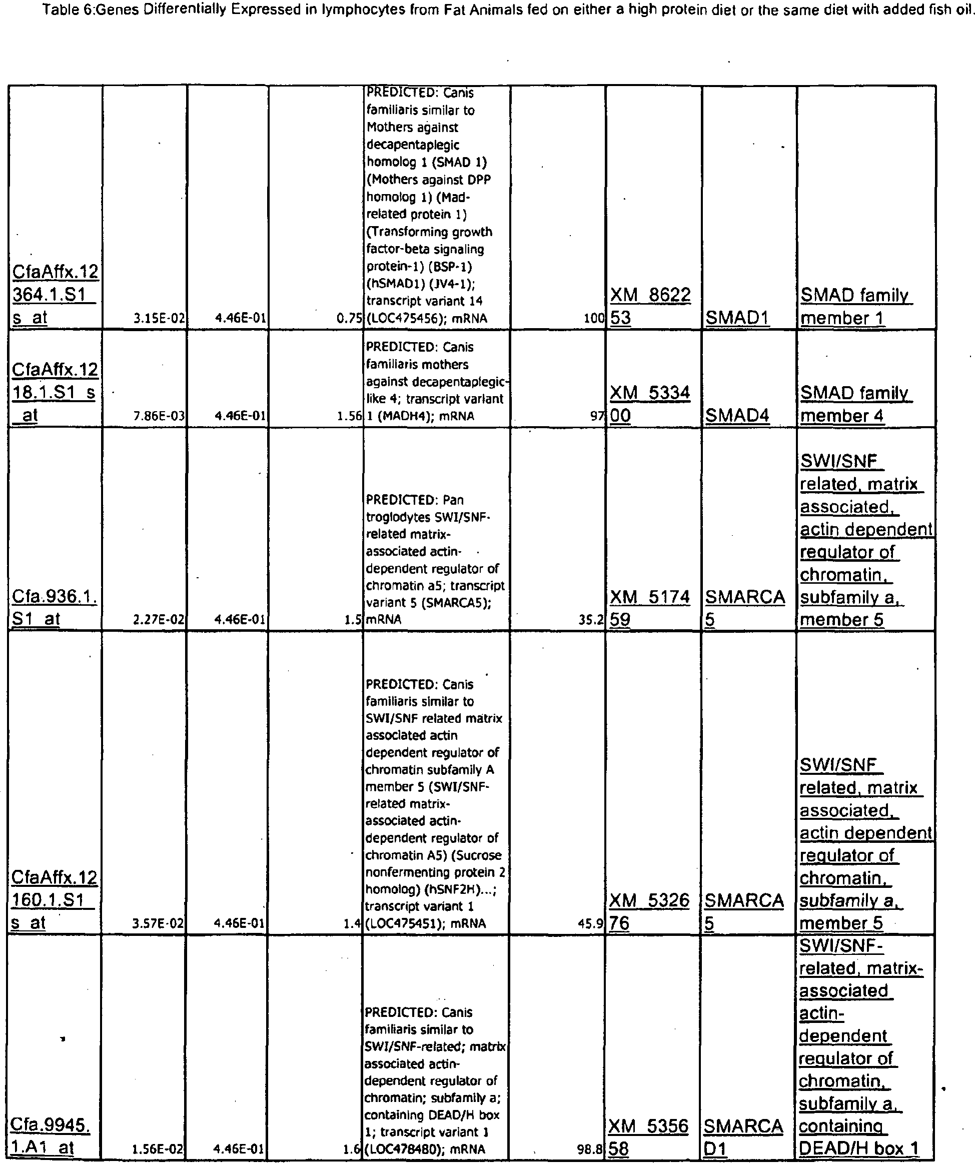

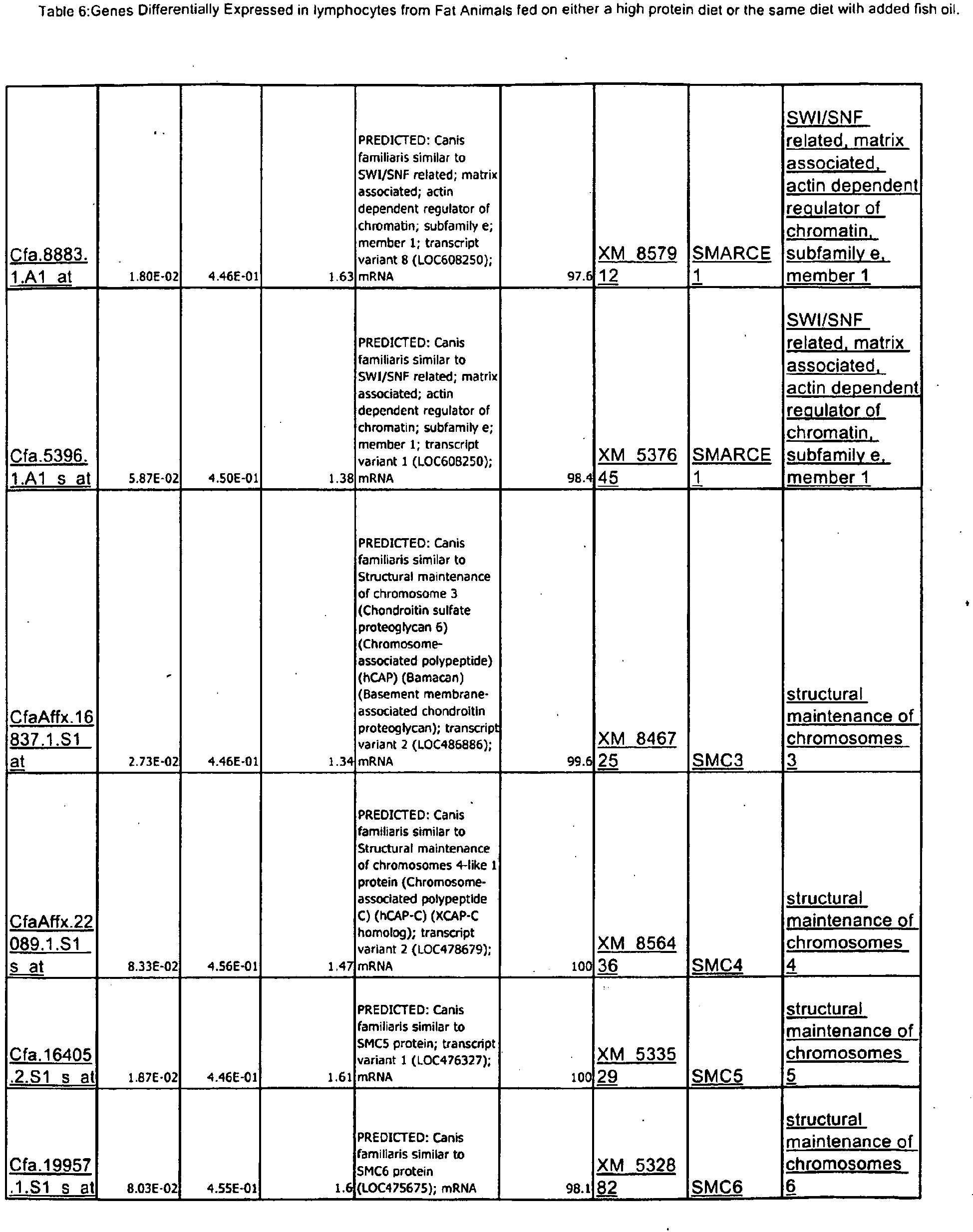

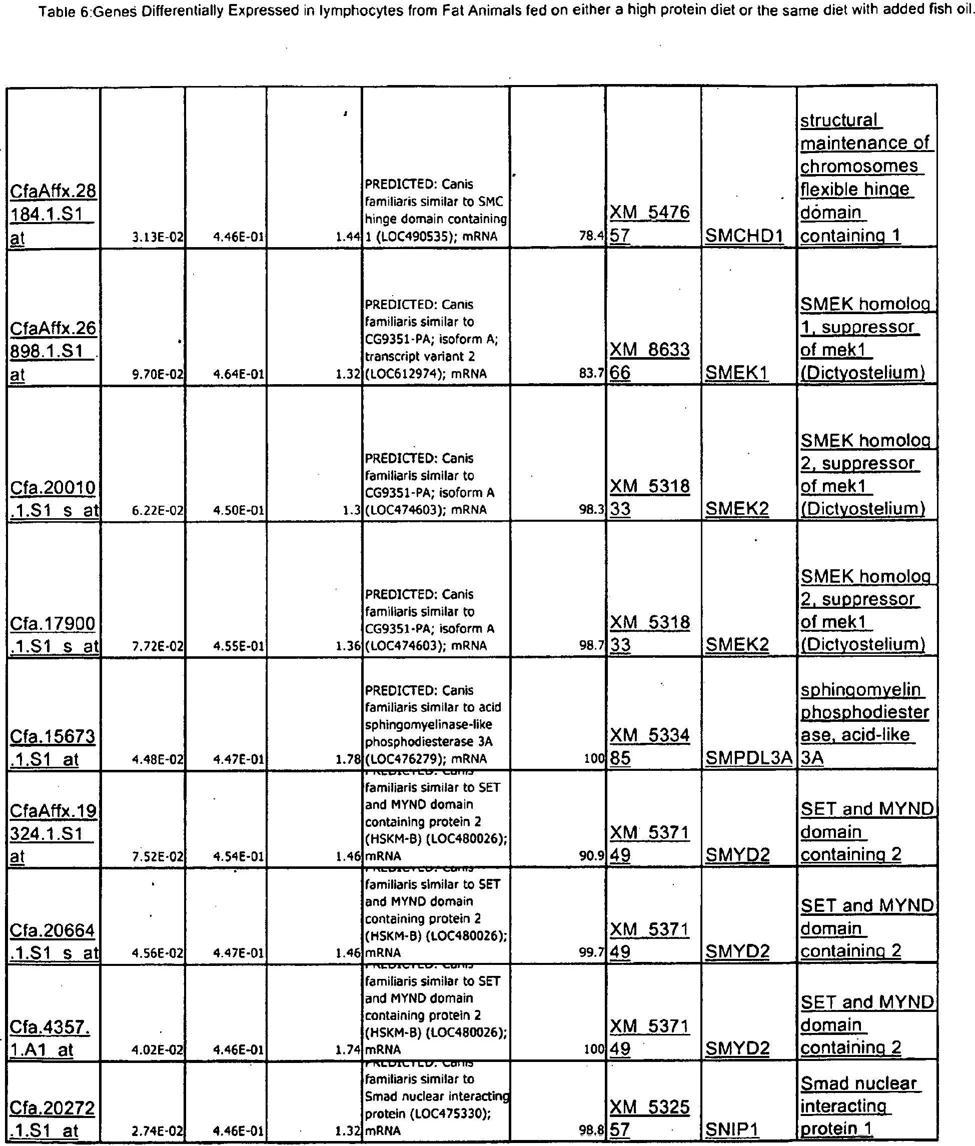

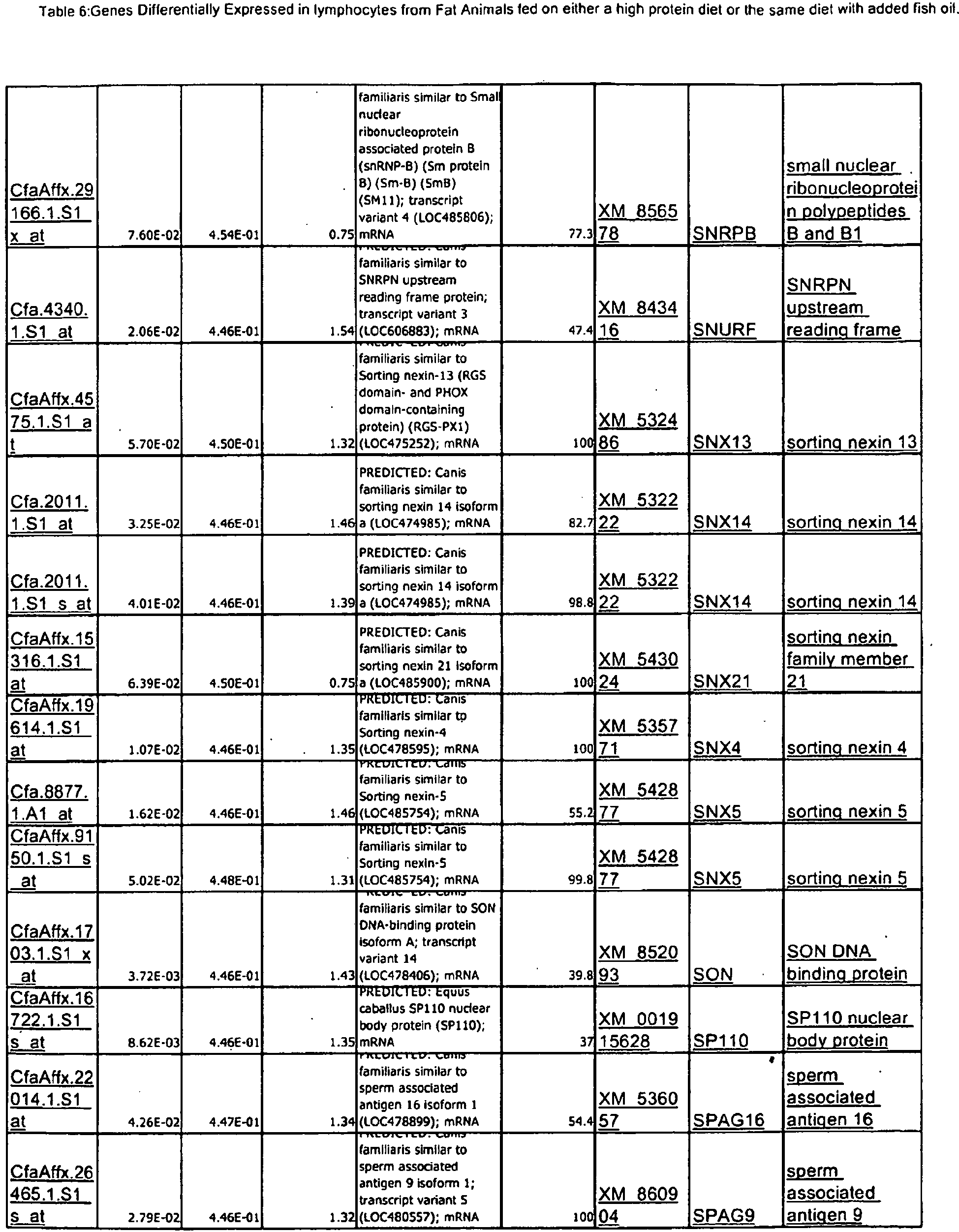

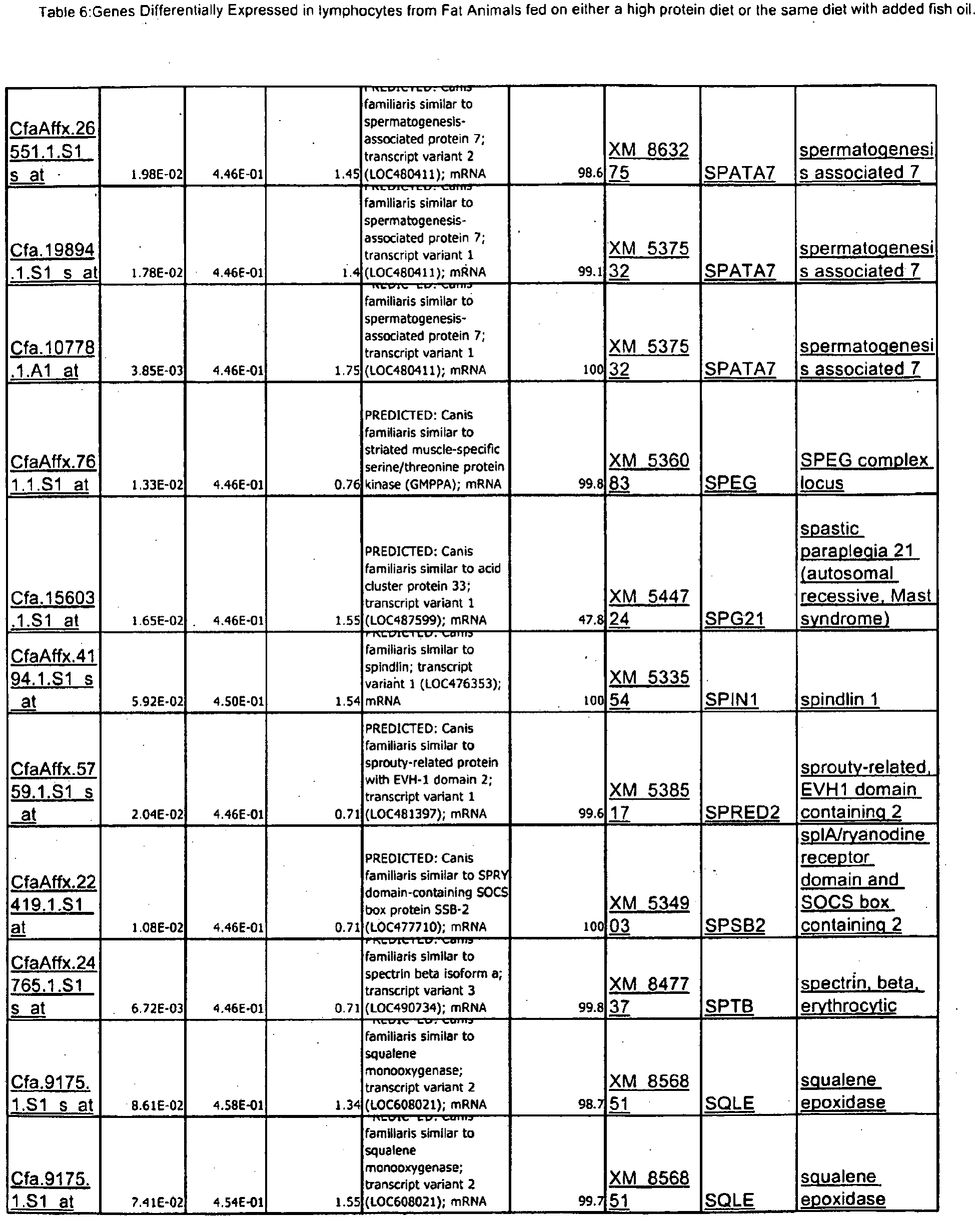

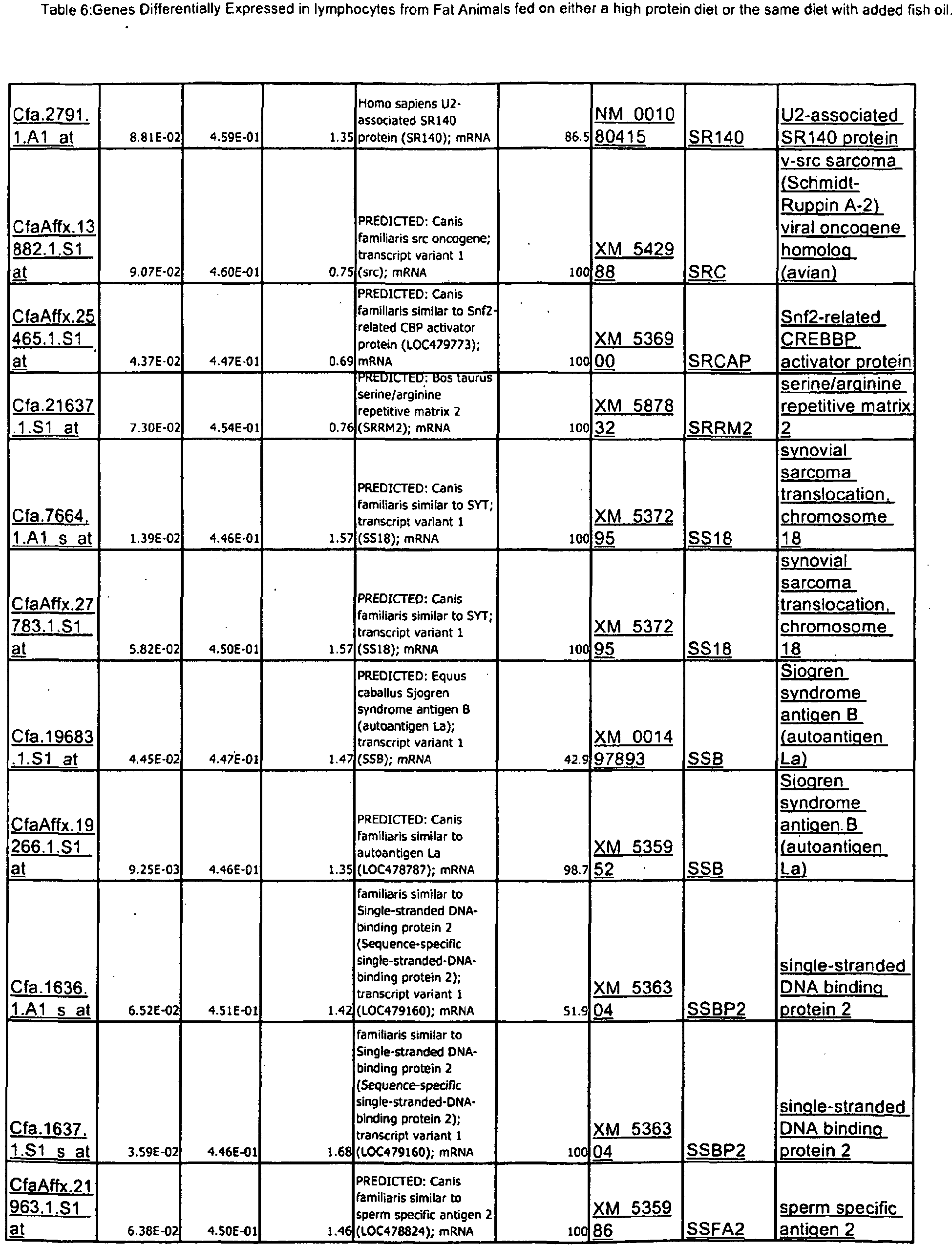

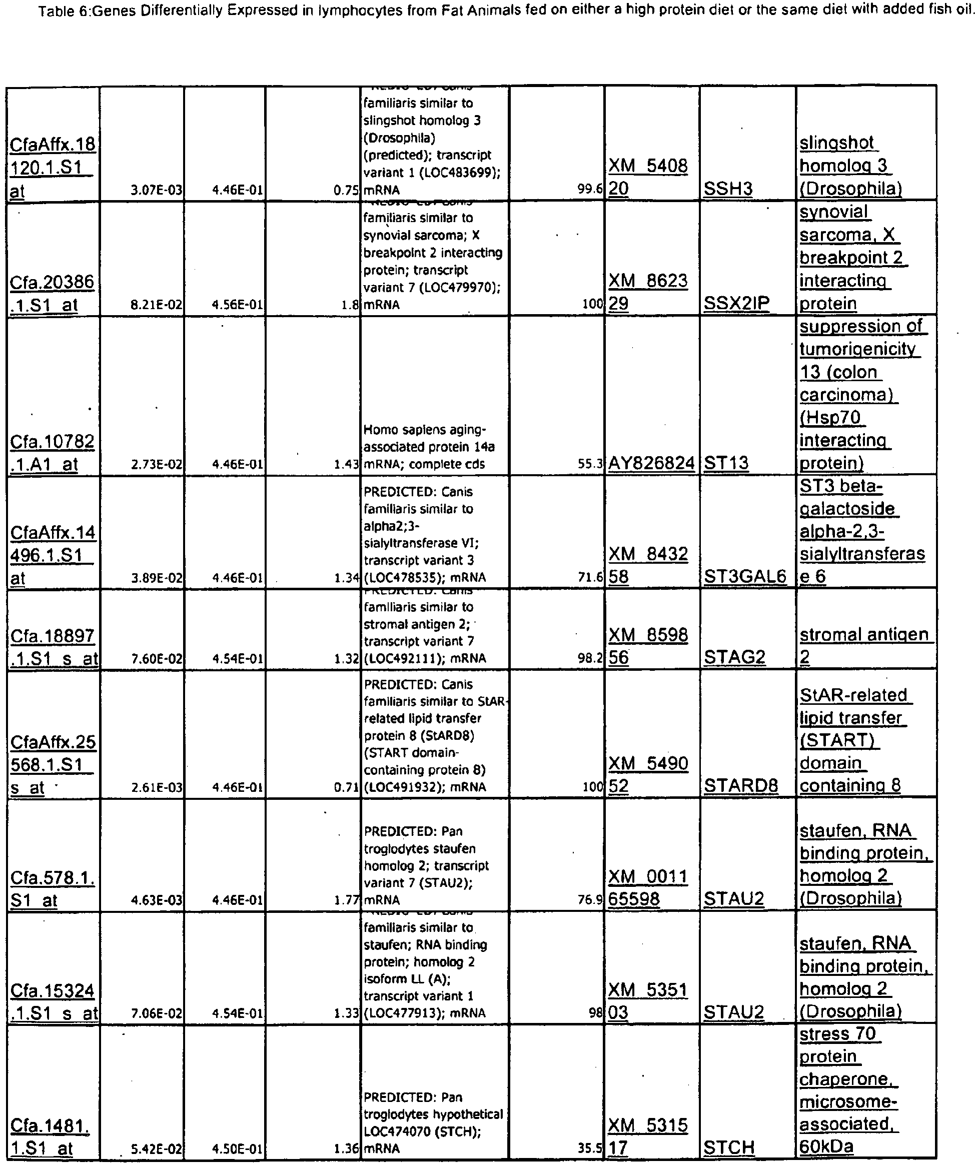

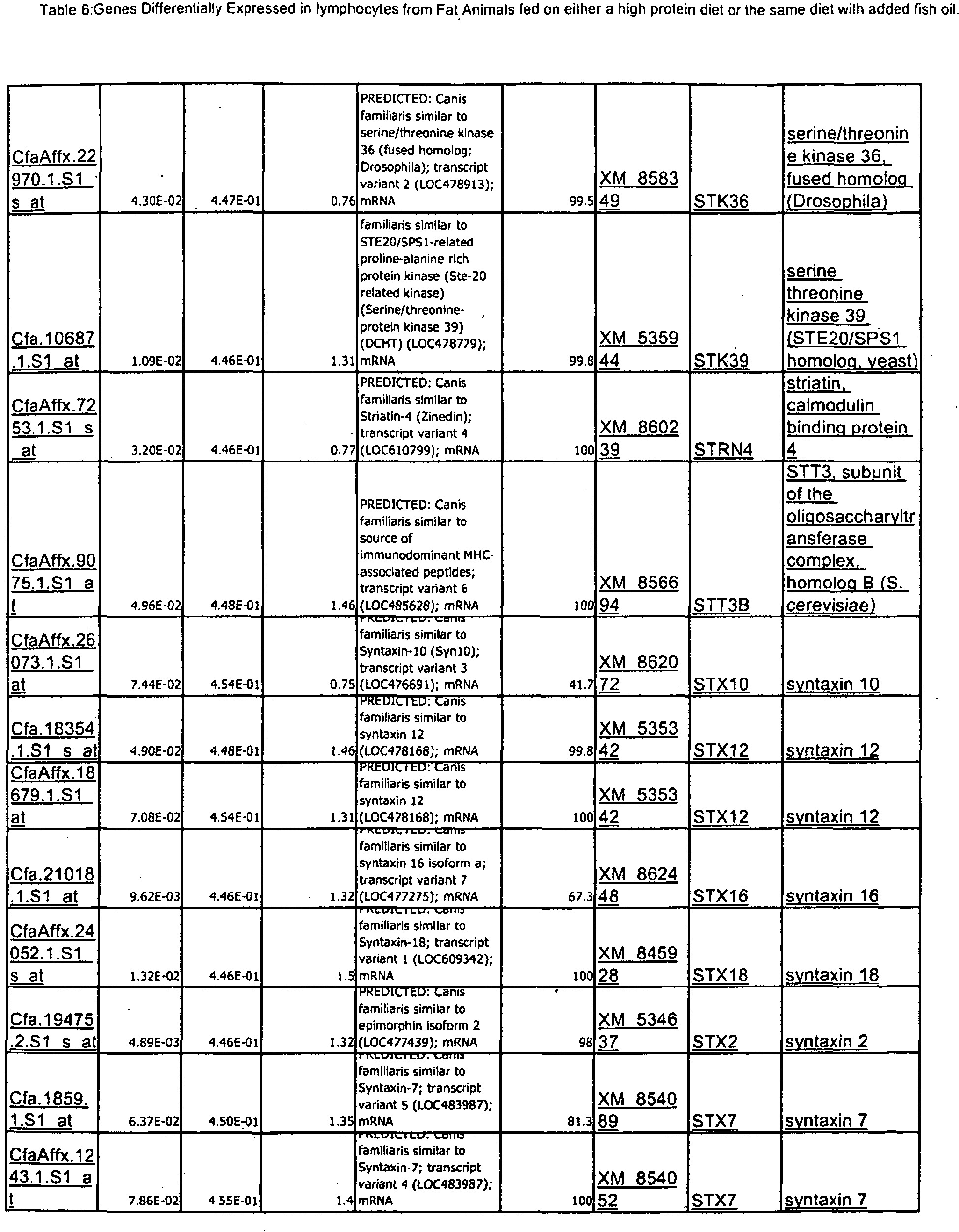

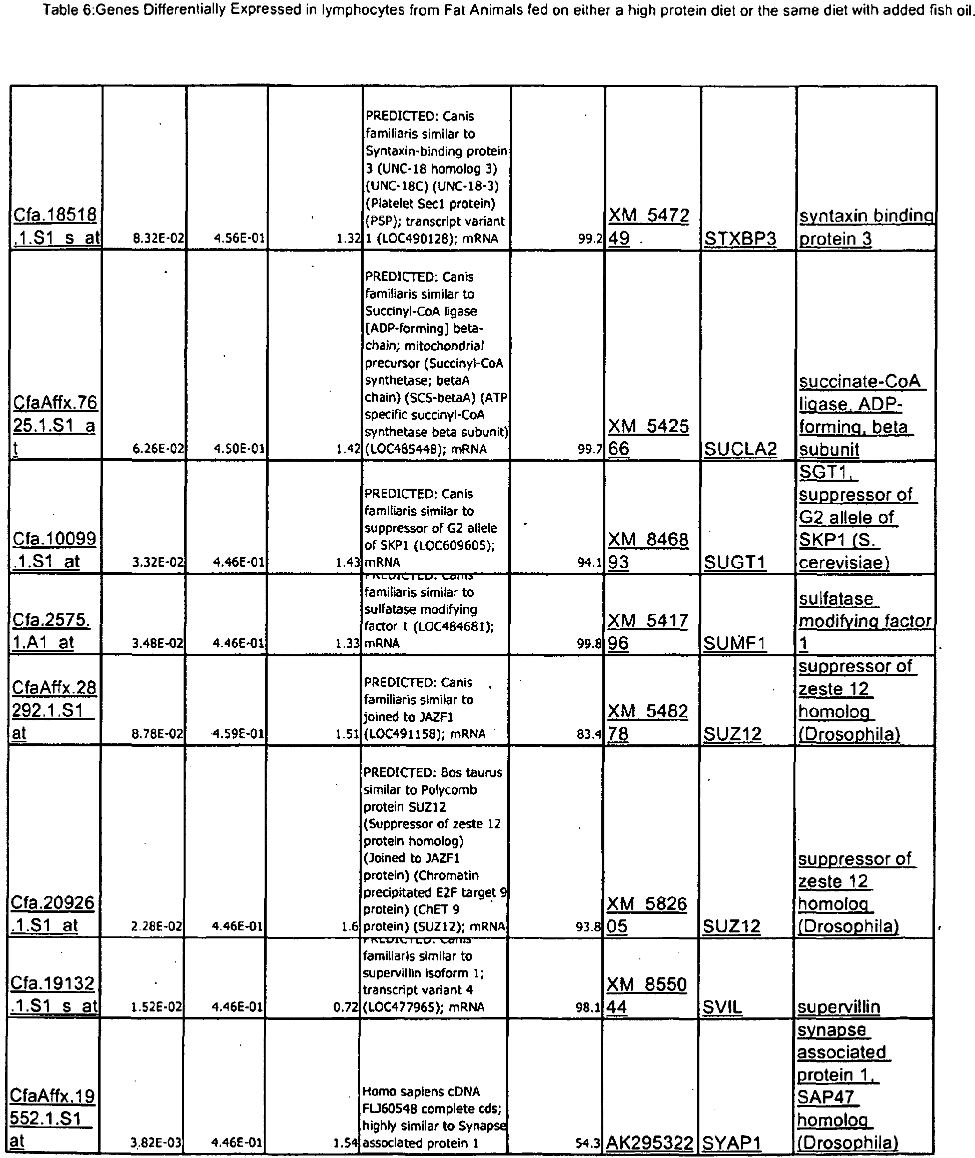

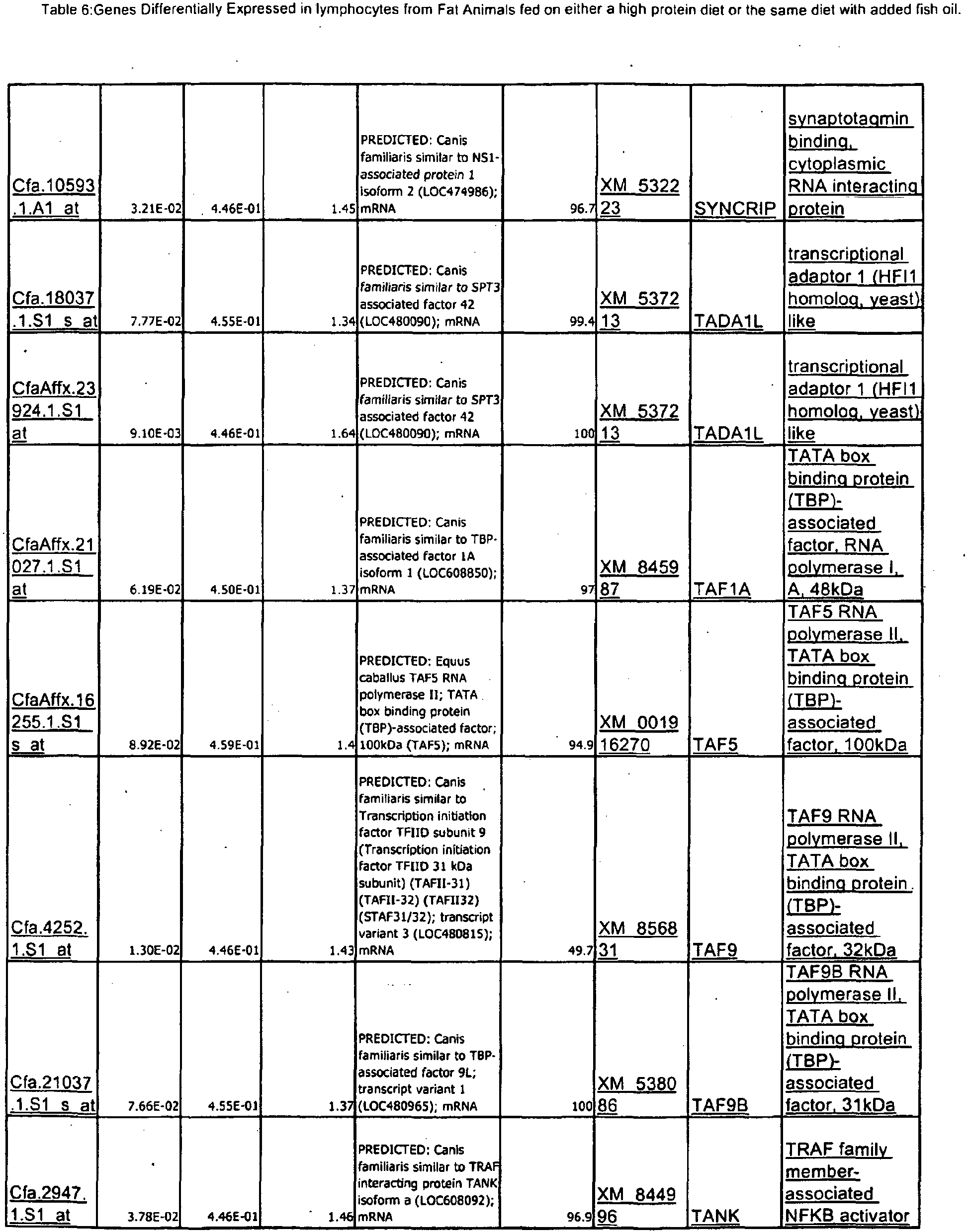

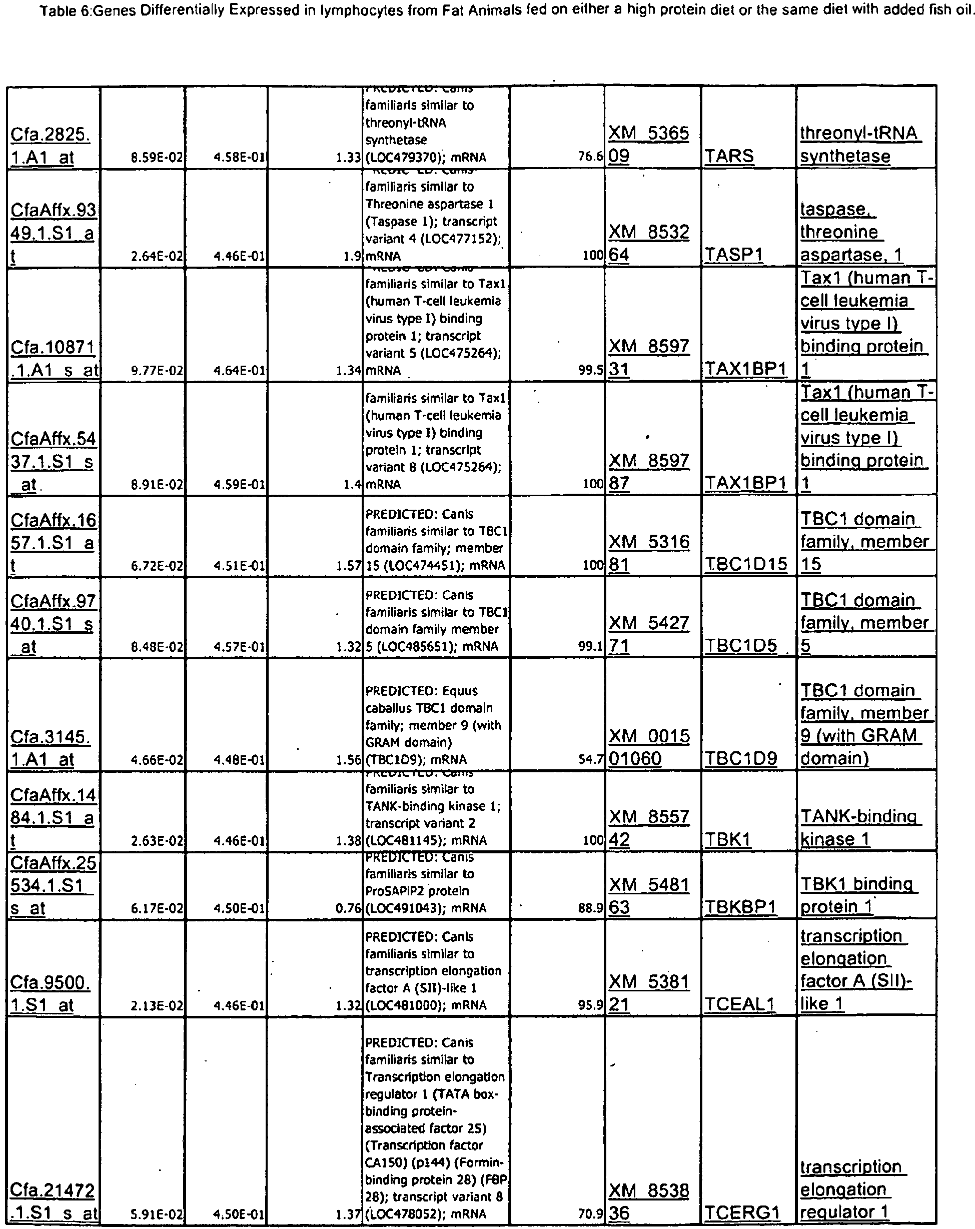

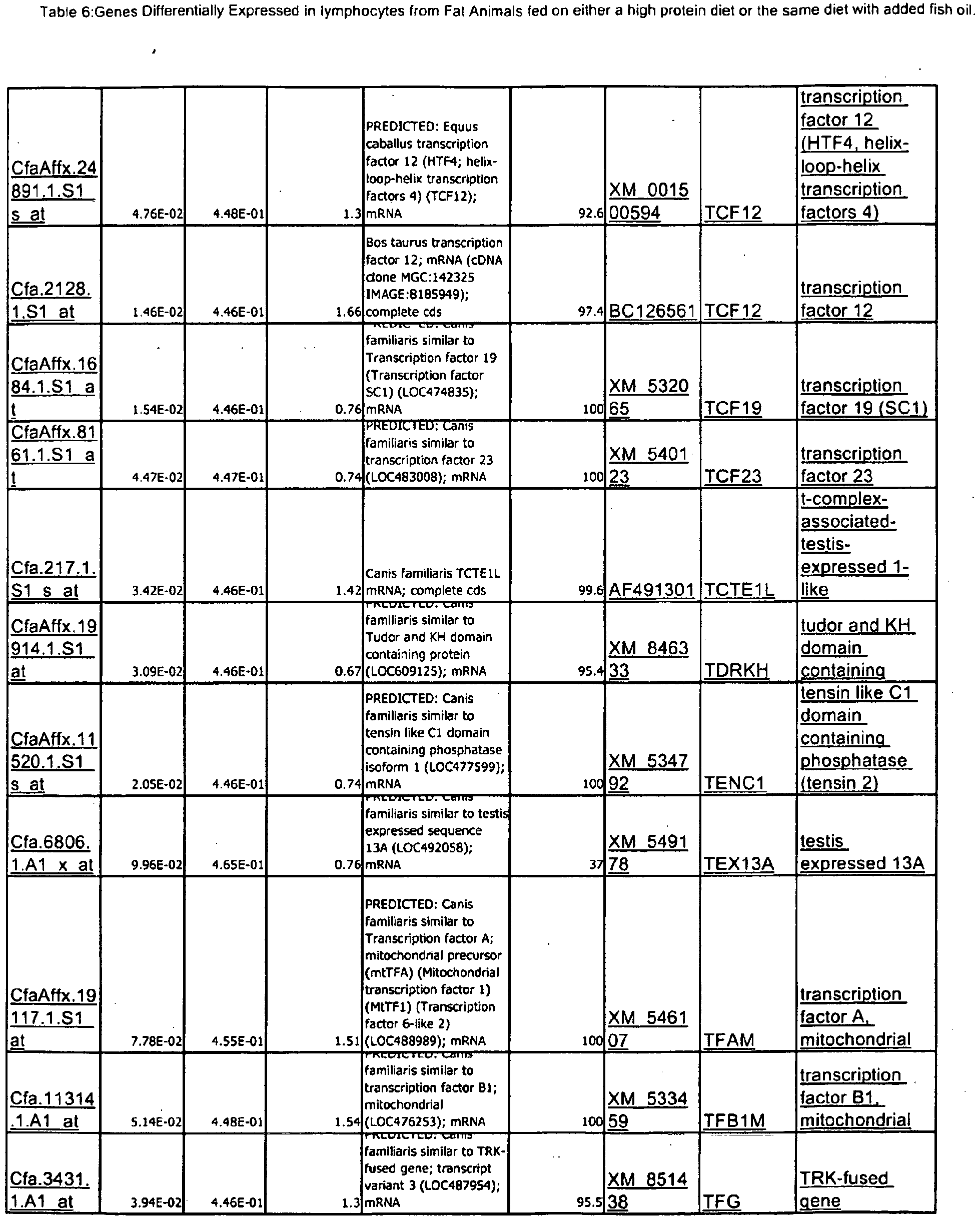

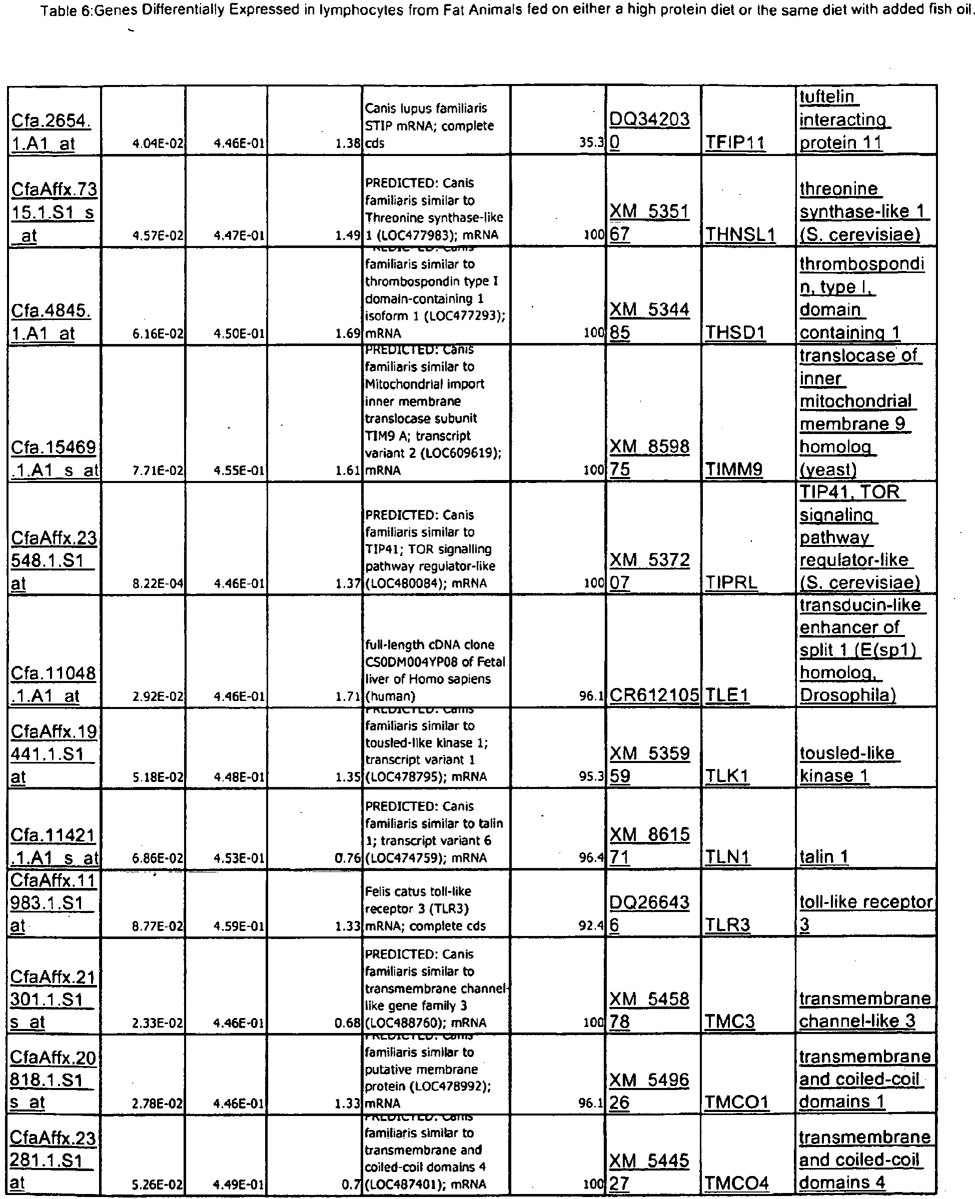

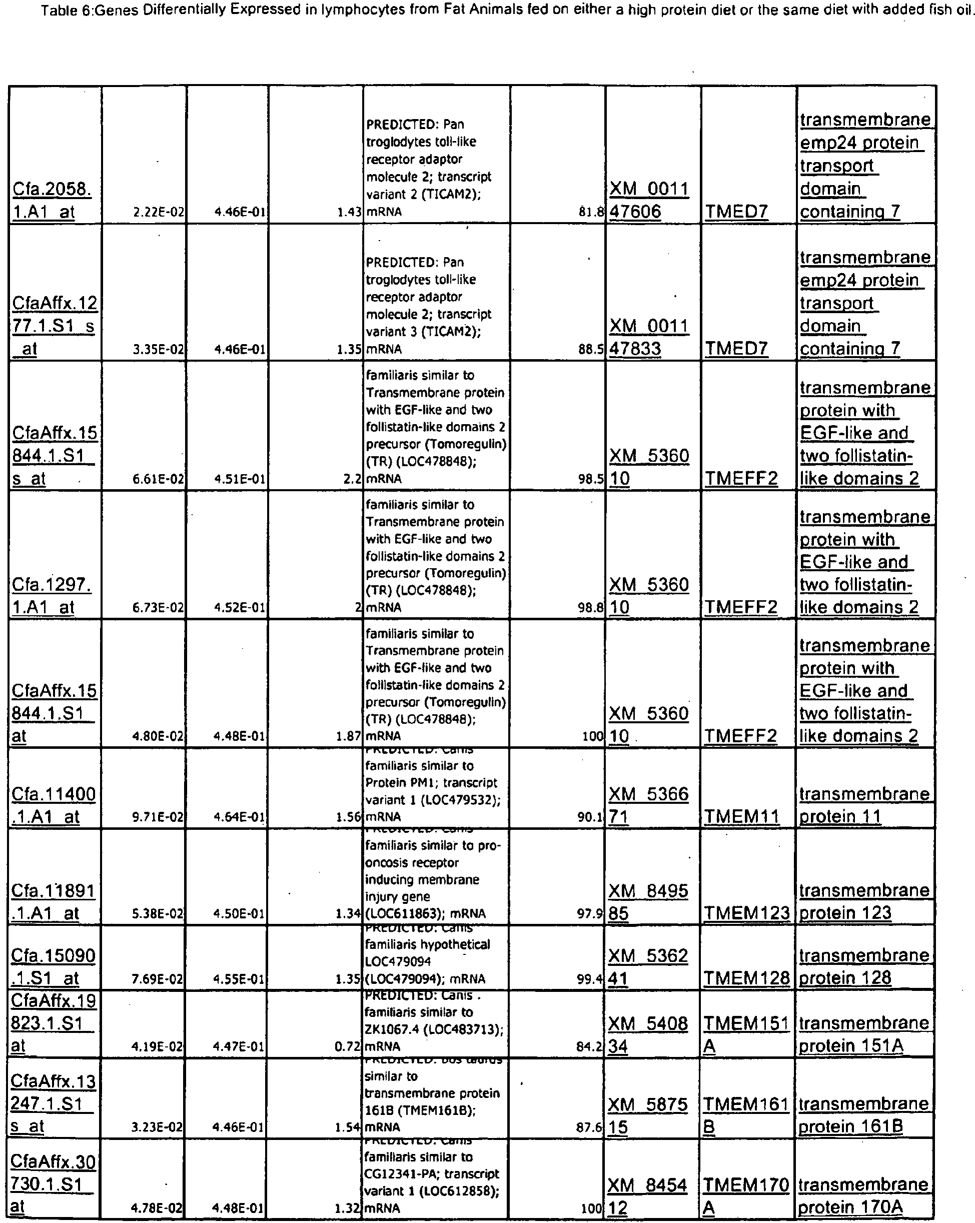

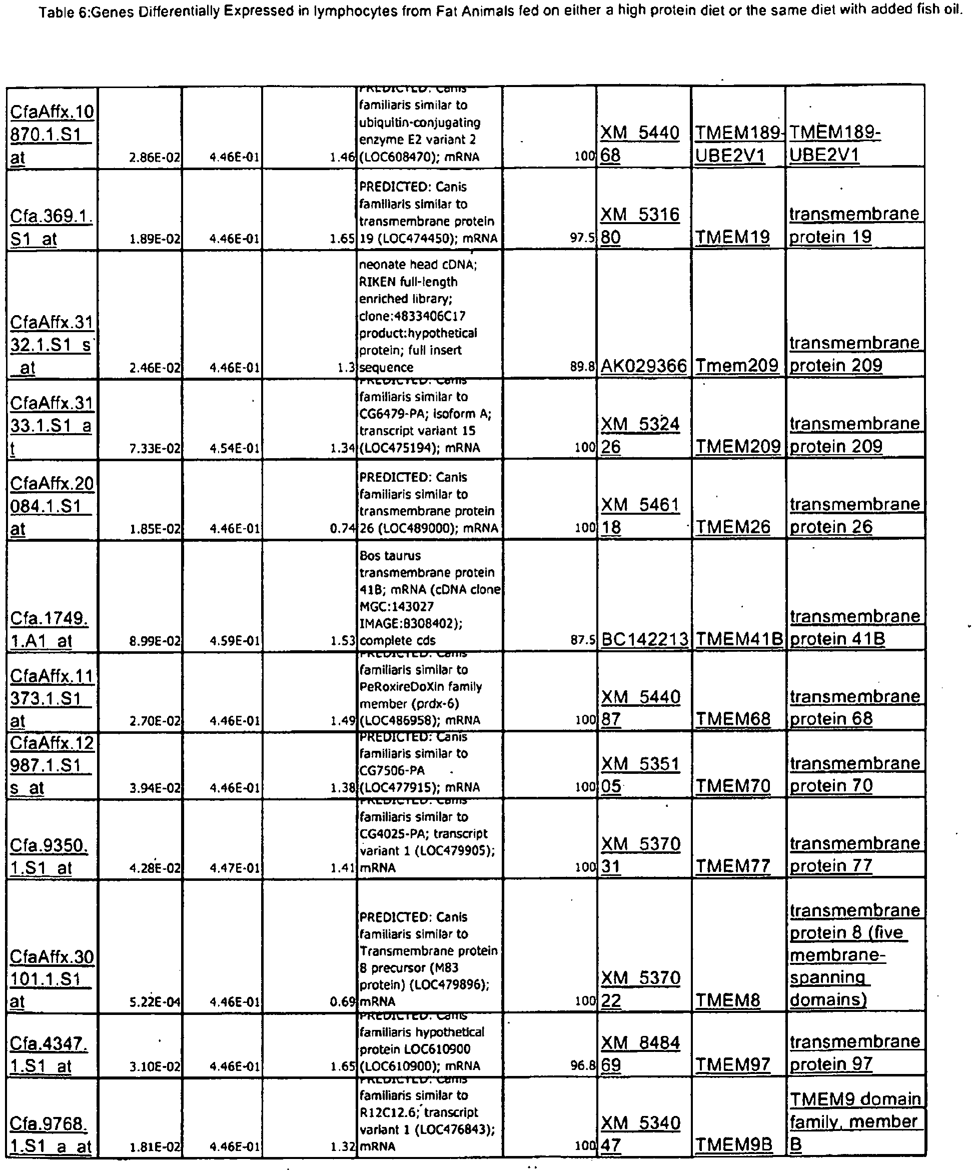

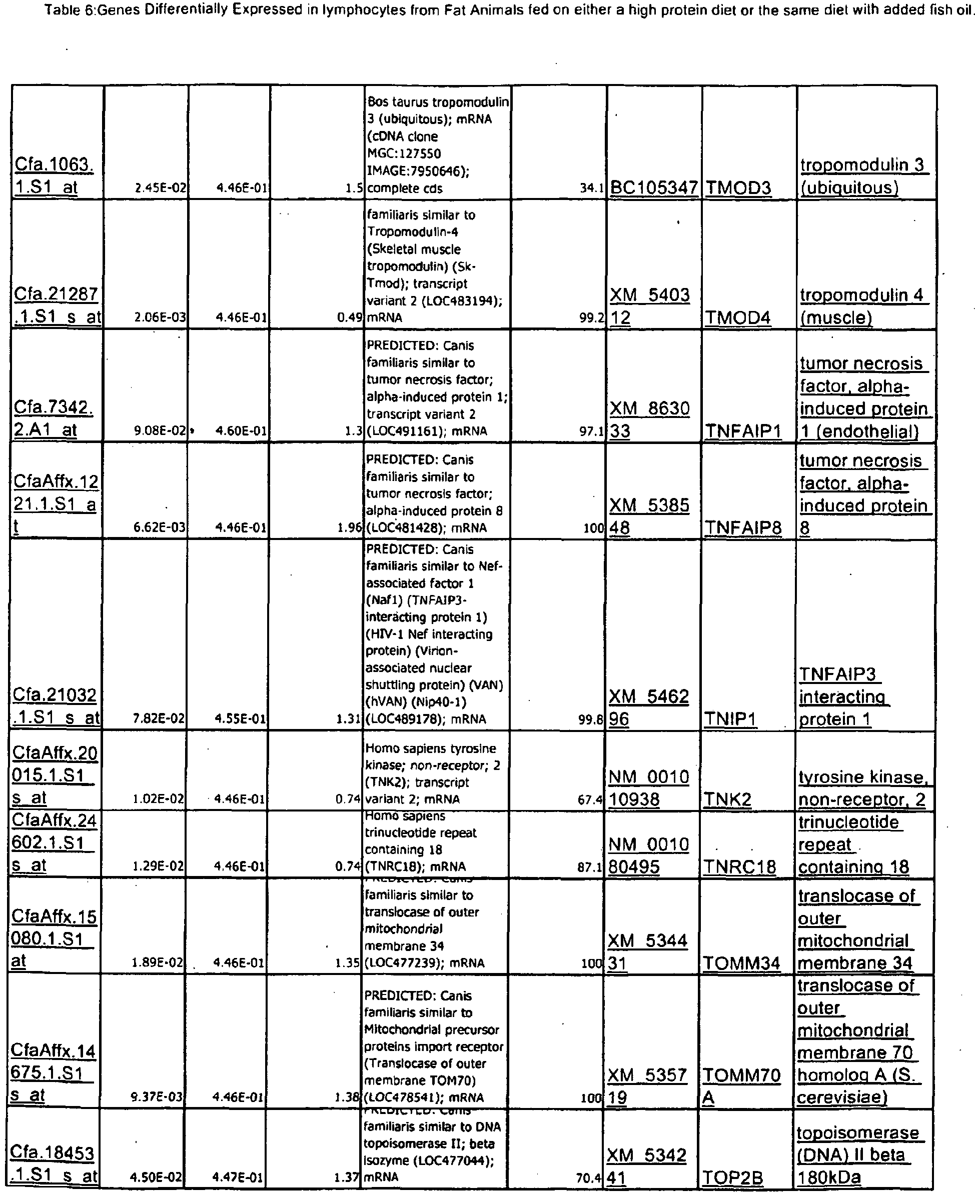

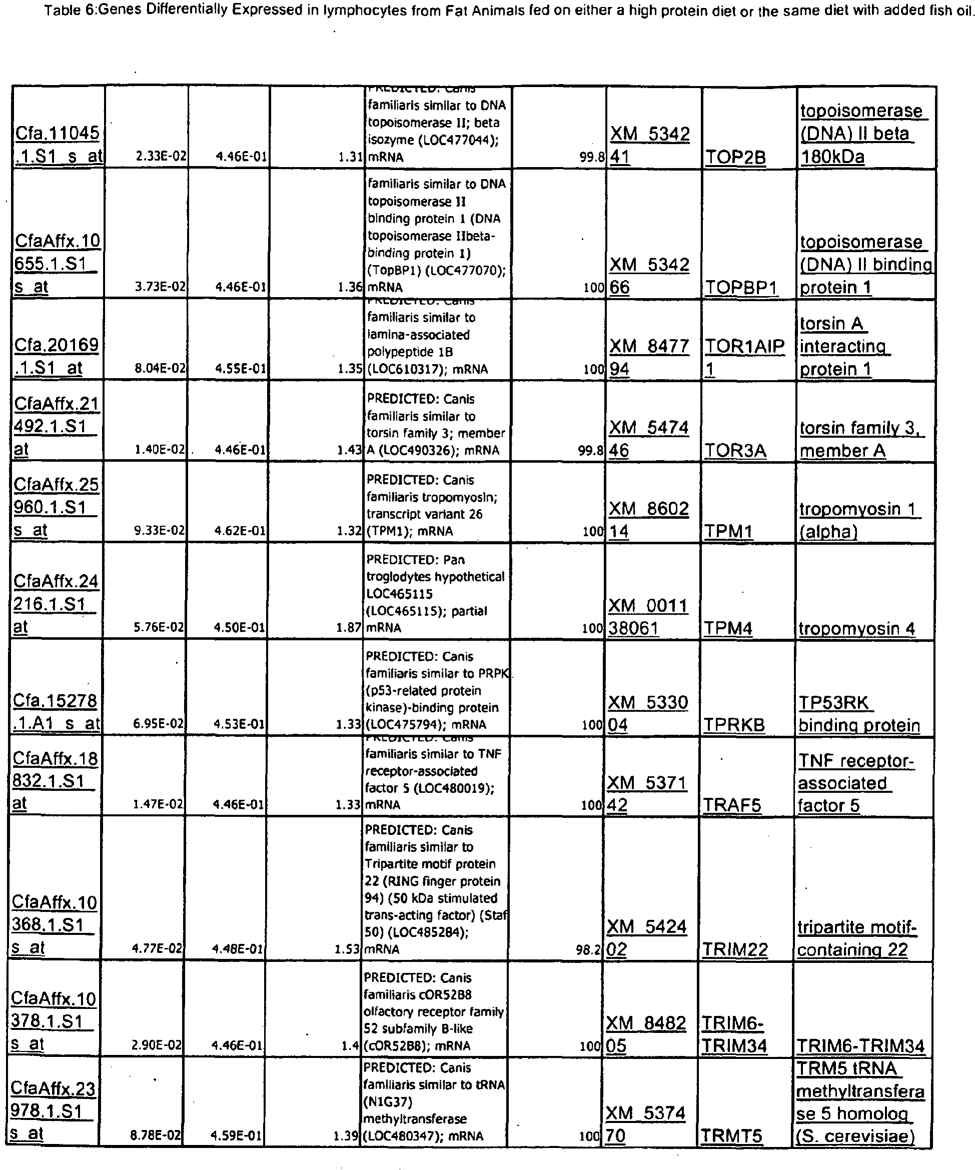

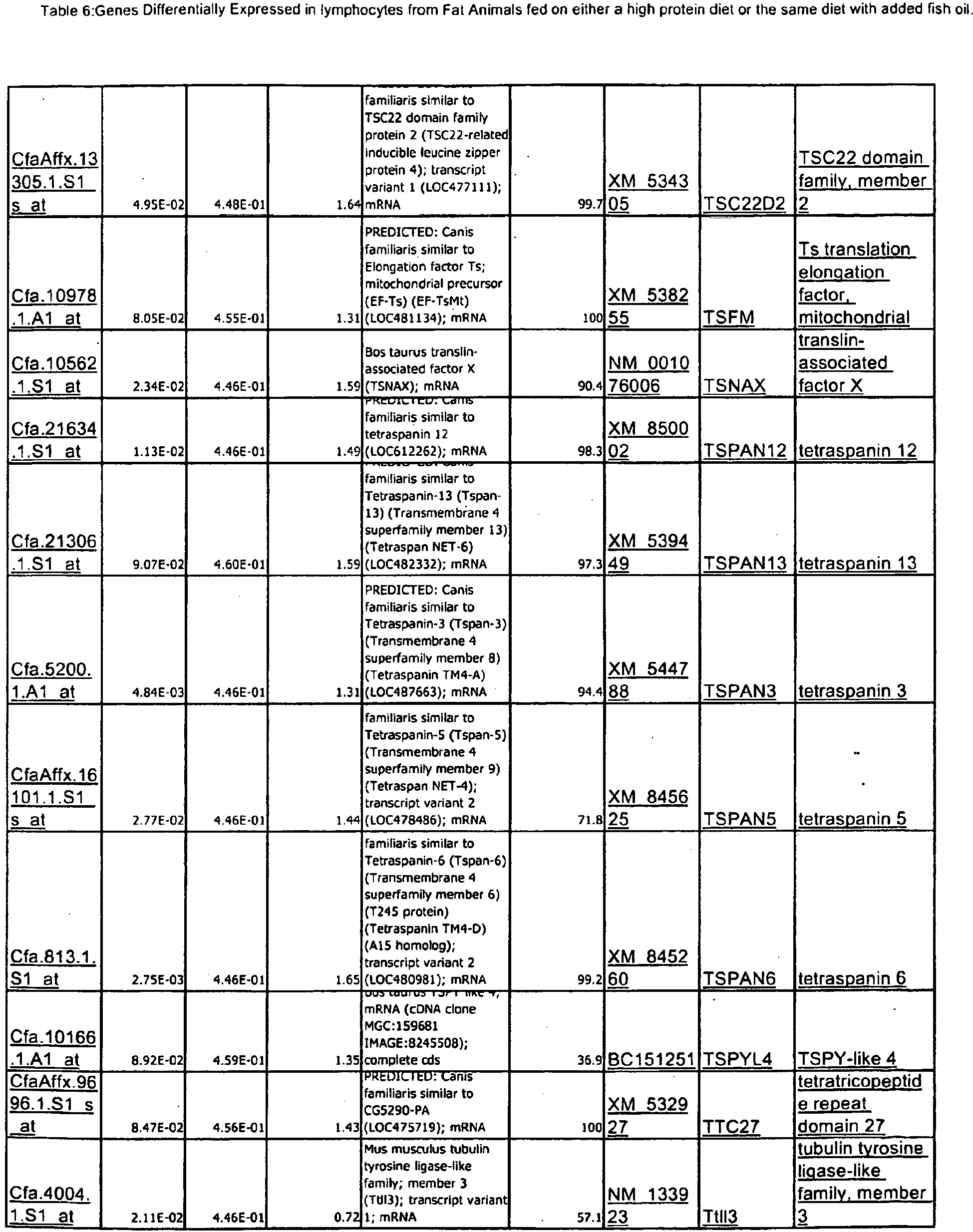

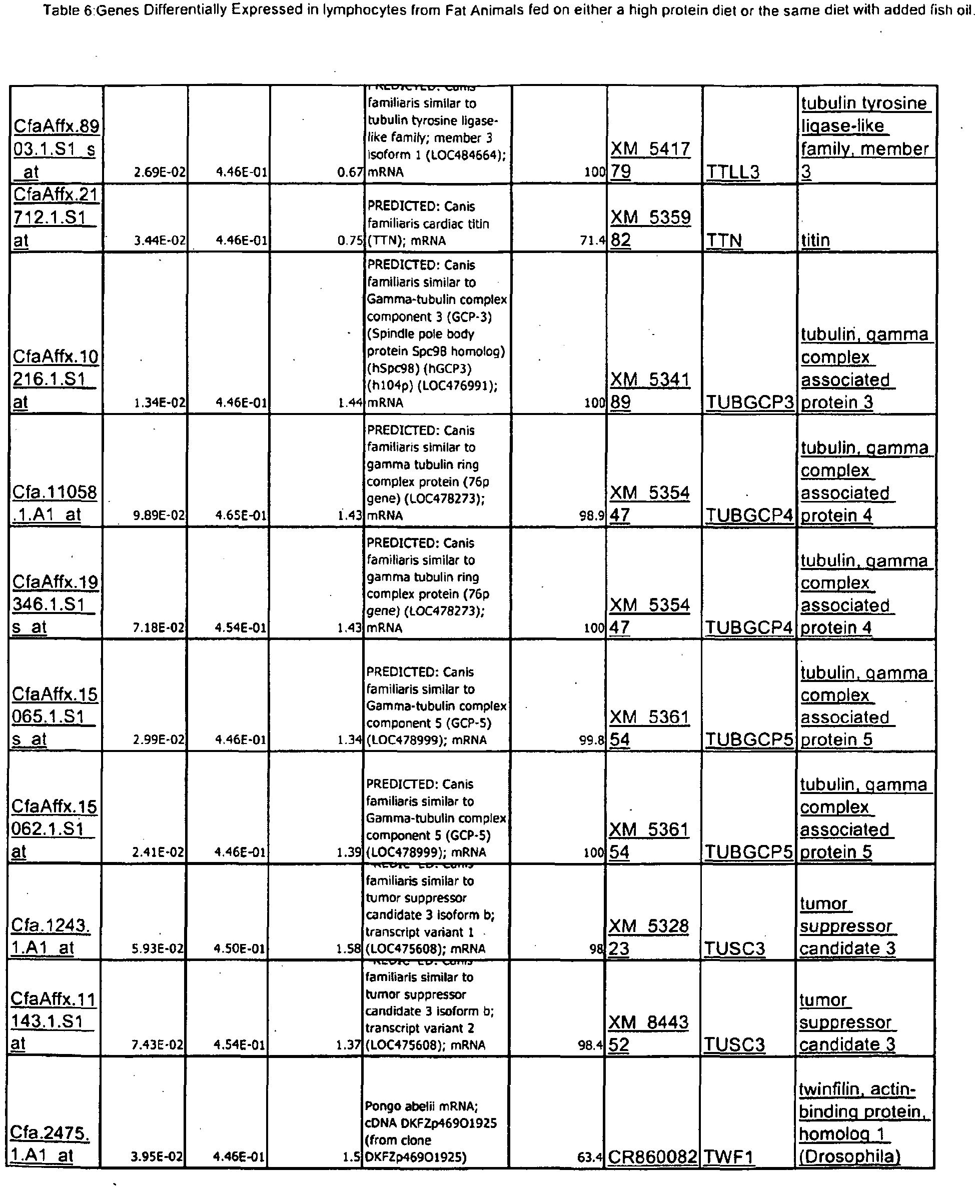

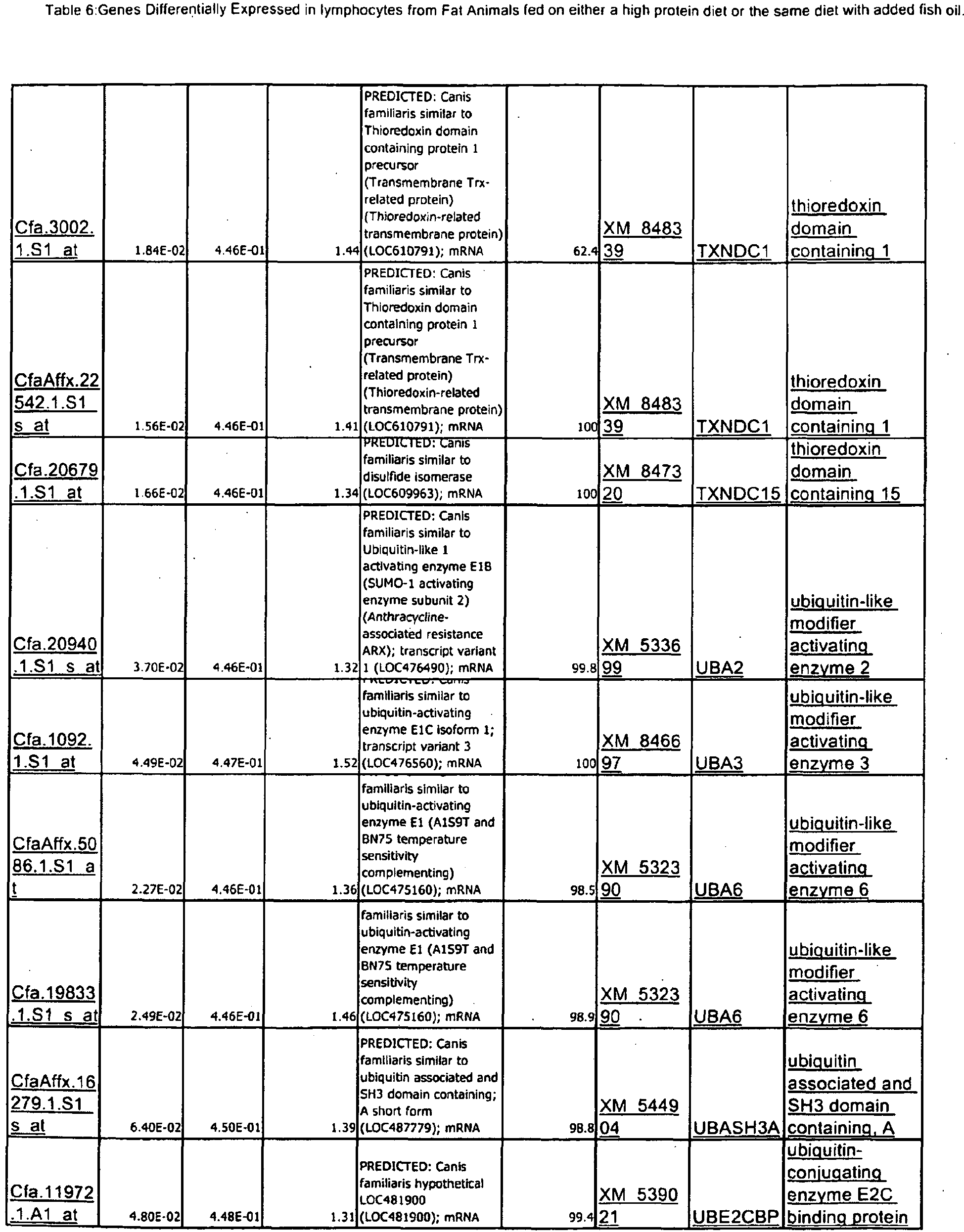

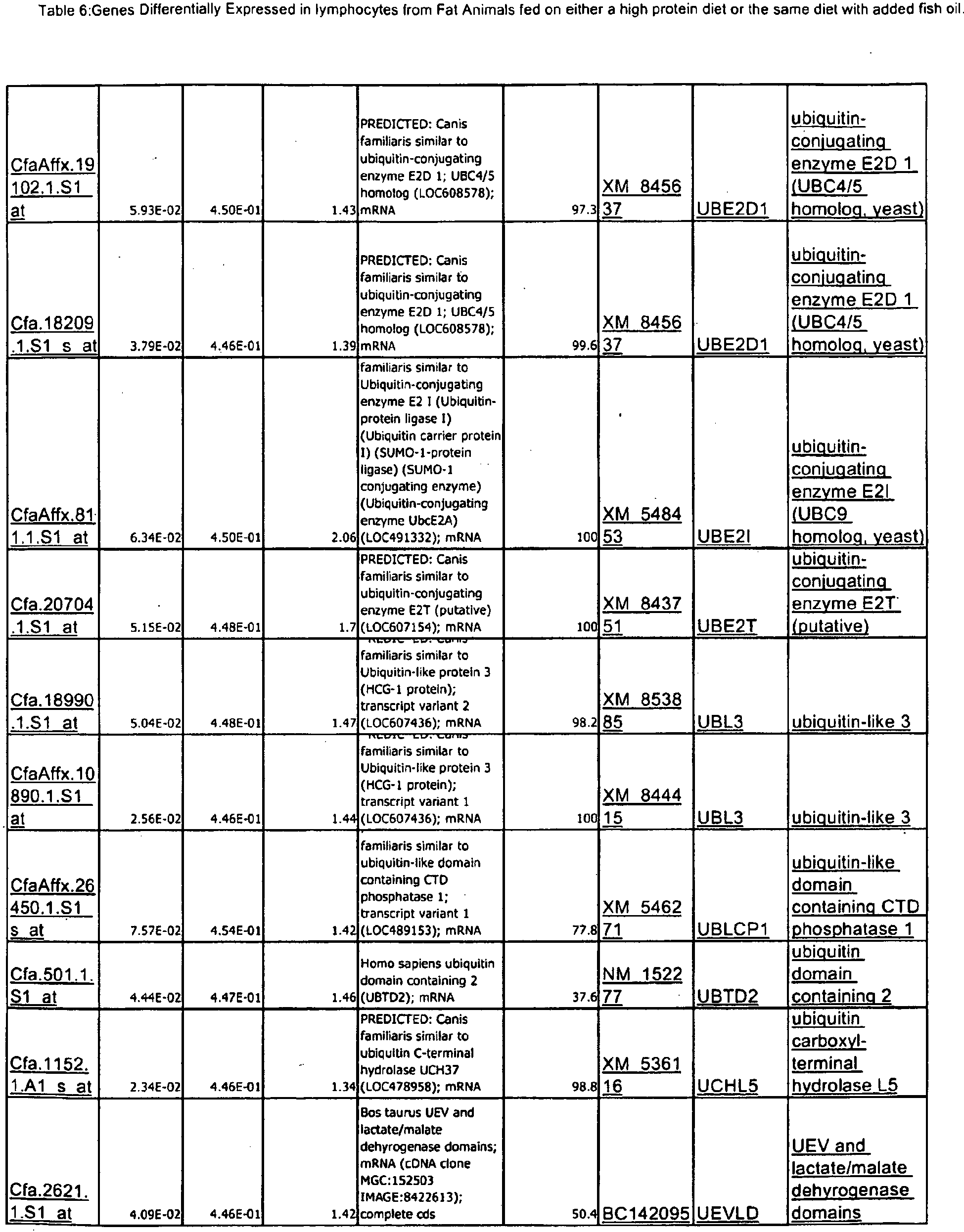

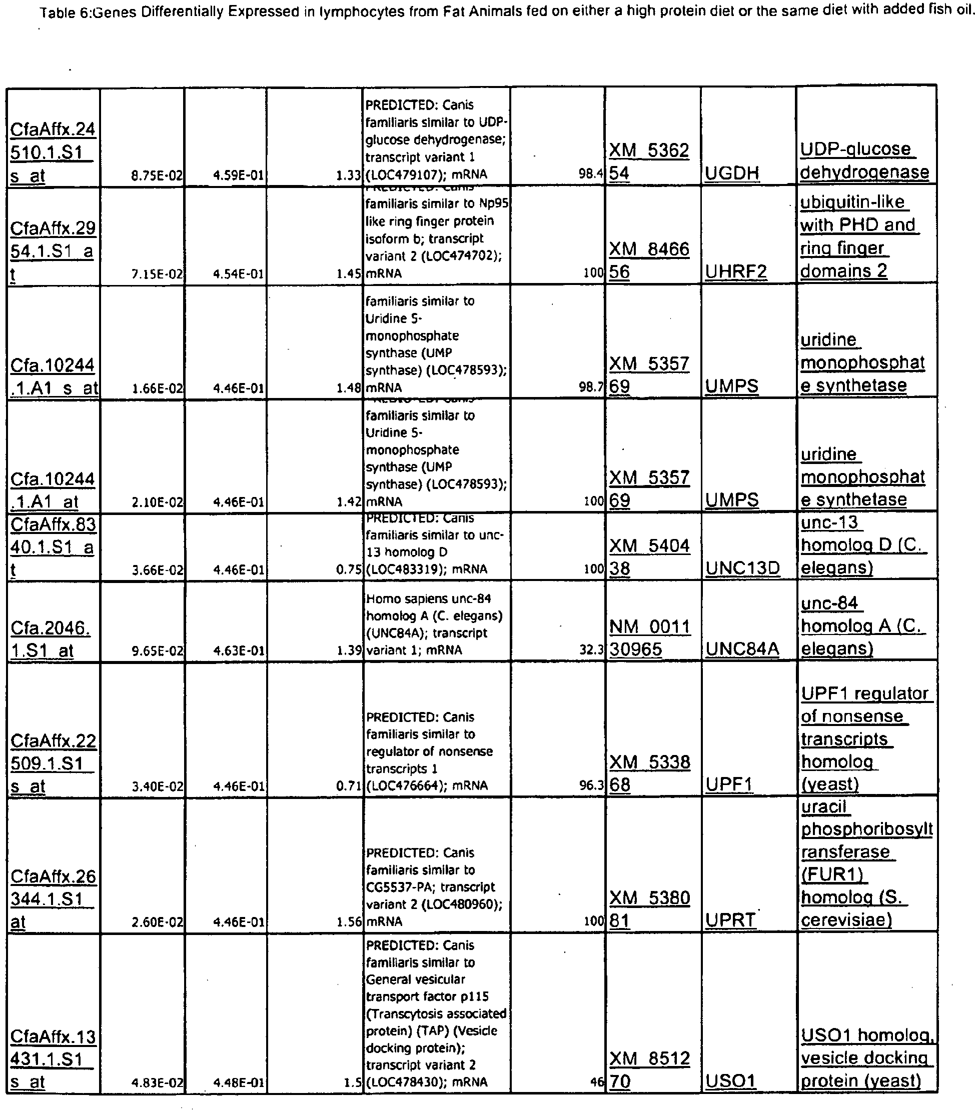

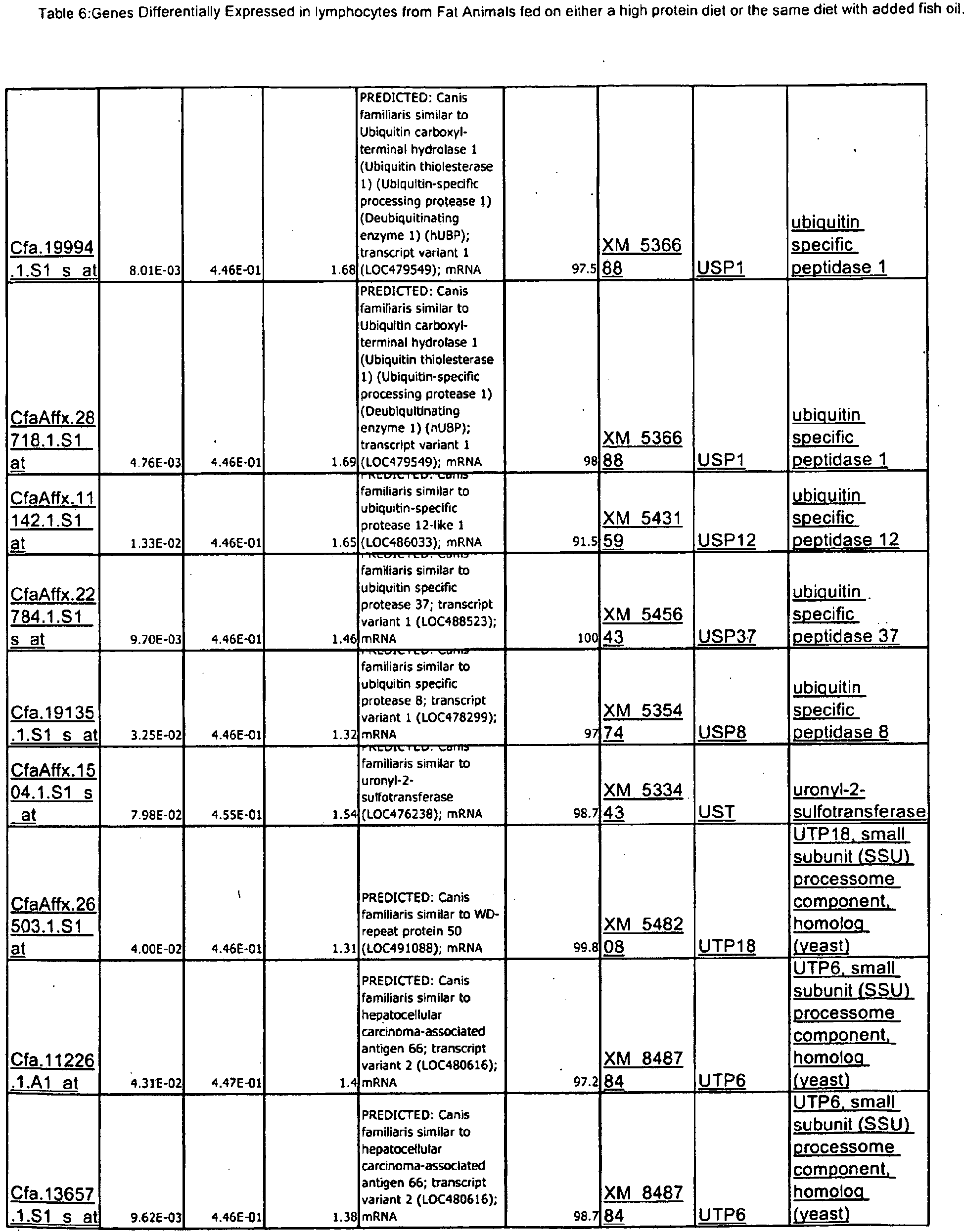

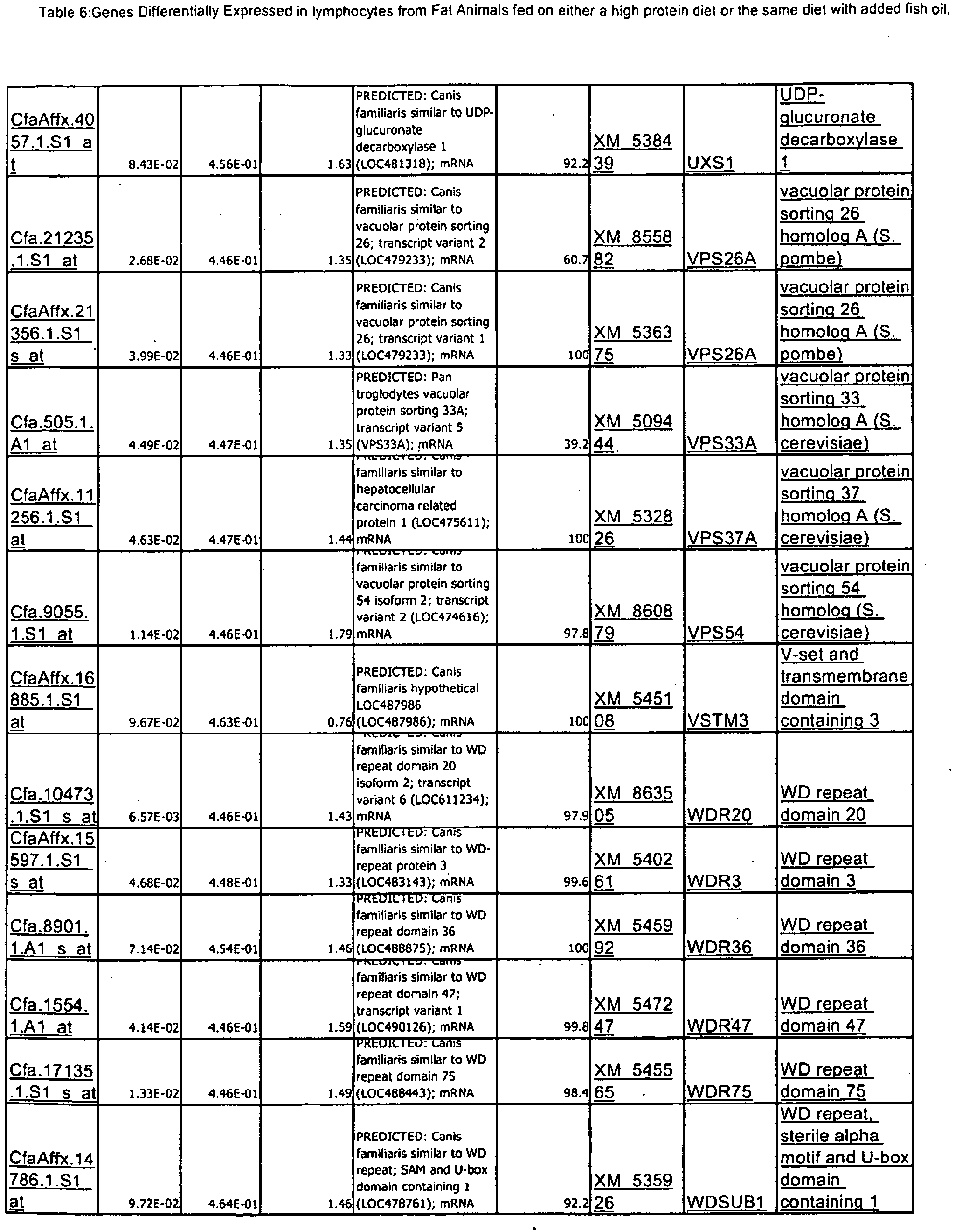

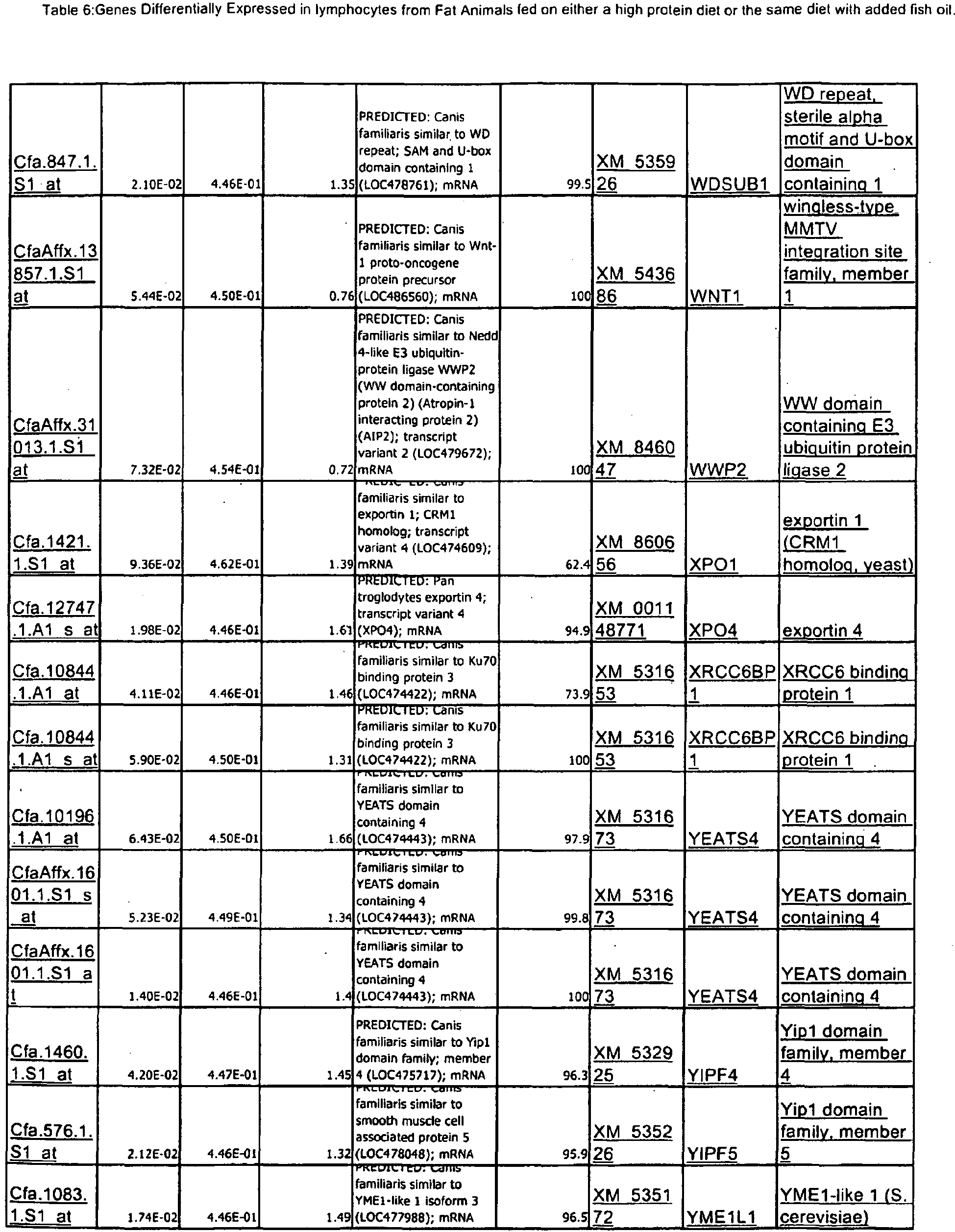

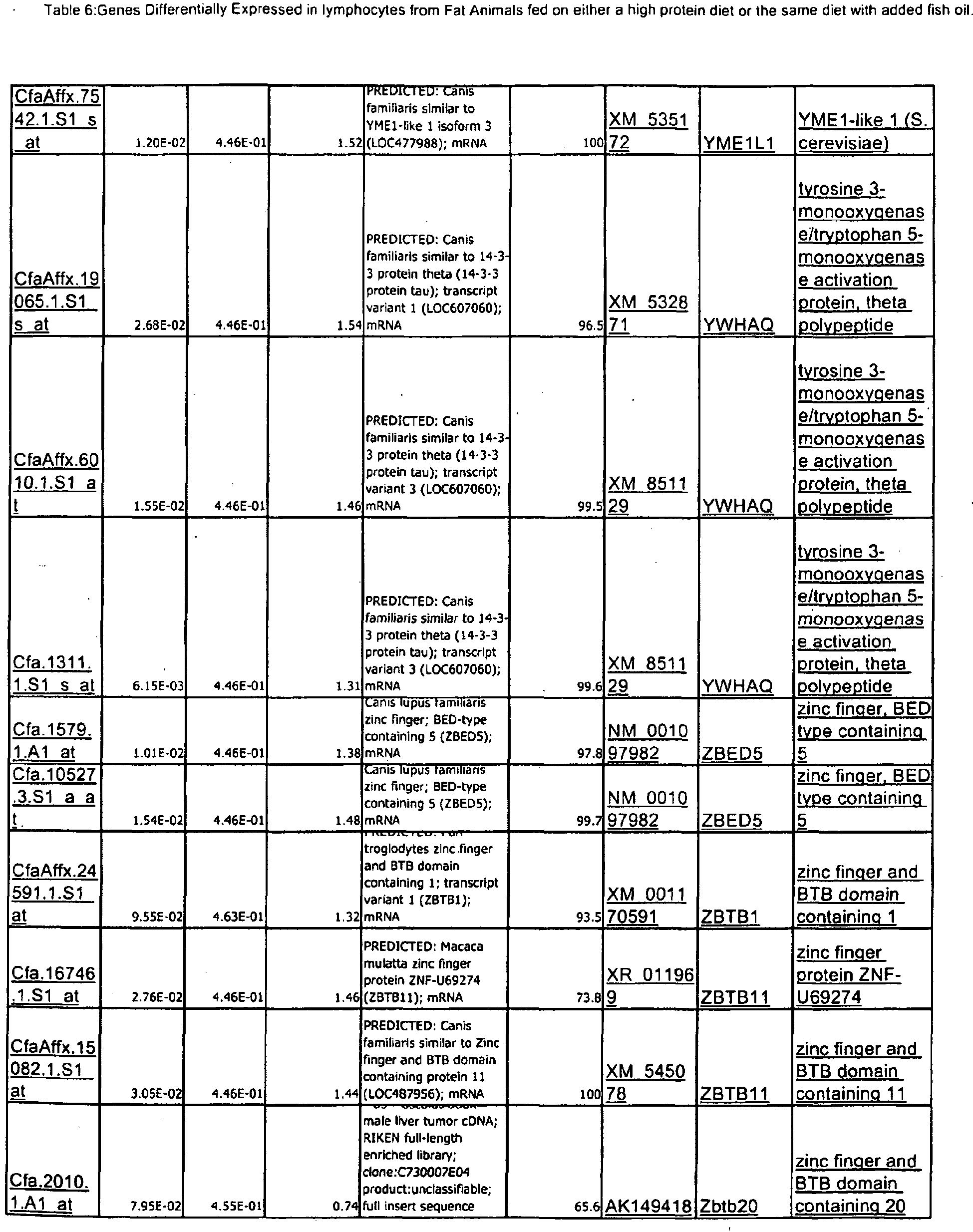

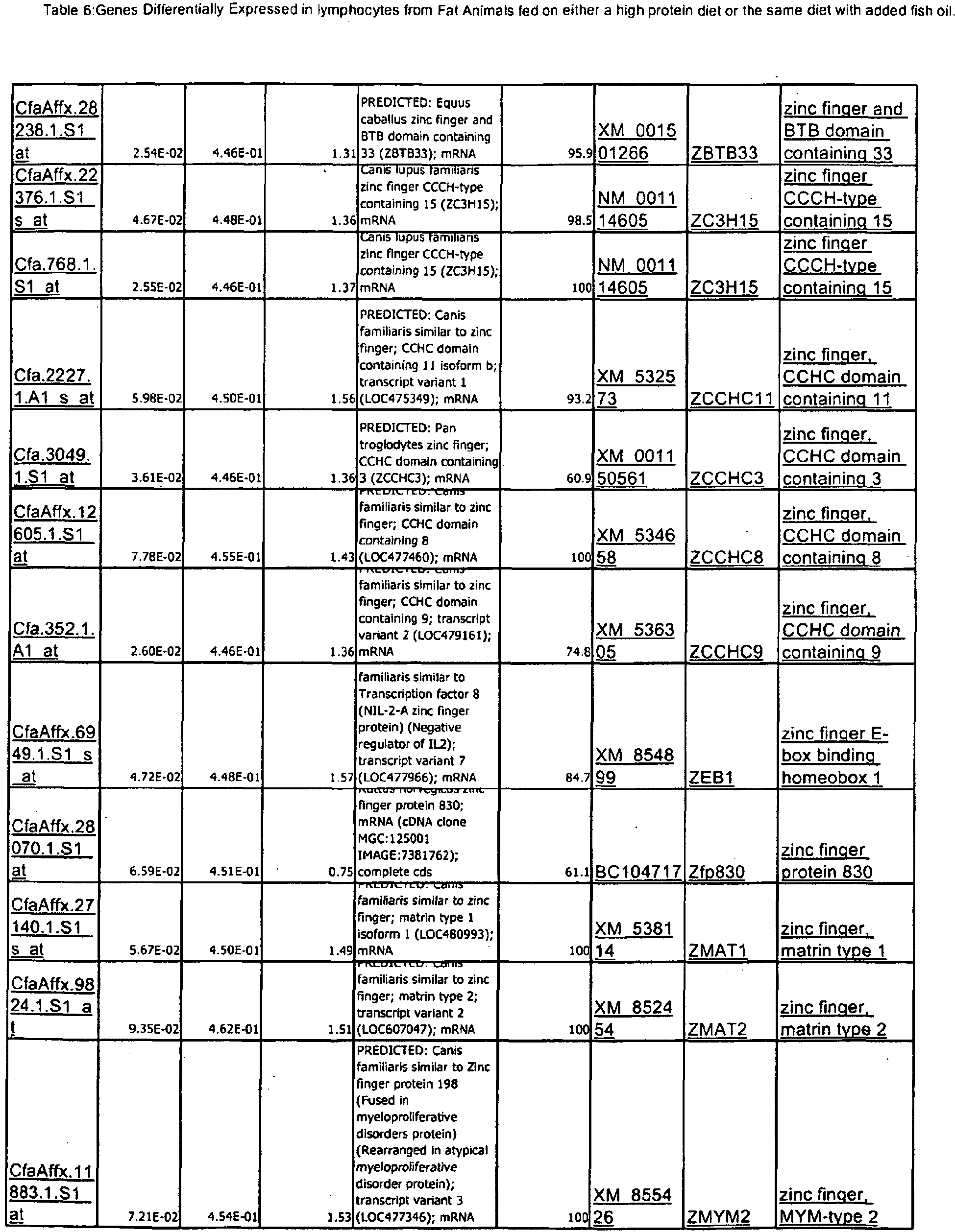

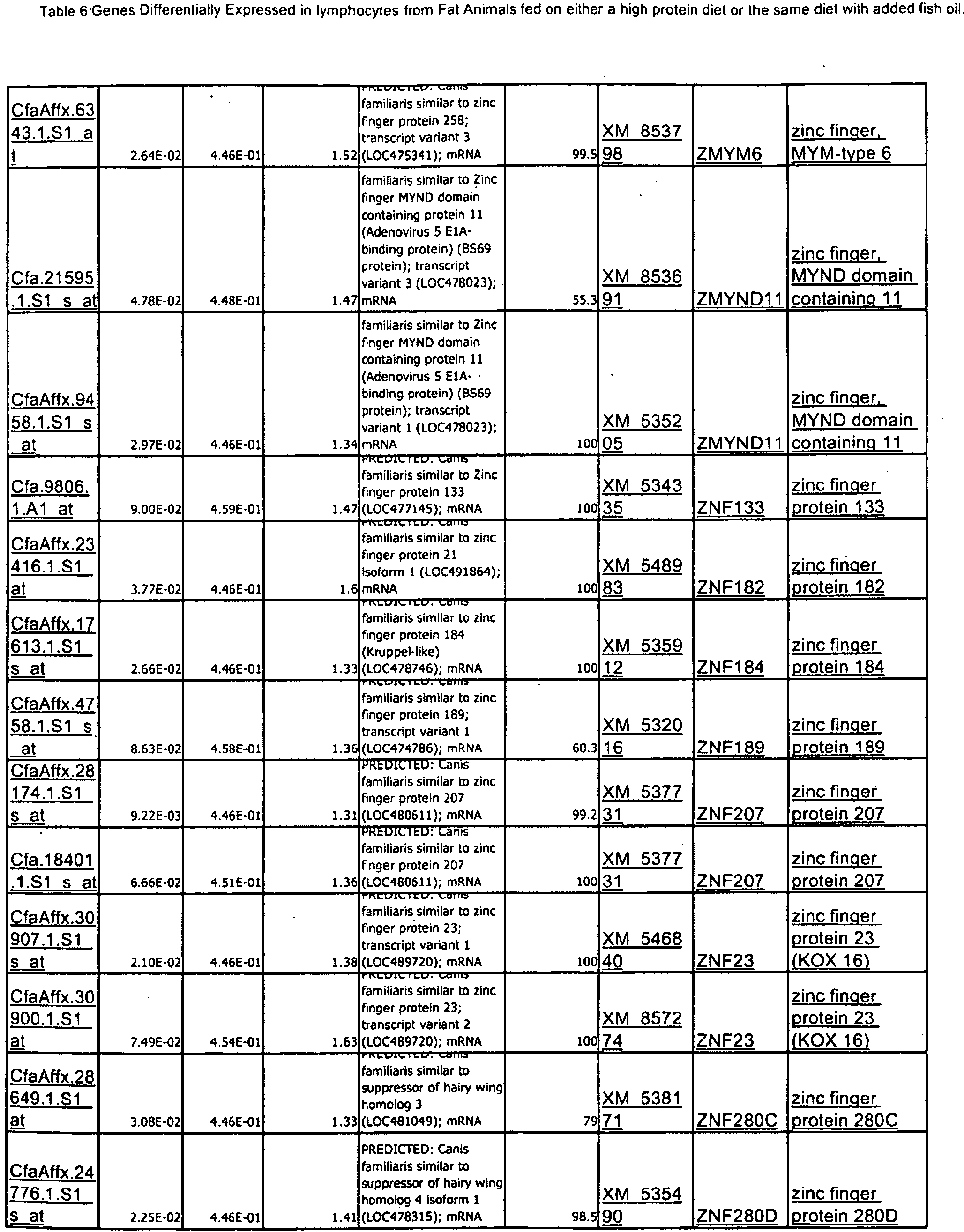

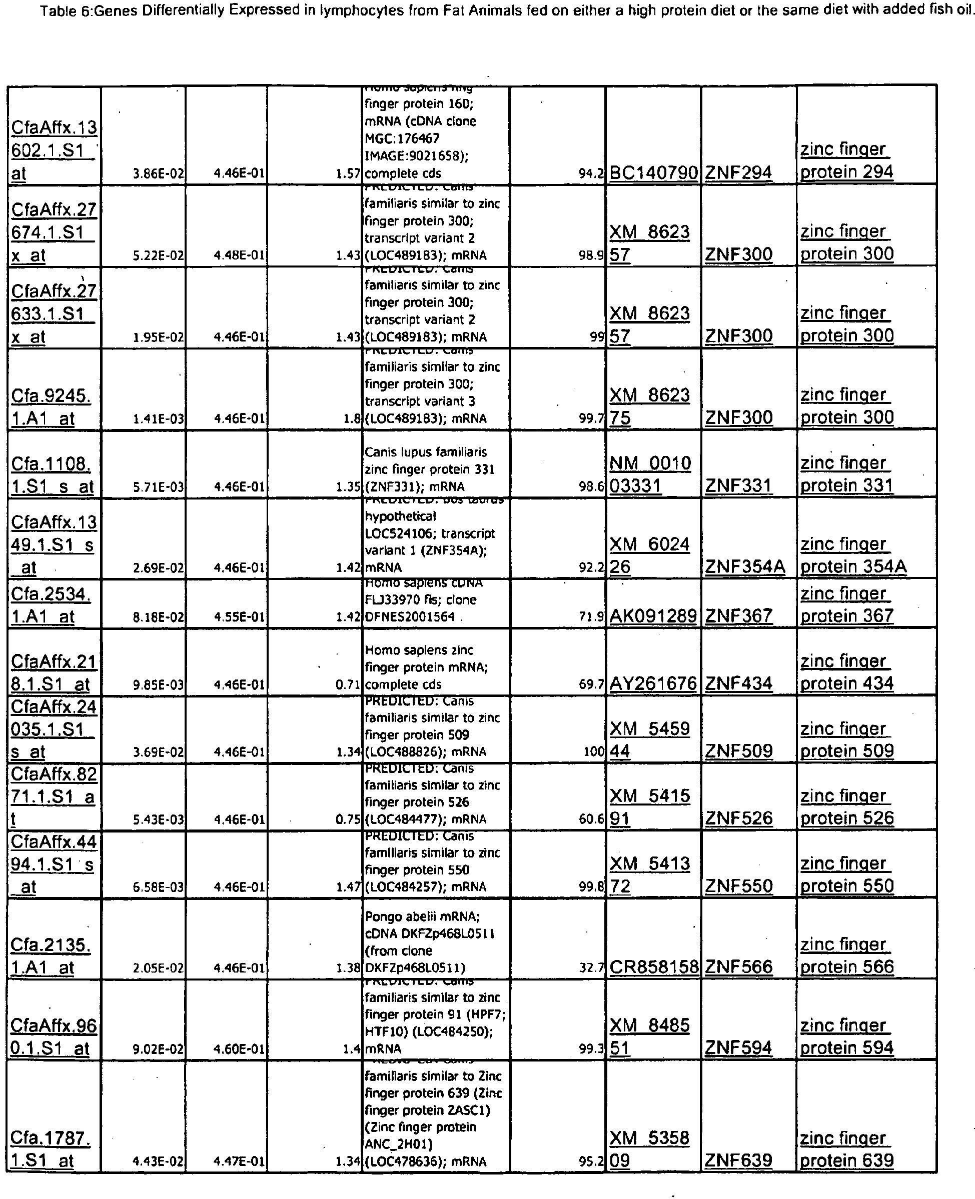

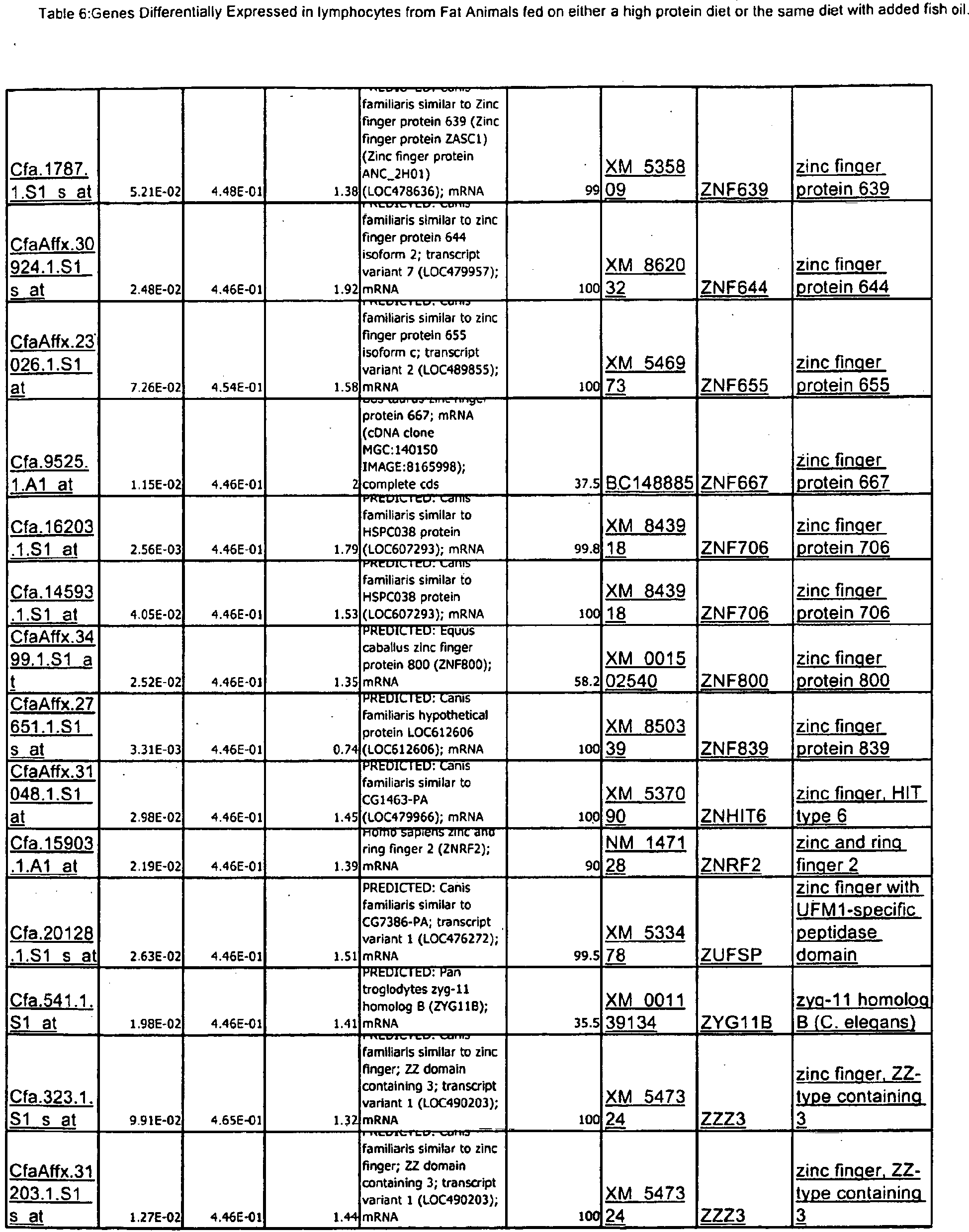

### Diets Containing Higher Amounts Of Long Chain Fatty Acids Promote Weight Loss And Can Be Used To Re-Program The Gene Expression Of The Animal So That It Reflects A Propensity To Become Lean And Potentially Maintain Leanness

The data obtained from *in vitro* ingredient screens discussed above indicate that some ingredients that are high in long chain fatty acids ( see Table 5) may have the potential to affect the expression of genes involved in fat metabolism in a way that would promote leanness of the animal as a whole. This is determined by analyzing data obtained from adipose tissue and from the ingredient assays discussed above using conventional computer algorithm analyses. Code for algorithms useful in this regard are familiar to one of skill in the art and may be developed without undue experimentation. An example of such code is provided below:

SELECT A.PROBE, TO_CHAR( AVG(DECODE(A.EXPTDAY, 'D0', GENE_NORM_INT, null))/AVG(DECODE(A.EXPTDAY,'D14', GENE_NORM_INT, null)),'99999.99999') FATLEAN_FC, STATS_T_TEST_INDEPU( A.EXPTDAY, GENE_NORM_INT) P_VALUE, B.TOP_HIT_DEF,

COUNT(DISTINCT C.INGREDIENT), COUNT(DISTINCT D.INGREDIENT)

FROM GERIATRICS_RNRM2 A, TOP_PROBE_ANNOT_2_3 B, FILT_INDIV_CELLS_2 C, FILT_ACROSS_4_CELLS_2 D WHERE A.PROBE=B.PROBE AND A.PROBE=C.PROBE (+) AND A.PROBE=D.PROBE (+) AND UPPER(A.PROBE) NOT LIKE 'AFFX%' GROUP BY A.PROBE, B.TOP_HIT_DEF HAVING STATS_T_TEST_INDEPU( A.EXPTDAY, GENE_NORM_TNT) <=.01 AND AVG(DECODE(A.EXPTDAY, 'D0', GENE_NORM_INT, null))/AVG(DECODE(A.EXPTDAY, 'D14', GENE_NORM_INT, null)) >= 5 AND SUM(DECODE(PAMCALL,'P', 1,0))= 40 ORDER BY PROBE

To confirm the findings summarized in table 5 above that some bioactive dietary components such as EPA/DHA (1.5:1) as found in fish oil are more effective in bringing weight loss in dogs compared to other bioactive dietary components by acting on key genes associated with fatty acid metabolism (as shown in table 3) three high protein diets containing either no added long chain fatty acids (Diet A) or added linolenic acid (approximately 1% based on 100% dry matter basis, Diet B) or EPA/DHA (1.5:1, approximately 0.30%: 0.20 %) (Diet C) were developed for comparison to a high fiber diet that is known to induce weight loss in dogs. In the study, 45 clinically overweight dogs are all first fed a nutritionally complete control diet for 30 days prior to the start of the test. After the initial 30 days, the dogs are randomized into 4 groups. Three of the four groups receive one of the test diets and one group is given the high fiber diet as a control for a set period of time, e.g., 4 months. Results indicate that the three experimental foods (Diets A, B and C) have substantially higher digestibility than the higher fiber food. Results also indicate that approximately 38% of the dogs consuming the food containing EPA/DHA reach their weight loss goal at 90 days. Interestingly, dogs consuming the EPA/DHA food also maintain lean muscle mass and bone mineral content. The results also indicate that, at least at the clinical level, diets containing EPA/DHA may be as effective as high fiber diets in affecting weight loss.

### Example 5

### Possible Weight Loss Maintenance Experiment

Based on the results of the weight loss experiment discussed above, it is hypothesized that animals fed a diet containing EPA/DHA will not only lose weight but also will maintain the loss for a longer period of time compared to animals fed the other test and control high fiber diets.

In order to characterize the effects of Diets A, B, and C and the high figher diet on weight loss maintenance, one could perform, for example, the following type of experiment:

Overweight animals may be fed the four different diets (as described in Example 4) until they reach an optimum level of "leanness". They may then be randomized and divided into subgroups that either continue to be fed the same test diet that they were fed previously or are switched to a maintenance diet that is nutritionally balanced but is not designed to induce or maintain weight loss and does not include appreciable amounts of linolenic acid or EPA/DHA, for example.

The animals may then be observed for a set period of time, e.g., up to 3 months, with their weights recorded daily, their body condition scores determined weekly and their percentage body fat determined on a monthly basis using conventional DEXA technologies.

## Claims

1. A composition for canine consumption comprising:
(a) 26 wt. % to 35 wt. % of crude protein on a dry matter basis;
(b) 7.5 wt. % to 8.5 wt. % of crude fat on a dry matter basis;
(c) 20 wt. % to 30 wt. % of total dietary fiber on a dry matter basis; and
(d) 10 wt. % to 20 wt. % of crude fiber on a dry matter basis.

2. The composition of claim 1, wherein the protein is present in 28 wt. % to 33 wt. %, and preferably, wherein the protein is present in 30 wt. % to 31 wt. %, and/or,
wherein the crude fat is present in 7.6 wt. % to 8.0 wt. %.

3. The composition of claim 1, wherein the total dietary fiber is present in 22 wt. % to 28 wt. %, and preferably, wherein the total dietary fiber is present in 24 wt. % to 26 %, and optionally,
wherein the crude fiber is present in 12 wt. % to 18 wt. % , and preferably wherein the crude fiber is present in 14 wt. % to 16 %.

4. A composition for feline consumption comprising:
(a) 30 wt. % to 37 wt. % of crude protein on a dry matter basis;
(b) 7.5 wt. % to 9 wt. % of crude fat on a dry matter basis;
(c) 30 wt. % to 35 wt. % of total dietary fiber on a dry matter basis; and
(d) 20 wt. % to 25 wt. % of crude fiber on a dry matter basis.

5. The composition of claim 4, wherein the protein is present in an amount of 31 wt. % to 36 wt. %, and preferably, wherein the protein is present in an amount of 33% to 35%, and/or,
wherein the crude fat is present in an amount of or 8.0 wt. %, 8.5 wt. %.

6. The composition of claim 1 , wherein the total dietary fiber is present in an amount of 31 wt. % to 34 wt. %, and optionally, wherein the crude fiber is present in an amount of 21 wt. % to 24 wt. %.

7. The composition of claim 1 or claim 4, wherein the composition further comprises at least 0.02% omega-3 polyunsaturated acid, and preferably, 0.05% to 10% omega-3 polyunsaturated fatty acid, and more preferably, 0.1% to 6% omega-3 polyunsaturated fatty acid.

8. The composition of claim 7 wherein the omega-3 polyunsaturated fatty acid is Docosahexaenoic acid (DHA) and/or Eicosapentaenoic acid (EPA).

9. The composition of claim 1 or claim 4, wherein the composition is for use in medicine.

10. The composition of claim 1 or claim 4, wherein the composition is for use in preventing or treating obesity.

11. The composition of claim 1 or claim 4, and the composition for use according to claim 10, wherein the composition is for use in preventing or treating one or more of:
insulin resistance, pancreatitis, hypothyroidism, osteoarthritis, dyslipidemia, hypertension, ocular disorders, altered kidney function, respiratory diseases, artherosclerosis, diabetes mellitus, urinary tract disease, hepatic lipdosis, hepatic dyslipidemia, hepatic lipidosis, neoplasia, oral/dental disease, dermatopathy, lameness, hyperlipidemia, glucose metabolism disorders, and coronary disease.

## Patentansprüche

1. Eine für den Verzehr durch den Hund vorgesehene Zusammensetzung, die Folgendes umfasst:
(a) 26 bis 35 Gewichtsprozent Rohproteingehalt auf Trockensubstanzbasis;
(b) 7,5 bis 8,5 Gewichtsprozent Rohfettgehalt auf Trockensubstanzbasis;
(c) 20 bis 30 Gewichtsprozent Gesamtballaststoffgehalt auf Trockensubstanzbasis; und
(d) 10 bis 20 Gewichtsprozent Rohfasergehalt auf Trockensubstanzbasis.

2. Die Anspruch 1 entsprechende Zusammensetzung, die 28 bis 33 Gewichtsprozent Protein und vorzugsweise 30 bis 31 Gewichtsprozent Protein enthält, bzw.
die 7,6 bis 8,0 Gewichtsprozent Rohfett enthält.

3. Die Anspruch 1 entsprechende Zusammensetzung, die 22 bis 28 Gewichtsprozent und vorzugsweise 24 bis 26 Gewichtsprozent Gesamtballaststoffe enthält, bzw. wahlweise,
die 12 bis 18 Gewichtsprozent Rohfaser und vorzugsweise 14 bis 16 Gewichtsprozent Rohfaser enthält.

4. Eine für den Verzehr durch die Katze vorgesehene Zusammensetzung, die Folgendes umfasst:
(a) 30 bis 37 Gewichtsprozent Rohproteingehalt auf Trockensubstanzbasis;
(b) 7,5 bis 9 Gewichtsprozent Rohfettgehalt auf Trockensubstanzbasis;
(c) 30 bis 35 Gewichtsprozent Gesamtballaststoffgehalt auf Trockensubstanzbasis; und
(d) 20 bis 25 Gewichtsprozent Rohfasergehalt auf Trockensubstanzbasis.

5. Die Anspruch 4 entsprechende Zusammensetzung, die 31 bis 36 Gewichtsprozent Protein und vorzugsweise 33 bis 35 Gewichtsprozent Protein enthält, bzw.
die 8,0, 8,5 Gewichtsprozent Rohfett enthält.

6. Die Anspruch 1 entsprechende Zusammensetzung, die 31 bis 34 Gewichtsprozent Gesamtballaststoffe und wahlweise 21 bis 24 Gewichtsprozent Gesamtballaststoffe enthält.

7. Die Anspruch 1 oder Anspruch 4 entsprechende Zusammensetzung, die ferner zumindest 0,02 % mehrfach ungesättigte Omega-3-Fettsäure und vorzugsweise 0,05 % bis 10 % mehrfach ungesättigte Omega-3-Fettsäure oder noch mehr bevorzugt 0,1 % bis 6 % mehrfach ungesättigte Omega-3-Fettsäure enthält.

8. Die Anspruch 7 entsprechende Zusammensetzung, bei welcher die mehrfach ungesättigte Omega-3-Fettsäure Docosahexaensäure (DHA) bzw. Eicosapentaensäure (EPA) ist.

9. Die Anspruch 1 oder Anspruch 4 entsprechende Zusammensetzung für den Einsatz in der Medizin.

10. Die Anspruch 1 oder Anspruch 4 entsprechende Zusammensetzung, die zum Verhindern oder Behandeln von Übergewicht eingesetzt wird.

11. Die Anspruch 1 oder Anspruch 4 entsprechende Zusammensetzung, die Anspruch 10 entsprechend zum Verhindern oder Behandeln eines oder mehrerer der Folgenden eingesetzt wird:
Insulinresistenz, Pankreatitis, Hypothyreose, Osteoarthritis, Fettstoffwechselstörungen, Bluthochdruck, Augenerkrankungen, veränderte Nierenfunktion, Atemwegserkrankungen, Artherosklerose, Diabetes mellitus, Harnwegserkrankungen, hepatische Lipidose, hepatische Lipidstörungen, Neoplasie, Mund-/Zahnerkrankungen, Dermatopathie, Lahmheit, Hyperlipidämie, Störungen des Glukosestoffwechsels und Herzerkrankungen.

## Revendications

1. Une composition destinée à l'alimentation des chiens comprenant :
(a) de 26 % à 35 % en poids de protéine brute sur une base de matière sèche ;
(b) de 7,5 % à 8,5 % en poids de matière grasse brute sur une base de matière sèche ;
(c) de 20 % à 30 % en poids de fibre alimentaire totale sur une base de matière sèche ; et
(d) de 10 % à 20 % en poids de fibre brute sur une base de matière sèche.

2. La composition de la revendication 1, dans laquelle la protéine est présente de 28 % à 33 % en poids, et de préférence, dans laquelle la protéine est présente de 30 % à 31 % en poids, et/ou,
dans laquelle la matière grasse brute est présente de 7,6 % à 8,0 % en poids.

3. La composition de la revendication 1, dans laquelle la fibre alimentaire totale est présente de 22 % à 28 % en poids, et de préférence, dans laquelle la fibre alimentaire totale est présente de 24 % à 26 % en poids, et optionnellement,
dans laquelle la fibre brute est présente de 12 % à 18 % en poids, et de préférence dans laquelle la fibre brute est présente de 14 % à 16 % en poids.

4. Une composition destinée à l'alimentation des chats comprenant :
(a) de 30 % à 37 % en poids de protéine brute sur une base de matière sèche ;
(b) de 7,5 % à 9 % en poids de matière grasse brute sur une base de matière sèche ;
(c) de 30 % à 35 % en poids de fibre alimentaire totale sur une base de matière sèche ; et
(d) de 20 % à 25 % en poids de fibre brute sur une base de matière sèche.

5. La composition de la revendication 4, dans laquelle la protéine est présente dans un montant de 31 % à 36 % en poids, et de préférence, dans laquelle la protéine est présente dans un montant de 33 % à 35 % en poids, et/ou,
dans laquelle la matière grasse brute est présente dans un montant de soit 8,0 %, 8,5 % en poids.

6. La composition de la revendication 1, dans laquelle la fibre alimentaire totale est présente dans un montant de 31 % à 34 % en poids, et optionnellement, dans laquelle la fibre brute est présente dans un montant de 21 % à 24 % en poids.

7. La composition de la revendication 1 ou de la revendication 4, dans laquelle la composition comprend en outre au moins 0,02 % d'acide polyinsaturé oméga-3, et de préférence, de 0,05 % à 10 % d'acide gras polyinsaturé oméga-3, et de manière plus préférée, de 0,1 % à 6 % d'acide gras polyinsaturé oméga-3.

8. La composition de la revendication 7, dans laquelle l'acide gras polyinsaturé oméga-3 est de l'acide docosahexaénoïque (DHA) et/ou de l'acide eicosapentaénoïque (EPA).

9. La composition de la revendication 1 ou la revendication 4, dans laquelle la composition est destinée à être utilisée en médecine.

10. La composition de la revendication 1 ou la revendication 4, dans laquelle la composition est destinée à être utilisée pour prévenir ou traiter l'obésité.

11. La composition de la revendication 1 ou la revendication 4, et la composition destinée à être utilisée selon la revendication 10, dans laquelle la composition est destinée à être utilisée pour prévenir ou traiter un des troubles suivant :
résistance à l'insuline, pancréatite, hypothyroïdie, ostéoarthrite, dyslipidémie, hypertension, troubles de la vue, fonction rénale altérée, maladies respiratoires, athérosclérose, diabète sucré, maladie des voies urinaires, lipidose hépatique, dyslipidémie hépatique, néoplasie, maladie bucco-dentaire, dermatophatie, boiterie, hyperlipidémie, troubles du métabolisme du glucose, et maladie coronarienne.
